(19) Europäisches Patentamt
European Patent Office
Office européen des brevets

(11) EP 1 365 034 A2

(12) **EUROPEAN PATENT APPLICATION**

(43) Date of publication:
**26.11.2003 Bulletin 2003/48**

(51) Int Cl.[7]: **C12Q 1/68**, B01L 3/00, A61K 48/00, G06F 17/00

(21) Application number: **03010447.5**

(22) Date of filing: **09.05.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **21.05.2002 EP 02010291**
**13.02.2003 EP 03003112**

(71) Applicant: **Bayer Aktiengesellschaft**
**51368 Leverkusen (DE)**

(72) Inventors:
- **Wirtz, Ralph, Dr.**
  **50674 Köln (DE)**
- **Munnes, Marc, Dr.**
  **40699 Erkrath (DE)**
- **Kallabis, Harald, Dr.**
  **50161 Köln (DE)**

(54) **Methods and compositions for the prediction, diagnosis, prognosis, prevention and treatment of malignant neoplasia**

(57) The invention provides compositions, methods and uses, for the prediction., diagnosis, prognosis, prevention and treatment of malignant neoplasia and breast cancer in particular. Genes that are differentially expressed in breast tissue of breast cancer patients versus those of normal people are disclosed.

EP 1 365 034 A2

SEQUENCE LISTING

<110> Bayer AG

<120> METHODS AND COMPOSITIONS FOR THE PREDICTION, DIAGNOSIS, PROGNOSIS, PREVENTION AND TREATMENT OF MALIGNANT NEOPLASIA

<130> LeA 36108.1 EP

<150> EP02010291.9

<151> 2002-05-21

<160> 314

<170> PatentIn version 3.1

<210> 1

<211> 3846

<212> DNA

<213> Homo sapiens

<400> 1
gcctcccgcc agctcgcctc ggggaacagg acgcgcgtga gctcaggcgt ccccgcccca 60
gcttttctcg gaaccatgaa ccccaactgc gcccggtgcg gcaagatcgt gtatcccacg 120
gagaaggtga actgtctgga taagttctgg cataaagcat gcttccattg cgagacctgc 180
aagatgacac tgaacatgaa gaactacaag ggctacgaga agaagcccta ctgcaacgca 240
cactacccca agcagtcctt caccatggtg gcggacaccc cggaaaacct tcgcctcaag 300
caacagagtg agctccagag tcaggtgcgc tacaaggagg agtttgagaa gaacaagggc 360
aaaggtttca gcgtagtggc agacacgccc gagctccaga gaatcaagaa gacccaggac 420
cagatcagta atataaaata ccatgaggag tttgagaaga gccgcatggg ccctagcggg 480
ggcgagggca tggagccaga gcgtcgggat tcacaggacg gcagcagcta ccggcggccc 540
ctggagcagc agcagcctca ccacatcccg accagtgccc cggtttacca gcagccccag 600
cagcagccgg tggcccagtc ctatggtggc tacaaggagc ctgcagcccc agtctccata 660
cagcgcagcg ccccaggtgg tggcgggaag cggtaccgcg cggtgtatga ctacagcgcc 720
gccgacgagg acgaggtctc cttccaggac ggggacacca tcgtcaacgt gcagcagatc 780
gacgacggct ggatgtacgg gacggtggag cgcaccggcg acacggggat gctgccggcc 840
aactacgtgg aggccatctg aacccggagc gcccccatct gtcttcagca cattccacgg 900
catcgcatcc gtcctgggcg tgagccgtcc attcttcagt gtctctgttt tttaaaacct 960

```
gcgacagctt gtgattccta cccctcttcc agcttctttt gccaactgaa gccttcttct   1020
gccacttctg cgggctccct cctctggcag gcttcccccg tgatcgactt cttggttttc   1080
tctctggatg gaacgggtat gggcctctct gggggaggca gggctggaat gggagacctg   1140
ttggcctgtg ggcctcacct gcccctctgt tctctcccct cacatcctcc tgcccagctc   1200
ctcacatacc cacacattcc agggctgggg tgagcctgac tgccaggacc ccaggtcagg   1260
ggctccctac attccccaga gtgggatcca cttcttggtt cctgggatgg cgatggggac   1320
tctgccgctg tgtagggacc agtgggatgg gctctacctc tctttctcaa agagggggct   1380
ctgcccacct ggggtctctc tccctacctc cctcctcagg ggcaacaaca ggagaatggg   1440
gttcctgctg tggggcgaat tcatcccctc cccgcgcgtt ccttcgcaca ctgtgatttt   1500
gccctcctgc ccacgcagac ctgcagcggg caaagagctc ccgaggaagc acagcttggg   1560
tcaggttctt gcctttctta attttaggga cagctaccgg aaggagggga acaaggagtt   1620
ctcttccgca gccccttcc ccacgcccac ccccagtctc cagggaccct tgcctgcctc   1680
ctaggctgga agccatggtc ccgaagtgta gggcaagggt gcctcaggac cttttggtct   1740
tcagcctccc tcagccccca ggatctgggt taggtggccg ctcctccctg ctcctcatgg   1800
gaagatgtct cagagccttc catgacctcc cctccccagc ccaatgccaa gtggacttgg   1860
agctgcacaa agtcagcagg gaccactaaa tctccaagac ctggtgtgcg gaggcaggag   1920
catgtatgtc tgcaggtgtc tgacacgcaa gtgtgtgagt gtgagtgtga gagatggggc   1980
gggggtgtgt ctgtaggtgt ctctgggcct gtgtgtgggt ggggttatgt gagggtatga   2040
agagctgtct tcccctgaga gtttcctcag aacccacagt gagaggggag ggctcctggg   2100
gcagagaagt tccttaggtt ttctttggaa tgaaattcct ccttcccccc atctctgagt   2160
ggaggaagcc caccaatctg ccctttgcag tgtgtcaggg tggaaggtaa gaggttggtg   2220
tggagttggg gctgccatag ggtctgcagc ctgctggggc taagcggtgg aggaaggctc   2280
tgtcactcca ggcatatgtt tccccatctc tgtctggggc tacagaatag ggtggcagaa   2340
gtgtcaccct gtgggtgtct ccctcggggg ctcttcccct agacctcccc ctcacttaca   2400
taaagctccc ttgaagcaag aaagagggtc ccagggctgc aaaactggaa gcacagcctc   2460
ggggatgggg agggaaagac ggtgctatat ccagttcctg ctctctgctc atgggtggct   2520
gtgacaaccc tggcctcact tgattcatct ctggttttct tgccaccctc tgggagtccc   2580
catcccattt tcatcctgag cccaaccagg ccctgccatt ggcctcttgt cccttggcac   2640
acttgtaccc acaggtgagg ggcaggacct gaaggtattg gcctgttcaa caatcagtca   2700
tcatgggtgt ttttgtcaac tgcttgttaa ttgatttggg gatgtttgcc ccgaatgaga   2760
ggttgaggaa aagactgtgg gtggggaggc cctgcctgac ccatcccttt tcctttctgg   2820
ccccagccta ggtggaggca agtggaatat cttatattgg gcgatttggg ggctcgggga   2880
ggcagagaat ctcttgggag tcttgggtgg cgctggtgca ttctgtttcc tcttgatctc   2940
aaagcacaat gtggatttgg ggaccaaagg tcagggacac atccccttag aggacctgag   3000
tttgggagag tggtgagtgg aagggaggag cagcaagaag cagcctgttt tcactcagct   3060
taattctcct tcccagataa ggcaagccag tcatggaatc ttgctgcagg ccctccctct   3120
actcttcctg tcctaaaaat aggggccgtt ttcttacaca cccccagaga gaggagggac   3180
tgtcacactg gtgctgagtg accgggggct gctgggcgtc tgttctttac caaaaccatc   3240
catccctaga agagcacaga gccctgaggg gctgggctgg gctgggctga gcccctggtc   3300
ttctctacag ttcacagagg tctttcagct catttaatcc caggaaagag gcatcaaagc   3360
tagaatgtga atataacttt tgtgggccaa tactaagaat aacaagaagc ccagtggtga   3420
ggaaagtgcg ttctcccagc actgcctcct gttttctccc tctcatgtcc ctccagggaa   3480
aatgacttta ttgcttaatt tctgcctttc ccccctcaca catgcacttt tgggcctttt   3540
tttatagctg gaaaaaacaa aataccaccc tacaaacctg tatttaaaaa gaaacagaaa   3600
tgaccacgtg aaatttgcct ctgtccaaac atttcatccg tgtgtatgtg tatgtgtgtg   3660
agtgtgtgaa gccgccagtt catcttttta tatggggttg ttgtctcatt ttggtctgtt   3720
ttggtcccct ccctcgtggg cttgtgctcg ggatcaaacc tttctggcct gttatgattc   3780
tgaacatttg acttgaacca caagtgaatc tttctcctgg tgactcaaat aaaagtataa   3840
ttttta                                                              3846
```

<210> 2

<211> 1711

<212> DNA

<213> Homo sapiens

<400> 2
gagggaaggc aggaaggagg cagccgaagg ccgagctggg tggctggacc gggtgctggc 60
tgcgcgcgct gctttcggct cccacggcct ctcccatgcg ctgagggagc ccggctgcgg 120
gccggcggcg ggagggggagg ctcctctcca tggtccagaa gaccagcatg tcccggggcc 180

cttacccacc ctcccaggag atccccatgg aggtcttcga ccccagcccg cagggcaaat 240
acagcaagag gaaagggcga ttcaaacggt cagatgggag cacgtcctcg gataccacat 300
ccaacagctt tgtccgccag ggctcagcgg agtcctacac cagccgtcca tcagactctg 360
atgtatctct ggaggaggac cgggaagcct taaggaagga agcagagcgc caggcattag 420
cgcagctcga gaaggccaag accaagccag tggcatttgc tgtgcggaca aatgttggct 480
acaatccgtc tccaggggat gaggtgcctg tgcagggagt ggccatcacc ttcgagccca 540
aagacttcct gcacatcaag gagaaataca ataatgactg gtggatcggg cggctggtga 600
aggagggctg tgaggttggc ttcattccca gccccgtcaa actggacagc cttcgcctgc 660
tgcaggaaca gaagctgcgc cagaaccgcc tcggctccag caaatcaggc gataactcca 720
gttccagtct gggagatgtg gtgactggca cccgccgccc cacacccccct gccagtgcca 780
aacagaagca gaagtcgaca gagcatgtgc cccccctatga cgtggtgcct tccatgaggc 840
ccatcatcct ggtgggaccg tcgctcaagg gctacgaggt tacagacatg atgcagaaag 900
ctttatttga cttcttgaag catcggtttg atggcaggat ctccatcact cgtgtgacgg 960
cagatatttc cctggctaag cgctcagttc tcaacaaccc cagcaaacac atcatcattg 1020
agcgctccaa cacacgctcc agcctggctg aggtgcagag tgaaatcgag cgaatcttcg 1080
agctggcccg gacccttcag ttggtcgctc tggatgctga caccatcaat cacccagccc 1140
agctgtccaa gacctcgctg gcccccatca ttgtttacat caagatcacc tctcccaagg 1200
tacttcaaag gctcatcaag tcccgaggaa agtctcagtc caaacacctc aatgtccaaa 1260
tagcggcctc ggaaaagctg gcacagtgcc cccctgaaat gtttgacatc atcctggatg 1320
agaaccaatt ggaggatgcc tgcgagcatc tggcggagta cttggaagcc tattggaagg 1380
ccacacaccc gcccagcagc acgccaccca atccgctgct gaaccgcacc atggctaccg 1440
cagccctgcg ccgtagccct gcccctgtct ccaacctcca ggtacaggtg ctcacctcgc 1500
tcaggagaaa cctcggcttc tggggcgggc tggagtcctc acagcggggc agtgtggtgc 1560
cccaggagca ggaacatgcc atgtagtggg cgccctgccc gtcttccctc ctgctctggg 1620
gtcggaactg gagtgcaggg aacatggagg aggaagggaa gagctttatt ttgtaaaaaa 1680
ataagatgag cggcaaaaaa aaaaaaaaaa a 1711

<210> 3

<211> 698

<212> DNA

<213> Homo sapiens

<400> 3
ttttcctttc gctgctgcgg ccgcagccat gagtatgctc aggcttcaga agaggctcgc 60
ctctagtgtc ctccgctgtg gcaagaagaa ggtctggtta gaccccaatg agaccaatga 120
aatcgccaat gccaactccc gtcagcagat ccggaagctc atcaaagatg ggctgatcat 180
ccgcaagcct gtgacggtcc attcccgggc tcgatgccgg aaaaacacct tggcccgccg 240
gaagggcagg cacatgggca taggtaagcg gaagggtaca gccaatgccc gaatgccaga 300
gaaggtcaca tggatgagga gaatgaggat tttgcgccgg ctgctcagaa gataccgtga 360
atctaagaag atcgatcgcc acatgtatca cagcctgtac ctgaaggtga aggggaatgt 420
gttcaaaaac aagcggattc tcatggaaca catccacaag ctgaaggcag acaaggcccg 480
caagaagctc ctggctgacc aggctgaggc ccgcaggtct aagaccaagg aagcacgcaa 540
gcgccgtgaa gagcgcctcc aggccaagaa ggaggagatc atcaagactt tatccaagga 600
ggaagagacc aagaaataaa acctcccact ttgtctgtac atactggcct ctgtgattac 660
atagatcagc cattaaaata aaacaagcct taatctgc 698

<210> 4

<211> 5810

<212> DNA

<213> Homo sapiens

<400> 4
gggaagatgg cggcggcctc gagcaccctc ctcttcttgc cgccggggac ttcagattga 60
tccttcccgg gaagagtagg gactgctggt gccctgcgtc ccgggatccc gagccaactt 120
gtttcctccg ttagtggtgg ggaagggctt atccttttgt ggcggatcta gcttctcctc 180
gccttcagga tgaaagctca gggggaaac cgaggagtca gaaaagctga gtaagatgag 240
ttctctcctg gaacggctcc atgcaaaatt taaccaaaat agaccctgga gtgaaaccat 300
taagcttgtg cgtcaagtca tggagaagag ggttgtgatg agttctggag ggcatcaaca 360
tttggtcagc tgtttggaga cattgcagaa ggctctcaaa gtaacatctt taccagcaat 420
gactgatcgt ttggagtcca tagcaggaca gaatggactg ggctctcatc tcagtgccag 480
tggcactgaa tgttacatca cgtcagatat gttctatgtg gaagtgcagt tagatcctgc 540
aggacagctt tgtgatgtaa aagtggctca ccatggggag aatcctgtga gctgtccgga 600
gcttgtacag cagctaaggg aaaaaaattc tgatgaattt tctaagcacc ttaagggcct 660
tgttaatctg tataaccttc caggggacaa caaactgaag actaaaatgt acttggctct 720
ccaatcctta gaacaagatc tttctaaaat ggcaattatg tactggaaag caactaatgc 780
tggtcccttg gataagattc ttcatggaag tgttggctat ctcacaccaa ggagtggggg 840
tcatttaatg aacctgaagt actatgtctc tccttctgac ctactggatg acaagactgc 900
atctcccatc attttgcatg agaataatgt ttctcgatct ttgggcatga atgcatcagt 960
gacaattgaa ggaacatctg ctgtgtacaa actcccaatt gcaccattaa ttatggggtc 1020
acatccagtt gacaataaat ggacccctte cttctcctca atcaccagtg ccaacagtgt 1080
tgatcttcct gcctgtttct tcttgaaatt tccccagcca atcccagtat ctagagcatt 1140
tgttcagaaa ctgcagaact gcacaggaat tccattgttt gaaactcaac caacttatgc 1200
accccctgtat gaactgatca ctcagtttga gctatcaaag gaccctgacc ccataccttt 1260
gaatcacaac atgagatttt atgctgctct tcctggtcag cagcactgct atttcctcaa 1320
caaggatgct cctcttccag atggccgaag tctacaggga acccttgtta gcaaaatcac 1380
ctttcagcac cctggccgag ttcctcttat cctaaatctg atcagacacc aagtggccta 1440
taacaccctc attggaagct gtgtcaaaag aactattctg aaagaagatt ctcctgggct 1500
tctccaattt gaagtgtgtc ctctctcaga gtctcgtttc agcgtatctt ttcagcaccc 1560
tgtgaatgac tccctggtgt gtgtggtaat ggatgtgcag ggcttaacac atgtgagctg 1620
taaactctac aaagggctgt cggatgcact gatctgcaca gatgacttca ttgccaaagt 1680
tgttcaaaga tgtatgtcca tccctgtgac gatgagggct attcggagga aagctgaaac 1740
cattcaagcc gacaccccag cactgtccct cattgcagag acagttgaag acatggtgaa 1800
aaagaacctg cccccggcta gcagcccagg gtatggcatg accacaggca acaacccaat 1860
gagtggtacc actacatcaa ccaacacctt tccgggggggt cccattgcca ccttgtttaa 1920
tatgagcatg agcatcaaag atcggcatga gtcggtgggc catggggagg acttcagcaa 1980
ggtgtctcag aacccaattc ttaccagttt gttgcaaatc acagggaacg gggggtctac 2040
cattggctcg agtccgaccc ctcctcatca cacgccgcca cctgtctctt cgatggccgg 2100
caacaccaag aaccacccga tgctcatgaa ccttctcaaa gataatcctg cccaggattt 2160
ctcaaccctt tatggaagca gccctttaga aaggcagaac tcctcttccg gctcaccccg 2220
catggaaata tgctcgggga gcaacaagac caagaaaaag aagtcatcaa gattaccacc 2280
tgagaaacca aagcaccaga ctgaagatga ctttcagagg gagctatttt caatggatgt 2340
tgactcacag aaccctatct ttgatgtcaa catgacagct gacacgctgg atacgccaca 2400
catcactcca gctccaagcc agtgtagcac tcccccaaca acttacccac aaccagtacc 2460
tcaccccccaa cccagtattc aaaggatggt ccgactatcc agttcagaca gcattggccc 2520
agatgtaact gacatccttt cagacattgc agaagaagct tctaaacttc ccagcactag 2580
tgatgattgc ccagccattg gcacccctct tcgagattct tcaagctctg ggcattctca 2640
gagtaccctg tttgactctg atgtctttca aactaacaat aatgaaaatc catacactga 2700
tccagctgat cttattgcag atgctgctgg aagccccagt agtgactctc ctaccaatca 2760
tttttttcat gatggagtag atttcaatcc tgatttattg aacagccaga gccaaagtgg 2820
ttttggagaa gaatattttg atgaaagcag ccaaagtggg gataatgatg atttcaaagg 2880

```
atttgcatct caggcactaa atactttggg ggtgccaatg cttggaggtg ataatgggga   2940
gaccaagttt aagggcaata accaagccga cacagttgat ttcagtatta tttcagtagc   3000
cggcaaagct ttagctcctg cagatcttat ggagcatcac agtggtagtc agggtccttt   3060
actgaccact ggggacttag ggaaagaaaa gactcaaaag agggtaaagg aaggcaatgg   3120
caccagtaat agtactctct cggggcccgg attagacagc aaaccaggga agcgcagtcg   3180
gaccccttct aatgatggga aaagcaaaga taagcctcca aagcggaaga aggcagacac   3240
tgagggaaag tctccatctc atagttcttc taacagacct tttacccccac ctaccagtac   3300
aggtggatct aaatcgccag gcagtgcagg aagatctcag actcccccag gtgttgccac   3360
accacccatt cccaaaatca ctattcagat tcctaaggga acagtgatgg tgggcaagcc   3420
ttcctctcac agtcagtata ccagcagtgg ttctgtgtct tcctcaggca gcaaaagcca   3480
ccatagccat tcttcctcct cttcctcatc tgcttccacc tcagggaaga tgaaaagcag   3540
taaatcagaa ggttcatcaa gttccaagtt aagtagcagt atgtattcta gccaggggtc   3600
ttctggatct agccagtcca aaaattcatc ccagtctggg gggaagccag gctcctctcc   3660
cataaccaag catggactga gcagtggctc tagcagcacc aagatgaaac ctcaaggaaa   3720
gccatcatca cttatgaatc cttctttaag taaaccaaac atatcccctt ctcattcaag   3780
gccacctgga ggctctgaca agcttgcctc tccaatgaag cctgttcctg gaactcctcc   3840
atcctctaaa gccaagtccc ctatcagttc aggttctggt ggttctcata tgtctggaac   3900
tagttcaagc tctggcatga agtcatcttc agggttagga tcctcaggct cgttgtccca   3960
gaaaactccc ccatcatcta attcctgtac ggcatcttcc tcctcctttt cctcaagtgg   4020
ctcttccatg tcatcctctc agaaccagca tgggagttct aaaggaaaat ctcccagcag   4080
aaacaagaag ccgtccttga cagctgtcat agataaactg aagcatgggg ttgtcaccag   4140
tggccctggg ggtgaagacc cactggacgg ccagatgggg gtgagcacaa attcttccag   4200
ccatcctatg tcctccaaac ataacatgtc aggaggagag tttcagggca agcgtgagaa   4260
aagtgataaa gacaaatcaa aggtttccac ctccgggagt tcagtggatt cttctaagaa   4320
gacctcagag tcaaaaaatg tggggagcac aggtgtggca aaaattatca tcagtaagca   4380
tgatggaggc tcccctagca ttaaagccaa agtgactttg cagaaacctg ggaaagtag    4440
tggagaaggg cttaggcctc aaatggcttc ttctaaaaac tatggctctc cactcatcag   4500
tggttccact ccaaagcatg agcgtggctc tcccagccat agtaagtcac cagcatatac   4560
cccccagaat ctggacagtg aaagtgagtc aggctcctcc atagcagaga aatcttatca   4620
gaatagtccc agctcagacg atggtatccg accacttcca gaatacagca cagagaaaca   4680
taagaagcac aaaaaggaaa agaagaaagt aaaagacaaa gatagggacc gagaccggga   4740
caaagaccga gacaagaaaa aatctcatag catcaagcca gagagttggt ccaaatcacc   4800
catctcttca gaccagtcct tgtctatgac aagtaacaca atcttatctg cagacagacc   4860
ctcaaggctc agcccagact ttatgattgg ggaggaagat gatgatctta tggatgtggc   4920
cctgattggg aattaggaac cttatttcct aaaagaaaca gggccagagg aaaaaaaact   4980
attgataagt ttataggcaa accaccataa ggggtgagtc agacaggtct gatttggtta   5040
agaatcctaa atggcatggc tttgacatca agctgggtga attagaaagg catatccaga   5100
ccctattaaa gaaaccacag ggtttgattc tggttaccag gaagtcttct ttgttcctgt   5160
gccagaaaga aagttaaaat acttgcttaa gaaagggagg ggggtgggag gggtgtaggg   5220
agagggaagg gagggaaaca gttttgtggg aaatattcat atatatttc ttctcccttt    5280
ttccattttt aggccatgtt ttaaactcat tttagtgcat gtatatgaag ggctgggcag   5340
aaaatgaaaa agcaatacat tccttgatgc atttgcatga aggttgttca actttgtttg   5400
aggtagttgt ccgtttgagt catgggcaaa tgaaggactt tggtcatttt ggacacttaa   5460
gtaatgtttg gtgtctgttt cttaggagtg actgggggag ggaagattat tttagctatt   5520
tatttgtaat attttaaccc tttatctgtt tgtttttata cagtgtttcg ttctaaatct   5580
atgaggttta gggttcaaaa tgatggaagg ccgaagagca aggcttatat ggtggtaggg   5640
agcttatagc ttgtgctaat actgtagcat caagcccaag caaattagtc agagcccgcc   5700
tttagagtta aatataatag aaaaaccaaa atgatatttt tattttagga gggtttaaat   5760
agggttcaga gatcatagga atattaggag ttacctctct gtggaggtat              5810
```

<210> 5

<211> 5515

<212> DNA

<213> Homo sapiens

<400> 5

```
ctttttcccc ttcttcaggt caggggaaag ggaatgccca attcagagag acatgggggc      60
aagaaggacg ggagtggagg agcttctgga actttgcagc cgtcatcggg aggcggcagc     120
tctaacagca gagagcgtca ccgcttggta tcgaagcaca agcggcataa gtccaaacac     180
tccaaagaca tggggttggt gaccccggaa gcagcatccc tgggcacagt tatcaaacct     240
ttggtggagt atgatgatat cagctctgat tccgacacct tctccgatga catggccttc     300
aaactagacc gaagggagaa cgacgaacgt cgtggatcag atcggagcga ccgcctgcac     360
aaacatcgtc accaccagca caggcgttcc cgggacttac taaaagctaa acagaccgaa     420
aaagaaaaaa gccaagaagt ctccagcaag tcgggatcga tgaaggaccg gatatcggga     480
agttcaaagc gttcgaatga ggagactgat gactatggga aggcgcaggt agccaaaagc     540
agcagcaagg aatccaggtc atccaagctc cacaaggaga agaccaggaa agaacgggag     600
ctgaagtctg ggcacaaaga ccggagtaaa agtcatcgaa aaagggaaac acccaaaagt     660
tacaaaacag tggacagccc aaaacggaga tccaggagcc cccacaggaa gtggtctgac     720
agctccaaac aagatgatag cccctcggga gcttcttatg gccaagatta tgaccttagt     780
ccctcacgat ctcatacctc gagcaattat gactcctaca agaaaagtcc tggaagtacc     840
tcgagaaggc agtcggtcag tccccccttac aaggagcctt cggcctacca gtccagcacc     900
cggtcaccga gcccctacag taggcgacag agatctgtca gtccctatag caggagacgg     960
tcgtccagct acgaaagaag tggctcttac agcgggcgat cgcccagtcc ctatggtcga    1020
aggcggtcca gcagcccttt cctgagcaag cggtctctga gtcggagtcc actccccagt    1080
aggaaatcca tgaagtccag aagtagaagt cctgcatatt caagacattc atcttctcat    1140
agtaaaaaga agagatccag ttcacgcagt cgtcattcca gtatctcacc tgtcaggctt    1200
ccacttaatt ccagtctggg agctgaactc agtaggaaaa agaaggaaag agcagctgct    1260
gctgctgcag caaagatgga tggaaaggag tccaagggtt cacctgtatt tttgcctaga    1320
aaagagaaca gttcagtaga ggctaaggat tcaggtttgg agtctaaaaa gttacccaga    1380
agtgtaaaat tggaaaaatc tgccccagat actgaactgg tgaatgtaac acatctaaac    1440
acagaggtaa aaaattcttc agatacaggg aaagtaaagt tggatgagaa ctccgagaag    1500
catcttgtta aagatttgaa agcacaggga acaagagact ctaaacccat agcactgaaa    1560
gaggagattg ttactccaaa ggagacagaa acatcagaaa aggagacccc tccacctctt    1620
cccacaattg cttctccccc acccccctcta ccaactacta cccctccacc tcagacaccc    1680
cctttgccac ctttgcctcc aataccagct cttccacagc aaccacctct gcctccttct    1740
cagccagcat ttagtcaggt tcctgcttcc agtacttcaa ctttgccccc ttctactcac    1800
tcaaagacat ctgctgtgtc ctctcaggca aattctcagc cccctgtaca ggtttctgtg    1860
aagactcaag tatctgtaac agctgctatt ccacacctga aaacttcaac gttgcctcct    1920
ttgcccctcc caccccttatt acctggaggt gatgacatgg atagtccaaa agaaactctt    1980
ccttcaaaac ctgtgaagaa agagaaggaa cagaggacac gtcacttact cacagacctt    2040
cctctccctc cagagctccc tggtggagat ctgtctcccc cagactctcc agaaccaaag    2100
gcaatcacac cacctcagca accatataaa aagagaccaa aaatttgttg tcctcgttat    2160
ggagaaagaa gacaaacaga aagcgactgg gggaaacgct gtgtggacaa gtttgacatt    2220
attgggatta ttggagaagg aacctatggc caagtatata aagccaggga caaagacaca    2280
ggagaactag tggctctgaa gaaggtgaga ctagacaatg agaaagaggg cttcccaatc    2340
acagccattc gtgaaatcaa aatccttcgt cagttaatcc accgaagtgt tgttaacatg    2400
aaggaaattg tcacagataa acaagatgca ctggatttca agaaggacaa aggtgccttt    2460
taccttgtat ttgagtatat ggaccatgac ttaatgggac tgctagaatc tggtttggtg    2520
cacttttctg aggaccatat caagtcgttc atgaaacagc taatggaagg attggaatac    2580
tgtcacaaaa agaatttcct gcatcgggat attaagtgtt ctaacatttt gctgaataac    2640
agtgggcaaa tcaaactagc agattttgga cttgctcggc tctataactc tgaagagagt    2700
cgccccttaca caaacaaagt cattactttg tggtaccgac ctccagaact actgctagga    2760
gaggaacgtt acacaccagc catagatgtt tggagctgtg gatgtattct tggggaacta    2820
ttcacaaaga agcctatttt tcaagccaat ctggaactgg ctcagctaga actgatcagc    2880
cgactttgtg gtagcccttg tccagctgtg tggcctgatg ttatcaaact gccctacttc    2940
aacaccatga aaccgaagaa gcaatatcga aggcgtctac gagaagaatt ctctttcatt    3000
ccttctgcag cacttgattt attggaccac atgctgacac tagatcctag taagcggtgc    3060
acagctgaac agaccctaca gagcgacttc cttaaagatg tcgaactcag caaaatggct    3120
cctccagacc tcccccactg gcaggattgc catgagttgt ggagtaagaa acggcgacgt    3180
cagcgacaaa gtggtgttgt agtcgaagag ccacctccat ccaaaacttc tcgaaaagaa    3240
actacctcag ggacaagtac tgagcctgtg aagaacagca gcccagcacc acctcagcct    3300
gctcctggca aggtggagtc tggggctggg gatgcaatag gccttgctga catcacacaa    3360
```

```
cagctgaatc aaagtgaatt ggcagtgtta ttaaacctgc tgcagagcca aaccgacctg   3420
agcatccctc aaatggcaca gctgcttaac atccactcca acccagagat gcagcagcag   3480
ctggaagccc tgaaccaatc catcagtgcc ctgacggaag ctacttccca gcagcaggac   3540
tcagagacca tggccccaga ggagtctttg aaggaagcac cctctgcccc agtgatcctg   3600
ccttcagcag aacagatgac ccttgaagct tcaagcacac cagctgacat gcagaatata   3660
ttggcagttc tcttgagtca gctgatgaaa acccaagagc cagcaggcag tctggaggaa   3720
aacaacagtg acaagaacag tgggccacag ggccccgaa gaactcccac aatgccacag   3780
gaggaggcag cagcatgtcc tcctcacatt cttccaccag agaagaggcc ccctgagccc   3840
cccggacctc caccgccgcc acctccaccc cctctggttg aaggcgatct ttccagcgcc   3900
ccccaggagt tgaacccagc cgtgacagcc gccttgctgc aactttatc ccagcctgaa   3960
gcagagcctc ctggccacct gccacatgag caccaggcct tgagaccaat ggagtactcc   4020
acccgacccc gtccaaacag gacttatgga aacactgatg ggcctgaaac agggttcagt   4080
gccattgaca ctgatgaacg aaactctggt ccagccttga cagaatcctt ggtccagacc   4140
ctggtgaaga acaggacctt ctcaggctct ctgagccacc ttggggagtc cagcagttac   4200
cagggcacag ggtcagtgca gtttccaggg gaccaggacc tccgttttgc cagggtcccc   4260
ttagcgttac acccggtggt cgggcaacca ttcctgaagg ctgagggaag cagcaattct   4320
gtggtacatg cagagaccaa attgcaaaac tatggggagc tggggccagg aaccactggg   4380
gccagcagct caggagcagg ccttcactgg ggggcccaa ctcagtcttc tgcttatgga   4440
aaactctatc ggggcctac aagagtccca ccaagagggg gaagagggag aggagttcct   4500
tactaaccca gagacttcag tgtcctgaaa gattcctttc ctatccatcc ttccatccag   4560
ttctctgaat ctttaatgaa atcatttgcc agagcgaggt aatcatctgc atttggctac   4620
tgcaaagctg tccgttgtat tccttgctca cttgctacta gcaggcgact taggaaataa   4680
tgatgttggc accagttccc cctggatggg ctatagccag aacatttact tcaactctac   4740
cttagtagat acaagtagag aatatggaga ggatcattac attgaaaagt aaatgtttta   4800
ttagttcatt gcctgcactt actggtcgga agagagaaag aacagtttca gtattgagat   4860
ggctcaggag aggctctttg atttttaaag ttttggggtg gggggttgtg tgtggtttct   4920
ttcttttgaa ttttaattta ggtgttttgg gtttttttcc tttaaagaga atagtgttca   4980
caaaatttga gctgctcttt ggcttttgct ataagggaaa cagagtggcc tggctgattt   5040
gaataaatgt ttctttcctc tccaccatct cacattttgc ttttaagtga acactttttc   5100
cccattgagc atcttgaaca tacttttttt ccaaataaat tactcatcct taaagtttac   5160
tccactttga caaaagatac gcccttctcc ctgcacataa agcaggttgt agaacgtggc   5220
attcttgggc aagtaggtag actttaccca gtctctttcc tttttgctg atgtgtgctc   5280
tctctctctc tttctctctc tctctctctc tctctctctc tctgtctgtc tcgcttgctc   5340
gctctcgctg tttctctctc tttgaggcat ttgtttggaa aaaatcgttg agatgcccaa   5400
gaacctggga taattcttta ctttttttga aataaaggaa aggaaattca aaaaaaaaaa   5460
aaaaaaaaaa aaaaaaaaaa aaaaaaaaaa aaaaaaaaaa aaaaaaaaaa aaaaa        5515
```

<210> 6

<211> 6131

<212> DNA

<213> Homo sapiens

<400> 6

```
gaattctagg cccagttctg tgtttcccct gtgtgttcct aggcaggtca gtttccctcc     60
atgggcctct gtaagatgag gagttggaga ggtacattct caggctactt tcaactccca    120
gccaagtgac tcaagagtcc caggcagcac cagcacccct atctccaagg cctcctgatg    180
tgtgtctcta tttagaactt aatccaacct acccaacatc agatcagtgt cttaccagcc    240
caaggtccct ggggagcctc ctagagggag agagccctgc ccacccagat tgagggtaaa    300
ggcctccccg tgctcatttt tgtaccacca cagtgcttgg cacatggtag acatcaaaat    360
gtgtgtgctg aaagtataat tgaagttgtg tatatatgtc agctagagtg tctggagggg    420
cagaaatgtg ggtctaaaac atacaaatgc tccaaatggg gtgtgggcaa gggtctgtct    480
acaccaggct gtgattacct gctcacatac atgtgtctat ctgagtaggg gtatgttatc    540
tatttttcta caccacaggg tgaggaacag gtatatgtgt gcatgtgtat gcatccgtgt    600
gtgtgtgtat gtgtgtgtgc atgagtgtgt gtgtgtgtgt ccaaagccac ctcttcaacc    660
```

```
tgtgccattt gtatctgtgt ctggcccaat gagagtgttg aaaggtgagc cacaagataa    720
aacagcaact tcctacctcc cttatcaaga cagctgtctg acctacctcc ccttggccac    780
tcttgggatt actggggttg gcttcagtat tttcagattt ttcagaaggg gaggagaatg    840
cttgagtctc atccaggaac ttaggcagtt ctcagcactg cctgctcctc ctccctcaaa    900
taaccaagtc tgaagaccag gagagaaagc cgctggtgga ctggtcacct gtctggcagt    960
gggaggagga gagtgagagg tttctaggta ggaatccaga cttagaccct cccctccacc   1020
cccagatggg tggtgcacag gctcatctcg cggcccctcc ccactccacc ctaacatgga   1080
tacgcccccca acaaccaagg aaagatctcc catcggctga ctccacagat acacacatgt   1140
ccccacagac acacacacgc ccatgcagag gcacagacat ccaggcacat ctttcccttt   1200
ctctgtcttt cccttggttt gaatttcgtt tagccacata tgttgtgtgt gcgtgagggt   1260
gggtggggga ggggcagaca gggatgaggg atggcatggt gccaacatct acctatgggg   1320
ctcgggccag ggacgcccct tacagccatc ctgggagggg gtctcagctg tccctttgtg   1380
gccaagggga ccctcctggg gagtggggc aagcacagag gtcctttctc cccaacccgg   1440
ggtctggtcc ctgacccacc ttggggggcct gcaggggagg aaatggacag agcgggaccc   1500
tgagggagca tagaattggc caccacgagc cccagtgtc cagccttgcc accccattgt   1560
tcccgtgagg gggtctctat atacagggg caactcctcc caccttcctc tcaatccctg   1620
ctttccctgc gttgggcggg gaggggaggg cggcagaaat atttatttat ttcctttatt   1680
tatttaattt tttttttttt tttttggagt agagagtgac agatggcggc gggtcccggg   1740
ggagccggct ctcccccagt gcagacgcat gccaatcacc gtctctcatg tgatagctgc   1800
tgcccgtgac gtgccaagcc catatggcct ggcatagagg ctggtacccc gcctggtaga   1860
gatgccacac tcgctccgcg gttcgcatgg cgctctgaag acgccggcgc ccgccgcctt   1920
gaggagccgc tgcccccgct ccctgaagat gggggaacaa tgaaataagc gagaagatcc   1980
ctcttctccc ccctctctct cttgcccccct cccccccctcc cctcccctct ccccttgact   2040
cctctccgag gtaagttgtc cgaaagggag cgagatctga cccgccggtt gggaggaggg   2100
gcggcagctt cggccgacag gagggtcctc aaatacctcc ttcctgggat gatgcccccc   2160
tcattgggtg ggcatcggag gggccccagg ttctctctcc cttaggggct gcagcccagg   2220
gggctgcaga ggaggtgtct ctgcctgcga tgggctcggt ggggggggaa ggcaggatca   2280
cggagggggga tatgcgaaga ggccgagacg gaggacccct ccatggttgt cccaaaaagc   2340
ctgccacctt tccccaccac cgaaaaaagg gaagcaaaca aacaaatttg gatttttccc   2400
ccatcaatcc caaaatacaa cgagatctga agagccttgt gggagggagt cagcttgaag   2460
ggggaagggg gtccctgacc gcagagggga cggactgggc tcgcttctct cagtctcctc   2520
cccacgcccc gctgcttcag tcctcgccgc ccagagccgg ctccgggagc tggggacgca   2580
tcggctagag gagacgatcc tcccgcctct ggaattgggg gtgcgggggt gggggccgag   2640
caaggggcgg cgcgcagcca agttgcaaat tggattaggg agcgtggggg tgagagccac   2700
gggaggggtg agggagctgg gccgggggc ccgggccgcg agagcgcgga gcggggcagc   2760
tgtccccacc ggcggccgac cagcctctct ccaccgccag gagagaacgg gctttcaggg   2820
cgagcgcgcc gcctcccctg gcaaagatat ctggtcccta aaaccccccac ccggtccctg   2880
ccctgaccct gagaagaagc aggcgcgggg agcagccccc cattcaagcg aggggcggag   2940
ccggggccca gcgccgggga gagggcctgg gccgagatcc caggccggca gccgggtagg   3000
gctgggccgg ctctgggcgg ggcaggcggc ggaggtgggc atccagggta gcctaggcag   3060
gagcccgcac gagactcggg ggtggaggag ggttgtgggg gggcgtcggt accccagcgc   3120
gcccctcact ttgtgctgtc tgtctcccct tcccgcccgc ggggcgccct caggcaccat   3180
gctgacccgc ctgttcagcg agcccggcct tctctcggac gtgcccaagt tcgccagctg   3240
gggcgacggc gaagacgacg agccgaggag cgacaagggc gacgcgccgc caccgccacc   3300
gcctgcgccc gggccagggg ctccggggcc agcccgggcg gccaagccag tccctctccg   3360
tggagaagag gggacggagg ccacgttggc cgaggtcaag gaggaaggcg agctgggggg   3420
agaggaggag gaggaagagg aggaggaaga aggactggac gaggcggagg gcgagcggcc   3480
caagaagcgc gggcccaaga agcgcaagat gaccaaggcg cgcttggagc gctccaagct   3540
tcggcggcag aaggcgaacg cgcgggagcg caaccgcatg cacgacctga acgcagccct   3600
ggacaacctg cgcaaggtgg tgccctgcta ctccaagacg cagaagctgt ccaagatcga   3660
gacgctgcgc ctagccaaga actatatctg ggcgctctcg gagatcctgc gctccggcaa   3720
gcggccagac ctagtgtcct acgtgcagac tctgtgcaag ggtctgtcgc agcccaccac   3780
caatctggtg gccggctgtc tgcagctcaa ctctcgcaac ttcctcacgg agcaaggcgc   3840
cgacggtgcc ggccgcttcc acggctcggg cggcccgttc gccatgcacc cctacccgta   3900
cccgtgctcg cgcctggcgg gcgcacagtg ccaggcggcc ggcggcctgg gcggcggcgc   3960
ggcgcacgcc ctgcggaccc acggctactg cgccgcctac gagacgctgt atgcggcggc   4020
aggcggtggc ggcgcgagcc cggactacaa cagctccgag tacgagggcc cgctcagccc   4080
cccgctctgt ctcaatggca acttctcact caagcaggac tcctcgcccg accacgagaa   4140
aagctaccac tactctatgc actactcggc gctgcccggt tcgcgccacg gccacgggct   4200
```

agtcttcggc tcgtcggctg tgcgcggggg cgtccactcg gagaatctct tgtcttacga 4260
tatgcacctt caccacgacc ggggccccat gtacgaggag ctcaatgcgt tttttcataa 4320
ctgagacttc gcgccggctc ccttcttttt cttttgcctt tgcccgcccc cctgtcccca 4380
gcccccagca gcgcagggta cacccccatc ctaccccggc gccgggcgcg gggagcgggc 4440
caccggtcct gccgctctcc tggggcagcg cagtcctgtt acctgtgggt ggcctgtccc 4500
aggggcctcg cttcccccag gggactcgcc ttctctctcc ccaaggggtt ccctcctcct 4560
ctctcccaag gagtgcttct ccagggacct ctctccgggg gctccctgga ggcacccctc 4620
ccccattccc aatatcttcg ctgaggtttc ctcctccccc tcctccctgc aggcccaagg 4680
cgttggtaag gggcagctg agcaatggaa cgcgtttccc cctctcatta ttattttaaa 4740
aacagacacc cagctgccga ggcaaaaagg agccaggcgc tccctctttc ttgaagaggg 4800
tagtattttg ggcgccggag cccgggcctg gaacgccctc acccgcaacc tccagtctcc 4860
gcgttttgcg attttaattt tggcgggagg ggaagtggat tgagaggaaa gagagaggcc 4920
aagacaattt gtaactagaa tccgtttttc ccttttcctt tttttaaaca aacaaacata 4980
caaaaaaaaa aaaaaaaaaa aaaaaaaaaa aagctaagag gcgacggaag ccgaacgcag 5040
agtccggatc ggagagaaaa cgcagtaagg acttttagaa gcaataaaag gcaaaaaaaa 5100
caaaaaacaa aaaaacaaac aaaaaaaaac cactactacc aataatcaaa gacacaaata 5160
tctatgcaag gaggctccac tgagcctcgc ggcccggccc ggccccggga tgccccgccc 5220
ggcctgcggg ccgccccgcc cgagcgcgga tctgtgcact ttggtgaagt gggggcccgc 5280
gccgcccccct cccccctcccc aggttcttac aatcagtgac tcggagattt ggggccccag 5340
tgccactgcc ctcccccgcc ccgtccccgt tgtgcgtcat gctgtttttt aaaaacctgt 5400
ttccaaattt gtatggaatg gcaaactgtt ggggggtcgg tttggggagg gagggtttgc 5460
atgaaagaca cacgcacacc acaccgcacg cacaagcagg cccggcgccg gcgtccgggg 5520
ggcagaagga ggtgagctcg ccggctcctc ctccccgcgg ccattctgtc ccctcctggg 5580
gtgaggggtg gggatggaga cctgggggca gccccacccc tgcccggact gtgcctcggt 5640
gggtgccacc tggcgatttc cggtgtctgg agagagtatt ttttggtcca aggagtcctc 5700
ttggctttag ctggtgggtg ggcggggaga ggtctgaggg ctcctactgg aggttccccc 5760
aaaaagggc aaaaggagac cctctgccca ccggaggcag gggatcaggc atccaaatac 5820
acgatgcaaa aatgcaatcc cacaggcgac acacccacac actcacccac acacacgcaa 5880
ttttaccttc ctcttgtagc gaagatgaaa ctcccgtcgg acacccgaag tgcattgcgt 5940
gtttctgttc agtttaatga cgattaataa atatttatgt aaatgagatg caaagccgga 6000
ccggtttctc acggtggcct catttcattg aggggggaga gaaggtttga gctggggctg 6060
gggtgatgaa ggcagagtgt caagtgactg tgcagaggcc aaacagaggg acttcccagc 6120
aaaaagcact g 6131

<210> 7

<211> 2020

<212> DNA

<213> Homo sapiens

<400> 7
gctactgagg ccgcggagcc ggactgcggt tggggcggga agagccgggg ccgtggctga 60
catggagcag ccctgctgct gaggccgcgc cctccccgcc ctgaggtggg ggcccaccag 120
gatgagcaag ctgcccaggg agctgacccg agacttggag cgcagcctgc ctgccgtggc 180
ctccctgggc tcctcactgt cccacagcca gagcctctcc tcgcacctcc ttccgccgcc 240
tgagaagcga agggccatct ctgatgtccg ccgcaccttc tgtctcttcg tcaccttcga 300
cctgctcttc atctccctgc tctggatcat cgaactgaat accaacacag gcatccgtaa 360
gaacttggag caggagatca tccagtacaa ctttaaaact tccttcttcg acatctttgt 420
cctggccttc ttccgcttct ctggactgct cctaggctat gccgtgctgc agctccggca 480
ctggtgggtg attgcggtca cgacgctggt gtccagtgca ttcctcattg tcaaggtcat 540
cctctctgag ctgctcagca aaggggcatt tggctacctg ctccccatcg tctcttttgt 600
cctcgcctgg ttggagacct ggttccttga cttcaaagtc ctaccccagg aagctgaaga 660
ggagcgatgg tatcttgccg cccaggttgc tgttgcccgt ggacccctgc tgttctccgg 720
tgctctgtcc gagggacagt tctattcacc cccagaatcc tttgcagggt ctgacaatga 780
atcagatgaa gaagttgctg ggaagaaaag tttctctgct caggagcggg agtacatccg 840

ccaggggaag gaggccacgg cagtggtgga ccagatcttg gcccaggaag agaactggaa 900
gtttgagaag aataatgaat atggggacac cgtgtacacc attgaagttc cctttcacgg 960
caagacgttt atcctgaaga ccttcctgcc ctgtcctgcg gagctcgtgt accaggaggt 1020
gatcctgcag cccgagagga tggtgctgtg gaacaagaca gtgactgcct gccagatcct 1080
gcagcgagtg gaagacaaca ccctcatctc ctatgacgtg tctgcagggg ctgcgggcgg 1140
cgtggtctcc ccaagggact tcgtgaatgt ccggcgcatt gagcggcgca gggaccgata 1200
cttgtcatca gggatcgcca cctcacacag tgccaagccc ccgacgcaca aatatgtccg 1260
gggagagaat ggccctgggg gcttcatcgt gctcaagtcg gccagtaacc cccgtgtttg 1320
cacctttgtc tggattctta atacagatct caagggccgc ctgccccggt acctcatcca 1380
ccagagcctc gcggccacca tgtttgaatt tgcctttcac ctgcgacagc gcatcagcga 1440
gctgggggcc cgggcgtgac tgtgcccccct cccaccctgc gggccagggt cctgtcgcca 1500
ccacttccag agccagaaag ggtgccagtt gggctcgcac tgcccacatg ggacctggcc 1560
ccaggctgtc accctccacc gagccacgca gtgcctggag ttgactgact gagcaggctg 1620
tggggtggag cactggactc cggggcccca ctggctggag gaagtggggt ctggcctgtt 1680
gatgtttaca tggcgccctg cctcctggag gaccagattg ctctgcccca ccttgccagg 1740

gcagggtctg ggctgggcac ctgacttggc tggggaggac cagggccctg ggcagggcag 1800
ggcagcctgt cacccgtgtg aagatgaagg ggctcttcat ctgcctgcgc tctcgtcggt 1860
ttttttagga ttattgaaag agtctgggac ccttgttggg gagtgggtgg caggtggggg 1920
tggctgctg gccatgaatc tctgcctctc ccaggctgtc cccctcctcc cagggcctcc 1980
tgggggacct ttgtattaag ccaattaaaa acatgaattt 2020

<210> 8

<211> 1730

<212> DNA

<213> Homo sapiens

<400> 8
gtggtgaggg tgactgggga ctaggcacta ggcctttggt gcaggcgcct gaggacktgg 60
ttgcactctc ccttctgggg atatgccctt gagcccaggc agaggagagc acagcccagg 120
gcaggacctg gcagccctgg tacagagccc agagggggca tcagttcctg ctggtcctgc 180
tctgtttaca gacaasctgc tgtcctccct gcaaagggga gtgggtgggg cagagggcaa 240
ktgccagggg ggcacaaggc tgggcatgtg gctggcatga gacggtgtct gagtaatgtc 300
aggcacctgg aggcattgac cccaggacct tggaccccag acctctgacc gtggggcagc 360
cagcgtccag gtaccccaac ccctgccctg ggtccggcgt ccccccatta gtgagtcttg 420
gctctactta tagcatctga caccagaggg gccgaaaata gcccctggag aagggggagg 480
agggggctat ttaaagggcc tgggaggggga gagagaatga ggagtgatca tggctacctc 540
agagctgagc tgcgaggtgt cggaggagaa ctgtgagcgc cgggaggcct tctgggcaga 600
atggaaggat ctgacactgt ccacacggcc cgaggagggg tgagtgtggg tctgctagag 660
tccctgcctc tgctccccca gagcaccctc actgagccat gaggccagag catgaagccc 720
tggagaaatt tctgggggtg ggggcaggaa gaatgcccca tggggagagc aaaggggaac 780
caccccttcct gcccccaggt cccagcagcc caggggagcc ccccacccag cctgtgccca 840
gagagcaaca gctcccagga gctcactgcc cctccccctct ccccagctgc tccctgcatg 900
aggaggacac ccagagacat gagacctacc accagcaggg gcagtgccag gtgctggtgc 960
agcgctcgcc ctggctgatg atgcggatgg gcatcctcgg ccgtgggctg caggagtacc 1020
agctgcccta ccagcgggta ctgccgctgc ccatcttcac ccctgccaag atgggcgcca 1080
ccaaggagga gcgtgaggac acccccatcc agcttcagga gctgctggcg ctggagacag 1140
ccctgggtgg ccagtgtgtg gaccgccagg aggtggctga gatcacaaag cagctgcccc 1200
ctgtggtgcc tgtcagcaag cccggtgcmc ttcgtcgctc cctgtcccgc tccatgtccc 1260
aggaagcaca gagaggctga gagggactgt gacttgggct ccgctgtgcc cgccccctgg 1320
gctgggccct tcctggctag gacctgtgga ggggcagctc gctggcccat ggctgctttg 1380
tagtttgccc agagttgggg gctaggggag gggggagcca gaggccagga tgcctgagcc 1440
ccctgagttc ccaaagggag ggtggcagag acagtgggca ctaagggtgg agagttgggg 1500
gccagcacag ctgaggaccc tcagccccag gagaagggac aaaaggtact ggtgagggca 1560

agaggtgcct gggaggagtg gccctgatcc aggaaaatgt gaggggaatc tggaacgctc 1620
taggcagaag aagctgggag ggagggggag gtgaaaaggg cagaggcaag gatggtgggg 1680
cccccagcac cctctgttag tgccgcaata aatgctcaat catgtgccag 1730

<210> 9

<211> 3799

<212> DNA

<213> Homo sapiens

<400> 9
ctggcactgg gtggtaacca gcaagccagc tggcatccgc atccagggtt tgtttcaatg 60
atgtctcgtg gagaatatgg aggggctggt gccaggactg tccttggctt tgcctcgggg 120
tgtgaacggg gtcagtgacc tctaaaacta acctgcctct cagttctgaa tccagacaga 180
atcaatcctc agctgtgtct cgctccacac cccctgccct ggaagccagg gaaggttgga 240
ggtgctaggg ggtcaggctc ccctctgtga cccctgcagc tgttgtggtg actcatgtcc 300
caacctagct gcctctccca aggagacttt cccctgggac aagggggagg gaatggcatg 360
gaggaggccc acatcaagcg gggccaggaa cccacggtgg caggagctgg gctggtgacc 420
tacccaggc agaagggccc gggactcatc cagaggggaa ggaagggtc ttcaggaaga 480
ccacggagat gccacaggca gaattggctt cccatctggg agataggtgg ggagaccctg 540
gcattttgac agccagaacc tggggtgctg agcagaatct tcatgcctgg cctggccgcc 600
ttcggaggga agctggaggg ttgggtgcga gaggagtggg gtcagagccc ctacatccgc 660
aggaccccaa atcggctggg ccccaaggcc cggactgcgc tccccggtgg ccccggcggc 720
cctccgcgaa tgcgtcctgc ccctcccctg cccaagccct ctgccctcac ccgggtccgg 780
cgccgccccc gaagtggcgg gaacaacccg aacccgaacc ttctgtcctc gggagccccc 840
agataagcgg ctgggaaccc gcggggcccg caggggaggc ccggctgttc cgcccgctaa 900
gtgcattagc acagctcacc tcccctatcg cgcctgccat cggacgggca gtgccgcgcc 960
ctgctctggg gcccccggag cgaccacagc ggaggccgga acggactgtc ctttctgggg 1020
cggggtgggg aggggtgtc gctggagggc ccggtggcat agcaacggac gagagaggcc 1080
tggaggaggg gcggggaggg ggagttgtgt ggcagttcta agggaagggt gggtgctggg 1140
acgggtgtcc gggagggagg ggagcctggc ggggtctggg gcctcgtcgc ggagggcgct 1200
gcgaggggga aactggggaa agggcctaat tccccagtct ccacctcgaa tcaggaaaga 1260
gaaggggcgg gctgctgggc aaaagaggtg aatggctgcg gggggctgga gaagagagat 1320
gggaggggcc ggccggcggg ggtgaggggg tctaaagatt gtgggggtga ggaactgagg 1380
gtgggggcg cccagaggcg ggactcgggg cggggcaggc gaggcggagg gcgagggctg 1440
cgggagcaag tacggagccg ggggtgtggg ggacgattgc cgctgcagcc gccgccccac 1500
tcacctccgg tgtgtctgca gcccggacac taagggagat ggatgaatgg gtggggagga 1560
tgcggcgcac atggccccgg gcggctcggc ggtcagctgc cgccccccaca gcggaccggt 1620
cggggcgggg gtcgggcggt agaaaaaagg gccgcgaggc gagcggggca ctgggcggac 1680
cgcggcggca gcatgagcgg cgcagaccgt agccccaatg cgggcgcagc ccctgactcg 1740
gccccgggcc aggcggcggt ggcttcggcc taccagcgct tcgagccgcg cgcctacctc 1800
cgcaacaact acgcgccccc tcgcggggac ctgtgcaacc cgaacggcgt cgggccgtgg 1860
aagctgcgct gcttggcgca gaccttcgcc accggtgagc gggggaaact gaggcacgag 1920
ggacaagagg tcgtcgggga gtgaaagcag gcgcagggaa ataaaaagaa ggaaagggag 1980
acagaccagg cgcctaacag atggggacca agaaacaaga gatagctgag aggtgcaaac 2040
agaagagaaa aaggagcaac atcccttagg agaggggcag aggagagaga ggtggagaga 2100
gggggcggag agtgctcaga attgagagct aaggtggggg atgcaggaca gactgaggtg 2160
gagatgcata ggaggaaatg gaggcagatg tgggacaggg gtgagaaact ccaggatttc 2220
ctcgctgagc ctggctggta ggtatagttg ttttctttct ttttctttat tttattttca 2280
tttatttact tatttttatt ttttatttgt tttgagacgg agtttcgctc ttgttgccca 2340
ggctggagta caatggcgcc atctcggctc actgcaacct ccgcctcccc gggttcaagc 2400
gattctcttg cctcagcttc cctagtagct gggattacag gcatgcgccc ccatgcctgg 2460
ctaatttatt tgtattttta gtagagacgg gacttctcca tgttggtcag gctggtctcg 2520
aactcccaac cttaggatcc acccaccccg gcctcccaaa gtgctgggat tacaggtgtg 2580
agccactgcg cccggccagt aggtatagtc ttctagatgt gaaacctgag tctcagagcg 2640

gtgaagttcc cttccgaagg gcagcccatg ttggagctgg gttcagtcta actctggggc 2700
caatgctttt tccagatgga gacacatttg cagaggagaa ggaagaacta gagagaggca 2760
gggagatgca ggggagggaa gggtaaggag gcaggggctg cctgggctgg ctggcaccag 2820
gaccctcttc ctctgccctg cccaggtgaa gtgtccggac gcaccctcat cgacattggt 2880
tcaggcccca ccgtgtacca gctgctcagt gcctgcagcc actttgagga catcaccatg 2940
acagatttcc tggaggtcaa ccgccaggag ctggggcgct ggctgcagga ggagccgggg 3000
gccttcaact ggagcatgta cagccaacat gcctgcctca ttgagggcaa ggggtaagga 3060
ctggggggtg agggttgggg aggaggcttc ccatagagtg gctggttggg gcaacagagg 3120
cctgagcgta gaacagcctt gagccctgcc ttgtgcctcc tgcacaggga atgctggcag 3180
gataaggagc gccagctgcg agccagggtg aaacgggtcc tgcccatcga cgtgcaccag 3240
ccccagcccc tgggtgctgg gagcccagct cccctgcctg ctgacgccct ggtctctgcc 3300
ttctgcttgg aggctgtgag cccagatctt gccagctttc agcgggccct ggaccacatc 3360
accacgctgc tgaggcctgg ggggcacctc ctcctcatcg ggggccctgga ggagtcgtgg 3420
tacctggctg gggaggccag gctgacggtg gtgccagtgt ctgaggagga ggtgagggag 3480
gccctggtgc gtagtggcta caaggtccgg gacctccgca cctatatcat gcctgcccac 3540
cttcagacag gcgtagatga tgtcaagggc gtcttcttcg cctgggctca gaaggttggg 3600
ctgtgagggc tgtacctggt gccctgtggc cccccacccac ctggattccc tgttctttga 3660
agtggcacct aataaagaaa taataccctg ccgctgcggt cagtgctgtg tgtggctctc 3720
ctgggaagca gcaagggccc agagatctga gtgtccgggt aggggagaca ttcaccctag 3780
gctttttttc cagaagctt 3799

<210> 10

<211> 4530

<212> DNA

<213> Homo sapiens

<400> 10
aattctcgag ctcgtcgacc ggtcgacgag ctcgaggtc gacgagctcg agggcgcgcg 60
cccggccccc acccctcgca gcaccccgcg ccccgcgccc tcccagccgg gtccagccgg 120
agccatgggg ccggagccgc agtgagcacc atggagctgg cggccttgtg ccgctggggg 180
ctcctcctcg ccctcttgcc ccccggagcc gcgagcaccc aagtgtgcac cggcacagac 240
atgaagctgc ggctccctgc cagtcccgag acccacctgg acatgctccg ccacctctac 300
cagggctgcc aggtggtgca gggaaacctg gaactcacct acctgcccac caatgccagc 360
ctgtccttcc tgcaggatat ccaggaggtg cagggctacg tgctcatcgc tcacaaccaa 420
gtgaggcagg tcccactgca gaggctgcgg attgtgcgag gcacccagct ctttgaggac 480
aactatgccc tggccgtgct agacaatgga gacccgctga acaataccac ccctgtcaca 540
ggggcctccc caggaggcct gcgggagctg cagcttcgaa gcctcacaga gatcttgaaa 600
ggaggggtct tgatccagcg gaaccccccag ctctgctacc aggacacgat tttgtggaag 660
gacatcttcc acaagaacaa ccagctggct ctcacactga tagacaccaa ccgctctcgg 720
gcctgccacc cctgttctcc gatgtgtaag ggctcccgct gctggggaga gagttctgag 780
gattgtcaga gcctgacgcg cactgtctgt gccggtggct gtgcccgctg caagggggcca 840
ctgcccactg actgctgcca tgagcagtgt gctgccggct gcacgggccc caagcactct 900
gactgcctgg cctgcctcca cttcaaccac agtggcatct gtgagctgca ctgcccagcc 960
ctggtcacct acaacacaga cacgtttgag tccatgccca atcccgaggg ccggtataca 1020
ttcggcgcca gctgtgtgac tgcctgtccc tacaactacc tttctacgga cgtgggatcc 1080
tgcaccctcg tctgcccccct gcacaaccaa gaggtgacag cagaggatgg aacacagcgg 1140
tgtgagaagt gcagcaagcc ctgtgcccga gtgtgctatg gtctgggcat ggagcacttg 1200
cgagaggtga gggcagttac cagtgccaat atccaggagt ttgctggctg caagaagatc 1260
tttgggagcc tggcatttct gccggagagc tttgatgggg acccagcctc caacactgcc 1320
ccgctccagc cagagcagct ccaagtgttt gagactctgg aagagatcac aggttaccta 1380
tacatctcag catggccgga cagcctgcct gacctcagcg tcttccagaa cctgcaagta 1440
atccggggac gaattctgca caatggcgcc tactcgctga ccctgcaagg gctgggcatc 1500
agctggctgg ggctgcgctc actgagggaa ctgggcagtg gactggccct catccaccat 1560
aacacccacc tctgcttcgt gcacacggtg ccctgggacc agctctttcg gaacccgcac 1620

```
caagctctgc tccacactgc caaccggcca gaggacgagt gtgtgggcga gggcctggcc   1680
tgccaccagc tgtgcgcccg agggcactgc tggggtccag ggcccaccca gtgtgtcaac   1740
tgcagccagt tccttcgggg ccaggagtgc gtggaggaat gccgagtact gcaggggctc   1800
cccagggagt atgtgaatgc caggcactgt ttgccgtgcc accctgagtg tcagccccag   1860
aatggctcag tgacctgttt tggaccggag gctgaccagt gtgtggcctg tgcccactat   1920
aaggaccctc ccttctgcgt ggcccgctgc cccagcggtg tgaaacctga cctctcctac   1980
atgcccatct ggaagtttcc agatgaggag ggcgcatgcc agccttgccc catcaactgc   2040
acccactcct gtgtggacct ggatgacaag ggctgccccg ccgagcagag agccagccct   2100
ctgacgtcca tcgtctctgc ggtggttggc attctgctgg tcgtggtctt gggggtggtc   2160
tttgggatcc tcatcaagcg acggcagcag aagatccgga agtacacgat gcggagactg   2220
ctgcaggaaa cggagctggt ggagccgctg acacctagcg gagcgatgcc caaccaggcg   2280
cagatgcgga tcctgaaaga gacggagctg aggaaggtga aggtgcttgg atctggcgct   2340
tttggcacag tctacaaggg catctggatc cctgatgggg agaatgtgaa aattccagtg   2400
gccatcaaag tgttgaggga aaacacatcc cccaaagcca acaaagaaat cttagacgaa   2460
gcatacgtga tggctggtgt gggctcccca tatgtctccc gccttctggg catctgcctg   2520
acatccacgg tgcagctggt gacacagctt atgccctatg gctgcctctt agaccatgtc   2580
cgggaaaacc gcggacgcct gggctcccag gacctgctga actggtgtat gcagattgcc   2640
aaggggatga gctacctgga ggatgtgcgg ctcgtacaca gggacttggc cgctcggaac   2700
gtgctggtca agagtcccaa ccatgtcaaa attacagact tcgggctggc tcggctgctg   2760
gacattgacg agacagagta ccatgcagat gggggcaagg tgcccatcaa gtggatggcg   2820
ctggagtcca ttctccgccg gcggttcacc caccagagtg atgtgtggag ttatggtgtg   2880
actgtgtggg agctgatgac ttttggggcc aaaccttacg atgggatccc agcccgggag   2940
atccctgacc tgctggaaaa gggggagcgg ctgccccagc cccccatctg caccattgat   3000
gtctacatga tcatggtcaa atgttggatg attgactctg aatgtcggcc aagattccgg   3060
gagttggtgt ctgaattctc ccgcatggcc agggaccccc agcgctttgt ggtcatccag   3120
aatgaggact tgggcccagc cagtcccttg gacagcacct tctaccgctc actgctggag   3180
gacgatgaca tgggggacct ggtggatgct gaggagtatc tggtacccca gcagggcttc   3240
ttctgtccag accctgcccc gggcgctggg ggcatggtcc accacaggca ccgcagctca   3300
tctaccagga gtggcggtgg ggacctgaca ctagggctgg agccctctga agaggaggcc   3360
cccaggtctc cactggcacc ctccgaaggg gctggctccg atgtatttga tggtgacctg   3420
ggaatggggg cagccaaggg gctgcaaagc ctccccacac atgaccccag ccctctacag   3480
cggtacagtg aggaccccac agtacccctg ccctctgaga ctgatggcta cgttgccccc   3540
ctgacctgca gcccccagcc tgaatatgtg aaccagccag atgttcggcc ccagccccct   3600
tcgccccgag agggccctct gcctgctgcc cgacctgctg gtgccactct ggaaagggcc   3660
aagactctct ccccagggaa gaatggggtc gtcaaagacg tttttgcctt tgggggtgcc   3720
gtggagaacc ccgagtactt gacaccccag ggaggagctg cccctcagcc ccaccctcct   3780
cctgccttca gcccagcctt cgacaacctc tattactggg accaggaccc accagagcgg   3840
ggggctccac ccagcacctt caaagggaca cctacggcag agaacccaga gtacctgggt   3900
ctggacgtgc cagtgtgaac cagaaggcca agtccgcaga agccctgatg tgtcctcagg   3960
gagcagggaa ggcctgactt ctgctggcat caagaggtgg gagggccctc cgaccacttc   4020
caggggaacc tgccatgcca ggaacctgtc ctaaggaacc ttccttcctg cttgagttcc   4080
cagatggctg gaaggggtcc agcctcgttg gaagaggaac agcactgggg agtctttgtg   4140
gattctgagg ccctgcccaa tgagactcta gggtccagtg gatgccacag cccagcttgg   4200
ccctttcctt ccagatcctg ggtactgaaa gccttaggga agctggcctg agaggggaag   4260
cggccctaag ggagtgtcta agaacaaaag cgacccattc agagactgtc cctgaaacct   4320
agtactgccc cccatgagga aggaacagca atggtgtcag tatccaggct ttgtacagag   4380
tgcttttctg tttagttttt actttttttg ttttgttttt ttaaagacga aataaagacc   4440
caggggagaa tgggtgttgt atggggaggc aagtgtgggg ggtccttctc cacacccact   4500
ttgtccattt gcaaatatat tttggaaaac                                    4530
```

<210> 11

<211> 2205

<212> DNA

<213> Homo sapiens

<400> 11
cacagggctc ccccccgcct ctgacttctc tgtccgaagt cgggacaccc tcctaccacc 60
tgtagagaag cgggagtgga tctgaaataa aatccaggaa tctgggggtt cctagacgga 120
gccagacttc ggaacgggtg tcctgctact cctgctgggg ctcctccagg acaagggcac 180
acaactggtt ccgttaagcc cctctctcgc tcagacgcca tggagctgga tctgtctcca 240

cctcatctta gcagctctcc ggaagacctt tggccagccc ctgggacccc tcctgggact 300
ccccggcccc ctgatacccc tctgcctgag gaggtaaaga ggtcccagcc tctcctcatc 360
ccaaccaccg gcaggaaact tcgagaggag gagaggcgtg ccacctccct cccctctatc 420
cccaacccct tccctgagct ctgcagtcct ccctcacaga gcccaattct cgggggcccc 480
tccagtgcaa gggggctgct cccccgcgat gccagccgcc cccatgtagt aaaggtgtac 540
agtgaggatg gggcctgcag gtctgtggag gtggcagcag gtgccacagc tcgccacgtg 600
tgtgaaatgc tggtgcagcg agctcacgcc ttgagcgacg agacctgggg gctggtggag 660
tgccaccccc acctagcact ggagcggggt ttggaggacc acgagtccgt ggtggaagtg 720
caggctgcct ggcccgtggg cggagatagc cgcttcgtct tccggaaaaa cttcgccaag 780
tacgaactgt tcaagagctc cccacactcc ctgttcccag aaaaaatggt ctccagctgt 840
ctcgatgcac acactggtat atcccatgaa gacctcatcc agaacttcct gaatgctggc 900
agctttcctg agatccaggg ctttctgcag ctgcggggtt caggacggaa gctttggaaa 960
cgctttttct gtttcttgcg ccgatctggc ctctattact ccaccaaggg cacctctaag 1020
gatccgaggc acctgcagta cgtggcagat gtgaacgagt ccaacgtgta cgtggtgacg 1080
cagggccgca agctctacgg gatgcccact gacttcggtt tctgtgtcaa gcccaacaag 1140
cttcgaaatg gacacaaggg gcttcggatc ttctgcagtg aagatgagca gagccgcacc 1200
tgctggctgg ctgccttccg cctcttcaag tacggggtgc agctgtacaa gaattaccag 1260
caggcacagt ctcgccatct gcatccatct tgtttgggct ccccaccctt gagaagtgcc 1320
tcagataata ccctggtggc catggacttc tctggccatg ctgggcgtgt cattgagaac 1380
ccccgggagg ctctgagtgt ggccctggag gaggcccagg cctggaggaa gaagacaaac 1440
caccgcctca gcctgcccat gccagcctcc ggcacgagcc tcagtgcagc catccaccgc 1500
acccaactct ggttccacgg gcgcatttcc cgtgaggaga gccagcggct tattggacag 1560
cagggcttgg tagacggcct gttcctggtc cgggagagtc agcggaaccc ccagggcttt 1620
gtcctctctt tgtgccacct gcagaaagtg aagcattatc tcatcctgcc gagcgaggag 1680
gagggtcgcc tgtacttcag catggatgat ggccagaccc gcttcactga cctgctgcag 1740
ctcgtggagt tccaccagct gaaccgcggc atcctgccgt gcttgctgcg ccattgctgc 1800
acgcgggtgg ccctctgacc aggccgtgga ctggctcatg cctcagcccg ccttcaggct 1860
gcccgccgcc cctccaccca tccagtggac tctggggcgc ggccacaggg gacgggatga 1920
ggagcgggag ggttccgcca ctccagtttt ctcctctgct tctttgcctc cctcagatag 1980
aaaacagccc ccactccagt ccactcctga cccctctcct caagggaagg ccttgggtgg 2040
ccccctctcc ttctcctagc tctggaggtg ctgctctagg gcagggaatt atgggagaag 2100
tgggggcagc ccaggcggtt tcacgcccca cactttgtac agaccgagag gccagttgat 2160
ctgctctgtt ttatactagt gacaataaag attatttttt gatac 2205

<210> 12

<211> 2177

<212> DNA

<213> Homo sapiens

<400> 12
gaattcgcgg ccgctggttt gcagctgctc cgtcatcgtg cggcccgacg ctatctcgcg 60
ctcgtgtgca ggcccggctc ggctcctggt ccccggtgcg agggttaacg cgaggccccg 120
gcctcggtcc ccggactagg ccgtgacccc gggtgccatg aagcaggagg gctcggcgcg 180
gcgccgcggc gcggacaagg cgaaaccgcc gcccggcgga ggagaacaag aacccccacc 240
gccgccggcc ccccaggatg tggagatgaa agaggaggca gcgacggtg gcgggtcaac 300
gggggaggca gacggcaaga cggcggcggc agcggttgag cactcccagc gagagctgga 360

cacagtcacc ttggaggaca tcaaggagca cgtgaaacag ctagagaaag cggtttcagg 420
caaggagccg agattcgtgc tgcgggccct gcggatgctg ccttccacat cacgccgcct 480
caaccactat gttctgtata aggctgtgca gggcttcttc acttcaaata atgccactcg 540
agactttttg ctccccttcc tggaagagcc catggacaca gaggctgatt tacagttccg 600
tccccgcacg ggaaaagctg cgtcgacacc cctcctgcct gaagtggaag cctatctcca 660
actcctcgtg gtcatcttca tgatgaacag caagcgctac aaagaggcac agaagatctc 720
tgatgatctg atgcagaaga tcagtactca gaaccgccgg gccctagacc ttgtagccgc 780
aaagtgttac tattatcacg cccgggtcta tgagttcctg gacaagctgg atgtggtgcg 840
cagcttcttg catgctcggc tccggacagc tacgcttcgg catgacgcag acgggcaggc 900
caccctgttg aacctcctgc tgcggaatta cctacactac agcttgtacg accaggctga 960
gaagctggtg tccaagtctg tgttcccaga gcaggccaac aacaatgagt gggccaggta 1020
cctctactac acagggcgaa tcaaagccat ccagctggag tactcagagg cccggagaac 1080
gatgaccaac gcccttcgca aggcccctca gcacacagct gtcggcttca aacagacggt 1140
gcacaagctt ctcatcgtgg tggagctgtt gctgggggag atccctgacc ggctgcagtt 1200
ccgccagccc tccctcaagc gctcactcat gccctatttc cttctgactc aagctgtcag 1260
gacaggaaac ctagccaagt tcaaccaggt cctggatcag tttggggaga agtttcaagc 1320
agatgggacc tacaccctaa ttatccggct gcggcacaac gtgattaaga caggtgtacg 1380
catgatcagc ctctcctatt cccgaatctc cttggctgac atcgcccaga agctgcagtt 1440
ggatagcccc gaagatgcag agttcattgt tgccaaggcc atccgggatg gtgtcattga 1500
ggccagcatc aaccacgaga agggctatgt ccaatccaag gagatgattg acatctattc 1560
cacccgagag ccccagctag ccttccacca gcgcatctcc ttctgcctag atatccacaa 1620
catgtctgtc aaggccatga ggtttcctcc caaatcgtac aacaaggact tggagtctgc 1680
agaggaacgg cgtgagcgag aacagcagga cttggagttt gccaaggaga tggcagaaga 1740
tgatgatgac agcttccctt gagctggggg gctggggagg ggtagggggga atggggacag 1800
gctctttccc ccttgggggt cccctgccca gggcactgtc cccattttcc cacacacagc 1860
tcatatgctg cattcgtgca gggggtgggg gtgctgggag ccagccaccc tgacctcccc 1920
cagggctcct ccccagccgg tgacttactg tacagcaggc aggagggtgg gcaggcaacc 1980
tccccgggca gggtcctggc cagcagtgtg ggagcaggag gggaaggata gttctgtgta 2040
ctcctttagg gagtgggggga ctagaactgg gatgtcttgg cttgtatgtt ttttgaagct 2100
tcgattatga tttttaaaca ataaaaagtt ctcccaaaaa aaaaaaaaaa aaaaaaaaaa 2160
aaagcggccg cgaattc 2177

<210> 13

<211> 2960

<212> DNA

<213> Homo sapiens

<400> 13
ctgccgcttc caggcgtcta tcagcggctc agcctttgtt cagctgttct gttcaaacac 60
tctggggcca ttcaggcctg ggtggggcag cgggaggaag ggagtttgag gggggcaagg 120
cgacgtcaaa ggaggatcag agattccaca atttcacaaa actttcgcaa acagcttttt 180
gttccaaccc ccctgcattg tcttggacac caaatttgca taaatcctgg gaagttatta 240
ctaagcctta gtcgtggccc caggtaattt cctcccaggc ctccatgggg ttatgtataa 300
agggccccct agagctgggc cccaaaacag cccggagcct gcagcccagc ccccacccaga 360
cccatggctg gacctgccac ccagagcccc atgaagctga tgggtgagtg tcttggccca 420
ggatgggaga gccgcctgcc ctggcatggg agggaggctg gtgtgacaga ggggctgggg 480
atccccgttc tgggaatggg gattaaaggc acccagtgtc cccgagaggg cctcaggtgg 540
tagggaacag catgtctcct gagcccgctc tgtccccagc cctgcagctg ctgctgtggc 600
acagtgcact ctggacagtg caggaagcca cccccctggg ccctgccagc tccctgcccc 660
agagcttcct gctcaagtgc ttagagcaag tgaggaagat ccagggcgat ggcgcagcgc 720
tccaggagaa gctggtgagt gaggtgggtg agagggctgt ggagggaagc ccggtgggga 780
gagctaaggg ggatggaact gcagggccaa catcctctgg aagggacatg ggagaatatt 840
aggagcagtg gagctgggga aggctgggaa gggacttggg gaggaggacc ttggtgggga 900
cagtgctcgg gagggctggc tgggatggga gtggaggcat cacattcagg agaaagggca 960

agggcccctg tgagatcaga gagtgggggt gcagggcaga gaggaactga acagcctggc 1020
aggacatgga gggaggggaa agaccagaga gtcggggagg acccgggaag gagcggcgac 1080
ccggccacgg cgagtctcac tcagcatcct tccatcccca gtgtgccacc tacaagctgt 1140
gccaccccga ggagctggtg ctgctcggac actctctggg catcccctgg gctcccctga 1200
gcagctgccc cagccaggcc ctgcagctgg tgagtgtcag gaaaggataa ggctaatgag 1260
gagggggaag gagaggagga acacccatgg gctcccccat gtctccaggt tccaagctgg 1320
gggcctgacg tatctcaggc agcacccccct aactcttccg ctctgtctca caggcaggct 1380
gcttgagcca actccatagc ggcctttttcc tctaccaggg gctcctgcag gccctggaag 1440
ggatctcccc cgagttgggt cccaccttgg acacactgca gctggacgtc gccgactttg 1500
ccaccaccat ctggcagcag gtgagccttg ttgggcaggg tggccaaggt cgtgctggca 1560
ttctgggcac cacagccggg cctgtgtatg ggccctgtcc atgctgtcag cccccagcat 1620
ttcctcattt gtaataacgc ccactcagaa gggcccaacc actgatcaca gctttccccc 1680
acagatggaa gaactgggaa tggcccctgc cctgcagccc acccagggtg ccatgccggc 1740
cttcgcctct gctttccagc gccgggcagg aggggtcctg gttgcctccc atctgcagag 1800
cttcctggag gtgtcgtacc gcgttctacg ccaccttgcc cagccctgag ccaagccctc 1860
cccatcccat gtatttatct ctatttaata tttatgtcta tttaagcctc atatttaaag 1920
acagggaaga gcagaacgga gccccaggcc tctgtgtcct tccctgcatt tctgagtttc 1980
attctcctgc ctgtagcagt gagaaaaagc tcctgtcctc ccatcccctg gactgggagg 2040
tagataggta aataccaagt atttattact atgactgctc cccagccctg gctctgcaat 2100
gggcactggg atgagccgct gtgagcccct ggtcctgagg gtccccacct gggacccttg 2160
agagtatcag gtctcccacg tgggagacaa gaaatccctg tttaatattt aaacagcagt 2220
gttccccatc tgggtccttg cacccctcac tctggcctca gccgactgca cagcggcccc 2280
tgcatcccct tggctgtgag gcccctggac aagcagaggt ggccagagct gggaggcatg 2340
gccctggggt cccacgaatt tgctggggaa tctcgttttt cttcttaaga cttttgggac 2400
atggtttgac tcccgaacat caccgacgtg tctcctgttt ttctgggtgg cctcgggaca 2460
cctgccctgc ccccacgagg gtcaggactg tgactctttt tagggccagg caggtgcctg 2520
gacatttgcc ttgctggatg gggactgggg atgtgggagg gagcagacag gaggaatcat 2580
gtcaggcctg tgtgtgaaag gaagctccac tgtcaccctc cacctcttca ccccccactc 2640
accagtgtcc cctccactgt cacattgtaa ctgaacttca ggataataaa gtgtttgcct 2700
ccagtcacgt ccttcctcct tcttgagtcc agctggtgcc tggccagggg ctggggaggt 2760
ggctgaaggg tgggagaggc cagagggagg tcggggagga ggtctgggga ggaggtccag 2820
ggaggaggag gaaagttctc aagttcgtct gacattcatt ccgttagcac atatttatct 2880
gagcacctac tctgtgcaga cgctgggcta agtgctgggg acacagcagg gaacaaggca 2940
gacatggaat ctgcactcga 2960

<210> 14

<211> 850

<212> DNA

<213> Homo sapiens

<220>

<221> misc_feature

<222> (3)..(4)

<223> n=a, c, g or t

<220>

<221> misc_feature

<222> (9)..(9)

<223> n=a, c, g or t

<220>

<221> misc_feature

<222> (11)..(11)

<223> n=a, c, g or t

<220>

<221> misc_feature

<222> (18)..(18)

<223> n=a, c, g or t

<220>

<221> misc_feature

<222> (202)..(202)

<223> n=a, c, g or t

<220>

<221> misc_feature

<222> (205)..(205)

<223> n=a, c, g or t

<220>

<221> misc_feature

<222> (273)..(273)

<223> n=a, c, g or t

<220>

<221> misc_feature

<222> (327)..(327)

<223> n=a, c, g or t

<220>

<221> misc_feature

<222> (367)..(367)

<223> n=a, c, g or t

<220>

<221> misc_feature

<222> (581)..(581)

<223> n=a, c, g or t

<220>

<221> misc_feature

<222> (599)..(599)

<223> n=a, c, g or t

<220>

<221> misc_feature

<222> (628)..(628)

<223> n=a, c, g or t

<220>

<221> misc_feature

<222> (673)..(673)

<223> n=a, c, g or t

<220>

<221> misc_feature

<222> (675)..(675)

<223> n=a, c, g or t

<220>

<221> misc_feature

<222> (682)..(682)

<223> n=a, c, g or t

<220>

<221> misc_feature

<222> (693)..(693)

<223> n=a, c, g or t

<220>

<221> misc_feature

<222> (698)..(698)

<223> n=a, c, g or t

<220>

<221> misc_feature

<222> (700)..(700)

<223> n=a, c, g or t

<220>

<221> misc_feature

<222> (720)..(720)

<223> n=a, c, g or t

<220>

<221> misc_feature

<222> (730)..(730)

<223> n=a, c, g or t

<220>

<221> misc_feature

<222> (734)..(734)

<223> n=a, c, g or t

<220>

<221> misc_feature

<222> (742)..(743)

<223> n=a, c, g or t

<220>

<221> misc_feature

<222> (746)..(746)

<223> n=a, c, g or t

<220>

<221> misc_feature

<222> (748)..(748)

<223> n=a, c, g or t

<220>

<221> misc_feature

<222> (752)..(752)

<223> n=a, c, g or t

<220>

<221> misc_feature

<222> (762)..(762)

<223> n=a, c, g or t

<220>

<221> misc_feature

<222> (767)..(767)

<223> n=a, c, g or t

<220>

<221> misc_feature

<222> (777)..(777)

<223> n=a, c, g or t

<220>

<221> misc_feature

<222> (783)..(784)

<223> n=a, c, g or t

<220>

<221> misc_feature

<222> (789)..(789)

<223> n=a, c, g or t

<220>

<221> misc_feature

<222> (794)..(794)

<223> n=a, c, g or t

<220>

<221> misc_feature

<222> (797)..(798)

<223> n=a, c, g or t

<220>

<221> misc_feature

<222> (803)..(805)

<223> n=a, c, g or t

<220>

<221> misc_feature

<222> (810)..(810)

<223> n=a, c, g or t

<220>

<221> misc_feature

<222> (817)..(817)

<223> n=a, c, g or t

<220>

<221> misc_feature

<222> (826)..(827)

<223> n=a, c, g or t

<220>

<221> misc_feature

<222> (831)..(832)

<223> n=a, c, g or t

<220>

<221> misc_feature

<222> (834)..(834)

<223> n=a, c, g or t

<220>

<221> misc_feature

<222> (837)..(838)

<223> n=a, c, g or t

<220>

<221> misc_feature

<222> (840)..(840)

<223> n=a, c, g or t

<220>

<221> misc_feature

<222> (844)..(844)

<223> n=a, c, g or t

<220>

<221> misc_feature

<222> (846)..(848)

<223> n=a, c, g or t

<400> 14
ttnnctttnt ngccatgncc agttcaactc agcctctcag ttccacacgg acaacatgcg 60
ggaccctctg aaccgagtcc tggccaacct gttcctgctc atctcctcca tcctggggtc 120
tcgcaccgct ggcccccaca cccagttcgt gcagtggttc atggaggagt gtgtggactg 180
cctggagcag ggtggccgtg gnagngtcct gcagttcatg cccttcacca ccgtgtcgga 240
actggtgaag gtgtcagcca tgtctagccc canggtggtt ctggccatca cggacctcag 300
cctgcccctg ggccgccagg tggctgntaa agccattgct gcactctgag ggcttggca 360
tggccgnagt gggggctggg gactggcgca gccccaggcg cctccaaggg aagcagtgag 420
gaaagatgag gcatcgtgcc tcacatccgt tccacatggt gcaagagcct ctagcggctt 480
ccagttcccc gctcctgact cctgactcca ggatgtctcc cggtttcttc ttttcaaaat 540
tttcctctcc atcttgctgg caactgagga gagtgagcag nctggaccac aagcccagng 600
ggtcacccct gtgttgcgcc cgcccagncc aggagtagtc ttacctcttg aggaactttc 660
ttggatggaa agngngtttt tntgtgttgt gtntgtgnan gtgtttttcg gggtttttttn 720
gggcaatatn ttangggaat cnnccntncg cncatttttt cnttagagct ccccggngga 780

aanntcttna tccnctnnct ttnnnctccn tcacctncct tctttnntct nntnttnncn 840
tccncnnncc 850

<210> 15

<211> 2309

<212> DNA

<213> Homo sapiens

<400> 15
ccccgggcgc aggaggcggg cggcccggcc ccaccggccc cccatggacg cccccagcac 60
ggggcgctga gacccccgcg tcgctgccca gcccggtccg gcgcgccacg ccagggatct 120
ctggacagga caagactccg aagctactcc cccagcacac agcccgggac ccacaaaccc 180
agcttgcccc cagccctccc acctgccact ccctggcccc tcccaccgcc cgcccccctt 240
ggggcgcagg gcatggtgtg aaaggccaag tgctgaggcg ggtatcatgg gtgctgtgcc 300
ctagggcctg ggtggcaggg ggtgggtggc ctgtgggtgt gccgggggggg ccagtgtgcc 360
caccccagtc tcttggcgtg ctggagggca tcctggatgg aattgaagtg aatggaacag 420
aagccaagca aggtggagtg tgggtcagac ccagaggaga acagtgccag gtcaccagat 480
ggaaagcgaa aaagaaagaa cggccaatgt tccctgaaaa ccagcatgtc agggtatatc 540
cctagttacc tggacaaaga cgagcagtgt gtcgtgtgtg gggacaaggc aactggttat 600
cactaccgct gtatcacttg tgagggctgc aagggcttct ttcgccgcac aatccagaag 660
aacctccatc ccacctattc ctgcaaatat gacagctgct gtgtcattga caagatcacc 720
cgcaatcagt gccagctgtg ccgcttcaag aagtgcatcg ccgtgggcat ggccatggac 780
ttggttctag atgactcgaa gcgggtggcc aagcgtaagc tgattgagca gaaccgggag 840
cggcggcgga aggaggagat gatccgatca ctgcagcagc gaccagagcc cactcctgaa 900
gagtgggatc tgatccacat tgccacagag gcccatcgca gcaccaatgc ccagggcagc 960
cattggaaac agaggcggaa attcctgccc gatgacattg gccagtcacc cattgtctcc 1020
atgccggacg gagacaaggt ggacctggaa gccttcagcg agtttaccaa gatcatcacc 1080
ccggccatca cccgtgtggt ggactttgcc aaaaaactgc ccatgttctc cgagctgcct 1140
tgcgaagacc agatcatcct cctgaagggg tgctgcatgg agatcatgtc cctgcgggcg 1200
gctgtccgct acgaccctga gagcgacacc ctgacgctga gtggggagat ggctgtcaag 1260
cgggagcagc tcaagaatgg cggcctgggc gtagtctccg acgccatctt tgaactgggc 1320
aagtcactct ctgcctttaa cctggatgac acggaagtgg ctctgctgca ggctgtgctg 1380
ctaatgtcaa cagaccgctc gggcctgctg tgtgtggaca agatcgagaa gagtcaggag 1440
gcgtacctgc tggcgttcga gcactacgtc aaccaccgca aacacaacat tccgcacttc 1500
tggcccaagc tgctgatgaa ggagagagaa gtgcagagtt cgattctgta caaggggggca 1560
gcggcagaag gccggccggg cgggtcactg ggcgtccacc cggaaggaca gcagcttctc 1620
ggaatgcatg ttgttcaggg tccgcaggtc cggcagcttg agcagcagct tggtgaagcg 1680
ggaagtctcc aagggccggt tcttcagcac cagagcccga agagcccgca gcagcgtctc 1740
ctggagctgc tccaccgaag cggaattctc catgcccgag cggtctgtgg ggaagacgac 1800
agcagtgagg cggactcccc gagctcctct gaggaggaac cggaggtctg cgaggacctg 1860
gcaggcaatg cagcctctcc ctgaagcccc ccagaaggcc gatggggaag gagaaggagt 1920
gccatacctt ctcccaggcc tctgccccaa gagcaggagg tgcctgaaag ctgggagcgt 1980
gggctcagca gggctggtca cctcccatcc cgtaagacca ccttcccttc ctcagcaggc 2040
caaacatggc cagactccct tgctttttgc tgtgtagttc cctctgcctg ggatgccctt 2100
ccccctttct ctgcctggca acatcttact tgtcctttga ggccccaact caagtgtcac 2160
ctccttcccc agctccccca ggcagaaata gttgtctgtg cttccttggt tcatgcttct 2220
actgtgacac ttatctcact gttttataat tagtcgggca tgagtctgtt tcccaagcta 2280
gactgtgtct gaatcatgtc tgtatcccg 2309

<210> 16

<211> 2355

<212> DNA

<213> Homo sapiens

<400> 16

```
ccgttgcctc aacgtccaac ccttctgcag ggctgcagtc cggccacccc aagaccttgc     60
tgcagggtgc ttcggatcct gatcgtgagt cgcggggtcc actccccgcc cttagccagt    120
gcccaggggg caacagcggc gatcgcaacc tctagtttga gtcaaggtcc agtttgaatg    180
accgctctca gctggtgaag acatgaccac cctggactcc aacaacaaca caggtggcgt    240
catcacctac attggctcca gtggctcctc cccaagccgc accagccctg aatccctcta    300
tagtgacaac tccaatggca gcttccagtc cctgacccaa ggctgtccca cctacttccc    360
accatccccc actggctccc tcacccaaga cccggctcgc tcctttggga gcattccacc    420
cagcctgagt gatgacggct cccccttcttc ctcatcttcc tcgtcgtcat cctcctcctc    480
cttctataat gggagccccc ctgggagtct acaagtggcc atggaggaca gcagccgagt    540
gtcccccagc aagagcacca gcaacatcac caagctgaat ggcatggtgt tactgtgtaa    600
agtgtgtggg gacgttgcct cgggcttcca ctacggtgtg ctcgcctgcg agggctgcaa    660
gggctttttc cgtcggagca tccagcagaa catccagtac aaaaggtgtc tgaagaatga    720
gaattgctcc atcgtccgca tcaatcgcaa ccgctgccag caatgtcgct tcaagaagtg    780
tctctctgtg ggcatgtctc gagacgctgt gcgttttggg cgcatcccca aacgagagaa    840
gcagcggatg cttgctgaga tgcagagtgc catgaacctg gccaacaacc agttgagcag    900
ccagtgcccg ctggagactt cacccaccca gcaccccacc ccaggcccca tgggcccctc    960
gccacccccct gctccggtcc cctcacccct ggtgggcttc tcccagtttc cacaacagct   1020
gacgcctccc agatccccaa gccctgagcc cacagtggag gatgtgatat cccaggtggc   1080
ccgggcccat cgagagatct tcacctacgc ccatgacaag ctgggcagct cacctggcaa   1140
cttcaatgcc aaccatgcat caggtagccc tccagccacc accccacatc gctgggaaaa   1200
tcagggctgc ccacctgccc ccaatgacaa caacaccttg gctgcccagc gtcataacga   1260
ggccctaaat ggtctgcgcc aggctccctc ctcctaccct cccacctggc ctcctggccc   1320
tgcacaccac agctgccacc agtccaacag caacgggcac cgtctatgcc ccacccacgt   1380
gtatgcagcc ccagaaggca aggcacctgc caacagtccc cggcagggca actcaaagaa   1440
tgttctgctg gcatgtccta tgaacatgta cccgcatgga cgcagtgggc gaacggtgca   1500
ggagatctgg gaggatttct ccatgagctt cacgcccgct gtgcgggagg tggtagagtt   1560
tgccaaacac atcccgggct tccgtgacct ttctcagcat gaccaagtca ccctgcttaa   1620
ggctggcacc tttgaggtgc tgatggtgcg ctttgcttcg ttgttcaacg tgaaggacca   1680
gacagtgatg ttcctaagcc ggaccaccta cagcctgcag gagcttggtg ccatgggcat   1740
gggagacctg ctcagtgcca tgttcgactt cagcgagaag ctcaactccc tggcgcttac   1800
cgaggaggag ctgggcctct tcaccgcggt ggtgcttgtc tctgcagacc gctcgggcat   1860
ggagaattcc gcttcggtgg agcagctcca ggagacgctg ctgcgggctc ttcgggctct   1920
ggtgctgaag aaccggccct tggagacttc ccgcttcacc aagctgctgc tcaagctgcc   1980
ggacctgcgg accctgaaca acatgcattc cgagaagctg ctgtccttcc gggtggacgc   2040
ccagtgaccc gcccggccgg ccttctgccg ctgcccccctt gtacagaatc gaactctgca   2100
cttctctctc ctttacgaga cgaaaaggaa aagcaaacca gaatcttatt tatattgtta   2160
taaaatattc caagatgagc ctctggcccc ctgagccttc ttgtaaatac ctgcctccct   2220
cccccatcac cgaacttccc ctcctcccct atttaaacca ctctgtctcc cccacaaccc   2280
tcccctggcc ctctgatttg ttctgttcct gtctcaaatc caatagttca cagctaaaaa   2340
aaaaaaaaaa aaaag                                                     2355
```

<210> 17

<211> 4119

<212> DNA

<213> Homo sapiens

<400> 17

```
gaattccgtt gctgtcgcac acacacacac acacacacac acaccccaac acacacacac      60
acaccccaac acacacacac acacacacac acacacacac acacacacac acacagcggg     120
atggccgagc gccgcacgcg tagcacgccg ggactagcta tccagcctcc cagcagcctc     180
tgcgacgggc gcggtgcgta agtacctcgc cggtggtggc cgttctccgt aagatggcgg     240
accggcggcg gcagcgcgct tcgcaagaca ccgaggacga ggaatctggt gcttcgggct     300
ccgacagcgg cggctccccg ttgcggggag gcgggagctg cagcggtagc gccggaggcg     360
gcggcagcgg ctctctgcct tcacagcgcg gaggccgaac cggggccctt catctgcggc     420
gggtggagag cgggggcgcc aagagtgctg aggagtcgga gtgtgagagt gaagatggca     480
ttgaaggtga tgctgttctc tcggattatg aaagtgcaga agactcggaa ggtgaagaag     540
gtgaatacag tgaagaggaa aactccaaag tggagctgaa atcagaagct aatgatgctg     600
ttaattcttc aacaaaagaa gagaagggag aagaaaagcc tgacaccaaa agcactgtga     660
ctggagagag gcaaagtggg gacggacagg agagcacaga gcctgtggag aacaaagtgg     720
gtaaaaaggg ccctaagcat ttggatgatg atgaagatcg gaagaatcca gcatacatac     780
ctcggaaagg gctcttcttt gagcatgatc ttcgagggca aactcaggag gaggaagtca     840
gacccaaggg gcgtcagcga aagctatgga aggatgaggg tcgctgggag catgacaagt     900
tccgggaaga tgagcaggcc ccaaagtccc gacaggagct cattgctctt tatggttatg     960
acattcgctc agctcataat cctgatgaca tcaaacctcg aagaatccgg aaaccccgat    1020
atgggagtcc tccacaaaga gatccaaact ggaacggtga gcggctaaac aagtctcatc    1080
gccaccaggg tcttgggggc accctaccac caaggacatt tattaacagg aatgctgcag    1140
gtaccggccg tatgtctgca cccaggaatt attctcgatc tgggggcttc aaggaaggtc    1200
gtgctggttt taggcctgtg gaagctggtg ggcagcatgg tggccggtct ggtgagactg    1260
ttaagcatga gattagttac cggtcacggc gcctagagca gacttctgtg agggatccat    1320
ctccagaagc agatgctcca gtgcttggca gtcctgagaa ggaagaggca gcctcagagc    1380
caccagctgc tgctcctgat gctgcaccac cacccccctga taggcccatt gagaagaaat    1440
cctattcccg ggcaagaaga actcgaacca aagttggaga tgcagtcaag cttgcagagg    1500
aggtgccccc tcctcctgaa ggactgattc cagcacctcc agtcccagaa accaccccaa    1560
ctccacctac taagactggg acctgggaag ctccggtgga ttctagtaca agtggacttg    1620
agcaagatgt ggcacaacta aatatagcag aacagaattg gagtccgggg cagccttctt    1680
tcctgcaacc acgggaactt cgaggtatgc ccaaccatat acacatggga gcaggacctc    1740
cacctcagtt taaccggatg gaagaaatgg gtgtccaggg tggtcgagcc aaacgctatt    1800
catcccagcg gcaaagacct gtgccagagc cccccgcccc tccagtgcat atcagtatca    1860
tggagggaca ttactatgat ccactgcagt tccagggacc aatctatacc catggtgaca    1920
gccctgcccc gctgcctcca cagggcatgc ttgtgcagcc aggaatgaac cttccccacc    1980
caggtttaca tccccaccag acaccagctc ctctgcccaa tccaggcctc tatccccac     2040
cagtgtccat gtctccagga cagccaccac ctcagcagtt gcttgctcct acttactttt    2100
ctgctccagg cgtcatgaac tttggtaatc ccagttaccc ttatgctcca ggggcactgc    2160
ctcccccacc accgcctcat ctgtatccta atacacaggc cccatcacag gtatatggag    2220
gagtgaccta ctataacccc gcccagcagc aggtgcagcc aaagccctcc ccaccccgga    2280
ggactcccca gccagtcacc atcaagcccc ctccacctga ggttgtaagc aggggttcca    2340
gttaatacaa gtttctgaat attttaaatc ttaacatcat ataaaaagca gcagaggtga    2400
gaactcagaa gagaaataca gctggctatc tactaccaga agggcttcaa agatataggg    2460
tgtggctcct accagcaaac agctgaaaga ggaggacccc tgccttcctc tgaggacagg    2520
ctctagagag agggagaaac aagtggacct cgtcccatct tcactcttca cttgagttgg    2580
ctgtgttcgg gggagcagag agagccagac agccccaagc ttctgagtct agatacagaa    2640
gcccatgtct tctgctgttc ttcacttctg ggaaattgaa gtgtcttctg ttcccaagga    2700
agctccttcc tgtttgtttt gttttctaag atgttcattt ttaaagcctg gcttcttatc    2760
cttaatatta ttttaatttt ttctctttgt ttctgtttct tgctctctct ccctgccttt    2820
aaatgaaaca agtctagtct tctggttttc tagcccctct ggattccctt ttgactcttc    2880
```

cgtgcatccc agataatgga gaatgtatca gccagccttc cccaccaagt ctaaaaagac 2940
ctggcctttc actttagtt ggcatttgtt atcctcttgt atacttgtat tcccttaact 3000
ctaaccctgt ggaagcatgg ctgtctgcac agagggtccc attgtgcaga aaagctcaga 3060
gtaggtgggt aggagccctt ctctttgact taggtttttta ggagtctgag catccatcaa 3120
tacctgtact atgatgggct tctgttctct gctgagggcc aataccctac tgtggggaga 3180
gatggcacac cagatgcttt tgtgagaaag ggatggtgga gtgagagcct ttgcctttag 3240
gggtgtgtat tcacatagtc ctcagggctc agtcttttga ggtaagtgga attagagggc 3300
cttgcttctc ttctttccat tcttcttgct acaccccttt tccagttgct gtggaccaat 3360
gcatctcttt aaaggcaaat attatccagc aagcagtcta ccctgtcctt tgcaattgct 3420
cttctccacg tctttcctgc tacaagtgtt ttagatgtta ctaccttatt ttccccgaat 3480
tctatttttg tccttgcaga cagaatataa aaactcctgg gcttaaggcc taaggaagcc 3540
agtcaccttc tgggcaaggg ctcctatctt tcctccctat ccatggcact aaaccacttc 3600
tctgctgcct ctgtggaaga gattcctatt actgcagtac atacgtctgc caggggtaac 3660
ctggccactg tccctgtcct tctacagaac ctgagggcaa agatggtggc tgtgtctctc 3720
cccggtaatg tcactgtttt tattccttcc atctagcagc tggcctaatc actctgagtc 3780
acaggtgtgg gatggagagt ggggagaggc acttaatctg taacccccaa ggaggaaata 3840
actaagagat tcttctaggg gtagctggtg gttgtgcctt ttgtaggctg ttccctttgc 3900
cttaaacctg aagatgtctc ctcaagcctg tgggcagcat gcccagattc ccagacctta 3960
agacactgtg agagttgtct ctgttggtcc actgtgttta gttgcaagga tttttccatg 4020
tgtggtggtg tttttttgtta ctgttttaaa gggtgcccat ttgtgatcag cattgtgact 4080
tggagataat aaaatttaga ctataaactt gaaaaaaaa 4119

<210> 18

<211> 2653

<212> DNA

<213> Homo sapiens

<400> 18
gagcgcggct ggagtttgct gctgccgctg tgcagtttgt tcaggggctt gtggcggtga 60
gtccgagagg ctgcgtgtga gagacgtgag aaggatcctg cactgaggag gtggaaagaa 120
gaggattgct cgaggaggcc tggggtctgt gagacagcgg agctgggtga aggctgcggg 180
ttccggcgag gcctgagctg tgctgtcgtc atgcctcaaa cccgatccca ggcacaggct 240
acaatcagtt ttccaaaaag gaagctgtct cgggcattga acaaagctaa aaactccagt 300
gatgccaaac tagaaccaac aaatgtccaa accgtaacct gttctcctcg tgtaaaagcc 360
ctgcctctca gccccaggaa acgtctgggc gatgacaacc tatgcaacac tccccattta 420
cctccttgtt ctccaccaaa gcaaggcaag aaagagaatg gtccccctca ctcacataca 480
cttaagggac gaagattggt atttgacaat cagctgacaa ttaagtctcc tagcaaaaga 540
gaactagcca aagttcacca aaacaaaata ctttcttcag ttagaaaaag tcaagagatc 600
acaacaaatt ctgagcagag atgtccactg aagaaagaat ctgcatgtgt gagactattc 660
aagcaagaag gcacttgcta ccagcaagca aagctggtcc tgaacacagc tgtcccagat 720
cggctgcctg ccagggaaag ggagatggat gtcatcagga atttcttgag ggaacacatc 780
tgtgggaaaa aagctggaag cctttacctt tctggtgctc ctggaactgg aaaaactgcc 840
tgcttaagcc ggattctgca agacctcaag aaggaactga aaggctttaa aactatcatg 900
ctgaattgca tgtccttgag gactgcccag gctgtattcc cagctattgc tcaggagatt 960
tgtcaggaag aggtatccag gccagctggg aaggacatga tgaggaaatt ggaaaaacat 1020
atgactgcag agaagggccc catgattgtg ttggtattgg acgagatgga tcaactggac 1080
agcaaaggcc aggatgtatt gtacacgcta tttgaatggc catggctaag caattctcac 1140
ttggtgctga ttggtattgc taataccctg gatctcacag atagaattct acctaggctt 1200
caagctagag aaaaatgtaa gccacagctg ttgaacttcc caccttatac cagaaatcag 1260
atagtcacta ttttgcaaga tcgacttaat caggtatcta gagatcaggt tctggacaat 1320
gctgcagttc aattctgtgc ccgcaaagtc tctgctgttt caggagatgt tcgcaaagca 1380
ctggatgttt gcaggagagc tattgaaatt gtagagtcag atgtcaaaag ccagactatt 1440
ctcaaaccac tgtctgaatg taaatcacct tctgagcctc tgattcccaa gagggttggt 1500
cttattcaca tatcccaagt catctcagaa gttgatggta acaggatgac cttgagccaa 1560

gagggagcac aagattcctt ccctcttcag cagaagatct tggtttgctc tttgatgctc 1620
ttgatcaggc agttgaaaat caaagaggtc actctgggga agttatatga agcctacagt 1680
aaagtctgtc gcaaacagca ggtggcggct gtggaccagt cagagtgttt gtcactttca 1740
gggctcttgg aagccagggg cattttagga ttaaagagaa acaaggaaac ccgtttgaca 1800
aaggtgtttt tcaagattga agagaaagaa atagaacatg ctctgaaaga taaagcttta 1860
attggaaata tcttagctac tggattgcct taaattcttc tcttacaccc cacccgaaag 1920
tattcagctg gcatttagag agctacagtc ttcattttag tgctttacac attcgggcct 1980
gaaaacaaat atgacctttt ttacttgaag ccaatgaatt ttaatctata gattctttaa 2040
tattagcaca gaataatatc tttgggtctt actattttta cccataaaag tgaccaggta 2100
gacccttttt aattacattc actacttcta ccacttgtgt atctctagcc aatgtgcttg 2160
caagtgtaca gatctgtgta gaggaatgtg tgtatattta cctcttcgtt tgctcaaaca 2220
tgagtgggta tttttttgtt tgtttttttt gttgttgttg tttttgaggc gcgtctcacc 2280
ctgttgccca ggctggagtg caatggcgcg ttctctgctc actacagcac ccgcttccca 2340
ggttgaagtg attctcttgc ctcagcctcc cgagtagctg ggattacagg tgcccaccac 2400
cgcgcccagc taattttta atttttagta gagacagggt tttaccatgt tggccaggct 2460
ggtcttgaac tcctgaccct caagtgatct gcccaccttg gcctccctaa gtgctgggat 2520
tataggcgtg agccaccatg ctcagccatt aaggtatttt gttaagaact ttaagtttag 2580
ggtaagaaga atgaaaatga tccagaaaaa tgcaagcaag tccacatgga gatttggagg 2640
acactggtta aag 2653

<210> 19

<211> 2907

<212> DNA

<213> Homo sapiens

<400> 19
gccatctggg cccaggcccc atgccccgag gaggggtggt ctgaagccca ccagagcccc 60
ctgccagact gtctgcctcc cttctgactg tggccgcttg gcatggccag caacagcagc 120
tcctgcccga cacctggggg cgggcacctc aatgggtacc cggtgcctcc ctacgccttc 180
ttcttccccc ctatgctggg tggactctcc ccgccaggcg ctctgaccac tctccagcac 240
cagcttccag ttagtggata tagcacacca tccccagcca ccattgagac ccagagcagc 300
agttctgaag agatagtgcc cagccctccc tcgccacccc ctctaccccg catctacaag 360
ccttgctttg tctgtcagga caagtcctca ggctaccact atggggtcag cgcctgtgag 420
ggctgcaagg gcttcttccg ccgcagcatc cagaagaaca tggtgtacac gtgtcaccgg 480
gacaagaact gcatcatcaa caaggtgacc cggaaccgct gccagtactg ccgactgcag 540
aagtgctttg aagtgggcat gtccaaggag tctgtgagaa acgaccgaaa caagaagaag 600
aaggaggtgc ccaagcccga gtgctctgag agctacacgc tgacgccgga ggtgggggag 660
ctcattgaga aggtgcgcaa agcgcaccag gaaaccttcc ctgccctctg ccagctgggc 720
aaatacacta cgaacaacag ctcagaacaa cgtgtctctc tggacattga cctctgggac 780
aagttcagtg aactctccac caagtgcatc attaagactg tggagttcgc caagcagctg 840
cccggcttca ccaccctcac catcgccgac cagatcaccc tcctcaaggc tgcctgcctg 900
gacatcctga tcctgcggat ctgcacgcgg tacacgcccg agcaggacac catgaccttc 960
tcggacgggc tgaccctgaa ccggacccag atgcacaacg ctggcttcgg ccccctcacc 1020
gacctggtct ttgccttcgc caaccagctg ctgcccctgg agatggatga tgcggagacg 1080
gggctgctca gcgccatctg cctcatctgc ggagaccgcc aggacctgga gcagccggac 1140
cgggtggaca tgctgcagga gccgctgctg gaggcgctaa aggtctacgt gcggaagcgg 1200
aggcccagcc gcccccacat gttccccaag atgctaatga agattactga cctgcgaagc 1260
atcagcgcca aggggggctga gcgggtgatc acgctgaaga tggagatccc gggctccatg 1320
ccgcctctca tccaggaaat gttggagaac tcagagggcc tggacactct gagcggacag 1380
ccgggggggtg gggggcggga cgggggtggc ctggcccccc cgccaggcag ctgtagcccc 1440
agcctcagcc ccagctccaa cagaagcagc ccggccaccc actccccgtg accgcccacg 1500
ccacatggac acagccctcg ccctccgccc cggcttttct ctgcctttct accgaccatg 1560
tgaccccgca ccagccctgc ccccacctgc cctcccgggc agtactgggg accttccctg 1620
ggggacgggg agggaggagg cagcgactcc ttggacagag gcctgggccc tcagtggact 1680

```
gcctgctccc acagcctggg ctgacgtcag aggccgaggc caggaactga gtgaggcccc   1740
tggtcctggg tctcaggatg ggtcctgggg gcctcgtgtt catcaagaca cccctctgcc   1800
cagctcacca catcttcatc accagcaaac gccaggactt ggctccccca tcctcagaac   1860
tcacaagcca ttgctcccca gctggggaac ctcaacctcc cccctgcctc ggttggtgac   1920
agagggggtg ggacaggggc gggggttcc ccctgtacat accctgccat accaacccca    1980
ggtattaatt ctcgctggtt ttgtttttat tttaattttt ttgttttgat tttttttaata   2040
agaattttca ttttaagcac atttatactg aaggaatttg tgctgtgtat tggggggagc   2100
tggatccaga gctggagggg gtgggtccgg gggagggagt ggctcggaag gggcccccac   2160
tctcctttca tgtccctgtg ccccccagtt ctcctcctca gccttttcct cctcagtttt   2220
ctctttaaaa ctgtgaagta ctaactttcc aaggcctgcc ttcccctccc tcccactgga   2280
gaagccgcca gcccctttct ccctctgcct gaccactggg tgtggacggt gtggggcagc   2340
cctgaaagga caggctcctg gccttggcac ttgcctgcac ccaccatgag gcatggagca   2400
gggcagagca agggccccgg gacagagttt tcccagacct ggctcctcgg cagagctgcc   2460
tcccgtcagg gcccacatca tctaggctcc ccagccccca ctgtgaaggg gctggccagg   2520
ggcccgagct gcccccaccc ccggcctcag ccaccagcac ccccataggg cccccagaca   2580
ccacacacat gcgcgtgcgc acacacacaa acacacacac actggacagt agatgggccg   2640
acacacactt ggcccgagtt cctccatttc cctggcctgc cccccacccc caacctgtcc   2700
cacccccgtg ccccctcctt accccgcagg acgggcctac agggggtct cccctcaccc    2760
ctgcaccccc agctggggga gctggctctg ccccgacctc cttcaccagg ggttggggcc   2820
ccttcccctg gagcccgtgg gtgcacctgt tactgttggg ctttccactg agatctactg   2880
gataaagaat aaagttctat ttattct                                      2907
```

<210> 20

<211> 2096

<212> DNA

<213> Homo sapiens

<220>

<221> misc_feature

<222> (23)..(23)

<223> n=a, c, g or t

<220>

<221> misc_feature

<222> (27)..(27)

<223> n=a, c, g or t

<220>

<221> misc_feature

<222> (80)..(80)

<223> n=a, c, g or t

<220>

<221> misc_feature

<222> (120)..(120)

<223> n=a, c, g or t

<400> 20
```
agatgtttaa aaatactttg atnctcngtt tccacctctc ttaaattgtc tttccctatg      60
ttaaatatac agtcatcacn ttgctgaaaa aagttcgcaa tgagaacaat catctaaaan     120
tggctgtaac taggtcaggc gcggttgctc atgcctgtaa tcccaccact ttgggaggcc     180
gaggcaattg gatcacctga ggtcaggatt ttgagaccag cttgaccaac atggtggaat     240
cccatctcta ctaaaaatac aaaaaattag ccgggtgtgg tggcacaccc ctgtaatccc     300
acctactcag gaggctgagg caggaaaatc ccttgaaccc aggaggcaaa ggttgcattg     360
agccgaaata acaccactgc actccagcct ggacgataga gtgagacccc atctcaaaaa     420
aagagcagct gtgacaaatg cctgtattga attgcaggtc agtcttccac ctccactacc     480
ggtgccaaaa aaagggctgc cccaaaagga actaaaaggg atccagcttt gaattctggt     540
gtctctcaaa agcctgatcc tgccaaaacc aagaatcgcc gcaaaaggaa gccatccact     600
tctgatgatt ctgactctaa ttttgagaaa attgtttcga aagcagtcac aagcaaggtg     660
agtgttgatc ctagtcagtc cttttgctgt agatgttctg aaacacgtaa ctaagccatt     720
gttcttaaaa atttggcata tctttaagaa aattaactct catattctgt tagcttttac     780
tgtacatatt tagttttaac aaagttaaat atgccactta tttggccaat ggaagagttg     840
gccttagatc tgcttcttat tacttggtag aaaatagaaa actccttgaa tatagtgtct     900
tgatacattt ttttacatta caattatgtt gtcagattta caatgtgcaa gttacctggg     960
cttttctctt ttagaaatcc aagggggaga gtgatgactt ccatatggac tttgactcag    1020
ctgtggctcc tcgggcaaaa tctgtacggg caaagaaacc tataaagtac ctggaagagt    1080
cagatgaaga tgatctgttt taaaatgtga ggcgattatt ttaagtaatt atcttaccaa    1140
gcccaagact ggttttaaag ttacctgaag ctcttaactt cctcccctct gaatttagtt    1200
tggggaaggt gtttttagta caagacatca aagtgaagta aagcccaagt gttctttagc    1260
tttttataat actgtataaa tagtgaccat ctcatgggca ttgtttttctt ctctgctttg   1320
tctgtgtttt gagtctgctt cttttgtctt taaaacctga tttttaagtt cttctgaact    1380
gtagaaatag ctatctgatc acttcagcgt aaagcagtgt gtttattaac catccactaa    1440
gctaaaacta gagcagtttg atttaaaagt gtcactcttc ctccttttct actttcagta    1500
gatatgagat agagcataat tatctgtttt atcttagttt tatacataat ttaccatcag    1560
atagaacttt atggttctag tacagatact ctactacact cagcctctta tgtgccaagt    1620
ttttctttaa gcaatgagaa attgctcatg ttcttcatct tctcaaatca tcagaggccg    1680
aagaaaaaca ctttggctgt gtctataact tgacacagtc aatagaatga agaaaattag    1740
agtagttatg tgattatttc agctcttgac ctgtcccctc tggctgcctc tgagtctgaa    1800
tctcccaaag agagaaacca atttctaaga ggactggatt gcagaagact cggggacaac    1860
atttgatcca agatcttaaa tgttatattg ataaccatgc tcagcaatga gctattagat    1920
tcattttggg aaatctccat aatttcaatt tgtaaacttt gttaagacct gtctacattg    1980
ttatatgtgt gtgacttgag taatgttatc aacgtttttg taaatattta ctatgttttt    2040
ctattagcta aattccaaca attttgtact ttaataaaat gttctaaaca ttgaaa        2096
```

<210> 21

<211> 2160

<212> DNA

<213> Homo sapiens

<400> 21
agccccctgc ccctcgccgc cccccgccgc ctgcctgggc cgggccgagg atgcggcgca 60
gcgcctcggc ggccaggctt gctcccctcc ggcacgcctg ctaacttccc ccgctacgtc 120
cccgttcgcc cgccgggccg ccccgtctcc ccgcggcctc cgggtccggg tcctccagga 180
cggccaggcc gtgccgccgt gtgccctccg ccgctcgccc gcgcgccgcg cgctccccgc 240
ctgcgcccag cgcccccgcgc ccgcgcccca gtcctcgggc ggtccatgct gcccctctgc 300
ctcgtggccg ccctgctgct ggccgccggg cccgggccga gcctgggcga cgaagccatc 360
cactgcccgc cctgctccga ggagaagctg gcgcgctgcc gcccccccgt gggctgcgag 420
gagctggtgc gagaggcggg ctgcggctgt tgcgccactt gcgccctggg cttggggatg 480
ccctgcgggg tgtacacccc ccgttgcggc tcgggcctgc gctgctaccc gccccgaggg 540
gtggagaagc ccctgcacac actgatgcac gggcaaggcg tgtgcatgga gctggcggag 600
atcgaggcca tccaggaaag cctgcagccc tctgacaagg acgagggtga ccaccccaac 660
aacagcttca gcccctgtag cgcccatgac cgcaggtgcc tgcagaagca cttcgccaaa 720
attcgagacc ggagcaccag tgggggcaag atgaaggtca atggggcgcc ccgggaggat 780
gcccggcctg tgccccaggg ctcctgccag agcgagctgc accgggcgct ggagcggctg 840
gccgcttcac agagccgcac ccacgaggac ctctacttca tccccatccc caactgcgac 900
cgcaacggca acttccaccc caagcagtgt cacccagctc tggatgggca gcgtggcaag 960
tgctggtgtg tggaccggaa gacgggggtg aagcttccgg ggggcctgga gccaaagggg 1020
gagctggact gccaccagct ggctgacagc tttcgagagt gaggcctgcc agcaggccag 1080
ggactcagcg tcccctgcta ctcctgtgct ctggaggctg cagagctgac ccagagtgga 1140
gtctgagtct gagtcctgtc tctgcctgcg gcccagaagt ttccctcaaa tgcgcgtgtg 1200
cacgtgtgcg tgtgcgtgcg tgtgtgtgtg tttgtgagca tgggtgtgcc cttggggtaa 1260
gccagagcct ggggtgttct ctttggtgtt acacagccca agaggactga gactggcact 1320
tagcccaaga ggtctgagcc ctggtgtgtt tccagatcga tcctggattc actcactcac 1380
tcattccttc actcatccag ccacctaaaa acatttactg accatgtact acgtgccagc 1440
tctagttttc agccttggga ggttttattc tgacttcctc tgattttggc atgtggagac 1500
actcctataa ggagagttca agcctgtggg agtagaaaaa tctcattccc agagtcagag 1560
gagaagagac atgtaccttg accatcgtcc ttcctctcaa gctagcccag agggtgggag 1620
cctaaggaag cgtggggtag cagatggagt aatggtcacg aggtccagac ccactcccaa 1680
agctcagact tgccaggctc cctttctctt cttccccagg tccttccttt aggtctggtt 1740
gttgcaccat ctgcttggtt ggctggcagc tgagagccct gctgtgggag agcgaagggg 1800
gtcaaaggaa gacttgaagc acagagggct agggaggtgg ggtacatttc tctgagcagt 1860
cagggtggga agaaagaatg caagagtgga ctgaatgtgc ctaatggaga agacccacgt 1920
gctaggggat gaggggcttc ctgggtcctg ttcccctacc ccatttgtgg tcacagccat 1980
gaagtcaccg ggatgaacct atccttccag tggctcgctc cctgtagctc tgcctccctc 2040
tccatatctc cttcccctac acctccctcc ccacacctcc ctactcccct gggcatcttc 2100
tggcttgact ggatggaagg agacttagga acctaccagt tggccatgat gtcttttctt 2160

<210> 22

<211> 2215

<212> DNA

<213> Homo sapiens

<400> 22
ctgcagggag ccatgattgc accactgcac tccagcctgg gcaacagagt gagaccatgt 60
ctcaagaaaa aaaaaaaaga aagaaaccac tgctctaggc taaatcccag ccagagttgg 120
agccacccag ctaaactggc ctgttttccc tcatttcctt ccccgaaggt atgcctgtgt 180
caagatgagg tcacggacga ttacatcgga gacaacacca cagtggacta cactttgttc 240
gagtctttgt gctccaagaa ggacgtgcgg aactttaaag cctggttcct ccctatcatg 300
tactccatca tttgtttcgt gggcctactg ggcaatgggc tggtcgtgtt gacctatatc 360
tatttcaaga ggctcaagac catgaccgat acctacctgc tcaacctggc ggtggcagac 420
atcctcttcc tcctgaccct tcccttctgg gcctacagcg cggccaagtc ctgggtcttc 480
ggtgtccact tttgcaagct catctttgcc atctacaaga tgagcttctt cagtggcatg 540
ctcctacttc tttgcatcag cattgaccgc tacgtggcca tcgtccaggc tgtctcagct 600

```
caccgccacc gtgcccgcgt ccttctcatc agcaagctgt cctgtgtggg catctggata    660
ctagccacag tgctctccat cccagagctc ctgtacagtg acctccagag gagcagcagt    720
gagcaagcga tgcgatgctc tctcatcaca gagcatgtgg aggcctttat caccatccag    780
gtggcccaga tggtgatcgg ctttctggtc cccctgctgg ccatgagctt ctgttacctt    840
gtcatcatcc gcaccctgct ccaggcacgc aactttgagc gcaacaaggc catcaaggtg    900
atcatcgctg tggtcgtggt cttcatagtc ttccagctgc cctacaatgg ggtggtcctg    960
gcccagacgg tggccaactt caacatcacc agtagcacct gtgagctcag taagcaactc   1020
aacatcgcct acgacgtcac ctacagcctg gcctgcgtcc gctgctgcgt caaccctttc   1080
ttgtacgcct tcatcggcgt caagttccgc aacgatctct tcaagctctt caaggacctg   1140
ggctgcctca gccaggagca gctccggcag tggtcttcct gtcggcacat ccggcgctcc   1200
tccatgagtg tggaggccga gaccaccacc accttctccc cataggcgac tcttctgcct   1260
ggactagagg gacctctccc agggtccctg ggtggggat agggagcaga tgcaatgact   1320
caggacatcc ccccgccaaa agctgctcag ggaaaagcag ctctcccctc agagtgcaag   1380
ccctgctcca gaagttagct tcaccccaat cccagctacc tcaaccaatg ccgaaaaaga   1440
cagggctgat aagctaacac cagacagaca acactgggaa acagaggcta ttgtccccta   1500
aaccaaaaac tgaaagtgaa agtccagaaa ctgttcccac ctgctggagt gaaggggcca   1560
aggagggtga gtgcaagggg cgtgggagtg gcctgaagag tcctctgaat gaaccttctg   1620
gcctcccaca gactcaaatg ctcagaccag ctcttccgaa aaccaggcct tatctccaag   1680
accagagata gtggggagac ttcttggctt ggtgaggaaa agcggacatc agctggtcaa   1740
acaaactctc tgaacccctc cctccatcgt ttcttcact gtcctccaag ccagcgggaa   1800
tggcagctgc cacgccgccc taaaagcaca ctcatcccct cacttgccgc gtcgccctcc   1860
caggctctca acaggggaga gtgtggtgtt tcctgcaggc caggccagct gcctccgcgt   1920
gatcaaagcc acactctggg ctccagagtg ggatgacat gcactcagct cttggctcca   1980
ctgggatggg aggagaggac aagggaaatg tcaggggcgg ggagggtgac agtggccgcc   2040
caaggccacg agcttgttct ttgttctttg tcacagggac tgaaaacctc tcctcatgtt   2100
ctgctttcga ttcgttaaga gagcaacatt ttacccacac acagataaag ttttcccttg   2160
aggaaacaac agctttaaaa gaaaaaagaa aaaaaaagct tggtaagtca agtag        2215
```

<210> 23

<211> 958

<212> DNA

<213> Homo sapiens

<400> 23

```
ggggccggac gcgagggggcg gggcgagcgc gggacaaagg gaagcgaagc cggagctgcg      60
ggcgcttttt ctgcccgcgg tgtctcagat tcattcttaa ggaactgaga acttaatctt    120
ccaaaatgtc aaaaagacca tcttatgccc cacctcccac cccagctcct gcaacacaaa    180
tgcccagcac accagggttt gtgggataca atccatacag tcatctcgcc tacaacaact    240
acaggctggg agggaacccg agcaccaaca gccgggtcac ggcatcctct ggtatcacga    300
ttccaaaacc cccaaagcca ccagataagc cgctgatgcc ctacatgagg tacagcagaa    360
aggtctggga ccaagtaaag gcttccaacc ctgacctaaa gttgtgggag attggcaaga    420
ttattggtgg catgtggcga gatctcactg atgaagaaaa acaagaatat ttaaacgaat    480
acgaagcaga aaagatagag tacaatgaat ctatgaaggc ctatcataat tccccccgcgt    540
accttgctta cataaatgca aaaagtcgtg cagaagctgc tttagaggaa gaaagtcgac    600
agagacaatc tcgcatggag aaaggagaac cgtacatgag cattcagcct gctgaagatc    660
cagatgatta tgatgatggc ttttcaatga agcatacagc caccgcccgt ttccagagaa    720
accaccgcct catcagtgaa attcttagtg agagtgtggt gccagacgtt cggtcagttg    780
tcacaacagc tagaatgcag gtcctcaaac ggcaggtcca gtccttaatg gttcatcagc    840
gaaaactaga agctgaactt cttcaaatag aggaacgaca ccaggagaag aagaggaaat    900
tcctggaaag cacagattca tttaacaatg aacttaaaag gttgtgcggt ctgaaagt       958
```

<210> 24

<211> 6483

<212> DNA

<213> Homo sapiens

<400> 24
aagcttctaa ttgcagttca accacctgtt acatatcttc aggaaaaaat cacaacctct 60
caacttcaac ttcctcttct ataaattaga aataacaata accacacctg taaccccagc 120
actttgggag gccaaggcag gcagatcaag aggtgaggag attgagacca tcctggctaa 180
catgatgaaa ccctgtctct accaaaaaga caaaaaatta gccaggtatg gtggcacaca 240
cctgtagtcc cagctactcg ggaggctgag gcaggagaat ggcgtgaacc cgggaggtgg 300
agcttgcagt gagccgagat ggcgccactg cactccagcc tgggcgacag agcaagcctc 360
cgtctaaaaa aaaaaaaaga aagaaagaaa gaaagaaaga aaagaaataa taataaccac 420
cattcctatc tcaacagctt gttctagaaa tttttaaagc acagtatcac aaacagcact 480
acataattgt aaaacatgta tgaatatata catccaaaca acagcaatgt catagcctat 540
gggtagatat aatcttatac aatgtaccaa aatcccaatt tacttcacta gacaaactgt 600
tataccaaat tctgtacaca gtatatccaa gaaaatgtgt tgtttttatt gagaaactga 660
acctagcttg ggaacacatg tgcacagtct agttcataat atttggtgca agtatcattc 720
tctaatatag atttacattt ttgcaagcaa atttttactt gcaatcgtaa catatccaaa 780
ttttcccttt ttactcaatc agaacttagt gtaaagtact acaagttagt tcttcggatt 840
tcatgctaag aaaataatgc agattttctg cattattatg gtcttcacag aaaccttaac 900
tatgatgaat ttaaaagtgc aaaataatcc aggataactt tatgatttca catttttttaa 960
tgttaaaaat aatgccatca ttaattagaa aattctaaaa tcattacttc cactttctta 1020
ggcaaaatat caatatactc tcatttgcca aataaattaa aagatctcct acaaacacaa 1080
tctcctaaat tgtggtttta tggctttaat gttttatgtg tggcaactat tgatgctagt 1140
taaaatttta gaaactcttt ctttttgatt ccctacagtt gtctacaaga accttattgt 1200
agcatgatcc tgccagactt tatactattt gttgctccaa ttaaaactgt ttaaaacatg 1260
aatttgaaaa atcttatttt aactataatt ttgtagctga aactttttttt tctaaacttt 1320
gcaaacattc tatgcaacct gaattagtgc tgagaaaatt ggatcttaat ggttgctcaa 1380
tgttcttcaa caggtgaaaa gcataataaa acatgctcat ctgaactcca cccattttca 1440
atttcaacat agcatacctc gtgtttattc ttagggcaaa ttcaaaattg tacatattag 1500
gattggttat tactgaagat aatttatgca atcataagcc aaagatgcta agttggcaaa 1560
aagaaaacaa tgtaagtaag caaactctaa cacatgtgga cacaccctct cagtatataa 1620
aggcttgtca ctgtccttgg tagcaggcac tccctgggct aaacagcatc accatgtctg 1680
ttcgatacag ctcaagcaag cactactctt cctcccgcag tggaggagga ggaggaggag 1740
gaggatgtgg aggaggagga ggagtgtcat ccctaagaat ttctagcagc aaaggctccc 1800
ttggtggagg atttagctca ggggggttca gtggtggctc ttttagccgt gggagctctg 1860
gtggggggatg ctttggggggc tcatcaggtg gctatggagg attaggaggt tttggtggag 1920
gtagctttca tggaagctat ggaagtagca gctttggtgg gagttatgga ggcagctttg 1980
gagggggcaa tttcggaggt ggcagctttg gtgggggcag ctttggtgga ggcggctttg 2040
gtggaggcgg ctttggagga ggctttggtg gtggatttgg aggagatggt ggccttctct 2100
ctggaaatga aaaagtaacc atgcagaatc tgaatgaccg cctggcttcc tacttggaca 2160
aagttcgggc tctggaagaa tcaaactatg agctggaagg caaaatcaag gagtggtatg 2220
aaaagcatgg caactcacat caggggggagc ctcgtgacta cagcaaatac tacaaaacca 2280
tcgatgacct taaaaatcag gtaagaggta tttttaaatc cagctttaag tatcttgtcc 2340
atgtaatcca gacagatgaa tcttaaatta agcacaatgt ggctgttcac tatgcttacc 2400
catgttactt tcttccttca aaaataaccc agtctcatca aagataaaca tctgtgaaac 2460
tatggtcatg gcaatcttca tccagcaagt gtgctacttg tcttaagagg atgggagatt 2520
tactaagcac ttttgaggtt ttaatgagca tacaatgagt ccacagttaa aatatgctag 2580
gctatttaca aatgtagaaa ctgaaaaaaa aaatcatgat atgaatcaga acaaaatgtt 2640
attcagactg ataacaagcc atattcagta ccaacatggc aagaaaaata aattttccag 2700
tatgaaaatg ggacactgct tgcttctaag gaatttctga attgtaccta ttgtgtacca 2760
gttcagagtg tatttattta ttagtattta tcatgagtta aacaaatgca ggtgtgagtc 2820
agccaaagca tggctgaaat acatggaaat cacatagtct aaaagaggag ggcacactta 2880

caggaataca tctatataat tccagttagt tttcagaaag gaataattcg tgtacagaaa 2940
tacaagactg gagaaattcc aagagaacaa ataattcaaa gttaagtata tgggtaagcc 3000
tgcaatattt catatttaaa ataaaaaatt ttcccaagat tttgtaagag aacaacataa 3060
aagtgcagag tgcatctatg tcactacaaa agccatatct gcatctgacc tcttctcaaa 3120
taactgtgcc tctccctcca gattctcaac ctaacaactg ataatgccaa catcctgctt 3180
cagatcgaca atgccaggct ggcagctgat gacttcaggc tgaagtaagt taagtgatcg 3240
ttgtataata ctatcacaac gaatacatca gtggttttta acaatgactt gggatgccct 3300
caataacatt tacatttttc tgaattcacc caaagttaaa tagtattgga gttatctgag 3360
aaattttcca tgtcagtgtt accttttttgg caatattaaa ggaagaaaat gcatattaaa 3420
gtaactgcta aggtttttttc cattaaacca ctattacttc taagagaact gtacatgaca 3480
aatattgcca ttacatgaga tcaactatgt agttgctttt taaatagtct ctgcccagat 3540
acatctcccc tatataagtt ataaccagta ttgatatcat gcttgtttca ggtatgagaa 3600
tgaggtagct ctgcgccaga gcgtggaggc tgacatcaac ggcctgcgta gggtgctgga 3660
tgagctgacc ctgaccaagg ctgacctgga gatgcaaatt gagagcctga ctgaagagct 3720
ggcctatctg aagaagaacc acgaggaggt gacacaaaag ttatactttt cccagccaaa 3780
agagagttca ttatggtcct cgtgtagcca ataaatcttt ctgttcctca aacaggaaat 3840
gaaagacctt cgaaatgtgt ccactggtga tgtgaatgtg gaaatgaatg ctgccccggg 3900
tgttgatctg actcaacttc tgaataacat gagaagccaa tatgaacaac ttgctgaaca 3960
aaaccgcaaa gatgctgaag cctggttcaa tgaaaaggta aagtaatctt ccttatagtg 4020
aaactcatgg aggttttatc atttcagaat ttcctcaccc ttttccttgt ttttaatact 4080
ctagagcaag gaactgacta cagaaattga taataacatt gaacagatat ccagctataa 4140
atctgagatt actgaattga gacgtaatgt acaagctctg gagatagaac tacagtccca 4200
actggccttg gtatgttaac tctcatgaaa tgacttcaac tttatcatac aaagtttcat 4260
gctcacctaa gaatatgcaa tgcaacaaaa aaatgcagag ttggaggtaa gaaagagaaa 4320
acaaagtgaa gctcatgtta atggaggaaa agtactacta gtgttgatct aaaagtgctg 4380
aaactgaaat ggtgccatta aacatacaac aaattctgtt catttctta ttcttctata 4440
taatgcctta ctaaataatc aaataagcgt caccatactc aactgaacaa ggaagtcact 4500
aagccacaaa aaaatccgtt tcagaaacaa tccctggaag cctccttggc agaaacagaa 4560
ggtcgctact gtgtgcagct ctcacagatt cacgcccaga tatccgctct ggaagaacag 4620
ttgcaacaga ttcgagctga aaccgagtgc cagaatactg aataccaaca actcctggat 4680
attaagatcc gactggagaa tgaaattcaa acctaccgca gcctgctaga aggagaggga 4740
aggtaaatta taacatgaaa agttatccca gtttctttta ttcaatattc cagatagcaa 4800
ggcttatcta aaccccaaga agatgccaga gaatgagagg aagggaggag agagggtaga 4860
gtacagaaaa aggagtacgc aaccgcaatc tcactttctc atgaatttgg cccaaaatga 4920
ttcttaagag ttctgtgaac ttaacattgt tttcaaagga tgggttttaa aatatatacc 4980
tggcagggtt ttatttttttc aacacgtttt gcttattttc taaattaacg gcaactggaa 5040
agctacccac cgttttccaa cgttagagat aaccgaatgt gacctcaccc cgtttagttc 5100
cggaggcggc ggacgcggcg gcggaagttt cggcggcggc tacggcggcg gaagctccgg 5160
cggcggaagc tccggcggcg gctacggcgg cggccacggc ggcagttccg gcggcggcta 5220
cggaggcgga agctccggcg gcggaagctc cggcggcggc tacgggggcg gaagctccag 5280
cggcggccac ggcggcggaa gctccagcgg cggccacggc ggcagttcca gcggcggcta 5340
cggtggtggc agttccggcg gcggcggcgg cggctacggg ggcggcagct ccggcggcgg 5400
cagcagctcc ggcggcggat acggcggcgg cagctccagc ggaggccaca agtcctcctc 5460
ttccgggtcc gtgggcgagt cttcatctaa gggaccaagg tcagcagaaa ctagctgggg 5520
taatctagaa ttagttttaa cttcctgtga tggttttttt gcgctttaag ctctagagtt 5580
gttttaaaaa attaaaaatc ttagagacgg ttccgtttgc atttgttcac aaactactct 5640
taacaccagc cgtgaaaaat ggcatgatca aaatgtcata ccttaagcat ttttttgggc 5700
ttaacaatgt aaagttgaaa tttccttctt tttacaatat ttgcttgtta attactaagg 5760
atccctacag actgtttaaa attttttttc catcattcac acagatacta acaaaaccag 5820
agtaatcaag acaattattg aagaggtggc gcccgacggt agagttcttt catctatggt 5880
tgaatcagaa accaagaaac actactatta aactgcatca agaggaaaga gtctcccttc 5940
acacagacca ttatttacag atgcatggaa aacaaagtct ccaagaaaac acttctgtct 6000
tgatggtcta tggaaataga ccttgaaaat aaggtgtcta caaggtgttt tgtggtttct 6060
gtatttcttc ttttcacttt accacaaagt gttctttaat ggaaagaaaa acaactttgt 6120
gttctcattt actaatgaat ttcaataaac tttcttactg atgcaaacta tcccaatttg 6180
tcagaattta tctttactta agtacataat actctttaaa attaaagatt agtaacccat 6240
agcagttgaa ggttgatgta tccagaaatt cggaagacag aactattgtc atgccttttc 6300
taagtttttt aatcatgtat gttcagacca ccgtcagtaa attcactgag taaagtctgt 6360
aaatccccaa tattactctt taagatacac aatatgtgga aggctcccag ctctctggct 6420

ttaaattatt tcaatcctgg aaattctgga atatctcaaa tataaccccc aaaataataa 6480
taa 6483

<210> 25

<211> 1871

<212> DNA

<213> Homo sapiens

<400> 25
agttgtggcc accttcccca ggccatggat ctctccaaca acaccatgtc actctcagtg 60
cgcacccccg gactgtcccg gcggctctcc tcgcagagtg tgataggcag acccaggggc 120
atgtctgctt ccagtgttgg aagtggttat gggggaagtg cctttggctt tggagccagc 180
tgtgggggag gcttttctgc tgcttccatg tttggttcta gttccggctt tgggggtggc 240
tccggaagtt ccatggcagg aggactgggt gctggttatg ggagagccct gggtggaggt 300
agctttggag ggctggggat gggatttggg ggcagcccag gaggtggctc tctaggtatt 360
ctctcgggca atgatggagg ccttctttct ggatcagaaa aagaaactat gcaaaatctt 420
aatgatagat tagcttccta cctggataag gtgcgagctc tagaagaggc taatactgag 480
ctagaaaata aaattcgaga atggtatgaa acacgaggaa ctgggactgc agatgcttca 540
cagagcgatt acagcaaata ttatccactg attgaagacc tcaggaataa gatcatttca 600
gccagcattg gaaatgccca gctcctcttg cagattgaca atgcgagact agctgctgag 660
gacttcagga tgaagtatga gaatgaactg gccctgcgcc agggcgtaga ggccgacatc 720
aatggcctgc gccgggtgct ggacgagctg accctgacca ggaccgacct ggagatgcag 780
atcgagagcc tgaacgagga gctggcctac atgaagaaga accacgagga tgagctccaa 840
agcttccggg tgggcggccc aggcgaggtc agcgtagaaa tggacgctgc cccgggagtg 900
gacctcacca ggctcctcaa tgatatgcgg gcgcagtatg aaaccatcgc tgagcagaat 960
cggaaggacg ctgaagcctg gttcattgaa aagagcgggg agctccgtaa ggagattagc 1020
accaacaccg agcagcttca gtccagcaag agcgaggtca ccgacctgcg tcgcgccttt 1080
cagaacctgg agatcgagct acagtcccag ctcgccatga agaaatccct ggaggactcc 1140
ttggccgaag ccgagggcga ttactgcgcg cagctgtccc aggtgcagca gctcatcagc 1200
aacctggagg cacagctgct ccaggtgcgc gcggacgcag agcgccagaa cgtggaccac 1260
cagcggctgc tgaatgtcaa ggcccgcctg gagctggaga ttgagaccta ccgccgcctg 1320
ctggacgggg aggcccaagg tgatggtttg gaggaaagtt tatttgtgac agactccaaa 1380
tcacaagcac agtcaactga ttcctctaaa gacccaacca aaacccgaaa aatcaagaca 1440
gttgtgcagg agatggtgaa tggtgaggtg gtctcatctc aagttcagga aattgaagaa 1500
ctaatgtaaa atttcacaag atctgcccca tgattggttc cttaggaaca agaaatttac 1560
aagtagaaat tattcctttc agagtaacat gctgtattac ttcaatccct atttttgtct 1620
gttccatttt ctttggattc cctattcaca ttgaatcctt tttgcccttc tgaaacaata 1680
ttcagtcaca agtcattttg gtcatgttgg tctttgtaac aaatcaaaat taccttatat 1740
ccttctggac aactggagta gtcttttaac gaactttctt ctggtaaccc ggaatatttt 1800
cttaatcata gagctttact caagtagtat tgttttaata gagttaattg taataaaaga 1860
tgaatggtaa a 1871

<210> 26

<211> 1447

<212> DNA

<213> Homo sapiens

<400> 26
ctgcaactgg ttctgcgagg gctccttcaa tggcagcgag aaggagacta tgcagttcct 60
gaacgaccgc ctggccagct acctggagaa ggtgcgtcac gtggagcggg acaacgcgga 120
gctggagaac ctcatccggg agcggtctca gcagcaggag cccttgctgt gccccagcta 180
ccagtcctac ttcaagacca ttgaggagct ccagcagaag atcctgtgca gcaagtctga 240
gaatgccagg ctggtggtgc agatcgacaa tgccaagctg gctgcagatg acttcagaac 300
caagtaccag acggagcagt ccctgcggca gctggtggag tccgacatca acagcctgcg 360
caggattctg gatgagctga ccctgtgcag gtctgacctg gaggcccaga tggagtccct 420
gaaggaggag ctgctgtccc tcaagcagaa ccatgagcag gaagtcaaca ccttgcgctg 480
ccagcttgga gaccgcctca acgtggaggt ggacgctgct cccgctgtgg acctgaacca 540
ggtcctgaac gagaccagga atcagtatga ggccctggtg gaaaccaacc gcagggaagt 600
ggagcaatgg ttcgccacgc agaccgagga gctgaacaag caggtggtat ccagctcgga 660
gcagctgcag tcctaccagg cggagatcat cgagctgaga cgcacagtca atgccctgga 720
gatcgagctg caggcccagc acaacctgcg atactctctg gaaaacacgc tgacagagag 780
cgaggcccgc tacagctccc agctgtccca ggtgcagagc ctgatcacca acgtggagtc 840
ccagctggcg gagatccgca gtgacctgga gcggcagaac caggagtatc aggtgctgct 900
ggacgtgcgg gcgcggctgg agtgtgagat caacacatac cggagcctgc tggagagcga 960
ggactgcaag ctgccctcca accccgcgc caccaccaat gcatgtgaaa agcccattgg 1020
atcctgtgtc accaatcctt gtggtcctcg ttcccgctgt gggccttgca acacctttgg 1080
gtactagata ccctggggcc agcagaagta tagcatgaag acagaactac catcggtggg 1140
ccagttctgc ctctctgaca accatcagcc accggacccc accccgaggc atcaccacaa 1200
atcatggtct ggaaggagaa caaatgccca gcgtttgggt ctgactctga gcctagggct 1260
actgatcctc ctcaccccag gtccctctcc tgtagtcagt ctgagttctg atggtcagag 1320
gttggagctg tgacagtggc atacgaggtg ttttgttctc tctgctgctt ctacctttat 1380
tgcagttccc caaatcgcct aataaacttt cctcttgcaa agcagacaaa aaaaaaaaaa 1440
aaaaaaa 1447

<210> 27

<211> 261

<212> PRT

<213> Homo sapiens

<400> 27
Met Asn Pro Asn Cys Ala Arg Cys Gly Lys Ile Val Tyr Pro Thr Glu
1 5 10 15
Lys Val Asn Cys Leu Asp Lys Phe Trp His Lys Ala Cys Phe His Cys
20 25 30
Glu Thr Cys Lys Met Thr Leu Asn Met Lys Asn Tyr Lys Gly Tyr Glu
35 40 45
Lys Lys Pro Tyr Cys Asn Ala His Tyr Pro Lys Gln Ser Phe Thr Met
50 55 60
Val Ala Asp Thr Pro Glu Asn Leu Arg Leu Lys Gln Gln Ser Glu Leu
65 70 75 80
Gln Ser Gln Val Arg Tyr Lys Glu Glu Phe Glu Lys Asn Lys Gly Lys
85 90 95
Gly Phe Ser Val Val Ala Asp Thr Pro Glu Leu Gln Arg Ile Lys Lys
100 105 110
Thr Gln Asp Gln Ile Ser Asn Ile Lys Tyr His Glu Glu Phe Glu Lys
115 120 125
Ser Arg Met Gly Pro Ser Gly Gly Glu Gly Met Glu Pro Glu Arg Arg
130 135 140
Asp Ser Gln Asp Gly Ser Ser Tyr Arg Arg Pro Leu Glu Gln Gln Gln
145 150 155 160

Pro His His Ile Pro Thr Ser Ala Pro Val Tyr Gln Gln Pro Gln Gln
165 170 175
Gln Pro Val Ala Gln Ser Tyr Gly Gly Tyr Lys Glu Pro Ala Ala Pro
180 185 190
Val Ser Ile Gln Arg Ser Ala Pro Gly Gly Gly Gly Lys Arg Tyr Arg
195 200 205
Ala Val Tyr Asp Tyr Ser Ala Ala Asp Glu Asp Glu Val Ser Phe Gln
210 215 220
Asp Gly Asp Thr Ile Val Asn Val Gln Gln Ile Asp Asp Gly Trp Met
225 230 235 240
Tyr Gly Thr Val Glu Arg Thr Gly Asp Thr Gly Met Leu Pro Ala Asn
245 250 255
Tyr Val Glu Ala Ile
260

<210> 28

<211> 478

<212> PRT

<213> Homo sapiens

<400> 28
Met Val Gln Lys Thr Ser Met Ser Arg Gly Pro Tyr Pro Pro Ser Gln
1 5 10 15
Glu Ile Pro Met Glu Val Phe Asp Pro Ser Pro Gln Gly Lys Tyr Ser
20 25 30
Lys Arg Lys Gly Arg Phe Lys Arg Ser Asp Gly Ser Thr Ser Ser Asp
35 40 45
Thr Thr Ser Asn Ser Phe Val Arg Gln Gly Ser Ala Glu Ser Tyr Thr
50 55 60
Ser Arg Pro Ser Asp Ser Asp Val Ser Leu Glu Glu Asp Arg Glu Ala
65 70 75 80
Leu Arg Lys Glu Ala Glu Arg Gln Ala Leu Ala Gln Leu Glu Lys Ala
85 90 95
Lys Thr Lys Pro Val Ala Phe Ala Val Arg Thr Asn Val Gly Tyr Asn
100 105 110
Pro Ser Pro Gly Asp Glu Val Pro Val Gln Gly Val Ala Ile Thr Phe
115 120 125
Glu Pro Lys Asp Phe Leu His Ile Lys Glu Lys Tyr Asn Asn Asp Trp
130 135 140
Trp Ile Gly Arg Leu Val Lys Glu Gly Cys Glu Val Gly Phe Ile Pro
145 150 155 160
Ser Pro Val Lys Leu Asp Ser Leu Arg Leu Leu Gln Glu Gln Lys Leu
165 170 175
Arg Gln Asn Arg Leu Gly Ser Ser Lys Ser Gly Asp Asn Ser Ser Ser
180 185 190
Ser Leu Gly Asp Val Val Thr Gly Thr Arg Arg Pro Thr Pro Pro Ala
195 200 205
Ser Ala Lys Gln Lys Gln Lys Ser Thr Glu His Val Pro Pro Tyr Asp
210 215 220
Val Val Pro Ser Met Arg Pro Ile Ile Leu Val Gly Pro Ser Leu Lys
225 230 235 240
Gly Tyr Glu Val Thr Asp Met Met Gln Lys Ala Leu Phe Asp Phe Leu
245 250 255

Lys His Arg Phe Asp Gly Arg Ile Ser Ile Thr Arg Val Thr Ala Asp
260 265 270
Ile Ser Leu Ala Lys Arg Ser Val Leu Asn Asn Pro Ser Lys His Ile
275 280 285
Ile Ile Glu Arg Ser Asn Thr Arg Ser Ser Leu Ala Glu Val Gln Ser
290 295 300
Glu Ile Glu Arg Ile Phe Glu Leu Ala Arg Thr Leu Gln Leu Val Ala
305 310 315 320
Leu Asp Ala Asp Thr Ile Asn His Pro Ala Gln Leu Ser Lys Thr Ser
325 330 335
Leu Ala Pro Ile Ile Val Tyr Ile Lys Ile Thr Ser Pro Lys Val Leu
340 345 350
Gln Arg Leu Ile Lys Ser Arg Gly Lys Ser Gln Ser Lys His Leu Asn
355 360 365
Val Gln Ile Ala Ala Ser Glu Lys Leu Ala Gln Cys Pro Pro Glu Met
370 375 380
Phe Asp Ile Ile Leu Asp Glu Asn Gln Leu Glu Asp Ala Cys Glu His
385 390 395 400
Leu Ala Glu Tyr Leu Glu Ala Tyr Trp Lys Ala Thr His Pro Pro Ser
405 410 415
Ser Thr Pro Pro Asn Pro Leu Leu Asn Arg Thr Met Ala Thr Ala Ala
420 425 430
Leu Arg Arg Ser Pro Ala Pro Val Ser Asn Leu Gln Val Gln Val Leu
435 440 445
Thr Ser Leu Arg Arg Asn Leu Gly Phe Trp Gly Gly Leu Glu Ser Ser
450 455 460
Gln Arg Gly Ser Val Val Pro Gln Glu Gln Glu His Ala Met
465 470 475

<210> 29

<211> 196

<212> PRT

<213> Homo sapiens

<400> 29
Met Ser Met Leu Arg Leu Gln Lys Arg Leu Ala Ser Ser Val Leu Arg
1 5 10 15
Cys Gly Lys Lys Lys Val Trp Leu Asp Pro Asn Glu Thr Asn Glu Ile
20 25 30
Ala Asn Ala Asn Ser Arg Gln Gln Ile Arg Lys Leu Ile Lys Asp Gly
35 40 45
Leu Ile Ile Arg Lys Pro Val Thr Val His Ser Arg Ala Arg Cys Arg
50 55 60
Lys Asn Thr Leu Ala Arg Arg Lys Gly Arg His Met Gly Ile Gly Lys
65 70 75 80
Arg Lys Gly Thr Ala Asn Ala Arg Met Pro Glu Lys Val Thr Trp Met
85 90 95
Arg Arg Met Arg Ile Leu Arg Arg Leu Leu Arg Arg Tyr Arg Glu Ser
100 105 110
Lys Lys Ile Asp Arg His Met Tyr His Ser Leu Tyr Leu Lys Val Lys
115 120 125
Gly Asn Val Phe Lys Asn Lys Arg Ile Leu Met Glu His Ile His Lys
130 135 140

Leu Lys Ala Asp Lys Ala Arg Lys Lys Leu Leu Ala Asp Gln Ala Glu
145 150 155 160
Ala Arg Arg Ser Lys Thr Lys Glu Ala Arg Lys Arg Arg Glu Glu Arg
165 170 175
Leu Gln Ala Lys Lys Glu Glu Ile Ile Lys Thr Leu Ser Lys Glu Glu
180 185 190
Glu Thr Lys Lys
195

<210> 30

<211> 1566

<212> PRT

<213> Homo sapiens

<400> 30
Met Ser Ser Leu Leu Glu Arg Leu His Ala Lys Phe Asn Gln Asn Arg
1 5 10 15
Pro Trp Ser Glu Thr Ile Lys Leu Val Arg Gln Val Met Glu Lys Arg
20 25 30
Val Val Met Ser Ser Gly Gly His Gln His Leu Val Ser Cys Leu Glu
35 40 45
Thr Leu Gln Lys Ala Leu Lys Val Thr Ser Leu Pro Ala Met Thr Asp
50 55 60
Arg Leu Glu Ser Ile Ala Gly Gln Asn Gly Leu Gly Ser His Leu Ser
65 70 75 80
Ala Ser Gly Thr Glu Cys Tyr Ile Thr Ser Asp Met Phe Tyr Val Glu
85 90 95
Val Gln Leu Asp Pro Ala Gly Gln Leu Cys Asp Val Lys Val Ala His
100 105 110
His Gly Glu Asn Pro Val Ser Cys Pro Glu Leu Val Gln Gln Leu Arg
115 120 125
Glu Lys Asn Ser Asp Glu Phe Ser Lys His Leu Lys Gly Leu Val Asn
130 135 140
Leu Tyr Asn Leu Pro Gly Asp Asn Lys Leu Lys Thr Lys Met Tyr Leu
145 150 155 160
Ala Leu Gln Ser Leu Glu Gln Asp Leu Ser Lys Met Ala Ile Met Tyr
165 170 175
Trp Lys Ala Thr Asn Ala Gly Pro Leu Asp Lys Ile Leu His Gly Ser
180 185 190
Val Gly Tyr Leu Thr Pro Arg Ser Gly Gly His Leu Met Asn Leu Lys
195 200 205
Tyr Tyr Val Ser Pro Ser Asp Leu Leu Asp Asp Lys Thr Ala Ser Pro
210 215 220
Ile Ile Leu His Glu Asn Asn Val Ser Arg Ser Leu Gly Met Asn Ala
225 230 235 240
Ser Val Thr Ile Glu Gly Thr Ser Ala Val Tyr Lys Leu Pro Ile Ala
245 250 255
Pro Leu Ile Met Gly Ser His Pro Val Asp Asn Lys Trp Thr Pro Ser
260 265 270
Phe Ser Ser Ile Thr Ser Ala Asn Ser Val Asp Leu Pro Ala Cys Phe
275 280 285
Phe Leu Lys Phe Pro Gln Pro Ile Pro Val Ser Arg Ala Phe Val Gln
290 295 300

Lys Leu Gln Asn Cys Thr Gly Ile Pro Leu Phe Glu Thr Gln Pro Thr
305 310 315 320
Tyr Ala Pro Leu Tyr Glu Leu Ile Thr Gln Phe Glu Leu Ser Lys Asp
325 330 335
Pro Asp Pro Ile Pro Leu Asn His Asn Met Arg Phe Tyr Ala Ala Leu
340 345 350
Pro Gly Gln Gln His Cys Tyr Phe Leu Asn Lys Asp Ala Pro Leu Pro
355 360 365
Asp Gly Arg Ser Leu Gln Gly Thr Leu Val Ser Lys Ile Thr Phe Gln
370 375 380
His Pro Gly Arg Val Pro Leu Ile Leu Asn Leu Ile Arg His Gln Val
385 390 395 400
Ala Tyr Asn Thr Leu Ile Gly Ser Cys Val Lys Arg Thr Ile Leu Lys
405 410 415

Glu Asp Ser Pro Gly Leu Leu Gln Phe Glu Val Cys Pro Leu Ser Glu
420 425 430
Ser Arg Phe Ser Val Ser Phe Gln His Pro Val Asn Asp Ser Leu Val
435 440 445
Cys Val Val Met Asp Val Gln Gly Leu Thr His Val Ser Cys Lys Leu
450 455 460
Tyr Lys Gly Leu Ser Asp Ala Leu Ile Cys Thr Asp Asp Phe Ile Ala
465 470 475 480
Lys Val Val Gln Arg Cys Met Ser Ile Pro Val Thr Met Arg Ala Ile
485 490 495
Arg Arg Lys Ala Glu Thr Ile Gln Ala Asp Thr Pro Ala Leu Ser Leu
500 505 510
Ile Ala Glu Thr Val Glu Asp Met Val Lys Lys Asn Leu Pro Pro Ala
515 520 525
Ser Ser Pro Gly Tyr Gly Met Thr Thr Gly Asn Asn Pro Met Ser Gly
530 535 540
Thr Thr Thr Ser Thr Asn Thr Phe Pro Gly Gly Pro Ile Ala Thr Leu
545 550 555 560
Phe Asn Met Ser Met Ser Ile Lys Asp Arg His Glu Ser Val Gly His
565 570 575
Gly Glu Asp Phe Ser Lys Val Ser Gln Asn Pro Ile Leu Thr Ser Leu
580 585 590
Leu Gln Ile Thr Gly Asn Gly Gly Ser Thr Ile Gly Ser Ser Pro Thr
595 600 605
Pro Pro His His Thr Pro Pro Pro Val Ser Ser Met Ala Gly Asn Thr
610 615 620
Lys Asn His Pro Met Leu Met Asn Leu Leu Lys Asp Asn Pro Ala Gln
625 630 635 640
Asp Phe Ser Thr Leu Tyr Gly Ser Ser Pro Leu Glu Arg Gln Asn Ser
645 650 655
Ser Ser Gly Ser Pro Arg Met Glu Ile Cys Ser Gly Ser Asn Lys Thr
660 665 670
Lys Lys Lys Lys Ser Ser Arg Leu Pro Pro Glu Lys Pro Lys His Gln
675 680 685
Thr Glu Asp Asp Phe Gln Arg Glu Leu Phe Ser Met Asp Val Asp Ser
690 695 700
Gln Asn Pro Ile Phe Asp Val Asn Met Thr Ala Asp Thr Leu Asp Thr
705 710 715 720
Pro His Ile Thr Pro Ala Pro Ser Gln Cys Ser Thr Pro Pro Thr Thr
725 730 735
Tyr Pro Gln Pro Val Pro His Pro Gln Pro Ser Ile Gln Arg Met Val
740 745 750
Arg Leu Ser Ser Ser Asp Ser Ile Gly Pro Asp Val Thr Asp Ile Leu
755 760 765

Ser Asp Ile Ala Glu Glu Ala Ser Lys Leu Pro Ser Thr Ser Asp Asp
770 775 780
Cys Pro Ala Ile Gly Thr Pro Leu Arg Asp Ser Ser Ser Ser Gly His
785 790 795 800
Ser Gln Ser Thr Leu Phe Asp Ser Asp Val Phe Gln Thr Asn Asn Asn
805 810 815
Glu Asn Pro Tyr Thr Asp Pro Ala Asp Leu Ile Ala Asp Ala Ala Gly
820 825 830
Ser Pro Ser Ser Asp Ser Pro Thr Asn His Phe Phe His Asp Gly Val
835 840 845
Asp Phe Asn Pro Asp Leu Leu Asn Ser Gln Ser Gln Ser Gly Phe Gly
850 855 860
Glu Glu Tyr Phe Asp Glu Ser Ser Gln Ser Gly Asp Asn Asp Asp Phe
865 870 875 880
Lys Gly Phe Ala Ser Gln Ala Leu Asn Thr Leu Gly Val Pro Met Leu
885 890 895
Gly Gly Asp Asn Gly Glu Thr Lys Phe Lys Gly Asn Asn Gln Ala Asp
900 905 910
Thr Val Asp Phe Ser Ile Ile Ser Val Ala Gly Lys Ala Leu Ala Pro
915 920 925

Ala Asp Leu Met Glu His His Ser Gly Ser Gln Gly Pro Leu Leu Thr
930 935 940
Thr Gly Asp Leu Gly Lys Glu Lys Thr Gln Lys Arg Val Lys Glu Gly
945 950 955 960
Asn Gly Thr Ser Asn Ser Thr Leu Ser Gly Pro Gly Leu Asp Ser Lys
965 970 975
Pro Gly Lys Arg Ser Arg Thr Pro Ser Asn Asp Gly Lys Ser Lys Asp
980 985 990
Lys Pro Pro Lys Arg Lys Lys Ala Asp Thr Glu Gly Lys Ser Pro Ser
995 1000 1005
His Ser Ser Ser Asn Arg Pro Phe Thr Pro Pro Thr Ser Thr Gly
1010 1015 1020
Gly Ser Lys Ser Pro Gly Ser Ala Gly Arg Ser Gln Thr Pro Pro
1025 1030 1035
Gly Val Ala Thr Pro Pro Ile Pro Lys Ile Thr Ile Gln Ile Pro
1040 1045 1050
Lys Gly Thr Val Met Val Gly Lys Pro Ser Ser His Ser Gln Tyr
1055 1060 1065
Thr Ser Ser Gly Ser Val Ser Ser Ser Gly Ser Lys Ser His His
1070 1075 1080
Ser His Ser Ser Ser Ser Ser Ser Ser Ala Ser Thr Ser Gly Lys
1085 1090 1095
Met Lys Ser Ser Lys Ser Glu Gly Ser Ser Ser Ser Lys Leu Ser
1100 1105 1110
Ser Ser Met Tyr Ser Ser Gln Gly Ser Ser Gly Ser Ser Gln Ser
1115 1120 1125
Lys Asn Ser Ser Gln Ser Gly Gly Lys Pro Gly Ser Ser Pro Ile
1130 1135 1140
Thr Lys His Gly Leu Ser Ser Gly Ser Ser Ser Thr Lys Met Lys
1145 1150 1155
Pro Gln Gly Lys Pro Ser Ser Leu Met Asn Pro Ser Leu Ser Lys
1160 1165 1170
Pro Asn Ile Ser Pro Ser His Ser Arg Pro Pro Gly Gly Ser Asp
1175 1180 1185
Lys Leu Ala Ser Pro Met Lys Pro Val Pro Gly Thr Pro Pro Ser
1190 1195 1200
Ser Lys Ala Lys Ser Pro Ile Ser Ser Gly Ser Gly Gly Ser His
1205 1210 1215

Met Ser Gly Thr Ser Ser Ser Ser Gly Met Lys Ser Ser Ser Gly
1220 1225 1230
Leu Gly Ser Ser Gly Ser Leu Ser Gln Lys Thr Pro Pro Ser Ser
1235 1240 1245
Asn Ser Cys Thr Ala Ser Ser Ser Ser Phe Ser Ser Ser Gly Ser
1250 1255 1260
Ser Met Ser Ser Ser Gln Asn Gln His Gly Ser Ser Lys Gly Lys
1265 1270 1275
Ser Pro Ser Arg Asn Lys Lys Pro Ser Leu Thr Ala Val Ile Asp
1280 1285 1290
Lys Leu Lys His Gly Val Val Thr Ser Gly Pro Gly Gly Glu Asp
1295 1300 1305
Pro Leu Asp Gly Gln Met Gly Val Ser Thr Asn Ser Ser Ser His
1310 1315 1320
Pro Met Ser Ser Lys His Asn Met Ser Gly Gly Glu Phe Gln Gly
1325 1330 1335
Lys Arg Glu Lys Ser Asp Lys Asp Lys Ser Lys Val Ser Thr Ser
1340 1345 1350
Gly Ser Ser Val Asp Ser Ser Lys Lys Thr Ser Glu Ser Lys Asn
1355 1360 1365
Val Gly Ser Thr Gly Val Ala Lys Ile Ile Ile Ser Lys His Asp
1370 1375 1380
Gly Gly Ser Pro Ser Ile Lys Ala Lys Val Thr Leu Gln Lys Pro
1385 1390 1395
Gly Glu Ser Ser Gly Glu Gly Leu Arg Pro Gln Met Ala Ser Ser
1400 1405 1410
Lys Asn Tyr Gly Ser Pro Leu Ile Ser Gly Ser Thr Pro Lys His
1415 1420 1425
Glu Arg Gly Ser Pro Ser His Ser Lys Ser Pro Ala Tyr Thr Pro
1430 1435 1440
Gln Asn Leu Asp Ser Glu Ser Glu Ser Gly Ser Ser Ile Ala Glu
1445 1450 1455
Lys Ser Tyr Gln Asn Ser Pro Ser Ser Asp Asp Gly Ile Arg Pro
1460 1465 1470
Leu Pro Glu Tyr Ser Thr Glu Lys His Lys Lys His Lys Lys Glu
1475 1480 1485
Lys Lys Lys Val Lys Asp Lys Asp Arg Asp Arg Asp Arg Asp Lys
1490 1495 1500
Asp Arg Asp Lys Lys Lys Ser His Ser Ile Lys Pro Glu Ser Trp
1505 1510 1515
Ser Lys Ser Pro Ile Ser Ser Asp Gln Ser Leu Ser Met Thr Ser
1520 1525 1530
Asn Thr Ile Leu Ser Ala Asp Arg Pro Ser Arg Leu Ser Pro Asp
1535 1540 1545
Phe Met Ile Gly Glu Glu Asp Asp Asp Leu Met Asp Val Ala Leu
1550 1555 1560
Ile Gly Asn
1565

<210> 31

<211> 1490

<212> PRT

<213> Homo sapiens

<400> 31

```
Met Pro Asn Ser Glu Arg His Gly Gly Lys Lys Asp Gly Ser Gly Gly
1               5                   10                  15
Ala Ser Gly Thr Leu Gln Pro Ser Ser Gly Gly Gly Ser Ser Asn Ser
            20                  25                  30
Arg Glu Arg His Arg Leu Val Ser Lys His Lys Arg His Lys Ser Lys
        35                  40                  45
His Ser Lys Asp Met Gly Leu Val Thr Pro Glu Ala Ala Ser Leu Gly
    50                  55                  60
Thr Val Ile Lys Pro Leu Val Glu Tyr Asp Asp Ile Ser Ser Asp Ser
65                  70                  75                  80
Asp Thr Phe Ser Asp Asp Met Ala Phe Lys Leu Asp Arg Arg Glu Asn
                85                  90                  95
Asp Glu Arg Arg Gly Ser Asp Arg Ser Asp Arg Leu His Lys His Arg
            100                 105                 110
His His Gln His Arg Arg Ser Arg Asp Leu Leu Lys Ala Lys Gln Thr
        115                 120                 125
Glu Lys Glu Lys Ser Gln Glu Val Ser Ser Lys Ser Gly Ser Met Lys
    130                 135                 140
Asp Arg Ile Ser Gly Ser Ser Lys Arg Ser Asn Glu Glu Thr Asp Asp
145                 150                 155                 160
Tyr Gly Lys Ala Gln Val Ala Lys Ser Ser Ser Lys Glu Ser Arg Ser
                165                 170                 175
Ser Lys Leu His Lys Glu Lys Thr Arg Lys Glu Arg Glu Leu Lys Ser
            180                 185                 190
Gly His Lys Asp Arg Ser Lys Ser His Arg Lys Arg Glu Thr Pro Lys
        195                 200                 205
Ser Tyr Lys Thr Val Asp Ser Pro Lys Arg Arg Ser Arg Ser Pro His
    210                 215                 220
Arg Lys Trp Ser Asp Ser Ser Lys Gln Asp Asp Ser Pro Ser Gly Ala
225                 230                 235                 240
Ser Tyr Gly Gln Asp Tyr Asp Leu Ser Pro Ser Arg Ser His Thr Ser
                245                 250                 255
Ser Asn Tyr Asp Ser Tyr Lys Lys Ser Pro Gly Ser Thr Ser Arg Arg
            260                 265                 270
Gln Ser Val Ser Pro Pro Tyr Lys Glu Pro Ser Ala Tyr Gln Ser Ser
        275                 280                 285
Thr Arg Ser Pro Ser Pro Tyr Ser Arg Arg Gln Arg Ser Val Ser Pro
    290                 295                 300
Tyr Ser Arg Arg Arg Ser Ser Ser Tyr Glu Arg Ser Gly Ser Tyr Ser
305                 310                 315                 320
Gly Arg Ser Pro Ser Pro Tyr Gly Arg Arg Arg Ser Ser Ser Pro Phe
                325                 330                 335
Leu Ser Lys Arg Ser Leu Ser Arg Ser Pro Leu Pro Ser Arg Lys Ser
            340                 345                 350
Met Lys Ser Arg Ser Arg Ser Pro Ala Tyr Ser Arg His Ser Ser Ser
        355                 360                 365
His Ser Lys Lys Lys Arg Ser Ser Ser Arg Ser Arg His Ser Ser Ile
    370                 375                 380
Ser Pro Val Arg Leu Pro Leu Asn Ser Ser Leu Gly Ala Glu Leu Ser
385                 390                 395                 400
Arg Lys Lys Lys Glu Arg Ala Ala Ala Ala Ala Ala Ala Lys Met Asp
                405                 410                 415
Gly Lys Glu Ser Lys Gly Ser Pro Val Phe Leu Pro Arg Lys Glu Asn
            420                 425                 430
Ser Ser Val Glu Ala Lys Asp Ser Gly Leu Glu Ser Lys Lys Leu Pro
        435                 440                 445
Arg Ser Val Lys Leu Glu Lys Ser Ala Pro Asp Thr Glu Leu Val Asn
    450                 455                 460
```

Val Thr His Leu Asn Thr Glu Val Lys Asn Ser Ser Asp Thr Gly Lys
465 470 475 480
Val Lys Leu Asp Glu Asn Ser Glu Lys His Leu Val Lys Asp Leu Lys
485 490 495
Ala Gln Gly Thr Arg Asp Ser Lys Pro Ile Ala Leu Lys Glu Glu Ile
500 505 510
Val Thr Pro Lys Glu Thr Glu Thr Ser Glu Lys Glu Thr Pro Pro Pro
515 520 525
Leu Pro Thr Ile Ala Ser Pro Pro Pro Pro Leu Pro Thr Thr Thr Pro
530 535 540
Pro Pro Gln Thr Pro Pro Leu Pro Pro Leu Pro Pro Ile Pro Ala Leu
545 550 555 560
Pro Gln Gln Pro Pro Leu Pro Pro Ser Gln Pro Ala Phe Ser Gln Val
565 570 575
Pro Ala Ser Ser Thr Ser Thr Leu Pro Pro Ser Thr His Ser Lys Thr
580 585 590
Ser Ala Val Ser Ser Gln Ala Asn Ser Gln Pro Pro Val Gln Val Ser
595 600 605
Val Lys Thr Gln Val Ser Val Thr Ala Ala Ile Pro His Leu Lys Thr
610 615 620
Ser Thr Leu Pro Pro Leu Pro Leu Pro Pro Leu Leu Pro Gly Gly Asp
625 630 635 640
Asp Met Asp Ser Pro Lys Glu Thr Leu Pro Ser Lys Pro Val Lys Lys
645 650 655
Glu Lys Glu Gln Arg Thr Arg His Leu Leu Thr Asp Leu Pro Leu Pro
660 665 670
Pro Glu Leu Pro Gly Gly Asp Leu Ser Pro Pro Asp Ser Pro Glu Pro
675 680 685
Lys Ala Ile Thr Pro Pro Gln Gln Pro Tyr Lys Lys Arg Pro Lys Ile
690 695 700
Cys Cys Pro Arg Tyr Gly Glu Arg Arg Gln Thr Glu Ser Asp Trp Gly
705 710 715 720
Lys Arg Cys Val Asp Lys Phe Asp Ile Ile Gly Ile Ile Gly Glu Gly
725 730 735
Thr Tyr Gly Gln Val Tyr Lys Ala Arg Asp Lys Asp Thr Gly Glu Leu
740 745 750
Val Ala Leu Lys Lys Val Arg Leu Asp Asn Glu Lys Glu Gly Phe Pro
755 760 765
Ile Thr Ala Ile Arg Glu Ile Lys Ile Leu Arg Gln Leu Ile His Arg
770 775 780
Ser Val Val Asn Met Lys Glu Ile Val Thr Asp Lys Gln Asp Ala Leu
785 790 795 800
Asp Phe Lys Lys Asp Lys Gly Ala Phe Tyr Leu Val Phe Glu Tyr Met
805 810 815
Asp His Asp Leu Met Gly Leu Leu Glu Ser Gly Leu Val His Phe Ser
820 825 830
Glu Asp His Ile Lys Ser Phe Met Lys Gln Leu Met Glu Gly Leu Glu
835 840 845
Tyr Cys His Lys Lys Asn Phe Leu His Arg Asp Ile Lys Cys Ser Asn
850 855 860
Ile Leu Leu Asn Asn Ser Gly Gln Ile Lys Leu Ala Asp Phe Gly Leu
865 870 875 880
Ala Arg Leu Tyr Asn Ser Glu Glu Ser Arg Pro Tyr Thr Asn Lys Val
885 890 895
Ile Thr Leu Trp Tyr Arg Pro Pro Glu Leu Leu Leu Gly Glu Glu Arg
900 905 910
Tyr Thr Pro Ala Ile Asp Val Trp Ser Cys Gly Cys Ile Leu Gly Glu
915 920 925

Leu Phe Thr Lys Lys Pro Ile Phe Gln Ala Asn Leu Glu Leu Ala Gln
930 935 940
Leu Glu Leu Ile Ser Arg Leu Cys Gly Ser Pro Cys Pro Ala Val Trp
945 950 955 960
Pro Asp Val Ile Lys Leu Pro Tyr Phe Asn Thr Met Lys Pro Lys Lys
965 970 975
Gln Tyr Arg Arg Arg Leu Arg Glu Glu Phe Ser Phe Ile Pro Ser Ala
980 985 990
Ala Leu Asp Leu Leu Asp His Met Leu Thr Leu Asp Pro Ser Lys Arg
995 1000 1005
Cys Thr Ala Glu Gln Thr Leu Gln Ser Asp Phe Leu Lys Asp Val
1010 1015 1020
Glu Leu Ser Lys Met Ala Pro Pro Asp Leu Pro His Trp Gln Asp
1025 1030 1035
Cys His Glu Leu Trp Ser Lys Lys Arg Arg Arg Gln Arg Gln Ser
1040 1045 1050
Gly Val Val Val Glu Glu Pro Pro Pro Ser Lys Thr Ser Arg Lys
1055 1060 1065
Glu Thr Thr Ser Gly Thr Ser Thr Glu Pro Val Lys Asn Ser Ser
1070 1075 1080
Pro Ala Pro Pro Gln Pro Ala Pro Gly Lys Val Glu Ser Gly Ala
1085 1090 1095
Gly Asp Ala Ile Gly Leu Ala Asp Ile Thr Gln Gln Leu Asn Gln
1100 1105 1110
Ser Glu Leu Ala Val Leu Leu Asn Leu Leu Gln Ser Gln Thr Asp
1115 1120 1125
Leu Ser Ile Pro Gln Met Ala Gln Leu Leu Asn Ile His Ser Asn
1130 1135 1140
Pro Glu Met Gln Gln Gln Leu Glu Ala Leu Asn Gln Ser Ile Ser
1145 1150 1155
Ala Leu Thr Glu Ala Thr Ser Gln Gln Gln Asp Ser Glu Thr Met
1160 1165 1170
Ala Pro Glu Glu Ser Leu Lys Glu Ala Pro Ser Ala Pro Val Ile
1175 1180 1185
Leu Pro Ser Ala Glu Gln Met Thr Leu Glu Ala Ser Ser Thr Pro
1190 1195 1200
Ala Asp Met Gln Asn Ile Leu Ala Val Leu Leu Ser Gln Leu Met
1205 1210 1215
Lys Thr Gln Glu Pro Ala Gly Ser Leu Glu Glu Asn Asn Ser Asp
1220 1225 1230
Lys Asn Ser Gly Pro Gln Gly Pro Arg Arg Thr Pro Thr Met Pro
1235 1240 1245
Gln Glu Glu Ala Ala Ala Cys Pro Pro His Ile Leu Pro Pro Glu
1250 1255 1260
Lys Arg Pro Pro Glu Pro Pro Gly Pro Pro Pro Pro Pro Pro Pro
1265 1270 1275
Pro Pro Leu Val Glu Gly Asp Leu Ser Ser Ala Pro Gln Glu Leu
1280 1285 1290
Asn Pro Ala Val Thr Ala Ala Leu Leu Gln Leu Leu Ser Gln Pro
1295 1300 1305
Glu Ala Glu Pro Pro Gly His Leu Pro His Glu His Gln Ala Leu
1310 1315 1320
Arg Pro Met Glu Tyr Ser Thr Arg Pro Arg Pro Asn Arg Thr Tyr
1325 1330 1335
Gly Asn Thr Asp Gly Pro Glu Thr Gly Phe Ser Ala Ile Asp Thr
1340 1345 1350
Asp Glu Arg Asn Ser Gly Pro Ala Leu Thr Glu Ser Leu Val Gln
1355 1360 1365

Thr Leu Val Lys Asn Arg Thr Phe Ser Gly Ser Leu Ser His Leu
1370 1375 1380
Gly Glu Ser Ser Ser Tyr Gln Gly Thr Gly Ser Val Gln Phe Pro
1385 1390 1395
Gly Asp Gln Asp Leu Arg Phe Ala Arg Val Pro Leu Ala Leu His
1400 1405 1410
Pro Val Val Gly Gln Pro Phe Leu Lys Ala Glu Gly Ser Ser Asn
1415 1420 1425
Ser Val Val His Ala Glu Thr Lys Leu Gln Asn Tyr Gly Glu Leu
1430 1435 1440
Gly Pro Gly Thr Thr Gly Ala Ser Ser Ser Gly Ala Gly Leu His
1445 1450 1455
Trp Gly Gly Pro Thr Gln Ser Ser Ala Tyr Gly Lys Leu Tyr Arg
1460 1465 1470
Gly Pro Thr Arg Val Pro Pro Arg Gly Gly Arg Gly Arg Gly Val
1475 1480 1485
Pro Tyr
1490

<210> 32

<211> 381

<212> PRT

<213> Homo sapiens

<400> 32
Met Leu Thr Arg Leu Phe Ser Glu Pro Gly Leu Leu Ser Asp Val Pro
1 5 10 15
Lys Phe Ala Ser Trp Gly Asp Gly Glu Asp Asp Glu Pro Arg Ser Asp
20 25 30
Lys Gly Asp Ala Pro Pro Pro Pro Pro Pro Ala Pro Gly Pro Gly Ala
35 40 45
Pro Gly Pro Ala Arg Ala Ala Lys Pro Val Pro Leu Arg Gly Glu Glu
50 55 60
Gly Thr Glu Ala Thr Leu Ala Glu Val Lys Glu Glu Gly Glu Leu Gly
65 70 75 80
Gly Glu Glu Glu Glu Glu Glu Glu Glu Glu Glu Gly Leu Asp Glu Ala
85 90 95
Glu Gly Glu Arg Pro Lys Lys Arg Gly Pro Lys Lys Arg Lys Met Thr
100 105 110
Lys Ala Arg Leu Glu Arg Ser Lys Leu Arg Arg Gln Lys Ala Asn Ala
115 120 125
Arg Glu Arg Asn Arg Met His Asp Leu Asn Ala Ala Leu Asp Asn Leu
130 135 140
Arg Lys Val Val Pro Cys Tyr Ser Lys Thr Gln Lys Leu Ser Lys Ile
145 150 155 160
Glu Thr Leu Arg Leu Ala Lys Asn Tyr Ile Trp Ala Leu Ser Glu Ile
165 170 175
Leu Arg Ser Gly Lys Arg Pro Asp Leu Val Ser Tyr Val Gln Thr Leu
180 185 190
Cys Lys Gly Leu Ser Gln Pro Thr Thr Asn Leu Val Ala Gly Cys Leu
195 200 205
Gln Leu Asn Ser Arg Asn Phe Leu Thr Glu Gln Gly Ala Asp Gly Ala
210 215 220

Gly Arg Phe His Gly Ser Gly Gly Pro Phe Ala Met His Pro Tyr Pro
225 230 235 240
Tyr Pro Cys Ser Arg Leu Ala Gly Ala Gln Cys Gln Ala Ala Gly Gly
245 250 255
Leu Gly Gly Gly Ala Ala His Ala Leu Arg Thr His Gly Tyr Cys Ala
260 265 270
Ala Tyr Glu Thr Leu Tyr Ala Ala Ala Gly Gly Gly Gly Ala Ser Pro
275 280 285
Asp Tyr Asn Ser Ser Glu Tyr Glu Gly Pro Leu Ser Pro Pro Leu Cys
290 295 300
Leu Asn Gly Asn Phe Ser Leu Lys Gln Asp Ser Ser Pro Asp His Glu
305 310 315 320
Lys Ser Tyr His Tyr Ser Met His Tyr Ser Ala Leu Pro Gly Ser Arg
325 330 335
His Gly His Gly Leu Val Phe Gly Ser Ser Ala Val Arg Gly Gly Val
340 345 350
His Ser Glu Asn Leu Leu Ser Tyr Asp Met His Leu His His Asp Arg
355 360 365
Gly Pro Met Tyr Glu Glu Leu Asn Ala Phe Phe His Asn
370 375 380

<210> 33

<211> 445

<212> PRT

<213> Homo sapiens

<400> 33
Met Ser Lys Leu Pro Arg Glu Leu Thr Arg Asp Leu Glu Arg Ser Leu
1 5 10 15
Pro Ala Val Ala Ser Leu Gly Ser Ser Leu Ser His Ser Gln Ser Leu
20 25 30
Ser Ser His Leu Leu Pro Pro Pro Glu Lys Arg Arg Ala Ile Ser Asp
35 40 45
Val Arg Arg Thr Phe Cys Leu Phe Val Thr Phe Asp Leu Leu Phe Ile
50 55 60
Ser Leu Leu Trp Ile Ile Glu Leu Asn Thr Asn Thr Gly Ile Arg Lys
65 70 75 80
Asn Leu Glu Gln Glu Ile Ile Gln Tyr Asn Phe Lys Thr Ser Phe Phe
85 90 95
Asp Ile Phe Val Leu Ala Phe Phe Arg Phe Ser Gly Leu Leu Leu Gly
100 105 110
Tyr Ala Val Leu Gln Leu Arg His Trp Trp Val Ile Ala Val Thr Thr
115 120 125
Leu Val Ser Ser Ala Phe Leu Ile Val Lys Val Ile Leu Ser Glu Leu
130 135 140
Leu Ser Lys Gly Ala Phe Gly Tyr Leu Leu Pro Ile Val Ser Phe Val
145 150 155 160
Leu Ala Trp Leu Glu Thr Trp Phe Leu Asp Phe Lys Val Leu Pro Gln
165 170 175
Glu Ala Glu Glu Glu Arg Trp Tyr Leu Ala Ala Gln Val Ala Val Ala
180 185 190
Arg Gly Pro Leu Leu Phe Ser Gly Ala Leu Ser Glu Gly Gln Phe Tyr
195 200 205

Ser Pro Pro Glu Ser Phe Ala Gly Ser Asp Asn Glu Ser Asp Glu Glu
210 215 220
Val Ala Gly Lys Lys Ser Phe Ser Ala Gln Glu Arg Glu Tyr Ile Arg
225 230 235 240
Gln Gly Lys Glu Ala Thr Ala Val Val Asp Gln Ile Leu Ala Gln Glu
245 250 255
Glu Asn Trp Lys Phe Glu Lys Asn Asn Glu Tyr Gly Asp Thr Val Tyr
260 265 270
Thr Ile Glu Val Pro Phe His Gly Lys Thr Phe Ile Leu Lys Thr Phe
275 280 285
Leu Pro Cys Pro Ala Glu Leu Val Tyr Gln Glu Val Ile Leu Gln Pro
290 295 300
Glu Arg Met Val Leu Trp Asn Lys Thr Val Thr Ala Cys Gln Ile Leu
305 310 315 320
Gln Arg Val Glu Asp Asn Thr Leu Ile Ser Tyr Asp Val Ser Ala Gly
325 330 335
Ala Ala Gly Gly Val Val Ser Pro Arg Asp Phe Val Asn Val Arg Arg
340 345 350
Ile Glu Arg Arg Arg Asp Arg Tyr Leu Ser Ser Gly Ile Ala Thr Ser
355 360 365
His Ser Ala Lys Pro Pro Thr His Lys Tyr Val Arg Gly Glu Asn Gly
370 375 380
Pro Gly Gly Phe Ile Val Leu Lys Ser Ala Ser Asn Pro Arg Val Cys
385 390 395 400
Thr Phe Val Trp Ile Leu Asn Thr Asp Leu Lys Gly Arg Leu Pro Arg
405 410 415
Tyr Leu Ile His Gln Ser Leu Ala Ala Thr Met Phe Glu Phe Ala Phe
420 425 430
His Leu Arg Gln Arg Ile Ser Glu Leu Gly Ala Arg Ala
435 440 445

<210> 34

<211> 167

<212> PRT

<213> Homo sapiens

<400> 34
Met Ala Thr Ser Glu Leu Ser Cys Glu Val Ser Glu Glu Asn Cys Glu
1 5 10 15
Arg Arg Glu Ala Phe Trp Ala Glu Trp Lys Asp Leu Thr Leu Ser Thr
20 25 30
Arg Pro Glu Glu Gly Cys Ser Leu His Glu Glu Asp Thr Gln Arg His
35 40 45
Glu Thr Tyr His Gln Gln Gly Gln Cys Gln Val Leu Val Gln Arg Ser
50 55 60
Pro Trp Leu Met Met Arg Met Gly Ile Leu Gly Arg Gly Leu Gln Glu
65 70 75 80
Tyr Gln Leu Pro Tyr Gln Arg Val Leu Pro Leu Pro Ile Phe Thr Pro
85 90 95
Ala Lys Met Gly Ala Thr Lys Glu Glu Arg Glu Asp Thr Pro Ile Gln
100 105 110
Leu Gln Glu Leu Leu Ala Leu Glu Thr Ala Leu Gly Gly Gln Cys Val
115 120 125

Asp Arg Gln Glu Val Ala Glu Ile Thr Lys Gln Leu Pro Pro Val Val
    130                 135                 140
Pro Val Ser Lys Pro Gly Ala Leu Arg Arg Ser Leu Ser Arg Ser Met
145                 150                 155                 160
Ser Gln Glu Ala Gln Arg Gly
                165

<210> 35

<211> 282

<212> PRT

<213> Homo sapiens

<400> 35
Met Ser Gly Ala Asp Arg Ser Pro Asn Ala Gly Ala Ala Pro Asp Ser
1               5                   10                  15
Ala Pro Gly Gln Ala Ala Val Ala Ser Ala Tyr Gln Arg Phe Glu Pro
            20                  25                  30
Arg Ala Tyr Leu Arg Asn Asn Tyr Ala Pro Pro Arg Gly Asp Leu Cys
        35                  40                  45
Asn Pro Asn Gly Val Gly Pro Trp Lys Leu Arg Cys Leu Ala Gln Thr
    50                  55                  60
Phe Ala Thr Gly Glu Val Ser Gly Arg Thr Leu Ile Asp Ile Gly Ser
65                  70                  75                  80
Gly Pro Thr Val Tyr Gln Leu Leu Ser Ala Cys Ser His Phe Glu Asp
                85                  90                  95
Ile Thr Met Thr Asp Phe Leu Glu Val Asn Arg Gln Glu Leu Gly Arg
            100                 105                 110
Trp Leu Gln Glu Glu Pro Gly Ala Phe Asn Trp Ser Met Tyr Ser Gln
        115                 120                 125
His Ala Cys Leu Ile Glu Gly Lys Gly Glu Cys Trp Gln Asp Lys Glu
    130                 135                 140
Arg Gln Leu Arg Ala Arg Val Lys Arg Val Leu Pro Ile Asp Val His
145                 150                 155                 160
Gln Pro Gln Pro Leu Gly Ala Gly Ser Pro Ala Pro Leu Pro Ala Asp
                165                 170                 175
Ala Leu Val Ser Ala Phe Cys Leu Glu Ala Val Ser Pro Asp Leu Ala
            180                 185                 190
Ser Phe Gln Arg Ala Leu Asp His Ile Thr Thr Leu Leu Arg Pro Gly
        195                 200                 205
Gly His Leu Leu Leu Ile Gly Ala Leu Glu Glu Ser Trp Tyr Leu Ala
    210                 215                 220
Gly Glu Ala Arg Leu Thr Val Val Pro Val Ser Glu Glu Glu Val Arg
225                 230                 235                 240
Glu Ala Leu Val Arg Ser Gly Tyr Lys Val Arg Asp Leu Arg Thr Tyr
                245                 250                 255
Ile Met Pro Ala His Leu Gln Thr Gly Val Asp Asp Val Lys Gly Val
            260                 265                 270
Phe Phe Ala Trp Ala Gln Lys Val Gly Leu
        275                 280

<210> 36

<211> 1255

<212> PRT

<213> Homo sapiens

<400> 36
Met Glu Leu Ala Ala Leu Cys Arg Trp Gly Leu Leu Leu Ala Leu Leu
1 5 10 15
Pro Pro Gly Ala Ala Ser Thr Gln Val Cys Thr Gly Thr Asp Met Lys
20 25 30
Leu Arg Leu Pro Ala Ser Pro Glu Thr His Leu Asp Met Leu Arg His
35 40 45
Leu Tyr Gln Gly Cys Gln Val Val Gln Gly Asn Leu Glu Leu Thr Tyr
50 55 60
Leu Pro Thr Asn Ala Ser Leu Ser Phe Leu Gln Asp Ile Gln Glu Val
65 70 75 80
Gln Gly Tyr Val Leu Ile Ala His Asn Gln Val Arg Gln Val Pro Leu
85 90 95
Gln Arg Leu Arg Ile Val Arg Gly Thr Gln Leu Phe Glu Asp Asn Tyr
100 105 110
Ala Leu Ala Val Leu Asp Asn Gly Asp Pro Leu Asn Asn Thr Thr Pro
115 120 125
Val Thr Gly Ala Ser Pro Gly Gly Leu Arg Glu Leu Gln Leu Arg Ser
130 135 140
Leu Thr Glu Ile Leu Lys Gly Gly Val Leu Ile Gln Arg Asn Pro Gln
145 150 155 160
Leu Cys Tyr Gln Asp Thr Ile Leu Trp Lys Asp Ile Phe His Lys Asn
165 170 175
Asn Gln Leu Ala Leu Thr Leu Ile Asp Thr Asn Arg Ser Arg Ala Cys
180 185 190
His Pro Cys Ser Pro Met Cys Lys Gly Ser Arg Cys Trp Gly Glu Ser
195 200 205
Ser Glu Asp Cys Gln Ser Leu Thr Arg Thr Val Cys Ala Gly Gly Cys
210 215 220
Ala Arg Cys Lys Gly Pro Leu Pro Thr Asp Cys Cys His Glu Gln Cys
225 230 235 240
Ala Ala Gly Cys Thr Gly Pro Lys His Ser Asp Cys Leu Ala Cys Leu
245 250 255
His Phe Asn His Ser Gly Ile Cys Glu Leu His Cys Pro Ala Leu Val
260 265 270
Thr Tyr Asn Thr Asp Thr Phe Glu Ser Met Pro Asn Pro Glu Gly Arg
275 280 285
Tyr Thr Phe Gly Ala Ser Cys Val Thr Ala Cys Pro Tyr Asn Tyr Leu
290 295 300
Ser Thr Asp Val Gly Ser Cys Thr Leu Val Cys Pro Leu His Asn Gln
305 310 315 320
Glu Val Thr Ala Glu Asp Gly Thr Gln Arg Cys Glu Lys Cys Ser Lys
325 330 335
Pro Cys Ala Arg Val Cys Tyr Gly Leu Gly Met Glu His Leu Arg Glu
340 345 350
Val Arg Ala Val Thr Ser Ala Asn Ile Gln Glu Phe Ala Gly Cys Lys
355 360 365
Lys Ile Phe Gly Ser Leu Ala Phe Leu Pro Glu Ser Phe Asp Gly Asp
370 375 380

Pro Ala Ser Asn Thr Ala Pro Leu Gln Pro Glu Gln Leu Gln Val Phe
385 390 395 400
Glu Thr Leu Glu Glu Ile Thr Gly Tyr Leu Tyr Ile Ser Ala Trp Pro
405 410 415
Asp Ser Leu Pro Asp Leu Ser Val Phe Gln Asn Leu Gln Val Ile Arg
420 425 430

Gly Arg Ile Leu His Asn Gly Ala Tyr Ser Leu Thr Leu Gln Gly Leu
435 440 445
Gly Ile Ser Trp Leu Gly Leu Arg Ser Leu Arg Glu Leu Gly Ser Gly
450 455 460
Leu Ala Leu Ile His His Asn Thr His Leu Cys Phe Val His Thr Val
465 470 475 480
Pro Trp Asp Gln Leu Phe Arg Asn Pro His Gln Ala Leu Leu His Thr
485 490 495
Ala Asn Arg Pro Glu Asp Glu Cys Val Gly Glu Gly Leu Ala Cys His
500 505 510
Gln Leu Cys Ala Arg Gly His Cys Trp Gly Pro Gly Pro Thr Gln Cys
515 520 525
Val Asn Cys Ser Gln Phe Leu Arg Gly Gln Glu Cys Val Glu Glu Cys
530 535 540
Arg Val Leu Gln Gly Leu Pro Arg Glu Tyr Val Asn Ala Arg His Cys
545 550 555 560
Leu Pro Cys His Pro Glu Cys Gln Pro Gln Asn Gly Ser Val Thr Cys
565 570 575
Phe Gly Pro Glu Ala Asp Gln Cys Val Ala Cys Ala His Tyr Lys Asp
580 585 590
Pro Pro Phe Cys Val Ala Arg Cys Pro Ser Gly Val Lys Pro Asp Leu
595 600 605
Ser Tyr Met Pro Ile Trp Lys Phe Pro Asp Glu Glu Gly Ala Cys Gln
610 615 620
Pro Cys Pro Ile Asn Cys Thr His Ser Cys Val Asp Leu Asp Asp Lys
625 630 635 640
Gly Cys Pro Ala Glu Gln Arg Ala Ser Pro Leu Thr Ser Ile Val Ser
645 650 655
Ala Val Val Gly Ile Leu Leu Val Val Val Leu Gly Val Val Phe Gly
660 665 670
Ile Leu Ile Lys Arg Arg Gln Gln Lys Ile Arg Lys Tyr Thr Met Arg
675 680 685
Arg Leu Leu Gln Glu Thr Glu Leu Val Glu Pro Leu Thr Pro Ser Gly
690 695 700
Ala Met Pro Asn Gln Ala Gln Met Arg Ile Leu Lys Glu Thr Glu Leu
705 710 715 720
Arg Lys Val Lys Val Leu Gly Ser Gly Ala Phe Gly Thr Val Tyr Lys
725 730 735
Gly Ile Trp Ile Pro Asp Gly Glu Asn Val Lys Ile Pro Val Ala Ile
740 745 750
Lys Val Leu Arg Glu Asn Thr Ser Pro Lys Ala Asn Lys Glu Ile Leu
755 760 765
Asp Glu Ala Tyr Val Met Ala Gly Val Gly Ser Pro Tyr Val Ser Arg
770 775 780
Leu Leu Gly Ile Cys Leu Thr Ser Thr Val Gln Leu Val Thr Gln Leu
785 790 795 800
Met Pro Tyr Gly Cys Leu Leu Asp His Val Arg Glu Asn Arg Gly Arg
805 810 815
Leu Gly Ser Gln Asp Leu Leu Asn Trp Cys Met Gln Ile Ala Lys Gly
820 825 830
Met Ser Tyr Leu Glu Asp Val Arg Leu Val His Arg Asp Leu Ala Ala
835 840 845

Arg Asn Val Leu Val Lys Ser Pro Asn His Val Lys Ile Thr Asp Phe
850 855 860
Gly Leu Ala Arg Leu Leu Asp Ile Asp Glu Thr Glu Tyr His Ala Asp
865 870 875 880
Gly Gly Lys Val Pro Ile Lys Trp Met Ala Leu Glu Ser Ile Leu Arg
885 890 895
Arg Arg Phe Thr His Gln Ser Asp Val Trp Ser Tyr Gly Val Thr Val
900 905 910
Trp Glu Leu Met Thr Phe Gly Ala Lys Pro Tyr Asp Gly Ile Pro Ala
915 920 925
Arg Glu Ile Pro Asp Leu Leu Glu Lys Gly Glu Arg Leu Pro Gln Pro
930 935 940
Pro Ile Cys Thr Ile Asp Val Tyr Met Ile Met Val Lys Cys Trp Met
945 950 955 960
Ile Asp Ser Glu Cys Arg Pro Arg Phe Arg Glu Leu Val Ser Glu Phe
965 970 975
Ser Arg Met Ala Arg Asp Pro Gln Arg Phe Val Val Ile Gln Asn Glu
980 985 990
Asp Leu Gly Pro Ala Ser Pro Leu Asp Ser Thr Phe Tyr Arg Ser Leu
995 1000 1005
Leu Glu Asp Asp Asp Met Gly Asp Leu Val Asp Ala Glu Glu Tyr
1010 1015 1020
Leu Val Pro Gln Gln Gly Phe Phe Cys Pro Asp Pro Ala Pro Gly
1025 1030 1035
Ala Gly Gly Met Val His His Arg His Arg Ser Ser Ser Thr Arg
1040 1045 1050
Ser Gly Gly Gly Asp Leu Thr Leu Gly Leu Glu Pro Ser Glu Glu
1055 1060 1065
Glu Ala Pro Arg Ser Pro Leu Ala Pro Ser Glu Gly Ala Gly Ser
1070 1075 1080
Asp Val Phe Asp Gly Asp Leu Gly Met Gly Ala Ala Lys Gly Leu
1085 1090 1095
Gln Ser Leu Pro Thr His Asp Pro Ser Pro Leu Gln Arg Tyr Ser
1100 1105 1110
Glu Asp Pro Thr Val Pro Leu Pro Ser Glu Thr Asp Gly Tyr Val
1115 1120 1125
Ala Pro Leu Thr Cys Ser Pro Gln Pro Glu Tyr Val Asn Gln Pro
1130 1135 1140
Asp Val Arg Pro Gln Pro Pro Ser Pro Arg Glu Gly Pro Leu Pro
1145 1150 1155
Ala Ala Arg Pro Ala Gly Ala Thr Leu Glu Arg Ala Lys Thr Leu
1160 1165 1170
Ser Pro Gly Lys Asn Gly Val Val Lys Asp Val Phe Ala Phe Gly
1175 1180 1185
Gly Ala Val Glu Asn Pro Glu Tyr Leu Thr Pro Gln Gly Gly Ala
1190 1195 1200
Ala Pro Gln Pro His Pro Pro Pro Ala Phe Ser Pro Ala Phe Asp
1205 1210 1215
Asn Leu Tyr Tyr Trp Asp Gln Asp Pro Pro Glu Arg Gly Ala Pro
1220 1225 1230
Pro Ser Thr Phe Lys Gly Thr Pro Thr Ala Glu Asn Pro Glu Tyr
1235 1240 1245
Leu Gly Leu Asp Val Pro Val
1250 1255

<210> 37

<211> 532

<212> PRT

<213> Homo sapiens

<400> 37

Met Glu Leu Asp Leu Ser Pro Pro His Leu Ser Ser Ser Pro Glu Asp
1 5 10 15
Leu Trp Pro Ala Pro Gly Thr Pro Pro Gly Thr Pro Arg Pro Pro Asp
20 25 30
Thr Pro Leu Pro Glu Glu Val Lys Arg Ser Gln Pro Leu Leu Ile Pro
35 40 45
Thr Thr Gly Arg Lys Leu Arg Glu Glu Glu Arg Arg Ala Thr Ser Leu
50 55 60
Pro Ser Ile Pro Asn Pro Phe Pro Glu Leu Cys Ser Pro Pro Ser Gln
65 70 75 80
Ser Pro Ile Leu Gly Gly Pro Ser Ser Ala Arg Gly Leu Leu Pro Arg
85 90 95
Asp Ala Ser Arg Pro His Val Val Lys Val Tyr Ser Glu Asp Gly Ala
100 105 110
Cys Arg Ser Val Glu Val Ala Ala Gly Ala Thr Ala Arg His Val Cys
115 120 125
Glu Met Leu Val Gln Arg Ala His Ala Leu Ser Asp Glu Thr Trp Gly
130 135 140
Leu Val Glu Cys His Pro His Leu Ala Leu Glu Arg Gly Leu Glu Asp
145 150 155 160
His Glu Ser Val Val Glu Val Gln Ala Ala Trp Pro Val Gly Gly Asp
165 170 175
Ser Arg Phe Val Phe Arg Lys Asn Phe Ala Lys Tyr Glu Leu Phe Lys
180 185 190
Ser Ser Pro His Ser Leu Phe Pro Glu Lys Met Val Ser Ser Cys Leu
195 200 205
Asp Ala His Thr Gly Ile Ser His Glu Asp Leu Ile Gln Asn Phe Leu
210 215 220
Asn Ala Gly Ser Phe Pro Glu Ile Gln Gly Phe Leu Gln Leu Arg Gly
225 230 235 240
Ser Gly Arg Lys Leu Trp Lys Arg Phe Phe Cys Phe Leu Arg Arg Ser
245 250 255
Gly Leu Tyr Tyr Ser Thr Lys Gly Thr Ser Lys Asp Pro Arg His Leu
260 265 270
Gln Tyr Val Ala Asp Val Asn Glu Ser Asn Val Tyr Val Val Thr Gln
275 280 285
Gly Arg Lys Leu Tyr Gly Met Pro Thr Asp Phe Gly Phe Cys Val Lys
290 295 300
Pro Asn Lys Leu Arg Asn Gly His Lys Gly Leu Arg Ile Phe Cys Ser
305 310 315 320
Glu Asp Glu Gln Ser Arg Thr Cys Trp Leu Ala Ala Phe Arg Leu Phe
325 330 335
Lys Tyr Gly Val Gln Leu Tyr Lys Asn Tyr Gln Gln Ala Gln Ser Arg
340 345 350
His Leu His Pro Ser Cys Leu Gly Ser Pro Pro Leu Arg Ser Ala Ser
355 360 365
Asp Asn Thr Leu Val Ala Met Asp Phe Ser Gly His Ala Gly Arg Val
370 375 380

Ile Glu Asn Pro Arg Glu Ala Leu Ser Val Ala Leu Glu Glu Ala Gln
385 390 395 400
Ala Trp Arg Lys Lys Thr Asn His Arg Leu Ser Leu Pro Met Pro Ala
405 410 415
Ser Gly Thr Ser Leu Ser Ala Ala Ile His Arg Thr Gln Leu Trp Phe
420 425 430
His Gly Arg Ile Ser Arg Glu Glu Ser Gln Arg Leu Ile Gly Gln Gln
435 440 445
Gly Leu Val Asp Gly Leu Phe Leu Val Arg Glu Ser Gln Arg Asn Pro
450 455 460
Gln Gly Phe Val Leu Ser Leu Cys His Leu Gln Lys Val Lys His Tyr
465 470 475 480
Leu Ile Leu Pro Ser Glu Glu Glu Gly Arg Leu Tyr Phe Ser Met Asp
485 490 495
Asp Gly Gln Thr Arg Phe Thr Asp Leu Leu Gln Leu Val Glu Phe His
500 505 510
Gln Leu Asn Arg Gly Ile Leu Pro Cys Leu Leu Arg His Cys Cys Thr
515 520 525
Arg Val Ala Leu
530

<210> 38

<211> 534

<212> PRT

<213> Homo sapiens

<400> 38
Met Lys Gln Glu Gly Ser Ala Arg Arg Arg Gly Ala Asp Lys Ala Lys
1 5 10 15
Pro Pro Pro Gly Gly Gly Glu Gln Glu Pro Pro Pro Pro Pro Ala Pro
20 25 30
Gln Asp Val Glu Met Lys Glu Glu Ala Ala Thr Gly Gly Gly Ser Thr
35 40 45
Gly Glu Ala Asp Gly Lys Thr Ala Ala Ala Ala Val Glu His Ser Gln
50 55 60
Arg Glu Leu Asp Thr Val Thr Leu Glu Asp Ile Lys Glu His Val Lys
65 70 75 80
Gln Leu Glu Lys Ala Val Ser Gly Lys Glu Pro Arg Phe Val Leu Arg
85 90 95
Ala Leu Arg Met Leu Pro Ser Thr Ser Arg Arg Leu Asn His Tyr Val
100 105 110
Leu Tyr Lys Ala Val Gln Gly Phe Phe Thr Ser Asn Asn Ala Thr Arg
115 120 125
Asp Phe Leu Leu Pro Phe Leu Glu Glu Pro Met Asp Thr Glu Ala Asp
130 135 140
Leu Gln Phe Arg Pro Arg Thr Gly Lys Ala Ala Ser Thr Pro Leu Leu
145 150 155 160
Pro Glu Val Glu Ala Tyr Leu Gln Leu Leu Val Val Ile Phe Met Met
165 170 175
Asn Ser Lys Arg Tyr Lys Glu Ala Gln Lys Ile Ser Asp Asp Leu Met
180 185 190
Gln Lys Ile Ser Thr Gln Asn Arg Arg Ala Leu Asp Leu Val Ala Ala
195 200 205

Lys Cys Tyr Tyr Tyr His Ala Arg Val Tyr Glu Phe Leu Asp Lys Leu
    210                 215                 220
Asp Val Val Arg Ser Phe Leu His Ala Arg Leu Arg Thr Ala Thr Leu
225                 230                 235                 240
Arg His Asp Ala Asp Gly Gln Ala Thr Leu Leu Asn Leu Leu Leu Arg
                245                 250                 255
Asn Tyr Leu His Tyr Ser Leu Tyr Asp Gln Ala Glu Lys Leu Val Ser
            260                 265                 270
Lys Ser Val Phe Pro Glu Gln Ala Asn Asn Asn Glu Trp Ala Arg Tyr
        275                 280                 285
Leu Tyr Tyr Thr Gly Arg Ile Lys Ala Ile Gln Leu Glu Tyr Ser Glu
    290                 295                 300
Ala Arg Arg Thr Met Thr Asn Ala Leu Arg Lys Ala Pro Gln His Thr
305                 310                 315                 320
Ala Val Gly Phe Lys Gln Thr Val His Lys Leu Leu Ile Val Val Glu
                325                 330                 335
Leu Leu Leu Gly Glu Ile Pro Asp Arg Leu Gln Phe Arg Gln Pro Ser
            340                 345                 350
Leu Lys Arg Ser Leu Met Pro Tyr Phe Leu Leu Thr Gln Ala Val Arg
        355                 360                 365
Thr Gly Asn Leu Ala Lys Phe Asn Gln Val Leu Asp Gln Phe Gly Glu
    370                 375                 380
Lys Phe Gln Ala Asp Gly Thr Tyr Thr Leu Ile Ile Arg Leu Arg His
385                 390                 395                 400
Asn Val Ile Lys Thr Gly Val Arg Met Ile Ser Leu Ser Tyr Ser Arg
                405                 410                 415
Ile Ser Leu Ala Asp Ile Ala Gln Lys Leu Gln Leu Asp Ser Pro Glu
            420                 425                 430
Asp Ala Glu Phe Ile Val Ala Lys Ala Ile Arg Asp Gly Val Ile Glu
        435                 440                 445
Ala Ser Ile Asn His Glu Lys Gly Tyr Val Gln Ser Lys Glu Met Ile
    450                 455                 460
Asp Ile Tyr Ser Thr Arg Glu Pro Gln Leu Ala Phe His Gln Arg Ile
465                 470                 475                 480
Ser Phe Cys Leu Asp Ile His Asn Met Ser Val Lys Ala Met Arg Phe
                485                 490                 495
Pro Pro Lys Ser Tyr Asn Lys Asp Leu Glu Ser Ala Glu Glu Arg Arg
            500                 505                 510
Glu Arg Glu Gln Gln Asp Leu Glu Phe Ala Lys Glu Met Ala Glu Asp
        515                 520                 525
Asp Asp Asp Ser Phe Pro
    530

<210> 39

<211> 207

<212> PRT

<213> Homo sapiens

<400> 39
Met Ala Gly Pro Ala Thr Gln Ser Pro Met Lys Leu Met Ala Leu Gln
1               5                   10                  15
Leu Leu Leu Trp His Ser Ala Leu Trp Thr Val Gln Glu Ala Thr Pro
            20                  25                  30

Leu Gly Pro Ala Ser Ser Leu Pro Gln Ser Phe Leu Leu Lys Cys Leu
35 40 45
Glu Gln Val Arg Lys Ile Gln Gly Asp Gly Ala Ala Leu Gln Glu Lys
50 55 60
Leu Val Ser Glu Cys Ala Thr Tyr Lys Leu Cys His Pro Glu Glu Leu
65 70 75 80
Val Leu Leu Gly His Ser Leu Gly Ile Pro Trp Ala Pro Leu Ser Ser
85 90 95
Cys Pro Ser Gln Ala Leu Gln Leu Ala Gly Cys Leu Ser Gln Leu His
100 105 110
Ser Gly Leu Phe Leu Tyr Gln Gly Leu Leu Gln Ala Leu Glu Gly Ile
115 120 125
Ser Pro Glu Leu Gly Pro Thr Leu Asp Thr Leu Gln Leu Asp Val Ala
130 135 140
Asp Phe Ala Thr Thr Ile Trp Gln Gln Met Glu Glu Leu Gly Met Ala
145 150 155 160
Pro Ala Leu Gln Pro Thr Gln Gly Ala Met Pro Ala Phe Ala Ser Ala
165 170 175
Phe Gln Arg Arg Ala Gly Gly Val Leu Val Ala Ser His Leu Gln Ser
180 185 190
Phe Leu Glu Val Ser Tyr Arg Val Leu Arg His Leu Ala Gln Pro
195 200 205

<210> 40

<211> 989

<212> PRT

<213> Homo sapiens

<400> 40
Met Lys Val Val Asn Leu Lys Gln Ala Ile Leu Gln Ala Trp Lys Glu
1 5 10 15
Arg Trp Ser Tyr Tyr Gln Trp Ala Ile Asn Met Lys Lys Phe Phe Pro
20 25 30
Lys Gly Ala Thr Trp Asp Ile Leu Asn Leu Ala Asp Ala Leu Leu Glu
35 40 45
Gln Ala Met Ile Gly Pro Ser Pro Asn Pro Leu Ile Leu Ser Tyr Leu
50 55 60
Lys Tyr Ala Ile Ser Ser Gln Met Val Ser Tyr Ser Ser Val Leu Thr
65 70 75 80
Ala Ile Ser Lys Phe Asp Asp Phe Ser Arg Asp Leu Cys Val Gln Ala
85 90 95
Leu Leu Asp Ile Met Asp Met Phe Cys Asp Arg Leu Ser Cys His Gly
100 105 110
Lys Ala Glu Glu Cys Ile Gly Leu Cys Arg Ala Leu Leu Ser Ala Leu
115 120 125
His Trp Leu Leu Arg Cys Thr Ala Ala Ser Ala Glu Arg Leu Arg Glu
130 135 140
Gly Leu Glu Ala Gly Thr Pro Ala Ala Gly Glu Lys Gln Leu Ala Met
145 150 155 160
Cys Leu Gln Arg Leu Glu Lys Thr Leu Ser Ser Thr Lys Asn Arg Ala
165 170 175
Leu Leu His Ile Ala Lys Leu Glu Glu Ala Ser Ser Trp Thr Ala Ile
180 185 190

Glu His Ser Leu Leu Lys Leu Gly Glu Ile Leu Thr Asn Leu Ser Asn
195 200 205
Pro Gln Leu Arg Ser Gln Ala Glu Gln Cys Gly Thr Leu Ile Arg Ser
210 215 220
Ile Pro Thr Met Leu Ser Val His Ala Glu Gln Met His Lys Thr Gly
225 230 235 240
Phe Pro Thr Val His Ala Val Ile Leu Leu Glu Gly Thr Met Asn Leu
245 250 255
Thr Gly Glu Thr Gln Ser Leu Val Glu Gln Leu Thr Met Val Lys Arg
260 265 270
Met Gln His Ile Pro Thr Pro Leu Phe Val Leu Glu Ile Trp Lys Ala
275 280 285
Cys Phe Val Gly Leu Ile Glu Ser Pro Glu Gly Thr Glu Glu Leu Lys
290 295 300
Trp Thr Ala Phe Thr Phe Leu Lys Ile Pro Gln Val Leu Val Lys Leu
305 310 315 320
Lys Lys Tyr Ser His Gly Asp Lys Asp Phe Thr Glu Asp Val Asn Cys
325 330 335
Ala Phe Glu Phe Leu Leu Lys Leu Thr Pro Leu Leu Asp Lys Ala Asp
340 345 350
Gln Arg Cys Asn Cys Asp Cys Thr Asn Phe Leu Leu Gln Glu Cys Gly
355 360 365
Lys Gln Gly Leu Leu Ser Glu Ala Ser Val Asn Asn Leu Met Ala Lys
370 375 380
Arg Lys Ala Asp Arg Glu His Ala Pro Gln Gln Lys Ser Gly Glu Asn
385 390 395 400
Ala Asn Ile Gln Pro Asn Ile Gln Leu Ile Leu Arg Ala Glu Pro Thr
405 410 415
Val Thr Asn Ile Leu Lys Thr Met Asp Ala Asp His Ser Lys Ser Pro
420 425 430
Glu Gly Leu Leu Gly Val Leu Gly His Met Leu Ser Gly Lys Ser Leu
435 440 445
Asp Leu Leu Leu Ala Ala Ala Ala Ala Thr Gly Lys Leu Lys Ser Phe
450 455 460
Ala Arg Lys Phe Ile Asn Leu Asn Glu Phe Thr Thr Tyr Gly Ser Glu
465 470 475 480
Glu Ser Thr Lys Pro Ala Ser Val Arg Ala Leu Leu Phe Asp Ile Ser
485 490 495
Phe Leu Met Leu Cys His Val Ala Gln Thr Tyr Gly Ser Glu Val Ile
500 505 510
Leu Ser Glu Ser Arg Thr Gly Ala Glu Val Pro Phe Phe Glu Thr Trp
515 520 525
Met Gln Thr Cys Met Pro Glu Glu Gly Lys Ile Leu Asn Pro Asp His
530 535 540
Pro Cys Phe Arg Pro Asp Ser Thr Lys Val Glu Ser Leu Val Ala Leu
545 550 555 560
Leu Asn Asn Ser Ser Glu Met Lys Leu Val Gln Met Lys Trp His Glu
565 570 575
Ala Cys Leu Ser Ile Ser Ala Ala Ile Leu Glu Ile Leu Asn Ala Trp
580 585 590
Glu Asn Gly Val Leu Ala Phe Glu Ser Ile Gln Lys Ile Thr Asp Asn
595 600 605
Ile Lys Gly Lys Val Cys Ser Leu Ala Val Cys Ala Val Ala Trp Leu
610 615 620
Val Ala His Val Arg Met Leu Gly Leu Asp Glu Arg Glu Lys Ser Leu
625 630 635 640
Gln Met Ile Arg Gln Leu Ala Gly Pro Leu Phe Ser Glu Asn Thr Leu
645 650 655

Gln Phe Tyr Asn Glu Arg Val Val Ile Met Asn Ser Ile Leu Glu Arg
660 665 670
Met Cys Ala Asp Val Leu Gln Gln Thr Ala Thr Gln Ile Lys Phe Pro
675 680 685
Ser Thr Gly Val Asp Thr Met Pro Tyr Trp Asn Leu Leu Pro Pro Lys
690 695 700
Arg Pro Ile Lys Glu Val Leu Thr Asp Ile Phe Ala Lys Val Leu Glu
705 710 715 720
Lys Gly Trp Val Asp Ser Arg Ser Ile His Ile Phe Asp Thr Leu Leu
725 730 735
His Met Gly Gly Val Tyr Trp Phe Cys Asn Asn Leu Ile Lys Glu Leu
740 745 750
Leu Lys Glu Thr Arg Lys Glu His Thr Leu Arg Ala Val Glu Leu Leu
755 760 765
Tyr Ser Ile Phe Cys Leu Asp Met Gln Gln Val Thr Leu Val Leu Leu
770 775 780
Gly His Ile Leu Pro Gly Leu Leu Thr Asp Ser Ser Lys Trp His Ser
785 790 795 800
Leu Met Asp Pro Pro Gly Thr Ala Leu Ala Lys Leu Ala Val Trp Cys
805 810 815
Ala Leu Ser Ser Tyr Ser Ser His Lys Gly Gln Ala Ser Thr Arg Gln
820 825 830
Lys Lys Arg His Arg Glu Asp Ile Glu Asp Tyr Ile Ser Leu Phe Pro
835 840 845
Leu Asp Asp Val Gln Pro Ser Lys Leu Met Arg Leu Leu Ser Ser Asn
850 855 860
Glu Asp Asp Ala Asn Ile Leu Ser Ser Pro Thr Asp Arg Ser Met Ser
865 870 875 880
Ser Ser Leu Ser Ala Ser Gln Leu His Thr Val Asn Met Arg Asp Pro
885 890 895
Leu Asn Arg Val Leu Ala Asn Leu Phe Leu Leu Ile Ser Ser Ile Leu
900 905 910
Gly Ser Arg Thr Ala Gly Pro His Thr Gln Phe Val Gln Trp Phe Met
915 920 925
Glu Glu Cys Val Asp Cys Leu Glu Gln Gly Gly Arg Gly Ser Val Leu
930 935 940
Gln Phe Met Pro Phe Thr Thr Val Ser Glu Leu Val Lys Val Ser Ala
945 950 955 960
Met Ser Ser Pro Lys Val Val Leu Ala Ile Thr Asp Leu Ser Leu Pro
965 970 975
Leu Gly Arg Gln Val Ala Ala Lys Ala Ile Ala Ala Leu
980 985

<210> 41

<211> 490

<212> PRT

<213> Homo sapiens

<400> 41
Met Glu Gln Lys Pro Ser Lys Val Glu Cys Gly Ser Asp Pro Glu Glu
1 5 10 15
Asn Ser Ala Arg Ser Pro Asp Gly Lys Arg Lys Arg Lys Asn Gly Gln
20 25 30

Cys Ser Leu Lys Thr Ser Met Ser Gly Tyr Ile Pro Ser Tyr Leu Asp
35 40 45
Lys Asp Glu Gln Cys Val Val Cys Gly Asp Lys Ala Thr Gly Tyr His
50 55 60
Tyr Arg Cys Ile Thr Cys Glu Gly Cys Lys Gly Phe Phe Arg Arg Thr
65 70 75 80
Ile Gln Lys Asn Leu His Pro Thr Tyr Ser Cys Lys Tyr Asp Ser Cys
85 90 95
Cys Val Ile Asp Lys Ile Thr Arg Asn Gln Cys Gln Leu Cys Arg Phe
100 105 110
Lys Lys Cys Ile Ala Val Gly Met Ala Met Asp Leu Val Leu Asp Asp
115 120 125
Ser Lys Arg Val Ala Lys Arg Lys Leu Ile Glu Gln Asn Arg Glu Arg
130 135 140
Arg Arg Lys Glu Glu Met Ile Arg Ser Leu Gln Gln Arg Pro Glu Pro
145 150 155 160
Thr Pro Glu Glu Trp Asp Leu Ile His Ile Ala Thr Glu Ala His Arg
165 170 175
Ser Thr Asn Ala Gln Gly Ser His Trp Lys Gln Arg Arg Lys Phe Leu
180 185 190
Pro Asp Asp Ile Gly Gln Ser Pro Ile Val Ser Met Pro Asp Gly Asp
195 200 205
Lys Val Asp Leu Glu Ala Phe Ser Glu Phe Thr Lys Ile Ile Thr Pro
210 215 220
Ala Ile Thr Arg Val Val Asp Phe Ala Lys Lys Leu Pro Met Phe Ser
225 230 235 240
Glu Leu Pro Cys Glu Asp Gln Ile Ile Leu Leu Lys Gly Cys Cys Met
245 250 255
Glu Ile Met Ser Leu Arg Ala Ala Val Arg Tyr Asp Pro Glu Ser Asp
260 265 270
Thr Leu Thr Leu Ser Gly Glu Met Ala Val Lys Arg Glu Gln Leu Lys
275 280 285
Asn Gly Gly Leu Gly Val Val Ser Asp Ala Ile Phe Glu Leu Gly Lys
290 295 300
Ser Leu Ser Ala Phe Asn Leu Asp Asp Thr Glu Val Ala Leu Leu Gln
305 310 315 320
Ala Val Leu Leu Met Ser Thr Asp Arg Ser Gly Leu Leu Cys Val Asp
325 330 335
Lys Ile Glu Lys Ser Gln Glu Ala Tyr Leu Leu Ala Phe Glu His Tyr
340 345 350
Val Asn His Arg Lys His Asn Ile Pro His Phe Trp Pro Lys Leu Leu
355 360 365
Met Lys Glu Arg Glu Val Gln Ser Ser Ile Leu Tyr Lys Gly Ala Ala
370 375 380
Ala Glu Gly Arg Pro Gly Gly Ser Leu Gly Val His Pro Glu Gly Gln
385 390 395 400
Gln Leu Leu Gly Met His Val Val Gln Gly Pro Gln Val Arg Gln Leu
405 410 415
Glu Gln Gln Leu Gly Glu Ala Gly Ser Leu Gln Gly Pro Val Leu Gln
420 425 430
His Gln Ser Pro Lys Ser Pro Gln Gln Arg Leu Leu Glu Leu Leu His
435 440 445
Arg Ser Gly Ile Leu His Ala Arg Ala Val Cys Gly Glu Asp Asp Ser
450 455 460
Ser Glu Ala Asp Ser Pro Ser Ser Ser Glu Glu Glu Pro Glu Val Cys
465 470 475 480
Glu Asp Leu Ala Gly Asn Ala Ala Ser Pro
485 490

<210> 42

<211> 614

<212> PRT

<213> Homo sapiens

<400> 42

```
Met Thr Thr Leu Asp Ser Asn Asn Asn Thr Gly Gly Val Ile Thr Tyr
1               5                   10                  15
Ile Gly Ser Ser Gly Ser Ser Pro Ser Arg Thr Ser Pro Glu Ser Leu
            20                  25                  30
Tyr Ser Asp Asn Ser Asn Gly Ser Phe Gln Ser Leu Thr Gln Gly Cys
        35                  40                  45
Pro Thr Tyr Phe Pro Pro Ser Pro Thr Gly Ser Leu Thr Gln Asp Pro
    50                  55                  60
Ala Arg Ser Phe Gly Ser Ile Pro Pro Ser Leu Ser Asp Asp Gly Ser
65                  70                  75                  80
Pro Ser Ser Ser Ser Ser Ser Ser Ser Ser Ser Ser Ser Phe Tyr Asn
                85                  90                  95
Gly Ser Pro Pro Gly Ser Leu Gln Val Ala Met Glu Asp Ser Ser Arg
            100                 105                 110
Val Ser Pro Ser Lys Ser Thr Ser Asn Ile Thr Lys Leu Asn Gly Met
        115                 120                 125
Val Leu Leu Cys Lys Val Cys Gly Asp Val Ala Ser Gly Phe His Tyr
    130                 135                 140
Gly Val Leu Ala Cys Glu Gly Cys Lys Gly Phe Phe Arg Arg Ser Ile
145                 150                 155                 160
Gln Gln Asn Ile Gln Tyr Lys Arg Cys Leu Lys Asn Glu Asn Cys Ser
                165                 170                 175
Ile Val Arg Ile Asn Arg Asn Arg Cys Gln Gln Cys Arg Phe Lys Lys
            180                 185                 190
Cys Leu Ser Val Gly Met Ser Arg Asp Ala Val Arg Phe Gly Arg Ile
        195                 200                 205
Pro Lys Arg Glu Lys Gln Arg Met Leu Ala Glu Met Gln Ser Ala Met
    210                 215                 220
Asn Leu Ala Asn Asn Gln Leu Ser Ser Gln Cys Pro Leu Glu Thr Ser
225                 230                 235                 240
Pro Thr Gln His Pro Thr Pro Gly Pro Met Gly Pro Ser Pro Pro Pro
                245                 250                 255
Ala Pro Val Pro Ser Pro Leu Val Gly Phe Ser Gln Phe Pro Gln Gln
            260                 265                 270
Leu Thr Pro Pro Arg Ser Pro Ser Pro Glu Pro Thr Val Glu Asp Val
        275                 280                 285
Ile Ser Gln Val Ala Arg Ala His Arg Glu Ile Phe Thr Tyr Ala His
    290                 295                 300
Asp Lys Leu Gly Ser Ser Pro Gly Asn Phe Asn Ala Asn His Ala Ser
305                 310                 315                 320
Gly Ser Pro Pro Ala Thr Thr Pro His Arg Trp Glu Asn Gln Gly Cys
                325                 330                 335
Pro Pro Ala Pro Asn Asp Asn Asn Thr Leu Ala Ala Gln Arg His Asn
            340                 345                 350
Glu Ala Leu Asn Gly Leu Arg Gln Ala Pro Ser Ser Tyr Pro Pro Thr
        355                 360                 365
```

Trp Pro Pro Gly Pro Ala His His Ser Cys His Gln Ser Asn Ser Asn
370 375 380

Gly His Arg Leu Cys Pro Thr His Val Tyr Ala Ala Pro Glu Gly Lys
385 390 395 400

Ala Pro Ala Asn Ser Pro Arg Gln Gly Asn Ser Lys Asn Val Leu Leu
405 410 415

Ala Cys Pro Met Asn Met Tyr Pro His Gly Arg Ser Gly Arg Thr Val
420 425 430

Gln Glu Ile Trp Glu Asp Phe Ser Met Ser Phe Thr Pro Ala Val Arg
435 440 445

Glu Val Val Glu Phe Ala Lys His Ile Pro Gly Phe Arg Asp Leu Ser
450 455 460

Gln His Asp Gln Val Thr Leu Leu Lys Ala Gly Thr Phe Glu Val Leu
465 470 475 480

Met Val Arg Phe Ala Ser Leu Phe Asn Val Lys Asp Gln Thr Val Met
485 490 495

Phe Leu Ser Arg Thr Thr Tyr Ser Leu Gln Glu Leu Gly Ala Met Gly
500 505 510

Met Gly Asp Leu Leu Ser Ala Met Phe Asp Phe Ser Glu Lys Leu Asn
515 520 525

Ser Leu Ala Leu Thr Glu Glu Glu Leu Gly Leu Phe Thr Ala Val Val
530 535 540

Leu Val Ser Ala Asp Arg Ser Gly Met Glu Asn Ser Ala Ser Val Glu
545 550 555 560

Gln Leu Gln Glu Thr Leu Leu Arg Ala Leu Arg Ala Leu Val Leu Lys
565 570 575

Asn Arg Pro Leu Glu Thr Ser Arg Phe Thr Lys Leu Leu Leu Lys Leu
580 585 590

Pro Asp Leu Arg Thr Leu Asn Asn Met His Ser Glu Lys Leu Leu Ser
595 600 605

Phe Arg Val Asp Ala Gln
610

<210> 43

<211> 703

<212> PRT

<213> Homo sapiens

<400> 43

Met Ala Asp Arg Arg Arg Gln Arg Ala Ser Gln Asp Thr Glu Asp Glu
1 5 10 15

Glu Ser Gly Ala Ser Gly Ser Asp Ser Gly Gly Ser Pro Leu Arg Gly
20 25 30

Gly Gly Ser Cys Ser Gly Ser Ala Gly Gly Gly Gly Ser Gly Ser Leu
35 40 45

Pro Ser Gln Arg Gly Gly Arg Thr Gly Ala Leu His Leu Arg Arg Val
50 55 60

Glu Ser Gly Gly Ala Lys Ser Ala Glu Glu Ser Glu Cys Glu Ser Glu
65 70 75 80

Asp Gly Ile Glu Gly Asp Ala Val Leu Ser Asp Tyr Glu Ser Ala Glu
85 90 95

Asp Ser Glu Gly Glu Glu Gly Glu Tyr Ser Glu Glu Glu Asn Ser Lys
100 105 110

```
Val Glu Leu Lys Ser Glu Ala Asn Asp Ala Val Asn Ser Ser Thr Lys
        115                 120                 125
Glu Glu Lys Gly Glu Glu Lys Pro Asp Thr Lys Ser Thr Val Thr Gly
    130                 135                 140
Glu Arg Gln Ser Gly Asp Gly Gln Glu Ser Thr Glu Pro Val Glu Asn
145                 150                 155                 160
Lys Val Gly Lys Lys Gly Pro Lys His Leu Asp Asp Asp Glu Asp Arg
                165                 170                 175
Lys Asn Pro Ala Tyr Ile Pro Arg Lys Gly Leu Phe Phe Glu His Asp
            180                 185                 190
Leu Arg Gly Gln Thr Gln Glu Glu Glu Val Arg Pro Lys Gly Arg Gln
        195                 200                 205
Arg Lys Leu Trp Lys Asp Glu Gly Arg Trp Glu His Asp Lys Phe Arg
    210                 215                 220
Glu Asp Glu Gln Ala Pro Lys Ser Arg Gln Glu Leu Ile Ala Leu Tyr
225                 230                 235                 240
Gly Tyr Asp Ile Arg Ser Ala His Asn Pro Asp Asp Ile Lys Pro Arg
                245                 250                 255
Arg Ile Arg Lys Pro Arg Tyr Gly Ser Pro Pro Gln Arg Asp Pro Asn
            260                 265                 270
Trp Asn Gly Glu Arg Leu Asn Lys Ser His Arg His Gln Gly Leu Gly
        275                 280                 285
Gly Thr Leu Pro Pro Arg Thr Phe Ile Asn Arg Asn Ala Ala Gly Thr
    290                 295                 300
Gly Arg Met Ser Ala Pro Arg Asn Tyr Ser Arg Ser Gly Gly Phe Lys
305                 310                 315                 320
Glu Gly Arg Ala Gly Phe Arg Pro Val Glu Ala Gly Gly Gln His Gly
                325                 330                 335
Gly Arg Ser Gly Glu Thr Val Lys His Glu Ile Ser Tyr Arg Ser Arg
            340                 345                 350
Arg Leu Glu Gln Thr Ser Val Arg Asp Pro Ser Pro Glu Ala Asp Ala
        355                 360                 365
Pro Val Leu Gly Ser Pro Glu Lys Glu Glu Ala Ala Ser Glu Pro Pro
    370                 375                 380
Ala Ala Ala Pro Asp Ala Ala Pro Pro Pro Pro Asp Arg Pro Ile Glu
385                 390                 395                 400
Lys Lys Ser Tyr Ser Arg Ala Arg Arg Thr Arg Thr Lys Val Gly Asp
                405                 410                 415
Ala Val Lys Leu Ala Glu Glu Val Pro Pro Pro Pro Glu Gly Leu Ile
            420                 425                 430
Pro Ala Pro Pro Val Pro Glu Thr Thr Pro Thr Pro Pro Thr Lys Thr
        435                 440                 445
Gly Thr Trp Glu Ala Pro Val Asp Ser Ser Thr Ser Gly Leu Glu Gln
    450                 455                 460
Asp Val Ala Gln Leu Asn Ile Ala Glu Gln Asn Trp Ser Pro Gly Gln
465                 470                 475                 480
Pro Ser Phe Leu Gln Pro Arg Glu Leu Arg Gly Met Pro Asn His Ile
                485                 490                 495
His Met Gly Ala Gly Pro Pro Pro Gln Phe Asn Arg Met Glu Glu Met
            500                 505                 510
Gly Val Gln Gly Gly Arg Ala Lys Arg Tyr Ser Ser Gln Arg Gln Arg
        515                 520                 525
Pro Val Pro Glu Pro Pro Ala Pro Pro Val His Ile Ser Ile Met Glu
    530                 535                 540
Gly His Tyr Tyr Asp Pro Leu Gln Phe Gln Gly Pro Ile Tyr Thr His
545                 550                 555                 560
Gly Asp Ser Pro Ala Pro Leu Pro Pro Gln Gly Met Leu Val Gln Pro
                565                 570                 575
```

Gly Met Asn Leu Pro His Pro Gly Leu His Pro His Gln Thr Pro Ala
580 585 590
Pro Leu Pro Asn Pro Gly Leu Tyr Pro Pro Pro Val Ser Met Ser Pro
595 600 605
Gly Gln Pro Pro Pro Gln Gln Leu Leu Ala Pro Thr Tyr Phe Ser Ala
610 615 620
Pro Gly Val Met Asn Phe Gly Asn Pro Ser Tyr Pro Tyr Ala Pro Gly
625 630 635 640
Ala Leu Pro Pro Pro Pro Pro Pro His Leu Tyr Pro Asn Thr Gln Ala
645 650 655
Pro Ser Gln Val Tyr Gly Gly Val Thr Tyr Tyr Asn Pro Ala Gln Gln
660 665 670
Gln Val Gln Pro Lys Pro Ser Pro Pro Arg Arg Thr Pro Gln Pro Val
675 680 685
Thr Ile Lys Pro Pro Pro Pro Glu Val Val Ser Arg Gly Ser Ser
690 695 700

<210> 44

<211> 560

<212> PRT

<213> Homo sapiens

<400> 44
Met Pro Gln Thr Arg Ser Gln Ala Gln Ala Thr Ile Ser Phe Pro Lys
1 5 10 15
Arg Lys Leu Ser Arg Ala Leu Asn Lys Ala Lys Asn Ser Ser Asp Ala
20 25 30
Lys Leu Glu Pro Thr Asn Val Gln Thr Val Thr Cys Ser Pro Arg Val
35 40 45
Lys Ala Leu Pro Leu Ser Pro Arg Lys Arg Leu Gly Asp Asp Asn Leu
50 55 60
Cys Asn Thr Pro His Leu Pro Pro Cys Ser Pro Pro Lys Gln Gly Lys
65 70 75 80
Lys Glu Asn Gly Pro Pro His Ser His Thr Leu Lys Gly Arg Arg Leu
85 90 95
Val Phe Asp Asn Gln Leu Thr Ile Lys Ser Pro Ser Lys Arg Glu Leu
100 105 110
Ala Lys Val His Gln Asn Lys Ile Leu Ser Ser Val Arg Lys Ser Gln
115 120 125
Glu Ile Thr Thr Asn Ser Glu Gln Arg Cys Pro Leu Lys Lys Glu Ser
130 135 140
Ala Cys Val Arg Leu Phe Lys Gln Glu Gly Thr Cys Tyr Gln Gln Ala
145 150 155 160
Lys Leu Val Leu Asn Thr Ala Val Pro Asp Arg Leu Pro Ala Arg Glu
165 170 175
Arg Glu Met Asp Val Ile Arg Asn Phe Leu Arg Glu His Ile Cys Gly
180 185 190
Lys Lys Ala Gly Ser Leu Tyr Leu Ser Gly Ala Pro Gly Thr Gly Lys
195 200 205
Thr Ala Cys Leu Ser Arg Ile Leu Gln Asp Leu Lys Lys Glu Leu Lys
210 215 220
Gly Phe Lys Thr Ile Met Leu Asn Cys Met Ser Leu Arg Thr Ala Gln
225 230 235 240

Ala Val Phe Pro Ala Ile Ala Gln Glu Ile Cys Gln Glu Glu Val Ser
245 250 255
Arg Pro Ala Gly Lys Asp Met Met Arg Lys Leu Glu Lys His Met Thr
260 265 270
Ala Glu Lys Gly Pro Met Ile Val Leu Val Leu Asp Glu Met Asp Gln
275 280 285
Leu Asp Ser Lys Gly Gln Asp Val Leu Tyr Thr Leu Phe Glu Trp Pro
290 295 300
Trp Leu Ser Asn Ser His Leu Val Leu Ile Gly Ile Ala Asn Thr Leu
305 310 315 320
Asp Leu Thr Asp Arg Ile Leu Pro Arg Leu Gln Ala Arg Glu Lys Cys
325 330 335
Lys Pro Gln Leu Leu Asn Phe Pro Pro Tyr Thr Arg Asn Gln Ile Val
340 345 350
Thr Ile Leu Gln Asp Arg Leu Asn Gln Val Ser Arg Asp Gln Val Leu
355 360 365
Asp Asn Ala Ala Val Gln Phe Cys Ala Arg Lys Val Ser Ala Val Ser
370 375 380
Gly Asp Val Arg Lys Ala Leu Asp Val Cys Arg Arg Ala Ile Glu Ile
385 390 395 400
Val Glu Ser Asp Val Lys Ser Gln Thr Ile Leu Lys Pro Leu Ser Glu
405 410 415
Cys Lys Ser Pro Ser Glu Pro Leu Ile Pro Lys Arg Val Gly Leu Ile
420 425 430
His Ile Ser Gln Val Ile Ser Glu Val Asp Gly Asn Arg Met Thr Leu
435 440 445
Ser Gln Glu Gly Ala Gln Asp Ser Phe Pro Leu Gln Gln Lys Ile Leu
450 455 460
Val Cys Ser Leu Met Leu Leu Ile Arg Gln Leu Lys Ile Lys Glu Val
465 470 475 480
Thr Leu Gly Lys Leu Tyr Glu Ala Tyr Ser Lys Val Cys Arg Lys Gln
485 490 495
Gln Val Ala Ala Val Asp Gln Ser Glu Cys Leu Ser Leu Ser Gly Leu
500 505 510
Leu Glu Ala Arg Gly Ile Leu Gly Leu Lys Arg Asn Lys Glu Thr Arg
515 520 525
Leu Thr Lys Val Phe Phe Lys Ile Glu Glu Lys Glu Ile Glu His Ala
530 535 540
Leu Lys Asp Lys Ala Leu Ile Gly Asn Ile Leu Ala Thr Gly Leu Pro
545 550 555 560

<210> 45

<211> 462

<212> PRT

<213> Homo sapiens

<400> 45
Met Ala Ser Asn Ser Ser Ser Cys Pro Thr Pro Gly Gly Gly His Leu
1 5 10 15
Asn Gly Tyr Pro Val Pro Pro Tyr Ala Phe Phe Phe Pro Pro Met Leu
20 25 30
Gly Gly Leu Ser Pro Pro Gly Ala Leu Thr Thr Leu Gln His Gln Leu
35 40 45

```
Pro Val Ser Gly Tyr Ser Thr Pro Ser Pro Ala Thr Ile Glu Thr Gln
    50                  55                  60
Ser Ser Ser Ser Glu Glu Ile Val Pro Ser Pro Pro Ser Pro Pro Pro
65                  70                  75                  80
Leu Pro Arg Ile Tyr Lys Pro Cys Phe Val Cys Gln Asp Lys Ser Ser
                85                  90                  95
Gly Tyr His Tyr Gly Val Ser Ala Cys Glu Gly Cys Lys Gly Phe Phe
            100                 105                 110
Arg Arg Ser Ile Gln Lys Asn Met Val Tyr Thr Cys His Arg Asp Lys
        115                 120                 125
Asn Cys Ile Ile Asn Lys Val Thr Arg Asn Arg Cys Gln Tyr Cys Arg
    130                 135                 140
Leu Gln Lys Cys Phe Glu Val Gly Met Ser Lys Glu Ser Val Arg Asn
145                 150                 155                 160
Asp Arg Asn Lys Lys Lys Lys Glu Val Pro Lys Pro Glu Cys Ser Glu
                165                 170                 175
Ser Tyr Thr Leu Thr Pro Glu Val Gly Glu Leu Ile Glu Lys Val Arg
            180                 185                 190
Lys Ala His Gln Glu Thr Phe Pro Ala Leu Cys Gln Leu Gly Lys Tyr
        195                 200                 205
Thr Thr Asn Asn Ser Ser Glu Gln Arg Val Ser Leu Asp Ile Asp Leu
    210                 215                 220
Trp Asp Lys Phe Ser Glu Leu Ser Thr Lys Cys Ile Ile Lys Thr Val
225                 230                 235                 240
Glu Phe Ala Lys Gln Leu Pro Gly Phe Thr Thr Leu Thr Ile Ala Asp
                245                 250                 255
Gln Ile Thr Leu Leu Lys Ala Ala Cys Leu Asp Ile Leu Ile Leu Arg
            260                 265                 270
Ile Cys Thr Arg Tyr Thr Pro Glu Gln Asp Thr Met Thr Phe Ser Asp
        275                 280                 285
Gly Leu Thr Leu Asn Arg Thr Gln Met His Asn Ala Gly Phe Gly Pro
    290                 295                 300
Leu Thr Asp Leu Val Phe Ala Phe Ala Asn Gln Leu Leu Pro Leu Glu
305                 310                 315                 320
Met Asp Asp Ala Glu Thr Gly Leu Leu Ser Ala Ile Cys Leu Ile Cys
                325                 330                 335
Gly Asp Arg Gln Asp Leu Glu Gln Pro Asp Arg Val Asp Met Leu Gln
            340                 345                 350
Glu Pro Leu Leu Glu Ala Leu Lys Val Tyr Val Arg Lys Arg Arg Pro
        355                 360                 365
Ser Arg Pro His Met Phe Pro Lys Met Leu Met Lys Ile Thr Asp Leu
    370                 375                 380
Arg Ser Ile Ser Ala Lys Gly Ala Glu Arg Val Ile Thr Leu Lys Met
385                 390                 395                 400
Glu Ile Pro Gly Ser Met Pro Pro Leu Ile Gln Glu Met Leu Glu Asn
                405                 410                 415
Ser Glu Gly Leu Asp Thr Leu Ser Gly Gln Pro Gly Gly Gly Gly Arg
            420                 425                 430
Asp Gly Gly Gly Leu Ala Pro Pro Pro Gly Ser Cys Ser Pro Ser Leu
        435                 440                 445
Ser Pro Ser Ser Asn Arg Ser Ser Pro Ala Thr His Ser Pro
    450                 455                 460
```

<210> 46

<211> 1531

<212> PRT

<213> Homo sapiens

<400> 46
Met Glu Val Ser Pro Leu Gln Pro Val Asn Glu Asn Met Gln Val Asn
1 5 10 15
Lys Ile Lys Lys Asn Glu Asp Ala Lys Lys Arg Leu Ser Val Glu Arg
20 25 30
Ile Tyr Gln Lys Lys Thr Gln Leu Glu His Ile Leu Leu Arg Pro Asp
35 40 45
Thr Tyr Ile Gly Ser Val Glu Leu Val Thr Gln Gln Met Trp Val Tyr
50 55 60
Asp Glu Asp Val Gly Ile Asn Tyr Arg Glu Val Thr Phe Val Pro Gly
65 70 75 80
Leu Tyr Lys Ile Phe Asp Glu Ile Leu Val Asn Ala Ala Asp Asn Lys
85 90 95
Gln Arg Asp Pro Lys Met Ser Cys Ile Arg Val Thr Ile Asp Pro Glu
100 105 110
Asn Asn Leu Ile Ser Ile Trp Asn Asn Gly Lys Gly Ile Pro Val Val
115 120 125
Glu His Lys Val Glu Lys Met Tyr Val Pro Ala Leu Ile Phe Gly Gln
130 135 140
Leu Leu Thr Ser Ser Asn Tyr Asp Asp Asp Glu Lys Lys Val Thr Gly
145 150 155 160
Gly Arg Asn Gly Tyr Gly Ala Lys Leu Cys Asn Ile Phe Ser Thr Lys
165 170 175
Phe Thr Val Glu Thr Ala Ser Arg Glu Tyr Lys Lys Met Phe Lys Gln
180 185 190
Thr Trp Met Asp Asn Met Gly Arg Ala Gly Glu Met Glu Leu Lys Pro
195 200 205
Phe Asn Gly Glu Asp Tyr Thr Cys Ile Thr Phe Gln Pro Asp Leu Ser
210 215 220
Lys Phe Lys Met Gln Ser Leu Asp Lys Asp Ile Val Ala Leu Met Val
225 230 235 240
Arg Arg Ala Tyr Asp Ile Ala Gly Ser Thr Lys Asp Val Lys Val Phe
245 250 255
Leu Asn Gly Asn Lys Leu Pro Val Lys Gly Phe Arg Ser Tyr Val Asp
260 265 270
Met Tyr Leu Lys Asp Lys Leu Asp Glu Thr Gly Asn Ser Leu Lys Val
275 280 285
Ile His Glu Gln Val Asn His Arg Trp Glu Val Cys Leu Thr Met Ser
290 295 300
Glu Lys Gly Phe Gln Gln Ile Ser Phe Val Asn Ser Ile Ala Thr Ser
305 310 315 320
Lys Gly Gly Arg His Val Asp Tyr Val Ala Asp Gln Ile Val Thr Lys
325 330 335
Leu Val Asp Val Val Lys Lys Lys Asn Lys Gly Gly Val Ala Val Lys
340 345 350
Ala His Gln Val Lys Asn His Met Trp Ile Phe Val Asn Ala Leu Ile
355 360 365
Glu Asn Pro Thr Phe Asp Ser Gln Thr Lys Glu Asn Met Thr Leu Gln
370 375 380

Pro Lys Ser Phe Gly Ser Thr Cys Gln Leu Ser Glu Lys Phe Ile Lys
385 390 395 400
Ala Ala Ile Gly Cys Gly Ile Val Glu Ser Ile Leu Asn Trp Val Lys
405 410 415
Phe Lys Ala Gln Val Gln Leu Asn Lys Lys Cys Ser Ala Val Lys His
420 425 430
Asn Arg Ile Lys Gly Ile Pro Lys Leu Asp Asp Ala Asn Asp Ala Gly
435 440 445
Gly Arg Asn Ser Thr Glu Cys Thr Leu Ile Leu Thr Glu Gly Asp Ser
450 455 460
Ala Lys Thr Leu Ala Val Ser Gly Leu Gly Val Val Gly Arg Asp Lys
465 470 475 480
Tyr Gly Val Phe Pro Leu Arg Gly Lys Ile Leu Asn Val Arg Glu Ala
485 490 495
Ser His Lys Gln Ile Met Glu Asn Ala Glu Ile Asn Asn Ile Ile Lys
500 505 510
Ile Val Gly Leu Gln Tyr Lys Lys Asn Tyr Glu Asp Glu Asp Ser Leu
515 520 525
Lys Thr Leu Arg Tyr Gly Lys Ile Met Ile Met Thr Asp Gln Asp Gln
530 535 540
Asp Gly Ser His Ile Lys Gly Leu Leu Ile Asn Phe Ile His His Asn
545 550 555 560
Trp Pro Ser Leu Leu Arg His Arg Phe Leu Glu Glu Phe Ile Thr Pro
565 570 575
Ile Val Lys Val Ser Lys Asn Lys Gln Glu Met Ala Phe Tyr Ser Leu
580 585 590
Pro Glu Phe Glu Glu Trp Lys Ser Ser Thr Pro Asn His Lys Lys Trp
595 600 605
Lys Val Lys Tyr Tyr Lys Gly Leu Gly Thr Ser Thr Ser Lys Glu Ala
610 615 620
Lys Gln Tyr Phe Ala Asp Met Lys Arg His Arg Ile Gln Phe Lys Tyr
625 630 635 640
Ser Gly Pro Glu Asp Asp Ala Ala Ile Ser Leu Ala Phe Ser Lys Lys
645 650 655
Gln Ile Asp Asp Arg Lys Glu Trp Leu Thr Asn Phe Met Glu Asp Arg
660 665 670
Arg Gln Arg Lys Leu Leu Gly Leu Pro Glu Asp Tyr Leu Tyr Gly Gln
675 680 685
Thr Thr Thr Tyr Leu Thr Tyr Asn Asp Phe Ile Asn Lys Glu Leu Ile
690 695 700
Leu Phe Ser Asn Ser Asp Asn Glu Arg Ser Ile Pro Ser Met Val Asp
705 710 715 720
Gly Leu Lys Pro Gly Gln Arg Lys Val Leu Phe Thr Cys Phe Lys Arg
725 730 735
Asn Asp Lys Arg Glu Val Lys Val Ala Gln Leu Ala Gly Ser Val Ala
740 745 750
Glu Met Ser Ser Tyr His His Gly Glu Met Ser Leu Met Met Thr Ile
755 760 765
Ile Asn Leu Ala Gln Asn Phe Val Gly Ser Asn Asn Leu Asn Leu Leu
770 775 780
Gln Pro Ile Gly Gln Phe Gly Thr Arg Leu His Gly Gly Lys Asp Ser
785 790 795 800
Ala Ser Pro Arg Tyr Ile Phe Thr Met Leu Ser Ser Leu Ala Arg Leu
805 810 815
Leu Phe Pro Pro Lys Asp Asp His Thr Leu Lys Phe Leu Tyr Asp Asp
820 825 830
Asn Gln Arg Val Glu Pro Glu Trp Tyr Ile Pro Ile Ile Pro Met Val
835 840 845

Leu Ile Asn Gly Ala Glu Gly Ile Gly Thr Gly Trp Ser Cys Lys Ile
850 855 860
Pro Asn Phe Asp Val Arg Glu Ile Val Asn Asn Ile Arg Arg Leu Met
865 870 875 880
Asp Gly Glu Glu Pro Leu Pro Met Leu Pro Ser Tyr Lys Asn Phe Lys
885 890 895
Gly Thr Ile Glu Glu Leu Ala Pro Asn Gln Tyr Val Ile Ser Gly Glu
900 905 910
Val Ala Ile Leu Asn Ser Thr Thr Ile Glu Ile Ser Glu Leu Pro Val
915 920 925
Arg Thr Trp Thr Gln Thr Tyr Lys Glu Gln Val Leu Glu Pro Met Leu
930 935 940
Asn Gly Thr Glu Lys Thr Pro Pro Leu Ile Thr Asp Tyr Arg Glu Tyr
945 950 955 960
His Thr Asp Thr Thr Val Lys Phe Val Val Lys Met Thr Glu Glu Lys
965 970 975
Leu Ala Glu Ala Glu Arg Val Gly Leu His Lys Val Phe Lys Leu Gln
980 985 990
Thr Ser Leu Thr Cys Asn Ser Met Val Leu Phe Asp His Val Gly Cys
995 1000 1005
Leu Lys Lys Tyr Asp Thr Val Leu Asp Ile Leu Arg Asp Phe Phe
1010 1015 1020
Glu Leu Arg Leu Lys Tyr Tyr Gly Leu Arg Lys Glu Trp Leu Leu
1025 1030 1035
Gly Met Leu Gly Ala Glu Ser Ala Lys Leu Asn Asn Gln Ala Arg
1040 1045 1050
Phe Ile Leu Glu Lys Ile Asp Gly Lys Ile Ile Ile Glu Asn Lys
1055 1060 1065
Pro Lys Lys Glu Leu Ile Lys Val Leu Ile Gln Arg Gly Tyr Asp
1070 1075 1080
Ser Asp Pro Val Lys Ala Trp Lys Glu Ala Gln Gln Lys Val Pro
1085 1090 1095
Asp Glu Glu Glu Asn Glu Glu Ser Asp Asn Glu Lys Glu Thr Glu
1100 1105 1110
Lys Ser Asp Ser Val Thr Asp Ser Gly Pro Thr Phe Asn Tyr Leu
1115 1120 1125
Leu Asp Met Pro Leu Trp Tyr Leu Thr Lys Glu Lys Lys Asp Glu
1130 1135 1140
Leu Cys Arg Leu Arg Asn Glu Lys Glu Gln Glu Leu Asp Thr Leu
1145 1150 1155
Lys Arg Lys Ser Pro Ser Asp Leu Trp Lys Glu Asp Leu Ala Thr
1160 1165 1170
Phe Ile Glu Glu Leu Glu Ala Val Glu Ala Lys Glu Lys Gln Asp
1175 1180 1185
Glu Gln Val Gly Leu Pro Gly Lys Gly Gly Lys Ala Lys Gly Lys
1190 1195 1200
Lys Thr Gln Met Ala Glu Val Leu Pro Ser Pro Arg Gly Gln Arg
1205 1210 1215
Val Ile Pro Arg Ile Thr Ile Glu Met Lys Ala Glu Ala Glu Lys
1220 1225 1230
Lys Asn Lys Lys Lys Ile Lys Asn Glu Asn Thr Glu Gly Ser Pro
1235 1240 1245
Gln Glu Asp Gly Val Glu Leu Glu Gly Leu Lys Gln Arg Leu Glu
1250 1255 1260
Lys Lys Gln Lys Arg Glu Pro Gly Thr Lys Thr Lys Lys Gln Thr
1265 1270 1275
Thr Leu Ala Phe Lys Pro Ile Lys Lys Gly Lys Lys Arg Asn Pro
1280 1285 1290

Trp Ser Asp Ser Glu Ser Asp Arg Ser Ser Asp Glu Ser Asn Phe
1295 1300 1305
Asp Val Pro Pro Arg Glu Thr Glu Pro Arg Arg Ala Ala Thr Lys
1310 1315 1320
Thr Lys Phe Thr Met Asp Leu Asp Ser Asp Glu Asp Phe Ser Asp
1325 1330 1335
Phe Asp Glu Lys Thr Asp Asp Glu Asp Phe Val Pro Ser Asp Ala
1340 1345 1350
Ser Pro Pro Lys Thr Lys Thr Ser Pro Lys Leu Ser Asn Lys Glu
1355 1360 1365
Leu Lys Pro Gln Lys Ser Val Val Ser Asp Leu Glu Ala Asp Asp
1370 1375 1380
Val Lys Gly Ser Val Pro Leu Ser Ser Ser Pro Pro Ala Thr His
1385 1390 1395
Phe Pro Asp Glu Thr Glu Ile Thr Asn Pro Val Pro Lys Lys Asn
1400 1405 1410
Val Thr Val Lys Lys Thr Ala Ala Lys Ser Gln Ser Ser Thr Ser
1415 1420 1425
Thr Thr Gly Ala Lys Lys Arg Ala Ala Pro Lys Gly Thr Lys Arg
1430 1435 1440
Asp Pro Ala Leu Asn Ser Gly Val Ser Gln Lys Pro Asp Pro Ala
1445 1450 1455
Lys Thr Lys Asn Arg Arg Lys Arg Lys Pro Ser Thr Ser Asp Asp
1460 1465 1470
Ser Asp Ser Asn Phe Glu Lys Ile Val Ser Lys Ala Val Thr Ser
1475 1480 1485
Lys Lys Ser Lys Gly Glu Ser Asp Asp Phe His Met Asp Phe Asp
1490 1495 1500
Ser Ala Val Ala Pro Arg Ala Lys Ser Val Arg Ala Lys Lys Pro
1505 1510 1515
Ile Lys Tyr Leu Glu Glu Ser Asp Glu Asp Asp Leu Phe
1520 1525 1530

<210> 47

<211> 258

<212> PRT

<213> Homo sapiens

<400> 47
Met Leu Pro Leu Cys Leu Val Ala Ala Leu Leu Leu Ala Ala Gly Pro
1 5 10 15
Gly Pro Ser Leu Gly Asp Glu Ala Ile His Cys Pro Pro Cys Ser Glu
20 25 30
Glu Lys Leu Ala Arg Cys Arg Pro Pro Val Gly Cys Glu Glu Leu Val
35 40 45
Arg Glu Pro Gly Cys Gly Cys Cys Ala Thr Cys Ala Leu Gly Leu Gly
50 55 60
Met Pro Cys Gly Val Tyr Thr Pro Arg Cys Gly Ser Gly Leu Arg Cys
65 70 75 80
Tyr Pro Pro Arg Gly Val Glu Lys Pro Leu His Thr Leu Met His Gly
85 90 95
Gln Gly Val Cys Met Glu Leu Ala Glu Ile Glu Ala Ile Gln Glu Ser
100 105 110

Leu Gln Pro Ser Asp Lys Asp Glu Gly Asp His Pro Asn Asn Ser Phe
115 120 125
Ser Pro Cys Ser Ala His Asp Arg Arg Cys Leu Gln Lys His Phe Ala
130 135 140
Lys Ile Arg Asp Arg Ser Thr Ser Gly Gly Lys Met Lys Val Asn Gly
145 150 155 160
Ala Pro Arg Glu Asp Ala Arg Pro Val Pro Gln Gly Ser Cys Gln Ser
165 170 175
Glu Leu His Arg Ala Leu Glu Arg Leu Ala Ala Ser Gln Ser Arg Thr
180 185 190
His Glu Asp Leu Tyr Ile Ile Pro Ile Pro Asn Cys Asp Arg Asn Gly
195 200 205
Asn Phe His Pro Lys Gln Cys His Pro Ala Leu Asp Gly Gln Arg Gly
210 215 220
Lys Cys Trp Cys Val Asp Arg Lys Thr Gly Val Lys Leu Pro Gly Gly
225 230 235 240
Leu Glu Pro Lys Gly Glu Leu Asp Cys His Gln Leu Ala Asp Ser Phe
245 250 255
Arg Glu

<210> 48

<211> 378

<212> PRT

<213> Homo sapiens

<400> 48
Met Asp Leu Gly Lys Pro Met Lys Ser Val Leu Val Val Ala Leu Leu
1 5 10 15
Val Ile Phe Gln Val Cys Leu Cys Gln Asp Glu Val Thr Asp Asp Tyr
20 25 30
Ile Gly Asp Asn Thr Thr Val Asp Tyr Thr Leu Phe Glu Ser Leu Cys
35 40 45
Ser Lys Lys Asp Val Arg Asn Phe Lys Ala Trp Phe Leu Pro Ile Met
50 55 60
Tyr Ser Ile Ile Cys Phe Val Gly Leu Leu Gly Asn Gly Leu Val Val
65 70 75 80
Leu Thr Tyr Ile Tyr Phe Lys Arg Leu Lys Thr Met Thr Asp Thr Tyr
85 90 95
Leu Leu Asn Leu Ala Val Ala Asp Ile Leu Phe Leu Leu Thr Leu Pro
100 105 110
Phe Trp Ala Tyr Ser Ala Ala Lys Ser Trp Val Phe Gly Val His Phe
115 120 125
Cys Lys Leu Ile Phe Ala Ile Tyr Lys Met Ser Phe Phe Ser Gly Met
130 135 140
Leu Leu Leu Leu Cys Ile Ser Ile Asp Arg Tyr Val Ala Ile Val Gln
145 150 155 160
Ala Val Ser Ala His Arg His Arg Ala Arg Val Leu Leu Ile Ser Lys
165 170 175
Leu Ser Cys Val Gly Ile Trp Ile Leu Ala Thr Val Leu Ser Ile Pro
180 185 190
Glu Leu Leu Tyr Ser Asp Leu Gln Arg Ser Ser Ser Glu Gln Ala Met
195 200 205

Arg Cys Ser Leu Ile Thr Glu His Val Glu Ala Phe Ile Thr Ile Gln
210 215 220
Val Ala Gln Met Val Ile Gly Phe Leu Val Pro Leu Leu Ala Met Ser
225 230 235 240
Phe Cys Tyr Leu Val Ile Ile Arg Thr Leu Leu Gln Ala Arg Asn Phe
245 250 255
Glu Arg Asn Lys Ala Ile Lys Val Ile Ile Ala Val Val Val Val Phe
260 265 270
Ile Val Phe Gln Leu Pro Tyr Asn Gly Val Val Leu Ala Gln Thr Val
275 280 285
Ala Asn Phe Asn Ile Thr Ser Ser Thr Cys Glu Leu Ser Lys Gln Leu
290 295 300
Asn Ile Ala Tyr Asp Val Thr Tyr Ser Leu Ala Cys Val Arg Cys Cys
305 310 315 320
Val Asn Pro Phe Leu Tyr Ala Phe Ile Gly Val Lys Phe Arg Asn Asp
325 330 335
Leu Phe Lys Leu Phe Lys Asp Leu Gly Cys Leu Ser Gln Glu Gln Leu
340 345 350
Arg Gln Trp Ser Ser Cys Arg His Ile Arg Arg Ser Ser Met Ser Val
355 360 365
Glu Ala Glu Thr Thr Thr Thr Phe Ser Pro
370 375

<210> 49

<211> 411

<212> PRT

<213> Homo sapiens

<400> 49
Met Ser Lys Arg Pro Ser Tyr Ala Pro Pro Pro Thr Pro Ala Pro Ala
1 5 10 15
Thr Gln Met Pro Ser Thr Pro Gly Phe Val Gly Tyr Asn Pro Tyr Ser
20 25 30
His Leu Ala Tyr Asn Asn Tyr Arg Leu Gly Gly Asn Pro Ser Thr Asn
35 40 45
Ser Arg Val Thr Ala Ser Ser Gly Ile Thr Ile Pro Lys Pro Pro Lys
50 55 60
Pro Pro Asp Lys Pro Leu Met Pro Tyr Met Arg Tyr Ser Arg Lys Val
65 70 75 80
Trp Asp Gln Val Lys Ala Ser Asn Pro Asp Leu Lys Leu Trp Glu Ile
85 90 95
Gly Lys Ile Ile Gly Gly Met Trp Arg Asp Leu Thr Asp Glu Glu Lys
100 105 110
Gln Glu Tyr Leu Asn Glu Tyr Glu Ala Glu Lys Ile Glu Tyr Asn Glu
115 120 125
Ser Met Lys Ala Tyr His Asn Ser Pro Ala Tyr Leu Ala Tyr Ile Asn
130 135 140
Ala Lys Ser Arg Ala Glu Ala Ala Leu Glu Glu Glu Ser Arg Gln Arg
145 150 155 160
Gln Ser Arg Met Glu Lys Gly Glu Pro Tyr Met Ser Ile Gln Pro Ala
165 170 175
Glu Asp Pro Asp Asp Tyr Asp Asp Gly Phe Ser Met Lys His Thr Ala
180 185 190

Thr Ala Arg Phe Gln Arg Asn His Arg Leu Ile Ser Glu Ile Leu Ser
195 200 205
Glu Ser Val Val Pro Asp Val Arg Ser Val Val Thr Thr Ala Arg Met
210 215 220
Gln Val Leu Lys Arg Gln Val Gln Ser Leu Met Val His Gln Arg Lys
225 230 235 240
Leu Glu Ala Glu Leu Leu Gln Ile Glu Glu Arg His Gln Glu Lys Lys
245 250 255
Arg Lys Phe Leu Glu Ser Thr Asp Ser Phe Asn Asn Glu Leu Lys Arg
260 265 270
Leu Cys Gly Leu Lys Val Glu Val Asp Met Glu Lys Ile Ala Ala Glu
275 280 285
Ile Ala Gln Ala Glu Glu Gln Ala Arg Lys Arg Gln Glu Glu Arg Glu
290 295 300
Lys Glu Ala Ala Glu Gln Ala Glu Arg Ser Gln Ser Ser Ile Val Pro
305 310 315 320
Glu Glu Glu Gln Ala Ala Asn Lys Gly Glu Glu Lys Lys Asp Asp Glu
325 330 335
Asn Ile Pro Met Glu Thr Glu Glu Thr His Leu Glu Glu Thr Thr Glu
340 345 350
Ser Gln Gln Asn Gly Glu Glu Gly Thr Ser Thr Pro Glu Asp Lys Glu
355 360 365
Ser Gly Gln Glu Gly Val Asp Ser Met Ala Glu Glu Gly Thr Ser Asp
370 375 380
Ser Asn Thr Gly Ser Glu Ser Asn Ser Ala Thr Val Glu Glu Pro Pro
385 390 395 400
Thr Asp Pro Ile Pro Glu Asp Glu Lys Lys Glu
405 410

<210> 50

<211> 593

<212> PRT

<213> Homo sapiens

<400> 50
Met Ser Val Arg Tyr Ser Ser Ser Lys His Tyr Ser Ser Ser Arg Ser
1 5 10 15
Gly Gly Gly Gly Gly Gly Gly Gly Cys Gly Gly Gly Gly Gly Val Ser
20 25 30
Ser Leu Arg Ile Ser Ser Ser Lys Gly Ser Leu Gly Gly Gly Phe Ser
35 40 45
Ser Gly Gly Phe Ser Gly Gly Ser Phe Ser Arg Gly Ser Ser Gly Gly
50 55 60
Gly Cys Phe Gly Gly Ser Ser Gly Gly Tyr Gly Gly Leu Gly Gly Phe
65 70 75 80
Gly Gly Gly Ser Phe His Gly Ser Tyr Gly Ser Ser Ser Phe Gly Gly
85 90 95
Ser Tyr Gly Gly Ser Phe Gly Gly Gly Asn Phe Gly Gly Gly Ser Phe
100 105 110
Gly Gly Gly Ser Phe Gly Gly Gly Gly Phe Gly Gly Gly Gly Phe Gly
115 120 125
Gly Gly Phe Gly Gly Gly Phe Gly Gly Asp Gly Gly Leu Leu Ser Gly
130 135 140

Asn Glu Lys Val Thr Met Gln Asn Leu Asn Asp Arg Leu Ala Ser Tyr
145 150 155 160
Leu Asp Lys Val Arg Ala Leu Glu Glu Ser Asn Tyr Glu Leu Glu Gly
165 170 175
Lys Ile Lys Glu Trp Tyr Glu Lys His Gly Asn Ser His Gln Gly Glu
180 185 190
Pro Arg Asp Tyr Ser Lys Tyr Tyr Lys Thr Ile Asp Asp Leu Lys Asn
195 200 205
Gln Ile Leu Asn Leu Thr Thr Asp Asn Ala Asn Ile Leu Leu Gln Ile
210 215 220
Asp Asn Ala Arg Leu Ala Ala Asp Asp Phe Arg Leu Lys Tyr Glu Asn
225 230 235 240
Glu Val Ala Leu Arg Gln Ser Val Glu Ala Asp Ile Asn Gly Leu Arg
245 250 255
Arg Val Leu Asp Glu Leu Thr Leu Thr Lys Ala Asp Leu Glu Met Gln
260 265 270
Ile Glu Ser Leu Thr Glu Glu Leu Ala Tyr Leu Lys Lys Asn His Glu
275 280 285
Glu Glu Met Lys Asp Leu Arg Asn Val Ser Thr Gly Asp Val Asn Val
290 295 300
Glu Met Asn Ala Ala Pro Gly Val Asp Leu Thr Gln Leu Leu Asn Asn
305 310 315 320
Met Arg Ser Gln Tyr Glu Gln Leu Ala Glu Gln Asn Arg Lys Asp Ala
325 330 335
Glu Ala Trp Phe Asn Glu Lys Ser Lys Glu Leu Thr Thr Glu Ile Asp
340 345 350
Asn Asn Ile Glu Gln Ile Ser Ser Tyr Lys Ser Glu Ile Thr Glu Leu
355 360 365
Arg Arg Asn Val Gln Ala Leu Glu Ile Glu Leu Gln Ser Gln Leu Ala
370 375 380
Leu Lys Gln Ser Leu Glu Ala Ser Leu Ala Glu Thr Glu Gly Arg Tyr
385 390 395 400
Cys Val Gln Leu Ser Gln Ile His Ala Gln Ile Ser Ala Leu Glu Glu
405 410 415
Gln Leu Gln Gln Ile Arg Ala Glu Thr Glu Cys Gln Asn Thr Glu Tyr
420 425 430
Gln Gln Leu Leu Asp Ile Lys Ile Arg Leu Glu Asn Glu Ile Gln Thr
435 440 445
Tyr Arg Ser Leu Leu Glu Gly Glu Gly Ser Ser Gly Gly Gly Gly Arg
450 455 460
Gly Gly Gly Ser Phe Gly Gly Gly Tyr Gly Gly Gly Ser Ser Gly Gly
465 470 475 480
Gly Ser Ser Gly Gly Gly Tyr Gly Gly Gly His Gly Gly Ser Ser Gly
485 490 495
Gly Gly Tyr Gly Gly Gly Ser Ser Gly Gly Gly Ser Ser Gly Gly Gly
500 505 510
Tyr Gly Gly Gly Ser Ser Ser Gly Gly His Gly Gly Gly Ser Ser Ser
515 520 525
Gly Gly His Gly Gly Ser Ser Ser Gly Gly Tyr Gly Gly Gly Ser Ser
530 535 540
Gly Gly Gly Gly Gly Gly Tyr Gly Gly Gly Ser Ser Gly Gly Gly Ser
545 550 555 560
Ser Ser Gly Gly Gly Tyr Gly Gly Gly Ser Ser Ser Gly Gly His Lys
565 570 575
Ser Ser Ser Ser Gly Ser Val Gly Glu Ser Ser Ser Lys Gly Pro Arg
580 585 590

Tyr

<210> 51

<211> 494

<212> PRT

<213> Homo sapiens

<400> 51
Met Asp Leu Ser Asn Asn Thr Met Ser Leu Ser Val Arg Thr Pro Gly
1 5 10 15
Leu Ser Arg Arg Leu Ser Ser Gln Ser Val Ile Gly Arg Pro Arg Gly
20 25 30
Met Ser Ala Ser Ser Val Gly Ser Gly Tyr Gly Gly Ser Ala Phe Gly
35 40 45
Phe Gly Ala Ser Cys Gly Gly Gly Phe Ser Ala Ala Ser Met Phe Gly
50 55 60
Ser Ser Ser Gly Phe Gly Gly Gly Ser Gly Ser Ser Met Ala Gly Gly
65 70 75 80
Leu Gly Ala Gly Tyr Gly Arg Ala Leu Gly Gly Gly Ser Phe Gly Gly
85 90 95
Leu Gly Met Gly Phe Gly Gly Ser Pro Gly Gly Gly Ser Leu Gly Ile
100 105 110
Leu Ser Gly Asn Asp Gly Gly Leu Leu Ser Gly Ser Glu Lys Glu Thr
115 120 125
Met Gln Asn Leu Asn Asp Arg Leu Ala Ser Tyr Leu Asp Lys Val Arg
130 135 140
Ala Leu Glu Glu Ala Asn Thr Glu Leu Glu Asn Lys Ile Arg Glu Trp
145 150 155 160
Tyr Glu Thr Arg Gly Thr Gly Thr Ala Asp Ala Ser Gln Ser Asp Tyr
165 170 175
Ser Lys Tyr Tyr Pro Leu Ile Glu Asp Leu Arg Asn Lys Ile Ile Ser
180 185 190
Ala Ser Ile Gly Asn Ala Gln Leu Leu Leu Gln Ile Asp Asn Ala Arg
195 200 205
Leu Ala Ala Glu Asp Phe Arg Met Lys Tyr Glu Asn Glu Leu Ala Leu
210 215 220
Arg Gln Gly Val Glu Ala Asp Ile Asn Gly Leu Arg Arg Val Leu Asp
225 230 235 240
Glu Leu Thr Leu Thr Arg Thr Asp Leu Glu Met Gln Ile Glu Ser Leu
245 250 255
Asn Glu Glu Leu Ala Tyr Met Lys Lys Asn His Glu Asp Glu Leu Gln
260 265 270
Ser Phe Arg Val Gly Gly Pro Gly Glu Val Ser Val Glu Met Asp Ala
275 280 285
Ala Pro Gly Val Asp Leu Thr Arg Leu Leu Asn Asp Met Arg Ala Gln
290 295 300
Tyr Glu Thr Ile Ala Glu Gln Asn Arg Lys Asp Ala Glu Ala Trp Phe
305 310 315 320
Ile Glu Lys Ser Gly Glu Leu Arg Lys Glu Ile Ser Thr Asn Thr Glu
325 330 335
Gln Leu Gln Ser Ser Lys Ser Glu Val Thr Asp Leu Arg Arg Ala Phe
340 345 350
Gln Asn Leu Glu Ile Glu Leu Gln Ser Gln Leu Ala Met Lys Lys Ser
355 360 365
Leu Glu Asp Ser Leu Ala Glu Ala Glu Gly Asp Tyr Cys Ala Gln Leu
370 375 380

Ser Gln Val Gln Gln Leu Ile Ser Asn Leu Glu Ala Gln Leu Leu Gln
385 390 395 400
Val Arg Ala Asp Ala Glu Arg Gln Asn Val Asp His Gln Arg Leu Leu
405 410 415
Asn Val Lys Ala Arg Leu Glu Leu Glu Ile Glu Thr Tyr Arg Arg Leu
420 425 430
Leu Asp Gly Glu Ala Gln Gly Asp Gly Leu Glu Glu Ser Leu Phe Val
435 440 445
Thr Asp Ser Lys Ser Gln Ala Gln Ser Thr Asp Ser Ser Lys Asp Pro
450 455 460
Thr Lys Thr Arg Lys Ile Lys Thr Val Val Gln Glu Met Val Asn Gly
465 470 475 480
Glu Val Val Ser Ser Gln Val Gln Glu Ile Glu Glu Leu Met
485 490

<210> 52

<211> 361

<212> PRT

<213> Homo sapiens

<400> 52
Cys Asn Trp Phe Cys Glu Gly Ser Phe Asn Gly Ser Glu Lys Glu Thr
1 5 10 15
Met Gln Phe Leu Asn Asp Arg Leu Ala Ser Tyr Leu Glu Lys Val Arg
20 25 30
His Val Glu Arg Asp Asn Ala Glu Leu Glu Asn Leu Ile Arg Glu Arg
35 40 45
Ser Gln Gln Gln Glu Pro Leu Leu Cys Pro Ser Tyr Gln Ser Tyr Phe
50 55 60
Lys Thr Ile Glu Glu Leu Gln Gln Lys Ile Leu Cys Ser Lys Ser Glu
65 70 75 80
Asn Ala Arg Leu Val Val Gln Ile Asp Asn Ala Lys Leu Ala Ala Asp
85 90 95
Asp Phe Arg Thr Lys Tyr Gln Thr Glu Gln Ser Leu Arg Gln Leu Val
100 105 110
Glu Ser Asp Ile Asn Ser Leu Arg Arg Ile Leu Asp Glu Leu Thr Leu
115 120 125
Cys Arg Ser Asp Leu Glu Ala Gln Met Glu Ser Leu Lys Glu Glu Leu
130 135 140
Leu Ser Leu Lys Gln Asn His Glu Gln Glu Val Asn Thr Leu Arg Cys
145 150 155 160
Gln Leu Gly Asp Arg Leu Asn Val Glu Val Asp Ala Ala Pro Ala Val
165 170 175
Asp Leu Asn Gln Val Leu Asn Glu Thr Arg Asn Gln Tyr Glu Ala Leu
180 185 190
Val Glu Thr Asn Arg Arg Glu Val Glu Gln Trp Phe Ala Thr Gln Thr
195 200 205
Glu Glu Leu Asn Lys Gln Val Val Ser Ser Ser Glu Gln Leu Gln Ser
210 215 220
Tyr Gln Ala Glu Ile Ile Glu Leu Arg Arg Thr Val Asn Ala Leu Glu
225 230 235 240
Ile Glu Leu Gln Ala Gln His Asn Leu Arg Tyr Ser Leu Glu Asn Thr
245 250 255

Leu Thr Glu Ser Glu Ala Arg Tyr Ser Ser Gln Leu Ser Gln Val Gln
260 265 270

Ser Leu Ile Thr Asn Val Glu Ser Gln Leu Ala Glu Ile Arg Ser Asp
275 280 285

Leu Glu Arg Gln Asn Gln Glu Tyr Gln Val Leu Leu Asp Val Arg Ala
290 295 300

Arg Leu Glu Cys Glu Ile Asn Thr Tyr Arg Ser Leu Leu Glu Ser Glu
305 310 315 320

Asp Cys Lys Leu Pro Ser Asn Pro Cys Ala Thr Thr Asn Ala Cys Glu
325 330 335

Lys Pro Ile Gly Ser Cys Val Thr Asn Pro Cys Gly Pro Arg Ser Arg
340 345 350

Cys Gly Pro Cys Asn Thr Phe Gly Tyr
355 360

<210> 53

<211> 3282

<212> DNA

<213> Homo sapiens

<400> 53
atgaaggaga tggtaggagg ctgctgcgta tgttcggacg agaggggctg ggccgagaac 60
ccgctggtct actgcgatgg gcacgcgtgc agcgtggccg tccaccaagc ttgctatggc 120
atcgttcagg tgccaacggg accctggttc tgccggaaat gtgaatctca ggagcgagca 180
gccagggtga ggtgtgagct gtgcccacac aaagacgggg cattgaagag gactgataat 240
ggaggctggg cacacgtggt gtgtgccctc tacatccccg aggtgcaatt tgccaacgtg 300
ctcaccatgg agcccatcgt gctgcagtac gtgcctcatg atcgcttcaa caagacctgt 360
tacatctgcg aggagacggg ccgggagagc aaggcggcct cgggagcctg catgacctgt 420
aaccgccatg gatgtcgaca agctttccac gtcacctgtg cccaaatggc aggcttgctg 480
tgtgaggaag aagtgctgga ggtggacaac gtcaagtact gcggctactg caaataccac 540
ttcagcaaga tgaagacatc ccggcacagc agcgggggag gcggaggagg cgctggagga 600
ggaggtggca gcatgggggg aggtggcagt ggtttcatct ctgggaggag aagccggtca 660
gcctcaccat ccacgcagca ggagaagcac cccacccacc acgagagggg ccagaagaag 720
agtcgaaagg acaaagaacg ccttaagcag aagcacaaga agcggcctga gtcgcccccc 780
agcatcctca ccccgcccgt ggtccccact gctgacaagg tctcctcctc ggcttcctct 840
tcctcccacc acgaggccag cacgcaggag acctctgaga gcagcaggga gtcaaagggg 900
aaaaagtctt ccagccatag cctgagtcat aaagggaaga aactgagcag tgggaaaggt 960
gtgagcagtt ttacctccgc ctcctcttct tcctcctcct cttcctcctc ctctgggggg 1020
cccttccagc ctgcagtctc gtccctgcag agctcccctg acttctctgc attccccaag 1080
ctggagcagc cagaggagga caagtactcc aagcccacag cccccgcccc ttcagcccct 1140
ccttctccct cagctcccga gcccccaag gctgaccttt ttgagcagaa ggtggtcttc 1200
tctggctttg ggcccatcat gcgcttctcc accaccacct ccagctcagg ccgggcccgg 1260
gcgccctccc ctggggacta taagtctccc cacgtcacgg ggtctggggc ctcggcaggc 1320
acccacaaac ggatgcccgc actgagtgcc acccctgtgc ctgctgatga gacccctgag 1380
acaggcctga aggagaagaa gcacaaagcc agcaagagga gccgccatgg gccaggccgt 1440
cccaagggca gccggaacaa ggagggcact ggggggccag ctgccccatc cttgcccagt 1500
gcccagctgg ctggctttac cgccactgct gcctcaccct tctctggagg ttccctggtc 1560
agctccggcc tgggaggtct gtcctcccga acctttgggc cttctgggag cttgcccagc 1620
ttgagcctgg agtcccccctt actaggggca ggcatctaca ccagtaataa ggaccccatc 1680
tcccacagtg gcgggatgct gcgggctgtc tgcagcaccc ctctctcctc cagcctcctg 1740
gggcccccag ggacctcggc cctgccccgc ctcagccgct ccccgttcac cagcaccctc 1800
ccctcctctt ctgcttctat ctccaccact caggtgtttt ctctggctgg ctctaccttt 1860
agcctccctt ctacccacat ctttggaacc cccatgggtg ccgttaatcc cctcctctcc 1920
caagctgaga gcagccacac agagccagac ctggaggact gcagcttccg gtgtcggggg 1980

acctcccctc aggagagtct gtcttccatg tcccccatca gcagcctccc cgcactcttc 2040
gaccagacag cctctgcacc ctgtgggggc ggccagttag acccggcggc cccagggacg 2100
actaacatgg agcagcttct ggagaagcag ggcgacgggg aggccggcgt caacatcgtg 2160
gagatgctga aggcgctgca cgcgctgcag aaggagaacc agcggctgca agagcagatc 2220
ctgagcctga cggccaaaaa ggagcggctg cagattctca acgtgcagct ctctgtgccc 2280
ttccctgccc tgcctgctgc cctgcctgcc gccaacggcc ctgtccctgg gccctatggc 2340
ctgcctcccc aagccgggag cagcgactcc ttgagcacca gcaagagccc tccgggaaag 2400
agcagcctcg gcctggacaa ctcgctgtcc acttcttctg aggacccaca ctcaggctgc 2460
ccgagccgca gcagctcgtc gctgtccttc cacagcacgc cccaccgct gcccctcctc 2520
cagcagagcc ctgccactct gcccctggcc ctgcctgggg cccctgcccc actcccgccc 2580
cagccgcaga acgggttggg ccgggcaccc ggggcagcgg ggctgggggc catgcccatg 2640
gctgaggggc tgttgggggg gctggcaggc agtgggggcc tgcccctcaa tgggctcctt 2700
gggggttga atggggccgc tgccccaac cccgcaagct tgagccaggc tggcggggcc 2760
cccacgctgc agctgccagg ctgtctcaac agccttacag agcagcagag acatctcctt 2820
cagcagcaag agcagcagct ccagcaactc cagcagctcc tggcctcccc gcagctgacc 2880
ccggaacacc agactgttgt ctaccagatg atccagcaga tccagcagaa acgggagctg 2940
cagcgtctgc agatggctgg gggctcccag ctgcccatgg ccagcctgct ggcaggaagc 3000
tccaccccgc tgctgtctgc gggtacccct ggcctgctgc ccacagcgtc tgctccaccc 3060
ctgctgcccg ctggagccct agtggctccc tcgcttggca acaacacaag tctcatggcc 3120
gcagcagctg cagctgcagc agtagcagca gcaggcggac ctccagtcct cactgcccag 3180
accaacccct tcctcagcct gtcgggagca gagggcagtg gcggtggccc caaaggaggg 3240
accgctgaca aaggagcctc agccaaccag gaaaaaggct aa 3282

<210> 54

<211> 2227

<212> DNA

<213> Homo sapiens

<400> 54
gagagcccga acaggaagag ggtacagctt tgtgcaggtc acatgcccac tgcagccctc 60
cagcctctgg tccccagagc ggactttgga agctgaactg cttttgttgc tggaagactt 120
atgttataat ttaccctggg tggaccaggg tcgtacaaaa gggcaacgct ccccagtccc 180
cccactcccg accccggaat catgcatcgg actacacgga tcaaaatcac agagctgaac 240
ccccacctca tgtgtgccct ctgcgggggg tacttcatcg acgccaccac tatcgtggag 300
tgcctgcatt ccttctgcaa aacctgcatc gtgcgctacc tggagaccaa caaatactgc 360
cccatgtgtg acgtgcaggt ccataaaacc cggccgctgc tgagcatcag gtctgacaaa 420
acacttcaag acattgtcta caaattggtc cctgggcttt ttaaagatga gatgaaacgg 480
cggcgggatt tctatgcagc gtacccctg acggaggtcc ccaacggctc caatgaggac 540
cgcggcgagg tcttggagca ggagaagggg gctctgagtg atgatgagat tgtcagcctc 600
tccatcgaat tctacgaagg tgccagggac cgggatgaga agaagggccc cctggagaat 660
ggggatgggg acaaagagaa aacaggggtg cgcttcctgc gatgcccagc agccatgacc 720
gtcatgcatc ttgccaagtt tctccgcaac aagatggatg tgcccagcaa gtacaaggtg 780
gaggttctgt acgaggacga gccactgaag gaatactaca ccctcatgga catcgcctac 840
atctacccct ggcggcggaa cgggcctctc cccctcaagt accgtgtcca gccagcctgc 900
aagcggctca ccctagccac ggtgcccacc ccctccgagg gcaccaacac cagcggggcg 960
tccgagtgtg agtcagtcag cgacaaggct cccagccctg ccaccctgcc agccacctcc 1020
tcctccctgc ccagcccagc caccccatcc catggctctc ccagttccca tgggcctcca 1080
gccacccacc ctacctcccc cactccccct tcgacagcca gtggggccac cacagctgcc 1140
aacgggggta gcttgaactg cctgcagaca ccatcctcca ccagcagggg gcgcaagatg 1200
actgtcaacg gcgctcccgt gcccccctta acttgaggcc agggaccctc tcccttcttc 1260
cagccaagcc tctccactcc ttccactttt tctgggccct tttttccact tcttctactt 1320
tccccagctc ttcccacctt gggggtgggg ggcgggtttt ataaataaat atatatatat 1380
atgtacatag gaaaaaccaa atatacatac ttattttcta tggaccaacc agattaattt 1440
aaatgccaca ggaaacaaac tttatgtgtg tgtgtatgtg tggaaaatgg tgttcatttt 1500

ttttgggggg ggtcttgtgt aatttgctgt ttttggggggt gcctggagat gaactggatg 1560
ggccactgga gtctcaataa agctctgcac catcctcgct gtttcccaag gcaggtggtg 1620
tgttgggggc cccttcagac ccaaagcttt aggcatgatt ccaactggct gcatatagga 1680
gtcagttaga attgtttctt tctctccccg tttctctccc catcttggct gctgtcctgc 1740
ctctgaccag tggccgcccc ccgcgttgtt gaatgtccag aaattgctaa gaacagtgcc 1800
ttttacaaat gcagtttatc cctggttctg aggagcaagt gcagggtgga ggtggcacct 1860
gcatcacctc ctcctcttgc agtggaaact ttgtgcaaag aatagatagt tctgcctctt 1920
tttttttttt ttcctgtgtg tgtggccttt gcatcattta tcttgtggaa aagaagattc 1980
aggccctgag aggtctcagc tcttggagga gggctaaggc tttagcattg tgaagcgctg 2040
cacccccacc aaccttaccc tcaccgggga accctcacta gcaggactgg tggtggagtc 2100
tcacctgggg cctagagtgg aagtgggggt gggttaacct cacacaagca cagatcccag 2160
actttgccag aggcaaacag ggaattccgc cgatactgac gggctccagg agtcgtcgcc 2220
acactcg 2227

<210> 55

<211> 4283

<212> DNA

<213> Homo sapiens

<400> 55
ttgcgggaaa gagccaaacc ctggcgttgg ggggcccggg cggggagccc ctcccgcggt 60
ccacagcgac gcctgcccag ccctcctccc cttccggctc cggcacgggg ccccgaggcg 120
ttcggaggcc aggcgggttt ctgtcaggcc cggggaggag gggcgggcgg ggcggccgct 180
gcctccccgg gacgggccgt accacgcgga cggggaggac ggggccaggg gactgcaggg 240
cggctgcacc gcccggggc ggggtgcgga gcgggccggc gggctccccg gggcggggcg 300
ggagggcggg gcgtggggcg gacggaacca ccggggcggg gtgggaggta acgggacggg 360
cgcgaccatg gcgcggtgag ggagcggggg tggggatcgg tccgggggag gcctgaggcc 420
gctggcttgt gcgctgtctc cgccgccccc ctctttcgcc gccgccgccg ccgccccggg 480
catgtcgtcc aactgcacca gcaccacggc ggtggcggtg gcgccgctca gcgccagcaa 540
gaccaagacc aagaagaagc atttcgtgtg ccagaaagtg aagctattcc gggccagcga 600
gccgatcctc agcgtcctga tgtggggggt gaaccacacg atcaatgagc tgagcaatgt 660
tcctgttcct gtcatgctaa tgccagatga cttcaaagcc tacagcaaga tcaaggtgga 720
caatcatctc ttcaataagg agaacctgcc cagccgcttt aagtttaagg agtattgccc 780
catggtgttc cgaaaccttc gggagaggtt tggaattgat gatcaggatt accagaattc 840
agtgacgcgc agcgccccca tcaacagtga cagccagggt cggtgtggca cgcgtttcct 900
caccacctac gaccggcgct ttgtcatcaa gactgtgtcc agcgaggacg tggcggagat 960
gcacaacatc ttaaagaaat accaccagtt tatagtggag tgtcatggca acacgctttt 1020
gccacagttc ctgggcatgt accgcctgac cgtggatggt gtggaaacct acatggtggt 1080
taccaggaac gtgttcagcc atcggctcac tgtgcatcgc aagtatgacc tcaagggttc 1140
tacggttgcc agagaagcga gcgacaagga gaaggccaag gacttgccaa cattcaaaga 1200
caatgacttc ctcaatgaag ggcagaagct gcatgtggga gaggagagta aaaagaactt 1260
cctggagaaa ctgaagcggg acgttgagtt cttggcacag ctgaagatca tggactacag 1320
cctgctggtg ggcatccacg acgtggaccg ggcagagcag gaggagatgg aggtggagga 1380
gcgggcagag gacgaggagt gtgagaatga tggggtgggt ggcaacctac tctgctccta 1440
tggcacacct ccggacagcc ctggcaacct cctcagcttt cctcggttct ttggtcctgg 1500
ggaattcgac ccctctgttg acgtctatgc catgaaaagc catgaaagtt cccccaagaa 1560
ggaggtgtat ttcatggcca tcattgatat cctcacgcca tacgatacaa agaagaaagc 1620
tgcacatgct gccaaaacgg tgaaacacgg ggcaggggcc gagatctcga ctgtgaaccc 1680
tgagcagtac tccaaacgct tcaacgagtt tatgtccaac atcctgacgt agttctcttc 1740
taccttcagc cagagccaga gagctggata tggggtcggg gatcgggagt tagggagaag 1800
ggtgtatttg ggctagatgg gagggtggga gcagagtcgg gtttgggagg gctttagcaa 1860
tgagactgca gcctgtgaca ccgaaagaga ctttagctga agaggagggg gatgtgctgt 1920
gtgtgcacct gctcacagga tgtaacccca ccttctgctt accettgatt ttttctcccc 1980
atttgacacc caggttaaaa aggggttccc tttttggtac cttgtaacct tttaagatac 2040

```
cttggggcta gagatgactt cgtgggttta tttgggtttt gtttctgaaa tttcattgct   2100
ccaggtttgc tatttataat catatttcat cagcctaccc accctcccca tctttgctga   2160
gctctcagtt cccttcaatt aaagagatac ccagtagacc cagcacaagg gtccttccag   2220
aaccaagtgc tatggatgcc agattggaga ggtcagacac ctcgccctgc tgcatttgct   2280
cttgtctgga ttaactttgt aatttatgga gtattgtgca caacttcctc cacctttccc   2340
ttggattcaa gtgaaaactg ttgcattatt cctccatcct gtctggaata caccaggtca   2400
acaccagaga tctcagatca gaatcagaga tctcagaggg gaataagttc atcctcatgg   2460
gatggtgagg ggcaggaaag cggctgggct cttggacacc tggttctcag agaaccctgt   2520
gatgatcacc caagccccag gctgtcttag cccctggagt tcagaagtcc tctctgtaaa   2580
gcctgcctcc cactaggtca agaggaacta gagtaccttt ggatttatca ggaccctcat   2640

gtttaaatgg ttatttccct ttgggaaaac ttcagaaact gatgtatcaa atgaggccct   2700
gtgccctcga tctatttcct tcttccttct gacctcctcc caggcactct tacttctagc   2760
cgaactctta gctctgggca gatctccaag cgcctggagt gctttttagc agagacacct   2820
cgttaagctc cgggatgacc ttgtaggaga tctgtctccc tgtgcctgga gagttacagc   2880
cagcaaggtg cccccatctt agagtgtggt gtccaaacgt gaggtggctt cctagttaca   2940
tgaggatgtg atccaggaaa tccagtttgg aggcttgatg tgggttttga cctggcctca   3000
gccttggggc tgtttttcct tgttgccccg ctctagactt ttagcagatc tgcagcccac   3060
aggctttttt ggaaggagtg gcttcctgca ggtgttccac ctgccttcgg agcctgccac   3120
ccaggccctc agaactgagc cacaggctgc tctggccagg agagaaacag ctctgttgtt   3180
ctgcattggg ggaggtacat tcctgcatct tctcaccccc tcaaccagga actggggatt   3240
tgggatgaga tatggtcaga cttgtagata accccaaaga tgtgaagatc gcttgtgaaa   3300
ccattttgaa tgaatagatt ggtttcctgt ggctccctcc aaacctggcc aagcccagct   3360
tccgaagcag gaaccagcac tgtctctgtg cctgactcac agcatatagg tcaggaaaga   3420
atggagacgg cattcttgga cttcactggg gctgctggat tggatgggaa accttctgga   3480
agaggcagat gggggtcaaa ccactgcctt ggccccagga aggggccata ggtaggtctg   3540
aacaactgcc gcaagaccac tacatgactt agggaacttg aaaccaactg gctcatggag   3600
aaaacaaatt tgacttggga aagggattat gtaggaataa tgtttggact tgatttcccc   3660
acgtcataat gaagaatgga agtttggatc tgctcctcgt caggcgcagc atctctgaag   3720
cttggaaagc tgtcttccag cctccaaacc tggccaagcc cagcttccga agcaggaacc   3780
agcactgtct ctgtgcctga ctcacagcat ataggtcagg aaagaatgga gacggcattc   3840
ttggacttca ctggggctgc tggattggat gggaaacctt ctggaagagg cagatggggg   3900
tcaaaccact gccttggccc caggaagggg ccataggtag gtctgaacaa ctgccgcaag   3960
accactacat gacttaggga acttgaaacc aactggctca tggagaaaac aaatttgact   4020
tgggaaaggg attatgtagg aataatgttt ggacttgatt tccccacgtc ataatgaaga   4080
atggaagttt ggatctgctc ctcgtcaggc gcagcatctc tgaagcttgg aaagctgtct   4140
tccagcagcc tccgtggcct cgggttccta ccggcttctc tgcatttggt ctgctgatca   4200
tgttgccata atgtgtatgg aaagtgtaac acattcttac tggttaaaga cgactaccag   4260
gtatctaact tgtttaacat tga                                           4283
```

<210> 56

<211> 6140

<212> DNA

<213> Homo sapiens

<400> 56

```
gcggccgcag cctgagccag ggccccctcc ctcgtcagga ccgggggcagc aagcaggccg    60
ggggcaggtc cgggcaccca ccatgcgagg cgagctctgg ctcctggtgc tggtgctcag   120
ggaggctgcc cgggcgctga gcccccagcc cggagcaggt cacgatgagg gcccaggctc   180
tggatgggct gccaaaggga ccgtgcgggg ctggaaccgg agagcccgag agagccctgg   240
gcatgtgtca gagccggaca ggacccagct gagccaggac ctgggtgggg gcaccctggc   300
catggacacg ctgccagata acaggaccag ggtggtggag gacaaccaca gctattatgt   360
gtcccgtctc tatggcccca gcgagcccca cagccgggaa ctgtgggtag atgtggccga   420
ggccaaccgg agccaagtga agatccacac aatactctcc aacacccacc ggcaggcttc   480
```

gagagtggtc ttgtcctttg atttcccttt ctacgggcat cctctgcggc agatcaccat 540
agcaactgga ggcttcatct tcatgggga cgtgatccat cggatgctca cagctactca 600
gtatgtggcg cccctgatgg ccaacttcaa ccctggctac tccgacaact ccacagttgt 660
ttactttgac aatgggacag tctttgtggt tcagtgggac cacgtttatc tccaaggctg 720
ggaagacaag ggcagtttca ccttccaggc agctctgcac catgacggcc gcattgtctt 780
tgcctataaa gagatcccta tgtctgtccc ggaaatcagc tcctcccagc atcctgtcaa 840
aaccggccta tcggatgcct tcatgattct caatccatcc ccggatgtgc cagaatctcg 900
gcgaaggagc atctttgaat accaccgcat agagctggac cccagcaagg tcaccagcat 960
gtcggccgtg gagttcaccc cattgccgac ctgcctgcag cataggagct gtgacgcctg 1020
catgtcctca gacctgacct tcaactgcag ctggtgccat gtcctccaga gatgctccag 1080
tggctttgac cgctatcgcc aggagtggat ggactatggc tgtgcacagg aggcagaggg 1140
caggatgtgc gaggacttcc aggatgagga ccacgactca gcctcccctg acacttcctt 1200
cagcccctat gatggagacc tcaccactac ctcctcctcc ctcttcatcg acagcctcac 1260
cacagaagat gacaccaagt tgaatcccta tgcaggagga gacggccttc agaacaacct 1320
gtcccccaag acaaagggca ctcctgtgca cctgggcacc atcgtgggca tcgtgctggc 1380
agtcctcctc gtggcggcca tcatcctggc tggaatttac atcaatggcc accccacatc 1440
caatgctgcg ctcttcttca tcgagcgtag acctcaccac tggccagcca tgaagtttcg 1500
cagccacccct gaccattcca cctatgcgga ggtggagccc tcgggccatg agaaggaggg 1560
cttcatggag gctgagcagt gctgagaaca ccaagtctcc cctttgaaga ctttgaggcc 1620
acagaaaaga cagttaaagc aaagaagaga agtgactttt cctggcctct cccagcatgc 1680
cctgggctga gatgagatgg tggtttatgg ctccagagct gctgttcgct tcgtcagcac 1740
accccgaata ttgaagaggg ggccaaaaaa caaccacatg gatttttat aggaacaaca 1800
acctaatctc atcctgtttt gatgcaaggg ttctcttctg tgtcttgtaa ccatgaaaca 1860
gcagaagaac taacataact aactccattt ttgtttaagg ggcctttacc tattcctgca 1920
cctaggctag gataacttta gagcactgac ataaaacgca aaaacaggaa tcatgccgtt 1980
tgcaaaacta actctgggat taaaggggaa gcatgtaaac agctaactgt ttttgttaaa 2040
gatttatagg aatgaggagg tttggctatt gtcacatgac agactgttag ccaaggacaa 2100
agaagttctg caaacctccc ctggaccctt gctggtgtcc agatgtctgc ggttgtcagc 2160
cccttccttt cccccgacct aaacataaaa gacaaggcaa agcccgcata attttaagac 2220
ggttctttag gacattagtc caccatcttc ttggtttgct ggctctccga aataaagtcc 2280
ctttccttgc tccaactcct tgtctctcaa cgtattggct atgacgcagc aagcagaatg 2340
aatttggact cagttacagg ctgtcaatgg tctgctctgt agcagtctca gagcctcccc 2400
gacccactac ctggagatag ccagatagcc agatgccctg ctcctggcca cctttaaagc 2460
ccctgcatat gacacaggtt aactaaagtc aagattgggg ctgctgcatt ccaggttccc 2520
tagactcaca agctggtcct tggccaggtg cagtggctca cgcctgtaat cccagcactt 2580
tgggaggctg aggcaggcgg atcacctgaa gtcagaagtt tgagaccagc ctggccaaca 2640
taattaaaat gtctctacta aaaatacaaa aaattagctg ggtgtggtga cgcttgcctg 2700
tatcccagct actcaggaag ctgagacacg agaatcactt gaacctggga ggcagaggtt 2760
gcagtgagct cagatagtgc cactgcactc cagcctgggt gacagagcga gactccgtct 2820
caaaaaaaaa aaaagaaagc agaacctcat ggctatagag ttggcatttt agccccagct 2880
tctgtagctc tgaaagccta aagaaggtat tctctccatc tgttaaacac agtatagtgg 2940
ctctcagccc ttggggcatg ttatcatggg agggaagtca aataagagga gagaaaagaa 3000
ctcaaggggg aaactgcatt tttaggcttt gctctcttac cttgcccttt ctactcagaa 3060
ccaataactt ctgcatcaaa acatgttaca gcctgcatca agggctttac ccccaacctgc 3120
agcccagcct tccctgggtg agcttgctat gcgcagccac atttaccatg tggggctccc 3180
tattctgatg gcctgttcgg tgccgggttt actcactgcc ctgttctgat gtcagtgcct 3240
gtacatacct ccaaaggcag gacttgcctg ataaatattt ttcctcctct gaactggatt 3300
ttataggcat taaagacaag tcgggtggct agaggctcc ttgagacata cctagcaggg 3360
aactgcaggt ggattctgtt gagaggcaaa gcacctgagt ggttgggaca caggcagctg 3420
gcatgggagg gacttttttt gagacagggt ctcactgtgt cgcccagggc aaggatgccc 3480
aaagacacca ggttggagag gcacctgcca actacttgct ttccctggag cctgcatgtg 3540
cctgtggggt ggggaggcgt aggggtctac ggctgcctga gatgggtgtg cacagtgtgt 3600
gaagtaccta cctccttgcc ttgctggact gtcagccagt cgcagggccg gccacaagac 3660
ccatgtctcc atctggtcat actccatagc taccaagtta acctgctcta aactttggag 3720
aactggatct gtccaataaa cgcttatttg gccaagcctg atggctcgtg cctgtactcc 3780
cagcactttg ggaggctgag gtgggagggt tgcttgagcc caggggtttg agaccagctt 3840
gggcaacaac aacaaaaatg ccaggtgtgg tggggtgcac ctgtagtccc agctactagg 3900
gaggctgagc caggaggatc acttgagccc gggaggttga ggctgcagtg gggggtcata 3960
atcatgccac tgtactccag cctgggtgac agagtgagac cctgtctccg aaaaaaaaaa 4020

aaaaaaaaga acggaaaaag aaatgcttac attgtcaggg atcctgtaga caatcattaa 4080
ctctatgaga tgcttggttc tatttttttg ggagactttg tccaagtgtt ttggcttaag 4140
aaatccatag gcctctcttg gtgacacatc tctagtactt tttgtcataa acaaacaggc 4200
catctgccgc caaatacatc cactccccat gccactgaca tcctatgggt cagccaggct 4260
tgctttgact gaggccgagg catctggaac tttctctgcc tgcaggggct agcagcagag 4320
gcttcaccgc atcaccaccc cttcctccac tcctgacatt ctttcccttc agggatccaa 4380
aatggttggc cgagctccca gtgggaaaac gtgtgctaga gttggggagt gagatgagtg 4440
gtgctgtcca tggaatcagg ccacagcagg aactgcccca ctggccattt gagacacaca 4500
caggtggtaa atgctctgct ggtgggctgt gcttccctca ttcagagagc tctgttacag 4560
cccactgtgt cctttagaag cttgaaagga acccaactct ttgctgcact gtcctttttc 4620
ttcctcaaat tcagaccctc cttccaccgg caccccccta ctccaccctc agctcttcct 4680
tgcctggttt atcaagcaga gctgaggccc cacgtttcca actctgattg tcacttgcat 4740
cttcacaaag gataaaccac ggagcaactg gaaaaccatc agccaagcgt tcggatgagt 4800
ctggttattg gtccaccccc gaccagattc ccttacactt aactcacttc tttctttggc 4860
aatgaccctc atgacatgta taaatgggta tgactaagaa gaggctgtga tctaacattt 4920
atttgctgcc attttttact ctggggagaa gcagccccaa ctcatcactg ggaaagaact 4980
ccccctgcaa accagctaaa tttgataatt taaaccccct gcccctaaaa cttctcacag 5040
agctggggag ttggtggcaa ctttccaagt caaggtcttg cttagaaagt ccttcactac 5100
atggccaggt gcagtggctc acgcctgtag tcccaggtac ttgggagcct gaggcaggag 5160
gattgcttga gctcaggagt tcaaggctgc agagagctat gatcatccca ctgcatttgt 5220
ttaaaaataa atttttaaaa tttgtgtgtt ttatcagggg tctcctgtac agtgtatctg 5280
tgtatgtttg tgtgtgtgtt tgtatacagc cttgtttaat gttttgagca ataagatatg 5340
cacacacagg tattttgttg ctaaagagat tggacaaggt tgtagctgtg ctcaggcttc 5400
agcttggttt gttaaattga gagataaaca atgacaagag ctgccagcca accacactat 5460
tcaaaaagca aagtgttcac cactaaagct aaccattcat ctggttgcag gcaaggctaa 5520
ggctctctct cctctagttc ctggaacaga ctcacagatt ggcatgaagc actgatcagg 5580
ggctgcactc agactccctg gccaagcaaa cctacaccag aagagtcagt gtcacagata 5640
tgatgcggcc aatctctgtc tccaaaaacc tacctgaact taatggtaga attcaaagat 5700
ctggggactg agggcaccca gccttctaaa acacaatgta ttcatgtgtt tagtgtaaac 5760
tctctgcatg gattctcagt gttaataata aaaggaagca ttcttttaca actcctgctg 5820
tgtgcaaaag aaagtgcaaa ggatttggag tggcattccg aagatcacca cacatacctt 5880
ggttctgatg gctgctgaac tccgacttct tcgctgagac atgactgtgg gaacagcctc 5940
cagctatctg ctcatcagag gtgctttcct caacctcctg caccacctcc aagagaaaca 6000
gcctaaaaag aaaccccagc tgtttactta tattggtctg taaatccctg gaagtaaacc 6060
ccatgcattt ttatctactg tctgaggaca tacaataaat ctgagaaagt ctatgctgtc 6120
aaaaaaaaaa aaaaaaaaaa 6140

<210> 57

<211> 2098

<212> DNA

<213> Homo sapiens

<400> 57
gcaggagcac gtggagaggc cgggtagcca cagcggcagc tccagcccgg cccggcagcg 60
acatggaaga tatacaaaca aatgcggaac tgaaaagcac tcaggagcag tctgtgcccg 120
cagaaagtgc agcggttttg aatgactaca gtttaaccaa atctcatgaa atggaaaatg 180
tggacagtgg agaaggccca gccaatgaag atgaagacat aggagatgat tcaatgaaag 240
tgaaagatga atacagtgaa agagatgaga atgttttaaa gtcagaaccc atgggaaatg 300
cagaagagcc tgaaatccct tacagctatt caagagaata taatgaatat gaaaacatta 360
agttggagag acatgttgtc tcattcgata gtagcaggcc aaccagtgga aagatgaact 420
gcgatgtgtg tggattatcc tgcatcagct tcaatgtctt aatggttcat aagcgaagcc 480
atactggtga acgcccattc cagtgtaatc agtgtggggc atcttttact cagaaaggta 540
acctcctccg ccacattaaa ctgcacacag gggaaaaacc ttttaagtgt cacctctgca 600
actatgcatg ccaaagaaga gatgcgctca cggggcatct taggacacat tctgtggaga 660

```
aaccctacaa atgtgagttt tgtggaagga gttacaagca gagaagttcc cttgaggagc    720
acaaggagcg ctgccgtaca tttcttcaga gcactgaccc aggggacact gcaagtgcgg    780
aggcaagaca catcaaagca gagatgggaa gtgaaagagc tctcgtactg gacagattag    840
caagcaatgt ggcaaaacga aaaagctcaa tgcctcagaa attcattggt gagaagcgcc    900
actgctttga tgtcaactat aattcaagtt acatgtatga gaaagagagt gagctcatac    960
agacccgcat gatggaccaa gccatcaata acgccatcag ctatcttggc gccgaagccc   1020
tgtgcccctt ggtccagaca ccgcctgctc ccacctcgga gatggttcca gttatcagca   1080
gcatgtatcc catagccctc acccgggctg agatgtcaaa cggtgcccct caagagctgg   1140
aaaggaaaag catcctcctt ccagagaaga gcgtgccttc tgagagaggc ctctctccca   1200
acaatagtgg ccacgactcc acggacactg acagcaacca tgaagaacgc cagaatcaca   1260
tctatcagca aaatcacatg gtcctgtctc gggcccgcaa tgggatgcca cttctgaagg   1320
aggttccccg ctcttacgaa ctcctcaagc ccccgcccat ctgcccaaga gactctgtca   1380
aagtgatcga caaggaaggg gaggtgatgg atgtgtatcg gtgtgaccac tgccgcgtcc   1440
tcttcctgga ctatgtgatg ttcacgattc acatgggctg ccacggcttc cgtgaccctt   1500
tcgagtgtaa catgtgtgga gatcgaagcc atgatcggta tgaattctcg tctcacatag   1560
ccagaggaga acacagaagc ctgctgaagt gaatatctgg tctcagggat tgctcctatg   1620
tattcagcat cgtttctaaa aacagttgac ctcgcctaac agattgctct caaaacatac   1680
tcagttccaa acttcttttc ataccatttt tagctgtgtt cacaggggta gccagagaaa   1740
cactgtcttc cttcagaaat tattcgcagg tctagcatat tattactttt gtgaaacctt   1800
tgtttcccca tcagggactt gaattttatg gaatttaaaa gccaaaaagg tatttggtca   1860
ttatcttcta cagcagtgga atgagtggtc ccggagatgt gctatatgaa acattctttc   1920
tgagatatat caaccacacg tggaaaagcc tttcagtcat acatgcaaat ccacaaagag   1980
gaagagctga ccagctgacc ttgctgggaa gcctcaccct tctgcccttc acaggctgaa   2040
gggttaagat ctaatctccc taatctaaat gacagtctaa gagtaagtaa aagaacag     2098
```

<210> 58

<211> 2947

<212> DNA

<213> Homo sapiens

<400> 58

```
atgccaattc ctcctccccc gccaccccca cctggtcctc ctccacctcc cacatttcat      60
caggcaaaca cagagcagcc caagctgagt agagatgagc agcggggtcg aggcgccctc    120
ttacaggaca tttgcaaagg gaccaagctg aagaaggtga ccaacattaa tgatcggagt    180
gctcccatcc tcgagaagcc gaaaggaagc agtggtggct atggctctgg aggagctgcc    240
ctgcagccca agggaggtct cttccaagga ggagtgctga agcttcgacc tgtgggagcc    300
aaggatggtt cagagaacct agctggtaag ccagccctgc aaatccccag ttctcgagct    360
gctgccccaa ggcctccagt atctgccgcc agcggcgtc ctcaggatga tacagacagc     420
agccgggcct cactcccaga actgccccgg atgcagagac cctctttacc ggacctctct    480
cggcctaata ccaccagcag tacgggcatg aagcacagct cctctgcccc tcccccacca    540
cccccagggc ggcgtgccaa cgcacccccc acacctctgc ctatgcacag cagcaaagcc    600
cccgcctaca acagagagaa acccttgcca ccgacgcctg gacaaaggct tcaccctggt    660
cgagagggac ctcctgctcc acccccagtc aaaccacctc cttcccctgt gaatatcaga    720
acaggaccaa gtggccagtc tctggctcct cctcctccgc cttaccgcca gcctcctggg    780
gtccccaatg gaccctctag ccccactaat gagtcagccc ctgagctgcc acagagacac    840
aattctttgc ataggaagac accagggcct gtcagaggcc tagcacctcc tccacccacc    900
tcggcctccc catctttact gagtaatagg ccacctcccc cagcccgaga ccctcccagt    960
cggggagcag ctcctccacc cccaccacct gtgatccgaa atggtgccag ggatgctccc   1020
cctcccccac caccataccg aatgcatggg tcagaacccc cgagccgagg aaagccccca   1080
cctccaccct caaggacgcc agctgggcca ccccctcctc ctccaccgcc cctgaggaat   1140
ggccacagag attctatcac cactgtccgg tctttcttgg atgattttga gtcaaagtat   1200
tccttccatc cagtagaaga ctttcctgct ccagaagaat ataaacactt tcagaggata   1260
tatcccagca aaacaaaccg agctgcccgt ggagccccac ctctgccacc cattctcagg   1320
tgaagcctgg cttggtcccg ttcctcagga aaaggatgga ccttctcttc ttctcagatg   1380
```

gtcccttcca ttcccctgaa acctgcatga gagctcctaa catgtttctc caatgcaatc 1440
aagccctaga ctccaaatgt cctcccagct cacctccatc tatgcatctc atctctggat 1500
ttggtgatca gactctatat tgacagtagg atctcaaacc ctgcatccat ccttcctcca 1560
gcaagccctg ctagccacat gaggaacaag tttccgtgtc ttctgccttc ctcttgggga 1620
aaggtgcctt gttgtgatga attaactcac tgttagggca gggtggagaa tggtactcct 1680
tccttctcct gtccactgtg ggggaagctt ggcaggtata ttatatttca tcatttagga 1740
ggctggcatg accaggactt atgggtggga ggggagcatt tttagtgaag caagaaagga 1800
gtttgccaag aagtgatctg ttttaaaggt catatttgga gaaagggcaa ggaattgggt 1860
ctgctttatt tttgggggta ttttgttttt gttctcacct gctgcccccc caccccacca 1920
ccccagggat aaattggata taaacactaa atactaatca gttgaactta acatttaata 1980
aaaagaaagg gtgaaataaa ctgaagacca ttttagaact agtcagttct ctgcagcaaa 2040
gggaacagga gccatttgaa ccctctggga cccctcaccc cactgcttca gggtgctagg 2100
ctgagggatg tttttcctcc cccttaccgc ccatgcccctt gaaagaaaag tcactttttg 2160
tggagggcat cattcattcc tgattcacaa accccaaaaa cctctggtgg gagataggaa 2220
gatagggcgt gggcctgggc cttaacctca atcttgtgtc tgcctcagtc ttttctgact 2280
ggccctgaag ttgtcagtgg ctctttctgt ccttcagccc ctggaaggtg ctccaggata 2340
acaaagaagg gcaggttgaa gcccctcatg gaaggagctg gctttgtggg gctgcaaagg 2400
acttttaagt cctgcctgta ctgaagttca cagcccacct gactgagcag actcttcctg 2460
ttcctttctc taccaccctt gccttcccag gactgcacgg tttaacacag cagagtacag 2520
aagggtgaag aagtgagcag aggcttatga agatattcag atactcttct atgccaggaa 2580
gcacaaagac tttgttgaga tttgcctcag ttcagtagat cttccttggc agccagccat 2640
aggttgtttc tttgtcttcc gggtcctaaa gagcacagag aaaatggagg tccccagtct 2700
aggtaggaag ctgattggat gaggacttct ttttttccga cagcaggatg gggctcttgg 2760
gctccacaca ccagatgctt tggttttcta caactgttgc tatgtgtaga gggtgctcag 2820
agcgtggcat gagagcaagg agaccatggc tactctttga aatggatggg gaaaattagc 2880
ttaaaaattt aatcacgaga ttgcgccact gcactccagc ctgggcgaca gagccagact 2940
ccgtctc 2947

<210> 59

<211> 784

<212> DNA

<213> Homo sapiens

<400> 59
gagcggttgc gcagtgaagg ctagaccccgg tttactggaa ttgctctggc gatcgagggg 60
tcctagtaca ccgcaatcat gtctattatg tcctataacg gaggggccgt catggccatg 120
aaggggaaga actgtgtggc catcgctgca gacaggcgct tcgggatcca ggcccagatg 180
gtgaccacgg acttccagaa gatctttccc atgggtgacc ggctgtacat cggtctggcc 240
gggctcgcca ctgacgtcca gacagttgcc cagcgcctca agttccggct gaacctgtat 300
gagttgaagg aaggtcggca gatcaaacct tataccctca tgagcatggt ggccaacctc 360
ttgtatgaga aacggtttgg cccttactac actgagccag tcattgccgg gttggacccg 420
aagaccttta agcccttcat ttgctctcta gacctcatcg gctgccccat ggtgactgat 480
gactttgtgg tcagtggcac ctgcgccgaa caaatgtacg gaatgtgtga gtccctctgg 540
gagcccaaca tggatccgga tcacctgttt gaaaccatct cccaagccat gctgaatgct 600
gtggaccggg atgcagtgtc aggcatggga gtcattgtcc acatcatcga gaaggacaaa 660
atcaccacca ggacactgaa ggcccgaatg gactaaccct gttcccagag cccacttttt 720
tttcttttt tgaaataaaa tagcctgtct ttcaaaaaaa aaaaaaaaaa aaaaaaaaaa 780
aaaa 784

<210> 60

<211> 3033

<212> DNA

<213> Homo sapiens

<400> 60
atactcctaa gctcctcccc cggcggcgag ccagggagaa aggatggccg gcctggcggc 60
gcggttggtc ctgctagctg gggcagcggc gctggcgagc ggctcccagg gcgaccgtga 120
gccggtgtac cgcgactgcg tactgcagtg cgaagagcag aactgctctg gggcgctct 180
gaatcacttc cgctcccgcc agccaatcta catgagtcta gcaggctgga cctgtcggga 240
cgactgtaag tatgagtgta tgtgggtcac cgttgggctc tacctccagg aaggtcacaa 300
agtgcctcag ttccatggca agtggccctt ctcccggttc ctgttctttc aagagccggc 360
atcggccgtg gcctcgtttc tcaatggcct ggccagcctg gtgatgctct gccgctaccg 420
caccttcgtg ccagcctcct cccccatgta ccacacctgt gtggccttcg cctgggtgtc 480
cctcaatgca tggttctggt ccacagtctt ccacaccagg gacactgacc tcacagagaa 540
aatggactac ttctgtgcct ccactgtcat cctacactca atctacctgt gctgcgtcag 600
gtgagcctgc ctgggtggct gcaggggcaa aatcgaaccc tgggggcaga aaggggtcac 660
ccagccttcc cctgggggcc ttcttcacta gtctcccaac acctacgccc cccaaccccc 720
aacacatcag ctgtcctggg tgaggactct ggggtaggac tggggccct ggctcctgac 780
aaggagctgt agcacttgct gcccagctgt ggcctgtttg gtggggagag ggtagtgac 840
ttcaggggcc atgcaccaat gttgggggga ggagatgctt cagggaatgc tgctctgggg 900
atgggccacc tgccctctga gcaaccctgg acggtggggc aggaccgtgg ggctgcagca 960
cccagctgtg gtcagtgcct tccgggctct cctgctgctc atgctgaccg tgcacgtctc 1020
ctacctgagc ctcatccgct tcgactatgg ctacaacctg gtggccaacg tggctattgg 1080
cctggtcaac gtggtgtggt ggctggcctg gtgcctgtgg aaccagcggc ggctgcctca 1140
cgtgcgcaag tgcgtggtgg tggtcttgct gctgcagggg ctgtccctgc tcgagctgct 1200
tgacttccca ccgctcttct gggtcctgga tgcccatgcc atctggcaca tcagcaccat 1260
ccctgtccac gtcctctttt tcagctttct ggaagatgac agcctgtacc tgctgaagga 1320
atcagaggac aagttcaagc tggactgaag accttggagc gagtctgccc cagtggggat 1380
cctgcccccg ccctgctggc ctcccttctc ccctcaaccc ttgagatgat ttctcttttt 1440
caacttcttg aacttggaca tgaaggatgt gggcccagaa tcatgtggcc agcccacccc 1500
ctgttggccc tcaccagcct tggagtctgt tctagggaag gcctcccagc atctgggact 1560
cgagagtggg cagcccctct acctcctgga gctgaactgg ggtggaactg agtgtgttct 1620
tagctctacc gggaggacag ctgcctgttt cctccccacc agcctcctcc ccacatcccc 1680
agctgcctgg ctgggtcctg aagccctctg tctacctggg agaccaggga ccacaggcct 1740
tagggataca gggggtcccc ttctgttacc acccccacc ctcctccagg acaccactag 1800
gtggtgctgg atgcttgttc tttggccagc caaggttcac ggcgattctc cccatgggat 1860
cttgagggac caagctgctg ggattgggaa ggagtttcac cctgaccgtt gccctagcca 1920
ggttcccagg aggcctcacc atactccctt tcagggccag ggctccagca agcccagggc 1980
aaggatcctg tgctgctgtc tggttgagag cctgccaccg tgtgtcggga gtgtgggcca 2040
ggctgagtgc ataggtgaca gggccgtgag catgggcctg ggtgtgtgtg agctcaggcc 2100
taggtgcgca gtgtggagac gggtgttgtc ggggaagagg tgtggcttca aagtgtgtgt 2160
gtgcaggggg tgggtgtgtt agcgtgggtt aggggaacgt gtgtgcgcgt gctggtgggc 2220
atgtgagatg agtgactgcc ggtgaatgtg tccacagttg agaggttgga gcaggatgag 2280
ggaatcctgt caccatcaat aatcacttgt ggagcgccag ctctgcccaa gacgccacct 2340
gggcggacag ccaggagctc tccatggcca ggctgcctgt gtgcatgttc cctgtctggt 2400
gccccttgc ccgcctcctg caaacctcac aggtccccca cacaacagtg ccctccagaa 2460
gcagcccctc ggaggcagag gaaggaaaat ggggatggct ggggctctct ccatcctcct 2520
tttctccttg ccttcgcatg gctggccttc ccctccaaaa cctccattcc cctgctgcca 2580
gccccttgc catagcctga ttttggggag gaggaagggg cgatttgagg gagaagggga 2640
gaaagcttat ggctgggtct ggtttcttcc cttcccagag ggtcttactg ttccagggtg 2700
gccccagggc aggcaggggc cacactatgc ctgcgccctg gtaaaggtga cccctgccat 2760
ttaccagcag ccctggcatg ttcctgcccc acaggaatag aatggaggga gctccagaaa 2820
ctttccatcc caaaggcagt ctccgtggtt gaagcagact ggatttttgc tctgcccctg 2880

acccccttgtc cctctttgag ggaggggagc tatgctagga ctccaacctc agggactcgg 2940
gtggcctgcg ctagcttctt ttgatactga aaacttttaa ggtgggaggg tggcaaggga 3000
tgtgcttaat aaatcaattc caagcctcac ctg 3033

<210> 61

<211> 1174

<212> DNA

<213> Homo sapiens

<400> 61
aagctcctcc cccggcggcg agccagggag aaaggatggc cggcctggcg gcgcggttgg 60
tcctgctagc tggggcagcg gcgctggcga gcggctccca gggcgaccgt gagccggtgt 120
accgcgactg cgtactgcag tgcgaagagc agaactgctc tgggggcgct ctgaatcact 180
tccgctcccg ccagccaatc tacatgagtc tagcaggctg gacctgtcgg gacgactgta 240
agtatgagtg tatgtgggtc accgttgggc tctacctcca ggaaggtcac aaagtgcctc 300
agttccatgg caagtggccc ttctcccggt tcctgttctt tcaagagccg gcatcggccg 360
tggcctcgtt tctcaatggc ctggccagcc tggtgatgct ctgccgctac cgcaccttcg 420
tgccagcctc ctcccccatg taccacacct gtgtggcctt cgcctgggtg tccctcaatg 480
catggttctg gtccacagtc ttccacacca gggacactga cctcacagag aaaatggact 540
acttctgtgc ctccactgtc atcctacact caatctacct gtgctgcgtc aggaccgtgg 600
ggctgcagca cccagctgtg gtcagtgcct tccgggctct cctgctgctc atgctgaccg 660
tgcacgtctc ctacctgagc ctcatccgct tcgactatgg ctacaacctg gtggccaacg 720
tggctattgg cctggtcaac gtggtgtggt ggctggcctg gtgcctgtgg aaccagcggc 780
ggctgcctca cgtgcgcaag tgcgtggtgg tggtcttgct gctgcagggg ctgtccctgc 840
tcgagctgct tgacttccca ccgctcttct gggtcctgga tgcccatgcc atctggcaca 900
tcagcaccat ccctgtccac gtcctctttt tcagctttct ggaagatgac agcctgtacc 960
tgctgaagga atcagaggac aagttcaagc tggttgaagc agactggatt tttgctctgc 1020
ccctgacccc ttgtccctct ttgagggagg ggagctatgc taggactcca acctcaggga 1080
ctcgggtggc ctgcgctagc ttcttttgat actgaaaact tttaaggtgg gagggtggca 1140
agggatgtgc ttaataaatc aattccaagc ctca 1174

<210> 62

<211> 3167

<212> DNA

<213> Homo sapiens

<400> 62
aagctcctcc cccggcggcg agccagggag aaaggatggc cggcctggcg gcgcggttgg 60
tcctgctagc tggggcagcg gcgctggcga gcggctccca gggcgaccgt gagccggtgt 120

accgcgactg cgtactgcag tgcgaagagc agaactgctc tgggggcgct ctgaatcact 180
tccgctcccg ccagccaatc tacatgagtc tagcaggctg gacctgtcgg gacgactgta 240
agtatgagtg tatgtgggtc accgttgggc tctacctcca ggaaggtcac aaagtgcctc 300
agttccatgg caagtggccc ttctcccggt tcctgttctt tcaagagccg gcatcggccg 360
tggcctcgtt tctcaatggc ctggccagcc tggtgatgct ctgccgctac cgcaccttcg 420
tgccagcctc ctcccccatg taccacacct gtgtggcctt cgcctggatg agaaaactga 480
ggcacagcaa ggctaaataa cttgcccaag gacacacagg aaatgcagag ccaggaactg 540
aaccctggca gtctggctgt agggcttgca ttcttaatga taccactacc tcccaaatct 600
gaggaaaggg tgtccctcaa tgcatggttc tggtccacag tcttccacac cagggacact 660

```
gacctcacag agaaaatgga ctacttctgt gcctccactg tcatcctaca ctcaatctac      720
ctgtgctgcg tcaggtgagc ctgcctgggt ggctgcaggg gcaaaatcga accctggggg      780
cagaaagggg tcacccagcc ttcccctggg ggccttcttc actagtctcc caacacctac      840
gccccccaac ccccaacaca tcagctgtcc tgggtgagga ctctggggta ggactggggg      900
ccctggctcc tgacaaggag ctgtagcact tgctgcccag ctgtggcctg tttggtgggg      960
agaggggtag tgacttcagg ggccatgcac caatgttggg gggaggagat gcttcaggga     1020
atgctgctct ggggatgggc cacctgccct ctgagcaacc ctggacggtg gggcaggacc     1080
gtggggctgc agcacccagc tgtggtcagt gccttccggg ctctcctgct gctcatgctg     1140
accgtgcacg tctcctacct gagcctcatc cgcttcgact atggctacaa cctggtggcc     1200
aacgtggcta ttggcctggt caacgtggtg tggtggctgg cctggtgcct gtggaaccag     1260
cggcggctgc ctcacgtgcg caagtgcgtg gtggtggtct tgctgctgca ggggctgtcc     1320
ctgctcgagc tgcttgactt cccaccgctc ttctgggtcc tggatgccca tgccatctgg     1380
cacatcagca ccatccctgt ccacgtcctc tttttcagct ttctggaaga tgacagcctg     1440
tacctgctga aggaatcaga ggacaagttc aagctggact gaagaccttg gagcgagtct     1500
gccccagtgg ggatcctgcc cccgccctgc tggcctccct tctcccctca accccttgaga    1560
tgattttctc ttttcaactt cttgaacttg gacatgaagg atgtgggccc agaatcatgt     1620
ggccagccca ccccctgttg gccctcacca gccttggagt ctgttctagg gaaggcctcc     1680
cagcatctgg gactcgagag tgggcagccc ctctacctcc tggagctgaa ctggggtgga     1740
actgagtgtg ttcttagctc taccgggagg acagctgcct gtttcctccc caccagcctc     1800
ctccccacat ccccagctgc ctggctgggt cctgaagccc tctgtctacc tgggagacca     1860
gggaccacag gccttaggga tacagggggt ccccttctgt taccaccccc caccctcctc     1920
caggacacca ctaggtggtg ctggatgctt gttctttggc cagccaaggt tcacggcgat     1980
tctccccatg ggatcttgag ggaccaagct gctgggattg ggaaggagtt tcaccctgac     2040
cgttgcccta gccaggttcc caggaggcct caccatactc cctttcaggg ccagggctcc     2100
agcaagccca gggcaaggat cctgtgctgc tgtctggttg agagcctgcc accgtgtgtc     2160
gggagtgtgg gccaggctga gtgcataggt gacagggccg tgagcatggg cctgggtgtg     2220
tgtgagctca ggcctaggtg cgcagtgtgg agacgggtgt tgtcggggaa gaggtgtggc     2280
ttcaaagtgt gtgtgtgcag gggtgggtg tgttagcgtg ggttagggga acgtgtgtgc      2340
gcgtgctggt gggcatgtga gatgagtgac tgccggtgaa tgtgtccaca gttgagaggt     2400
tggagcagga tgagggaatc ctgtcaccat caataatcac ttgtggagcg ccagctctgc     2460
ccaagacgcc acctgggcgg acagccagga gctctccatg gccaggctgc ctgtgtgcat     2520
gttccctgtc tggtgcccct ttgcccgcct cctgcaaacc tcacagggtc cccacacaac     2580
agtgccctcc agaagcagcc cctcggaggc agaggaagga aaatggggat ggctggggct     2640
ctctccatcc tcctttttctc cttgccttcg catggctggc cttcccctcc aaaacctcca    2700
ttcccctgct gccagcccct ttgccatagc ctgattttgg ggaggaggaa ggggcgattt     2760
gagggagaag gggagaaagc ttatggctgg gtctggtttc ttcccttccc agagggtctt     2820
actgttccag ggtggcccca gggcaggcag gggccacact atgcctgcgc cctggtaaag     2880
gtgacccctg ccatttacca gcagccctgg catgttcctg ccccacagga atagaatgga     2940
gggagctcca gaaactttcc atcccaaagg cagtctccgt ggttgaagca gactggattt     3000
ttgctctgcc cctgacccct tgtccctctt tgagggaggg gagctatgct aggactccaa     3060
cctcagggac tcgggtggcc tgcgctagct tcttttgata ctgaaaactt ttaaggtggg     3120
agggtggcaa gggatgtgct taataaatca attccaagcc tcacctg                  3167
```

<210> 63

<211> 2733

<212> DNA

<213> Homo sapiens

<220>

<221> misc_feature

<222> (2694)..(2694)

<223> n=a, c, g or t

<220>

<221> misc_feature

<222> (2724)..(2724)

<223> n=a, c, g or t

<400> 63
agggagaaag gatggccggc ctggcggcgc ggttggtcct gctagctggg gcagcggcgc 60
tggcgagcgg ctcccagggc gaccgtgagc cggtgtaccg cgactgcgta ctgcagtgcg 120
aagagcagaa ctgctctggg ggcgctctga atcacttccg ctcccgccag ccaatctaca 180
tgagtctagc aggctggacc tgtcgggacg actgtaagta tgagtgtatg tgggtcaccg 240
ttgggctcta cctccaggaa ggtcacaaag tgcctcagtt ccatggcaag tggcccttct 300
cccggttcct gttctttcaa gagccggcat cggccgtggc ctcgtttctc aatggcctgg 360
ccagcctggt gatgctctgc cgctaccgca ccttcgtgcc agcctcctcc cccatgtacc 420
acacctgtgt ggccttcgcc tgggtgtccc tcaatgcatg gttctggtcc acagtcttcc 480
acaccaggga cactgaccta cagagaaaat ggactacttc tgtgcctcct gtatcctaca 540
ctcaatctac ctgtgctgcg tcaggaccgt ggggctgcag cacccagctg tggtcaagtg 600
ccttccgggc tctcctgctg ctcatgctga ccgtgcacgt ctcctacctg agcctcatcc 660
gcttcgacta tggctacaac ctggtggcca acgtggctat tggcctggtc aacgtggtgt 720
ggtggctggc ctggtgcctg tggaaccagc ggcggctgcc tcacgtgcgc aagtgcgtgg 780
tggtggtctt gctgctgcag ggcctgtccc tgctcgagct gcttgacttc ccaccgctct 840
tctgggtcct ggatgcccat gccatctggc acatcagcac catccctgtc cacgtcctct 900
ttttcagctt tctggaagat gacagcctgt acctgctgaa ggaatcagag gacaagttca 960
agctggactg agaccttgga gcgaagtctg ccccagtggg gatcctgccc ccgccctgct 1020
ggcctccctt ctcccctcaa cccttgagat gattttctct tttcaacttc ttgaacttgg 1080
acatgaagga tgtgggccca gaatcatgtg gccagcccac cccctgttgg ccctcaccag 1140
ccttggagtc tgttctaggg aaggcctccc agcatctggg actcgagagt gggcagcccc 1200
tctacctcct ggactgaact ggggtggaac tgagtgtgtt cttagctcta ccgggaggac 1260
agctgcctgt ttcctcccca ccagcctcct ccccacatcc ccagctgcct ggctgggtcc 1320
tgaagccctc tgtctacctg ggagaccagg gtaccacagg ccttagggat acagggggtc 1380
cccttctgtt accacccccc accctcctcc aggacaccac taggtggtgc tggatgcttg 1440
ttctttggcc agccaaggtt cacggcgatt ctccccatgg gatcttgagg gaccaagctg 1500
ctgggattgg gaaggagttt caccctgacc gttgccctag ccaggttccc aggaggcctc 1560
accatactcc ctttcagggc cagggctcca gcaagcccag ggcaaggatc ctgtgctgct 1620
gtctggttga gagcctgcca ccgtgtgtcg ggagtgtggg ccaggctgag tgcataggtg 1680
acagggccgt gagcatgggc ctgggtgtgt gtgagctcag gcctaggtgc gcagtgtgga 1740
gacgggtgtt gtcggggaag aggtgtggct tcaaagtgtg tgtgtgcagg gggtgggtgt 1800
gttagcgtgg gttaggggaa cgtgtgtgcg cgtgctggtg ggcatgtgag atgagtgact 1860
gccggtgaat gtgtccacag ttgagaggtt ggagcaggat gagggaatcc tgtcaccatc 1920
aataatcact tgtggagcgc cagctctgcc caagacgcca cctggcggga cagccaggag 1980
ctctccatgg ccaggctgcc tgtgtgcatg ttccctgtct ggtgcccctt tgcccgcctc 2040
ctgcaaacct cacagggtcc ccacacaaca gtgccctcca gaagcagccc ctcggaggca 2100
gaggaaggaa aatggggatg gctggggctc tctccatcct ccttttctcc ttgccttcgc 2160
atggctggcc ttcccctcca aaacctccat tcccctgctg ccagcccctt tgccatagcc 2220
tgattttggg gaggaggaag ggcgatttg agggagaagg ggagaaagct tatggctggg 2280
tctggtttct tcccttccca gaggtcttta ctgttccagg gtggccccag gcagcagggc 2340
cacactatgc ctgcgccctg gtaaaggtga cccctgccat ttaccagcag ccctggcatg 2400

ttcctgcccc acaggaatag aatggaggga gctccagaaa ctttccatcc caaaggcagt 2460
ctccgtggtt gaagcagact ggatttttgc tctgcccctg acccccttgtc cctctttgag 2520
ggagggggagc tatgctagga ctccaacctc agggactcgg gtggcctgcg ctagcttctt 2580
ttgatactga aaacttttaa ggtgggaggg tggcaaggga tgtgcttaag cggccgcgaa 2640
ttcaaaaagc ttctcgagag tacttctaga gcggccgcgg gcccatcgat tttnccaccc 2700
gggtggggta cccaggtaag tgtnccccat atc 2733

<210> 64

<211> 2546

<212> DNA

<213> Homo sapiens

<400> 64
aagctcctcc cccggcggcg agccagggag aaaggatggc cggcctggcg gcgcggttgg 60
tcctgctagc tggggcagcg gcgctggcga gcggctccca gggcgaccgt gagccggtgt 120
accgcgactg cgtactgcag tgcgaagagc agaactgctc tgggggcgct ctgaatcact 180
tccgctcccg ccagccaatc tacatgagtc tagcaggctg gacctgtcgg gacgactgta 240
agtatgagtg tatgtgggtc accgttgggc tctacctcca ggaaggtcac aaagtgcctc 300
agttccatgg caagtggccc ttctcccggt tcctgttctt tcaagagccg gcatcggccg 360
tggcctcgtt tctcaatggc ctggccagcc tggtgatgct ctgccgctac cgcaccttcg 420
tgccagcctc ctcccccatg taccacacct gtgtggcctt cgcctgggtg tccctcaatg 480
catggttctg gtccacagtc ttccacacca gggacactga cctcacagag aaaatggact 540
acttctgtgc ctccactgtc atcctacact caatctacct gtgctgcgtc aggcctggtc 600
aacgtggtgt ggtggctggc ctggtgcctg tggaaccagc ggcggctgcc tcacgtgcgc 660
aagtgcgtgg tggtggtctt gctgctgcag ggctgtccc tgctcgagct gcttgacttc 720
ccaccgctct tctgggtcct ggatgcccat gccatctggc acatcagcac catccctgtc 780
cacgtcctct tttcagcttt tctggaagat gacagcctgt acctgctgaa ggaatcagag 840
gacaagttca agctggactg aagaccttgg agcgagtctg ccccagtggg gatcctgccc 900
ccgccctgct ggcctccctt ctcccctcaa cccttgagat gatttctct tttcaacttc 960
ttgaacttgg acatgaagga tgtgggccca gaatcatgtg gccagcccac cccctgttgg 1020
ccctcaccag ccttggagtc tgttctaggg aaggcctccc agcatctggg actcgagagt 1080
gggcagcccc tctacctcct ggagctgaac tggggtggaa ctgagtgtgt tcttagctct 1140
accgggagga cagctgcctg tttcctcccc accagcctcc tccccacatc cccagctgcc 1200
tggctgggtc ctgaagccct ctgtctacct gggagaccag ggaccacagg ccttagggat 1260
acagggggtc cccttctgtt accacccccc accctcctcc aggacaccac taggtggtgc 1320
tggatgcttg ttctttggcc agccaaggtt cacggcgatt ctccccatgg gatcttgagg 1380
gaccaagctg ctgggattgg gaaggagttt caccctgacc gttgccctag ccaggttccc 1440
aggaggcctc accatactcc ctttcagggc cagggctcca gcaagcccag ggcaaggatc 1500
ctgtgctgct gtctggttga gagcctgcca ccgtgtgtcg ggagtgtggg ccaggctgag 1560
tgcataggtg acagggccgt gagcatgggc ctgggtgtgt gtgagctcag gcctaggtgc 1620
gcagtgtgga gacgggtgtt gtcggggaag aggtgtggct tcaaagtgtg tgtgtgcagg 1680
gggtgggtgt gttagcgtgg gttaggggaa cgtgtgtgcg cgtgctggtg ggcatgtgag 1740
atgagtgact gccggtgaat gtgtccacag ttgagaggtt ggagcaggat gagggaatcc 1800
tgtcaccatc aataatcact tgtggagcgc cagctctgcc caagacgcca cctgggcgga 1860
cagccaggag ctctccatgg ccaggctgcc tgtgtgcatg ttccctgtct ggtgcccctt 1920
tgcccgcctc ctgcaaacct cacagggtcc ccacacaaca gtgccctcca gaagcagccc 1980
ctcggaggca gaggaaggaa aatggggatg gctggggctc tctccatcct ccttttctcc 2040
ttgccttcgc atggctggcc ttcccctcca aaacctccat tcccctgctg ccagcccctt 2100
tgccatagcc tgattttggg gaggaggaag gggcgatttg agggagaagg ggagaaagct 2160
tatggctggg tctggtttct tcccttccca gagggtctta ctgttccagg gtggccccag 2220
ggcaggcagg ggccacacta tgcctgcgcc ctggtaaagg tgacccctgc catttaccag 2280
cagccctggc atgttcctgc cccacaggaa tagaatggag ggagctccag aaactttcca 2340
tcccaaaggc agtctccgtg gttgaagcag actggatttt tgctctgccc ctgacccctt 2400
gtccctcttt gagggagggg agctatgcta ggactccaac ctcagggact cgggtggcct 2460

gcgctagctt cttttgatac tgaaaacttt taaggtggga gggtggcaag ggatgtgctt 2520
aataaatcaa ttccaagcct cacctg 2546

<210> 65

<211> 2683

<212> DNA

<213> Homo sapiens

<400> 65
aagctcctcc cccggcggcg agccagggag aaaggatggc cggcctggcg gcgcggttgg 60
tcctgctagc tggggcagcg gcgctggcga gcggctccca gggcgaccgt gagccggtgt 120
accgcgactg cgtactgcag tgcgaagagc agaactgctc tgggggcgct ctgaatcact 180
tccgctcccg ccagccaatc tacatgagtc tagcaggctg gacctgtcgg gacgactgta 240
agtatgagtg tatgtgggtc accgttgggc tctacctcca ggaaggtcac aaagtgcctc 300
agttccatgg caagtggccc ttctcccggt tcctgttctt tcaagagccg gcatcggccg 360
tggcctcgtt tctcaatggc ctggccagcc tggtgatgct ctgccgctac cgcaccttcg 420
tgccagcctc ctcccccatg taccacacct gtgtggcctt cgcctgggtg tccctcaatg 480
catggttctg gtccacagtc ttccacacca gggacactga cctcacagag aaaatggact 540
acttctgtgc ctccactgtc atcctacact caatctacct gtgctgcgtc aggaccgtgg 600
ggctgcagca cccagctgtg gtcagtgcct tccgggctct cctgctgctc atgctgaccg 660
tgcacgtctc ctacctgagc ctcatccgct tcgactatgg ctacaacctg gtggccaacg 720
tggctattgg cctggtcaac gtggtgtggt ggctggcctg gtgcctgtgg aaccagcggc 780
ggctgcctca cgtgcgcaag tgcgtggtgg tggtcttgct gctgcagggg ctgtccctgc 840
tcgagctgct tgacttccca ccgctcttct gggtcctgga tgcccatgcc atctggcaca 900
tcagcaccat ccctgtccac gtcctctttt tcagctttct ggaagatgac agcctgtacc 960
tgctgaagga atcagaggac aagttcaagc tggactgaag accttggagc gagtctgccc 1020
cagtggggat cctgcccccg ccctgctggc ctcccttctc ccctcaaccc ttgagatgat 1080
tttctctttt caacttcttg aacttggaca tgaaggatgt gggcccagaa tcatgtggcc 1140
agcccacccc ctgttggccc tcaccagcct tggagtctgt tctagggaag gcctcccagc 1200
atctgggact cgagagtggg cagcccctct acctcctgga gctgaactgg ggtggaactg 1260
agtgtgttct tagctctacc gggaggacag ctgcctgttt cctccccacc agcctcctcc 1320
ccacatcccc agctgcctgg ctgggtcctg aagccctctg tctacctggg agaccaggga 1380
ccacaggcct tagggataca gggggtcccc ttctgttacc acccccccacc ctcctccagg 1440
acaccactag gtggtgctgg atgcttgttc tttggccagc caaggttcac ggcgattctc 1500
cccatgggat cttgagggac caagctgctg ggattgggaa ggagtttcac cctgaccgtt 1560
gccctagcca ggttcccagg aggcctcacc atactccctt tcagggccag ggctccagca 1620
agcccagggc aaggatcctg tgctgctgtc tggttgagag cctgccaccg tgtgtcggga 1680
gtgtgggcca ggctgagtgc ataggtgaca gggccgtgag catgggcctg ggtgtgtgtg 1740
agctcaggcc taggtgcgca gtgtggagac gggtgttgtc ggggaagagg tgtggcttca 1800
aagtgtgtgt gtgcaggggg tgggtgtgtt agcgtgggtt aggggaacgt gtgtgcgcgt 1860
gctggtgggc atgtgagatg agtgactgcc ggtgaatgtg tccacagttg agaggttgga 1920
gcaggatgag ggaatcctgt caccatcaat aatcacttgt ggagcgccag ctctgcccaa 1980
gacgccacct gggcggacag ccaggagctc tccatggcca ggctgcctgt gtgcatgttc 2040
cctgtctggt gccccttgc ccgcctcctg caaacctcac aggtccccca cacaacagtg 2100
ccctccagaa gcagcccctc ggaggcagag gaaggaaaat ggggatggct ggggctctct 2160
ccatcctcct tttctccttg ccttcgcatg gctggccttc ccctccaaaa cctccattcc 2220
cctgctgcca gcccctttgc catagcctga ttttggggag gaggaagggg cgatttgagg 2280
gagaagggga gaaagcttat ggctgggtct ggtttcttcc cttcccagag ggtcttactg 2340
ttccagggtg gccccagggc aggcaggggc cacactatgc ctgcgccctg gtaaaggtga 2400
cccctgccat ttaccagcag ccctggcatg ttcctgcccc acaggaatag aatggaggga 2460
gctccagaaa ctttccatcc caaaggcagt ctccgtggtt gaagcagact ggatttttgc 2520
tctgcccctg acccttgtc cctctttgag ggaggggagc tatgctagga ctccaacctc 2580
agggactcgg gtggcctgcg ctagcttctt ttgatactga aaacttttaa ggtgggaggg 2640
tggcaaggga tgtgcttaat aaatcaattc caagcctcac ctg 2683

<210> 66

<211> 2341

<212> DNA

<213> Homo sapiens

<400> 66
aagctcctcc cccggcggcg agccagggag aaaggatggc cggcctggcg gcgcggttgg 60
tcctgctagc tggggcagcg gcgctggcga gcggctccca gggcgaccgt gagccggtgt 120
accgcgactg cgtactgcag tgcgaagagc agaactgctc tgggggcgct ctgaatcact 180
tccgctcccg ccagccaatc tacatgagtc tagcaggctg gacctgtcgg gacgactgta 240
agtatgagtg tatgtgggtc accgttgggc tctacctcca ggaaggtcac aaagtgcctc 300
agttccatgg caagtggccc ttctcccggt tcctgttctt tcaagagccg gcatcggccg 360
tggcctcgtt tctcaatggc ctggccagcc tggtgatgct ctgccgctac cgcaccttcg 420
tgccagcctc ctcccccatg taccacacct gtgtggcctt cgcctgggtg tccctcaatg 480
catggttctg gtccacagtc ttccacacca gggacactga cctcacagag aaaatggact 540
acttctgtgc ctccactgtc atcctacact caatctacct gtgctgcgtc agctttctgg 600
aagatgacag cctgtacctg ctgaaggaat cagaggacaa gttcaagctg gactgaagac 660

cttggagcga gtctgcccca gtggggatcc tgcccccgcc ctgctggcct cccttctccc 720
ctcaaccctt gagatgattt tctcttttca acttcttgaa cttggacatg aaggatgtgg 780
gcccagaatc atgtggccag cccacccccct gttggccctc accagccttg gagtctgttc 840
tagggaaggc ctcccagcat ctgggactcg agagtgggca gcccctctac ctcctggagc 900
tgaactgggg tggaactgag tgtgttctta gctctaccgg gaggacagct gcctgtttcc 960
tccccaccag cctcctcccc acatccccag ctgcctggct gggtcctgaa gccctctgtc 1020
tacctgggag accagggacc acaggcctta gggatacagg gggtcccctt ctgttaccac 1080
cccccacccct cctccaggac accactaggt ggtgctggat gcttgttctt tggccagcca 1140
aggttcacgg cgattctccc catgggatct tgagggacca agctgctggg attgggaagg 1200
agtttcaccc tgaccgttgc cctagccagg ttcccaggag gcctcaccat actccctttc 1260
agggccaggg ctccagcaag cccagggcaa ggatcctgtg ctgctgtctg gttgagagcc 1320
tgccaccgtg tgtcgggagt gtgggccagg ctgagtgcat aggtgacagg gccgtgagca 1380
tgggcctggg tgtgtgtgag ctcaggccta ggtgcgcagt gtggagacgg gtgttgtcgg 1440
ggaagaggtg tggcttcaaa gtgtgtgtgt gcaggggggtg ggtgtgttag cgtgggttag 1500
gggaacgtgt gtgcgcgtgc tggtgggcat gtgagatgag tgactgccgg tgaatgtgtc 1560
cacagttgag aggttggagc aggatgaggg aatcctgtca ccatcaataa tcacttgtgg 1620
agcgccagct ctgcccaaga cgccacctgg gcggacagcc aggagctctc catggccagg 1680
ctgcctgtgt gcatgttccc tgtctggtgc cccctttgccc gcctcctgca aacctcacag 1740
ggtccccaca caacagtgcc ctccagaagc agcccctcgg aggcagagga aggaaaatgg 1800
ggatggctgg ggctctctcc atcctccttt tctccttgcc ttcgcatggc tggccttccc 1860
ctccaaaacc tccattcccc tgctgccagc ccctttgcca tagcctgatt ttggggagga 1920
ggaaggggcg atttgaggga gaaggggaga aagcttatgg ctgggtctgg tttcttccct 1980
tcccagaggg tcttactgtt ccagggtggc cccagggcag gcaggggcca cactatgcct 2040
gcgccctggt aaaggtgacc cctgccattt accagcagcc ctggcatgtt cctgccccac 2100
aggaatagaa tggagggagc tccagaaact ttccatccca aaggcagtct ccgtggttga 2160
agcagactgg atttttgctc tgcccctgac cccttgtccc tctttgaggg aggggagcta 2220
tgctaggact ccaacctcag ggactcgggt ggcctgcgct agcttctttt gatactgaaa 2280
acttttaagg tgggagggtg gcaagggatg tgcttaataa atcaattcca agcctcacct 2340
g 2341

<210> 67

<211> 2109

<212> DNA

<213> Homo sapiens

<400> 67
gattcggccg gagctgccag cggggaggct gcagccgcgg gttgttacag ctgctggagc 60
agcagcggcc cccgctcccg ggaaccgttc ccgggccgtt gatcttcggc cccacacgaa 120
cagcagagag gggcagcagg atgaatgtgg gcacagcgca cagcgaggtg aaccccaaca 180
cgcgggtgat gaacagccgt ggcatctggc tctcctacgt gctggccatc ggtctcctcc 240
acatcgtgct gctgagcatc ccgtttgtga gtgtccctgt cgtctggacc ctcaccaacc 300
tcattcacaa catgggcatg tatatcttcc tgcacacggt gaaggggaca cccctttgaga 360
ccccggacca gggcaaggcg aggctgctaa cccactggga gcagatggat tatggggtcc 420
agttcacggc ctctcggaag ttcttgacca tcacacccat cgtgctgtac ttcctcacca 480
gcttctacac taagtacgac cagatccatt ttgtgctcaa caccgtgtcc ctgatgagcg 540
tgcttatccc caagctgccc cagctccacg gagtccggat tttggaatc aataagtact 600
gagagtgcag ccccttcccc tgcccagggt ggcaggggag gggtagggta aaaggcatgt 660
gctgcaacac tgaagacaga aagaagaagc ctctggacac tgccagagat gggggttgag 720
cctctggcct aatttccccc ctcgcttccc ccagtagcca acttggagta gcttgtagtg 780
gggttggggt aggccccctg ggctctgacc ttttctgaat tttttgatct cttccttttg 840
ctttttgaat agagactcca tggagttggt catggaatgg gctgggctcc tgggctgaac 900
atggaccacg cagttgcgac aggaggccag gggaaaaacc cctgctcact tgtttgccct 960
caggcagcca aagcacttta acccctgcat agggagcaga gggcggtacg gcttctggat 1020
tgtttcactg tgattcctag gtttttttcga tgccatgcag tgtgtgcttt tgtgtatgga 1080
agcaagtgtg ggatgggtct ttgcctttct gggtagggag ctgtctaatc caagtcccag 1140
gcttttggca gcttctctgc aacccaccgt gggtcctggt tgggagtggg gagggtcagg 1200
ttggggaaag atggggtaga gtgtagatgg cttggttcca gaggtgaggg ggccagggct 1260
gctgccatcc tggcctggtg gaggttgggg agctgtagga gagctagtga gtcgagactt 1320
agaagaatgg ggccacatag cagcagagga ctggtgtaag ggagggaggg gtagggacag 1380
aagctagacc caatctcctt tgggatgtgg gcagggaggg aagcaggctt ggagggttaa 1440
tttacccaca gaatgtgata gtaatagggg agggaggctg ctgtgggttt aactcctggg 1500
ttggctgttg ggtagacagg tggggaaaag gcccgtgagt cattgtaagc acaggtccaa 1560
cttggccctg actcctgcgg gggtatgggg aagctgtgac agaaacgatg ggtgctgtgg 1620
tcctctgcag gccctcaccc cttaacttcc tcatgcagac tggcactggg cagggcctct 1680
catgtggcag ccacatgtgg cgttgtgagg ccaccccatg tggggtctgt ggtgagagtc 1740
ctgtaggatc cctgctcaag cagcacagag gaaggggcaa gacgtggcct gtaggcactg 1800
tctcagcctg cagagaagaa agtgaggccg ggagcctgag cctgggctgg agccttctcc 1860
cctccccagt tggactaggg gcagtgttaa ttttgaaaag gtgtgggtcc ctgtgtcctt 1920
ttccaggggt ccaagggaac aggagaggtc actgggcctg ttttctccct cctgaccctg 1980
catctcccac cctgtgtatc atagggaact ttcaccttaa aatctttcta agcaaagtgt 2040
gaataggatt tttactccct ttgtacagta ttctgaggaa cgcaaataaa agggcaacat 2100
gtttctgtt 2109

<210> 68

<211> 2423

<212> DNA

<213> Homo sapiens

<400> 68
gagagccgag ctagcgacga gcagtcgttg cggccgccgg cgccgcggga ggtggtggag 60
gcctagccgg agccgagagg tctcttgttc ccgtcccacg gtcccggcgt cacccctccg 120
gcgcccagtc cccgtcccgg aactcccggg cctgtcctgg gcccccggtc tgtgcactcc 180
gctcgccgca gcgcccggcc cgggccgcac ccgccggccc catgaggagg gacgtgaacg 240
gagtgaccaa gagcaggttt gagatgttct caaatagtga tgaagctgta atcaataaaa 300
aacttcccaa agaactcctg ttacggatat ttctttttct agatgttgtt accctgtgcc 360
gctgtgctca ggtctccagg gcctggaatg ttctggctct ggatggcagt aactggcagc 420
gaattgacct atttgatttc cagagggata ttgagggccg agtagtggag aatatttcaa 480
aacgatgtgg gggcttttta cgaaagttaa gtcttcgtgg atgtcttgga gtgggagaca 540
atgcattaag aacctttgca caaaactgca ggaacattga agtactgaat ctaaatgggt 600
gtacaaagac aacagacgct acatgtacta gccttagcaa gttctgttcc aaactcaggc 660
accttgactt ggcttcctgt acatcaataa caaacatgtc tctaaaagct ctgagtgagg 720
gatgtccact gttggagcag ttgaacattt cctggtgtga ccaagtaacc aaggatggca 780
ttcaagcact agtgaggggc tgtgggggtc tcaaggcctt attcttaaaa ggctgcacgc 840
agctagaaga tgaagctctc aagtacatag gtgcacactg ccctgaactg gtgactttga 900
acttgcagac ttgcttgcaa atcacagatg aaggtctcat tactatatgc agagggtgcc 960
ataagttaca atccctttgt gcctctggct gctccaacat cacagatgcc atcctgaatg 1020
ctctaggtca gaactgccca cggcttagaa tattggaagt ggcaagatgt tctcaattaa 1080
cagatgtggg ctttaccact ctagccagga attgccatga acttgaaaag atggacctgg 1140
aagagtgtgt tcagataaca gatagcacat taatccaact ttctatacac tgtcctcgac 1200
ttcaagtatt gagtctgtct cactgtgagc tgatcacaga tgatggaatt cgtcacctgg 1260
ggaatggggc ctgcgcccat gaccagctgg aggtgattga gctggacaac tgcccactaa 1320
tcacagatgc atccctggag cacttgaaga gctgtcatag ccttgagcgg atagaactct 1380
atgactgcca gcaaatcaca cgggctggaa tcaagagact caggacccat ttacccaata 1440
ttaaagtcca cgcctacttc gcacctgtca ctccaccccc atcagtaggg ggcagcagac 1500
agcgcttctg cagatgctgc atcatcctat gacaatggag gtggtcaacc ttggcgaact 1560
gagtatttaa tgacacttct agagctaccg tggagtctct ccagtggaag caaccccagt 1620
gttctgagca agggttacaa agtgagggag ggcagtgtcc agatccccag agccacacat 1680
acatacacat acacaccctt acccccatcc actctagctt tgtgaccatg ggactgaagt 1740
ttgtgatggc tttttatca agtagattgg taaaatttaa ccattcctgt tgaggtgccc 1800
ataagaaaat catagggccaa gatagggagg ggcattccag caaaccccgt gttaatgcta 1860
ctgtggtttt taaatttttg tctaggggtt tctttgggga ttttagaaca gcatctgctg 1920
tcctccgggg tcaagaaaag catggaaaga caatatatga tgtacccagg gaccagaaag 1980
aaaatttctt tgcatcttag aaatggtaga cattcattgt gactaaagag cttctatgct 2040
tccttgtttc catgccaaca tgctgagcat gctcacaaag aaggctcgtc cattcctcct 2100
gtgttttagt atttggccca gaggtttcct aaatggttgc cttgaaatca ctgtggtcca 2160
aatgtaattc ttacacactc aaattatcac tgtctgtagc acacttgtgc acctgtctta 2220
cattctctgt tgctcccccc cacactcttg ctcagtctgt cacctgttca gtctgcttac 2280
tcactcaatt gttacccttt tgctgttgtc gtgtttacag tttgcatttt gaatgattag 2340
ttgggattac caaacatttt ttaaaaagat attatcaata aatattttt taattctaaa 2400
ttttaaaaaa aaaaaaaaaa aaa 2423

<210> 69

<211> 1841

<212> DNA

<213> Homo sapiens

<400> 69
agctgggacc ggagggtgag cccggcagag gcagagacac acgcggagag gaggagaggc 60
tgagggaggg aggtggagaa ggacgggaga ggcagagaga ggagacacgc agagacactc 120
aggaggggag agacaccgag acgcagagac actcaggagg ggagagacac cgagacgcag 180
agacacccag gccgggggagc gcgagggagc gaggcacaga cctggctcag cgagcgcggg 240
gggcgagccc cgagtcccga gagcctgggg gcgcgcccag cccgggcgcc gaccctcctc 300

ccgctcccgc gccctcccct cggcgggcac ggtattttta tccgtgcgcg aacagccctc 360
ctcctcctct cgccgcacag cccgccgcct gcgcggggga gcccagcaca gaccgccgcc 420
gggaccccga gtcgcgcacc ccagccccac cgcccacccc gcgcgccatg gaccccaagg 480
accgcaagaa gatccagttc tcggtgcccg cgccccctag ccagctcgac cccgccagg 540
tggagatgat ccggcgcagg agaccaacgc ctgccatgct gttccggctc tcagagcact 600
cctcaccaga ggaggaagcc tccccccacc agagagcctc aggagagggg caccatctca 660
agtcgaagag acccaacccc tgtgcctaca caccaccttc gctgaaagct gtgcagcgca 720
ttgctgagtc tcacctgcag tctatcagca atttgaatga gaaccaggcc tcagaggagg 780
aggatgagct gggggagctt cgggagctgg gttatccaag agaggaagat gaggaggaag 840
aggaggatga tgaagaagag gaagaagaag aggacagcca ggctgaagtc ctgaaggtca 900
tcaggcagtc tgctgggcaa aagacaacct gtggccaggg tctggaaggg ccctgggagc 960
gcccaccccc tctggatgag tccgagagag atggaggctc tgaggaccaa gtggaagacc 1020
cagcactaag tgagcctggg gaggaacctc agcgcccttc cccctctgag cctggcacat 1080
aggcacccag cctgcatctc ccaggaggaa gtggagggga catcgctgtt ccccagaaac 1140
ccactctatc ctcaccctgt tttgtgctct tcccctcgcc tgctagggct gcggcttctg 1200
acttctagaa gactaaggct ggtctgtgtt tgcttgtttg cccacctttg gctgataccc 1260
agagaacctg ggcacttgct gcctgatgcc cacccctgcc agtcattcct ccattcaccc 1320
agcgggaggt gggatgtgag acagcccaca ttggaaaatc cagaaaaccg ggaacaggga 1380
tttgcccttc acaattctac tccccagatc ctctcccctg gacacaggag acccacaggg 1440
caggacccta agatctgggg aaaggaggtc ctgagaacct tgaggtaccc ttagatcctt 1500
ttctacccac tttcctatgg aggattccaa gtcaccactt ctctcaccgg cttctaccag 1560
ggtccaggac taaggcgttt ttctccatag cctcaacatt ttgggaatct tcccttaatc 1620
accccttgctc ctcctgggtg cctggaagat ggactggcag agacctcttt gttgcgtttt 1680
gtgctttgat gccaggaatg ccgcctagtt tatgtccccg gtggggcaca cagcgggggg 1740
cgccaggttt tccttgtccc ccagctgctc tgcccctttc cccttcttcc ctgactccag 1800
gcctgaaccc ctcccgtgct gtaataaatc tttgtaaata a 1841

<210> 70

<211> 748

<212> DNA

<213> Homo sapiens

<400> 70
ggccgcgatg agcggggagc cggggcagac gtccgtagcg ccccctcccg aggaggtcga 60
gccgggcagt ggggtccgca tcgtggtgga gtactgtgaa ccctgcggct tcgaggcgac 120
ctacctggag ctggccagtg ctgtgaagga gcagtatccg ggcatcgaga tcgagtcgcg 180
cctcgggggc acaggtgcct ttgagataga gataaatgga cagctggtgt tctccaagct 240
ggagaatggg ggctttccct atgagaaaga tctcattgag gccatccgaa gagccagtaa 300
tggagaaacc ctagaaaaga tcaccaacag ccgtcctccc tgcgtcatcc tgtgactgca 360
caggactctg ggttcctgct ctgttctggg gtccaaacct tggtctccct ttggtcctgc 420
tgggagctcc ccctgcctct ttcccctact tagctcctta gcaaagagac cctggcctcc 480
actttgccct ttgggtacaa agaaggaata gaagattccg tggccttggg ggcaggagag 540
agacactctc catgaacact tctccagcca cctcataccc ccttcccagg gtaagtgccc 600
acgaaagccc agtccactct tcgcctcggt aatacctgtc tgatgccaca gattttattt 660
attctcccct aacccagggc aatgtcagct attggcagta aagtggcgct acaaacacta 720
aaaaaaaaaa aaaaaaaaaa aaaaaaaa 748

<210> 71

<211> 795

<212> DNA

<213> Homo sapiens

<400> 71
tacggctgcg agaagacgac agaagctaga cccaatctcc tttgggatgt gggcagggag 60
ggaagcaggc ttggagggtt aatttaccca cagaatgtga tagtaatagg ggagggaggc 120
tgctgcgggt ttaactcctg ggttggctgt tgggtagaca ggtggggaaa aggcccgtga 180
gtcattgtaa gcacaggtcc aacttggccc tgactcctgc gggggtatgg ggaagctgtg 240
acagaaacga tgggtgctgt ggtcctctgc aggccctcac cccttaactt cctcatacag 300
actggcactg ggcagggcct ctcatgtggc agccacatgt ggcgttgtga ggccacccca 360
tgtggggtct gtggtgagag tcctgtagga tccctgctca agcagcacag aggaaggggc 420
aagacgtggc ctgtaggcac tgtctcagcc tgcagagaag aaagtgaggc cgggagcctg 480
agcctgggct ggagccttct cccctcccca gttggactag ggcagtgtt aattttgaaa 540
aggtgtgggt ccctgtgtcc tcttccaggg gtccaaggga acaggagagg tcactgggcc 600
tgttttctcc ctcctgaccc tgcatctccc accccgtgta tcatagggaa ctttcacctt 660
aaaatctttc taagcaaagt gtgaatagga tttttactcc ctttgtacag tattctgaga 720
aacgcaaata aaagggcaac atgtttctgt taaaaaaaaa aaaaagtacg caaaaaaaaa 780
aaaaaaaaaa aaaaa 795

<210> 72

<211> 2356

<212> DNA

<213> Homo sapiens

<400> 72
ggcacgaggc cggaagtgac ctctagagcg gtggtgaaac tggcagttga cggctcctgg 60
gactagatcc cgcgaggtag cccccgaact atttctctac gttttctctt gatcctcccg 120
aaatcttcca gatccgcgta gtgaggaatc gtctccaccg tcatgggggg cggagacctg 180
aatctgaaga agagctggca cccgcagacc ctcaggaatg tggagaaagt gtggaaggcc 240
gagcagaagc atgaggctga gcggaagaag attgaggagc ttcagcggga gctgcgagaa 300
gagagagccc gggaagagat gcagcgctat gcggaggatg ttggggccgt caagaaaaaa 360
gaagaaaagt tggactggat gtaccagggt cctggtggga tggtgaaccg tgacgagtac 420
ctgctggggc gccccattga caaatatgtt tttgagaaga tggaggagaa ggaggcaggc 480
tgctcttctg aaacaggact tctcccaggc tctatctttg ccccatcagg tgccaattcc 540
cttcttgaca tggccagcaa gatccgggag gacccactct tcatcatcag gaagaaggag 600
gaggagaaaa aacgagaggt attaaataat ccagtgaaaa tgaagaaaat caaagaattg 660
ttgcaaatga gtctggaaaa aaaggagaag aagaaaaaga aggagaagaa aaagaagcac 720
aagaaacata agcacagaag ctcgagtagt gatcgttcca gcagcgagga tgagcacagt 780
gcagggagat cacagaagaa gatggcaaat tcctcccctg ttttgtccaa agtccctgga 840
tatggcttac aggtccggaa ctctgaccgt aaccagggtc ttcagggtcc tctgacagca 900
gagcaaaaga gagggcatgg gatgaagaac cattccagat ccagaagctc ctcccactca 960
cccccaagac atgccagcaa gaagagcacc agggaagcag ggtcccggga caggaggtct 1020
cgatccctgg gcagaaggtc acggtcccca agacccagca aactgcacaa ctctaaggtg 1080
aacaggagag agacaggcca aactaggagc ccatcaccta aaaaagaggt ctaccaaagg 1140
cgacatgctc ccggatacac cagaaaactc tctgcagagg aattagagcg aaaacggcaa 1200
gagatgatgg aaaacgccaa atggagggag gaggagagac tgaacatcct caagaggcat 1260
gctaaggatg aggaacggga gcagaggcta gagaagctgg actcccggga tgggaagttc 1320

atccaccgca tgaagctgga gagtgcatct acttcctccc tggaggatcg ggtgaagcgg 1380
aatatctact ctttacagag aacttcggta gctctggaga agaactttat gaaaagatga 1440
aaactgtccc ctctcttatt ggttttcctg cattttccag ggaagctgct gaccccttaa 1500
ttctctttat aagagttcaa atgacttctt tcacagatgt caaaccacca gtgttcaaag 1560
tgaccctgct tcattgagtc ctgaaacagc tcacttcctt tgagagctag tgtgacttgc 1620
tttgtgggac actcagtaac tttgggtttt gactctttaa cgggtgggca ctggaccatc 1680
tcggtgggag tgcttgtgcc actctggaag gctgttccct ggggttgtga tgtttatcat 1740
gccacttcct tcttacctgt gccaacagac ctatttcact gcctcagcgt acaccagacc 1800
cttcagaaac ctctctggtg tcacccagat agattgtgct tactgagaca aatgaacgtt 1860
tacttgattt agaagataat gtgacagaat gatgtcaggt taggtcaaag ccaagggagt 1920
gacagaatct ggaaaatcaa acaatacaaa aagccctaaa tgaactgtta actatttgat 1980
ctttggatgt aaaattgtaa tgcgtatatg tacaaatgta caatttttac atgcttttaa 2040
aaaaggttag ctttgtgaaa ataccttgtt tggtcaatga ctttactggg taatagaacc 2100
acattgaacc ttgatggcaa gtaatacaat aaggcaggcc agctcgtttt tctctctgaa 2160
tctggctggt ttaggaggag cctgggttta tcgacgagat ctggagtatc tattcttttc 2220
cactgcttgc agtctccaat gtaggcagtg taaaggtata gtaaaatgat tttaggagtc 2280
agaaccaaat tgccaatatg ctccatggct cctaaaggaa aataaaatgg aagtttttaa 2340
aaaaaaaaaa aaaaaa 2356

<210> 73

<211> 1646

<212> DNA

<213> Homo sapiens

<400> 73
gtggaatgtc atcagttaag gctattttca tttcttttgt ggatcttcag ttgcttcagg 60
ccatctggat gtatacatgc aggtcacagg gaatatgatg gcttagcttg ggttcagagg 120
cctgacacct caggctgcca aatgtggaag atttaaatac ttgaaccaat accctcctcc 180
caaaaactga aattggcttc tgtttctgag ttggtccagg cgcaatgttc agcgtatttg 240
aggaaatcac aagaattgta gttaaggaga tggatgctgg aggggatatg attgccgtta 300
gaagccttgt tgatgctgat agattccgct gcttccatct ggtgggggag aagagaactt 360
tctttggatg ccggcactac acaacaggcc tcaccctgat ggacattctg gacacacatg 420
gggacaagtg gttagatgaa ctggattctg ggctccaagg tcaaaaggct gagtttcaaa 480
ttctggataa tgtagactca acgggagagt tgatagtgag attacccaaa gaaataacaa 540
tttcaggcag tttccagggc ttccaccatc agaaaatcaa gatatcggag aaccggatat 600
cccagcagta tctggctacc cttgaaaaca ggaagctgaa gagggaacta ccctttcat 660
tccgatcaat taatacgaga gaaaacctgt atctggtgac agaaactctg gagacggtaa 720
aggaggaaac cctgaaaagc gaccggcaat ataaattttg gagccagatc tctcagggcc 780
atctcagcta taaacacaag ggccaaaggg aagtgaccat ccccccaaat cgggtcctga 840
gctatcgagt aaagcagctt gtcttcccca acaaggagac gatgagaaag tctttgggtt 900
cggaggattc cagaaacatg aaggagaagt tggaggacat ggagagtgtc ctcaaggacc 960
tgacagagga gaagagaaaa gatgtgctaa actccctcgc taagtgcctc ggcaaggagg 1020
atattcggca ggatctagag caaagagtat ctgaggtcct gatttccggg gagctacaca 1080
tggaggaccc agacaagcct ctcctaagca gcctttttaa tgctgctggg gtcttggtag 1140
aagcgcgtgc aaaagccatt ctggacttcc tggatgccct gctagagctg tctgaagagc 1200
agcagtttgt ggctgaggcc ctggagaagg ggacccttcc tctgttgaag gaccaggtga 1260
aatctgtcat ggagcagaac tgggatgagc tggccagcag tcctcctgac atggactatg 1320
accctgaggc acgaattctc tgtgcgctgt atgttgttgt ctctatcctg ctggagctgg 1380
ctgaggggcc tacctctgtc tcttcctaac tacaaaagcc ctttctcccc acaagcctct 1440
gggttttccc tttaccagtc tgtcctcact gccatcgcca ctaccatcct gtcaccagtg 1500
ggacctcttt aaaacaagca gccaaccatt ctttgatgta tcccattcgc tccatgttaa 1560
catccaaaac cagcctggat ttcatacatg gacttctgat taaaagtggc aggttgtgca 1620
tgttaaaaaa aaaaaaaaaa aaaaaa 1646

<210> 74

<211> 3340

<212> DNA

<213> Homo sapiens

<400> 74

```
cgggcgccca gagacagcgc cgcctcagat atcctgctgg atgacattgt ccttacccat     60
tctctcttcc tcccgacgga gaaatttctg caggagctac accagtactt tgttcgggca    120
ggaggcatgg agggccctga agggctgggc cggaagcaag cctgtctagc catgcttctc    180
catttcttgg acacctacca ggggctgctt caagaggaag agggggccgg ccacatcatc    240
aaggatctat acctgctaat tatgaaggac gagtcccttt accagggcct ccgagaggac    300
actctgaggc tgcaccagct ggtggagacg gtggaactaa agattccaga ggagaaccag    360
ccacccagca agcaggtgaa gccactcttc cgccacttcc gccggataga ctcctgtctg    420
cagacccggg tggccttccg gggctctgat gagatcttct gccgtgtata catgcctgac    480
cactcttatg tgaccatacg cagccgcctt tcagcatctg tgcaggacat tctgggctct    540
gtgacggaga aacttcaata ttcagaggag cccgcggggc gtgaggattc cctcatcctg    600
gtagctgtgt cctcctctgg agagaaggtc cttctccagc ccactgagga ctgtgttttc    660
accgcactgg gcatcaacag ccacctgttt gcctgtactc gggacagcta tgaggctctg    720
gtgcccctcc ccgaggagat ccaggtctcc cctggagaca cagagatcca ccgagtggag    780
cctgaggacg ttgccaacca cctaactgcc ttccactggg agctgttccg atgtgtgcat    840
gagctggagt tcgtggacta cgtgttccac ggggagcgcg gccgccggga gacggccaac    900
ttggagctgc tgctgcagcg ctgcagcgag gtcacgcact gggtggccac cgaagtgctg    960
ctctgcgagg ccccgggcaa gcgcgcgcag ctgctcaaga agttcatcaa gatcgcggcc   1020
ctctgcaagc agaaccagga cctgctgtct ttctacgccg tggtcatggg gctggacaac   1080
gccgctgtca gccgccttcg actcacctgg gagaagctgc cagggaaatt caagaacttg   1140
tttcgcaaat ttgagaacct gacggacccc tgcaggaacc acaaaagcta ccgagaagtg   1200
atctccaaaa tgaagccccc tgtgattccc ttcgtgcctc tgatcctcaa agacctgact   1260
ttcctgcacg aagggagtaa gacccttgta gatggtttgg tgaacatcga gaagctgcat   1320
tcagtggccg aaaaagtgag gacaatccgc aaataccgga gccggcccct ttgcctggac   1380
atggaggcat cccccaatca cctgcagacc aaggcctatg tgcgccagtt tcaggtcatc   1440
gacaaccaga acctcctctt cgagctctcc tacaagctgg aggcaaacag tcagtgagag   1500
tggaggctcc agtcagaccc gccagatcct tgggcacctg gcactcaagc actttgcacg   1560
atgtctcaac caacatctga catctttccc gtggagcaac ttcctgctcc acgggaaaga   1620
ggtcgatgga tttacccctg gacccataag tctgttcatc ctgctgaagt cccctcccca   1680
ttgctccttc aagccaaaac tacactttgc tggttcctgt cccctctgag aaaggggata   1740
gaaagctcct tcctctatgt cctcccatcg agatctgttc tggggatgga gcttccaact   1800
tcctcttgca gcaggaaaga atgctgctca cccttctgtc ttgcagagtg ggattgtggg   1860
agggattggc agccttcttc tccaccacct gtccagcttc ttcctggtca gggctgggac   1920
ccccaggaat attatgttgc cgtgtgtgtg tgtgtgtgtg tgtgtgtgtg tgtgtgtgtg   1980
tgtgtcttct tttagggagc aggagtgcat ctggtaattg agggtggatg ttgtgtgtgc   2040
tggggagggg tccttctgtt tggtgctacc cttgtctact ctgcccctgg atggtgcggg   2100
gtgctttctc cacccccaca ctccctgctc agctcctcgt gctgccctgc atgcccaggc   2160
ttgtgagcca aggtgctttt tggggcaggg agtagcagca ggtgggaggg gttacccatc   2220
agcccttgca agtcccccac tcaggcctct ggaaggtcca gggatgggct ctgatgagag   2280
ggtaaaagat gctcagggaa acacaggcct cagctgccta gaggaccctc cccctgcctt   2340
gcagtgggct cgggtagagc agtatcagga gctagggttg tctgctgccc acactcctgc   2400
tttttgggat atctaactgc taaggaggga gttgacatcc cccttctggc tcatgtgtct   2460
gacaccaaca acatggtctc cgtccctctc tcttagactc tccctttgtc ctccccatag   2520
agctggggtg gggtggatcc ctatactggg gcaggcagcc ccaaagtggg ggaggggggat   2580
ggcagagact gtaaaggcgc cactggactc tggcaaggcc tttattacct ttactccctc   2640
cctctcccat caccagcctc aaggcctgag ggtgcagggg gctcctggca gctactgggt   2700
gaggtttcct ggcacagact caccccttctt tctggcacca ctctttccct tttgaagaga   2760
cagcaacagc cgtagcaaaa gcagctgctg ctcctgctat gagggtgtat atattttttta   2820
cccaaagctc tggaattgta catttatttt ttaaaactca aagagggaaa gagccttgta   2880
```

tcatatgtga acattgtatc ataggtaatg ttgtacagac ccttttatac agtgatctgt 2940
cttgttcctg cagcaaaaat cctctatgga cataggaggt gctgtgtccc atgccttctt 3000
gccctgacag tgtcccatgg gccccttct gctccctgcc ccctccctgc tactgctgat 3060
gcactgtcct ctccctgcag cccctggctt cccagccttc ctcctgaccc cttccaacag 3120
ccttggaact ccagctgcca ccaccctctg ggtcggacac tgggacccac tggcccagtc 3180
ttggctgctg cttaccccta gccttgatgc ctgcccaggg acccccagcc ccctcccgtt 3240
gccctgcagc tttaacagag tgaaccatgt gtattgtaca ggcgcggttg tcattgcaga 3300
aaccgctggg tggagaagaa gccgataaag tctatgaatc 3340

<210> 75

<211> 4005

<212> DNA

<213> Homo sapiens

<400> 75
gggcaacagt ctgcccacct gtggacacca gatcctggga gctcctggtt agcaagtgag 60
atctctggga tgtcagtgag gctggttgaa gaccagaggt aaactgcaga ggtcaccacc 120
cccaccatgt cccaggtgat gtccagccca ctgctggcag gaggccatgc tgtcagcttg 180
gcgccttgtg atgagcccag gaggaccctg cacccagcac ccagccccag cctgccaccc 240
cagtgttctt actacaccac ggaaggctgg ggagcccagg ccctgatggc ccccgtgccc 300
tgcatggggc cccctggccg actccagcaa gccccacagg tggaggccaa agccacctgc 360
ttcctgccgt cccctggtga gaaggccttg ggaccccag aggaccttga ctcctacatt 420
gacttctcac tggagagcct caatcagatg atcctggaac tggaccccac cttccagctg 480
cttccccag ggactggggg ctcccaggct gagctggccc agagcaccat gtcaatgaga 540
aagaaggagg aatctgaagc cttggacata aagtacatcg aggtgacctc cgccagatca 600
aggtgccacg attggcccca gcactgctcc agcccctctg tcaccccgcc cttcggctcc 660
cctcgcagtg gtggcctcct cctttccaga gacgtccccc gagagacacg aagcagcagt 720
gagagcctca tcttctctgg gaaccagggc agggggcacc agcgccctct gccccctca 780
gagggtctct cccctcgacc cccaaattcc cccagcatct caatcccttg catggggagc 840
aaggcctcga gcccccatgg tttgggctcc ccgctggtgg cttctccaag actggagaag 900
cggctgggag gcctggcccc acagcggggc agcaggatct ctgtgctgtc agccagccca 960
gtgtctgatg tcagctatat gtttggaagc agccagtccc tcctgcactc cagcaactcc 1020
agccatcagt catcttccag atccttggaa agtccagcca actcttcctc cagcctccac 1080
agccttggct cagtgtccct gtgtacaaga cccagtgact tccaggctcc cagaaacccc 1140
accctaacca tgggccaacc cagaacaccc cactctccac cactggccaa agaacatgcc 1200
agcatctgcc ccccatccat caccaactcc atggtggaca tacccattgt gctgatcaac 1260
ggctgcccag aaccagggtc ttctccaccc cagcggaccc caggacacca gaactccgtt 1320
caacctggag ctgcttctcc cagcaacccc tgtccagcca ccaggagcaa cagccagacc 1380
ctgtcagatg ccccctttac cacatgccca gagggtcccg ccagggacat gcagcccacc 1440
atgaagttcg tgatggacac atctaaatac tggtttaagc caaacatcac ccgagagcaa 1500
gcaatcgagc tgctgaggaa ggaggagcca ggggcttttg tcataaggga cagctcttca 1560
taccgaggct ccttcggcct ggccctgaag gtgcaggagg ttccccgcgtc tgctcagaat 1620
cgaccaggtg aggacagcaa tgacctcatc cgacacttcc tcatcgagtc gtctgccaaa 1680
ggagtgcatc tcaaaggagc agatgaggag ccctactttg ggagcctctc tgccttcgtg 1740
tgccagcatt ccatcatggc cctggccctg ccctgcaaac tcaccatccc acagagagaa 1800
ctgggaggtg cagatggggc ctcggactct acagacagcc cagcctcctg ccagaagaaa 1860
tctgcgggct gccacaccct gtacctgagc tcagtgagcg tggagaccct gactggagcc 1920
ctggccgtgc agaaagccat ctccaccacc tttgagaggg acatcctccc cacgcccacc 1980
gtggtccact tcgaagtcac agagcagggc atcactctga ctgatgtcca gaggaaggtg 2040
tttttccggc gccattaccc actcaccacc ctccgcttct gtggtatgga ccctgagcaa 2100
cggaagtggc agaagtactg caaaccctcc tggatctttg ggtttgtggc caagagccag 2160
acagagcctc aggagaacgt atgccacctc tttgcggagt atgacatggt ccagccagcc 2220
tcgcaggtca tcggcctggt gactgctctg ctgcaggacg cagaaaggat gtaggggaga 2280
gactgcctgt gcacctaacc aacacctcca ggggctcgct aaggagcccc cctccacccc 2340

```
ctgaatgggt gtggcttgtg gccatattga cagaccaatc tatgggacta gggggattgg 2400
catcaagttg acacccttga acctgctatg gccttcagca gtcaccatca tccagacccc 2460
ccgggcctca gtttcctcaa tcatagaaga agaccaatag acaagatcag ctgttcttag 2520
atgctggtgg gcatttgaac atgctcctcc atgattctga agcatgcaca cctctgaaga 2580
cccctgcatg aaaataacct ccaaggaccc tctgacccca tcgacctggg ccctgcccac 2640
acaacagtct gagcaagaga cctgcagccc ctgtttcgtg gcagacagca ggtgcctggc 2700
ggtgacccac ggggctcctg gcttgcagct ggtgatggtc aagaactgac tacaaaacag 2760
gaatggatag actctatttc cttccatatc tgttcctctg ttccttttcc cactttctgg 2820
gtggcttttt gggtccaccc agccaggatg ctgcaggcca agctgggtgt ggtatttagg 2880
gcagctcagc aggggaact tgtccccatg gtcagaggag acccagctgt cctgcacccc 2940
cttgcagatg agtatcaccc catcttttct ttccacttgg tttttatttt tatttttttt 3000
gagacagagt ctcactgtca cccaggctga actgcagtgg tgtgatctag gctcactgca 3060
acctccacct cccaggttca agcaattatc ctgcctcagg ctcccgagta gctgggatta 3120
caggcatgtg caactcaccc agctaatttt gtatttttag tagagacagg gtttcaccat 3180
gttggccagg ctggtcttga actcctgacc gcaggtaatc cacctgcttc ggcctcccaa 3240
agtgctggga ttacaggcgc aagccaccca gcccagcttc tttccattcc ttgataggcg 3300
agtattccaa agctggtatc gtagctgccc taatgttgca tattaggcgg cgggggcaga 3360
gataagggcc atctctctgt gattctgcct cagctcctgt cttgctgagc cctcccccaa 3420
cccacgctcc aacacacaca cacacacaca cacacacaca cacacacaca cacacacaca 3480
cacgcccctc tactgctatg tggcttcaac cagcctcaca gccacacggg ggaagcagag 3540
agtcaagaat gcaaagaggc cgcttcccta agaggcttgg aggagctggg ctctatccca 3600
cacccacccc caccccaccc ccacccagcc tccagaagct ggaaccattt ctcccgcagg 3660
cctgagttcc taaggaaacc accctaccgg ggtggaaggg agggtcaggg aagaaaccca 3720
ctcttgctct acgaggagca agtgcctgcc ccctcccagc agccagccct gccaaagttg 3780
cattatcttt ggccaaggct gggcctgacg gttatgattt cagccctggg cctgcaggag 3840
aggctgagat cagcccaccc agccagtggt cgagcactgc cccgccgcca aagtctgcag 3900
aatgtgagat gaggttctca aggtcacagg ccccagtccc agcctggggg ctggcagagg 3960
cccccatata ctctgctaca gctcctatca tgaaaaataa aatgt 4005
```

<210> 76

<211> 1093

<212> PRT

<213> Homo sapiens

<400> 76

```
Met Lys Glu Met Val Gly Gly Cys Cys Val Cys Ser Asp Glu Arg Gly
1               5                   10                  15
Trp Ala Glu Asn Pro Leu Val Tyr Cys Asp Gly His Ala Cys Ser Val
            20                  25                  30
Ala Val His Gln Ala Cys Tyr Gly Ile Val Gln Val Pro Thr Gly Pro
        35                  40                  45
Trp Phe Cys Arg Lys Cys Glu Ser Gln Glu Arg Ala Ala Arg Val Arg
    50                  55                  60
Cys Glu Leu Cys Pro His Lys Asp Gly Ala Leu Lys Arg Thr Asp Asn
65                  70                  75                  80
Gly Gly Trp Ala His Val Val Cys Ala Leu Tyr Ile Pro Glu Val Gln
                85                  90                  95
Phe Ala Asn Val Leu Thr Met Glu Pro Ile Val Leu Gln Tyr Val Pro
            100                 105                 110
His Asp Arg Phe Asn Lys Thr Cys Tyr Ile Cys Glu Glu Thr Gly Arg
        115                 120                 125
Glu Ser Lys Ala Ala Ser Gly Ala Cys Met Thr Cys Asn Arg His Gly
    130                 135                 140
```

Cys Arg Gln Ala Phe His Val Thr Cys Ala Gln Met Ala Gly Leu Leu
145 150 155 160
Cys Glu Glu Glu Val Leu Glu Val Asp Asn Val Lys Tyr Cys Gly Tyr
165 170 175
Cys Lys Tyr His Phe Ser Lys Met Lys Thr Ser Arg His Ser Ser Gly
180 185 190
Gly Gly Gly Gly Gly Ala Gly Gly Gly Gly Gly Ser Met Gly Gly Gly
195 200 205
Gly Ser Gly Phe Ile Ser Gly Arg Arg Ser Arg Ser Ala Ser Pro Ser
210 215 220
Thr Gln Gln Glu Lys His Pro Thr His His Glu Arg Gly Gln Lys Lys
225 230 235 240
Ser Arg Lys Asp Lys Glu Arg Leu Lys Gln Lys His Lys Lys Arg Pro
245 250 255
Glu Ser Pro Pro Ser Ile Leu Thr Pro Pro Val Val Pro Thr Ala Asp
260 265 270
Lys Val Ser Ser Ser Ala Ser Ser Ser Ser His His Glu Ala Ser Thr
275 280 285
Gln Glu Thr Ser Glu Ser Ser Arg Glu Ser Lys Gly Lys Lys Ser Ser
290 295 300
Ser His Ser Leu Ser His Lys Gly Lys Lys Leu Ser Ser Gly Lys Gly
305 310 315 320
Val Ser Ser Phe Thr Ser Ala Ser Ser Ser Ser Ser Ser Ser Ser Ser
325 330 335
Ser Ser Gly Gly Pro Phe Gln Pro Ala Val Ser Ser Leu Gln Ser Ser
340 345 350
Pro Asp Phe Ser Ala Phe Pro Lys Leu Glu Gln Pro Glu Glu Asp Lys
355 360 365
Tyr Ser Lys Pro Thr Ala Pro Ala Pro Ser Ala Pro Pro Ser Pro Ser
370 375 380
Ala Pro Glu Pro Pro Lys Ala Asp Leu Phe Glu Gln Lys Val Val Phe
385 390 395 400
Ser Gly Phe Gly Pro Ile Met Arg Phe Ser Thr Thr Thr Ser Ser Ser
405 410 415
Gly Arg Ala Arg Ala Pro Ser Pro Gly Asp Tyr Lys Ser Pro His Val
420 425 430
Thr Gly Ser Gly Ala Ser Ala Gly Thr His Lys Arg Met Pro Ala Leu
435 440 445
Ser Ala Thr Pro Val Pro Ala Asp Glu Thr Pro Glu Thr Gly Leu Lys
450 455 460
Glu Lys Lys His Lys Ala Ser Lys Arg Ser Arg His Gly Pro Gly Arg
465 470 475 480
Pro Lys Gly Ser Arg Asn Lys Glu Gly Thr Gly Gly Pro Ala Ala Pro
485 490 495
Ser Leu Pro Ser Ala Gln Leu Ala Gly Phe Thr Ala Thr Ala Ala Ser
500 505 510
Pro Phe Ser Gly Gly Ser Leu Val Ser Ser Gly Leu Gly Gly Leu Ser
515 520 525
Ser Arg Thr Phe Gly Pro Ser Gly Ser Leu Pro Ser Leu Ser Leu Glu
530 535 540
Ser Pro Leu Leu Gly Ala Gly Ile Tyr Thr Ser Asn Lys Asp Pro Ile
545 550 555 560
Ser His Ser Gly Gly Met Leu Arg Ala Val Cys Ser Thr Pro Leu Ser
565 570 575
Ser Ser Leu Leu Gly Pro Pro Gly Thr Ser Ala Leu Pro Arg Leu Ser
580 585 590
Arg Ser Pro Phe Thr Ser Thr Leu Pro Ser Ser Ser Ala Ser Ile Ser
595 600 605

Thr Thr Gln Val Phe Ser Leu Ala Gly Ser Thr Phe Ser Leu Pro Ser
610 615 620
Thr His Ile Phe Gly Thr Pro Met Gly Ala Val Asn Pro Leu Leu Ser
625 630 635 640
Gln Ala Glu Ser Ser His Thr Glu Pro Asp Leu Glu Asp Cys Ser Phe
645 650 655
Arg Cys Arg Gly Thr Ser Pro Gln Glu Ser Leu Ser Ser Met Ser Pro
660 665 670
Ile Ser Ser Leu Pro Ala Leu Phe Asp Gln Thr Ala Ser Ala Pro Cys
675 680 685
Gly Gly Gly Gln Leu Asp Pro Ala Ala Pro Gly Thr Thr Asn Met Glu
690 695 700
Gln Leu Leu Glu Lys Gln Gly Asp Gly Glu Ala Gly Val Asn Ile Val
705 710 715 720
Glu Met Leu Lys Ala Leu His Ala Leu Gln Lys Glu Asn Gln Arg Leu
725 730 735
Gln Glu Gln Ile Leu Ser Leu Thr Ala Lys Lys Glu Arg Leu Gln Ile
740 745 750
Leu Asn Val Gln Leu Ser Val Pro Phe Pro Ala Leu Pro Ala Ala Leu
755 760 765
Pro Ala Ala Asn Gly Pro Val Pro Gly Pro Tyr Gly Leu Pro Pro Gln
770 775 780
Ala Gly Ser Ser Asp Ser Leu Ser Thr Ser Lys Ser Pro Pro Gly Lys
785 790 795 800
Ser Ser Leu Gly Leu Asp Asn Ser Leu Ser Thr Ser Ser Glu Asp Pro
805 810 815
His Ser Gly Cys Pro Ser Arg Ser Ser Ser Ser Leu Ser Phe His Ser
820 825 830
Thr Pro Pro Pro Leu Pro Leu Leu Gln Gln Ser Pro Ala Thr Leu Pro
835 840 845
Leu Ala Leu Pro Gly Ala Pro Ala Pro Leu Pro Pro Gln Pro Gln Asn
850 855 860
Gly Leu Gly Arg Ala Pro Gly Ala Ala Gly Leu Gly Ala Met Pro Met
865 870 875 880
Ala Glu Gly Leu Leu Gly Gly Leu Ala Gly Ser Gly Gly Leu Pro Leu
885 890 895
Asn Gly Leu Leu Gly Gly Leu Asn Gly Ala Ala Ala Pro Asn Pro Ala
900 905 910
Ser Leu Ser Gln Ala Gly Gly Ala Pro Thr Leu Gln Leu Pro Gly Cys
915 920 925
Leu Asn Ser Leu Thr Glu Gln Gln Arg His Leu Leu Gln Gln Gln Glu
930 935 940
Gln Gln Leu Gln Gln Leu Gln Gln Leu Leu Ala Ser Pro Gln Leu Thr
945 950 955 960
Pro Glu His Gln Thr Val Val Tyr Gln Met Ile Gln Gln Ile Gln Gln
965 970 975
Lys Arg Glu Leu Gln Arg Leu Gln Met Ala Gly Gly Ser Gln Leu Pro
980 985 990
Met Ala Ser Leu Leu Ala Gly Ser Ser Thr Pro Leu Leu Ser Ala Gly
995 1000 1005
Thr Pro Gly Leu Leu Pro Thr Ala Ser Ala Pro Pro Leu Leu Pro
1010 1015 1020
Ala Gly Ala Leu Val Ala Pro Ser Leu Gly Asn Asn Thr Ser Leu
1025 1030 1035
Met Ala Ala Ala Ala Ala Ala Ala Ala Val Ala Ala Ala Gly Gly
1040 1045 1050
Pro Pro Val Leu Thr Ala Gln Thr Asn Pro Phe Leu Ser Leu Ser
1055 1060 1065

Gly Ala Glu Gly Ser Gly Gly Gly Pro Lys Gly Gly Thr Ala Asp
1070 1075 1080
Lys Gly Ala Ser Ala Asn Gln Glu Lys Gly
1085 1090

<210> 77

<211> 344

<212> PRT

<213> Homo sapiens

<400> 77
Met His Arg Thr Thr Arg Ile Lys Ile Thr Glu Leu Asn Pro His Leu
1 5 10 15
Met Cys Ala Leu Cys Gly Gly Tyr Phe Ile Asp Ala Thr Thr Ile Val
20 25 30
Glu Cys Leu His Ser Phe Cys Lys Thr Cys Ile Val Arg Tyr Leu Glu
35 40 45
Thr Asn Lys Tyr Cys Pro Met Cys Asp Val Gln Val His Lys Thr Arg
50 55 60
Pro Leu Leu Ser Ile Arg Ser Asp Lys Thr Leu Gln Asp Ile Val Tyr
65 70 75 80
Lys Leu Val Pro Gly Leu Phe Lys Asp Glu Met Lys Arg Arg Arg Asp
85 90 95
Phe Tyr Ala Ala Tyr Pro Leu Thr Glu Val Pro Asn Gly Ser Asn Glu
100 105 110
Asp Arg Gly Glu Val Leu Glu Gln Glu Lys Gly Ala Leu Ser Asp Asp
115 120 125
Glu Ile Val Ser Leu Ser Ile Glu Phe Tyr Glu Gly Ala Arg Asp Arg
130 135 140
Asp Glu Lys Lys Gly Pro Leu Glu Asn Gly Asp Gly Asp Lys Glu Lys
145 150 155 160
Thr Gly Val Arg Phe Leu Arg Cys Pro Ala Ala Met Thr Val Met His
165 170 175
Leu Ala Lys Phe Leu Arg Asn Lys Met Asp Val Pro Ser Lys Tyr Lys
180 185 190
Val Glu Val Leu Tyr Glu Asp Glu Pro Leu Lys Glu Tyr Tyr Thr Leu
195 200 205
Met Asp Ile Ala Tyr Ile Tyr Pro Trp Arg Arg Asn Gly Pro Leu Pro
210 215 220
Leu Lys Tyr Arg Val Gln Pro Ala Cys Lys Arg Leu Thr Leu Ala Thr
225 230 235 240
Val Pro Thr Pro Ser Glu Gly Thr Asn Thr Ser Gly Ala Ser Glu Cys
245 250 255
Glu Ser Val Ser Asp Lys Ala Pro Ser Pro Ala Thr Leu Pro Ala Thr
260 265 270
Ser Ser Ser Leu Pro Ser Pro Ala Thr Pro Ser His Gly Ser Pro Ser
275 280 285
Ser His Gly Pro Pro Ala Thr His Pro Thr Ser Pro Thr Pro Pro Ser
290 295 300
Thr Ala Ser Gly Ala Thr Thr Ala Ala Asn Gly Gly Ser Leu Asn Cys
305 310 315 320
Leu Gln Thr Pro Ser Ser Thr Ser Arg Gly Arg Lys Met Thr Val Asn
325 330 335

Gly Ala Pro Val Pro Pro Leu Thr
340

<210> 78

<211> 416

<212> PRT

<213> Homo sapiens

<400> 78
Met Ser Ser Asn Cys Thr Ser Thr Thr Ala Val Ala Val Ala Pro Leu
1 5 10 15
Ser Ala Ser Lys Thr Lys Thr Lys Lys Lys His Phe Val Cys Gln Lys
20 25 30
Val Lys Leu Phe Arg Ala Ser Glu Pro Ile Leu Ser Val Leu Met Trp
35 40 45
Gly Val Asn His Thr Ile Asn Glu Leu Ser Asn Val Pro Val Pro Val
50 55 60
Met Leu Met Pro Asp Asp Phe Lys Ala Tyr Ser Lys Ile Lys Val Asp
65 70 75 80
Asn His Leu Phe Asn Lys Glu Asn Leu Pro Ser Arg Phe Lys Phe Lys
85 90 95
Glu Tyr Cys Pro Met Val Phe Arg Asn Leu Arg Glu Arg Phe Gly Ile
100 105 110
Asp Asp Gln Asp Tyr Gln Asn Ser Val Thr Arg Ser Ala Pro Ile Asn
115 120 125
Ser Asp Ser Gln Gly Arg Cys Gly Thr Arg Phe Leu Thr Thr Tyr Asp
130 135 140
Arg Arg Phe Val Ile Lys Thr Val Ser Ser Glu Asp Val Ala Glu Met
145 150 155 160
His Asn Ile Leu Lys Lys Tyr His Gln Phe Ile Val Glu Cys His Gly
165 170 175
Asn Thr Leu Leu Pro Gln Phe Leu Gly Met Tyr Arg Leu Thr Val Asp
180 185 190
Gly Val Glu Thr Tyr Met Val Val Thr Arg Asn Val Phe Ser His Arg
195 200 205
Leu Thr Val His Arg Lys Tyr Asp Leu Lys Gly Ser Thr Val Ala Arg
210 215 220
Glu Ala Ser Asp Lys Glu Lys Ala Lys Asp Leu Pro Thr Phe Lys Asp
225 230 235 240
Asn Asp Phe Leu Asn Glu Gly Gln Lys Leu His Val Gly Glu Glu Ser
245 250 255
Lys Lys Asn Phe Leu Glu Lys Leu Lys Arg Asp Val Glu Phe Leu Ala
260 265 270
Gln Leu Lys Ile Met Asp Tyr Ser Leu Leu Val Gly Ile His Asp Val
275 280 285
Asp Arg Ala Glu Gln Glu Glu Met Glu Val Glu Glu Arg Ala Glu Asp
290 295 300
Glu Glu Cys Glu Asn Asp Gly Val Gly Gly Asn Leu Leu Cys Ser Tyr
305 310 315 320
Gly Thr Pro Pro Asp Ser Pro Gly Asn Leu Leu Ser Phe Pro Arg Phe
325 330 335
Phe Gly Pro Gly Glu Phe Asp Pro Ser Val Asp Val Tyr Ala Met Lys
340 345 350

Ser His Glu Ser Ser Pro Lys Lys Glu Val Tyr Phe Met Ala Ile Ile
355 360 365
Asp Ile Leu Thr Pro Tyr Asp Thr Lys Lys Lys Ala Ala His Ala Ala
370 375 380
Lys Thr Val Lys His Gly Ala Gly Ala Glu Ile Ser Thr Val Asn Pro
385 390 395 400
Glu Gln Tyr Ser Lys Arg Phe Asn Glu Phe Met Ser Asn Ile Leu Thr
405 410 415

<210> 79

<211> 500

<212> PRT

<213> Homo sapiens

<400> 79
Met Arg Gly Glu Leu Trp Leu Leu Val Leu Val Leu Arg Glu Ala Ala
1 5 10 15
Arg Ala Leu Ser Pro Gln Pro Gly Ala Gly His Asp Glu Gly Pro Gly
20 25 30
Ser Gly Trp Ala Ala Lys Gly Thr Val Arg Gly Trp Asn Arg Arg Ala
35 40 45
Arg Glu Ser Pro Gly His Val Ser Glu Pro Asp Arg Thr Gln Leu Ser
50 55 60
Gln Asp Leu Gly Gly Gly Thr Leu Ala Met Asp Thr Leu Pro Asp Asn
65 70 75 80
Arg Thr Arg Val Val Glu Asp Asn His Ser Tyr Tyr Val Ser Arg Leu
85 90 95
Tyr Gly Pro Ser Glu Pro His Ser Arg Glu Leu Trp Val Asp Val Ala
100 105 110
Glu Ala Asn Arg Ser Gln Val Lys Ile His Thr Ile Leu Ser Asn Thr
115 120 125
His Arg Gln Ala Ser Arg Val Val Leu Ser Phe Asp Phe Pro Phe Tyr
130 135 140
Gly His Pro Leu Arg Gln Ile Thr Ile Ala Thr Gly Gly Phe Ile Phe
145 150 155 160
Met Gly Asp Val Ile His Arg Met Leu Thr Ala Thr Gln Tyr Val Ala
165 170 175
Pro Leu Met Ala Asn Phe Asn Pro Gly Tyr Ser Asp Asn Ser Thr Val
180 185 190
Val Tyr Phe Asp Asn Gly Thr Val Phe Val Val Gln Trp Asp His Val
195 200 205
Tyr Leu Gln Gly Trp Glu Asp Lys Gly Ser Phe Thr Phe Gln Ala Ala
210 215 220
Leu His His Asp Gly Arg Ile Val Phe Ala Tyr Lys Glu Ile Pro Met
225 230 235 240
Ser Val Pro Glu Ile Ser Ser Ser Gln His Pro Val Lys Thr Gly Leu
245 250 255
Ser Asp Ala Phe Met Ile Leu Asn Pro Ser Pro Asp Val Pro Glu Ser
260 265 270
Arg Arg Arg Ser Ile Phe Glu Tyr His Arg Ile Glu Leu Asp Pro Ser
275 280 285
Lys Val Thr Ser Met Ser Ala Val Glu Phe Thr Pro Leu Pro Thr Cys
290 295 300

Leu Gln His Arg Ser Cys Asp Ala Cys Met Ser Ser Asp Leu Thr Phe
305 310 315 320
Asn Cys Ser Trp Cys His Val Leu Gln Arg Cys Ser Ser Gly Phe Asp
325 330 335
Arg Tyr Arg Gln Glu Trp Met Asp Tyr Gly Cys Ala Gln Glu Ala Glu
340 345 350
Gly Arg Met Cys Glu Asp Phe Gln Asp Glu Asp His Asp Ser Ala Ser
355 360 365
Pro Asp Thr Ser Phe Ser Pro Tyr Asp Gly Asp Leu Thr Thr Thr Ser
370 375 380
Ser Ser Leu Phe Ile Asp Ser Leu Thr Thr Glu Asp Asp Thr Lys Leu
385 390 395 400
Asn Pro Tyr Ala Gly Gly Asp Gly Leu Gln Asn Asn Leu Ser Pro Lys
405 410 415
Thr Lys Gly Thr Pro Val His Leu Gly Thr Ile Val Gly Ile Val Leu
420 425 430
Ala Val Leu Leu Val Ala Ala Ile Ile Leu Ala Gly Ile Tyr Ile Asn
435 440 445
Gly His Pro Thr Ser Asn Ala Ala Leu Phe Phe Ile Glu Arg Arg Pro
450 455 460
His His Trp Pro Ala Met Lys Phe Arg Ser His Pro Asp His Ser Thr
465 470 475 480
Tyr Ala Glu Val Glu Pro Ser Gly His Glu Lys Glu Gly Phe Met Glu
485 490 495
Ala Glu Gln Cys
500

<210> 80

<211> 509

<212> PRT

<213> Homo sapiens

<400> 80
Met Glu Asp Ile Gln Thr Asn Ala Glu Leu Lys Ser Thr Gln Glu Gln
1 5 10 15
Ser Val Pro Ala Glu Ser Ala Ala Val Leu Asn Asp Tyr Ser Leu Thr
20 25 30
Lys Ser His Glu Met Glu Asn Val Asp Ser Gly Glu Gly Pro Ala Asn
35 40 45
Glu Asp Glu Asp Ile Gly Asp Asp Ser Met Lys Val Lys Asp Glu Tyr
50 55 60
Ser Glu Arg Asp Glu Asn Val Leu Lys Ser Glu Pro Met Gly Asn Ala
65 70 75 80
Glu Glu Pro Glu Ile Pro Tyr Ser Tyr Ser Arg Glu Tyr Asn Glu Tyr
85 90 95
Glu Asn Ile Lys Leu Glu Arg His Val Val Ser Phe Asp Ser Ser Arg
100 105 110
Pro Thr Ser Gly Lys Met Asn Cys Asp Val Cys Gly Leu Ser Cys Ile
115 120 125
Ser Phe Asn Val Leu Met Val His Lys Arg Ser His Thr Gly Glu Arg
130 135 140
Pro Phe Gln Cys Asn Gln Cys Gly Ala Ser Phe Thr Gln Lys Gly Asn
145 150 155 160

Leu Leu Arg His Ile Lys Leu His Thr Gly Glu Lys Pro Phe Lys Cys
165 170 175
His Leu Cys Asn Tyr Ala Cys Gln Arg Arg Asp Ala Leu Thr Gly His
180 185 190
Leu Arg Thr His Ser Val Glu Lys Pro Tyr Lys Cys Glu Phe Cys Gly
195 200 205
Arg Ser Tyr Lys Gln Arg Ser Ser Leu Glu Glu His Lys Glu Arg Cys
210 215 220
Arg Thr Phe Leu Gln Ser Thr Asp Pro Gly Asp Thr Ala Ser Ala Glu
225 230 235 240
Ala Arg His Ile Lys Ala Glu Met Gly Ser Glu Arg Ala Leu Val Leu
245 250 255
Asp Arg Leu Ala Ser Asn Val Ala Lys Arg Lys Ser Ser Met Pro Gln
260 265 270
Lys Phe Ile Gly Glu Lys Arg His Cys Phe Asp Val Asn Tyr Asn Ser
275 280 285
Ser Tyr Met Tyr Glu Lys Glu Ser Glu Leu Ile Gln Thr Arg Met Met
290 295 300
Asp Gln Ala Ile Asn Asn Ala Ile Ser Tyr Leu Gly Ala Glu Ala Leu
305 310 315 320
Cys Pro Leu Val Gln Thr Pro Pro Ala Pro Thr Ser Glu Met Val Pro
325 330 335
Val Ile Ser Ser Met Tyr Pro Ile Ala Leu Thr Arg Ala Glu Met Ser
340 345 350
Asn Gly Ala Pro Gln Glu Leu Glu Arg Lys Ser Ile Leu Leu Pro Glu
355 360 365
Lys Ser Val Pro Ser Glu Arg Gly Leu Ser Pro Asn Asn Ser Gly His
370 375 380
Asp Ser Thr Asp Thr Asp Ser Asn His Glu Glu Arg Gln Asn His Ile
385 390 395 400
Tyr Gln Gln Asn His Met Val Leu Ser Arg Ala Arg Asn Gly Met Pro
405 410 415
Leu Leu Lys Glu Val Pro Arg Ser Tyr Glu Leu Leu Lys Pro Pro Pro
420 425 430
Ile Cys Pro Arg Asp Ser Val Lys Val Ile Asp Lys Glu Gly Glu Val
435 440 445
Met Asp Val Tyr Arg Cys Asp His Cys Arg Val Leu Phe Leu Asp Tyr
450 455 460
Val Met Phe Thr Ile His Met Gly Cys His Gly Phe Arg Asp Pro Phe
465 470 475 480
Glu Cys Asn Met Cys Gly Asp Arg Ser His Asp Arg Tyr Glu Phe Ser
485 490 495
Ser His Ile Ala Arg Gly Glu His Arg Ser Leu Leu Lys
500 505

<210> 81

<211> 440

<212> PRT

<213> Homo sapiens

<400> 81

```
Met Pro Ile Pro Pro Pro Pro Pro Pro Pro Pro Gly Pro Pro Pro Pro
1               5                   10                  15
Pro Thr Phe His Gln Ala Asn Thr Glu Gln Pro Lys Leu Ser Arg Asp
            20                  25                  30
Glu Gln Arg Gly Arg Gly Ala Leu Leu Gln Asp Ile Cys Lys Gly Thr
        35                  40                  45
Lys Leu Lys Lys Val Thr Asn Ile Asn Asp Arg Ser Ala Pro Ile Leu
    50                  55                  60
Glu Lys Pro Lys Gly Ser Ser Gly Gly Tyr Gly Ser Gly Gly Ala Ala
65                  70                  75                  80
Leu Gln Pro Lys Gly Gly Leu Phe Gln Gly Gly Val Leu Lys Leu Arg
                85                  90                  95
Pro Val Gly Ala Lys Asp Gly Ser Glu Asn Leu Ala Gly Lys Pro Ala
            100                 105                 110
Leu Gln Ile Pro Ser Ser Arg Ala Ala Ala Pro Arg Pro Pro Val Ser
        115                 120                 125
Ala Ala Ser Gly Arg Pro Gln Asp Asp Thr Asp Ser Ser Arg Ala Ser
    130                 135                 140
Leu Pro Glu Leu Pro Arg Met Gln Arg Pro Ser Leu Pro Asp Leu Ser
145                 150                 155                 160
Arg Pro Asn Thr Thr Ser Ser Thr Gly Met Lys His Ser Ser Ser Ala
                165                 170                 175
Pro Pro Pro Pro Pro Pro Gly Arg Arg Ala Asn Ala Pro Pro Thr Pro
            180                 185                 190
Leu Pro Met His Ser Ser Lys Ala Pro Ala Tyr Asn Arg Glu Lys Pro
        195                 200                 205
Leu Pro Pro Thr Pro Gly Gln Arg Leu His Pro Gly Arg Glu Gly Pro
    210                 215                 220
Pro Ala Pro Pro Pro Val Lys Pro Pro Pro Ser Pro Val Asn Ile Arg
225                 230                 235                 240
Thr Gly Pro Ser Gly Gln Ser Leu Ala Pro Pro Pro Pro Pro Tyr Arg
                245                 250                 255
Gln Pro Pro Gly Val Pro Asn Gly Pro Ser Ser Pro Thr Asn Glu Ser
            260                 265                 270
Ala Pro Glu Leu Pro Gln Arg His Asn Ser Leu His Arg Lys Thr Pro
        275                 280                 285
Gly Pro Val Arg Gly Leu Ala Pro Pro Pro Pro Thr Ser Ala Ser Pro
    290                 295                 300
Ser Leu Leu Ser Asn Arg Pro Pro Pro Pro Ala Arg Asp Pro Pro Ser
305                 310                 315                 320
Arg Gly Ala Ala Pro Pro Pro Pro Pro Pro Val Ile Arg Asn Gly Ala
                325                 330                 335
Arg Asp Ala Pro Pro Pro Pro Pro Pro Tyr Arg Met His Gly Ser Glu
            340                 345                 350
Pro Pro Ser Arg Gly Lys Pro Pro Pro Pro Ser Arg Thr Pro Ala
        355                 360                 365
Gly Pro Pro Pro Pro Pro Pro Pro Pro Leu Arg Asn Gly His Arg Asp
    370                 375                 380
Ser Ile Thr Thr Val Arg Ser Phe Leu Asp Asp Phe Glu Ser Lys Tyr
385                 390                 395                 400
Ser Phe His Pro Val Glu Asp Phe Pro Ala Pro Glu Glu Tyr Lys His
                405                 410                 415
Phe Gln Arg Ile Tyr Pro Ser Lys Thr Asn Arg Ala Ala Arg Gly Ala
            420                 425                 430
Pro Pro Leu Pro Pro Ile Leu Arg
        435                 440
```

<210> 82

<211> 205

<212> PRT

<213> Homo sapiens

<400> 82
Met Ser Ile Met Ser Tyr Asn Gly Gly Ala Val Met Ala Met Lys Gly
1 5 10 15
Lys Asn Cys Val Ala Ile Ala Ala Asp Arg Arg Phe Gly Ile Gln Ala
20 25 30
Gln Met Val Thr Thr Asp Phe Gln Lys Ile Phe Pro Met Gly Asp Arg
35 40 45
Leu Tyr Ile Gly Leu Ala Gly Leu Ala Thr Asp Val Gln Thr Val Ala
50 55 60
Gln Arg Leu Lys Phe Arg Leu Asn Leu Tyr Glu Leu Lys Glu Gly Arg
65 70 75 80
Gln Ile Lys Pro Tyr Thr Leu Met Ser Met Val Ala Asn Leu Leu Tyr
85 90 95
Glu Lys Arg Phe Gly Pro Tyr Tyr Thr Glu Pro Val Ile Ala Gly Leu
100 105 110
Asp Pro Lys Thr Phe Lys Pro Phe Ile Cys Ser Leu Asp Leu Ile Gly
115 120 125
Cys Pro Met Val Thr Asp Asp Phe Val Val Ser Gly Thr Cys Ala Glu
130 135 140
Gln Met Tyr Gly Met Cys Glu Ser Leu Trp Glu Pro Asn Met Asp Pro
145 150 155 160
Asp His Leu Phe Glu Thr Ile Ser Gln Ala Met Leu Asn Ala Val Asp
165 170 175
Arg Asp Ala Val Ser Gly Met Gly Val Ile Val His Ile Ile Glu Lys
180 185 190
Asp Lys Ile Thr Thr Arg Thr Leu Lys Ala Arg Met Asp
195 200 205

<210> 83

<211> 190

<212> PRT

<213> Homo sapiens

<400> 83
Leu Thr Arg Ser Cys Ser Thr Cys Cys Pro Ala Val Ala Cys Leu Val
1 5 10 15
Gly Arg Gly Val Val Thr Ser Gly Ala Met His Gln Cys Trp Gly Glu
20 25 30
Glu Met Leu Gln Gly Met Leu Leu Trp Gly Trp Ala Thr Cys Pro Leu
35 40 45
Ser Asn Pro Gly Arg Trp Gly Arg Thr Val Gly Leu Gln His Pro Ala
50 55 60
Val Val Ser Ala Phe Arg Ala Leu Leu Leu Leu Met Leu Thr Val His
65 70 75 80

Val Ser Tyr Leu Ser Leu Ile Arg Phe Asp Tyr Gly Tyr Asn Leu Val
85 90 95
Ala Asn Val Ala Ile Gly Leu Val Asn Val Val Trp Trp Leu Ala Trp
100 105 110
Cys Leu Trp Asn Gln Arg Arg Leu Pro His Val Arg Lys Cys Val Val
115 120 125
Val Val Leu Leu Leu Gln Gly Leu Ser Leu Leu Glu Leu Leu Asp Phe
130 135 140
Pro Pro Leu Phe Trp Val Leu Asp Ala His Ala Ile Trp His Ile Ser
145 150 155 160
Thr Ile Pro Val His Val Leu Phe Phe Ser Phe Leu Glu Asp Asp Ser
165 170 175
Leu Tyr Leu Leu Lys Glu Ser Glu Asp Lys Phe Lys Leu Asp
180 185 190

<210> 84

<211> 368

<212> PRT

<213> Homo sapiens

<400> 84
Ala Pro Pro Pro Ala Ala Ser Gln Gly Glu Arg Met Ala Gly Leu Ala
1 5 10 15
Ala Arg Leu Val Leu Leu Ala Gly Ala Ala Ala Leu Ala Ser Gly Ser
20 25 30
Gln Gly Asp Arg Glu Pro Val Tyr Arg Asp Cys Val Leu Gln Cys Glu
35 40 45
Glu Gln Asn Cys Ser Gly Gly Ala Leu Asn His Phe Arg Ser Arg Gln
50 55 60
Pro Ile Tyr Met Ser Leu Ala Gly Trp Thr Cys Arg Asp Asp Cys Lys
65 70 75 80
Tyr Glu Cys Met Trp Val Thr Val Gly Leu Tyr Leu Gln Glu Gly His
85 90 95
Lys Val Pro Gln Phe His Gly Lys Trp Pro Phe Ser Arg Phe Leu Phe
100 105 110
Phe Gln Glu Pro Ala Ser Ala Val Ala Ser Phe Leu Asn Gly Leu Ala
115 120 125
Ser Leu Val Met Leu Cys Arg Tyr Arg Thr Phe Val Pro Ala Ser Ser
130 135 140
Pro Met Tyr His Thr Cys Val Ala Phe Ala Trp Val Ser Leu Asn Ala
145 150 155 160
Trp Phe Trp Ser Thr Val Phe His Thr Arg Asp Thr Asp Leu Thr Glu
165 170 175
Lys Met Asp Tyr Phe Cys Ala Ser Thr Val Ile Leu His Ser Ile Tyr
180 185 190
Leu Cys Cys Val Arg Thr Val Gly Leu Gln His Pro Ala Val Val Ser
195 200 205
Ala Phe Arg Ala Leu Leu Leu Leu Met Leu Thr Val His Val Ser Tyr
210 215 220
Leu Ser Leu Ile Arg Phe Asp Tyr Gly Tyr Asn Leu Val Ala Asn Val
225 230 235 240
Ala Ile Gly Leu Val Asn Val Val Trp Trp Leu Ala Trp Cys Leu Trp
245 250 255

Asn Gln Arg Arg Leu Pro His Val Arg Lys Cys Val Val Val Val Leu
260 265 270

Leu Leu Gln Gly Leu Ser Leu Leu Glu Leu Leu Asp Phe Pro Pro Leu
275 280 285

Phe Trp Val Leu Asp Ala His Ala Ile Trp His Ile Ser Thr Ile Pro
290 295 300

Val His Val Leu Phe Phe Ser Phe Leu Glu Asp Asp Ser Leu Tyr Leu
305 310 315 320

Leu Lys Glu Ser Glu Asp Lys Phe Lys Leu Val Glu Ala Asp Trp Ile
325 330 335

Phe Ala Leu Pro Leu Thr Pro Cys Pro Ser Leu Arg Glu Gly Ser Tyr
340 345 350

Ala Arg Thr Pro Thr Ser Gly Thr Arg Val Ala Cys Ala Ser Phe Phe
355 360 365

<210> 85

<211> 190

<212> PRT

<213> Homo sapiens

<400> 85

Leu Thr Arg Ser Cys Ser Thr Cys Cys Pro Ala Val Ala Cys Leu Val
1 5 10 15

Gly Arg Gly Val Val Thr Ser Gly Ala Met His Gln Cys Trp Gly Glu
20 25 30

Glu Met Leu Gln Gly Met Leu Leu Trp Gly Trp Ala Thr Cys Pro Leu
35 40 45

Ser Asn Pro Gly Arg Trp Gly Arg Thr Val Gly Leu Gln His Pro Ala
50 55 60

Val Val Ser Ala Phe Arg Ala Leu Leu Leu Leu Met Leu Thr Val His
65 70 75 80

Val Ser Tyr Leu Ser Leu Ile Arg Phe Asp Tyr Gly Tyr Asn Leu Val
85 90 95

Ala Asn Val Ala Ile Gly Leu Val Asn Val Val Trp Trp Leu Ala Trp
100 105 110

Cys Leu Trp Asn Gln Arg Arg Leu Pro His Val Arg Lys Cys Val Val
115 120 125

Val Val Leu Leu Leu Gln Gly Leu Ser Leu Leu Glu Leu Leu Asp Phe
130 135 140

Pro Pro Leu Phe Trp Val Leu Asp Ala His Ala Ile Trp His Ile Ser
145 150 155 160

Thr Ile Pro Val His Val Leu Phe Phe Ser Phe Leu Glu Asp Asp Ser
165 170 175

Leu Tyr Leu Leu Lys Glu Ser Glu Asp Lys Phe Lys Leu Asp
180 185 190

<210> 86

<211> 318

<212> PRT

<213> Homo sapiens

<400> 86

Met Ala Gly Leu Ala Ala Arg Leu Val Leu Leu Ala Gly Ala Ala Ala
1 5 10 15
Leu Ala Ser Gly Ser Gln Gly Asp Arg Glu Pro Val Tyr Arg Asp Cys
20 25 30
Val Leu Gln Cys Glu Glu Gln Asn Cys Ser Gly Gly Ala Leu Asn His
35 40 45
Phe Arg Ser Arg Gln Pro Ile Tyr Met Ser Leu Ala Gly Trp Thr Cys
50 55 60
Arg Asp Asp Cys Lys Tyr Glu Cys Met Trp Val Thr Val Gly Leu Tyr
65 70 75 80
Leu Gln Glu Gly His Lys Val Pro Gln Phe His Gly Lys Trp Pro Phe
85 90 95
Ser Arg Phe Leu Phe Phe Gln Glu Pro Ala Ser Ala Val Ala Ser Phe
100 105 110
Leu Asn Gly Leu Ala Ser Leu Val Met Leu Cys Arg Tyr Arg Thr Phe
115 120 125
Val Pro Ala Ser Ser Pro Met Tyr His Thr Cys Val Ala Phe Ala Trp
130 135 140
Val Ser Leu Asn Ala Trp Phe Trp Ser Thr Val Phe His Thr Arg Asp
145 150 155 160
Thr Asp Leu Gln Arg Lys Trp Thr Thr Ser Val Pro Pro Val Ser Tyr
165 170 175
Thr Gln Ser Thr Cys Ala Ala Ser Gly Pro Trp Gly Cys Ser Thr Gln
180 185 190
Leu Trp Ser Ser Ala Phe Arg Ala Leu Leu Leu Leu Met Leu Thr Val
195 200 205
His Val Ser Tyr Leu Ser Leu Ile Arg Phe Asp Tyr Gly Tyr Asn Leu
210 215 220
Val Ala Asn Val Ala Ile Gly Leu Val Asn Val Val Trp Trp Leu Ala
225 230 235 240
Trp Cys Leu Trp Asn Gln Arg Arg Leu Pro His Val Arg Lys Cys Val
245 250 255
Val Val Val Leu Leu Leu Gln Gly Leu Ser Leu Leu Glu Leu Leu Asp
260 265 270
Phe Pro Pro Leu Phe Trp Val Leu Asp Ala His Ala Ile Trp His Ile
275 280 285
Ser Thr Ile Pro Val His Val Leu Phe Phe Ser Phe Leu Glu Asp Asp
290 295 300

Ser Leu Tyr Leu Leu Lys Glu Ser Glu Asp Lys Phe Lys Leu
305 310 315

<210> 87

<211> 226

<212> PRT

<213> Homo sapiens

<400> 87

Met Ala Gly Leu Ala Ala Arg Leu Val Leu Leu Ala Gly Ala Ala Ala
1 5 10 15
Leu Ala Ser Gly Ser Gln Gly Asp Arg Glu Pro Val Tyr Arg Asp Cys
20 25 30
Val Leu Gln Cys Glu Glu Gln Asn Cys Ser Gly Gly Ala Leu Asn His
35 40 45
Phe Arg Ser Arg Gln Pro Ile Tyr Met Ser Leu Ala Gly Trp Thr Cys
50 55 60
Arg Asp Asp Cys Lys Tyr Glu Cys Met Trp Val Thr Val Gly Leu Tyr
65 70 75 80
Leu Gln Glu Gly His Lys Val Pro Gln Phe His Gly Lys Trp Pro Phe
85 90 95
Ser Arg Phe Leu Phe Phe Gln Glu Pro Ala Ser Ala Val Ala Ser Phe
100 105 110
Leu Asn Gly Leu Ala Ser Leu Val Met Leu Cys Arg Tyr Arg Thr Phe
115 120 125
Val Pro Ala Ser Ser Pro Met Tyr His Thr Cys Val Ala Phe Ala Trp
130 135 140
Val Ser Leu Asn Ala Trp Phe Trp Ser Thr Val Phe His Thr Arg Asp
145 150 155 160
Thr Asp Leu Thr Glu Lys Met Asp Tyr Phe Cys Ala Ser Thr Val Ile
165 170 175
Leu His Ser Ile Tyr Leu Cys Cys Val Arg Pro Gly Gln Arg Gly Val
180 185 190
Val Ala Gly Leu Val Pro Val Glu Pro Ala Ala Ala Ala Ser Arg Ala
195 200 205
Gln Val Arg Gly Gly Gly Leu Ala Ala Ala Gly Ala Val Pro Ala Arg
210 215 220
Ala Ala
225

<210> 88

<211> 320

<212> PRT

<213> Homo sapiens

<400> 88

Met Ala Gly Leu Ala Ala Arg Leu Val Leu Leu Ala Gly Ala Ala Ala
1 5 10 15
Leu Ala Ser Gly Ser Gln Gly Asp Arg Glu Pro Val Tyr Arg Asp Cys
20 25 30
Val Leu Gln Cys Glu Glu Gln Asn Cys Ser Gly Gly Ala Leu Asn His
35 40 45
Phe Arg Ser Arg Gln Pro Ile Tyr Met Ser Leu Ala Gly Trp Thr Cys
50 55 60
Arg Asp Asp Cys Lys Tyr Glu Cys Met Trp Val Thr Val Gly Leu Tyr
65 70 75 80
Leu Gln Glu Gly His Lys Val Pro Gln Phe His Gly Lys Trp Pro Phe
85 90 95
Ser Arg Phe Leu Phe Phe Gln Glu Pro Ala Ser Ala Val Ala Ser Phe
100 105 110
Leu Asn Gly Leu Ala Ser Leu Val Met Leu Cys Arg Tyr Arg Thr Phe
115 120 125

Val Pro Ala Ser Ser Pro Met Tyr His Thr Cys Val Ala Phe Ala Trp
130 135 140
Val Ser Leu Asn Ala Trp Phe Trp Ser Thr Val Phe His Thr Arg Asp
145 150 155 160
Thr Asp Leu Thr Glu Lys Met Asp Tyr Phe Cys Ala Ser Thr Val Ile
165 170 175
Leu His Ser Ile Tyr Leu Cys Cys Val Arg Thr Val Gly Leu Gln His
180 185 190
Pro Ala Val Val Ser Ala Phe Arg Ala Leu Leu Leu Leu Met Leu Thr
195 200 205
Val His Val Ser Tyr Leu Ser Leu Ile Arg Phe Asp Tyr Gly Tyr Asn
210 215 220
Leu Val Ala Asn Val Ala Ile Gly Leu Val Asn Val Val Trp Trp Leu
225 230 235 240
Ala Trp Cys Leu Trp Asn Gln Arg Arg Leu Pro His Val Arg Lys Cys
245 250 255
Val Val Val Val Leu Leu Leu Gln Gly Leu Ser Leu Leu Glu Leu Leu
260 265 270
Asp Phe Pro Pro Leu Phe Trp Val Leu Asp Ala His Ala Ile Trp His
275 280 285
Ile Ser Thr Ile Pro Val His Val Leu Phe Phe Ser Phe Leu Glu Asp
290 295 300
Asp Ser Leu Tyr Leu Leu Lys Glu Ser Glu Asp Lys Phe Lys Leu Asp
305 310 315 320

<210> 89

<211> 217

<212> PRT

<213> Homo sapiens

<400> 89
Ala Pro Pro Pro Ala Ala Ser Gln Gly Glu Arg Met Ala Gly Leu Ala
1 5 10 15
Ala Arg Leu Val Leu Leu Ala Gly Ala Ala Ala Leu Ala Ser Gly Ser
20 25 30
Gln Gly Asp Arg Glu Pro Val Tyr Arg Asp Cys Val Leu Gln Cys Glu
35 40 45
Glu Gln Asn Cys Ser Gly Gly Ala Leu Asn His Phe Arg Ser Arg Gln
50 55 60
Pro Ile Tyr Met Ser Leu Ala Gly Trp Thr Cys Arg Asp Asp Cys Lys
65 70 75 80
Tyr Glu Cys Met Trp Val Thr Val Gly Leu Tyr Leu Gln Glu Gly His
85 90 95
Lys Val Pro Gln Phe His Gly Lys Trp Pro Phe Ser Arg Phe Leu Phe
100 105 110
Phe Gln Glu Pro Ala Ser Ala Val Ala Ser Phe Leu Asn Gly Leu Ala
115 120 125
Ser Leu Val Met Leu Cys Arg Tyr Arg Thr Phe Val Pro Ala Ser Ser
130 135 140
Pro Met Tyr His Thr Cys Val Ala Phe Ala Trp Val Ser Leu Asn Ala
145 150 155 160
Trp Phe Trp Ser Thr Val Phe His Thr Arg Asp Thr Asp Leu Thr Glu
165 170 175

Lys Met Asp Tyr Phe Cys Ala Ser Thr Val Ile Leu His Ser Ile Tyr
180 185 190
Leu Cys Cys Val Ser Phe Leu Glu Asp Asp Ser Leu Tyr Leu Leu Lys
195 200 205
Glu Ser Glu Asp Lys Phe Lys Leu Asp
210 215

<210> 90

<211> 153

<212> PRT

<213> Homo sapiens

<400> 90
Met Asn Val Gly Thr Ala His Ser Glu Val Asn Pro Asn Thr Arg Val
1 5 10 15
Met Asn Ser Arg Gly Ile Trp Leu Ser Tyr Val Leu Ala Ile Gly Leu
20 25 30
Leu His Ile Val Leu Leu Ser Ile Pro Phe Val Ser Val Pro Val Val
35 40 45
Trp Thr Leu Thr Asn Leu Ile His Asn Met Gly Met Tyr Ile Phe Leu
50 55 60
His Thr Val Lys Gly Thr Pro Phe Glu Thr Pro Asp Gln Gly Lys Ala
65 70 75 80
Arg Leu Leu Thr His Trp Glu Gln Met Asp Tyr Gly Val Gln Phe Thr
85 90 95
Ala Ser Arg Lys Phe Leu Thr Ile Thr Pro Ile Val Leu Tyr Phe Leu
100 105 110
Thr Ser Phe Tyr Thr Lys Tyr Asp Gln Ile His Phe Val Leu Asn Thr
115 120 125
Val Ser Leu Met Ser Val Leu Ile Pro Lys Leu Pro Gln Leu His Gly
130 135 140
Val Arg Ile Phe Gly Ile Asn Lys Tyr
145 150

<210> 91

<211> 436

<212> PRT

<213> Homo sapiens

<400> 91
Met Arg Arg Asp Val Asn Gly Val Thr Lys Ser Arg Phe Glu Met Phe
1 5 10 15
Ser Asn Ser Asp Glu Ala Val Ile Asn Lys Lys Leu Pro Lys Glu Leu
20 25 30
Leu Leu Arg Ile Phe Ser Phe Leu Asp Val Val Thr Leu Cys Arg Cys
35 40 45
Ala Gln Val Ser Arg Ala Trp Asn Val Leu Ala Leu Asp Gly Ser Asn
50 55 60

Trp Gln Arg Ile Asp Leu Phe Asp Phe Gln Arg Asp Ile Glu Gly Arg
65                  70                  75                  80
Val Val Glu Asn Ile Ser Lys Arg Cys Gly Gly Phe Leu Arg Lys Leu
                85                  90                  95
Ser Leu Arg Gly Cys Leu Gly Val Gly Asp Asn Ala Leu Arg Thr Phe
            100                 105                 110
Ala Gln Asn Cys Arg Asn Ile Glu Val Leu Asn Leu Asn Gly Cys Thr
        115                 120                 125
Lys Thr Thr Asp Ala Thr Cys Thr Ser Leu Ser Lys Phe Cys Ser Lys
    130                 135                 140
Leu Arg His Leu Asp Leu Ala Ser Cys Thr Ser Ile Thr Asn Met Ser
145                 150                 155                 160
Leu Lys Ala Leu Ser Glu Gly Cys Pro Leu Leu Glu Gln Leu Asn Ile
                165                 170                 175
Ser Trp Cys Asp Gln Val Thr Lys Asp Gly Ile Gln Ala Leu Val Arg
            180                 185                 190
Gly Cys Gly Gly Leu Lys Ala Leu Phe Leu Lys Gly Cys Thr Gln Leu
        195                 200                 205
Glu Asp Glu Ala Leu Lys Tyr Ile Gly Ala His Cys Pro Glu Leu Val
    210                 215                 220
Thr Leu Asn Leu Gln Thr Cys Leu Gln Ile Thr Asp Glu Gly Leu Ile
225                 230                 235                 240
Thr Ile Cys Arg Gly Cys His Lys Leu Gln Ser Leu Cys Ala Ser Gly
                245                 250                 255
Cys Ser Asn Ile Thr Asp Ala Ile Leu Asn Ala Leu Gly Gln Asn Cys
            260                 265                 270
Pro Arg Leu Arg Ile Leu Glu Val Ala Arg Cys Ser Gln Leu Thr Asp
        275                 280                 285
Val Gly Phe Thr Thr Leu Ala Arg Asn Cys His Glu Leu Glu Lys Met
    290                 295                 300
Asp Leu Glu Glu Cys Val Gln Ile Thr Asp Ser Thr Leu Ile Gln Leu
305                 310                 315                 320
Ser Ile His Cys Pro Arg Leu Gln Val Leu Ser Leu Ser His Cys Glu
                325                 330                 335
Leu Ile Thr Asp Asp Gly Ile Arg His Leu Gly Asn Gly Ala Cys Ala
            340                 345                 350
His Asp Gln Leu Glu Val Ile Glu Leu Asp Asn Cys Pro Leu Ile Thr
        355                 360                 365
Asp Ala Ser Leu Glu His Leu Lys Ser Cys His Ser Leu Glu Arg Ile
    370                 375                 380
Glu Leu Tyr Asp Cys Gln Gln Ile Thr Arg Ala Gly Ile Lys Arg Leu
385                 390                 395                 400
Arg Thr His Leu Pro Asn Ile Lys Val His Ala Tyr Phe Ala Pro Val
                405                 410                 415
Thr Pro Pro Pro Ser Val Gly Gly Ser Arg Gln Arg Phe Cys Arg Cys
            420                 425                 430
Cys Ile Ile Leu
        435

<210> 92

<211> 204

<212> PRT

<213> Homo sapiens

<400> 92
Met Asp Pro Lys Asp Arg Lys Lys Ile Gln Phe Ser Val Pro Ala Pro
1 5 10 15
Pro Ser Gln Leu Asp Pro Arg Gln Val Glu Met Ile Arg Arg Arg Arg
20 25 30
Pro Thr Pro Ala Met Leu Phe Arg Leu Ser Glu His Ser Ser Pro Glu
35 40 45
Glu Glu Ala Ser Pro His Gln Arg Ala Ser Gly Glu Gly His His Leu
50 55 60
Lys Ser Lys Arg Pro Asn Pro Cys Ala Tyr Thr Pro Pro Ser Leu Lys
65 70 75 80
Ala Val Gln Arg Ile Ala Glu Ser His Leu Gln Ser Ile Ser Asn Leu
85 90 95
Asn Glu Asn Gln Ala Ser Glu Glu Glu Asp Glu Leu Gly Glu Leu Arg
100 105 110
Glu Leu Gly Tyr Pro Arg Glu Glu Asp Glu Glu Glu Glu Glu Asp Asp
115 120 125
Glu Glu Glu Glu Glu Glu Glu Asp Ser Gln Ala Glu Val Leu Lys Val
130 135 140
Ile Arg Gln Ser Ala Gly Gln Lys Thr Thr Cys Gly Gln Gly Leu Glu
145 150 155 160
Gly Pro Trp Glu Arg Pro Pro Pro Leu Asp Glu Ser Glu Arg Asp Gly
165 170 175
Gly Ser Glu Asp Gln Val Glu Asp Pro Ala Leu Ser Glu Pro Gly Glu
180 185 190
Glu Pro Gln Arg Pro Ser Pro Ser Glu Pro Gly Thr
195 200

<210> 93

<211> 115

<212> PRT

<213> Homo sapiens

<400> 93
Met Ser Gly Glu Pro Gly Gln Thr Ser Val Ala Pro Pro Pro Glu Glu
1 5 10 15
Val Glu Pro Gly Ser Gly Val Arg Ile Val Val Glu Tyr Cys Glu Pro
20 25 30
Cys Gly Phe Glu Ala Thr Tyr Leu Glu Leu Ala Ser Ala Val Lys Glu
35 40 45
Gln Tyr Pro Gly Ile Glu Ile Glu Ser Arg Leu Gly Gly Thr Gly Ala
50 55 60
Phe Glu Ile Glu Ile Asn Gly Gln Leu Val Phe Ser Lys Leu Glu Asn
65 70 75 80
Gly Gly Phe Pro Tyr Glu Lys Asp Leu Ile Glu Ala Ile Arg Arg Ala
85 90 95
Ser Asn Gly Glu Thr Leu Glu Lys Ile Thr Asn Ser Arg Pro Pro Cys
100 105 110
Val Ile Leu
115

<210> 94

<211> 144

<212> PRT

<213> Homo sapiens

<400> 94

Met Gly Ala Val Val Leu Cys Arg Pro Ser Pro Leu Asn Phe Leu Ile
1               5                   10                  15
Gln Thr Gly Thr Gly Gln Gly Leu Ser Cys Gly Ser His Met Trp Arg
            20                  25                  30
Cys Glu Ala Thr Pro Cys Gly Val Cys Gly Glu Ser Pro Val Gly Ser
        35                  40                  45
Leu Leu Lys Gln His Arg Gly Arg Gly Lys Thr Trp Pro Val Gly Thr
    50                  55                  60
Val Ser Ala Cys Arg Glu Glu Ser Glu Ala Gly Ser Leu Ser Leu Gly
65                  70                  75                  80
Trp Ser Leu Leu Pro Ser Pro Val Gly Leu Gly Ala Val Leu Ile Leu
                85                  90                  95
Lys Arg Cys Gly Ser Leu Cys Pro Leu Pro Gly Val Gln Gly Asn Arg
            100                 105                 110
Arg Gly His Trp Ala Cys Phe Leu Pro Pro Asp Pro Ala Ser Pro Thr
        115                 120                 125
Pro Cys Ile Ile Gly Asn Phe His Leu Lys Ile Phe Leu Ser Lys Val
    130                 135                 140

<210> 95

<211> 425

<212> PRT

<213> Homo sapiens

<400> 95

Met Gly Gly Gly Asp Leu Asn Leu Lys Lys Ser Trp His Pro Gln Thr
1               5                   10                  15
Leu Arg Asn Val Glu Lys Val Trp Lys Ala Glu Gln Lys His Glu Ala
            20                  25                  30
Glu Arg Lys Lys Ile Glu Glu Leu Gln Arg Glu Leu Arg Glu Glu Arg
        35                  40                  45
Ala Arg Glu Glu Met Gln Arg Tyr Ala Glu Asp Val Gly Ala Val Lys
    50                  55                  60
Lys Lys Glu Glu Lys Leu Asp Trp Met Tyr Gln Gly Pro Gly Gly Met
65                  70                  75                  80
Val Asn Arg Asp Glu Tyr Leu Leu Gly Arg Pro Ile Asp Lys Tyr Val
                85                  90                  95
Phe Glu Lys Met Glu Glu Lys Glu Ala Gly Cys Ser Ser Glu Thr Gly
            100                 105                 110
Leu Leu Pro Gly Ser Ile Phe Ala Pro Ser Gly Ala Asn Ser Leu Leu
        115                 120                 125
Asp Met Ala Ser Lys Ile Arg Glu Asp Pro Leu Phe Ile Ile Arg Lys
    130                 135                 140
Lys Glu Glu Glu Lys Lys Arg Glu Val Leu Asn Asn Pro Val Lys Met
145                 150                 155                 160

Lys Lys Ile Lys Glu Leu Leu Gln Met Ser Leu Glu Lys Lys Glu Lys
165 170 175
Lys Lys Lys Lys Glu Lys Lys Lys Lys His Lys Lys His Lys His Arg
180 185 190
Ser Ser Ser Ser Asp Arg Ser Ser Ser Glu Asp Glu His Ser Ala Gly
195 200 205
Arg Ser Gln Lys Lys Met Ala Asn Ser Ser Pro Val Leu Ser Lys Val
210 215 220
Pro Gly Tyr Gly Leu Gln Val Arg Asn Ser Asp Arg Asn Gln Gly Leu
225 230 235 240
Gln Gly Pro Leu Thr Ala Glu Gln Lys Arg Gly His Gly Met Lys Asn
245 250 255
His Ser Arg Ser Arg Ser Ser Ser His Ser Pro Pro Arg His Ala Ser
260 265 270
Lys Lys Ser Thr Arg Glu Ala Gly Ser Arg Asp Arg Arg Ser Arg Ser
275 280 285
Leu Gly Arg Arg Ser Arg Ser Pro Arg Pro Ser Lys Leu His Asn Ser
290 295 300
Lys Val Asn Arg Arg Glu Thr Gly Gln Thr Arg Ser Pro Ser Pro Lys
305 310 315 320
Lys Glu Val Tyr Gln Arg Arg His Ala Pro Gly Tyr Thr Arg Lys Leu
325 330 335
Ser Ala Glu Glu Leu Glu Arg Lys Arg Gln Glu Met Met Glu Asn Ala
340 345 350
Lys Trp Arg Glu Glu Glu Arg Leu Asn Ile Leu Lys Arg His Ala Lys
355 360 365
Asp Glu Glu Arg Glu Gln Arg Leu Glu Lys Leu Asp Ser Arg Asp Gly
370 375 380
Lys Phe Ile His Arg Met Lys Leu Glu Ser Ala Ser Thr Ser Ser Leu
385 390 395 400
Glu Asp Arg Val Lys Arg Asn Ile Tyr Ser Leu Gln Arg Thr Ser Val
405 410 415
Ala Leu Glu Lys Asn Phe Met Lys Arg
420 425

<210> 96

<211> 394

<212> PRT

<213> Homo sapiens

<400> 96
Met Phe Ser Val Phe Glu Glu Ile Thr Arg Ile Val Val Lys Glu Met
1 5 10 15
Asp Ala Gly Gly Asp Met Ile Ala Val Arg Ser Leu Val Asp Ala Asp
20 25 30

Arg Phe Arg Cys Phe His Leu Val Gly Glu Lys Arg Thr Phe Phe Gly
35 40 45
Cys Arg His Tyr Thr Thr Gly Leu Thr Leu Met Asp Ile Leu Asp Thr
50 55 60
His Gly Asp Lys Trp Leu Asp Glu Leu Asp Ser Gly Leu Gln Gly Gln
65 70 75 80
Lys Ala Glu Phe Gln Ile Leu Asp Asn Val Asp Ser Thr Gly Glu Leu
85 90 95

Ile Val Arg Leu Pro Lys Glu Ile Thr Ile Ser Gly Ser Phe Gln Gly
100 105 110
Phe His His Gln Lys Ile Lys Ile Ser Glu Asn Arg Ile Ser Gln Gln
115 120 125
Tyr Leu Ala Thr Leu Glu Asn Arg Lys Leu Lys Arg Glu Leu Pro Phe
130 135 140
Ser Phe Arg Ser Ile Asn Thr Arg Glu Asn Leu Tyr Leu Val Thr Glu
145 150 155 160
Thr Leu Glu Thr Val Lys Glu Glu Thr Leu Lys Ser Asp Arg Gln Tyr
165 170 175
Lys Phe Trp Ser Gln Ile Ser Gln Gly His Leu Ser Tyr Lys His Lys
180 185 190
Gly Gln Arg Glu Val Thr Ile Pro Pro Asn Arg Val Leu Ser Tyr Arg
195 200 205
Val Lys Gln Leu Val Phe Pro Asn Lys Glu Thr Met Arg Lys Ser Leu
210 215 220
Gly Ser Glu Asp Ser Arg Asn Met Lys Glu Lys Leu Glu Asp Met Glu
225 230 235 240
Ser Val Leu Lys Asp Leu Thr Glu Glu Lys Arg Lys Asp Val Leu Asn
245 250 255
Ser Leu Ala Lys Cys Leu Gly Lys Glu Asp Ile Arg Gln Asp Leu Glu
260 265 270
Gln Arg Val Ser Glu Val Leu Ile Ser Gly Glu Leu His Met Glu Asp
275 280 285
Pro Asp Lys Pro Leu Leu Ser Ser Leu Phe Asn Ala Ala Gly Val Leu
290 295 300
Val Glu Ala Arg Ala Lys Ala Ile Leu Asp Phe Leu Asp Ala Leu Leu
305 310 315 320
Glu Leu Ser Glu Glu Gln Gln Phe Val Ala Glu Ala Leu Glu Lys Gly
325 330 335
Thr Leu Pro Leu Leu Lys Asp Gln Val Lys Ser Val Met Glu Gln Asn
340 345 350
Trp Asp Glu Leu Ala Ser Ser Pro Pro Asp Met Asp Tyr Asp Pro Glu
355 360 365
Ala Arg Ile Leu Cys Ala Leu Tyr Val Val Val Ser Ile Leu Leu Glu
370 375 380
Leu Ala Glu Gly Pro Thr Ser Val Ser Ser
385 390

<210> 97

<211> 456

<212> PRT

<213> Homo sapiens

<400> 97
Met Glu Gly Pro Glu Gly Leu Gly Arg Lys Gln Ala Cys Leu Ala Met
1 5 10 15
Leu Leu His Phe Leu Asp Thr Tyr Gln Gly Leu Leu Gln Glu Glu Glu
20 25 30
Gly Ala Gly His Ile Ile Lys Asp Leu Tyr Leu Leu Ile Met Lys Asp
35 40 45
Glu Ser Leu Tyr Gln Gly Leu Arg Glu Asp Thr Leu Arg Leu His Gln
50 55 60

Leu Val Glu Thr Val Glu Leu Lys Ile Pro Glu Glu Asn Gln Pro Pro
65 70 75 80
Ser Lys Gln Val Lys Pro Leu Phe Arg His Phe Arg Arg Ile Asp Ser
85 90 95
Cys Leu Gln Thr Arg Val Ala Phe Arg Gly Ser Asp Glu Ile Phe Cys
100 105 110
Arg Val Tyr Met Pro Asp His Ser Tyr Val Thr Ile Arg Ser Arg Leu
115 120 125
Ser Ala Ser Val Gln Asp Ile Leu Gly Ser Val Thr Glu Lys Leu Gln
130 135 140
Tyr Ser Glu Glu Pro Ala Gly Arg Glu Asp Ser Leu Ile Leu Val Ala
145 150 155 160
Val Ser Ser Ser Gly Glu Lys Val Leu Leu Gln Pro Thr Glu Asp Cys
165 170 175
Val Phe Thr Ala Leu Gly Ile Asn Ser His Leu Phe Ala Cys Thr Arg
180 185 190
Asp Ser Tyr Glu Ala Leu Val Pro Leu Pro Glu Glu Ile Gln Val Ser
195 200 205
Pro Gly Asp Thr Glu Ile His Arg Val Glu Pro Glu Asp Val Ala Asn
210 215 220
His Leu Thr Ala Phe His Trp Glu Leu Phe Arg Cys Val His Glu Leu
225 230 235 240
Glu Phe Val Asp Tyr Val Phe His Gly Glu Arg Gly Arg Arg Glu Thr
245 250 255
Ala Asn Leu Glu Leu Leu Leu Gln Arg Cys Ser Glu Val Thr His Trp
260 265 270
Val Ala Thr Glu Val Leu Leu Cys Glu Ala Pro Gly Lys Arg Ala Gln
275 280 285
Leu Leu Lys Lys Phe Ile Lys Ile Ala Ala Leu Cys Lys Gln Asn Gln
290 295 300
Asp Leu Leu Ser Phe Tyr Ala Val Val Met Gly Leu Asp Asn Ala Ala
305 310 315 320
Val Ser Arg Leu Arg Leu Thr Trp Glu Lys Leu Pro Gly Lys Phe Lys
325 330 335
Asn Leu Phe Arg Lys Phe Glu Asn Leu Thr Asp Pro Cys Arg Asn His
340 345 350
Lys Ser Tyr Arg Glu Val Ile Ser Lys Met Lys Pro Pro Val Ile Pro
355 360 365
Phe Val Pro Leu Ile Leu Lys Asp Leu Thr Phe Leu His Glu Gly Ser
370 375 380
Lys Thr Leu Val Asp Gly Leu Val Asn Ile Glu Lys Leu His Ser Val
385 390 395 400
Ala Glu Lys Val Arg Thr Ile Arg Lys Tyr Arg Ser Arg Pro Leu Cys
405 410 415
Leu Asp Met Glu Ala Ser Pro Asn His Leu Gln Thr Lys Ala Tyr Val
420 425 430
Arg Gln Phe Gln Val Ile Asp Asn Gln Asn Leu Leu Phe Glu Leu Ser
435 440 445
Tyr Lys Leu Glu Ala Asn Ser Gln
450 455

<210> 98

<211> 715

<212> PRT

<213> Homo sapiens

<400> 98

Met Ser Gln Val Met Ser Ser Pro Leu Leu Ala Gly Gly His Ala Val
1 5 10 15
Ser Leu Ala Pro Cys Asp Glu Pro Arg Arg Thr Leu His Pro Ala Pro
20 25 30
Ser Pro Ser Leu Pro Pro Gln Cys Ser Tyr Tyr Thr Thr Glu Gly Trp
35 40 45
Gly Ala Gln Ala Leu Met Ala Pro Val Pro Cys Met Gly Pro Pro Gly
50 55 60
Arg Leu Gln Gln Ala Pro Gln Val Glu Ala Lys Ala Thr Cys Phe Leu
65 70 75 80
Pro Ser Pro Gly Glu Lys Ala Leu Gly Thr Pro Glu Asp Leu Asp Ser
85 90 95
Tyr Ile Asp Phe Ser Leu Glu Ser Leu Asn Gln Met Ile Leu Glu Leu
100 105 110
Asp Pro Thr Phe Gln Leu Leu Pro Pro Gly Thr Gly Gly Ser Gln Ala
115 120 125
Glu Leu Ala Gln Ser Thr Met Ser Met Arg Lys Lys Glu Glu Ser Glu
130 135 140
Ala Leu Asp Ile Lys Tyr Ile Glu Val Thr Ser Ala Arg Ser Arg Cys
145 150 155 160
His Asp Trp Pro Gln His Cys Ser Ser Pro Ser Val Thr Pro Pro Phe
165 170 175
Gly Ser Pro Arg Ser Gly Gly Leu Leu Leu Ser Arg Asp Val Pro Arg
180 185 190
Glu Thr Arg Ser Ser Ser Glu Ser Leu Ile Phe Ser Gly Asn Gln Gly
195 200 205
Arg Gly His Gln Arg Pro Leu Pro Pro Ser Glu Gly Leu Ser Pro Arg
210 215 220
Pro Pro Asn Ser Pro Ser Ile Ser Ile Pro Cys Met Gly Ser Lys Ala
225 230 235 240
Ser Ser Pro His Gly Leu Gly Ser Pro Leu Val Ala Ser Pro Arg Leu
245 250 255
Glu Lys Arg Leu Gly Gly Leu Ala Pro Gln Arg Gly Ser Arg Ile Ser
260 265 270
Val Leu Ser Ala Ser Pro Val Ser Asp Val Ser Tyr Met Phe Gly Ser
275 280 285
Ser Gln Ser Leu Leu His Ser Ser Asn Ser Ser His Gln Ser Ser Ser
290 295 300
Arg Ser Leu Glu Ser Pro Ala Asn Ser Ser Ser Ser Leu His Ser Leu
305 310 315 320
Gly Ser Val Ser Leu Cys Thr Arg Pro Ser Asp Phe Gln Ala Pro Arg
325 330 335
Asn Pro Thr Leu Thr Met Gly Gln Pro Arg Thr Pro His Ser Pro Pro
340 345 350
Leu Ala Lys Glu His Ala Ser Ile Cys Pro Pro Ser Ile Thr Asn Ser
355 360 365
Met Val Asp Ile Pro Ile Val Leu Ile Asn Gly Cys Pro Glu Pro Gly
370 375 380
Ser Ser Pro Pro Gln Arg Thr Pro Gly His Gln Asn Ser Val Gln Pro
385 390 395 400
Gly Ala Ala Ser Pro Ser Asn Pro Cys Pro Ala Thr Arg Ser Asn Ser
405 410 415
Gln Thr Leu Ser Asp Ala Pro Phe Thr Thr Cys Pro Glu Gly Pro Ala
420 425 430
Arg Asp Met Gln Pro Thr Met Lys Phe Val Met Asp Thr Ser Lys Tyr
435 440 445

Trp Phe Lys Pro Asn Ile Thr Arg Glu Gln Ala Ile Glu Leu Leu Arg
450 455 460
Lys Glu Glu Pro Gly Ala Phe Val Ile Arg Asp Ser Ser Ser Tyr Arg
465 470 475 480
Gly Ser Phe Gly Leu Ala Leu Lys Val Gln Glu Val Pro Ala Ser Ala
485 490 495
Gln Asn Arg Pro Gly Glu Asp Ser Asn Asp Leu Ile Arg His Phe Leu
500 505 510
Ile Glu Ser Ser Ala Lys Gly Val His Leu Lys Gly Ala Asp Glu Glu
515 520 525
Pro Tyr Phe Gly Ser Leu Ser Ala Phe Val Cys Gln His Ser Ile Met
530 535 540
Ala Leu Ala Leu Pro Cys Lys Leu Thr Ile Pro Gln Arg Glu Leu Gly
545 550 555 560
Gly Ala Asp Gly Ala Ser Asp Ser Thr Asp Ser Pro Ala Ser Cys Gln
565 570 575
Lys Lys Ser Ala Gly Cys His Thr Leu Tyr Leu Ser Ser Val Ser Val
580 585 590
Glu Thr Leu Thr Gly Ala Leu Ala Val Gln Lys Ala Ile Ser Thr Thr
595 600 605
Phe Glu Arg Asp Ile Leu Pro Thr Pro Thr Val Val His Phe Glu Val
610 615 620
Thr Glu Gln Gly Ile Thr Leu Thr Asp Val Gln Arg Lys Val Phe Phe
625 630 635 640
Arg Arg His Tyr Pro Leu Thr Thr Leu Arg Phe Cys Gly Met Asp Pro
645 650 655
Glu Gln Arg Lys Trp Gln Lys Tyr Cys Lys Pro Ser Trp Ile Phe Gly
660 665 670
Phe Val Ala Lys Ser Gln Thr Glu Pro Gln Glu Asn Val Cys His Leu
675 680 685
Phe Ala Glu Tyr Asp Met Val Gln Pro Ala Ser Gln Val Ile Gly Leu
690 695 700
Val Thr Ala Leu Leu Gln Asp Ala Glu Arg Met
705 710 715

<210> 99

<211> 35

<212> DNA

<213> Artificial sequence

<220>

<223> PCR primer
<400> 99
ccatatataa aaccactgtc ctgtcctttg tggct 35

<210> 100

<211> 26

<212> DNA

<213> Artificial sequence

<220>

<223> PCR primer
<400> 100
cccccatctg tctgtctata tttgtc 26

<210> 101

<211> 22

<212> DNA

<213> Artificial sequence

<220>

<223> PCR primer
<400> 101
tgcctacgct gacgactatg tg 22

<210> 102

<211> 25

<212> DNA

<213> Artificial sequence

<220>

<223> PCR primer
<400> 102
tttggttttc tacaactgtt gctat 25

<210> 103

<211> 19

<212> DNA

<213> Artificial sequence

<220>

<223> PCR primer
<400> 103
gggctccaca caccagatg 19

<210> 104

<211> 21

<212> DNA

<213> Artificial sequence

<220>

<223> PCR primer
<400> 104
acgctctgag caccctctac a 21

<210> 105

<211> 31

<212> DNA

<213> Artificial sequence

<220>

<223> PCR primer
<400> 105
tgtcacaggg actgaaaacc tctcctcatg t 31

<210> 106

<211> 17

<212> DNA

<213> Artificial sequence

<220>

<223> PCR primer
<400> 106
cccaaggcca cgagctt 17

<210> 107

<211> 24

<212> DNA

<213> Artificial sequence

<220>

<223> PCR primer
<400> 107
tgttgctctc ttaacgaatc gaaa 24

<210> 108

<211> 29

<212> DNA

<213> Artificial sequence

<220>

<223> PCR primer
<400> 108
ctggtcaaac aaactctctg aacccctcc 29

<210> 109

<211> 20

<212> DNA

<213> Artificial sequence

<220>

<223> PCR primer
<400> 109
tggtgaggaa aagcggacat 20

<210> 110

<211> 21

<212> DNA

<213> Artificial sequence

<220>

<223> PCR primer
<400> 110
ctggcttgga ggacagtgaa g 21

<210> 111

<211> 24

<212> DNA

<213> Artificial sequence

<220>

<223> PCR primer
<400> 111
ccaagccctc cccatcccat gtat 24

<210> 112

<211> 21

<212> DNA

<213> Artificial sequence

<220>

<223> PCR primer
<400> 112
gaggtgtcgt accgcgttct a 21

<210> 113

<211> 21

<212> DNA

<213> Artificial sequence

<220>

<223> PCR primer
<400> 113
ccgttctgct cttccctgtc t 21

<210> 114

<211> 23

<212> DNA

<213> Artificial sequence

<220>

<223> PCR primer
<400> 114
ccagacccgc ttcactgacc tgc 23

<210> 115

<211> 20

<212> DNA

<213> Artificial sequence

<220>

<223> PCR primer
<400> 115
cgcctgtact tcagcatgga 20

<210> 116

<211> 18

<212> DNA

<213> Artificial sequence

<220>

<223> PCR primer
<400> 116
gcggttcagc tggtggaa 18

<210> 117

<211> 25

<212> DNA

<213> Artificial sequence

<220>

<223> PCR primer
<400> 117
accccgaggc atcaccacaa atcat 25

<210> 118

<211> 23

<212> DNA

<213> Artificial sequence

<220>

<223> PCR primer
<400> 118
agttctgcct ctctgacaac cat 23

<210> 119

<211> 23

<212> DNA

<213> Artificial sequence

<220>

<223> PCR primer
<400> 119
taggctcaga gtcagaccca aac 23

<210> 120

<211> 21

<212> DNA

<213> Artificial sequence

<220>

<223> PCR primer
<400> 120
ccctcgtggg cttgtgctcg g 21

<210> 121

<211> 21

<212> DNA

<213> Artificial sequence

<220>

<223> PCR primer
<400> 121
aagccgccag ttcatctttt t 21

<210> 122

<211> 25

<212> DNA

<213> Artificial sequence

<220>

<223> PCR primer
<400> 122
cttgtggttc aagtcaaatg ttcag 25

<210> 123

<211> 21

<212> DNA

<213> Artificial sequence

<220>

<223> PCR primer
<400> 123
tctgcctgcg ctctcgtcgg t 21

<210> 124

<211> 18

<212> DNA

<213> Artificial sequence

<220>

<223> PCR primer
<400> 124
gggctgggca cctgactt 18

<210> 125

<211> 20

<212> DNA

<213> Artificial sequence

<220>

<223> PCR primer
<400> 125
cccaacaagg gtcccagact 20

<210> 126

<211> 17

<212> DNA

<213> Artificial sequence

<220>

<223> PCR primer
<400> 126
cggcgcattg agcggcg 17

<210> 127

<211> 20

<212> DNA

<213> Artificial sequence

<220>

<223> PCR primer
<400> 127
cccaagggac ttcgtgaatg 20

<210> 128

<211> 21

<212> DNA

<213> Artificial sequence

<220>

<223> PCR primer
<400> 128
ggcgatccct gatgacaagt a 21

<210> 129

<211> 29

<212> DNA

<213> Artificial sequence

<220>

<223> PCR primer#
<400> 129
agcaccaact gtgaaccagg tacaatggc 29

<210> 130

<211> 19

<212> DNA

<213> Artificial sequence

<220>

<223> PCR primer
<400> 130
gagggaggct ctgctttgg 19

<210> 131

<211> 21

<212> DNA

<213> Artificial sequence

<220>

<223> PCR primer
<400> 131
tcacaactag cgggtgagga g 21

<210> 132

<211> 21

<212> DNA

<213> Artificial sequence

<220>

<223> PCR primer
<400> 132
tgcagaggaa cggcgtgagc g 21

<210> 133

<211> 22

<212> DNA

<213> Artificial sequence

<220>

<223> PCR primer
<400> 133
tgaggtttcc tcccaaatcg ta 22

<210> 134

<211> 22

<212> DNA

<213> Artificial sequence

<220>

<223> PCR primer
<400> 134
cagctcaagg gaagctgtca tc 22

<210> 135

<211> 24

<212> DNA

<213> Artificial sequence

<220>

<223> PCR primer
<400> 135
cccccacatg ttccccaaga tgct 24

<210> 136

<211> 21

<212> DNA

<213> Artificial sequence

<220>

<223> PCR primer
<400> 136
ggaggcgcta aaggtctacg t 21

<210> 137

<211> 21

<212> DNA

<213> Artificial sequence

<220>

<223> PCR primer
<400> 137
tgatgcttcg caggtcagta a 21

<210> 138

<211> 26

<212> DNA

<213> Artificial sequence

<220>

<223> PCR primer
<400> 138
ctcctgcccc tcctaaagct gaagcc 26

<210> 139

<211> 17

<212> DNA

<213> Artificial sequence

<220>

<223> PCR primer
<400> 139
ggacgcgtgg gcttttc 17

<210> 140

<211> 20

<212> DNA

<213> Artificial sequence

<220>

<223> PCR primer
<400> 140
tgtggctgtg gacacctttc 20

<210> 141

<211> 25

<212> DNA

<213> Artificial sequence

<220>

<223> PCR primer
<400> 141
ccacaagctg aaggcagaca aggcc 25

<210> 142

<211> 20

<212> DNA

<213> Artificial sequence

<220>

<223> PCR primer
<400> 142
gcggattctc atggaacaca 20

<210> 143

<211> 20

<212> DNA

<213> Artificial sequence

<220>

<223> PCR primer
<400> 143
ggtcagccag gagcttcttg 20

<210> 144

<211> 23

<212> DNA

<213> Artificial sequence

<220>

<223> PCR primer
<400> 144
accaccttgc gcaggttgtc cag 23

<210> 145

<211> 18

<212> DNA

<213> Artificial sequence

<220>

<223> PCR primer
<400> 145
cgcatgcacg acctgaac 18

<210> 146

<211> 23

<212> DNA

<213> Artificial sequence

<220>

<223> PCR primer
<400> 146
gtctcgatct tggacagctt ctg 23

<210> 147

<211> 22

<212> DNA

<213> Artificial sequence

<220>

<223> PCR primer
<400> 147
acactgtcca cacggcccga gg 22

<210> 148

<211> 21

<212> DNA

<213> Artificial sequence

<220>

<223> PCR primer
<400> 148
ctgggcagaa tggaaggatc t 21

<210> 149

<211> 22

<212> DNA

<213> Artificial sequence

<220>

<223> PCR primer
<400> 149
gggactctag cagacccaca ct 22

<210> 150

<211> 22

<212> DNA

<213> Artificial sequence

<220>

<223> PCR primer
<400> 150
cacccacctg gattccctgt tc 22

<210> 151

<211> 23

<212> DNA

<213> Artificial sequence

<220>

<223> PCR primer
<400> 151
cctttcagaca ggcgtagatg atg 23

<210> 152

<211> 29

<212> DNA

<213> Artificial sequence

<220>

<223> PCR primer
<400> 152
gggtattatt tctttattag gtgccactt 29

<210> 153

<211> 30

<212> DNA

<213> Artificial sequence

<220>

<223> PCR primer
<400> 153
ttccctaagg ctttcagtac ccaggatctg 30

<210> 154

<211> 18

<212> DNA

<213> Artificial sequence

<220>

<223> PCR primer
<400> 154
ccagcttggc cctttcct 18

<210> 155

<211> 23

<212> DNA

<213> Artificial sequence

<220>

<223> PCR primer
<400> 155
gaatgggtcg cttttgttct tag 23

<210> 156

<211> 22

<212> DNA

<213> Artificial sequence

<220>

<223> PCR primer
<400> 156
tcacggacct cagcctgccc ct 22

<210> 157

<211> 21

<212> DNA

<213> Artificial sequence

<220>

<223> PCR primer
<400> 157
tggtgaaggt gtcagccatg t 21

<210> 158

<211> 21

<212> DNA

<213> Artificial sequence

<220>

<223> PCR primer
<400> 158
tcagagtgca gcaatggctt t 21

<210> 159

<211> 20

<212> DNA

<213> Artificial sequence

<220>

<223> PCR primer
<400> 159
acctccttcc ccagctcccc 20

<210> 160

<211> 24

<212> DNA

<213> Artificial sequence

<220>

<223> PCR primer
<400> 160
ggcaacatct tacttgtcct ttga 24

<210> 161

<211> 25

<212> DNA

<213> Artificial sequence

<220>

<223> PCR primer
<400> 161
ccaaggaagc acagacaact atttc 25

<210> 162

<211> 30

<212> DNA

<213> Artificial sequence

<220>

<223> PCR primer
<400> 162
tcctccctat ccatggcact aaaccacttc 30

<210> 163

<211> 19

<212> DNA

<213> Artificial sequence

<220>

<223> PCR primer
<400> 163
tgggcaaggg ctcctatct 19

<210> 164

<211> 21

<212> DNA

<213> Artificial sequence

<220>

<223> PCR primer
<400> 164
gttacccctg gcagacgtat g 21

<210> 165

<211> 31

<212> DNA

<213> Artificial sequence

<220>

<223> PCR primer
<400> 165
tgcctctgag tctgaatctc ccaaagagag a 31

<210> 166

<211> 31

<212> DNA

<213> Artificial sequence

<220>

<223> PCR primer
<400> 166
gagtagttat gtgattattt cagctcttga c 31

<210> 167

<211> 21

<212> DNA

<213> Artificial sequence

<220>

<223> PCR primer
<400> 167
tcaaatgttg tccccgagtc t 21

<210> 168

<211> 34

<212> DNA

<213> Artificial sequence

<220>

<223> PCR primer
<400> 168
cagaaattcg gaagacagaa ctattgtcat gcct 34

<210> 169

<211> 27

<212> DNA

<213> Artificial sequence

<220>

<223> PCR primer
<400> 169
gattagtaac ccatagcagt tgaaggt 27

<210> 170

<211> 26

<212> DNA

<213> Artificial sequence

<220>

<223> PCR primer
<400> 170
atttactgac ggtggtctga acatac 26

<210> 171

<211> 31

<212> DNA

<213> Artificial sequence

<220>

<223> PCR primer
<400> 171
tgacagactc caaatcacaa gcacagtcaa c 31

<210> 172

<211> 25

<212> DNA

<213> Artificial sequence

<220>

<223> PCR primer
<400> 172
tgatggtttg gaggaaagtt tattt 25

<210> 173

<211> 24

<212> DNA

<213> Artificial sequence

<220>

<223> PCR primer
<400> 173
tttggttggg tctttagagg aatc 24

<210> 174

<211> 24

<212> DNA

<213> Artificial sequence

<220>

<223> PCR primer
<400> 174
tgccaaccat gcatcaggta gccc 24

<210> 175

<211> 20

<212> DNA

<213> Artificial sequence

<220>

<223> PCR primer
<400> 175
cagctcacct ggcaacttca 20

<210> 176

<211> 20

<212> DNA

<213> Artificial sequence

<220>

<223> PCR primer
<400> 176
cctgattttc ccagcgatgt 20

<210> 177

<211> 19

<212> DNA

<213> Artificial sequence

<220>

<223> PCR primer
<400> 177
cgccgctccc ggttctgct 19

<210> 178

<211> 20

<212> DNA

<213> Artificial sequence

<220>

<223> PCR primer
<400> 178
tggccaagcg taagctgatt 20

<210> 179

<211> 21

<212> DNA

<213> Artificial sequence

<220>

<223> PCR primer
<400> 179
gctgcagtga tcggatcatc t 21

<210> 180

<211> 22

<212> DNA

<213> Artificial Sequence

<220>

<223> MLLT6
<400> 180
caccatggag cccatcgtgc tg 22

<210> 181

<211> 19

<212> DNA

<213> Artificial Sequence

<220>

<223> MLLT6 for
<400> 181
atccccgagg tgcaatttg 19

<210> 182

<211> 21

<212> DNA

<213> Artificial Sequence

<220>

<223> MLLT6 rev
<400> 182
agcgatcatg aggcacgtac t 21

<210> 183

<211> 29

<212> DNA

<213> Artificial Sequence

<220>

<223> ZNF144
<400> 183
cctgccagag ataggagacc cagacagct 29

<210> 184

<211> 19

<212> DNA

<213> Artificial Sequence

<220>

<223> ZNF144 for
<400> 184
atccccctga gccttttca 19

<210> 185

<211> 19

<212> DNA

<213> Artificial Sequence

<220>

<223> ZNF144 rev
<400> 185
cagcctctgg tcccaccat 19

<210> 186

<211> 28

<212> DNA

<213> Artificial Sequence

<220>

<223> PIP5K2B
<400> 186
tgatcatcaa ttccaaacct ctccccgaa 28

<210> 187

<211> 19

<212> DNA

<213> Artificial Sequence

<220>

<223> PIP5K2B for
<400> 187
ccccatggtg ttccgaaac 19

<210> 188

<211> 19

<212> DNA

<213> Artificial Sequence

<220>

<223> PIP5K2B rev
<400> 188
tgccaggagc ctccatacc 19

<210> 189

<211> 29

<212> DNA

<213> Artificial Sequence

<220>

<223> TEM7
<400> 189
cagccttcta aaacacaatg tattcatgt 29

<210> 190

<211> 29

<212> DNA

<213> Artificial Sequence

<220>

<223> TEM7 for
<400> 190
cctgaactta atggtagaat tcaaagatc 29

<210> 191

<211> 27

<212> DNA

<213> Artificial Sequence

<220>

<223> TEM7 rev
<400> 191
tattaacact gagaatccat gcagaga 27

<210> 192

<211> 35

<212> DNA

<213> Artificial Sequence

<220>

<223> ZNFN1A3
<400> 192
tatctggtct cagggattgc tcctatgtat tcagc 35

<210> 193

<211> 20

<212> DNA

<213> Artificial Sequence

<220>

<223> ZNFN1A3 for
<400> 193
cacagagccc tgctgaagtg 20

<210> 194

<211> 23

<212> DNA

<213> Artificial Sequence

<220>

<223> ZNFN1A3 rev
<400> 194
gcgaggtcat tggtttttag aaa 23

<210> 195

<211> 22

<212> DNA

<213> Artificial Sequence

<220>

<223> WIRE
<400> 195
ctgtgatccg aaatggtgcc ag 22

<210> 196

<211> 20

<212> DNA

<213> Artificial Sequence

<220>

<223> WIRE for
<400> 196
ccgtctccac atccaaacct 20

<210> 197

<211> 20

<212> DNA

<213> Artificial Sequence

<220>

<223> WIRE rev
<400> 197
acccatgcat tcggtatggt 20

<210> 198

<211> 21

<212> DNA

<213> Artificial Sequence

<220>

<223> PSMB3
<400> 198
agtggcacct gcgccgaaca a 21

<210> 199

<211> 21

<212> DNA

<213> Artificial Sequence

<220>

<223> PSMB3 for
<400> 199
ccccatggtg actgatgact t 21

<210> 200

<211> 21

<212> DNA

<213> Artificial Sequence

<220>

<223> PSMB3 rev
<400> 200
ccagagggac tcacacattc c 21

<210> 201

<211> 29

<212> DNA

<213> Artificial Sequence

<220>

<223> MGC9753
<400> 201
ccagaaactt tccatcccaa aggcagtct 29

<210> 202

<211> 21

<212> DNA

<213> Artificial Sequence

<220>

<223> MGC9753 for
<400> 202
ctgccccaca ggaatagaat g 21

<210> 203

<211> 23

<212> DNA

<213> ARTIFICIAL SEQUENCE

<220>

<223> MGC9753 rev
<400> 203
aaaaatccag tctgcttcaa cca 23

<210> 204

<211> 20

<212> DNA

<213> ARTIFICIAL SEQUENCE

<220>

<223> ORMDL3
<400> 204
agctgcccca gctccacgga 20

<210> 205

<211> 21

<212> DNA

<213> ARTIFICIAL SEQUENCE

<220>

<223> ORMDL3 for
<400> 205
tccctgatga gcgtgcttat c 21

<210> 206

<211> 28

<212> DNA

<213> ARTIFICIAL SEQUENCE

<220>

<223> ORMDL3 rev
<400> 206
tctcagtact tattgattcc aaaaatcc 28

<210> 207

<211> 25

<212> DNA

<213> ARTIFICIAL SEQUENCE

<220>

<223> MGC15482
<400> 207
tccagtggaa gcaaccccag tgttc 25

<210> 208

<211> 25

<212> DNA

<213> ARTIFICIAL SEQUENCE

<220>

<223> MGC15482 for
<400> 208
cacttctaga gctaccgtgg agtct 25

<210> 209

<211> 22

<212> DNA

<213> ARTIFICIAL SEQUENCE

<220>

<223> MGC15482 rev
<400> 209
ccctcacttt gtaacccttg ct 22

<210> 210

<211> 20

<212> DNA

<213> ARTIFICIAL SEQUENCE

<220>

<223> PPP1R1B
<400> 210
cagcgtggcg caacaaccca 20

<210> 211

<211> 21

<212> DNA

<213> ARTIFICIAL SEQUENCE

<220>

<223> PPP1R1B for
<400> 211
gggattgttt cgccacacat a 21

<210> 212

<211> 20

<212> DNA

<213> ARTIFICIAL SEQUENCE

<220>

<223> PPP1R1B rev
<400> 212
ccgatgttaa ggcccatagc 20

<210> 213

<211> 27

<212> DNA

<213> ARTIFICIAL SEQUENCE

<220>

<223> MGC14832
<400> 213
taaaatgtcc ggccaacatg agttccc 27

<210> 214

<211> 17

<212> DNA

<213> ARTIFICIAL SEQUENCE

<220>

<223> MGC14832 for
<400> 214
cgcagtgcct ggcacat 17

<210> 215

<211> 20

<212> DNA

<213> ARTIFICIAL SEQUENCE

<220>

<223> MGC14832 rev
<400> 215
gacacccccct gacctatgga 20

<210> 216

<211> 25

<212> DNA

<213> ARTIFICIAL SEQUENCE

<220>

<223> LOC51242
<400> 216
cagtgacctc tcccgttccc ttgga 25

<210> 217

<211> 20

<212> DNA

<213> ARTIFICIAL SEQUENCE

<220>

<223> LOC51242 for
<400> 217
tgggtccctg tgtcctcttc 20

<210> 218

<211> 20

<212> DNA

<213> ARTIFICIAL SEQUENCE

<220>

<223> LOC51242 for
<400> 218
agggtcagga gggagaaaac 20

<210> 219

<211> 26

<212> DNA

<213> ARTIFICIAL SEQUENCE

<220>

<223> FLJ20291
<400> 219
ccagtgccca cccgttaaag agtcaa 26

<210> 220

<211> 24

<212> DNA

<213> ARTIFICIAL SEQUENCE

<220>

<223> FLJ20291 for
<400> 220
ttgtgggaca ctcagtaact ttgg 24

<210> 221

<211> 20

<212> DNA

<213> ARTIFICIAL SEQUENCE

<220>

<223> FLJ20291 rev
<400> 221
acaagcactc ccaccgagat 20

<210> 222

<211> 24

<212> DNA

<213> ARTIFICIAL SEQUENCE

<220>

<223> PRO2521
<400> 222
agtctgtcct cactgccatc gcca 24

<210> 223

<211> 21

<212> DNA

<213> ARTIFICIAL SEQUENCE

<220>

<223> PRO2521 for
<400> 223
aagcctctgg gttttccctt t 21

<210> 224

<211> 20

<212> DNA

<213> ARTIFICIAL SEQUENCE

<220>

<223> PRO2521 rev
<400> 224
cccactggtg acaggatggt 20

<210> 225

<211> 23

<212> DNA

<213> ARTIFICIAL SEQUENCE

<220>

<223> LINK-GEFII
<400> 225
catctgacat ctttcccgtg gag 23

<210> 226

<211> 21

<212> DNA

<213> ARTIFICIAL SEQUENCE

<220>

<223> LINK-GEFII for
<400> 226
ctttgcacga tgtctcaacc a 21

<210> 227

<211> 18

<212> DNA

<213> ARTIFICIAL SEQUENCE

<220>

<223> LINK-GEFII rev
<400> 227
tttcccgtgg agcaggaa 18

<210> 228

<211> 26

<212> DNA

<213> ARTIFICIAL SEQUENCE

<220>

<223> CTEN
<400> 228
ccgccgccta atatgcaaca ttaggg 26

<210> 229

<211> 23

<212> DNA

<213> ARTIFICIAL SEQUENCE

<220>

<223> CTEN for
<400> 229
cgagtattcc aaagctggta tcg 23

<210> 230

<211> 24

<212> DNA

<213> ARTIFICIAL SEQUENCE

<220>

<223> CTEN rev
<400> 230
atcacagaga gatggccctt atct 24

<210> 231

<211> 25

<212> DNA

<213> Artificial Sequence

<220>

<223> D17S946 forward primer
<400> 231
acagtctatc aagcagaaaa atcct 25

<210> 232

<211> 16

<212> DNA

<213> Artificial Sequence

<220>

<223> D17S946 reverse primer
<400> 232
tgccgtgcca gagaga 16

<210> 233

<211> 20

<212> DNA

<213> Artificial Sequence

<220>

<223> D17S1181 forward primer
<400> 233
gacaacagag cgagactccc 20

<210> 234

<211> 20

<212> DNA

<213> Artificial Sequence

<220>

<223> D17S1181 reverse primer
<400> 234
gcccagcctg tcacttattc 20

<210> 235

<211> 18

<212> DNA

<213> Artificial Sequence

<220>

<223> D17S2026 forward primer
<400> 235
tggtcattcg acaacgaa 18

<210> 236

<211> 18

<212> DNA

<213> Artificial Sequence

<220>

<223> D17S2026 reverse primer
<400> 236
cagcattgga tgcaatcc 18

<210> 237

<211> 20

<212> DNA

<213> Artificial Sequence

<220>

<223> D17S838 forward primer
<400> 237
ctccagaatc cagaccatga 20

<210> 238

<211> 20

<212> DNA

<213> Artificial Sequence

<220>

<223> D17S838 reverse primer
<400> 238
aggacagtgt gtagcccttc 20

<210> 239

<211> 20

<212> DNA

<213> Artificial Sequence

<220>

<223> D17S250 forward primer
<400> 239
ggaagaatca aatagacaat 20

<210> 240

<211> 24

<212> DNA

<213> Artificial Sequence

<220>

<223> D17S250 reverse primer
<400> 240
gctggccata tatatattta aacc 24

<210> 241

<211> 23

<212> DNA

<213> Artificial Sequence

<220>

<223> D17S1818 forward primer
<400> 241
cataggtatg ttcagaaatg tga 23

<210> 242

<211> 18

<212> DNA

<213> Artificial Sequence

<220>

<223> D17S1818 reverse primer
<400> 242
tgcctactgg aaaccaga 18

<210> 243

<211> 23

<212> DNA

<213> Artificial Sequence

<220>

<223> D17S614 forward primer
<400> 243
aaggggaagg ggctttcaaa gct 23

<210> 244

<211> 23

<212> DNA

<213> Artificial Sequence

<220>

<223> D17S614 reverse primer
<220>

<221> misc_feature

<222> (1)..(1)

<223> n=a, c, g or t

<400> 244
nggaggttgc agtgagccaa gat 23

<210> 245

<211> 23

<212> DNA

<213> Artificial Sequence

<220>

<223> D17S2019 forward primer
<400> 245
caaaagctta tgatgctcaa acc 23

<210> 246

<211> 22

<212> DNA

<213> Artificial Sequence

<220>

<223> D17S2019 reverse primer
<400> 246
ttgtttccct ttgactttct ga 22

<210> 247

<211> 25

<212> DNA

<213> Artificial Sequence

<220>

<223> D17S608 forward primer
<400> 247
taggttcacc tctcatttc ttcag 25

<210> 248

<211> 24

<212> DNA

<213> Artificial Sequence

<220>

<223> D17S608 reverse primer
<220>

<221> misc_feature

<222> (17)..(17)

<223> n=a, c, g or t

<400> 248
gtctgggtct ttatggngct tgtg 24

<211> 20

<212> DNA

<213> Artificial Sequence

<220>

<223> D17S1655 forward primer
<400> 249
cggaccagag tgttccatgg 20

<210> 250

<211> 20

<212> DNA

<213> Artificial Sequence

<220>

<223> D17S1655 reverse primer
<400> 250
gcatacagca ccctctacct 20

<210> 251

<211> 25

<212> DNA

<213> Artificial Sequence

<220>

<223> D17S2147 forward primer
<400> 251
aggggagaat aaataaaatc tgtgg 25

<210> 252

<211> 22

<212> DNA

<213> Artificial Sequence

<220>

<223> D17S2147 reverse primer
<400> 252
caggagtgag acactctcca tg 22

<210> 253

<211> 22

<212> DNA

<213> Artificial Sequence

<220>

<223> D17S754 forward primer
<400> 253
tggattcact gactcagcct gc 22

<210> 254

<211> 22

<212> DNA

<213> Artificial Sequence

<220>

<223> D17S754 reverse primer
<400> 254
gcgtgtctgt ctccatgtgt gc 22

<210> 255

<211> 18

<212> DNA

<213> Artificial Sequence

<220>

<223> D17S1814 forward primer
<400> 255
tccccaatga cggtgatg 18

<210> 256

<211> 20

<212> DNA

<213> Artificial Sequence

<220>

<223> D17S1814 reverse primer
<400> 256
ctggaggttg gcttgtggat 20

<210> 257

<211> 18

<212> DNA

<213> Artificial Sequence

<220>

<223> D17S2007 forward primer
<400> 257
ggtcccacga atttgctg 18

<210> 258

<211> 20

<212> DNA

<213> Artificial Sequence

<220>

<223> D17S2007 reverse primer
<400> 258
ccacccagaa aaacaggaga 20

<210> 259

<211> 20

<212> DNA

<213> Artificial Sequence

<220>

<223> D17S1246 forward primer
<400> 259
tcgatctcct gaccttgtga 20

<210> 260

<211> 20

<212> DNA

<213> Artificial Sequence

<220>

<223> D17S1246 reverse primer
<400> 260
ttgtcacccc attgcctttc 20

<210> 261

<211> 21

<212> DNA

<213> Artificial Sequence

<220>

<223> D17S1979 forward primer
<400> 261
ccttggatag attcagctcc c 21

<210> 262

<211> 21

<212> DNA

<213> Artificial Sequence

<220>

<223> D17S1979 reverse primer
<400> 262
cttgtccctt ctcaatcctc c 21

<210> 263

<211> 25

<212> DNA

<213> Artificial Sequence

<220>

<223> D17S1984 forward primer
<400> 263
ttaagcaagg ttttaattaa gctgc 25

<210> 264

<211> 21

<212> DNA

<213> Artificial Sequence

<220>

<223> D17S1984 reverse primer
<400> 264
gattacagtg ctccctctcc c 21

<210> 265

<211> 22

<212> DNA

<213> Artificial Sequence

<220>

<223> G11580 forward primer
<400> 265
ggttttaatt aagctgcatg gc 22

<210> 266

<211> 21

<212> DNA

<213> Artificial Sequence

<220>

<223> G11580 reverse primer
<400> 266
gattacagtg ctccctctcc c 21

<210> 267

<211> 20

<212> DNA

<213> Artificial Sequence

<220>

<223> D17S1867 forward primer
<400> 267
agtttgacac tgaggctttg 20

<210> 268

<211> 20

<212> DNA

<213> Artificial Sequence

<220>

<223> D17S1867 reverse primer
<400> 268
tttagacttg gtaactgccg 20

<210> 269

<211> 24

<212> DNA

<213> Artificial Sequence

<220>

<223> D17S1788 forward primer
<400> 269
tgcagatgcc taagaacttt tcag 24

<210> 270

<211> 19

<212> DNA

<213> Artificial Sequence

<220>

<223> D17S1788 reverse primer
<400> 270
gccatgatct cccaaagcc 19

<210> 271

<211> 18

<212> DNA

<213> Artificial Sequence

<220>

<223> D17S1836 forward primer
<400> 271
tcgaggttat ggtgagcc 18

<210> 272

<211> 24

<212> DNA

<213> Artificial Sequence

<220>

<223> D17S1836 reverse primer
<400> 272
aaactgtgtg tgtcaaagga tact 24

<210> 273

<211> 19

<212> DNA

<213> Artificial Sequence

<220>

<223> D17S1787 forward primer
<400> 273
gctgatctga agccaatga 19

<210> 274

<211> 19

<212> DNA

<213> Artificial Sequence

<220>

<223> D17S1787 reverse primer
<400> 274
tacatgaagg catggtctg 19

<210> 275

<211> 23

<212> DNA

<213> Artificial Sequence

<220>

<223> D17S1660 forward primer
<400> 275
ctaatataat cctgggcaca tgg 23

<210> 276

<211> 18

<212> DNA

<213> Artificial Sequence

<220>

<223> D17S1660 reverse primer
<400> 276
gctgcggacc agacagat 18

<210> 277

<211> 22

<212> DNA

<213> Artificial Sequence

<220>

<223> D17S2154 forward primer
<400> 277
gataaaaaca agcactggct cc 22

<210> 278

<211> 20

<212> DNA

<213> Artificial Sequence

<220>

<223> D17S2154 reverse primer
<400> 278
cccacggctt tcttgatcta 20

<210> 279

<211> 21

<212> DNA

<213> Artificial Sequence

<220>

<223> D17S1955 forward primer
<400> 279
tgtaatgtaa gccccatgag g 21

<210> 280

<211> 25

<212> DNA

<213> Artificial Sequence

<220>

<223> D17S1955 reverse primer
<400> 280
cactcaactc aacagtctaa aggtg 25

<210> 281

<211> 25

<212> DNA

<213> Artificial Sequence

<220>

<223> D17S2098 forward primer
<400> 281
gtgagttcaa gcatagtaat tatcc 25

<210> 282

<211> 23

<212> DNA

<213> Artificial Sequence

<220>

<223> D17S2098 reverse primer
<400> 282
attcagcctc agttcactgc ttc 23

<210> 283

<211> 20

<212> DNA

<213> Artificial Sequence

<220>

<223> D17S518 forward primer
<400> 283
gatccagtgg agactcagag 20

<210> 284

<211> 20

<212> DNA

<213> Artificial Sequence

<220>

<223> D17S518 reverse primer
<400> 284
tagtctctgg gacacccaga 20

<210> 285

<211> 25

<212> DNA

<213> Artificial Sequence

<220>

<223> D17S518 forward primer
<400> 285
attcctgagt gtctaccctg ttgag 25

<210> 286

<211> 17

<212> DNA

<213> Artificial Sequence

<220>

<223> D17S518 reverse primer
<400> 286
actgactgcg ccactgc 17

<210> 287

<211> 20

<212> DNA

<213> Artificial Sequence

<220>

<223> D11S4358 forward primer
<400> 287
tcgagaagga caaaatcacc 20

<210> 288

<211> 20

<212> DNA

<213> Artificial Sequence

<220>

<223> D11S4358 reverse primer
<400> 288
gaacagggtt agtccattcg 20

<210> 289

<211> 19

<212> DNA

<213> Artificial Sequence

<220>

<223> D17S964 forward primer
<400> 289
gttctttcct cttgtgggg 19

<210> 290

<211> 19

<212> DNA

<213> Artificial Sequence

<220>

<223> D17S964 reverse primer
<400> 290
agtcagctga gattgtgcc 19

<210> 291

<211> 20

<212> DNA

<213> Artificial Sequence

<220>

<223> D19S1091 forward primer
<400> 291
caagccaaga catcccagtt 20

<210> 292

<211> 20

<212> DNA

<213> Artificial Sequence

<220>

<223> D19S1091 reverse primer
<400> 292
ccccacacac agctcatatg 20

<210> 293

<211> 22

<212> DNA

<213> Artificial Sequence

<220>

<223> D17S1179 forward primer
<400> 293
ttttctctct cattccattg gg 22

<210> 294

<211> 20

<212> DNA

<213> Artificial Sequence

<220>

<223> D17S1179 reverse primer
<400> 294
gcaacagagg gagactccaa 20

<210> 295

<211> 19

<212> DNA

<213> Artificial Sequence

<220>

<223> D10S2160 forward primer
<400> 295
tcccatcccg taagacctc 19

<210> 296

<211> 25

<212> DNA

<213> Artificial Sequence

<220>

<223> D10S2160 reverse primer
<400> 296
tatggagtac ctactctatg ccagg 25

<210> 297

<211> 20

<212> DNA

<213> Artificial Sequence

<220>

<223> D17S1230 forward primer
<400> 297
attcaaagct ggatcccttt 20

<210> 298

<211> 20

<212> DNA

<213> Artificial Sequence

<220>

<223> D17S1230 reverse primer
<400> 298
agctgtgaca aatgcctgta 20

<210> 299

<211> 20

<212> DNA

<213> Artificial Sequence

<220>

<223> D17S1338 forward primer
<400> 299
tcacctgaga ttgggagacc 20

<210> 300

<211> 18

<212> DNA

<213> Artificial Sequence

<220>

<223> D17S1338 reverse primer
<400> 300
aagatggggc aggaatgg 18

<210> 301

<211> 19

<212> DNA

<213> Artificial Sequence

<220>

<223> D17S2011 forward primer
<400> 301
tcactgtcct ccaagccag 19

<210> 302

<211> 20

<212> DNA

<213> Artificial Sequence

<220>

<223> D17S2011 reverse primer
<400> 302
aaacaccaca ctctcccctg 20

<210> 303

<211> 20

<212> DNA

<213> Artificial Sequence

<220>

<223> D17S2011 forward primer
<400> 303
ttcttgggct tcccgtagcc 20

<210> 304

<211> 20

<212> DNA

<213> Artificial Sequence

<220>

<223> D17S2011 reverse primer
<400> 304
ggggcagacg acttctcctt 20

<210> 305

<211> 23

<212> DNA

<213> Artificial Sequence

<220>

<223> D17S2038 forward primer
<400> 305
ggggatacaa cctttaaagt tcc 23

<210> 306

<211> 25

<212> DNA

<213> Artificial Sequence

<220>

<223> D17S2038 reverse primer
<400> 306
attcacctaa tgaggattct tcttt 25

<210> 307

<211> 24

<212> DNA

<213> Artificial Sequence

<220>

<223> D17S2091 forward primer
<400> 307
gctgaaatag ccatcttgag ctac 24

<210> 308

<211> 23

<212> DNA

<213> Artificial Sequence

<220>

<223> D17S2091 reverse primer
<400> 308
tccgcatcct ttttaagagg cac 23

<210> 309

<211> 24

<212> DNA

<213> Artificial Sequence

<220>

<223> D17S649 forward primer
<400> 309
ctttcactct ttcagctgaa gagg 24

<210> 310

<211> 25

<212> DNA

<213> Artificial Sequence

<220>

<223> D17S649 reverse primer
<400> 310
tgacgtgcta tttcctgttt tgtct 25

<210> 311

<211> 18

<212> DNA

<213> Artificial Sequence

<220>

| No. | Doccode | Number of pages |
|---|---|---|
| 1 | 1001 | 6 |
| 2 | 1002 | 1 |
| 3 | DESC | 4 |
| 4 | CLMS | 1 |
| 5 | DRAW | 1 |
| 6 | ABST | 1 |
| 7 | PRIODOC | 8 |

<220>

<223> D17S1190 reverse primer
<400> 312
caacacacta ccccagga

## Description

### TECHNICAL FIELD OF THE INVENTION

The invention relates to methods and compositions for the prediction, diagnosis, prognosis, prevention and treatment of neoplastic disease. Neoplastic disease is often caused by chromosomal rearrangements which lead to over- or underexpression of the rearranged genes. The invention discloses genes which are overexpressed in neoplastic tissue and are useful as diagnostic markers and targets for treatment. Methods are disclosed for predicting, diagnosing and prognosing as well as preventing and treating neoplastic disease.

### BACKGROUND OF THE INVENTION

Chromosomal aberrations (amplifications, deletions, inversions, insertions, translocations and/or viral integrations) are of importance for the development of cancer and neoplastic lesions, as they account for deregulations of the respective regions. Amplifications of genomic regions have been described, in which genes of importance for growth characteristics, differentiation, invasiveness or resistance to therapeutic intervention are located. One of those regions with chromosomal aberrations is the region carrying the HER-2/neu gene which is amplified in breast cancer patients. In approximately 25% of breast cancer patients the HER-2/neu gene is overexpressed due to gene amplification. HER-2/neu overexpression correlates with a poor prognosis (relapse, overall survival, sensitivity to therapeutics). The importance of HER-2/neu for the prognosis of the disease progression has been described [Gusterson et al., 1992, (1)]. Gene specific antibodies raised against HER-2/neu (Herceptin™) have been generated to treat the respective cancer patients. However, only about 50% of the patients benefit from the antibody treatment with Herceptin™, which is most often combined with chemotherapeutic regimen. The discrepancy of HER-2/neu positive tumors (overexpressing HER-2/neu to similar extent) with regard to responsiveness to therapeutic intervention suggest, that there might be additional factors or genes being involved in growth and apoptotic characteristics of the respective tumor tissues. There seems to be no monocausal relationship between overexpression of the growth factor receptor HER-2/neu and therapy outcome. In line with this the measurement of commonly used tumor markers such as estrogen receptor, progesterone receptor, p53 and Ki-67 do provide only very limited information on clinical outcome of specific therapeutic decisions. Therefore there is a great need for a more detailed diagnostic and prognostic classification of tumors to enable improved therapy decisions and prediction of survival of the patients. The present invention addresses the need for additional markers by providing genes, which expression is deregulated in tumors and correlates with clinical outcome. One focus is the deregulation of genes present in specific chromosomal regions and their interaction in disease development and drug responsiveness.

HER-2/neu and other markers for neoplastic disease are commonly assayed with diagnostic methods such as immunohistochemistry (IHC) (e.g. HercepTest™ from DAKO Inc.) and Fluorescence-In-Situ-Hybridization (FISH) (e.g. quantitative measurement of the HER-2/neu and Topoisomerase II alpha with a fluorescence-*in-situ*-Hybridization kit from VYSIS). Additionally HER-2/neu can be assayed by detecting HER-2/neu fragments in serum with an ELISA test (BAYER Corp.) or a with a quantitative PCR kit which compares the amount of HER-2/neu gene with the amount of a non-amplified control gene in order to detect HER-2/neu gene amplifications (ROCHE). These methods, however, exhibit multiple disadvantages with regard to sensitivity, specificity, technical and personnel efforts, costs, time consumption, inter-lab reproducibility. These methods are also restricted with regard to measurement of multiple parameters within one patient sample ("multiplexing"). Usually only about 3 to 4 parameters (e.g. genes or gene products) can be detected per tissue slide. Therefore, there is a need to develop a fast and simple test to measure simultaneously multiple parameters in one sample. The present invention addresses the need for a fast and simple high-resolution method, that is able to detect multiple diagnostic and prognostic markers simultaneously.

### SUMMARY OF THE INVENTION

The present invention is based on discovery that chromosomal alterations in cancer tissues can lead to changes in the expression of genes that are encoded by the altered chromosomal regions. Exemplary 43 human genes have been identified that are co-amplified in neoplastic lesions from breast cancer tissue resulting in altered expression of several of these genes (Tables 1 to 4). These 43 genes are differentially expressed in breast cancer states, relative to their expression in normal, or non-breast cancer states. The present invention relates to derivatives, fragments, analogues and homologues of these genes and uses or methods of using of the same.

The present invention further relates to novel preventive, predictive, diagnostic, prognostic and therapeutic compositions and uses for malignant neoplasia and breast cancer in particular. Especially membrane bound marker gene products containing extracellular domains can be a particularly useful target for treatment methods as well as diagnostic and clinical monitoring methods.

It is a discovery of the present invention that several of these genes are characterized in that their gene products functionally interact in signaling cascades or by directly or indirectly influencing each other. This interaction is important for the normal physiology of certain non-neoplastic tissues (e.g. brain or neurogenic tissue). The deregulation of these genes in neoplastic lesions where they are normally exhibit of different level of activity or are not active, however, results in pathophysiology and affects the characteristics of the disease-associated tissue.

The present invention further relates to methods for detecting these deregulations in malignant neoplasia on DNA and mRNA level.

The present invention further relates to a method for the detection of chromosomal alterations characterized in that the relative abundance of individual mRNAs, encoded by genes, located in altered chromosomal regions is detected.

The present invention further relates to a method for the detection of the flanking breakpoints of named chromosomal alterations by measurement of DNA copy number by quantitative PCR or DNA-Arrays and DNA sequencing.

A method for the prediction, diagnosis or prognosis of malignant neoplasia by the detection of DNA sequences flanking named genomic breakpoint or are located within such.

The present invention further relates to a method for the detection of chromosomal alterations characterized in that the copy number of one or more genomic nucleic acid sequences located within an altered chromosomal region (s) is detected by quantitative PCR techniques (e.g. TaqMan™, Lightcycler™ and iCycler™).

The present invention further relates to a method for the prediction, diagnosis or prognosis of malignant neoplasia by the detection of at least 2 markers whereby the markers are genes and fragments thereof or genomic nucleic acid sequences that are located on one chromosomal region which is altered in malignant neoplasia and breast cancer in particular.

The present invention also discloses a method for the prediction, diagnosis or prognosis of malignant neoplasia by the detection of at least 2 markers whereby the markers are located on one or more chromosomal region(s) which is/are altered in malignant neoplasia; and the markers interact as (i) receptor and ligand or (ii) members of the same signal transduction pathway or (iii)members of synergistic signal transduction pathways or (iv) members of antagonistic signal transduction pathways or (v) transcription factor and transcription factor binding site.

Also dislcosed is a method for the prediction, diagnosis or prognosis of malignant neoplasia by the detection of at least one marker whereby the marker is a VNTR, SNP, RFLP or STS which is located on one chromosomal region which is altered in malignant neoplasia due to amplification and the marker is detected in (a) a cancerous and (b) a non cancerous tissue or biological sample from the same individual. A preferred embodiment is the detection of at least one VNTR marker of Table 6 or at least on SNP marker of Table 4 or combinations thereof.. Even more preferred can the detection, quantification and sizing of such polymorphic markers be achieved by methods of (a) for the comparative measurement of amount and size by PCR amplification and subsequent capillary electrophoresis, (b) for sequence determination and allelic discrimination by gel electrophoresis (e.g. SSCP, DGGE), real time kinetic PCR, direct DNA sequencing, pyro-sequencing, mass-specific allelic discrimination or resequencing by DNA array technologies, (c) for the dertermination of specific restriction patterns and subsequent electrophoretic separation and (d) for allelic discrimination by allel specific PCR (e.g. ASO). An even more favorable detection of a hetrozygous VNTR, SNP, RFLP or STS is done in a multiplex fashion, utilizing a variety of labeled primers (e.g. fluorescent, radioactive, bioactive) and a suitable capillary electrophoresis (CE) detection system.

In another embodiment the expression of these genes can be detected with DNA-arrays as described in WO9727317 and US6379895.

In a further embodiment the expression of these genes can be detected with bead based direct flourescent readout techniques such as described in WO9714028 and WO9952708.

In one embodiment, the invention pertains to a method of determining the phenotype of a cell or tissue, comprising detecting the differential expression, relative to a normal or untreated cell, of at least one polynucleotide comprising SEQ ID NO: 2 to 6, 8, 9, 11 to 16, 18, 19 or 21 to 26 or 53 to 75, wherein the polynucleotide is differentially expressed by at least about 1.5 fold, at least about 2 fold or at least about 3 fold.

In a further aspect the invention pertains to a method of determining the phenotype of a cell or tissue, comprising detecting the differential expression, relative to a normal or untreated cell, of at least one polynucleotide which hybridizes under stringent conditions to one of the polynucleotides of SEQ ID NO: 2 to 6, 8, 9, 11 to 16, 18, 19 or 21 to 26 or 53 to 75 and encodes a polypeptide exhibiting the same biological function as given in Table 2 or 3 for the respective polynucleotide, wherein the polynucleotide is differentially expressed by at least at least about 1.5 fold , at least about 2 fold or at least about 3 fold.

In another embodiment of the invention a polynucleotide comprising a polynucleotide selected from SEQ ID NO: 2 to 6, 8, 9, 11 to 16, 18, 19 or 21 to 26 and 53 to 75 or encoding one of the polypeptides with SEQ ID NO: 28 to 32, 34, 35, 37 to 42, 44, 45 or 47 to 52 or 76 to 98 can be used to identify cells or tissue in individuals which exhibit a phenotype predisposed to breast cancer or a diseased phenotype, thereby (a) predicting whether an individual is at risk for the development, or (b) diagnosing whether an individual is having, or (c) prognosing the progression or the outcome of the treatment malignant neoplasia and breast cancer in particular.

In yet another embodiment the invention provides a method for identifying genomic regions which are altered on the chromosomal level and encode genes that are linked by function and are differentially expressed in malignant neoplasia and breast cancer in particular.

In yet another embodiment the invention provides the genomic regions 17q12, 3p21 and 12q13 for use in prediction, diagnosis and prognosis as well as prevention and treatment of malignant neoplasia and breast cancer. In particular not only the intragenic regions, but also intergenic regions, pseudogenes or non-transcribed genes of said chromosomal regions can be used for diagnostic, predictive, prognostic and preventive and therapeutic compositions and methods.

In yet another embodiment the invention provides methods of screening for agents which regulate the activity of a polypeptide comprising a polypeptide selected from SEQ ID NO: 27 to 52 and 76 to 98 or encoded by a polynucleotide comprising a polynucleotide selected from SEQ ID NO: 1 to 26 and 53 to 75. A test compound is contacted with a polypeptide comprising a polypeptide selected from SEQ ID NO: 27 to 52 and 76 to 98 or encoded by a polynucleotide comprising a polynucleotide selected from SEQ ID NO: 1 to 26 and 53 to 75. Binding of the test compound to the polypeptide is detected. A test compound which binds to the polypeptide is thereby identified as a potential therapeutic agent for the treatment of malignant neoplasia and more particularly breast cancer.

In even another embodiment the invention provides another method of screening for agents which regulate the activity of a polypeptide comprising a polypeptide selected from SEQ ID NO: 27 to 52 and 76 to 98 or encoded by a polynucleotide comprising a polynucleotide selected from SEQ ID NO: 1 to 26 and 53 to 75. A test compound is contacted with a polypeptide comprising a polypeptide selected from SEQ ID NO: 27 to 52 and 76 to 98 or encoded by a polynucleotide comprising a polynucleotide selected from SEQ ID NO: 1 to 26 and 53 to 75. A biological activity mediated by the polypeptide is detected. A test compound which decreases the biological activity is thereby identified as a potential therapeutic agent for decreasing the activity of the polypeptide encoded by a polypeptide comprising a polypeptide selected from SEQ ID NO: 27 to 52 and 76 to 98 or encoded by a polynucleotide comprising a polynucleotide selected from SEQ ID NO: 1 to 26 and 53 to 75 in malignant neoplasia and breast cancer in particular. A test compound which increases the biological activity is thereby identified as a potential therapeutic agent for increasing the activity of the polypeptide encoded by a polypeptide selected from one of the polypeptides with SEQ ID NO: 27 to 52 and 76 to 98 or encoded by a polynucleotide comprising a polynucleotide selected from SEQ ID NO: 1 to 26 and 53 to 75 in malignant neoplasia and breast cancer in particular.

In another embodiment the invention provides a method of screening for agents which regulate the activity of a polynucleotide comprising a polynucleotide selected from SEQ ID NO: 1 to 26 and 53 to 75. A test compound is contacted with a polynucleotide comprising a polynucleotide selected from SEQ ID NO: 1 to 26 and 53 to 75. Binding of the test compound to the polynucleotide comprising a polynucleotide selected from SEQ ID NO: 1 to 26 and 53 to 75 is detected. A test compound which binds to the polynucleotide is thereby identified as a potential therapeutic agent for regulating the activity of a polynucleotide comprising a polynucleotide selected from SEQ ID NO: 1 to 26 and 53 to 75 in malignant neoplasia and breast cancer in particular.

The invention thus provides polypeptides selected from one of the polypeptides with SEQ ID NO: 27 to 52 and 76 to 98 or encoded by a polynucleotide comprising a polynucleotide selected from SEQ ID NO: 1 to 26 and 53 to 75 which can be used to identify compounds which may act, for example, as regulators or modulators such as agonists and antagonists, partial agonists, inverse agonists, activators, co-activators and inhibitors of the polypeptide comprising a polypeptide selected from SEQ ID NO: 27 to 52 and 76 to 98 or encoded by a polynucleotide comprising a polynucleotide selected from SEQ ID NO: 1 to 26 and 53 to 75. Accordingly, the invention provides reagents and methods for regulating a polypeptide comprising a polypeptide selected from SEQ ID NO: 27 to 52 and 76 to 98 or encoded by a polynucleotide comprising a polynucleotide selected from SEQ ID NO: 1 to 26 and 53 to 75 in malignant neoplasia and more particularly breast cancer. The regulation can be an up-or down regulation. Reagents that modulate the expression, stability or amount of a polynucleotide comprising a polynucleotide selected from SEQ ID NO: 1 to 26 and 53 to 75 or the activity of the polypeptide comprising a polypeptide selected from SEQ ID NO: 27 to 52 and 76 to 98 or encoded by a polynucleotide comprising a polynucleotide selected from SEQ ID NO: 1 to 26 and 53 to 75 can be a protein, a peptide, a peptidomimetic, a nucleic acid, a nucleic acid analogue (e.g. peptide nucleic acid, locked nucleic acid) or a small molecule. Methods that modulate the expression, stability or amount of a polynucleotide comprising a polynucleotide selected from SEQ ID NO: 1 to 26 and 53 to 75 or the activity of the polypeptide comprising a polypeptide selected from SEQ ID NO: 27 to 52 and 76 to 98 or encoded by a polynucleotide comprising a polynucleotide selected from SEQ ID NO: 1 to 26 and 53 to 75 can be gene replacement therapies, antisense, ribozyme and triplex nucleic acid approaches.

In one embodiment of the invention provides antibodies which specifically bind to a full-length or partial polypeptide comprising a polypeptide selected from SEQ ID NO: 27 to 52 and 76 to 98 or encoded by a polynucleotide comprising a polynucleotide selected from SEQ ID NO: 1 to 26 and 53 to 75 or a polynucleotide comprising a polynucleotide selected from SEQ ID NO: 1 to 26 and 53 to 75 for use in prediction, prevention, diagnosis, prognosis and treatment of malignant neoplasia and breast cancer in particular.

Yet another embodiment of the invention is the use of a reagent which specifically binds to a polynucleotide comprising a polynucleotide selected from SEQ ID NO: 1 to 26 and 53 to 75 or a polypeptide comprising a polypeptide selected from SEQ ID NO: 27 to 52 and 76 to 98 or encoded by a polynucleotide comprising a polynucleotide selected from SEQ ID NO: 1 to 26 and 53 to 75 in the preparation of a medicament for the treatment of malignant neoplasia and breast cancer in particular.

Still another embodiment is the use of a reagent that modulates the activity or stability of a polypeptide comprising a polypeptide selected from SEQ ID NO: 27 to 52 and 76 to 98 or encoded by a polynucleotide comprising a polynucleotide selected from SEQ ID NO: 1 to 26 and 53 to 75 or the expression, amount or stability of a polynucleotide comprising a polynucleotide selected from SEQ ID NO: 1 to 26 and 53 to 75 in the preparation of a medicament for the treatment of malignant neoplasia and breast cancer in particular.

Still another embodiment of the invention is a pharmaceutical composition which includes a reagent which specifically binds to a polynucleotide comprising a polynucleotide selected from SEQ ID NO: 1 to 26 and 53 to 75 or a polypeptide comprising a polypeptide selected from SEQ ID NO: 27 to 52 and 76 to 98 or encoded by a polynucleotide comprising a polynucleotide selected from SEQ ID NO: 1 to 26 and 53 to 75, and a pharmaceutically acceptable carrier.

Yet another embodiment of the invention is a pharmaceutical composition including a polynucleotide comprising a polynucleotide selected from SEQ ID NO: 1 to 26 and 53 to 75 or encoding a polypeptide comprising a polypeptide selected from SEQ ID NO: 27 to 52 and 76 to 98.

In one embodiment, a reagent which alters the level of expression in a cell of a polynucleotide comprising a polynucleotide selected from SEQ ID NO: 1 to 26 and 53 to 75 or encoding a polypeptide comprising a polypeptide selected from SEQ ID NO: 27 to 52 and 76 to 98, or a sequence complementary thereto, is identified by providing a cell, treating the cell with a test reagent, determining the level of expression in the cell of a polynucleotide comprising a polynucleotide selected from SEQ ID NO: 1 to 26 and 53 to 75 or encoding a polypeptide comprising a polypeptide selected from SEQ ID NO: 27 to 52 and 76 to 98 or a sequence complementary thereto, and comparing the level of expression of the polynucleotide in the treated cell with the level of expression of the polynucleotide in an untreated cell, wherein a change in the level of expression of the polynucleotide in the treated cell relative to the level of expression of the polynucleotide in the untreated cell is indicative of an agent which alters the level of expression of the polynucleotide in a cell.

The invention further provides a pharmaceutical composition comprising a reagent identified by this method.

Another embodiment of the invention is a pharmaceutical composition which includes a polypeptide comprising a polypeptide selected from SEQ ID NO: 27 to 52 and 76 to 98 or which is encoded by a polynucleotide comprising a polynucleotide selected from SEQ ID NO: 1 to 26 and 53 to 75.

A further embodiment of the invention is a pharmaceutical composition comprising a polynucleotide including a sequence which hybridizes under stringent conditions to a polynucleotide comprising a polynucleotide selected from SEQ ID NO: 1 to 26 and 53 to 75 and encoding a polypeptide exhibiting the same biological function as given for the respective polynucleotide in Table 2 or 3, or encoding a polypeptide comprising a polypeptide selected from SEQ ID NO: 27 to 52 and 76 to 98. Pharmaceutical compositions, useful in the present invention may further include fusion proteins comprising a polypeptide comprising a polynucleotide selected from SEQ ID NO: 27 to 52 and 76 to 98, or a fragment thereof, antibodies, or antibody fragments

## BRIEF DESCRIPTION OF THE DRAWINGS

Fig. 1 shows a sketch of the chromosome 17 with G-banding pattern and cytogenetic positions. In the blow out at the lower part of the figure a detailed view of the chromosomal area of the long arm of chromosome 17 (17q 12-21.1) is provided. Each vertical rectangle depicted in medium gray, represents a gene as labeled below or above the individual position. The order of genes depicted in this graph has been deduced from experiments questioning the amplification an over expression and from public available data (e.g. UCSC, NCBI or Ensemble).

Fig. 2 shows the same region as depicted before in Fig. 1 and a cluster representation of the individual expression values measured by DNA-chip hybridization. The gene representing squares are indicated by a dotted line. In the upper part of the cluster representation 4 tumor cell lines, of which two harbor a known HER-2/neu over expression (SKBR3 and AU565), are depicted with their individual expression profiles. Not only the HER-2/neu gene shows a clear over expression but as provided by this invention several other genes with in the surrounding. In the middle part of the cluster representation expression data obtained from immune histochemically characterized tumor samples are presented. Two of the depicted probes show a significant over expression of genes marked by the white rectangles. For additional information and comparison expression profiles of several non diseased human tissues (RNAs obtained from Clontech Inc.) are provided. Closest relation to the expression profile of HER-2/neu positive tumors displays human brain and neural tissue.

Fig. 3 provides data from DNA amplification measurements by qPCR (e.g. TaqMan). Data indicates that in several analyzed breast cancer cell lines harbor amplification of genes which were located in the previously described region (ARCHEON). Data were displayed for each gene on the x-axis and 40-Ct at the y-axis. Data were normalized to the expression level of GAPDH as seen in the first group of columns.

Fig. 4 represents a graphical overview on the amplified regions and provides information on the length of the individual amplification and over expression in the analyzed tumor cell lines. The length of the amplification and the composition of genes has a significant impact on the nature of the cancer cell and on the responsiveness on certain drugs, as described elsewhere.

## DETAILED DESCRIPTION OF THE INVENTION

*Definitions*

"Differential expression", as used herein, refers to both quantitative as well as qualitative differences in the genes' expression patterns depending on differential development and/or tumor growth. Differentially expressed genes may represent "marker genes," and/or "target genes". The expression pattern of a differentially expressed gene disclosed herein may be utilized as part of a prognostic or diagnostic breast cancer evaluation. Alternatively, a differentially expressed gene disclosed herein may be used in methods for identifying reagents and compounds and uses of these reagents and compounds for the treatment of breast cancer as well as methods of treatment.

"Biological activity" or "bioactivity" or "activity" or "biological function", which are used interchangeably, herein mean an effector or antigenic function that is directly or indirectly performed by a polypeptide (whether in its native or denatured conformation), or by any fragment thereof *in vivo* or *in vitro.* Biological activities include but are not limited to binding to polypeptides, binding to other proteins or molecules, enzymatic activity, signal transduction, activity as a DNA binding protein, as a transcription regulator, ability to bind damaged DNA, etc. A bioactivity can be modulated by directly affecting the subject polypeptide. Alternatively, a bioactivity can be altered by modulating the level of the polypeptide, such as by modulating expression of the corresponding gene.

The term "marker" or "biomarker" refers a biological molecule, e.g., a nucleic acid, peptide, hormone, etc., whose presence or concentration can be detected and correlated with a known condition, such as a disease state.

"Marker gene," as used herein, refers to a differentially expressed gene which expression pattern may be utilized as part of predictive, prognostic or diagnostic malignant neoplasia or breast cancer evaluation, or which, alternatively, may be used in methods for identifying compounds useful for the treatment or prevention of malignant neoplasia and breast cancer in particular. A marker gene may also have the characteristics of a target gene.

"Target gene", as used herein, refers to a differentially expressed gene involved in breast cancer in a manner by which modulation of the level of target gene expression or of target gene product activity may act to ameliorate symptoms of malignant neoplasia and breast cancer in particular. A target gene may also have the characteristics of a marker gene.

The term "biological sample", as used herein, refers to a sample obtained from an organism or from components (e.g., cells) of an organism. The sample may be of any biological tissue or fluid. Frequently the sample will be a "clinical sample" which is a sample derived from a patient. Such samples include, but are not limited to, sputum, blood, blood cells (e.g., white cells), tissue or fine needle biopsy samples, cell-containing bodyfluids, free floating nucleic acids, urine, peritoneal fluid, and pleural fluid, or cells therefrom. Biological samples may also include sections of tissues such as frozen sections taken for histological purposes.

By "array" or "matrix" is meant an arrangement of addressable locations or "addresses" on a device. The locations can be arranged in two dimensional arrays, three dimensional arrays, or other matrix formats. The number of locations can range from several to at least hundreds of thousands. Most importantly, each location represents a totally independent reaction site. Arrays include but are not limited to nucleic acid arrays, protein arrays and antibody arrays. A "nucleic acid array" refers to an array containing nucleic acid probes, such as oligonucleotides, polynucleotides or larger portions of genes. The nucleic acid on the array is preferably single stranded. Arrays wherein the probes are oligonucleotides are referred to as "oligonucleotide arrays" or "oligonucleotide chips." A "microarray," herein also refers to a "biochip" or "biological chip", an array of regions having a density of discrete regions of at least about $100/cm^2$, and preferably at least about $1000/cm^2$. The regions in a microarray have typical dimensions, e.g., diameters, in the range of between about 10-250 $\mu$m, and are separated from other regions in the array by about the same distance. A "protein array" refers to an array containing polypeptide probes or protein probes which can be in native form or denatured. An "antibody array" refers to an array containing antibodies which include but are not limited to monoclonal antibodies (e.g. from a mouse), chimeric antibodies, humanized antibodies or phage antibodies and single chain antibodies as well as fragments from antibodies.

The term "agonist", as used herein, is meant to refer to an agent that mimics or upregulates (e.g., potentiates or supplements) the bioactivity of a protein. An agonist can be a wild-type protein or derivative thereof having at least one bioactivity of the wild-type protein. An agonist can also be a compound that upregulates expression of a gene or which increases at least one bioactivity of a protein. An agonist can also be a compound which increases the interaction of a polypeptide with another molecule, e.g., a target peptide or nucleic acid.

The term "antagonist" as used herein is meant to refer to an agent that downregulates (e.g., suppresses or inhibits) at least one bioactivity of a protein. An antagonist can be a compound which inhibits or decreases the interaction between a protein and another molecule, e.g., a target peptide, a ligand or an enzyme substrate. An antagonist can also be a compound that downregulates expression of a gene or which reduces the amount of expressed protein present.

"Small molecule" as used herein, is meant to refer to a composition, which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies have extensive libraries of chemical and/or biological mixtures, often fungal, bacterial, or algal extracts, which can be screened with any of the assays of the invention to identify compounds that modulate a bioactivity.

The terms "modulated" or "modulation" or "regulated" or "regulation" and "differentially regulated" as used herein refer to both upregulation (i.e., activation or stimulation (e.g., by agonizing or potentiating) and down regulation [i.e., inhibition or suppression (e.g., by antagonizing, decreasing or inhibiting)].

"Transcriptional regulatory unit" refers to DNA sequences, such as initiation signals, enhancers, and promoters, which induce or control transcription of protein coding sequences with which they are operably linked. In preferred embodiments, transcription of one of the genes is under the control of a promoter sequence (or other transcriptional regulatory sequence) which controls the expression of the recombinant gene in a cell-type in which expression is intended. It will also be understood that the recombinant gene can be under the control of transcriptional regulatory sequences which are the same or which are different from those sequences which control transcription of the naturally occurring forms of the polypeptide.

The term "derivative" refers to the chemical modification of a polypeptide sequence, or a polynucleotide sequence. Chemical modifications of a polynucleotide sequence can include, for example, replacement of hydrogen by an alkyl, acyl, or amino group. A derivative polynucleotide encodes a polypeptide which retains at least one biological or immunological function of the natural molecule. A derivative polypeptide is one modified by glycosylation, pegylation, or any similar process that retains at least one biological or immunological function of the polypeptide from which it was derived.

The term "nucleotide analog" refers to oligomers or polymers being at least in one feature different from naturally occurring nucleotides, oligonucleotides or polynucleotides, but exhibiting functional features of the respective naturally occurring nucleotides (e.g. base paring, hybridization, coding information) and that can be used for said compositions. The nucleotide analogs can consist of non-naturally occurring bases or polymer backbones, examples of which are LNAs, PNAs and Morpholinos. The nucleotide analog has at least one molecule different from its naturally occurring counterpart or equivalent.

"BREAST CANCER GENES" or "BREAST CANCER GENE" as used herein refers to the polynucleotides of SEQ ID NO: 1 to 26 and 53 to 75, as well as derivatives, fragments, analogs and homologues thereof, the polypeptides encoded thereby, the polypeptides of SEQ ID NO: 27 to 52 and 76 to 98 as well as derivatives, fragments, analogs and homologues thereof and the corresponding genomic transcription units which can be derived or identified with standard techniques well known in the art using the information disclosed in Tables 1 to 5 and Figures 1 to 4. The GenBank, Locuslink ID and the UniGene accession numbers of the polynucleotide sequences of the SEQ ID NO: 1 to 26 and 53 to 75 and the polypeptides of the SEQ ID NO: 27 to 52 and 76 to 98 are shown in Table 1, the gene description, gene function and subcellular localization is given in Tables 2 and 3.

The term "chromosomal region" as used herein refers to a consecutive DNA stretch on a chromosome which can be defined by cytogenetic or other genetic markers such as e.g. restriction length polymorphisms (RFLPs), single nucleotide polymorphisms (SNPs), expressed sequence tags (ESTs), sequence tagged sites (STSs), micro-satellites, variable number of tandem repeats (VNTRs) and genes. Typically a chromosomal region consists of up to 2 Megabases (MB), up to 4 MB, up to 6 MB, up to 8 MB, up to 10 MB, up to 20 MB or even more MB.

The term "altered chromosomal region" or" abberant chromosomal region" refers to a structural change of the chromosomal composition and DNA sequence, which can occur by the following events: amplifications, deletions, inversions, insertions, translocations and/or viral integrations. A trisomy, where a given cell harbors more than two copies of a chromosome, is within the meaning of the term "amplification" of a chromosome or chromosomal region.

The present invention provides polynucleotide sequences and proteins encoded thereby, as well as probes derived from the polynucleotide sequences, antibodies directed to the encoded proteins, and predictive, preventive, diagnostic, prognostic and therapeutic uses for individuals which are at risk for or which have malignant neoplasia and breast cancer in particular. The sequences disclosure herein have been found to be differentially expressed in samples from breast cancer.

The present invention is based on the identification of 43 genes that are differentially regulated (up- or down-regulated) in tumor biopsies of patients with clinical evidence of breast cancer. The identification of 43 human genes which were not known to be differentially regulated in breast cancer states and their significance for the disease is described in the working examples herein. The characterization of the co-expression of these genes provides newly identified roles in breast cancer. The gene names, the database accession numbers (GenBank and UniGene) as well as the putative or known functions of the encoded proteins and their subcellular localization are given in Tables 1 to 4. The primer sequences used for the gene amplification are shown in Table 5.

In either situation, detecting expression of these genes in excess or in with lower level as compared to normal expression provides the basis for the diagnosis of malignant neoplasia and breast cancer. Furthermore, in testing the efficacy of compounds during clinical trials, a decrease in the level of the expression of these genes corresponds to a return from a disease condition to a normal state, and thereby indicates a positive effect of the compound.

Another aspect of the present invention is based on the observation that neighboring genes within defined genomic regions functionally interact and influence each others function directly or indirectly. A genomic region encoding functionally interacting genes that are co-amplified and co-expressed in neoplastic lesions has been defined as an "ARCHEON". (ARCHEON = **A**ltered **R**egion of Changed **C**hromosomal **E**xpression **O**bserved in **N**eoplasms). Chromosomal alterations often affect more than one gene. This is true for amplifications, duplications, insertions, integrations, inversions, translocations, and deletions. These changes can have influence on the expression level of single or multiple genes. Most commonly in the field of cancer diagnostics and treatment the changes of expression levels have been investigated for single, putative relevant target genes such as MLVI2 (5p14), NRASL3 (6p12), EGFR (7p12), c-myc (8q23), Cyclin D1 (11q13), IGF1R (15q25), HER-2/neu (17q12), PCNA (20q12). However, the altered expression level and interaction of multiple (i.e. more than two) genes within one genomic region with each other has not been addressed. Genes of an ARCHEON form gene clusters with tissue specific expression patterns. The mode of interaction of individual genes within such a gene cluster suspected to represent an ARCHEON can be either protein-protein or protein-nucleic acid interaction, which may be illustrated but not limited by the following examples: ARCHEON gene interaction may be in the same signal transduction pathway, may be receptor to ligand binding, receptor kinase and SH2 or SH3 binding, transcription factor to promoter binding, nuclear hormone receptor to transcription factor binding, phosphogroup donation (e.g. kinases) and acceptance (e.g. phosphoprotein), mRNA stabilizing protein binding and transcriptional processes. The individual activity and specificity of a pair genes and or the proteins encoded thereby or of a group of such in a higher order, may be readily deduced from literature, published or deposited within public databases by the skilled person. However in the context of an ARCHEON the interaction of members being part of an ARCHEON will potentiate, exaggerate or reduce their singular functions. This interaction is of importance in defined normal tissues in which they are normally co-expressed. Therefore, these clusters have been commonly conserved during evolution. The aberrant expression of members of these ARCHEON in neoplastic lesions, however, (especially within tissues in which they are normally not expressed) has influence on tumor characteristics such as growth, invasiveness and drug responsiveness. Due to the interaction of these neighboring genes it is of importance to determine the members of the ARCHEON which are involved in the deregulation events. In this regard amplification and deletion events in neoplastic lesions are of special interest.

The invention relates to a method for the detection of chromosomal alterations by (a) determining the relative mRNA abundance of individual mRNA species or (b) determining the copy number of one or more chromosomal region (s) by quantitative PCR. In one embodiment information on the genomic organization and spatial regulation of chromosomal regions is assessed by bioinformatic analysis of the sequence information of the human genome (UCSC, NCBI) and then combined with RNA expression data from GeneChip™ DNA-Arrays (AfFymetrix) and/or quantitative PCR (TaqMan) from RNA-samples or genomic DNA.

In a further embodiment the functional relationship of genes located on a chromosomal region which is altered (amplified or deleted) is established. The altered chromosomal region is defined as an ARCHEON if genes located on that region functionally interact.

The 17q 12 locus was investigated as one model system, harboring the HER-2/neu gene. By establishing a high-resolution assay to detect amplification events in neighboring genes, 43 genes that are commonly co-amplified in breast cancer cell lines and patient samples were identified. By gene array technologies and immunological methods their co-overexpression in tumor samples was demonstrated. Surprisingly, by clustering tissue samples with HER-2/neu positive Tumor samples, it was found that the expression pattern of this larger genomic region (consisting of 43 genes) is very similar to control brain tissue. HER-2/neu negative breast tumor tissue did not show a similar expression pattern. Indeed, some of the genes within these cluster are important for neural development (HER-2/neu, THRA) in mouse model systems or are described to be expressed in neural cells (NeuroD2). Moreover, by searching similar gene combinations in the human and rodent genome additional homologous chromosomal regions on chromosome 3p21 and 12q13 harboring several isoforms of the respective genes (see below) were found. There was a strong evidence for multiple interactions between the 43 candidate genes, as being part of identical pathways (HER-2, neu, GRB7, CrkRS, CDC6), influencing the expression of each other (HER-2/neu, THRA, RARA), interacting with each other (PPARGBP, THRA, RARA, NR1D1 or HER-2/neu, GRB7) or expressed in defined tissues (CACNB1, PPARGBP, etc.). Interestingly, the genomic regions of the ARCHEONs that were identified are amplified in acquired Tamoxifen resistance of HER-2/neu negative cells (MCF7), which are normally sensitive to Tamoxifen treatment [Achuthan et al., 2001,(2)].

Moreover, altered responsiveness to treatment due to the alterations of the genes within these ARCHEONs was observed. Surprisingly, genes within the ARCHEONs are of importance even in the absence of HER-2/neu homologues. Some of the genes within the ARCHEONs, do not only serve as marker genes for prognostic purposes, but have already been known as targets for therapeutic intervention. For example TOP2 alpha is a target of anthracyclins. THRA and RARA can be targeted by hormones and hormone analogs (e.g. T3, rT3, RA). Due to their high affinity binding sites and available screening assays (reporter assays based on their transcriptional potential) the hormone receptors which are shown to be linked to neoplastic pathophysiology for the first time herein are ideal targets for drug screening and treatment of malignant neoplasia and breast cancer in particular. In this regard it is essential to know which members of the ARCHEON are altered in the neoplastic lesions. Particularly it is important to know the nature, number and extent to which the ARCHEON genes are amplified or deleted. The ARCHEONs are flanked by similar, endogenous retroviruses (e.g. HERV-K= "human endogenous retrovirus"), some of which are activated in breast cancer. These viruses may have also been involved in the evolutionary duplication of the ARCHEONs.

The analysis of the 17q12 region proved data obtained by IHC and identified several additional genes being co-amplified with the HER-2/neu gene. Comparative Analysis of RNA-based quantitative RT-PCR (TaqMan) with DNA-based qPCR from tumor cell lines identified the same amplified region. Genes at the 17q11.2 -21. region are offered by way of illustration not by way of limitation. A graphical display of the described chromosomal region is provided in Figure 1.

*Biological relevance of the genes which are part of the 17q12 ARCHEON*

*MLN50*

By differential screening of cDNAs from breast cancer-derived metastatic axillary lymph nodes, TRAF4 and 3 other novel genes (MLN51, MLN62, MLN64) were identified that are overexpressed in breast cancer [Tomasetto et al., 1995, (3)]. One gene, which they designated MLN50, was mapped to 17q1 1-q21.3 by radioactive in situ hybridization. In breast cancer cell lines, overexpression of the 4 kb MLN50 mRNA was correlated with amplification of the gene and with amplification and overexpression of ERBB2, which maps to the same region. The authors suggested that the 2 genes belong to the same amplicon. Amplification of chromosomal region 17q11-q21 is one of the most common events occurring in human breast cancers. They reported that the predicted 261-amino acid MLN50 protein contains an N-terminal LIM domain and a C-terminal SH3 domain. They renamed the protein LASP1, for 'LIM and SH3 protein.' Northern blot analysis revealed that LASP1 mRNA was expressed at a basal level in all normal tissues examined and overexpressed in 8% of primary breast cancers. In most of these cancers, LASP1 and ERBB2 were simultaneously overexpressed.

*MLLT6*

The MLLT6 (AF17) gene encodes a protein of 1,093 amino acids, containing a leucine-zipper dimerization motif located 3-prime of the fusion point and a cysteine-rich domain at the end terminus. AF17 was found to contain stretches of amino acids previously associated with domains involved in transcriptional repression or activation.

Chromosome translocations involving band 1 1q23 are associated with approximately 10% of patients with acute lymphoblastic leukemia (ALL) and more than 5% of patients with acute myeloid leukemia (AML). The gene at 11q23 involved in the translocations is variously designated ALL1, HRX, MLL, and TAX1. The partner gene in one of the rarer translocations, t(11;17)(q23;q21), designated MLLT6 on 17q12.

*ZNF144 (Mel18)*

Mel18 cDNA encodes a novel cys-rich zinc finger motif. The gene is expressed strongly in most tumor cell lines, but its normal tissue expression was limited to cells of neural origin and was especially abundant in fetal neural cells. It belongs to a RING-finger motif family which includes BMI1. The MEL18/BMI1 gene family represents a mammalian homolog of the Drosophila 'polycomb' gene group, thereby belonging to a memory mechanism involved in maintaining the the expression pattern of key regulatory factors such as Hox genes. Bmi1, Mel18 and M33 genes, as representative examples of mouse Pc-G genes. Common phenotypes observed in knockout mice mutant for each of these genes indicate an important role for Pc-G genes not only in regulation of Hox gene expression and axial skeleton development but also in control of proliferation and survival of haematopoietic cell lineages. This is in line with the observed proliferative deregulation observed in lymphoblastic leukemia. The MEL18 gene is conserved among vertebrates. Its mRNA is expressed at high levels in placenta, lung, and kidney, and at lower levels in liver, pancreas, and skeletal muscle. Interestingly, cervical and lumbo-sacral-HOX gene expression is altered in several primary breast cancers with respect to normal breast tissue with the HoxB gene cluster being present on 17q distal to the 17q12 locus. Moreover, delay of differentiation with persistent nests of proliferating cells was found in endothelial cells cocultured with HOXB7-transduced SkBr3 cells, which exhibit a 17q12 amplification. Tumorigenicity of these cells has been evaluated in vivo. Xenograft in athymic nude mice showed that SkBr3/HOXB7 cells developed tumors with an increased number of blood vessels, either irradiated or not, whereas parental SkBr3 cells did not show any tumor take unless mice were sublethally irradiated. As part of this invention, we have found MEL18 to be overexpressed specifically in tumors bearing Her-2/neu gene amplification, which can be critical for Hox expression.

*PHOSPHATIDYLINOSITOL-4-PHOSPHATE 5-KINASE, TYPE II, BETA; PIP5K2B*

Phosphoinositide kinases play central roles in signal transduction. Phosphatidylinositol-4-phosphate 5-kinases (PIP5Ks) phosphorylate phosphatidylinositol 4-phosphate, giving rise to phosphatidylinositol 4,5-bisphosphate. The PIP5K enzymes exist as multiple isoforms that have various immunoreactivities, kinetic properties, and molecular masses. They are unique in that they possess almost no homology to the kinase motifs present in other phosphatidylinositol, protein, and lipid kinases. By screening a human fetal brain cDNA library with the PIP5K2B EST the full length gene could be isolated. The deduced 416-amino acid protein is 78% identical to PIP5K2A. Using SDS-PAGE, the authors estimated that bacterially expressed PIP5K2B has a molecular mass of 47 kD. Northern blot analysis detected a 6.3-kb PIP5K2B transcript which was abundantly expressed in several human tissues. PIP5K2B interacts specifically with the juxtamembrane region of the p55 TNF receptor (TNFR1) and PIP5K2B activity is increased in mammalian cells by treatment with TNF-alpha. A modeled complex with membrane-bound substrate and ATP shows how a phosphoinositide kinase can phosphorylate its substrate in situ at the membrane interface.The substrate-binding site is open on 1 side, consistent with dual specificity for phosphatidylinositol 3- and 5-phosphates. Although the amino acid sequence of PIP5K2A does not show homology to known kinases, recombinant PIP5K2A exhibited kinase activity. PIP5K2A contains a putative Src homology 3 (SH3) domain-binding sequence. Overexpression of mouse PIP5K1B in COS7 cells induced an increase in short actin fibers and a decrease in actin stress fibers.

*TEM7*

Using serial analysis of gene expression (SAGE) a partial cDNAs corresponding to several tumor endothelial markers (TEMs) that displayed elevated expression during tumor angiogenesis could be identified. Among the genes identified was TEM7. Using database searches and 5-prime RACE the entire TEM7 coding region, which encodes a 500-amino acid type I transmembrane protein,has been described.. The extracellular region of TEM7 contains a plexin-like domain and has weak homology to the ECM protein nidogen. The function of these domains, which are usually found in secreted and extracellular matrix molecules, is unknown. Nidogen itself belongs to the entactin protein family and helps to determine pathways of migrating axons by switching from circumferential to longitudinal migration. Entactin is involved in cell migration, as it promotes trophoblast outgrowth through a mechanism mediated by the RGD recognition site, and plays an important role during invasion of the endometrial basement membrane at implantation. As entactin promotes thymocyte adhesion but affects thymocyte migration only marginally, it is suggested that entactin may plays a role in thymocyte localization during T cell development.

In situ hybridization analysis of human colorectal cancer demonstrated that TEM7 was expressed clearly in the endothelial cells of the tumor stroma but not in the endothelial cells of normal colonic tissue. Using in situ hybridization to assay expression in various normal adult mouse tissues, they observed that TEM7 was largely undetectable in mouse tissues or tumors, but was abundantly expressed in mouse brain.

*ZNFN1A3*

By screening a B-cell cDNA library with a mouse Aiolos N-terminal cDNA probe, a cDNA encoding human Aiolos, or ZNFN1A3, was obtained. The deduced 509-amino acid protein, which is 86% identical to its mouse counterpart, has 4 DNA-binding zinc fingers in its N terminus and 2 zinc fingers that mediate protein dimerization in its C terminus. These domains are 100% and 96% homologous to the corresponding domains in the mouse protein, respectively. Northern blot analysis revealed strong expression of a major 11.0- and a minor 4.4-kb ZNFN1A3 transcript in peripheral blood leukocytes, spleen, and thymus, with lower expression in liver, small intestine, and lung.

Ikaros (ZNFN1A1), a hemopoietic zinc finger DNA-binding protein, is a central regulator of lymphoid differentiation and is implicated in leukemogenesis. The execution of normal function of Ikaros requires sequence-specific DNA binding, transactivation, and dimerization domains. Mice with a mutation in a related zinc finger protein, Aiolos, are prone to B-cell lymphoma. In chemically induced murine lymphomas allelic losses on markers surrounding the Znfhial gene were detected in 27% of the tumors analyzed. Moreover specific Ikaros expression was in primary mouse hormone-producing anterior pituitary cells and substantial for Fibroblast growth factor receptor 4 (FGFR4) expression, which itself is implicated in a multitude of endocrine cell hormonal and proliferative properties with FGFR4 being differentially expressed in normal and neoplastic pituitary. Moreover Ikaros binds to chromatin remodelling complexes containing SWI/SNF proteins, which antagonize Polycomb function. Intetrestingly at the telomeric end of the disclosed ARCHEON the SWI/SNF complex member SMARCE1 (= SWI/SNF-related, matrix-associated, actin-dependent regulators of chromatin) is located and part of the described amplification. Due to the related binding specificities of Ikaros and Palindrom Binding Protein (PBP) it is suggestive, that ZNFN1A3 is able to regulate the Her-2/neu enhancer.

*PPP1R1B*

Midbrain dopaminergic neurons play a critical role in multiple brain functions, and abnormal signaling through dopaminergic pathways has been implicated in several major neurologic and psychiatric disorders. One well-studied target for the actions of dopamine is DARPP32. In the densely dopamine- and glutamate-innervated rat caudate-putamen, DARPP32 is expressed in medium-sized spiny neurons that also express dopamine D1 receptors. The function of DARPP32 seems to be regulated by receptor stimulation. Both dopaminergic and glutamatergic (NMDA) receptor stimulation regulate the extent of DARPP32 phosphorylation, but in opposite directions.

The human DARPP32 was isolated from a striatal cDNA library. The 204-amino acid DARPP32 protein shares 88% and 85% sequence identity, respectively, with bovine and rat DARPP32 proteins. The DARPP32 sequence is particularly conserved through the N terminus, which represents the active portion of the protein. Northern blot analysis demonstrated that the 2.1-kb DARPP32 mRNA is more highly expressed in human caudate than in cortex. In situ hybridization to postmortem human brain showed a low level of DARPP32 expression in all neocortical layers, with the strongest hybridization in the superficial layers. CDK5 phosphorylated DARPP32 in vitro and in intact brain cells. Phospho-thr75 DARPP32 inhibits PKA in vitro by a competitive mechanism. Decreasing phospho-thr75 DARPP32 in striatal cells either by a CDK5-specific inhibitor or by using genetically altered mice resulted in increased dopamine-induced phosphorylation of PKA substrates and augmented peak voltage-gated calcium currents. Thus, DARPP32 is a bifunctional signal transduction molecule which, by distinct mechanisms, controls a serine/threonine kinase and a serine/threonine phosphatase.

DARPP32 and t-DARPP are overexpressed in gastric cancers. It's suggested that overexpression of these 2 proteins in gastric cancers may provide an important survival advantage to neoplastic cells. It could be demonstrated that Darpp32 is an obligate intermediate in progesterone-facilitated sexual receptivity in female rats and mice. The facilitative effect of progesterone on sexual receptivity in female rats was blocked by antisense oligonucleotides to Darpp32. Homozygous mice carrying a null mutation for the Darpp32 gene exhibited minimal levels of progesterone-facilitated sexual receptivity when compared to their wildtype littermates, and progesterone significantly increased hypothalamic cAMP levels and cAMP-dependent protein kinase activity.

*CACNB1*

In 1991 a cDNA clone encoding a protein with high homology to the beta subunit of the rabbit skeletal muscle dihydropyridine-sensitive calcium channel from a rat brain cDNA library [Pragnell et al., 1991, (4)]. This rat brain beta-subunit cDNA hybridized to a 3.4-kb message that was expressed in high levels in the cerebral hemispheres and hippocampus and much lower levels in cerebellum. The open reading frame encodes 597 amino acids with a predicted mass of 65,679 Da which is 82% homologous with the skeletal muscle beta subunit. The corresponding human beta-subunit gene was localized to chromosome 17 by analysis of somatic cell hybrids. The authors suggested that the encoded brain beta subunit, which has a primary structure highly similar to its isoform in skeletal muscle, may have a comparable role as an integral regulatory component of a neuronal calcium channel.

*RPL19*

The ribosome is the only organelle conserved between prokaryotes and eukaryotes. In eukaryotes, this organelle consists of a 60S large subunit and a 40S small subunit. The mammalian ribosome contains 4 species of RNA and approximately 80 different ribosomal proteins, most of which appear to be present in equimolar amounts. In mammalian cells, ribosomal proteins can account for up to 15% of the total cellular protein, and the expression of the different ribosomal protein genes, which can account for up to 7 to 9% of the total cellular mRNAs, is coordinately regulated to meet the cell's varying requirements for protein synthesis. The mammalian ribosomal protein genes are members of multigene families, most of which are composed of multiple processed pseudogenes and a single functional intron-containing gene. The presence of multiple pseudogenes hampered the isolation and study of the functional ribosomal protein genes. By study of somatic cell hybrids, it has been elucidated that DNA sequences complementary to 6 mammalian ribosomal protein cDNAs could be assigned to chromosomes 5, 8, and 17. Ten fragments mapped to 3 chromosomes [Nakamichi et al., 1986, (5)]. These are probably a mixture of functional (expressed) genes and pseudogenes. One that maps to 5q23-q33 rescues Chinese hamster emetine-resistance mutations in interspecies hybrids and is therefore the transcriptionally active RPS14 gene. In 1989 a PCR-based strategy for the detection of intron-containing genes in the presence of multiple pseudogenes was described. This technique was used to identify the intron-containing PCR products of 7 human ribosomal protein genes and to map their chromosomal locations by hybridization to human/rodent somatic cell hybrids [Feo et al., 1992, (6)]. All 7 ribosomal protein genes were found to be on different chromosomes: RPL19 on 17pl2-qll;RPL30 on 8; RPL35A on 18; RPL36A on 14; RPS6 on 9pter-p13; RPS11 on 19cen-qter; and RPS17 on 11pter-p13. These are also different sites from the chromosomal location of previously mapped ribosomal protein genes S14 on chromosome 5, S4 on Xq and Yp, and RP117A on 9q3-q34. By fluorescence in situ hybridization the position of the RPL19 gene was mapped to 17q11 [Davies et al., 1989, (7)].

*PPARBP, PBP, CRSP1, CRSP200, TRIP2, TRAP220, RB18A, DRIP230*

The thyroid hormone receptors (TRs) are hormone-dependent transcription factors that regulate expression of a variety of specific target genes. They must specifically interact with a number of proteins as they progress from their initial translation and nuclear translocation to heterodimerization with retinoid X receptors (RXRs), functional interactions with other transcription factors and the basic transcriptional apparatus, and eventually, degradation. To help elucidate the mechanisms that underlie the transcriptional effects and other potential functions of TRs, the yeast interaction trap, a version of the yeast 2-hybrid system, was used to identify proteins that specifically interact with the ligand-binding domain of rat TR-beta-1 (THRB) [Lee et al., 1995, (8)]. The authors isolated HeLa cell cDNAs encoding several different TR-interacting proteins (TRIPs), including TRIP2. TRIP2 interacted with rat Thrb only in the presence of thyroid hormone. It showed a ligand-independent interaction with RXR-alpha, but did not interact with the glucocorticoid receptor (NR3C1) under any condition. By immunoscreening a human B-lymphoma cell cDNA expression library with the anti-p53 monoclonal antibody PAb1801, PPARBP was identified, which was called RB18A for 'recognized by PAb1801 monoclonal antibody' [Drane et al., 1997, (9)]. The predicted 1,566-amino acid RB 18A protein contains several potential nuclear localization signals, 13 potential N-glycosylation sites, and a high number of potential phosphorylation sites. Despite sharing common antigenic determinants with p53, RB18A does not show significant nucleotide or amino acid sequence similarity with p53. Whereas the calculated molecular mass of RB18A is 166 kD, the apparent mass of recombinant RB18A was 205 kD by SDS-PAGE analysis. The authors demonstrated that RB18A shares functional properties with p53, including DNA binding, p53 binding, and self-oligomerization. Furthermore, RB18A was able to activate the sequence-specific binding of p53 to DNA, which was induced through an unstable interaction between both proteins. Northern blot analysis of human tissues detected an 8.5-kb RB18A transcript in all tissues examined except kidney, with highest expression in heart. Moreover mouse Pparbp, which was called Pbp for 'Ppar-binding protein,' as a protein that interacts with the Ppar-gamma (PPARG) ligand-binding domain in a yeast 2-hybrid system was identified [Zhu et al., 1997, (10)]. The authors found that Pbp also binds to PPAR-alpha (PPARA), RAR-alpha (RARA), RXR, and TR-beta-1 in vitro. The binding of Pbp to these receptors increased in the presence of specific ligands. Deletion of the last 12 amino acids from the C terminus of PPAR-gamma resulted in the abolition of interaction between Pbp and PPAR-gamma. Pbp modestly increased the transcriptional activity of PPAR-gamma, and a truncated form of Pbp acted as a dominant-negative repressor, suggesting that Pbp is a genuine transcriptional co-activator for PPAR. The predicted 1,560-amino acid Pbp protein contains 2 LXXLL motifs, which are considered necessary and sufficient for the binding of several co-activators to nuclear receptors. Northern blot analysis detected Pbp expression in all mouse tissues examined, with higher levels in liver, kidney, lung, and testis. In situ hybridization showed that Pbp is expressed during mouse ontogeny, suggesting a possible role for Pbp in cellular proliferation and differentiation. In adult mouse, in situ hybridization detected Pbp expression in liver, bronchial epithelium in the lung, intestinal mucosa, kidney cortex, thymic cortex, splenic follicles, and seminiferous epithelium in testis. Lateron PPARBP was identified, which was called TRAP220, from an immunopurified TR-alpha (THRA)-TRAP complex [Yuan et al., 1998, (11)]. The authors cloned Jurkat cell cDNAs encoding TRAP220. The predicted 1,581-amino acid TRAP220 protein contains LXXLL domains, which are found in other nuclear receptor-interacting proteins. TRAP220 is nearly identical to RB18A , with these proteins differing primarily by an extended N terminus on TRAP220. In the absence of TR-alpha, TRAP220 appears to reside in a single complex with other TRAPs. TRAP220 showed a direct ligand-dependent interaction with TR-alpha, which was mediated through the C terminus of TR-alpha and, at least in part, the LXXLL domains of TRAP220. TRAP220 also interacted with other nuclear receptors, including vitamin D receptor, RARA, RXRA, PPARA, PPARG, and estrogen receptor-alpha (ESR1; 133430), in a ligand-dependent manner. TRAP220 moderately stimulated human TR-alpha-mediated transcription in transfected cells, whereas a fragment containing the LXXLL motifs acted as a dominant-negative inhibitor of nuclear receptor-mediated transcription both in transfected cells and in cell-free transcription systems. Further studies indicated that TRAP220 plays a major role in anchoring other TRAPs to TR-alpha during the function of the TR-alpha-TRAP complex and that TRAP220 may be a global co-activator for the nuclear receptor superfamily. PBP, a nuclear receptor co-activator, interacts with estrogen receptor-alpha (ESR1) in the absence of estrogen. This interaction was enhanced in the presence of estrogen, but was reduced in the presence of the anti-estrogen Tamoxifen. Transfection of PBP into cultured cells resulted in enhancement of estrogen-dependent transcription, indicating that PBP serves as a co-activator in estrogen receptor signaling. To examine whether overexpression of PBP plays a role in breast cancer because of its co-activator function in estrogen receptor signaling, the levels of PBP expression in breast tumors was determined [Zhu et al., 1999, (12)]. High levels of PBP expression were detected in approximately 50% of primary breast cancers and breast cancer cell lines by ribonuclease protection analysis, in situ hybridization, and immunoperoxidase staining. By using FISH, the authors mapped the PBP gene to 17q12, a region that is amplified in some breast cancers. They found PBP gene amplification in approximately 24% (6 of 25) of breast tumors and approximately 30% (2 of 6) of breast cancer cell lines, implying that PBP gene overexpression can occur independent of gene amplification. They determined that the PBP gene comprises 17 exons that together span more than 37 kb. Their findings, in particular PBP gene amplification, suggested that PBP, by its ability to function as an estrogen receptor-alpha co-activator, may play a role in mammary epithelial differentiation and in breast carcinogenesis.

*NEUROD2*

Basic helix-loop-helix (bHLH) proteins are transcription factors involved in determining cell type during development. In 1995 a bHLH protein was described, termed NeuroD (for 'neurogenic differentiation'), that functions during neurogenesis [Lee et al., 1995, (13)]. The human NEUROD gene maps to chromosome 2q32. The cloning and characterization of 2 additional NEUROD genes, NEUROD2 and NEUROD3 was described in 1996 [McCormick et al., 1996, (14)]. Sequences for the mouse and human homologues were presented. NEUROD2 shows a high degree of homology to the bHLH region of NEUROD, whereas NEUROD3 is more distantly related. The authors found that mouse neuroD2 was initially expressed at embryonic day 11, with persistent expression in the adult nervous system. Similar to neuroD, neuroD2 appears to mediate neuronal differentiation. The human NEUROD2 was mapped to 17q12 by fluorescence in situ hybridization and the mouse homologue to chromosome 11 [Tamimi et al., 1997, (15)].

*TELETHONIN*

Telethonin is a sarcomeric protein of 19 kD found exclusively in striated and cardiac muscle It appears to be localized to the Z disc of adult skeletal muscle and cultured myocytes. Telethonin is a substrate of titin, which acts as a molecular 'ruler' for the assembly of the sarcomere by providing spatially defined binding sites for other sarcomeric proteins. After activation by phosphorylation and calcium/calmodulin binding, titin phosphorylates the C-terminal domain of telethonin in early differentiating myocytes. The telethonin gene has been mapped to 17q12, adjacent to the phenylethanolamine N-methyltransferase gene [Valle et al., 1997, (16)].

*PENT, PNMT*

Phenylethanolamine N-methyltransferase catalyzes the synthesis of epinephrine from norepinephrine, the last step of catecholamine biosynthesis. The cDNA clone was first isolated in 1998 for bovine adrenal medulla PNMT using mixed oligodeoxyribonucleotide probes whose synthesis was based on the partial amino acid sequence of tryptic peptides from the bovine enzyme [Kaneda et al., 1988, (17)]. Using a bovine cDNA as a probe, the authors screened a human pheochromocytoma cDNA library and isolated a cDNA clone with an insert of about 1.0 kb, which contained a complete coding region of the enzyme. Northern blot analysis of human pheochromocytoma polyadenylated RNA using this cDNA insert as the probe demonstrated a single RNA species of about 1,000 nucleotides, suggesting that this clone is a full-length cDNA. The nucleotide sequence showed that human PNMT has 282 amino acid residues with a predicted molecular weight of 30,853, including the initial methionine. The amino acid sequence was 88% homologous to that of bovine enzyme. The PNMT gene was found to consist of 3 exons and 2 introns spanning about 2,100 basepairs. It was demonstrated that in transgenic mice the gene is expressed in adrenal medulla and retina. A hybrid gene consisting of 2 kb of the PNMT 5-prime-flanking region fused to the simian virus 40 early region also resulted in tumor antigen mRNA expression in adrenal glands and eyes; furthermore, immunocytochemistry showed that the tumor antigen was localized in nuclei of adrenal medullary cells and cells of the inner nuclear cell layer of the retina, both prominent sites of epinephrine synthesis. The results indicate that the enhancer(s) for appropriate expression of the gene in these cell types are in the 2-kb 5-prime-flanking region of the gene.

Kaneda et al., 1988 (17), assigned the human PNMT gene to chromosome 17 by Southern blot analysis of DNA from mouse-human somatic cell hybrids. In 1992 the localization was narrowed down to 17q21-q22 by linkage analysis using RFLPs related to the PNMT gene and several 17q DNA markers [Hoehe et al., 1992, (18)]. The findings are of interest in light of the description of a genetic locus associated with blood pressure regulation in the stroke-prone spontaneously hypertensive rat (SHR-SP) on rat chromosome 10 in a conserved linkage synteny group corresponding to human chromosome 17q22-q24. See essential hypertension .

*MGC9753*

This gene maps on chromosome 17, at 17q12 according to RefSeq. It is expressed at very high level. It is defined by cDNA clones and produces, by alternative splicing, 7 different transcripts can be obtained (SEQ ID NO:60 to 66 and 83 to 89 ,Table 1), altogether encoding 7 different protein isoforms. Of specific interest is the putatively secreted isoform g, encoded by a mRNA of 2.55 kb. It's premessenger covers 16.94 kb on the genome. It has a very long 3' UTR. . The protein (226 aa, MW 24.6 kDa, pI 8.5) contains no Pfam motif. The MGC9753 gene produces, by alternative splicing, 7 types of transcripts, predicted to encode 7 distinct proteins. It contains 13 confirmed introns, 10 of which are alternative. Comparison to the genome sequence shows that 11 introns follow the consensual [gt-ag] rule, 1 is atypical with good support [tg_cg]. The six most abundant isoforms are designated by a) to i) and code for proteins as follows:

a) This mRNA is 3.03 kb long, its premessenger covers 16.95 kb on the genome. It has a very long 3' UTR. The protein (190 aa, MW 21.5 kDa, pI 7.2) contains no Pfam motif. It is predicted to localise in the endoplasmic reticulum.

c) This mRNA is 1.17 kb long, its premessenger covers 16.93 kb on the genome. It may be incomplete at the N terminus. The protein (368 aa, MW 41.5 kDa, pI 7.3) contains no Pfam motif.

d) This mRNA is 3.17 kb long, its premessenger covers 16.94 kb on the genome. It has a very long 3' UTR and 5'p UTR. . The protein (190 aa, MW 21.5 kDa, pI 7.2) contains no Pfam motif. It is predicted to localise in the endoplasmic reticulum.

g) This mRNA is 2.55 kb long, its premessenger covers 16.94 kb on the genome. It has a very long 3' UTR. . The protein (226 aa, MW 24.6 kDa, pI 8.5) contains no Pfam motif. It is predicted to be secreted.

h) This mRNA is 2.68 kb long, its premessenger covers 16.94 kb on the genome. It has a very long 3' UTR. . The protein (320 aa, MW 36.5 kDa, pI 6.8) contains no Pfam motif. It is predicted to localise in the endoplasmic reticulum.

i) This mRNA is 2.34 kb long, its premessenger covers 16.94 kb on the genome. It may be incomplete at the N terminus. It has a very long 3' UTR. . The protein (217 aa, MW 24.4 kDa, pI 5.9) contains no Pfam motif.

The MCG9753 gene may be homologue to the CAB2 gene located on chromosome 17q12. The CAB2, a human homologue of the yeast COS16 required for the repair of DNA double-strand breaks was cloned. Autofluorescence analysis of cells transfected with its GFP fusion protein demonstrated that CAB2 translocates into vesicles, suggesting that overexpression of CAB2 may decrease intercellular Mn-(2+) by accumulating it in the vesicles, in the same way as yeast.

*Her-2/neu, ERBB2, NGL, TKR1*

The oncogene originally called NEU was derived from rat neuro/glioblastoma cell lines. It encodes a tumor antigen, p185, which is serologically related to EGFR, the epidermal growth factor receptor. EGFR maps to chromosome 7. In1985 it was found, that the human homologue, which they designated NGL (to avoid confusion with neuraminidase, which is also symbolized NEU), maps to 17q12-q22 by in situ hybridization and to 17q21-qter in somatic cell hybrids [Yang-Feng et al., 1985, (19)]. Thus, the SRO is 17q21-q22. Moreover, in 1985 a potential cell surface receptor of the tyrosine kinase gene family was identified and characterized by cloning the gene [Coussens et al., 1985, (20)]. Its primary sequence is very similar to that of the human epidermal growth factor receptor. Because of the seemingly close relationship to the human EGF receptor, the authors called the gene HER2. By Southern blot analysis of somatic cell hybrid DNA and by in situ hybridization, the gene was assigned to 17q21-q22. This chromosomal location of the gene is coincident with the NEU oncogene, which suggests that the 2 genes may in fact be the same; indeed, sequencing indicates that they are identical. In1988 a correlation between overexpression of NEU protein and the large-cell, comedo growth type of ductal carcinoma was found [van de Vijver et al., 1988, (21)]. The authors found no correlation, however, with lymph-node status or tumor recurrence. The role of HER2/NEU in breast and ovarian cancer was described in 1989, which together account for one-third of all cancers in women and approximately one-quarter of cancer-related deaths in females [Slamon et al., 1989, (22)].

An ERBB-related gene that is distinct from the ERBB gene, called ERBB1 was found in 1985. ERBB2 was not amplified in vulva carcinoma cells with EGFR amplification and did not react with EGF receptor mRNA. About 30-fold amplification of ERBB2 was observed in a human adenocarcinoma of the salivary gland. By chromosome sorting combined with velocity sedimentation and Southern hybridization, the ERBB2 gene was assigned to chromosome 17 [Fukushige et al., 1986, (23)]. By hybridization to sorted chromosomes and to metaphase spreads with a genomic probe, they mapped the ERBB2 locus to 17q21. This is the chromosome 17 breakpoint in acute promyelocytic leukemia (APL). Furthermore, they observed amplification and elevated expression of the ERBB2 gene in a gastric cancer cell line. Antibodies against a synthetic peptide corresponding to 14 amino acid residues at the COOH-terminus of a protein deduced from the ERBB2 nucleotide sequence were raised in 1986. With these antibodies, the ERBB2 gene product from adenocarcinoma cells was precipitated and demonstrated to be a 185-kD glycoprotein with tyrosine kinase activity. A cDNA probe for ERBB2 and by in situ hybridization to APL cells with a 15;17 chromosome translocation located the gene to the proximal side of the breakpoint [Kaneko et al., 1987, (24)]. The authors suggested that both the gene and the breakpoint are located in band 17q21.1 and, further, that the ERBB2 gene is involved in the development of leukemia. In 1987 experiments indicated that NEU and HER2 are both the same as ERBB2 [Di Fiore et al., 1987, (25)]. The authors demonstrated that overexpression alone can convert the gene for a normal growth factor receptor, namely, ERBB2, into an oncogene. The ERBB2 to 17q11-q21 by in situ hybridization [Popescu et al., 1989, (26)]. By in situ hybridization to chromosomes derived from fibroblasts carrying a constitutional translocation between 15 and 17, they showed that the ERBB2 gene was relocated to the derivative chromosome 15; the gene can thus be localized to 17q12-q21.32. By family linkage studies using multiple DNA markers in the 17q12-q21 region the ERBB2 gene was placed on the genetic map of the region.

Interleukin-6 is a cytokine that was initially recognized as a regulator of immune and inflammatory responses, but also regulates the growth of many tumor cells, including prostate cancer. Overexpression of ERBB2 and ERBB3 has been implicated in the neoplastic transformation of prostate cancer. Treatment of a prostate cancer cell line with IL6 induced tyrosine phosphorylation of ERBB2 and ERBB3, but not ERBB1/EGFR. The ERBB2 forms a complex with the gp130 subunit of the IL6 receptor in an IL6-dependent manner. This association was important because the inhibition of ERBB2 activity resulted in abrogation of IL6-induced MAPK activation. Thus, ERBB2 is a critical component of IL6 signaling through the MAP kinase pathway [Qiu et al., 1998, (27)]. These findings showed how a cytokine receptor can diversify its signaling pathways by engaging with a growth factor receptor kinase.

Overexpression of ERBB2 confers Taxol resistance in breast cancers. Overexpression of ERBB2 inhibits Taxol-induced apoptosis [Yu et al., 1998, (28)]. Taxol activates CDC2 kinase in MDA-MB-435 breast cancer cells, leading to cell cycle arrest at the G2/M phase and, subsequently, apoptosis. A chemical inhibitor of CDC2 and a dominant-negative mutant of CDC2 blocked Taxol-induced apoptosis in these cells. Overexpression of ERBB2 in MDA-MB-435 cells by transfection transcriptionally upregulates CDKN1A which associates with CDC2, inhibits Taxol-mediated CDC2 activation, delays cell entrance to G2/M phase, and thereby inhibits Taxol-induced apoptosis. In CDKN1A antisense-transfected MDA-MB-435 cells or in p21-/- MEF cells, ERBB2 was unable to inhibit Taxol-induced apoptosis. Therefore, CDKN1A participates in the regulation of a G2/M checkpoint that contributes to resistance to Taxol-induced apoptosis in ERBB2-overexpressing breast cancer cells.

A secreted protein of approximately 68 kD was described, designated herstatin, as the product of an alternative ERBB2 transcript that retains intron 8 [Doherty et al., 1999, (29)]. This alternative transcript specifies 340 residues identical to subdomains I and II from the extracellular domain of p185ERBB2, followed by a unique C-terminal sequence of 79 amino acids encoded by intron 8. The recombinant product of the alternative transcript specifically bound to ERBB2-transfected cells and was chemically crosslinked to p185ERBB2, whereas the intron-encoded sequence alone also bound with high affinity to transfected cells and associated with p185 solubilized from cell extracts. The herstatin mRNA was expressed in normal human fetal kidney and liver, but was at reduced levels relative to p185ERBB2 mRNA in carcinoma cells that contained an amplified ERBB2 gene. Herstatin appears to be an inhibitor of p185ERBB2, because it disrupts dimers, reduces tyrosine phosphorylation of p185, and inhibits the anchorage-independent growth of transformed cells that overexpress ERBB2. The HER2 gene is amplified and HER2 is overexpressed in 25 to 30% of breast cancers, increasing the aggressiveness of the tumor. Finally, it was found that a recombinant monoclonal antibody against HER2 increased the clinical benefit of first-line chemotherapy in metastatic breast cancer that overexpresses HER2 [Slamon et al., 2001, (30)].

*GRB7*

Growth factor receptor tyrosine kinases (GF-RTKs) are involved in activating the cell cycle. Several substrates of GF-RTKs contain Src-homology 2 (SH2) and SH3 domains. SH2 domain-containing proteins are a diverse group of molecules important in tyrosine kinase signaling. Using the CORT (cloning of receptor targets) method to screen a high expression mouse library, the gene for murine Grb7, which encodes a protein of 535 amino acids, was isolated [Margolis et al., 1992, (31)]. GRB7 is homologous to ras-GAP (ras-GTPase-activating protein). It contains an SH2 domain and is highly expressed in liver and kidney. This gene defines the GRB7 family, whose members include the mouse gene Grb 10 and the human gene GRB14.

A putative GRB7 signal transduction molecule and a GRB7V novel splice variant from an invasive human esophageal carcinoma was isolated [Tanaka et al., 1998, (32)]. Although both GRB7 isoforms shared homology with the Mig-10 cell migration gene of Caenorhabditis elegans, the GRB7V isoform lacked 88 basepairs in the C terminus; the resultant frameshift led to substitution of an SH2 domain with a short hydrophobic sequence. The wildtype GRB7 protein, but not the GRB7V isoform, was rapidly tyrosyl phosphorylated in response to EGF stimulation in esophageal carcinoma cells. Analysis of human esophageal tumor tissues and regional lymph nodes with metastases revealed that GRB7V was expressed in 40% of GRB7-positive esophageal carcinomas. GRB7V expression was enhanced after metastatic spread to lymph nodes as compared to the original tumor tissues. Transfection of an antisense GRB7 RNA expression construct lowered endogenous GRB7 protein levels and suppressed the invasive phenotype exhibited by esophageal carcinoma cells. These findings suggested that GRB7 isoforms are involved in cell invasion and metastatic progression of human esophageal carcinomas. By sequence analysis, The GRB7 gene was mapped to chromosome 17q21-q22, near the topoisomerase-2 gene [Dong et al., 1997, (33)]. GRB-7 is amplified in concert with HER2 in several breast cancer cell lines and that GRB-7 is overexpressed in both cell lines and breast tumors. GRB-7, through its SH2 domain, binds tightly to HER2 such that a large fraction of the tyrosine phosphorylated HER2 in SKBR-3 cells is bound to GRB-7 [Stein et al., 1994, (34)].

*GCSF, CSF3*

Granulocyte colony-stimulating factor (or colony stimulating factor-3) specifically stimulates the proliferation and differentiation of the progenitor cells for granulocytes. The partial amino acid sequence of purified GCSF protein was determined, and by using oligonucleotides as probes, several GCSF cDNA clones were isolated from a human squamous carcinoma cell line cDNA library [Nagata et al., 1986, (35)]. Cloning of human GCSF cDNA shows that a single gene codes for a 177- or 180-amino acid mature protein of molecular weight 19,600. The authors found that the GCSF gene has 4 introns and that 2 different polypeptides are synthesized from the same gene by differential splicing of mRNA. The 2 polypeptides differ by the presence or absence of 3 amino acids. Expression studies indicate that both have authentic GCSF activity. A stimulatory activity from a glioblastoma multiform cell line being biologically and biochemically indistinguishable from GCSF produced by a bladder cell line was found in 1987. By somatic cell hybridization and in situ chromosomal hybridization, the GCSF gene was mapped to 17q11 in the region of the breakpoint in the 15;17 translocation characteristic of acute promyelocytic leukemia [Le Beau et al., 1987, (36)]. Further studies indicated that the gene is proximal to the said breakpoint and that it remains on the rearranged chromosome 17. Southern blot analysis using both conventional and pulsed field gel electrophoresis showed no rearranged restriction fragments. By use of a full-length cDNA clone as a hybridization probe in human-mouse somatic cell hybrids and in flow-sorted human chromosomes, the gene for GCSF was mapped to 17q21-q22 lateron

*THRA, THRA1, ERBA, EAR7, ERBA2, ERBA3*

Both human and mouse DNA have been demonstrated to have two distantly related classes of ERBA genes and that in the human genome multiple copies of one of the classes exist [Jansson et al., 1983, (37)]. A cDNA was isolated derived from rat brain messenger RNA on the basis of homology to the human thyroid receptor gene [Thompson et al., 1987, (38)]. Expression of this cDNA produced a high-affinity binding protein for thyroid hormones. Messenger RNA from this gene was expressed in tissue-specific fashion, with highest levels in the central nervous system and no expression in the liver. An increasing body of evidence indicated the presence of multiple thyroid hormone receptors. The authors suggested that there may be as many as 5 different but related loci. Many of the clinical and physiologic studies suggested the existence of multiple receptors. For example, patients had been identified with familial thyroid hormone resistance in which peripheral response to thyroid hormones is lost or diminished while neuronal functions are maintained. Thyroidologists recognize a form of cretinism in which the nervous system is severely affected and another form in which the peripheral functions of thyroid hormone are more dramatically affected.

The cDNA encoding a specific form of thyroid hormone receptor expressed in human liver, kidney, placenta, and brain was isolated [Nakai et al., 1988, (39)]. Identical clones were found in human placenta. The cDNA encodes a protein of 490 amino acids and molecular mass of 54,824. Designated thyroid hormone receptor type alpha-2 (THRA2), this protein is represented by mRNAs of different size in liver and kidney, which may represent tissue-specific processing of the primary transcript.

The THRA gene contains 10 exons spanning 27 kb of DNA. The last 2 exons of the gene are alternatively spliced. A 5-kb THRA1 mRNA encodes a predicted 410-amino acid protein; a 2.7-kb THRA2 mRNA encodes a 490-amino acid protein. A third isoform, TR-alpha-3, is derived by alternative splicing. The proximal 39 amino acids of the TH-alpha-2 specific sequences are deleted in TR-alpha-3. A second gene, THRB on chromosome 3, encodes 2 isoforms of TR-beta by alternative splicing. In 1989 the structure and function of the EAR1 and EAR7 genes was elucidated, both located on 17q21 [Miyajima et al., 1989, (40)]. The authors determined that one of the exons in the EAR7 coding sequence overlaps an exon of EAR1, and that the 2 genes are transcribed from opposite DNA strands. In addition, the EAR7 mRNA generates 2 alternatively spliced isoforms, referred to as EAR71 and EAR72, of which the EAR71 protein is the human counterpart of the chicken c-erbA protein.

The thyroid hormone receptors, beta, alpha-1, and alpha-2 3 mRNAs are expressed in all tissues examined and the relative amounts of the three mRNAs were roughly parallel. None of the 3 mRNAs was abundant in liver, which is the major thyroid hormone-responsive organ. This led to the assumption that another thyroid hormone receptor may be present in liver. It was found that ERBA, which potentiates ERBB, has an amino acid sequence different from that of other known oncogene products and related to those of the carbonic anhydrases [Debuire et al., 1984, (41)]. ERBA potentiates ERBB by blocking differentiation of erythroblasts at an immature stage. Carbonic anhydrases participate in the transport of carbon dioxide in erythrocytes. In 1986 it was shown that the ERBA protein is a high-affinity receptor for thyroid hormone. The cDNA sequence indicates a relationship to steroid-hormone receptors, and binding studies indicate that it is a receptor for thyroid hormones. It is located in the nucleus, where it binds to DNA and activates transcription.

Maternal thyroid hormone is transferred to the fetus early in pregnancy and is postulated to regulate brain development. The ontogeny of TR isoforms and related splice variants in 9 first-trimester fetal brains by semi-quantitative RT-PCR analysis has been investigated. Expression of the TR-beta-1, TR-alpha-1, and TR-alpha-2 isoforms was detected from 8.1 weeks' gestation. An additional truncated species was detected with the TR-alpha-2 primer set, consistent with the TR-alpha-3 splice variant described in the rat. All TR-alpha-derived transcripts were coordinately expressed and increased approximately 8-fold between 8.1 and 13.9 weeks' gestation. A more complex ontogenic pattern was observed for TR-beta-1, suggestive of a nadir between 8.4 and 12.0 weeks' gestation. The authors concluded that these findings point to an important role for the TR-alpha-1 isoform in mediating maternal thyroid hormone action during first-trimester fetal brain development.

The identification of the several types of thyroid hormone receptor may explain the normal variation in thyroid hormone responsiveness of various organs and the selective tissue abnormalities found in the thyroid hormone resistance syndromes. Members of sibships, who were resistant to thyroid hormone action, had retarded growth, congenital deafness, and abnormal bones, but had normal intellect and sexual maturation, as well as augmented cardiovascular activity. In this family abnormal T3 nuclear receptors in blood cells and fibroblasts have been demonstrated. The availability of cDNAs encoding the various thyroid hormone receptors was considered useful in determining the underlying genetic defect in this family.

The ERBA oncogene has been assigned to chromosome 17. The ERBA locus remains on chromosome 17 in the t(15;17) translocation of acute promyelocytic leukemia (APL). The thymidine kinase locus is probably translocated to chromosome 15; study of leukemia with t(17;21) and apparently identical breakpoint showed that TK was on 21q+. By in situ hybridization of a cloned DNA probe of c-erb-A to meiotic pachytene spreads obtained from uncultured spermatocytes it has been concluded that ERBA is situated at 17q21.33-17q22, in the same region as the break that generated the t(15;17) seen in APL. Because most of the grains were seen in 17q22, they suggested that ERBA is probably in the proximal region of 17q22 or at the junction between 17q22 and 17q21.33. By in situ hybridization it has been demonstrated, that that ERBA remains at 17q11-q12 in APL, whereas TP53, at 17q21-q22, is translocated to chromosome 15. Thus, ERBA must be at 17q11.2 just proximal to the breakpoint in the APL translocation and just distal to it in the constitutional translocation.

The aberrant THRA expression in nonfunctioning pituitary tumors has been hypothesized to reflect mutations in the receptor coding and regulatory sequences. They screened THRA mRNA and THRB response elements and ligand-binding domains for sequence anomalies. Screening THRA mRNA from 23 tumors by RNAse mismatch and sequencing candidate fragments identified 1 silent and 3 missense mutations, 2 in the common THRA region and 1 that was specific for the alpha-2 isoform. No THRB response element differences were detected in 14 nonfunctioning tumors, and no THRB ligand-binding domain differences were detected in 23 nonfunctioning tumors. Therefore it has been suggested that the novel thyroid receptor mutations may be of functional significance in terms of thyroid receptor action, and further definition of their functional properties may provide insight into the role of thyroid receptors in growth control in pituitary cells.

*RAR-alpha*

A cDNA encoding a protein that binds retinoic acid with high affinity has been cloned [Petkovich et al., 1987, (42)]. The protein was found to be homologous to the receptors for steroid hormones, thyroid hormones, and vitamin D3, and appeared to be a retinoic acid-inducible transacting enhancer factor. Thus, the molecular mechanisms of the effect of vitamin A on embryonic development, differentiation and tumor cell growth may be similar to those described for other members of this nuclear receptor family. In general, the DNA-binding domain is most highly conserved, both within and between the 2 groups of receptors (steroid and thyroid); Using a cDNA probe, the RAR-alpha gene has been mapped to 17q21 by in situ hybridization [Mattei et al., 1988, (43)]. Evidence has been presented for the existence of 2 retinoic acid receptors, RAR-alpha and RAR-beta, mapping to chromosome 17q21.1 and 3p24, respectively. The alpha and beta forms of RAR were found to be more homologous to the 2 closely related thyroid hormone receptors alpha and beta, located on 17q11.2 and 3p25-p21, respectively, than to any other members of the nuclear receptor family. These observations suggest that the thyroid hormone and retinoic acid receptors evolved by gene, and possibly chromosome, duplications from a common ancestor, which itself diverged rather early in evolution from the common ancestor of the steroid receptor group of the family. They noted that the counterparts of the human RARA and RARB genes are present in both the mouse and chicken. The involvement of RARA at the APL breakpoint may explain why the use of retinoic acid as a therapeutic differentiation agent in the treatment of acute myeloid leukemias is limited to APL. Almost all patients with APL have a chromosomal translocation t(15;17)(q22;q21). Molecular studies reveal that the translocation results in a chimeric gene through fusion between the PML gene on chromosome 15 and the RARA gene on chromosome 17. A hormone-dependent interaction of the nuclear receptors RARA and RXRA with CLOCK and MOP4 has been presented.

*CDC18 L, CDC 6*

In yeasts, Cdc6 (Saccharomyces cerevisiae) and Cdc18 (Schizosaccharomyces pombe) associate with the origin recognition complex (ORC) proteins to render cells competent for DNA replication. Thus, Cdc6 has a critical regulatory role in the initiation of DNA replication in yeast. cDNAs encoding Xenopus and human homologues of yeast CDC6 have been isolated [Williams et al., 1997, (44)]. They designated the human and Xenopus proteins p62(cdc6). Independently, in a yeast 2-hybrid assay using PCNA as bait, cDNAs encoding the human CDC6/Cdc 18 homologue have been isolated [Saha et al, 1998, (45)]. These authors reported that the predicted 560-amino acid human protein shares approximately 33% sequence identity with the 2 yeast proteins. On Western blots of HeLa cell extracts, human CDC6/Cdc18 migrates as a 66-kD protein. Although Northern blots indicated that CDC6/Cdc18 mRNA levels peak at the onset of S phase and diminish at the onset of mitosis in HeLa cells, the authors found that total CDC6/Cdc18 protein level is unchanged throughout the cell cycle. Immunofluorescent analysis of epitope-tagged protein revealed that human CDC6/Cdc18 is nuclear in G1 and cytoplasmic in S-phase cells, suggesting that DNA replication may be regulated by either the translocation of this protein between the nucleus and cytoplasm or by selective degradation of the protein in the nucleus. Immunoprecipitation studies showed that human CDC6/Cdc18 associates in vivo with cyclin A, CDK2,and ORC1. The association of cyclin-CDK2 with CDC6/Cdc18 was specifically inhibited by a factor present in mitotic cell extracts. Therefore it has been suggested that if the interaction between CDC6/Cdc18 with the S phase-promoting factor cyclin-CDK2 is essential for the initiation of DNA replication, the mitotic inhibitor of this interaction could prevent a premature interaction until the appropriate time in G1 Cdc6 is expressed selectively in proliferating but not quiescent mammalian cells, both in culture and within tissues in intact animals [Yan et al., 1998, (46)]. During the transition from a growth-arrested to a proliferative state, transcription of mammalian Cdc6 is regulated by E2F proteins, as revealed by a functional analysis of the human Cdc6 promoter and by the ability of exogenously expressed E2F proteins to stimulate the endogenous Cdc6 gene. Immunodepletion of Cdc6 by microinjection of anti-Cdc6 antibody blocked initiation of DNA replication in a human tumor cell line. The authors concluded that expression of human Cdc6 is regulated in response to mitogenic signals through transcriptional control mechanisms involving E2F proteins, and that Cdc6 is required for initiation of DNA replication in mammalian cells.

Using a yeast 2-hybrid system, co-purification of recombinant proteins, and immunoprecipitation, it has been demonstrated lateron that an N-terminal segment of CDC6 binds specifically to PR48, a regulatory subunit of protein phosphatase 2A (PP2A). The authors hypothesized that dephosphorylation of CDC6 by PP2A, mediated by a specific interaction with PR48 or a related B-double prime protein, is a regulatory event controlling initiation of DNA replication in mammalian cells. By analysis of somatic cell hybrids and by fluorescence in situ hybridization the human p62(cdc6) gene has been to 17q21.3.

*TOP2A, TOP2*

DNA topoisomerases are enzymes that control and alter the topologic states of DNA in both prokaryotes and eukaryotes. Topoisomerase II from eukaryotic cells catalyzes the relaxation of supercoiled DNA molecules, catenation, decatenation, knotting, and unknotting of circular DNA. It appears likely that the reaction catalyzed by topoisomerase II involves the crossing-over of 2 DNA segments. It has been estimated that there are about 100,000 molecules of topoisomerase II per HeLa cell nucleus, constituting about 0.1% of the nuclear extract. Since several of the abnormal characteristics of ataxia-telangiectasia appear to be due to defects in DNA processing, screening for these enzyme activities in 5 AT cell lines has been performed [Singh et al., 1988, (47)]. In comparison to controls, the level of DNA topoisomerase II, determined by unknotting of P4 phage DNA, was reduced substantially in 4 of these cell lines and to a lesser extent in the fifth. DNA topoisomerase I, assayed by relaxation of supercoil DNA, was found to be present at normal levels.

The entire coding sequence of the human TOP2 gene has been determined [Tsai-Pflugfelder et al., 1988, (48)].

In addition human cDNAs that had been isolated by screening a cDNA library derived from a mechlorethamine-resistant Burkitt lymphoma cell line (Raji-HN2) with a Drosophila Topo II cDNA had been sequenced [Chung et al., 1989, (49)]. The authors identified 2 classes of sequence representing 2 TOP2 isoenzymes, which have been named TOP2A and TOP2B. The sequence of 1 of the TOP2A cDNAs is identical to that of an internal fragment of the TOP2 cDNA isolated by Tsai-Pflugfelder et al., 1988 (48). Southern blot analysis indicated that the TOP2A and TOP2B cDNAs are derived from distinct genes. Northern blot analysis using a TOP2A-specific probe detected a 6.5-kb transcript in the human cell line U937. Antibodies against a TOP2A peptide recognized a 170-kD protein in U937 cell lysates. Therefore it was concluded that their data provide genetic and immunochemical evidence for 2 TOP2 isozymes. The complete structures of the TOP2A and TOP2B genes has been reported [Lang et al., 1998, (50)]. The TOP2A gene spans approximately 30 kb and contains 35 exons.

Tsai-Pflugfelder et al., 1988 (48) showed that the human enzyme is encoded by a single-copy gene which they mapped to 17q21-q22 by a combination of in situ hybridization of a cloned fragment to metaphase chromosomes and by Southern hybridization analysis with a panel of mouse-human hybrid cell lines. The assignment to chromosome 17 has been confirmed by the study of somatic cell hybrids. Because of co-amplification in an adenocarcinoma cell line, it was concluded that the TOP2A and ERBB2 genes may be closely linked on chromosome 17 [Keith et al., 1992, (51)]. Using probes that detected RFLPs at both the TOP2A and TOP2B loci, the demonstrated heterozygosity at a frequency of 0.17 and 0.37 for the alpha and beta loci, respectively. The mouse homologue was mapped to chromosome 11 [Kingsmore et al., 1993, (52)]. The structure and function of type II DNA topoisomerases has been reviewed [Watt et al., 1994, (53)]. DNA topoisomerase II-alpha is associated with the pol II holoenzyme and is a required component of chromatin-dependent co-activation. Specific inhibitors of topoisomerase II blocked transcription on chromatin templates, but did not affect transcription on naked templates. Addition of purified topoisomerase II-alpha reconstituted chromatin-dependent activation activity in reactions with core pol II. Therefore the transcription on chromatin templates seems to result in the accumulation of superhelical tension, making the relaxation activity of topoisomerase II essential for productive RNA synthesis on nucleosomal DNA.

*IGFBP4*

Six structurally distinct insulin-like growth factor binding proteins have been isolated and their cDNAs cloned: IGFBP1, IGFBP2, IGFBP3, IGFBP4, IGFBP5 and IGFBP6. The proteins display strong sequence homologies, suggesting that they are encoded by a closely related family of genes. The IGFBPs contain 3 structurally distinct domains each comprising approximately one-third of the molecule. The N-terminal domain 1 and the C-terminal domain 3 of the 6 human IGFBPs show moderate to high levels of sequence identity including 12 and 6 invariant cysteine residues in domains 1 and 3, respectively (IGFBP6 contains 10 cysteine residues in domain 1), and are thought to be the IGF binding domains. Domain 2 is defined primarily by a lack of sequence identity among the 6 IGFBPs and by a lack of cysteine residues, though it does contain 2 cysteines in IGFBP4. Domain 3 is homologous to the thyroglobulin type I repeat unit. Recombinant human insulin-like growth factor binding proteins 4, 5, and 6 have been characterized by their expression in yeast as fusion proteins with ubiquitin [Kiefer et al., 1992, (54)]. Results of the study suggested to the authors that the primary effect of the 3 proteins is the attenuation of IGF activity and suggested that they contribute to the control of IGF-mediated cell growth and metabolism.

Based on peptide sequences of a purified insulin-like growth factor-binding protein (IGFBP) rat IGFBP4 has been cloned by using PCR [Shimasaki et al., 1990, (55)]. They used the rat cDNA to clone the human ortholog from a liver cDNA library. Human IGFBP4 encodes a 258-amino acid polypeptide, which includes a 21-amino acid signal sequence. The protein is very hydrophilic, which may facilitate its ability as a carrier protein for the IGFs in blood. Northern blot analysis of rat tissues revealed expression in all tissues examined, with highest expression in liver. It was stated that IGFBP4 acts as an inhibitor of IGF-induced bone cell proliferation. The genomic region containing the IGFBP gene. The gene consists of 4 exons spanning approximately 15 kb of genomic DNA has been examined [Zazzi et al., 1998, (56)]. The upstream region of the gene contains a TATA box and a cAMP-responsive promoter.

By in situ hybridization, the IGFBP4 gene was mapped to 17q12-q21 [Bajalica et al., 1992, (57)]. Because the hereditary breast-ovarian cancer gene BRCA1 had been mapped to the same region, it has been investigated whether IGFBP4 is a candidate gene by linkage analysis of 22 BRCA1 families; the finding of genetic recombination suggested that it is not the BRCA1 gene [Tonin et al., 1993, (58)].

*EBI 1, CCR7, CMKBR7*

Using PCR with degenerate oligonucleotides, a lymphoid-specific member of the G protein-coupled receptor family has been identified and mapped mapped to 17q12-q21.2 by analysis of human/mouse somatic cell hybrid DNAs and fluorescence in situ hybridization. It has been shown that this receptor had been independently identified as the Epstein-Barr-induced cDNA (symbol EBI1) [Birkenbach et al., 1993, (59)]. EBI1 is expressed in normal lymphoid tissues and in several B- and T-lymphocyte cell lines. While the function and the ligand for EBI1 remains unknown, its sequence and gene structure suggest that it is related to receptors that recognize chemoattractants, such as interleukin-8, RANTES, C5a, and fMet-Leu-Phe. Like the chemoattractant receptors, EBI1 contains intervening sequences near its 5-prime end; however, EBI1 is unique in that both of its introns interrupt the coding region of the first extracellular domain. Mouse Ebi1 cDNA has been isolated and found to encode a protein with 86% identity to the human homologue.

Subsets of murine CD4+ T cells localize to different areas of the spleen after adoptive transfer. Naive and T helper-1 (TH1) cells, which express CCR7, home to the periarteriolar lymphoid sheath, whereas activated TH2 cells, which lack CCR7, form rings at the periphery of the T-cell zones near B-cell follicles. It has been found that retroviral transduction of TH2 cells with CCR7 forced them to localize in a TH1-like pattern and inhibited their participation in B-cell help in vivo but not in vitro. Apparently differential expression of chemokine receptors results in unique cellular migration patterns that are important for effective immune responses.

CCR7 expression divides human memory T cells into 2 functionally distinct subsets. CCR7-memory cells express receptors for migration to inflamed tissues and display immediate effector function. In contrast, $CCR7^+$ memory cells express lymph node homing receptors and lack immediate effector function, but efficiently stimulate dendritic cells and differentiate into $CCR7^-$ effector cells upon secondary stimulation. The $CCR7^+$ and $CCR7^-$ T cells, named central memory (T-CM) and effector memory (T-EM), differentiate in a step-wise fashion from naive T cells, persist for years after immunization, and allow a division of labor in the memory response.

CCR7 expression in memory $CD8^+$ T lymphocyte responses to HIV and to cytomegalovirus (CMV) tetramers has been evaluated. Most memory T lymphocytes express CD45RO, but a fraction express instead the CD45RA marker. Flow cytometric analyses of marker expression and cell division identified 4 subsets of HIV- and CMV-specific CD8+ T cells, representing a lineage differentiation pattern: $CD45RA^+CCR7^+$ (double-positive); $CD45RA^-CCR7^+$; $CD45RA^-CCR7^-$ (double-negative); $CD45RA^+CCR7^-$. The capacity for cell division, as measured by 5-(and 6-)carboxylfluorescein diacetate, succinimidyl ester, and intracellular staining for the Ki67 nuclear antigen, is largely confined to the $CCR7^+$ subsets and occurred more rapidly in cells that are also $CD45RA^+$. Although the double-negative cells did not divide or expand after stimulation, they did revert to positivity for either CD45RA or CCR7 or both. The $CD45RA^+CCR7^-$ cells, considered to be terminally differentiated, fail to divide, but do produce interferon-gamma and express high levels of perform. The representation of subsets specific for CMV and for HIV is distinct. Approximately 70% of HIV-specific $CD8^+$ memory T cells are double-negative or preterminally differentiated compared to 40% of CMV-specific cells. Approximately 50% of the CMV-specific $CD8^+$ memory T cells are terminally differentiated compared to fewer than 10% of the HIV-specific cells. It has been proposed that terminally differentiated CMV-specific cells are poised to rapidly intervene, while double-positive precursor cells remain for expansion and replenishment of the effector cell pool. Furthermore, high-dose antigen tolerance and the depletion of HIV-specific $CD4^+$ helper T-cell activity may keep the HIV-specific memory $CD8^+$ T cells at the double-negative stage, unable to differentiate to the terminal effector state. B lymphocytes recirculate between B cell-rich compartments (follicles or B zones) in secondary lymphoid organs, surveying for antigen. After antigen binding, B cells move to the boundary of B and T zones to interact with T-helper cells. Furthermore it has been demonstrated that antigen-engaged B cells have increased expression of CCR7, the receptor for the T-zone chemokines CCL19 (also known as ELC) and CCL21, and that they exhibit increased responsiveness to both chemoattractants. In mice lacking lymphoid CCL19 and CCL21 chemokines, or with B cells that lack CCR7, antigen engagement fails to cause movement to the T zone. Using retroviral-mediated gene transfer, the authors demonstrated that increased expression of CCR7 is sufficient to direct B cells to the T zone. Reciprocally, overexpression of CXCR5, the receptor for the B-zone chemokine CXCL13, is sufficient to overcome antigen-induced B-cell movement to the T zone. This points toward a mechanism of B-cell relocalization in response to antigen, and established that cell position in vivo can be determined by the balance of responsiveness to chemoattractants made in separate but adjacent zones.

*BAF57, SMARCE 1*

The SWI/SNF complex in S. cerevisiae and Drosophila is thought to facilitate transcriptional activation of specific genes by antagonizing chromatin-mediated transcriptional repression. The complex contains an ATP-dependent nucleosome disruption activity that can lead to enhanced binding of transcription factors. The BRGl/brm-associated factors, or BAF, complex in mammals is functionally related to SWI/SNF and consists of 9 to 12 subunits, some of which are homologous to SWI/SNF subunits. A 57-kD BAF subunit, BAF57, is present in higher eukaryotes, but not in yeast. Partial coding sequence has been obtained from purified BAF57 from extracts of a human cell line [Wang et al., 1998, (60)]. Based on the peptide sequences, they identified cDNAs encoding BAF57. The predicted 411-amino acid protein contains an HMG domain adjacent to a kinesin-like region. Both recombinant BAF57 and the whole BAF complex bind 4-way junction (4WJ) DNA, which is thought to mimic the topology of DNA as it enters or exits the nucleosome. The BAF57 DNA-binding activity has characteristics similar to those of other HMG proteins. It was found that complexes with mutations in the BAF57 HMG domain retain their DNA-binding and nucleosome-disruption activities. They suggested that the mechanism by which mammalian SWI/SNF-like complexes interact with chromatin may involve recognition of higher-order chromatin structure by 2 or more DNA-binding domains. RNase protection studies and Western blot analysis revealed that BAF57 is expressed ubiquitously. Several lines of evidence point toward the involvement of SWI/SNF factors in cancer development [Klochendler-Yeivin et al., 2002, (61)]. Moreover, SWI/SNF related genes are assigned to chromosomal regions that are frequently involved in somatic rearrangements in human cancers [Ring et al., 1998, (62)]. In this respect it is interesting that some of the SWI/SNF family members (i.e. SMARCC1, SMARCC2, SMARCD1 and SMARCD22 are neighboring 3 of the eucaryotic ARCHEONs we have identified (i.e. 3p21-p24, 12q13-q14 and 17q respectively)and which are part of the present invention. In this invention we could also map SMARCE1/BAF57 to the 17q12 region by PCR karyotyping.

*KRT 10, K10*

Keratin 10 is an intermediate filament (IF) chain which belongs to the acidic type I family and is expressed in terminally differentiated epidermal cells. Epithelial cells almost always co-express pairs of type I and type II keratins, and the pairs that are co-expressed are highly characteristic of a given epithelial tissue. For example, in human epidermis, 3 different pairs of keratins are expressed: keratins 5 (type II) and 14 (type I), characteristic of basal or proliferative cells; keratins 1 (type II) and 10 (type I), characteristic of superbasal terminally differentiating cells; and keratins 6 (type II) and 16 (type I) (and keratin 17 [type I]), characteristic of cells induced to hyper-proliferate by disease or injury, and epithelial cells grown in cell culture. The nucleotide sequence of a 1,700 bp cDNA encoding human epidermal keratin 10 (56.5 kD) [Darmon et al., 1987, (63)] has been published as well as the complete amino acid sequence of human keratin 10 [Zhou et al., 1988, (64)]. Polymorphism of the KRT10 gene, restricted to insertions and deletions of the glycine-richquasipeptide repeats that form the glycine-loop motif in the C-terminal domain, have been extensively described [Korge et al., 1992, (65)].

By use of specific cDNA clones in conjunction with somatic cell hybrid analysis and in situ hybridization, KRT10 gene has been mapped to 17q12-q21 in a region proximal to the breakpoint at 17q21 that is involved in a t(17;21)(q21; q22) translocation associated with a form of acute leukemia. KRT10 appeared to be telomeric to 3 other loci that map in the same region: CSF3, ERBA1, and HER2 [Lessin et al., 1988, (66)]. NGFR and HOX2 are distal to K9. It has been demonstrated that the KRT10, KRT13, and KRT15 genes are located in the same large pulsed field gel electrophoresis fragment [Romano et al., 1991, (67)]. A correlation of assignments of the 3 genes makes 17q21-q22 the likely location of the cluster. Transgenic mice expressing a mutant keratin 10 gene have the phenotype of epidermolytic hyperkeratosis , thus suggesting that a genetic basis for the human disorder resides in mutations in genes encoding suprabasal keratins KRT1 or KRT10 [Fuchs et al 1992, (68)]. The authors also showed that stimulation of basal cell proliferation can result from a defect in suprabasal cells and that distortion of nuclear shape or alterations in cytokinesis can occur when an intermediate filament network is perturbed. In a family with keratosis palmaris et plantaris without blistering either spontaneously or in response to mild mechanical or thermal stress and with no involvement of the skin and parts of the body other than the palms and soles, a tight linkage to an insertion-deletion polymorphism in the C-terminal coding region of the KRT10 gene (maximum lod score = 8.36 at theta = 0.00) was found [Rogaev et al., 1993, (69)]. It is noteworthy that it was a rare, high molecular weight allele of the KRT10 polymorphism that segregated with the disorder. The allele was observed once in 96 independent chromosomes from unaffected Caucasians. The KRT10 polymorphism arose from the insertion/deletion of imperfect (CCG)n repeats within the coding region and gave rise to a variable glycine loop motif in the C-terminal tail of the keratin 10 protein. It is possible that there was a pathogenic role for the expansion of the imperfect trinucleotide repeat.

*KRT12,K12*

Keratins are a group of water-insoluble proteins that form 10 nm intermediate filaments in epithelial cells. Approximately 30 different keratin molecules have been identified. They can be divided into acidic and basic-neutral subfamilies according to their relative charges, immunoreactivity, and sequence homologies to types I and II wool keratins, respectively. In vivo, a basic keratin usually is co-expressed and 'paired' with a particular acidic keratin to form a heterodimer. The expression of various keratin pairs is tissue specific, differentiation dependent, and developmentally regulated. The presence of specific keratin pairs is essential for the maintenance of the integrity of epithelium. For example, mutations in human K14/K5 pair and the K10/K1 pair underlie the skin diseases, epidermolysis bullosa simplex and epidermolytic hyperkeratosis, respectively. Expression of the K3 and K12 keratin pair have been found in the cornea of a wide number of species, including human, mouse, and chicken, and is regarded as a marker for corneal-type epithelial differentiation. The murine Krt12 (Krt1.12) gene and demonstrated that its expression is corneal epithelial cell specific, differentiation dependent, and developmentally regulated [Liu et al., 1993, (70)]. The corneal-specific nature of keratin 12 gene expression signifies keratin 12 plays a unique role in maintaining normal corneal epithelial function. Nevertheless, the exact function of keratin 12 remains unknown and no hereditary human corneal epithelial disorder has been linked directly to the mutation in the keratin 12 gene. As part of a study of the expression profile of human corneal epithelial cells, a cDNA with an open reading frame highly homologous to the cornea-specific mouse keratin 12 gene has been isolated [Nishida et al., 1996, (71)]. To elucidate the function of keratin 12 knockout mice lacking the Krt1.12 gene have been created by gene targeting techniques. The heterozygous mice appeared normal. Homozygous mice developed normally and suffered mild corneal epithelial erosion. The corneal epithelia were fragile and could be removed by gentle rubbing of the eyes or brushing. The corneal epithelium of the homozygotes did not express keratin 12 as judged by immunohistochemistry, Western immunoblot analysis with epitope-specific anti-keratin 12 antibodies, Northern hybridization, and in situ hybridization with an antisense keratin 12 riboprobe. The KRT12 gene has been mapped to 17q by study of radiation hybrids and localized it to the type I keratin cluster in the interval between D17S800 and D17S930 (17q12-q21) [Nishida et al., 1997, (72)]. The authors presented the exon-intron boundary structure of the KRT12 gene and mapped the gene to 17q12 by fluorescence in situ hybridization. The gene contains 7 introns, defining 8 exons that cover the coding sequence. Together the exons and introns span approximately 6 kb of genomic DNA.

Meesmann corneal dystrophy is an autosomal dominant disorder causing fragility of the anterior corneal epithelium, where the cornea-specific keratins K3 and K12 are expressed. Dominant-negative mutations in these keratins might be the cause of Meesmann corneal dystrophy. Indeed, linkage of the disorder to the K12 locus in Meesmann's original German kindred [Meesmann and Wilke, 1939, (73)] with Z(max) = 7.53 at theta = 0.0 has been found. In 2 pedigrees from Northern Ireland, they found that the disorder co-segregated with K12 in one pedigree and K3 in the other. Heterozygous missense mutations in K3 or in K12 (R135T, V143L,) in each family have been identified. All these mutations occurred in highly conserved keratin helix boundary motifs, where dominant mutations in other keratins have been found to compromise cytoskeletal function severely, leading to keratinocyte fragility.

The regions of the human KRT12 gene have been sequenced to enable mutation detection for all exons using genomic DNA as a template [Corden et al., 2000, (74)]. The authors found that the human genomic sequence spans 5,919 bp and consists of 8 exons. A microsatellite dinucleotide repeat was identified within intron 3, which was highly polymorphic and which they developed for use in genotype analysis. In addition, 2 mutations in the helix initiation motif of K12 were found in families with Meesmann corneal dystrophy. In an American kindred, a missense M129T mutation was found in the KRT12 gene. They stated that a total of 8 mutations in the KRT12 gene had been reported.

*Genetic interactions within ARCHEONs*

Genes involved in genomic alterations (amplifications, insertions, translocations, deletions, etc.) exhibit changes in their expression pattern. Of particular interest are gene amplifications, which account for gene copy numbers >2 per cell or deletions accounting for gene copy numbers <2 per cell. Gene copy number and gene expression of the respective genes do not necessarily correlate. Transcriptional overexpression needs an intact transcriptional context, as determined by regulatory regions at the chromosomal locus (promotor, enhancer and silencer), and sufficient amounts of transcriptional regulators being present in effective combinations. This is especially true for genomic regions, which expression is tightly regulated in specific tissues or during specific developmental stages. ARCHEONs are specified by gene clusters of more than two genes being directly neighboured or in chromosomal order, interspersed by a maximum of 10, preferably 7, more preferably 5 or at least 1 gene. The interspersed genes are also co-amplified but do not directly interact with the ARCHEON. Such an ARCHEON may spread over a chromosomal region of a maximum of 20, more preferably 10 or at least 6 Megabases. The nature of an ARCHEON is characterized by the simultaneous amplification and/or deletion and the correlating expression (i.e. upregulation or downregulation respectively) of the encompassed genes in a specific tissue, cell type, cellular or developmental state or time point. Such ARCHEONs are commonly conserved during evolution, as they play critical roles during cellular development. In case of these ARCHEONs whole gene clusters are overexpressed upon amplification as they harbor self-regulatory feedback loops, which stabilize gene expression and/or biological effector function even in abnormal biological settings, or are regulated by very similar transcription factor combinations, reflecting their simultaneous function in specific tissues at certain developmental stages. Therefore, the gene copy numbers correlates with the expression level especially for genes in gene clusters functioning as ARCHEONs. In case of abnormal gene expressions in neoplastic lesions it is of great importance to know whether the self-regulatory feedback loops have been conserved as they determine the biological activity of the ARCHEON gene members.

The intensive interaction between genes in ARCHEONs is described for the 17q12 ARCHEON (Fig. 1) by way of illustration not by limitation. In one embodiment the presence or absence of alterations of genes within distinct genomic regions are correlated with each other, as exemplified for breast cancer cell lines (Fig.3 and Fig. 4). This confers to the discovery of the present invention, that multiple interactions of said gene products of defined chromosomal localizations happen, that according to their respective alterations in abnormal tissue have predictive, diagnostic, prognostic and/or preventive and therapeutic value. These interactions are mediated directly or indirectly, due to the fact that the respective genes are part of interconnected or independent signaling networks or regulate cellular behavior (differentiation status, proliferative and /or apoptotic capacity, invasiveness, drug responsiveness, immune modulatory activities) in a synergistic, antagonistic or independent fashion. The order of functionally important genes within the ARCHEONs has been conserved during evolution (e.g. the ARCHEON on human chromosom 17q12 is present on mouse chromosome 11). Moreover, it has been found that the 17q12 ARCHEON is also present on human chromosome 3p21 and 12q13, both of which are also involved in amplification events and in tumor development. Most probably these homologous ARCHEONs were formed by duplications and rearrangements during vertebrate evolution. Homologous ARCHEONs consist of homologous genes and/or isoforms of specific gene families (e.g. RARA or RARB or RARG, THRA or THRB, TOP2A or TOP2B, RABSA or RABSB, BAF170 or BAF 155, BAF60A or BAF60B, WNT5A or WNT5B, IGFBP4 or IGFBP6). Moreover these regions are flanked by homologous chromosomal gene clusters (e.g. CACN, SCYA, HOX, Keratins). These ARCHEONs have diverged during evolution to fulfill their respective functions in distinct tissues (e.g. the 17q12 ARCHEON has one of its main functions in the central nervous system). Due to their tissue specific function extensive regulatory loops control the expression of the members of each ARCHEON. During tumor development these regulations become critical for the characteristics of the abnormal tissues with respect to differentiation, proliferation, drug responsiveness, invasiveness. It has been found that the co-amplification of genes within ARCHEONs can lead to co-expression of the respective gene products. Some of said genes also exhibit additional mutations or specific patterns of polymorphisms, which are substantial for the oncogenic capacities of these ARCHEONs. It is one of the critical features of such amplicons, which members of the ARCHEON have been conserved during tumor formation (e.g. during amplification and deletion events), thereby defining these genes as diagnostic marker genes. Moreover, the expression of the certain genes within the ARCHEON can be influenced by other members of the ARCHEON, thereby defining the regulatory and regulated genes as target genes for therapeutic intervention. It was also observed, that the expression of certain members of the ARCHEON is sensitive to drug treatment (e.g. TOPO2 alpha, RARA, THRA, HER-2) which defines these genes as "marker genes". Moreover several other genes are suitable for therapeutic intervention by antibodies (CACNB1, EBI1), ligands (CACNB1) or drugs like e.g. kinase inhibitors (CrkRS, CDC6). The following examples of interactions between members of ARCHEONs are offered by way of illustration, not by way of limitation.

EBI1/CCR7 is lymphoid-specific member of the G protein-coupled receptor family. EBI1 recognizes chemoattractants, such as interleukin-8, SCYAs, Rantes, C5a, and fMet-Leu-Phe. The capacity for cell division is largely confined to the $CCR7^+$ subsets in lymphocytes. Double-negative cells did not divide or expand after stimulation. $CCR7^-$ cells, considered to be terminally differentiated, fail to divide, but do produce interferon-gamma and express high levels of perforin. EBI1 is induced by viral activities such as the Eppstein-Barr-Virus. Therefore, EBI1 is associated with transformation events in lymphocytes. A functional role of EBI1 during tumor formation in non-lymphoid tissues has been investigated in this invention. Interestingly, also ERBA and ERBB, located in the same genomic region, are associated with lymphocyte transformation. Moreover, ligands of the receptor (i.e. SCYA5/Rantes) are in genomic proximity on 17q. Abnormal expression of both of these factors in lymphoid and non-lymphoid tissues establishes an autorgulatory feedback loop, inducing signaling events within the respective cells. Expression of lymphoid factors has effect on immune cells and modulates cellular behavior. This is of particular interest with regard to abnormal breast tissue being infiltrated by lymphocytes. In line with this, another immunmodulatory and proliferation factor is located nearby on 17q12. Granulocyte colony-stimulating factor (GCSF3) specifically stimulates the proliferation and differentiation of the progenitor cells for granulocytes. A stimulatory activity from a glioblastoma multiforme cell line being biologically and biochemically indistinguishable from GCSF produced by a bladder cell line has also been found. Colony-stimulating factors not only affects immune cells, but also induce cellular responses of non-immune cells, indicating possible involvement in tumor development upon abnormal expression. In addition several other genes of the 17q12 ARCHEON are involved in proliferation, survival, differentiation of immune cells and/or lymphoblastic leukemia, such as MLLT6, ZNF144 and ZNFN1A3, again demonstrating the related functions of the gene products in interconnected key processes within specific cell types. Aberrant expression of more than one of these genes in non-immune cells constitutes signalling activities, that contribute to the oncogenic activities that derive solely from overexpression of the Her-2/neu gene.

PPARBP has been found in complex with the tumorsuppressor gene of the p53 family. Moreover, PPARBP also binds to PPAR-alpha (PPARA), RAR-alpha (RARA), RXR, THRA and TR-beta-1. Due to it's ability to bind to thyroid hormone receptors it has been named TRIP2 and TRAP220. In this complexes PPARBP affects gene regulatory activities. Interestingly, PPARBP is located in genomic proximity to its interaction partners THRA and RARA. We have found PPARBP to be co-amplified with THRA and RARA in tumor tissue. THRA has been isolated from avian erythroblastosis virus in conjunction with ERBB and therefore was named ERBA. ERBA potentiates ERBB by blocking differentiation of erythroblasts at an immature stage. ERBA has been shown to influence ERBB expression. In this setting deletions of C-terminal portions of the THRA gene product are of influence. Aberrant THRA expression has also been found in nonfunctioning pituitary tumors, which has been hypothesized to reflect mutations in the receptor coding and regulatory sequences. THRA function promotes tumor cell development by regulating gene expression of regulatory genes and by influencing metabolic activities (e.g. of key enzymes of alternative metabolic pathways in tumors such as malic enzyme and genes responsible for lipogenesis). The observed activities of nuclear receptors not only reflect their transactivating potential, but are also due to posttranscriptional activities in the absence or presence of ligands. Co-amplification of THRA /ERBA and ERBB has been shown, but its influence on tumor development has been doubted as no overexpression could be demonstrated in breast tumors [van de Vijver et al., 1987, (75)]. THRA and RARA are part of nuclear receptor family whose function can be mediated as monomers, homodimers or heterodimers. RARA regulates differentiation of a broad spectrum of cells. Interactions of hormones with ERBB expression has been investigated. Ligands of RARA can inhibit the expression of amplified ERBB genes in breast tumors [Offterdinger et al., 1998, (76)]. As being part of this invention co-amplification and co-expression of THRA and RARA could be shown. It was also found that multiple genes, which are regulated by members of the thyroid hormone receptor - and retinoic acid receptor family, are differentially expressed in tumor samples, corresponding to their genomic alterations (amplification, mutation, deletion). These hormone receptor genes and respective target genes are useful to discriminate patient samples with respect to clinical features.

By expression analysis of multiple normal tissues, tumor samples and tumor cell lines and subsequent clustering of the 17q12 region, it was found that the expression profile of Her-2/neu positive tumor cells and tumor samples exhibits similarities with the expression pattern of tissue from the central nervous system (Fig. 2). This is in line with the observed malformations in the central nervous system of Her-2/neu and THRA knock-out mice. Moreover, it was found that NEUROD2, a nuclear factor involved specifically in neurogenesis, is commonly expressed in the respective samples. This led to the definition of the 17q12 Locus as being an "ARCHEON", whose primary function in normal organ development is defined to the central nervous system. Surprisingly, the expression of NEUROD2 was affected by therapeutic intervention. Strikingly, also ZNF144, TEM7, PIP5K and PPP1R1B are expressed in neuronal cells, where they display diverse tissue specific functions.

In addition Her-2/neu is often co-amplified with GRB7, a downstream member of the signaling cascade being involved in invasive properties of tumors. Surprisingly, we have found another member of the Her-2/neu signaling cascade being overexpressed in primary breast tumors TOB1 (= "Transducer of ERBB signaling"). Strong overexpression of TOB1 corellated with weaker overexpression of Her-2/neu, already indicating its involvement in oncogenic signaling activities. Amplification of Her-2/neu has been assigned to enhanced proliferative capacity, due to the identified downstream components of the signaling cascade (e.g. Ras-Raf-MAPK). In this respect it was surprising that some cdc genes, which are cell cycle dependent kinases, are part of the amplicons, which upon altered expression have great impact on cell cycle progression.

According to the observations described above the following examples of genes at 3q21-26 are offered by way of illustration, not by way of limitation.

→ WNT5A, CACNA1D, THRB, RARB, TOP2B, RAB5B, SMARCC1 (BAF155), RAF, WNT7A

The following examples of genes at 12q13 are offered by way of illustration, not by way of limitation.

→ CACNB3, Keratins, NR4A1, RAB5/13, RARgamma, STAT6, WNT10B, (GCN5), (SAS: Sarcoma Amplified Sequence), SMARCC2 (BAF170), SMARCD1 (BAF60A), (GAS41: Glioma Amplified Sequence), (CHOP), Her3, KRTHB, HOX C , IGFBP6, WNT5B

There is cross-talk between the amplified ARCHEONs described above and some other highly amplified genomic regions locate approximately at 1p13, 1q32, 2p16, 2q21, 3p12, 5p13, 6p12, 7p12, 7q21, 8q23, 11q13, 13q12, 19q13, 20q13 and 21q11. The above mentioned chromosomal regions are described by way of illustration not by way of limitation, as the amplified regions often span larger and/or overlapping positions at these chromosomal positions.

Additional alterations of non-transcribed genes, pseudogenes or intergenic regions of said chromosomal locations can be measured for prediction, diagnosis, prognosis, prevention and treatment of malignant neoplasia and breast cancer in particular. Some of the genes or genomic regions have no direct influence on the members of the ARCHEONs or the genes within distinct chromosomal regions but still retain marker gene function due to their chromosomal positioning in the neighborhood of functionally critical genes (e.g. Telethonin neighboring the Her-2/neu gene).

The invention further relates to the use of:

a) a polynucleotide comprising at least one of the sequences of SEQ ID NO: 1 to 26 or 53 to 75;

b) a polynucleotide which hybridizes under stringent conditions to a polynucleotide specified in (a) encoding a polypeptide exhibiting the same biological function as specified for the respective sequence in Table 2 or 3

c) a polynucleotide the sequence of which deviates from the polynucleotide specified in (a) and (b) due to the generation of the genetic code encoding a polypeptide exhibiting the same biological function as specified for the respective sequence in Table 2 or 3

d) a polynucleotide which represents a specific fragment, derivative or allelic variation of a polynucleotide sequence specified in (a) to (c)

e) an antisense molecule targeting specifically one of the polynucleotide sequences specified in (a) to (d);

f) a purified polypeptide encoded by a polynucleotide sequence specified in (a) to (d)

g) a purified polypeptide comprising at least one of the sequences of SEQ ID NO: 27 to 52 or 76 to 98;

h) an antibody capable of binding to one of the polynucleotide specified in (a) to (d) or a polypeptide specified in (f) and (g)

i) a reagent identified by any of the methods of claim 14 to 16 that modulates the amount or activity of a polynucleotide sequence specified in (a) to (d) or a polypeptide specified in (f) and (g)

in the preparation of a composition for the prevention, prediction, diagnosis, prognosis or a medicament for the treatment of malignant neoplasia and breast cancer in particular.

*Polynucleotides*

A "BREAST CANCER GENE" polynucleotide can be single- or double-stranded and comprises a coding sequence or the complement of a coding sequence for a "BREAST CANCER GENE" polypeptide. Degenerate nucleotide sequences encoding human "BREAST CANCER GENE" polypeptides, as well as homologous nucleotide sequences which are at least about 50, 55, 60, 65, 70, preferably about 75, 90, 96, or 98% identical to the nucleotide sequences of SEQ ID NO: 1 to 26or 53 to 75 also are "BREAST CANCER GENE" polynucleotides. Percent sequence identity between the sequences of two polynucleotides is determined using computer programs such as ALIGN which employ the FASTA algorithm, using an affine gap search with a gap open penalty of -12 and a gap extension penalty of -2. Complementary DNA (cDNA) molecules, species homologues, and variants of "BREAST CANCER GENE" polynucleotides which encode biologically active "BREAST CANCER GENE" polypeptides also are "BREAST CANCER GENE" polynucleotides.

*Preparation of Polynucleotides*

A naturally occurring "BREAST CANCER GENE" polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated "BREAST CANCER GENE" polynucleotides. For example, restriction enzymes and probes can be used to isolate polynucleotide fragments which comprises "BREAST CANCER GENE" nucleotide sequences. Isolated polynucleotides are in preparations which are free or at least 70, 80, or 90% free of other molecules.

"BREAST CANCER GENE" cDNA molecules can be made with standard molecular biology techniques, using "BREAST CANCER GENE" mRNA as a template. Any RNA isolation technique which does not select against the isolation of mRNA may be utilized for the purification of such RNA samples. See, for example, Sambrook et al., 1989, (77); and Ausubel, F. M. et al., 1989, (78), both of which are incorporated herein by reference in their entirety. Additionally, large numbers of tissue samples may readily be processed using techniques well known to those of skill in the art, such as, for example, the single-step RNA isolation process of Chomczynski, P. (1989, U.S. Pat. No. 4,843,155), which is incorporated herein by reference in its entirety.

"BREAST CANCER GENE" cDNA molecules can thereafter be replicated using molecular biology techniques known in the art and disclosed in manuals such as Sambrook et al., 1989, (77) . An amplification technique, such as PCR, can be used to obtain additional copies of polynucleotides of the invention, using either human genomic DNA or cDNA as a template.

Alternatively, synthetic chemistry techniques can be used to synthesizes "BREAST CANCER GENE" polynucleotides. The degeneracy of the genetic code allows alternate nucleotide sequences to be synthesized which will encode a "BREAST CANCER GENE" polypeptide or a biologically active variant thereof.

*Identification of differential expression*

Transcripts within the collected RNA samples which represent RNA produced by differentially expressed genes may be identified by utilizing a variety of methods which are ell known to those of skill in the art. For example, differential screening [Tedder, T. F. et al., 1988, (79)], subtractive hybridization [Hedrick, S. M. et al., 1984, (80); Lee, S. W. et al., 1984, (81)], and, preferably, differential display (Liang, P., and Pardee, A. B., 1993, U.S. Pat. No. 5,262,311, which is incorporated herein by reference in its entirety), may be utilized to identify polynucleotide sequences derived from genes that are differentially expressed.

Differential screening involves the duplicate screening of a cDNA library in which one copy of the library is screened with a total cell cDNA probe corresponding to the mRNA population of one cell type while a duplicate copy of the cDNA library is screened with a total cDNA probe corresponding to the mRNA population of a second cell type. For example, one cDNA probe may correspond to a total cell cDNA probe of a cell type derived from a control subject, while the second cDNA probe may correspond to a total cell cDNA probe of the same cell type derived from an experimental subject. Those clones which hybridize to one probe but not to the other potentially represent clones derived from genes differentially expressed in the cell type of interest in control versus experimental subjects.

Subtractive hybridization techniques generally involve the isolation of mRNA taken from two different sources, e.g., control and experimental tissue, the hybridization of the mRNA or single-stranded cDNA reverse-transcribed from the isolated mRNA, and the removal of all hybridized, and therefore double-stranded, sequences. The remaining non-hybridized, single-stranded cDNAs, potentially represent clones derived from genes that are differentially expressed in the two mRNA sources. Such single-stranded cDNAs are then used as the starting material for the construction of a library comprising clones derived from differentially expressed genes.

The differential display technique describes a procedure, utilizing the well known polymerase chain reaction (PCR; the experimental embodiment set forth in Mullis, K. B., 1987, U.S. Pat. No. 4,683,202) which allows for the identification of sequences derived from genes which are differentially expressed. First, isolated RNA is reverse-transcribed into single-stranded cDNA, utilizing standard techniques which are well known to those of skill in the art. Primers for the reverse transcriptase reaction may include, but are not limited to, oligo dT-containing primers, preferably of the reverse primer type of oligonucleotide described below. Next, this technique uses pairs of PCR primers, as described below, which allow for the amplification of clones representing a random subset of the RNA transcripts present within any given cell. Utilizing different pairs of primers allows each of the mRNA transcripts present in a cell to be amplified. Among such amplified transcripts may be identified those which have been produced from differentially expressed genes.

The reverse oligonucleotide primer of the primer pairs may contain an oligo dT stretch of nucleotides, preferably eleven nucleotides long, at its 5' end, which hybridizes to the poly(A) tail of mRNA or to the complement of a cDNA reverse transcribed from an mRNA poly(A) tail. Second, in order to increase the specificity of the reverse primer, the primer may contain one or more, preferably two, additional nucleotides at its 3' end. Because, statistically, only a subset of the mRNA derived sequences present in the sample of interest will hybridize to such primers, the additional nucleotides allow the primers to amplify only a subset of the mRNA derived sequences present in the sample of interest. This is preferred in that it allows more accurate and complete visualization and characterization of each of the bands representing amplified sequences.

The forward primer may contain a nucleotide sequence expected, statistically, to have the ability to hybridize to cDNA sequences derived from the tissues of interest. The nucleotide sequence may be an arbitrary one, and the length of the forward oligonucleotide primer may range from about 9 to about 13 nucleotides, with about 10 nucleotides being preferred. Arbitrary primer sequences cause the lengths of the amplified partial cDNAs produced to be variable, thus allowing different clones to be separated by using standard denaturing sequencing gel electrophoresis. PCR reaction conditions should be chosen which optimize amplified product yield and specificity, and, additionally, produce amplified products of lengths which may be resolved utilizing standard gel electrophoresis techniques. Such reaction conditions are well known to those of skill in the art, and important reaction parameters include, for example, length and nucleotide sequence of oligonucleotide primers as discussed above, and annealing and elongation step temperatures and reaction times. The pattern of clones resulting from the reverse transcription and amplification of the mRNA of two different cell types is displayed via sequencing gel electrophoresis and compared. Differences in the two banding patterns indicate potentially differentially expressed genes.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d (T) library does not yield a full-length cDNA. Genomic libraries can be useful for extension of sequence into 5' non-transcribed regulatory regions.

Commercially available capillary electrophoresis systems can be used to analyze the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate software (e.g. GENOTYPER and Sequence NAVIGATOR, Perkin Elmer; ABI), and the entire process from loading of samples to computer analysis and electronic data display can be computer controlled. Capillary electrophoresis is especially preferable for the sequencing of small pieces of DNA which might be present in limited amounts in a particular sample.

Once potentially differentially expressed gene sequences have been identified via bulk techniques such as, for example, those described above, the differential expression of such putatively differentially expressed genes should be corroborated. Corroboration may be accomplished via, for example, such well known techniques as Northern analysis and/or RT-PCR. Upon corroboration, the differentially expressed genes may be further characterized, and may be identified as target and/or marker genes, as discussed, below.

Also, amplified sequences of differentially expressed genes obtained through, for example, differential display may be used to isolate full length clones of the corresponding gene. The full length coding portion of the gene may readily be isolated, without undue experimentation, by molecular biological techniques well known in the art. For example, the isolated differentially expressed amplified fragment may be labeled and used to screen a cDNA library. Alternatively, the labeled fragment may be used to screen a genomic library.

An analysis of the tissue distribution of the mRNA produced by the identified genes may be conducted, utilizing standard techniques well known to those of skill in the art. Such techniques may include, for example, Northern analyses and RT-PCR. Such analyses provide information as to whether the identified genes are expressed in tissues expected to contribute to breast cancer. Such analyses may also provide quantitative information regarding steady state mRNA regulation, yielding data concerning which of the identified genes exhibits a high level of regulation in, preferably, tissues which may be expected to contribute to breast cancer.

Such analyses may also be performed on an isolated cell population of a particular cell type derived from a given tissue. Additionally, standard in situ hybridization techniques may be utilized to provide information regarding which cells within a given tissue express the identified gene. Such analyses may provide information regarding the biological function of an identified gene relative to breast cancer in instances wherein only a subset of the cells within the tissue is thought to be relevant to breast cancer.

*Identification of co-amplified genes*

Genes involved in genomic alterations (amplifications, insertions, translocations, deletions, etc.) are identified by PCR-based karyotyping in combination with database analysis. Of particular interest are gene amplifications, which account for gene copy numbers >2 per cell. Gene copy number and gene expression of the respective genes often correlates. Therefore clusters of genes being simultaneously overexpressed due to gene amplifications can be identified by expression analysis via DNA-chip technologies or quantitative RTPCR. For example, the altered expression of genes due to increased or decreased gene copy numbers can be determined by GeneArray™ technologies from Affymetrix or qRT-PCR with the TaqMan or iCycler Systems. Moreover combination of RNA with DNA analytic enables highly parallel and automated characterization of multiple genomic regions of variable length with high resolution in tissue or single cell samples. Furthermore these assays enable the correlation of gene transcription relative to gene copy number of target genes. As there is not necessarily a linear correlation of expression level and gene copy number and as there are synergistic or antagonistic effects in certain gene clusters, the identification on the RNA-level is easier and probably more relevant for the biological outcome of the alterations especially in tumor tissue.

*Detection of co-amplified genes in malignant neoplasia*

Chromosomal changes are commonly detected by FISH (=Fluorescence-In-Situ-Hybridization) and CGH (=Comparative Genomic Hybridization). For quantification of genomic regions genes or intergenic regions can be used. Such quantification measures the relative abundance of multiple genes with respect to each other (e.g. target gene vs. centromeric region or housekeeping genes). Changes in relative abundance can be detected in paraffin-embedded material even after extraction of RNA or genomic DNA. Measurement of genomic DNA has advantages compared to RNA-analysis due to the stability of DNA, which accounts for the possibility to perform also retrospective studies and offers multiple internal controls (genes not being altered, amplified or deleted) for standardization and exact calculations. Moreover, PCR-analysis of genomic DNA offers the advantage to investigate intergenic, highly variable regions or combinations of SNP's (=Single Nucleotide Polymorphisms), RFLPs, VNTRs and STRs (in general polypmorphic markers). Determination of SNPs or polypmorphic markers within defined genomic regions (e.g. SNP analysis by "Pyrosequencing™") has impact on the phenotype of the genomic alterations. For example it is of advantage to determine combinations of polymorphisms or haplotypes in order to characterize the biological potential of genes being part of amplified alleles. Of particular interest are polypmorphic markers in breakpoint regions, coding regions or regulatory regions of genes or intergenic regions. By determining predictive haplotypes with defined biological or clinical outcome it is possible to establish diagnostic and prognostic assays with non-tumor samples from patients. Depending on whether preferably one allele or both alleles to same extent are amplified (= linear or non-linear amplifications) haplotypes can be determined. Overrepresentation of specific polypmorphic markers combinations in cells or tissues with gene amplifications facilitates haplotype determination, as e.g. combinations of heterozygous polypmorphic markers in nucleic acids isolated from normal tissues, body fluids or biological samples of one patient become almost homozygous in neoplastic tissue of the very same patient. This "gain of homozygosity" corresponds to the measurement of altered genomic region due to amplification events and is suitable for identification of "gain of function"- alterations in tumors, which result in e.g. oncogenic or growth promoting activities. In contrast, the detection of "losses of heterozygosity" is used for identification of anti-oncogenes, gate keeper genes or checkpoint genes, that suppress oncogenic activities and negatively regulate cellular growth processes. This intrinsic difference clearly opposes the impact of the respective genomic regions for tumor development and emphasizes the significance of "gain of homozygosity" measurements disclosed in this invention. In addition to the analyses on SNPs, a comparative approach of blood leucocyte DNA and tumor DNA based on VNTR detection can reveal the existance of a formerely described ARCHEON. SNP and VNTR sequences and primer sets most suitable for detection of theARCHEON at 17q11-21 are disclosed in Table 4 and Table 6. Detection, quantification and sizing of such polymorphic markers can be achieved by methods known to those with skill in the art. In one embodiment of this invention we disclose the comparative measurement of amount and size of any of the disclosed VNTRs (Table 6) by PCR amplification and capillary electrophoresis. PCR can be carried out by standart protocols favorably in a linear amplification range (low cycle number) and detection by CE should be carried out by suppliers protocols (e.g. Agilent). More favorably the detection of the VNTRs disclosed in Table 6 can be carried out in a multiplex fashion, utilizing a variety of labeled primers (e.g. fluoreszent, radioactive, bioactive) and a suitable CE detection system (e.g. ABI 310). However the detection can also be performed on slab gels consiting of highly concentrated agarose or polyacrylamide with a monochromal DNA stain. Enhancement of resolution can be achieved by appropriate primer design and length variation to give best results in multiplex PCR.

It is also of interest to determine covalent modifications of DNA (e.g. methylation) or the associated chromatin (e.g. acetylation or methylation of associated proteins) within the altered genomic regions, that have impact on transcriptional activity of the genes. In general, by measuring multiple, short sequences (60-300 bp) these techniques enable high-resolution analysis of target regions, which cannot be obtained by conventional methods such as FISH analytic (2-100 kb). Moreover the PCR-based DNA analysis techniques offer advantages with regard to sensitivity, specificity, multiplexing, time consumption and low amount of patient material required. These techniques can be optimized by combination with microdissection or macrodissection to obtain purer starting material for analysis.

*Extending Polynucleotides*

In one embodiment of such a procedure for the identification and cloning of full length gene sequences, RNA may be isolated, following standard procedures, from an appropriate tissue or cellular source. A reverse transcription reaction may then be performed on the RNA using an oligonucleotide primer complimentary to the mRNA that corresponds to the amplified fragment, for the priming of first strand synthesis. Because the primer is anti-parallel to the mRNA, extension will proceed toward the 5' end of the mRNA. The resulting RNA hybrid may then be "tailed" with guanines using a standard terminal transferase reaction, the hybrid may be digested with RNase H, and second strand synthesis may then be primed with a poly-C primer. Using the two primers, the 5' portion of the gene is amplified using PCR. Sequences obtained may then be isolated and recombined with previously isolated sequences to generate a full-length cDNA of the differentially expressed genes of the invention. For a review of cloning strategies and recombinant DNA techniques, see e.g., Sambrook et al., (77); and Ausubel et al., (78).

Various PCR-based methods can be used to extend the polynucleotide sequences disclosed herein to detect upstream sequences such as promoters and regulatory elements. For example, restriction site PCR uses universal primers to retrieve unknown sequence adjacent to a known locus [Sarkar, 1993, (82)]. Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region [Triglia et al., 1988 ,(83)]. Primers can be designed using commercially available software, such as OLIGO 4.06 Primer Analysis software (National Biosciences Inc., Plymouth, Minn.), to be e.g. 2230 nucleotides in length, to have a GC content of 50% or more, and to anneal to the target sequence at temperatures about 68-72°C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA [Lagerstrom et al., 1991, (84)]. In this method, multiple restriction enzyme digestions and ligations also can be used to place an engineered double-stranded sequence into an unknown fragment of the DNA molecule before performing PCR.

Additionally, PCR, nested primers, and PROMOTERFINDER libraries (CLONTECH, Palo Alto, Calif.) can be used to walk genomic DNA (CLONTECH, Palo Alto, Calif.). This process avoids the need to screen libraries and is useful in finding intron/exon junctions.

The sequences of the identified genes may be used, utilizing standard techniques, to place the genes onto genetic maps, e.g., mouse [Copeland & Jenkins, 1991, (85)] and human genetic maps [Cohen, et al., 1993 ,(86)]. Such mapping information may yield information regarding the genes' importance to human disease by, for example, identifying genes which map near genetic regions to which known genetic breast cancer tendencies map.

*Identification of polynucleotide variants and homologues or splice variants*

Variants and homologues of the "BREAST CANCER GENE" polynucleotides described above also are "BREAST CANCER GENE" polynucleotides. Typically, homologous "BREAST CANCER GENE" polynucleotide sequences can be identified by hybridization of candidate polynucleotides to known "BREAST CANCER GENE" polynucleotides under stringent conditions, as is known in the art. For example, using the following wash conditions: 2 X SSC (0.3 M NaCl, 0.03 M sodium citrate, pH 7.0), 0.1% SDS, room temperature twice, 30 minutes each; then 2 X SSC, 0.1% SDS, 50 EC once, 30 minutes; then 2 X SSC, room temperature twice, 10 minutes each homologous sequences can be identified which contain at most about 25-30% basepair mismatches. More preferably, homologous polynucleotide strands contain 15-25% basepair mismatches, even more preferably 5-15% basepair mismatches.

Species homologues of the "BREAST CANCER GENE" polynucleotides disclosed herein also can be identified by making suitable probes or primers and screening cDNA expression libraries from other species, such as mice, monkeys, or yeast. Human variants of "BREAST CANCER GENE" polynucleotides can be identified, for example, by screening human cDNA expression libraries. It is well known that the $T_m$ of a double-stranded DNA decreases by 1-1.5°C with every 1% decrease in homology [Bonner et al., 1973, (87)]. Variants of human "BREAST CANCER GENE" polynucleotides or "BREAST CANCER GENE" polynucleotides of other species can therefore be identified by hybridizing a putative homologous "BREAST CANCER GENE" polynucleotide with a polynucleotide having a nucleotide sequence of one of the sequences of the SEQ ID NO: 1 to 26 or 53 to 75 or the complement thereof to form a test hybrid. The melting temperature of the test hybrid is compared with the melting temperature of a hybrid comprising polynucleotides having perfectly complementary nucleotide sequences, and the number or percent of basepair mismatches within the test hybrid is calculated.

Nucleotide sequences which hybridize to "BREAST CANCER GENE" polynucleotides or their complements following stringent hybridization and/or wash conditions also are "BREAST CANCER GENE" polynucleotides. Stringent wash conditions are well known and understood in the art and are disclosed, for example, in Sambrook et al., (77). Typically, for stringent hybridization conditions a combination of temperature and salt concentration should be chosen that is approximately 12-20°C below the calculated $T_m$ of the hybrid under study. The $T_m$ of a hybrid between a "BREAST CANCER GENE" polynucleotide having a nucleotide sequence of one of the sequences of the SEQ ID NO: 1 to 26 or 53 to 75 or the complement thereof and a polynucleotide sequence which is at least about 50, preferably about 75, 90, 96, or 98% identical to one of those nucleotide sequences can be calculated, for example, using the equation below [Bolton and McCarthy, 1962, (88):

$$T_m = 81.5°C - 16.6(\log_{10}[Na^+]) + 0.41(\%G + C) - 0.63(\%formamide) - 600/1),$$

where 1 = the length of the hybrid in basepairs.

Stringent wash conditions include, for example, 4 X SSC at 65°C, or 50% formamide, 4 X SSC at 28°C, or 0.5 X SSC, 0.1% SDS at 65°C. Highly stringent wash conditions include, for example, 0.2 X SSC at 65°C.

The biological function of the identified genes may be more directly assessed by utilizing relevant in vivo and in vitro systems. In vivo systems may include, but are not limited to, animal systems which naturally exhibit breast cancer predisposition, or ones which have been engineered to exhibit such symptoms, including but not limited to the apoE-deficient malignant neoplasia mouse model [Plump et al., 1992, (89)].

Splice variants derived from the same genomic region, encoded by the same pre mRNA can be identified by hybridization conditions described above for homology search. The specific characteristics of variant proteins encoded by splice variants of the same pre transcript may differ and can also be assayed as disclosed. A "BREAST CANCER GENE" polynucleotide having a nucleotide sequence of one of the sequences of the SEQ ID NO: 1 to 26 or 53 to 75 or the complement thereof may therefor differ in parts of the entire sequence as presented for SEQ ID NO: 60 and the encoded splice variants SEQ ID NO: 61 to 66. These refer to individual proteins SEQ ID NO: 83 to 89. The prediction of splicing events and the identification of the utilized acceptor and donor sites within the pre mRNA can be computed (e.g. Software Package GRAIL or GenomeSCAN) and verified by PCR method by those with skill in the art.

*Antisense oligonucleotides*

Antisense oligonucleotides are nucleotide sequences which are complementary to a specific DNA or RNA sequence. Once introduced into a cell, the complementary nucleotides combine with natural sequences produced by the cell to form complexes and block either transcription or translation. Preferably, an antisense oligonucleotide is at least 6 nucleotides in length, but can be at least 7, 8, 10, 12, 15, 20, 25, 30, 35, 40, 45, or 50 or more nucleotides long. Longer sequences also can be used. Antisense oligonucleotide molecules can be provided in a DNA construct and introduced into a cell as described above to decrease the level of "BREAST CANCER GENE" gene products in the cell.

Antisense oligonucleotides can be deoxyribonucleotides, ribonucleotides, peptide nucleic acids (PNAs; described in U.S. Pat. No. 5,714,331), locked nucleic acids (LNAs; described in WO 99/12826), or a combination of them. Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters[Brown, 1994, (126); Sonveaux, 1994, (127) and Uhlmann et al., 1990, (128)].

Modifications of "BREAST CANCER GENE" expression can be obtained by designing antisense oligonucleotides which will form duplexes to the control, 5', or regulatory regions of the "BREAST CANCER GENE". Oligonucleotides derived from the transcription initiation site, e.g., between positions 10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or chaperons. Therapeutic advances using triplex DNA have been described in the literature [Gee et al., 1994, (129)]. An antisense oligonucleotide also can be designed to block translation of mRNA by preventing the transcript from binding to ribosomes.

Precise complementarity is not required for successful complex formation between an antisense oligonucleotide and the complementary sequence of a "BREAST CANCER GENE" polynucleotide. Antisense oligonucleotides which comprise, for example, 2, 3, 4, or 5 or more stretches of contiguous nucleotides which are precisely complementary to a "BREAST CANCER GENE" polynucleotide, each separated by a stretch of contiguous nucleotides which are not complementary to adjacent "BREAST CANCER GENE" nucleotides, can provide sufficient targeting specificity for "BREAST CANCER GENE" mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular "BREAST CANCER GENE" polynucleotide sequence.

Antisense oligonucleotides can be modified without affecting their ability to hybridize to a "BREAST CANCER GENE" polynucleotide. These modifications can be internal or at one or both ends of the antisense molecule. For example, inter-nucleoside phosphate linkages can be modified by adding cholesteryl or diamine moieties with varying numbers of carbon residues between the amino groups and terminal ribose. Modified bases and/or sugars, such as arabinose instead of ribose, or a 3', 5' substituted oligonucleotide in which the 3' hydroxyl group or the 5' phosphate group are substituted, also can be employed in a modified antisense oligonucleotide. These modified oligonucleotides can be prepared by methods well known in the art[ art[ Agrawal et al., 1992, (130); Uhlmann et al., 1987, (131) and Uhlmann et al., (128)].

*Ribozymes*

Ribozymes are RNA molecules with catalytic activity [Cech, 1987, (132); Cech, 1990, (133) and Couture & Stinchcomb, 1996, (134)]. Ribozymes can be used to inhibit gene function by cleaving an RNA sequence, as is known in the art (e.g., Haseloff et al., U.S. Patent 5,641,673). The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences.

The transcribed sequence of a "BREAST CANCER GENE" can be used to generate ribozymes which will specifically bind to mRNA transcribed from a "BREAST CANCER GENE" genomic locus. Methods of designing and constructing ribozymes which can cleave other RNA molecules in trans in a highly sequence specific manner have been developed and described in the art [Haseloff et al., 1988, (135)]. For example, the cleavage activity of ribozymes can be targeted to specific RNAs by engineering a discrete "hybridization" region into the ribozyme. The hybridization region contains a sequence complementary to the target RNA and thus specifically hybridizes with the target [see, for example, Gerlach et al., EP 0 321201].

Specific ribozyme cleavage sites within a "BREAST CANCER GENE" RNA target can be identified by scanning the target molecule for ribozyme cleavage sites which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate "BREAST CANCER GENE" RNA targets also can be evaluated by testing accessibility to hybridization with complementary oligonucleotides using ribonuclease protection assays. Longer complementary sequences can be used to increase the affinity of the hybridization sequence for the target. The hybridizing and cleavage regions of the ribozyme can be integrally related such that upon hybridizing to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease "BREAST CANCER GENE" expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells.

As taught in Haseloff et al., U.S Pat. No. 5,641,673, ribozymes can be engineered so that ribozyme expression will occur in response to factors which induce expression of a target gene. Ribozymes also can be engineered to provide an additional level of regulation, so that destruction of mRNA occurs only when both a ribozyme and a target gene are induced in the cells.

*Polypeptides*

"BREAST CANCER GENE" polypeptides according to the invention comprise an polypeptide selected from SEQ ID NO: 27 to 52 and 76 to 98 or encoded by any of the polynucleotide sequences of the SEQ ID NO: 1 to 26 and 53 to 75 or derivatives, fragments, analogues and homologues thereof. A "BREAST CANCER GENE" polypeptide of the invention therefore can be a portion, a full-length, or a fusion protein comprising all or a portion of a "BREAST CANCER GENE" polypeptide.

*Protein Purification*

"BREAST CANCER GENE" polypeptides can be purified from any cell which expresses the enzyme, including host cells which have been transfected with "BREAST CANCER GENE" expression constructs. Breast tissue is an especially useful source of "BREAST CANCER GENE" polypeptides. A purified "BREAST CANCER GENE" polypeptide is separated from other compounds which normally associate with the "BREAST CANCER GENE" polypeptide in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis. A preparation of purified "BREAST CANCER GENE" polypeptides is at least 80% pure; preferably, the preparations are 90%, 95%, or 99% pure. Purity of the preparations can be assessed by any means known in the art, such as SDS-polyacrylamide gel electrophoresis.

*Obtaining Polypeptides*

"BREAST CANCER GENE" polypeptides can be obtained, for example, by purification from human cells, by expression of "BREAST CANCER GENE" polynucleotides, or by direct chemical synthesis.

*Biologically Active Variants*

"BREAST CANCER GENE" polypeptide variants which are biologically active, i.e., retain an "BREAST CANCER GENE" activity, also are "BREAST CANCER GENE" polypeptides. Preferably, naturally or non-naturally occurring "BREAST CANCER GENE" polypeptide variants have amino acid sequences which are at least about 60, 65, or 70, preferably about 75, 80, 85, 90, 92, 94, 96, or 98% identical to the any of the amino acid sequences of the polypeptides of SEQ ID NO: 27 to 52 or 76 to 98 or the polypeptides encoded by any of the polynucleotides of SEQ ID NO: 1 to 26 or 53 to 75 or a fragment thereof. Percent identity between a putative "BREAST CANCER GENE" polypeptide variant and of the polypeptides of SEQ ID NO: 27 to 52 or 76 to 98 or the polypeptides encoded by any of the polynucleotides of SEQ ID NO: 1 to 26 or 53 to 75 or a fragment thereof is determined by conventional methods. [See, for example, Altschul *et al.,* 1986, (90 and Henikoff & Henikoff, 1992, (91)]. Briefly, two amino acid sequences are aligned to optimize the alignment scores using a gap opening penalty of 10, a gap extension penalty of 1, and the "BLOSUM62" scoring matrix of Henikoff& Henikoff, (91).

Those skilled in the art appreciate that there are many established algorithms available to align two amino acid sequences. The "FASTA" similarity search algorithm of Pearson & Lipman is a suitable protein alignment method for examining the level of identity shared by an amino acid sequence disclosed herein and the amino acid sequence of a putative variant [Pearson & Lipman, 1988, (92), and Pearson, 1990, (93)]. Briefly, FASTA first characterizes sequence similarity by identifying regions shared by the query sequence (e.g., SEQ ID NO: 1 to 26 or 53 to 75) and a test sequence that have either the highest density of identities (if the ktup variable is 1) or pairs of identities (if ktup=2), without considering conservative amino acid substitutions, insertions, or deletions. The ten regions with the highest density of identities are then rescored by comparing the similarity of all paired amino acids using an amino acid substitution matrix, and the ends of the regions are "trimmed" to include only those residues that contribute to the highest score. If there are several regions with scores greater than the "cutoff" value (calculated by a predetermined formula based upon the length of the sequence the ktup value), then the trimmed initial regions are examined to determine whether the regions can be joined to form an approximate alignment with gaps. Finally, the highest scoring regions of the two amino acid sequences are aligned using a modification of the Needleman-Wunsch-Sellers algorithm [Needleman & Wunsch, 1970, (94), and Sellers, 1974, (95)], which allows for amino acid insertions and deletions. Preferred parameters for FASTA analysis are: ktup=1, gap opening penalty=10, gap extension penalty=1, and substitution matrix=BLOSUM62. These parameters can be introduced into a FASTA program by modifying the scoring matrix file ("SMATRIX"), as explained in Appendix 2 of Pearson, (93).

FASTA can also be used to determine the sequence identity of nucleic acid molecules using a ratio as disclosed above. For nucleotide sequence comparisons, the ktup value can range between one to six, preferably from three to six, most preferably three, with other parameters set as default.

Variations in percent identity can be due, for example, to amino acid substitutions, insertions, or deletions. Amino acid substitutions are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid insertions or deletions are changes to or within an amino acid sequence. They typically fall in the range of about 1 to 5 amino acids. Guidance in determining which amino acid residues can be substituted, inserted, or deleted without abolishing biological or immunological activity of a "BREAST CANCER GENE" polypeptide can be found using computer programs well known in the art, such as DNASTAR software. Whether an amino acid change results in a biologically active "BREAST CANCER GENE" polypeptide can readily be determined by assaying for "BREAST CANCER GENE" activity, as described for example, in the specific Examples, below. Larger insertions or deletions can also be caused by alternative splicing. Protein domains can be inserted or deleted without altering the main activity of the protein.

*Fusion Proteins*

Fusion proteins are useful for generating antibodies against "BREAST CANCER GENE" polypeptide amino acid sequences and for use in various assay systems. For example, fusion proteins can be used to identify proteins which interact with portions of a "BREAST CANCER GENE" polypeptide. Protein affinity chromatography or library-based assays for protein-protein interactions, such as the yeast two-hybrid or phage display systems, can be used for this purpose. Such methods are well known in the art and also can be used as drug screens.

A "BREAST CANCER GENE" polypeptide fusion protein comprises two polypeptide segments fused together by means of a peptide bond. The first polypeptide segment comprises at least 25, 50, 75, 100, 150, 200, 300, 400, 500, 600, 700 or 750 contiguous amino acids of an amino acid sequence encoded by any polynucleotide sequences of the SEQ ID NO: 1 to 26 or 53 to 75 or of a biologically active variant, such as those described above. The first polypeptide segment also can comprise full-length "BREAST CANCER GENE".

The second polypeptide segment can be a full-length protein or a protein fragment. Proteins commonly used in fusion protein construction include β-galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, Lex a DNA binding domain (DBD) fusions, GAL4 DNA binding domain fusions, and herpes simplex virus (HSV) BP16 protein fusions. A fusion protein also can be engineered to contain a cleavage site located between the "BREAST CANCER GENE" polypeptide-encoding sequence and the heterologous protein sequence, so that the "BREAST CANCER GENE" polypeptide can be cleaved and purified away from the heterologous moiety.

A fusion protein can be synthesized chemically, as is known in the art. Preferably, a fusion protein is produced by covalently linking two polypeptide segments or by standard procedures in the art of molecular biology. Recombinant DNA methods can be used to prepare fusion proteins, for example, by making a DNA construct which comprises coding sequences selected from any of the polynucleotide sequences of the SEQ ID NO: 1 to 26 and 53 to 75 in proper reading frame with nucleotides encoding the second polypeptide segment and expressing the DNA construct in a host cell, as is known in the art. Many kits for constructing fusion proteins are available from companies such as Promega Corporation (Madison, WI), Stratagene (La Jolla, CA), CLONTECH (Mountain View, CA), Santa Cruz Biotechnology (Santa Cruz, CA), MBL International Corporation (MIC; Watertown, MA), and Quantum Biotechnologies (Montreal, Canada; 1-888-DNA-KITS).

*Identification of Species Homologues*

Species homologues of human a "BREAST CANCER GENE" polypeptide can be obtained using "BREAST CANCER GENE" polypeptide polynucleotides (described below) to make suitable probes or primers for screening cDNA expression libraries from other species, such as mice, monkeys, or yeast, identifying cDNAs which encode homologues of a "BREAST CANCER GENE" polypeptide, and expressing the cDNAs as is known in the art.

*Expression of Polynucleotides*

To express a "BREAST CANCER GENE" polynucleotide, the polynucleotide can be inserted into an expression vector which contains the necessary elements for the transcription and translation of the inserted coding sequence. Methods which are well known to those skilled in the art can be used to construct expression vectors containing sequences encoding "BREAST CANCER GENE" polypeptides and appropriate transcriptional and translational control elements. These methods include in vitro recombinant DNA techniques, synthetic techniques, and in vivo genetic recombination. Such techniques are described, for example, in Sambrook et al., (77) and in Ausubel et al., (78).

A variety of expression vector/host systems can be utilized to contain and express sequences encoding a "BREAST CANCER GENE" polypeptide. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (e.g., baculovirus), plant cell systems transformed with virus expression vectors (e.g., cauliflower mosaic virus, CaMV; tobacco mosaic virus, TMV) or with bacterial expression vectors (e.g., Ti or pBR322 plasmids), or animal cell systems.

The control elements or regulatory sequences are those regions of the vector enhancers, promoters, 5' and 3' untranslated regions which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells (e.g., heat shock, RUBISCO, and storage protein genes) or from plant viruses (e.g., viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding a "BREAST CANCER GENE" polypeptide, vectors based on SV40 or EBV can be used with an appropriate selectable marker.

*Bacterial and Yeast Expression Systems*

In bacterial systems, a number of expression vectors can be selected depending upon the use intended for the "BREAST CANCER GENE" polypeptide. For example, when a large quantity of the "BREAST CANCER GENE" polypeptide is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding the "BREAST CANCER GENE" polypeptide can be ligated into the vector in frame with sequences for the amino terminal Met and the subsequent 7 residues of β-galactosidase so that a hybrid protein is produced. pIN vectors [Van Heeke & Schuster, (17)] or pGEX vectors (Promega, Madison, Wis.) also can be used to express foreign polypeptides as fusion proteins with glutathione S-transferase (GST).

In general, such fusion proteins are soluble and can easily be purified from lysed cells by adsorption to glutathione agarose beads followed by elution in the presence of free glutathione. Proteins made in such systems can be designed to include heparin, thrombin, or factor Xa protease cleavage sites so that the cloned polypeptide of interest can be released from the GST moiety at will.

In the yeast Saccharomyces cerevisiae, a number of vectors containing constitutive or inducible promoters such as alpha factor, alcohol oxidase, and PGH can be used. For reviews, see Ausubel et al., (4) and Grant et al., (18).

*Plant and Insect Expression Systems*

If plant expression vectors are used, the expression of sequences encoding "BREAST CANCER GENE" polypeptides can be driven by any of a number of promoters. For example, viral promoters such as the 35S and 19S promoters of CaMV can be used alone or in combination with the omega leader sequence from TMV [Takamatsu, 1987, (96)]. Alternatively, plant promoters such as the small subunit of RUBISCO or heat shock promoters can be used [Coruzzi et al., 1984, (97); Broglie et al., 1984, (98); Winter et al., 1991, (99)]. These constructs can be introduced into plant cells by direct DNA transformation or by pathogen-mediated transfection. Such techniques are described in a number of generally available reviews.

An insect system also can be used to express a "BREAST CANCER GENE" polypeptide. For example, in one such system Autographa californica nuclear polyhedrosis virus (AcNPV) is used as a vector to express foreign genes in Spodoptera frugiperda cells or in Trichoplusia larvae. Sequences encoding "BREAST CANCER GENE" polypeptides can be cloned into a nonessential region of the virus, such as the polyhedrin gene, and placed under control of the polyhedrin promoter. Successful insertion of "BREAST CANCER GENE" polypeptides will render the polyhedrin gene inactive and produce recombinant virus lacking coat protein. The recombinant viruses can then be used to infect S. frugiperda cells or Trichoplusia larvae in which "BREAST CANCER GENE" polypeptides can be expressed [Engelhard et al., 1994, (100)].

*Mammalian Expression Systems*

A number of viral-based expression systems can be used to express "BREAST CANCER GENE" polypeptides in mammalian host cells. For example, if an adenovirus is used as an expression vector, sequences encoding "BREAST CANCER GENE" polypeptides can be ligated into an adenovirus transcription/translation complex comprising the late promoter and tripartite leader sequence. Insertion in a nonessential E1 or E3 region of the viral genome can be used to obtain a viable virus which is capable of expressing a "BREAST CANCER GENE" polypeptide in infected host cells [Logan & Shenk, 1984, (101)]. If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (e.g., liposomes, polycationic amino polymers, or vesicles).

Specific initiation signals also can be used to achieve more efficient translation of sequences encoding "BREAST CANCER GENE" polypeptides. Such signals include the ATG initiation codon and adjacent sequences. In cases where sequences encoding a "BREAST CANCER GENE" polypeptide, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic. The efficiency of expression can be enhanced by the inclusion of enhancers which are appropriate for the particular cell system which is used [Scharf et al., 1994, (102)].

*Host Cells*

A host cell strain can be chosen for its ability to modulate the expression of the inserted sequences or to process the expressed "BREAST CANCER GENE" polypeptide in the desired fashion. Such modifications of the polypeptide include, but are not limited to, acetylation, carboxylation, glycosylation, phosphorylation, lipidation, and acylation. Posttranslational processing which cleaves a "prepro" form of the polypeptide also can be used to facilitate correct insertion, folding and/or function. Different host cells which have specific cellular machinery and characteristic mechanisms for Post-translational activities (e.g., CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express "BREAST CANCER GENE" polypeptides can be transformed using expression vectors which can contain viral origins of replication and/or endogenous expression elements and a selectable marker gene on the same or on a separate vector. Following the introduction of the vector, cells can be allowed to grow for 12 days in an enriched medium before they are switched to a selective medium. The purpose of the selectable marker is to confer resistance to selection, and its presence allows growth and recovery of cells which successfully express the introduced "BREAST CANCER GENE" sequences. Resistant clones of stably transformed cells can be proliferated using tissue culture techniques appropriate to the cell type [Freshney et al., 1986, (103).

Any number of selection systems can be used to recover transformed cell lines. These include, but are not limited to, the herpes simplex virus thymidine kinase (Wigler et al., 1977, (104)] and adenine phosphoribosyltransferase [Lowy et al., 1980, (105)] genes which can be employed in tk- or $aprt^-$ cells, respectively. Also, antimetabolite, antibiotic, or herbicide resistance can be used as the basis for selection. For example, dhfr confers resistance to methotrexate [Wigler et al., 1980, (106)], npt confers resistance to the aminoglycosides, neomycin and G418 [Colbere-Garapin et al., 1981, (107)], and als and pat confer resistance to chlorsulfuron and phosphinotricin acetyltransferase, respectively. Additional selectable genes have been described. For example, trpB allows cells to utilize indole in place of tryptophan, or hisD, which allows cells to utilize histinol in place of histidine [Hartman & Mulligan, 1988 ,(108)]. Visible markers such as anthocyanins, β-glucuronidase and its substrate GUS, and luciferase and its substrate luciferin, can be used to identify transformants and to quantify the amount of transient or stable protein expression attributable to a specific vector system [Rhodes et al., 1995, (109)].

*Detecting Expression and gene product*

Although the presence of marker gene expression suggests that the "BREAST CANCER GENE" polynucleotide is also present, its presence and expression may need to be confirmed. For example, if a sequence encoding a "BREAST CANCER GENE" polypeptide is inserted within a marker gene sequence, transformed cells containing sequences which encode a "BREAST CANCER GENE" polypeptide can be identified by the absence of marker gene function. Alternatively, a marker gene can be placed in tandem with a sequence encoding a "BREAST CANCER GENE" polypeptide under the control of a single promoter. Expression of the marker gene in response to induction or selection usually indicates expression of the "BREAST CANCER GENE" polynucleotide.

Alternatively, host cells which contain a "BREAST CANCER GENE" polynucleotide and which express a "BREAST CANCER GENE" polypeptide can be identified by a variety of procedures known to those of skill in the art. These procedures include, but are not limited to, DNA-DNA or DNA-RNA hybridization and protein bioassay or immunoassay techniques which include membrane, solution, or chip-based technologies for the detection and/or quantification of polynucleotide or protein. For example, the presence of a polynucleotide sequence encoding a "BREAST CANCER GENE" polypeptide can be detected by DNA-DNA or DNA-RNA hybridization or amplification using probes or fragments or fragments of polynucleotides encoding a "BREAST CANCER GENE" polypeptide. Nucleic acid amplification-based assays involve the use of oligonucleotides selected from sequences encoding a "BREAST CANCER GENE" polypeptide to detect transformants which contain a "BREAST CANCER GENE" polynucleotide.

A variety of protocols for detecting and measuring the expression of a "BREAST CANCER GENE" polypeptide, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on a "BREAST CANCER GENE" polypeptide can be used, or a competitive binding assay can be employed. These and other assays are described in Hampton et al., (110) and Maddox et al., 111).

A wide variety of labels and conjugation techniques are known by those skilled in the art and can be used in various nucleic acid and amino acid assays. Means for producing labeled hybridization or PCR probes for detecting sequences related to polynucleotides encoding "BREAST CANCER GENE" polypeptides include oligo labeling, nick translation, end-labeling, or PCR amplification using a labeled nucleotide. Alternatively, sequences encoding a "BREAST CANCER GENE" polypeptide can be cloned into a vector for the production of an mRNA probe. Such vectors are known in the art, are commercially available, and can be used to synthesize RNA probes in vitro by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

*Expression and Purification of Polypeptides*

Host cells transformed with nucleotide sequences encoding a "BREAST CANCER GENE" polypeptide can be cultured under conditions suitable for the expression and recovery of the protein from cell culture. The polypeptide produced by a transformed cell can be secreted or stored intracellular depending on the sequence and/or the vector used. As will be understood by those of skill in the art, expression vectors containing polynucleotides which encode "BREAST CANCER GENE" polypeptides can be designed to contain signal sequences which direct secretion of soluble "BREAST CANCER GENE" polypeptides through a prokaryotic or eukaryotic cell membrane or which direct the membrane insertion of membrane-bound "BREAST CANCER GENE" polypeptide.

As discussed above, other constructions can be used to join a sequence encoding a "BREAST CANCER GENE" polypeptide to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor Xa or enterokinase (Invitrogen, San Diego, CA) between the purification domain and the "BREAST CANCER GENE" polypeptide also can be used to facilitate purification. One such expression vector provides for expression of a fusion protein containing a "BREAST CANCER GENE" polypeptide and 6 histidine residues preceding a thioredoxin or an enterokinase cleavage site. The histidine residues facilitate purification by IMAC (immobilized metal ion affinity chromatography [Porath et al., 1992, (112)], while the enterokinase cleavage site provides a means for purifying the "BREAST CANCER GENE" polypeptide from the fusion protein. Vectors which contain fusion proteins are disclosed in Kroll et al., (113).

*Chemical Synthesis*

Sequences encoding a "BREAST CANCER GENE" polypeptide can be synthesized, in whole or in part, using chemical methods well known in the art (see Caruthers et al., (114) and Horn et al., (115). Alternatively, a "BREAST CANCER GENE" polypeptide itself can be produced using chemical methods to synthesize its amino acid sequence, such as by direct peptide synthesis using solid-phase techniques [Merrifield, 1963, (116) and Roberge et al., 1995, (117)]. Protein synthesis can be performed using manual techniques or by automation. Automated synthesis can be achieved, for example, using Applied Biosystems 431 A Peptide Synthesizer (Perkin Elmer). Optionally, fragments of "BREAST CANCER GENE" polypeptides can be separately synthesized and combined using chemical methods to produce a full-length molecule.

The newly synthesized peptide can be substantially purified by preparative high performance liquid chromatography [Creighton, 1983, (118)]. The composition of a synthetic "BREAST CANCER GENE" polypeptide can be confirmed by amino acid analysis or sequencing (e.g., the Edman degradation procedure; see Creighton, (118). Additionally, any portion of the amino acid sequence of the "BREAST CANCER GENE" polypeptide can be altered during direct synthesis and/or combined using chemical methods with sequences from other proteins to produce a variant polypeptide or a fusion protein.

*Production of Altered Polypeptides*

As will be understood by those of skill in the art, it may be advantageous to produce "BREAST CANCER GENE" polypeptide-encoding nucleotide sequences possessing non-natural occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.

The nucleotide sequences disclosed herein can be engineered using methods generally known in the art to alter "BREAST CANCER GENE" polypeptide-encoding sequences for a variety of reasons, including but not limited to, alterations which modify the cloning, processing, and/or expression of the polypeptide or mRNA product. DNA shuffling by random fragmentation and PCR re-assembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

*Predictive, Diagnostic and Prognostic Assays*

The present invention provides method for determining whether a subject is at risk for developing malignant neoplasia and breast cancer in particular by detecting one of the disclosed polynucleotide markers comprising any of the polynucleotides sequences of the SEQ ID NO: 2 to 6, 8, 9, 11 to 16, 18, 19 or 21 to 26 or 53 to 75 and/or the polypeptide markers encoded thereby or polypeptide markers comprising any of the polypeptide sequences of the SEQ ID NO: 28 to 32, 34, 35, 37 to 42, 44, 45 or 47 to 52 or 76 to 98 or at least 2 of the disclosed polynucleotides selected from SEQ ID NO: 1 to 26 and 53 to 75 or the at least 2 of the disclosed polypeptides selected from SEQ ID NO: 28 to 32 and 76 to 98 for malignant neoplasia and breast cancer in particular.

In clinical applications, biological samples can be screened for the presence and/or absence of the biomarkers identified herein. Such samples are for example needle biopsy cores, surgical resection samples, or body fluids like serum, thin needle nipple aspirates and urine. For example, these methods include obtaining a biopsy, which is optionally fractionated by cryostat sectioning to enrich diseases cells to about 80% of the total cell population. In certain embodiments, polynucleotides extracted from these samples may be amplified using techniques well known in the art. The expression levels of selected markers detected would be compared with statistically valid groups of diseased and healthy samples.

In one embodiment the diagnostic method comprises determining whether a subject has an abnormal mRNA and/or protein level of the disclosed markers, such as by Northern blot analysis, reverse transcription-polymerase chain reaction (RT-PCR), in situ hybridization, immunoprecipitation, Western blot hybridization, or immunohistochemistry. According to the method, cells are obtained from a subject and the levels of the disclosed biomarkers, protein or mRNA level, is determined and compared to the level of these markers in a healthy subject. An abnormal level of the biomarker polypeptide or mRNA levels is likely to be indicative of malignant neoplasia such as breast cancer.

In another embodiment the diagnostic method comprises determining whether a subject has an abnormal DNA content of said genes or said genomic loci, such as by Southern blot analysis, dot blot analysis, fluorescence or colorimetric In Situ hybridization, comparative genomic hybridization, genotpying by VNTR, STS-PCR or quantitative PCR. In general these assays comprise the usage of probes from representative genomic regions. The probes contain at least parts of said genomic regions or sequences complementary or analogous to said regions. In particular intra- or intergenic regions of said genes or genomic regions. The probes can consist of nucleotide sequences or sequences of analogous functions (e.g. PNAs, Morpholino oligomers) being able to bind to target regions by hybridization. In general genomic regions being altered in said patient samples are compared with unaffected control samples (normal tissue from the same or different patients, surrounding unaffected tissue, peripheral blood) or with genomic regions of the same sample that don't have said alterations and can therefore serve as internal controls. In a preferred embodiment regions located on the same chromosome are used. Alternatively, gonosomal regions and /or regions with defined varying amount in the sample are used. In one favored embodiment the DNA content, structure, composition or modification is compared that lie within distinct genomic regions. Especially favored are methods that detect the DNA content of said samples, where the amount of target regions are altered by amplification and or deletions. In another embodiment the target regions are analyzed for the presence of polymorphisms (e.g. Single Nucleotide Polymorphisms or mutations) that affect or predispose the cells in said samples with regard to clinical aspects, being of diagnostic, prognostic or therapeutic value. Preferably, the identification of sequence variations is used to define haplotypes that result in characteristic behavior of said samples with said clinical aspects.

The following examples of genes in 17q12-21.2 are offered by way of illustration, not by way of limitation.

One embodiment of the invention is a method for the prediction, diagnosis or prognosis of malignant neoplasia by the detection of at least10, at least 5, or at least 4, or at least 3 and more preferably at least 2 markers whereby the markers are genes and fragments thereof and/or genomic nucleic acid sequences that are located on one chromosomal region which is altered in malignant neoplasia.

One further embodiment of the invention is method for the prediction, diagnosis or prognosis of malignant neoplasia by the detection of at least 10, at least 5, or at least 4, or at least 3 and more preferably at least 2 markers whereby the markers (a) are genes and fragments thereof and/or genomic nucleic acid sequences that are located on one or more chromosomal region(s) which is/are altered in malignant neoplasia and (b) functionally interact as (i) receptor and ligand or (ii) members of the same signal transduction pathway or (iii)members of synergistic signal transduction pathways or (iv) members of antagonistic signal transduction pathways or (v) transcription factor and transcription factor binding site.

In one embodiment, the method for the prediction, diagnosis or prognosis of malignant neoplasia and breast cancer in particular is done by the detection of:

(a) polynucleotide selected from the polynucleotides of the SEQ ID NO: 2 to 6, 8, 9, 11 to 16, 18, 19, 21 to 26 or 53 to 75;

(b) a polynucleotide which hybridizes under stringent conditions to a polynucleotide specified in (a) encoding a polypeptide exhibiting the same biological function as specified for the respective sequence in Table 2 or 3;

(c) a polynucleotide the sequence of which deviates from the polynucleotide specified in (a) and (b) due to the generation of the genetic code encoding a polypeptide exhibiting the same biological function as specified for the respective sequence in Table 2 or 3;

(d) a polynucleotide which represents a specific fragment, derivative or allelic variation of a polynucleotide sequence specified in (a) to (c);

in a biological sample comprising the following steps: hybridizing any polynucleotide or analogous oligomer specified in (a) to (do) to a polynucleotide material of a biological sample, thereby forming a hybridization complex; and detecting said hybridization complex.

In another embodiment the method for the prediction, diagnosis or prognosis of malignant neoplasia is done as just described but, wherein before hybridization, the polynucleotide material of the biological sample is amplified.

In another embodiment the method for the diagnosis or prognosis of malignant neoplasia and breast cancer in particular is done by the detection of:

(a) a polynucleotide selected from the polynucleotides of the SEQ ID NO: 2 to 6, 8, 9, 11 to 16, 18, 19, 21 to 26 or 53 to 75;

(b) a polynucleotide which hybridizes under stringent conditions to a polynucleotide specified in (a) encoding a polypeptide exhibiting the same biological function as specified for the respective sequence in Table 2 or 3;

(c) a polynucleotide the sequence of which deviates from the polynucleotide specified in (a) and (b) due to the generation of the genetic code encoding a polypeptide exhibiting the same biological function as specified for the respective sequence in Table 2 or 3;

(d) a polynucleotide which represents a specific fragment, derivative or allelic variation of a polynucleotide sequence specified in (a) to (c);

(e) a polypeptide encoded by a polynucleotide sequence specified in (a) to (d)

(f) a polypeptide comprising any polypeptide of SEQ ID NO: 28 to 32, 34, 35, 37 to 42, 44, 45, 47 to 52 or 76 to 98;

comprising the steps of contacting a biological sample with a reagent which specifically interacts with the polynucleotide specified in (a) to (d) or the polypeptide specified in (e).

*DNA array technology*

In one embodiment, the present Invention also provides a method wherein polynucleotide probes are immobilized an a DNA chip in an organized array. Oligonucleotides can be bound to a solid Support by a variety of processes, including lithography. For example a chip can hold up to 4100,00 oligonucleotides (GeneChip, Affymetrix). The present invention provides significant advantages over the available tests for malignant neoplasia, such as breast cancer, because it increases the reliability of the test by providing an array of polynucleotide markers an a single chip.

The method includes obtaining a biopsy of an affected person, which is optionally fractionated by cryostat sectioning to enrich diseased cells to about 80% of the total cell population and the use of body fluids such as serum or urine, serum or cell containing liquids (e.g. derived from fine needle aspirates). The DNA or RNA is then extracted, amplified, and analyzed with a DNA chip to determine the presence of absence of the marker polynucleotide sequences. In one embodiment, the polynucleotide probes are spotted onto a substrate in a two-dimensional matrix or array. samples of polynucleotides can be labeled and then hybridized to the probes. Double-stranded polynucleotides, comprising the labeled sample polynucleotides bound to probe polynucleotides, can be detected once the unbound portion of the sample is washed away.

The probe polynucleotides can be spotted an substrates including glass, nitrocellulose, etc. The probes can be bound to the Substrate by either covalent bonds or by non-specific interactions, such as hydrophobic interactions. The sample polynucleotides can be labeled using radioactive labels, fluorophores, chromophores, etc. Techniques for constructing arrays and methods of using these arrays are described in EP 0 799 897; WO 97/29212; WO 97/27317; EP 0 785 280; WO 97/02357; U.S. Pat. No. 5,593,839; U.S. Pat. No. 5,578,832; EP 0 728 520; U.S. Pat. No. 5,599,695; EP 0 721 016; U.S. Pat. No. 5,556,752; WO 95/22058; and U.S. Pat. No. 5,631,734.

Further, arrays can be used to examine differential expression of genes and can be used to determine gene function. For example, arrays of the instant polynucleotide sequences can be used to determine if any of the polynucleotide sequences are differentially expressed between normal cells and diseased cells, for example. High expression of a particular message in a diseased sample, which is not observed in a corresponding normal sample, can indicate a breast cancer specific protein.

Accordingly, in one aspect, the invention provides probes and primers that are specific to the unique polynucleotide markers disclosed herein.

In one embodiment, the method comprises using a polynucleotide probe to determine the presence of malignant or breast cancer cells in particular in a tissue from a patient. Specifically, the method comprises:

1) providing a polynucleotide probe comprising a nucleotide sequence at least 12 nucleotides in length, preferably at least 15 nucleotides, more preferably, 25 nucleotides, and most preferably at least 40 nucleotides, and up to all or nearly all of the coding sequence which is complementary to a portion of the coding sequence of a polynucleotide selected from the polynucleotides of SEQ ID NO: 1 to 26 and 53 to 75 or a sequence complementary thereto and is

2) differentially expressed in malignant neoplasia, such as breast cancer;

3) obtaining a tissue sample from a patient with malignant neoplasia;

4) providing a second tissue sample from a patient with no malignant neoplasia;

5) contacting the polynucleotide probe under stringent conditions with RNA of each of said first and second tissue samples (e.g., in a Northern blot or in situ hybridization assay); and

6) comparing (a) the amount of hybridization of the probe with RNA of the first tissue sample, with (b) the amount of hybridization of the probe with RNA of the second tissue sample;

wherein a statistically significant difference in the amount of hybridization with the RNA of the first tissue sample as compared to the amount of hybridization with the RNA of the second tissue sample is indicative of malignant neoplasia and breast cancer in particular in the first tissue sample.

*Data analysis methods*

Comparison of the expression levels of one or more "BREAST CANCER GENES" with reference expression levels, e.g., expression levels in diseased cells of breast cancer or in normal counterpart cells, is preferably conducted using computer systems. In one embodiment, expression levels are obtained in two cells and these two sets of expression levels are introduced into a computer system for comparison. In a preferred embodiment, one set of expression levels is entered into a computer system for comparison with values that are already present in the computer system, or in computer-readable form that is then entered into the computer system.

In one embodiment, the invention provides a computer readable form of the gene expression profile data of the invention, or of values corresponding to the level of expression of at least one "BREAST CANCER GENE" in a diseased cell. The values can be mRNA expression levels obtained from experiments, e.g., microarray analysis. The values can also be mRNA levels normalised relative to a reference gene whose expression is constant in numerous cells under numerous conditions, e.g., GAPDH. In other embodiments, the values in the computer are ratios of, or differences between, normalized or non-normalized mRNA levels in different samples.

The gene expression profile data can be in the form of a table, such as an Excel table. The data can be alone, or it can be part of a larger database, e.g., comprising other expression profiles. For example, the expression profile data of the invention can be part of a public database. The computer readable form can be in a computer. In another embodiment, the invention provides a computer displaying the gene expression profile data.

In one embodiment, the invention provides a method for determining the similarity between the level of expression of one or more "BREAST CANCER GENES" in a first cell, e.g., a cell of a subject, and that in a second cell, comprising obtaining the level of expression of one or more "BREAST CANCER GENES" in a first cell and entering these values into a computer comprising a database including records comprising values corresponding to levels of expression of one or more "BREAST CANCER GENES" in a second cell, and processor instructions, e.g., a user interface, capable of receiving a selection of one or more values for comparison purposes with data that is stored in the computer. The computer may further comprise a means for converting the comparison data into a diagram or chart or other type of output.

In another embodiment, values representing expression levels of "BREAST CANCER GENES" are entered into a computer system, comprising one or more databases with reference expression levels obtained from more than one cell. For example, the computer comprises expression data of diseased and normal cells. Instructions are provided to the computer, and the computer is capable of comparing the data entered with the data in the computer to determine whether the data entered is more similar to that of a normal cell or of a diseased cell.

In another embodiment, the computer comprises values of expression levels in cells of subjects at different stages of breast cancer, and the computer is capable of comparing expression data entered into the computer with the data stored, and produce results indicating to which of the expression profiles in the computer, the one entered is most similar, such as to determine the stage of breast cancer in the subject.

In yet another embodiment, the reference expression profiles in the computer are expression profiles from cells of breast cancer of one or more subjects, which cells are treated *in vivo* or *in vitro* with a drug used for therapy of breast cancer. Upon entering of expression data of a cell of a subject treated *in vitro* or *in vivo* with the drug, the computer is instructed to compare the data entered to the data in the computer, and to provide results indicating whether the expression data input into the computer are more similar to those of a cell of a subject that is responsive to the drug or more similar to those of a cell of a subject that is not responsive to the drug. Thus, the results indicate whether the subject is likely to respond to the treatment with the drug or unlikely to respond to it.

In one embodiment, the invention provides a system that comprises a means for receiving gene expression data for one or a plurality of genes; a means for comparing the gene expression data from each of said one or plurality of genes to a common reference frame; and a means for presenting the results of the comparison. This system may further comprise a means for clustering the data.

In another embodiment, the invention provides a computer program for analyzing gene expression data comprising (i) a computer code that receives as input gene expression data for a plurality of genes and (ii) a computer code that compares said gene expression data from each of said plurality of genes to a common reference frame.

The invention also provides a machine-readable or computer-readable medium including program instructions for performing the following steps: (i) comparing a plurality of values corresponding to expression levels of one or more genes characteristic of breast cancer in a query cell with a database including records comprising reference expression or expression profile data of one or more reference cells and an annotation of the type of cell; and (ii) indicating to which cell the query cell is most similar based on similarities of expression profiles. The reference cells can be cells from subjects at different stages of breast cancer. The reference cells can also be cells from subjects responding or not responding to a particular drug treatment and optionally incubated *in vitro* or *in vivo* with the drug.

The reference cells may also be cells from subjects responding or not responding to several different treatments, and the computer system indicates a preferred treatment for the subject. Accordingly, the invention provides a method for selecting a therapy for a patient having breast cancer, the method comprising: (i) providing the level of expression of one or more genes characteristic of breast cancer in a diseased cell of the patient; (ii) providing a plurality of reference profiles, each associated with a therapy, wherein the subject expression profile and each reference profile has a plurality of values, each value representing the level of expression of a gene characteristic of breast cancer; and (iii) selecting the reference profile most similar to the subject expression profile, to thereby select a therapy for said patient. In a preferred embodiment step (iii) is performed by a computer. The most similar reference profile may be selected by weighing a comparison value of the plurality using a weight value associated with the corresponding expression data.

The relative abundance of an mRNA in two biological samples can be scored as a perturbation and its magnitude determined (i.e., the abundance is different in the two sources of mRNA tested), or as not perturbed (i.e., the relative abundance is the same). In various embodiments, a difference between the two sources of RNA of at least a factor of about 25% (RNA from one source is 25% more abundant in one source than the other source), more usually about 50%, even more often by a factor of about 2 (twice as abundant), 3 (three times as abundant) or 5 (five times as abundant) is scored as a perturbation. Perturbations can be used by a computer for calculating and expression comparisons.

Preferably, in addition to identifying a perturbation as positive or negative, it is advantageous to determine the magnitude of the perturbation. This can be carried out, as noted above, by calculating the ratio of the emission of the two fluorophores used for differential labeling, or by analogous methods that will be readily apparent to those of skill in the art.

The computer readable medium may further comprise a pointer to a descriptor of a stage of breast cancer or to a treatment for breast cancer.

In operation, the means for receiving gene expression data, the means for comparing the gene expression data, the means for presenting, the means for normalizing, and the means for clustering within the context of the systems of the present invention can involve a programmed computer with the respective functionalities described herein, implemented in hardware or hardware and software; a logic circuit or other component of a programmed computer that performs the operations specifically identified herein, dictated by a computer program; or a computer memory encoded with executable instructions representing a computer program that can cause a computer to function in the particular fashion described herein.

Those skilled in the art will understand that the systems and methods of the present invention may be applied to a variety of systems, including IBM-compatible personal computers running MS-DOS or Microsoft Windows.

The computer may have internal components linked to external components. The internal components may include a processor element interconnected with a main memory. The computer system can be an Intel Pentium®-based processor of 200 MHz or greater clock rate and with 32 MB or more of main memory. The external component may comprise a mass storage, which can be one or more hard disks (which are typically packaged together with the processor and memory). Such hard disks are typically of 1 GB or greater storage capacity. Other external components include a user interface device, which can be a monitor, together with an inputing device, which can be a "mouse", or other graphic input devices, and/or a keyboard. A printing device can also be attached to the computer.

Typically, the computer system is also linked to a network link, which can be part of an Ethernet link to other local computer systems, remote computer systems, or wide area communication networks, such as the Internet. This network link allows the computer system to share data and processing tasks with other computer systems.

Loaded into memory during operation of this system are several software components, which are both standard in the art and special to the instant invention. These software components collectively cause the computer system to function according to the methods of this invention. These software components are typically stored on a mass storage. A software component represents the operating system, which is responsible for managing the computer system and its network interconnections. This operating system can be, for example, of the Microsoft Windows' family, such as Windows 95, Windows 98, or Windows NT. A software component represents common languages and functions conveniently present on this system to assist programs implementing the methods specific to this invention. Many high or low level computer languages can be used to program the analytic methods of this invention. Instructions can be interpreted during run-time or compiled. Preferred languages include C/C++, and JAVA®. Most preferably, the methods of this invention are programmed in mathematical software packages which allow symbolic entry of equations and high-level specification of processing, including algorithms to be used, thereby freeing a user of the need to procedurally program individual equations or algorithms. Such packages include Matlab from Mathworks (Natick, Mass.), Mathematica from Wolfram Research (Champaign, Ill.), or S-Plus from Math Soft (Cambridge, Mass.). Accordingly, a software component represents the analytic methods of this invention as programmed in a procedural language or symbolic package. In a preferred embodiment, the computer system also contains a database comprising values representing levels of expression of one or more genes characteristic of breast cancer. The database may contain one or more expression profiles of genes characteristic of breast cancer in different cells.

In an exemplary implementation, to practice the methods of the present invention, a user first loads expression profile data into the computer system. These data can be directly entered by the user from a monitor and keyboard, or from other computer systems linked by a network connection, or on removable storage media such as a CD-ROM or floppy disk or through the network. Next the user causes execution of expression profile analysis software which performs the steps of comparing and, e.g., clustering co-varying genes into groups of genes.

In another exemplary implementation, expression profiles are compared using a method described in U.S. Patent No. 6,203,987. A user first loads expression profile data into the computer system. Geneset profile definitions are loaded into the memory from the storage media or from a remote computer, preferably from a dynamic geneset database system, through the network. Next the user causes execution of projection software which performs the steps of converting expression profile to projected expression profiles. The projected expression profiles are then displayed.

In yet another exemplary implementation, a user first leads a projected profile into the memory. The user then causes the loading of a reference profile into the memory. Next, the user causes the execution of comparison software which performs the steps of objectively comparing the profiles.

*Detection of variant polynucleotide sequence*

In yet another embodiment, the invention provides methods for determining whether a subject is at risk for developing a disease, such as a predisposition to develop malignant neoplasia, for example breast cancer, associated with an aberrant activity of any one of the polypeptides encoded by any of the polynucleotides of the SEQ ID NO: 1 to 26 or 53 to 75, wherein the aberrant activity of the polypeptide is characterized by detecting the presence or absence of a genetic lesion characterized by at least one of these:

(i) an alteration affecting the integrity of a gene encoding a marker polypeptides, or

(ii) the misexpression of the encoding polynucleotide.

To illustrate, such genetic lesions can be detected by ascertaining the existence of at least one of these:

I. a deletion of one or more nucleotides from the polynucleotide sequence

II. an addition of one or more nucleotides to the polynucleotide sequence

III. a substitution of one or more nucleotides of the polynucleotide sequence

IV. a gross chromosomal rearrangement of the polynucleotide sequence

V. a gross alteration in the level of a messenger RNA transcript of the polynucleotide sequence

VI. aberrant modification of the polynucleotide sequence, such as of the methylation pattern of the genomic DNA

VII. the presence of a non-wild type splicing pattern of a messenger RNA transcript of the gene

VIII. a non-wild type level of the marker polypeptide

IX. allelic loss of the gene

X. allelic gain of the gene

XI. inappropriate post-translational modification of the marker polypeptide

The present Invention provides assay techniques for detecting mutations in the encoding polynucleotide sequence. These methods include, but are not limited to, methods involving sequence analysis, Southern blot hybridization, restriction enzyme site mapping, and methods involving detection of absence of nucleotide pairing . between the polynucleotide to be analyzed and a probe.

Specific diseases or disorders, e.g., genetic diseases or disorders, are associated with specific allelic variants of polymorphic regions of certain genes, which do not necessarily encode a mutated protein. Thus, the presence of a specific allelic variant of a polymorphic region of a gene in a subject can render the subject susceptible to developing a specific disease or disorder. Polymorphic regions in genes, can be identified, by determining the nucleotide sequence of genes in populations of individuals. If a polymorphic region is identified, then the link with a specific disease can be determined by studying specific populations of individuals, e.g. individuals which developed a specific disease, such as breast cancer. A polymorphic region can be located in any region of a gene, e.g., exons, in coding or non coding regions of exons, introns, and promoter region.

In an exemplary embodiment, there is provided a polynucleotide composition comprising a polynucleotide probe including a region of nucleotide sequence which is capable of hybridising to a sense or antisense sequence of a gene or naturally occurring mutants thereof, or 5' or 3' flanking sequences or intronic sequences naturally associated with the subject genes or naturally occurring mutants thereof. The polynucleotide of a cell is rendered accessible for hybridization, the probe is contacted with the polynucleotide of the sample, and the hybridization of the probe to the sample polynucleotide is detected. Such techniques can be used to detect lesions or allelic variants at either the genomic or mRNA level, including deletions, substitutions, etc., as well as to determine mRNA transcript levels.

A preferred detection method is allele specific hybridization using probes overlapping the mutation or polymorphic site and having about 5, 10, 20, 25, or 30 nucleotides around the mutation or polymorphic region. In a preferred embodiment of the invention, several probes capable of hybridising specifically to allelic variants are attached to a solid phase support, e.g., a "chip". Mutation detection analysis using these chips comprising oligonucleotides, also termed "DNA probe arrays" is described e.g., in Cronin et al. (119). In one embodiment, a chip comprises all the allelic variants of at least one polymorphic region of a gene. The solid phase support is then contacted with a test polynucleotide and hybridization to the specific probes is detected. Accordingly, the identity of numerous allelic variants of one or more genes can be identified in a simple hybridization experiment.

In certain embodiments, detection of the lesion comprises utilizing the probe/primer in a polymerase chain reaction (PCR) (see, e.g. U.S. Patent Nos. 4,683,195 and 4,683,202), such as anchor PCR or RACE PCR, or, alternatively, in a ligase chain reaction (LCR) [Landegran et al., 1988, (120) and Nakazawa et al., 1994 (121)], the latter of which can be particularly useful for detecting point mutations in the gene; Abravaya et al., 1995 ,(122)]. In a merely illustrative embodiment, the method includes the steps of (i) collecting a sample of cells from a patient, (ii) isolating polynucleotide (e.g., genomic, mRNA or both) from the cells of the sample, (iii) contacting the polynucleotide sample with one or more primers which specifically hybridize to a polynucleotide sequence under conditions such that hybridization and amplification of the polynucleotide (if present) occurs, and (iv) detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication [Guatelli, J.C. et al., 1990, (123)], transcriptional amplification system [Kwoh, D.Y. et al., 1989, (124)], Q-Beta replicase [Lizardi, P.M. et al., 1988 ,(125)], or any other polynucleotide amplification method, followed by the detection of the amplified molecules using techniques well known to those of skill in the art. These detection schemes are especially useful for the detection of polynucleotide molecules if such molecules are present in very low numbers.

In a preferred embodiment of the subject assay, mutations in, or allelic variants, of a gene from a sample cell are identified by alterations in restriction enzyme cleavage patterns. For example, sample and control DNA is isolated, amplified (optionally), digested with one or more restriction endonucleases, and fragment length sizes are determined by gel electrophoresis. Moreover; the use of sequence specific ribozymes (see, for example, U.S. Patent No. 5,498,531) can be used to score for the presence of specific mutations by development or loss of a ribozyme cleavage site.

*In situ hybridization*

In one aspect, the method comprises *in situ* hybridization with a probe derived from a given marker polynucleotide, which sequence is selected from any of the polynucleotide sequences of the SEQ ID NO: 1 to 9, or 11 to 19 or 21 to 26 and 53 to 75 or a sequence complementary thereto. The method comprises contacting the labeled hybridization probe with a sample of a given type of tissue from a patient potentially having malignant neoplasia and breast cancer in particular as well as normal tissue from a person with no malignant neoplasia, and determining whether the probe labels tissue of the patient to a degree significantly different (e.g., by at least a factor of two, or at least a factor of five, or at least a factor of twenty, or at least a factor of fifty) than the degree to which normal tissue is labelled.

*Polypeptide detection*

The subject invention further provides a method of determining whether a cell sample obtained from a subject possesses an abnormal amount of marker polypeptide which comprises (a) obtaining a cell sample from the subject, (b) quantitatively determining the amount of the marker polypeptide in the sample so obtained, and (c) comparing the amount of the marker polypeptide so determined with a known standard, so as to thereby determine whether the cell sample obtained from the subject possesses an abnormal amount of the marker polypeptide. Such marker polypeptides may be detected by immunohistochemical assays, dot-blot assays, ELISA and the like.

*Antibodies*

Any type of antibody known in the art can be generated to bind specifically to an epitope of a "BREAST CANCER GENE" polypeptide. An antibody as used herein includes intact immunoglobulin molecules, as well as fragments thereof, such as Fab, $F(ab)_2$, and Fv, which are capable of binding an epitope of a "BREAST CANCER GENE" polypeptide. Typically, at least 6, 8, 10, or 12 contiguous amino acids are required to form an epitope. However, epitopes which involve non-contiguous amino acids may require more, e.g., at least 15, 25, or 50 amino acids.

An antibody which specifically binds to an epitope of a "BREAST CANCER GENE" polypeptide can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody which specifically binds to the immunogen.

Typically, an antibody which specifically binds to a "BREAST CANCER GENE" polypeptide provides a detection signal at least 5-, 10-, or 20-fold higher than a detection signal provided with other proteins when used in an immunochemical assay. Preferably, antibodies which specifically bind to "BREAST CANCER GENE" polypeptides do not detect other proteins in immunochemical assays and can immunoprecipitate a "BREAST CANCER GENE" polypeptide from solution.

"BREAST CANCER GENE" polypeptides can be used to immunize a mammal, such as a mouse, rat, rabbit, guinea pig, monkey, or human, to produce polyclonal antibodies. If desired, a "BREAST CANCER GENE" polypeptide can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels (e.g., aluminum hydroxide), and surface active substances (e.g. lysolecithin, pluronic polyols, polyanions, peptides, oil emulsions, keyhole limpet hemocyanin, and dinitrophenol). Among adjuvants used in humans, BCG (bacilli Calmette-Guerin) and Corynebacterium parvum are especially useful.

Monoclonal antibodies which specifically bind to a "BREAST CANCER GENE" polypeptide can be prepared using any technique which provides for the production of antibody molecules by continuous cell lines in culture. These techniques include, but are not limited to, the hybridoma technique, the human B cell hybridoma technique, and the EBV hybridoma technique [Kohler et al., 1985, (136); Kozbor et al., 1985, (137); Cote et al., 1983, (138) and Cole et al., 1984, (139)].

In addition, techniques developed for the production of chimeric antibodies, the splicing of mouse antibody genes to human antibody genes to obtain a molecule with appropriate antigen specificity and biological activity, can be used [Morrison et al., 1984, (140); Neuberger et al., 1984, (141); Takeda et al., 1985, (142)]. Monoclonal and other antibodies also can be humanized to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions. Alternatively, humanized antibodies can be produced using recombinant methods, as described in GB2188638B. Antibodies which specifically bind to a "BREAST CANCER GENE" polypeptide can contain antigen binding sites which are either partially or fully humanized, as disclosed in U.S. Patent 5,565,332.

Alternatively, techniques described for the production of single chain antibodies can be adapted using methods known in the art to produce single chain antibodies which specifically bind to "BREAST CANCER GENE" polypeptides. Antibodies with related specificity, but of distinct idiotypic composition, can be generated by chain shuffling from random combinatorial immunoglobulin libraries [Burton, 1991, (143)].

Single-chain antibodies also can be constructed using a DNA amplification method, such as PCR, using hybridoma cDNA as a template [Thirion et al., 1996, (144)]. Single-chain antibodies can be mono- or bispecific, and can be bivalent or tetravalent. Construction of tetravalent, bispecific single-chain antibodies is taught, for example, in Coloma & Morrison, (145). Construction of bivalent, bispecific single-chain antibodies is taught in Mallender & Voss, (146).

A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology [Verhaar et al., 1995, (147); Nicholls et al., 1993, (148)].

Antibodies which specifically bind to "BREAST CANCER GENE" polypeptides also can be produced by inducing in vivo production in the lymphocyte population or by screening immunoglobulin libraries or panels of highly specific binding reagents as disclosed in the literature [Orlandi et al., 1989, (149) and Winter et al., 1991, (150)].

Other types of antibodies can be constructed and used therapeutically in methods of the invention. For example, chimeric antibodies can be constructed as disclosed in WO 93/03151. Binding proteins which are derived from immunoglobulins and which are multivalent and multispecific, such as the antibodies described in WO 94/13804, also can be prepared.

Antibodies according to the invention can be purified by methods well known in the art. For example, antibodies can be affinity purified by passage over a column to which a "BREAST CANCER GENE" polypeptide is bound. The bound antibodies can then be eluted from the column using a buffer with a high salt concentration.

Immunoassays are commonly used to quantify the levels of proteins in cell samples, and many other immunoassay techniques are known in the art. The invention is not limited to a particular assay procedure, and therefore is intended to include both homogeneous and heterogeneous procedures. Exemplary immunoassays which can be conducted according to the invention include fluorescence polarisation immunoassay (FPIA), fluorescence immunoassay (FIA), enzyme immunoassay (EIA), nephelometric inhibition immunoassay (NIA), enzyme linked immunosorbent assay (ELISA), and radioimmunoassay (RIA). An indicator moiety, or label group, can be attached to the subject antibodies and is selected so as to meet the needs of various uses of the method which are often dictated by the availability of assay equipment and compatible immunoassay procedures. General techniques to be used in performing the various immunoassays noted above are known to those of ordinary skill in the art.

In another embodiment, the level of at least one product encoded by any of the polynucleotide sequences of the SEQ ID NO: 2 to 6, 8, 9, 11 to 16, 18, 19 or 21 to 26 or 53 to 75 or of at least 2 products encoded by a polynucleotide selected from SEQ ID NO: 1 to 26 and 53 to 75 or a sequence complementary thereto, in a biological fluid (e.g., blood or urine) of a patient may be determined as a way of monitoring the level of expression of the marker polynucleotide sequence in cells of that patient. Such a method would include the steps of obtaining a sample of a biological fluid from the patient, contacting the sample (or proteins from the sample) with an antibody specific for a encoded marker polypeptide, and determining the amount of immune complex formation by the antibody, with the amount of immune complex formation being indicative of the level of the marker encoded product in the sample. This determination is particularly instructive when compared to the amount of immune complex formation by the same antibody in a control sample taken from a normal individual or in one or more samples previously or subsequently obtained from the same person.

In another embodiment, the method can be used to determine the amount of marker polypeptide present in a cell, which in turn can be correlated with progression of the disorder, e.g., plaque formation. The level of the marker polypeptide can be used predictively to evaluate whether a sample of cells contains cells which are, or are predisposed towards becoming, plaque associated cells. The observation of marker polypeptide level can be utilized in decisions regarding, e.g., the use of more stringent therapies.

As set out above, one aspect of the present invention relates to diagnostic assays for determining, in the context of cells isolated from a patient, if the level of a marker polypeptide is significantly reduced in the sample cells. The term "significantly reduced" refers to a cell phenotype wherein the cell possesses a reduced cellular amount of the marker polypeptide relative to a normal cell of similar tissue origin. For example, a cell may have less than about 50%, 25%, 10%, or 5% of the marker polypeptide that a normal control cell. In particular, the assay evaluates the level of marker polypeptide in the test cells, and, preferably, compares the measured level with marker polypeptide detected in at least one control cell, e.g., a normal cell and/or a transformed cell of known phenotype.

Of particular importance to the subject invention is the ability to quantify the level of marker polypeptide as determined by the number of cells associated with a normal or abnormal marker polypeptide level. The number of cells with a particular marker polypeptide phenotype may then be correlated with patient prognosis. In one embodiment of the invention, the marker polypeptide phenotype of the lesion is determined as a percentage of cells in a biopsy which are found to have abnormally high/low levels of the marker polypeptide. Such expression may be detected by immunohistochemical assays, dot-blot assays, ELISA and the like.

*Immunohistochemistry*

Where tissue samples are employed, immunohistochemical staining may be used to determine the number of cells having the marker polypeptide phenotype. For such staining, a multiblock of tissue is taken from the biopsy or other tissue sample and subjected to proteolytic hydrolysis, employing such agents as protease K or pepsin. In certain embodiments, it may be desirable to isolate a nuclear fraction from the sample cells and detect the level of the marker polypeptide in the nuclear fraction.

The tissues samples are fixed by treatment with a reagent such as formalin, glutaraldehyde, methanol, or the like. The samples are then incubated with an antibody, preferably a monoclonal antibody, with binding specificity for the marker polypeptides. This antibody may be conjugated to a Label for subsequent detection of binding. samples are incubated for a time Sufficient for formation of the immuno-complexes. Binding of the antibody is then detected by virtue of a Label conjugated to this antibody. Where the antibody is unlabelled, a second labeled antibody may be employed, e.g., which is specific for the isotype of the anti-marker polypeptide antibody. Examples of labels which may be employed include radionuclides, fluorescence, chemiluminescence, and enzymes.

Where enzymes are employed, the Substrate for the enzyme may be added to the samples to provide a colored or fluorescent product. Examples of suitable enzymes for use in conjugates include horseradish peroxidase, alkaline phosphatase, malate dehydrogenase and the like. Where not commercially available, such antibody-enzyme conjugates are readily produced by techniques known to those skilled in the art.

In one embodiment, the assay is performed as a dot blot assay. The dot blot assay finds particular application where tissue samples are employed as it allows determination of the average amount of the marker polypeptide associated with a Single cell by correlating the amount of marker polypeptide in a cell-free extract produced from a predetermined number of cells.

In yet another embodiment, the invention contemplates using one or more antibodies which are generated against one or more of the marker polypeptides of this invention, which polypeptides are encoded by any of the polynucleotide sequences of the SEQ ID NO: 1 to 26 or 53 to 75. Such a panel of antibodies may be used as a reliable diagnostic probe for breast cancer. The assay of the present invention comprises contacting a biopsy sample containing cells, e.g., macrophages, with a panel of antibodies to one or more of the encoded products to determine the presence or absence of the marker polypeptides.

The diagnostic methods of the subject invention may also be employed as follow-up to treatment, e.g., quantification of the level of marker polypeptides may be indicative of the effectiveness of current or previously employed therapies for malignant neoplasia and breast cancer in particular as well as the effect of these therapies upon patient prognosis.

The diagnostic assays described above can be adapted to be used as prognostic assays, as well. Such an application takes advantage of the sensitivity of the assays of the Invention to events which take place at characteristic stages in the progression of plaque generation in case of malignant neoplasia. For example, a given marker gene may be up- or down-regulated at a very early stage, perhaps before the cell is developing into a foam cell, while another marker gene may be characteristically up or down regulated only at a much later stage. Such a method could involve the steps of contacting the mRNA of a test cell with a polynucleotide probe derived from a given marker polynucleotide which is expressed at different characteristic levels in breast cancer tissue cells at different stages of malignant neoplasia progression, and determining the approximate amount of hybridization of the probe to the mRNA of the cell, such amount being an indication of the level of expression of the gene in the cell, and thus an indication of the stage of disease progression of the cell; alternatively, the assay can be carried out with an antibody specific for the gene product of the given marker polynucleotide, contacted with the proteins of the test cell. A battery of such tests will disclose not only the existence of a certain arteriosclerotic plaque, but also will allow the clinician to select the mode of treatment most appropriate for the disease, and to predict the likelihood of success of that treatment.

The methods of the invention can also be used to follow the clinical course of a given breast cancer predisposition. For example, the assay of the Invention can be applied to a blood sample from a patient; following treatment of the patient for BREAST CANCER, another blood sample is taken and the test repeated. Successful treatment will result in removal of demonstrate differential expression, characteristic of the breast cancer tissue cells, perhaps approaching or even surpassing normal levels.

*Polypeptide activity*

In one embodiment the present invention provides a method for screening potentially therapeutic agents which modulate the activity of one or more "BREAST CANCER GENE" polypeptides, such that if the activity of the polypeptide is increased as a result of the upregulation of the "BREAST CANCER GENE" in a subject having or at risk for malignant neoplasia and breast cancer in particular, the therapeutic substance will decrease the activity of the polypeptide relative to the activity of the some polypeptide in a subject not having or not at risk for malignant neoplasia or breast cancer in particular but not treated with the therapeutic agent. Likewise, if the activity of the polypeptide as a result of the downregulation of the "BREAST CANCER GENE" is decreased in a subject having or at risk for malignant neoplasia or breast cancer in particular, the therapeutic agent will increase the activity of the polypeptide relative to the activity of the same polypeptide in a subject not having or not at risk for malignant neoplasia or breast cancer in particular, but not treated with the therapeutic agent.

The activity of the "BREAST CANCER GENE" polypeptides indicated in Table 2 or 3 may be measured by any means known to those of skill in the art, and which are particular for the type of activity performed by the particular polypeptide. Examples of specific assays which may be used to measure the activity of particular polynucleotides are shown below.

a) G protein coupled receptors

In one embodiment, the "BREAST CANCER GENE" polynucleotide may encode a G protein coupled receptor. In one embodiment, the present invention provides a method of screening potential modulators (inhibitors or activators) of the G protein coupled receptor by measuring changes in the activity of the receptor in the presence of a candidate modulator.

*1. $G_i$-coupled receptors*

Cells (such as CHO cells or primary cells) are stably transfected with the relevant receptor and with an inducible CRE-luciferase construct. Cells are grown in 50% Dulbecco's modified Eagle medium / 50% F12 (DMEM/F12) supplemented with 10% FBS, at 37°C in a humidified atmosphere with 10% $CO_2$ and are routinely split at a ratio of 1: 10 every 2 or 3 days. Test cultures are seeded into 384 - well plates at an appropriate density (e.g. 2000 cells / well in 35 µl cell culture medium) in DMEM/F12 with FBS, and are grown for 48 hours (range:~ 24 - 60 hours, depending on cell line). Growth medium is then exchanged against serum free medium (SFM; e.g. Ultra-CHO), containing 0,1% BSA. Test compounds dissolved in DMSO are diluted in SFM and transferred to the test cultures (maximal final concentration 10 µmolar), followed by addition of forskolin (~ 1 µmolar, final conc.) in SFM + 0,1% BSA 10 minutes later. In case of antagonist screening both, an appropriate concentration of agonist, and forskolin are added. The plates are incubated at 37°C in 10% $CO_2$ for 3 hours. Then the supernatant is removed, cells are lysed with lysis reagent (25 mmolar phosphate-buffer, pH 7,8, containing 2 mmolar DDT, 10% glycerol and 3% Triton X100). The luciferase reaction is started by addition of substrate-buffer (e.g. luciferase assay reagent, Promega) and luminescence is immediately determined (e.g. Berthold luminometer or Hamamatzu camera system).

*2. $G_s$-coupled receptors*

Cells (such as CHO cells or primary cells) are stably transfected with the relevant receptor and with an inducible CRE-luciferase construct. Cells are grown in 50% Dulbecco's modified Eagle medium / 50% F12 (DMEM/F12) supplemented with 10% FBS, at 37°C in a humidified atmosphere with 10% $CO_2$ and are routinely split at a ratio of 1: 10 every 2 or 3 days. Test cultures are seeded into 384 - well plates at an appropriate density (e.g. 1000 or 2000 cells / well in 35 µl cell culture medium) in DMEM/F12 with FBS, and are grown for 48 hours (range:~ 24 - 60 hours, depending on cell line). The assay is started by addition of test-compounds in serum free medium (SFM; e.g. Ultra-CHO) containing 0,1% BSA: Test compounds are dissolved in DMSO, diluted in SFM and transferred to the test cultures (maximal final concentration 10 µmolar, DMSO conc. < 0,6 %). In case of antagonist screening an appropriate concentration of agonist is added 5 - 10 minutes later. The plates are incubated at 37°C in 10% $CO_2$ for 3 hours. Then the cells are lysed with 10 µl lysis reagent per well (25 mmolar phosphate-buffer, pH 7,8 , containing 2 mmolar DDT, 10% glycerol and 3% Triton X100) and the luciferase reaction is started by addition of 20 µl substrate-buffer per well (e.g. luciferase assay reagent, Promega). Measurement of luminescence is started immediately (e.g. Berthold luminometer or Hamamatzu camera system).

3. $G_q$-coupled receptors

Cells (such as CHO cells or primary cells) are stably transfected with the relevant receptor. Cells expressing functional receptor protein are grown in 50% Dulbecco's modified Eagle medium / 50% F12 (DMEM/F12) supplemented with 10% FBS, at 37°C in a humidified atmosphere with 5% $CO_2$ and are routinely split at a cell line dependent ratio every 3 or 4 days. Test cultures are seeded into 384 - well plates at an appropriate density (e.g. 2000 cells / well in 35 µl cell culture medium) in DMEM/F12 with FBS, and are grown for 48 hours (range:~ 24 - 60 hours, depending on cell line). Growth medium is then exchanged against physiological salt solution (e.g. Tyrode solution). Test compounds dissolved in DMSO are diluted in Tyrode solution containing 0.1% BSA and transferred to the test cultures (maximal final concentration 10 molar). After addition of the receptor specific agonist the resulting Gq-mediated intracellular calcium increase is measured using appropriate read-out systems (e.g. calcium-sensitive dyes).

b) Ion channels

Ion channels are integral membrane proteins involved in electrical signaling, transmembrane signal transduction, and electrolyte and solute transport. By forming macromolecular pores through the membrane lipid bilayer, ion channels account for the flow of specific ion species driven by the electrochemical potential gradient for the permeating ion. At the single molecule level, individual channels undergo conformational transitions ("gating") between the 'open' (ion conducting) and 'closed' (non conducting) state. Typical single channel openings last for a few milliseconds and result in elementary transmembrane currents in the range of $10^{-9}$ - $10^{-12}$ Ampere. Channel gating is controlled by various chemical and/or biophysical parameters, such as neurotransmitters and intracellular second messengers ('ligand-gated' channels) or membrane potential ('voltage-gated' channels). Ion channels are functionally characterized by their ion selectivity, gating properties, and regulation by hormones and pharmacological agents. Because of their central role in signaling and transport processes, ion channels present ideal targets for pharmacological therapeutics in various pathophysiological settings.

In one embodiment, the "BREAST CANCER GENE" may encode an ion channel. In one embodiment, the present invention provides a method of screening potential activators or inhibitors of channels activity of the "BREAST CANCER GENE" polypeptide. Screening for compounds interaction with ion channels to either inhibit or promote their activity can be based on (1.) binding and (2.) functional assays in living cells[ Hille (183)].

1. For ligand-gated channels, e.g. ionotropic neurotransmitter/hormone receptors, assays can be designed detecting binding to the target by competition between the compound and a labeled ligand.

2. Ion channel function can be tested functionally in living cells. Target proteins are either expressed endogenously in appropriate reporter cells or are introduced recombinantly. Channel activity can be monitored by (2.1) concentration changes of the permeating ion (most prominently $Ca^{2+}$ ions), (2.2) by changes in the transmembrane electrical potential gradient, and (2.3) by measuring a cellular response (e.g. expression of a reporter gene, secretion of a neurotransmitter) triggered or modulated by the target activity.

2.1 Channel activity results in transmembrane ion fluxes. Thus activation of ionic channels can be monitored by the resulting changes in intracellular ion concentrations using luminescent or fluorescent indicators. Because of its wide dynamic range and availability of suitable indicators this applies particularly to changes in intracellular $Ca^{2+}$ ion concentration ($[Ca^{2+}]$i). $[Ca^{2+}]$i can be measured, for example, by aequorin luminescence or fluorescence dye technology (e.g. using Fluo-3, Indo-1, Fura-2). Cellular assays can be designed where either the $Ca^{2+}$ flux through the target channel itself is measured directly or where modulation of the target channel affects membrane potential and thereby the activity of co-expressed voltage-gated $Ca^{2+}$ channels.

2.2 Ion channel currents result in changes of electrical membrane potential ($V_m$) which can be monitored directly using potentiometric fluorescent probes. These electrically charged indicators (e.g. the anionic oxonol dye $DiBAC_4(3)$) redistribute between extra- and intracellular compartment in response to voltage changes. The equilibrium distribution is governed by the Nemst-equation. Thus changes in membrane potential results in concomitant changes in cellular fluorescence. Again, changes in $V_m$ might be caused directly by the activity of the target ion channel or through amplification and/or prolongation of the signal by channels co-expressed in the same cell.

2.3 Target channel activity can cause cellular $Ca^{2+}$ entry either directly or through activation of additional $Ca^{2+}$ channel (see 2.1). The resulting intracellular $Ca^{2+}$ signals regulate a variety of cellular responses, e.g. secretion or gene transcription. Therefore modulation of the target channel can be detected by monitoring secretion of a known hormone/transmitter from the target-expressing cell or through expression of a reporter gene (e.g. luciferase) controlled by an $Ca^{2+}$-responsive promoter element (e.g. cyclic AMP/ $Ca2^{+}$-responsive elements; CRE).

c) DNA-binding proteins and transcription factors

In one embodiment, the "BREAST CANCER GENE" may encode a DNA-binding protein or a transcription factor. The activity of such a DNA-binding protein or a transcription factor may be measured, for example, by a promoter assay which measures the ability of the DNA-binding protein or the transcription factor to initiate transcription of a test sequence linked to a particular promoter. In one embodiment, the present invention provides a method of screening test compounds for its ability to modulate the activity of such a DNA-binding protein or a transcription factor by measuring the changes in the expression of a test gene which is regulated by a promoter which is responsive to the transcription factor.

d) Promotor assays

A promoter assay was set up with a human hepatocellular carcinoma cell HepG2 that was stably transfected with a luciferase gene under the control of a gene of interest (e.g. thyroid hormone) regulated promoter. The vector 2xIROluc, which was used for transfection, carries a thyroid hormone responsive element (TRE) of two 12 bp inverted palindromes separated by an 8 bp spacer in front of a tk minimal promoter and the luciferase gene. Test cultures were seeded in 96 well plates in serum - free Eagle's Minimal Essential Medium supplemented with glutamine, tricine, sodium pyruvate, non - essential amino acids, insulin, selen, transferrin, and were cultivated in a humidified atmosphere at 10 % $CO_2$ at 37°C. After 48 hours of incubation serial dilutions of test compounds or reference compounds (L-T3, L-T4 e.g.) and co-stimulator if appropriate (final concentration 1 nM) were added to the cell cultures and incubation was continued for the optimal time (e.g. another 4-72 hours). The cells were then lysed by addition of buffer containing Triton X100 and luciferin and the luminescence of luciferase induced by T3 or other compounds was measured in a luminometer. For each concentration of a test compound replicates of 4 were tested. $EC_{50}$— values for each test compound were calculated by use of the Graph Pad Prism Scientific software.

*Screening Methods*

The invention provides assays for screening test compounds which bind to or modulate the activity of a "BREAST CANCER GENE" polypeptide or a "BREAST CANCER GENE" polynucleotide. A test compound preferably binds to a "BREAST CANCER GENE" polypeptide or polynucleotide. More preferably, a test compound decreases or increases "BREAST CANCER GENE" activity by at least about 10, preferably about 50, more preferably about 75, 90, or 100% relative to the absence of the test compound.

*Test Compounds*

Test compounds can be pharmacological agents already known in the art or can be compounds previously unknown to have any pharmacological activity. The compounds can be naturally occurring or designed in the laboratory. They can be isolated from microorganisms, animals, or plants, and can be produced recombinant, or synthesised by chemical methods known in the art. If desired, test compounds can be obtained using any of the numerous combinatorial library methods known in the art, including but not limited to, biological libraries, spatially addressable parallel solid phase or solution phase libraries, synthetic library methods requiring de-convolution, the one-bead one-compound library method, and synthetic library methods using affinity chromatography selection. The biological library approach is limited to polypeptide libraries, while the other four approaches are applicable to polypeptide, non-peptide oligomer, or small molecule libraries of compounds. [For review see Lam, 1997, (151)].

Methods for the synthesis of molecular libraries are well known in the art [see, for example, DeWitt et al., 1993, (152); Erb et al., 1994, (153); Zuckermann et al., 1994, (154); Cho et al., 1993, (155); Carell et al., 1994, (156) and Gallop et al., 1994, (157). Libraries of compounds can be presented in solution [see, e.g., Houghten, 1992, (158)], or on beads [Lam, 1991, (159)], DNA-chips [Fodor, 1993, (160)], bacteria or spores (Ladner, U.S. Patent 5,223,409), plasmids [Cull et al., 1992, (161)], or phage [Scott & Smith, 1990, (162); Devlin, 1990, (163); Cwirla et al., 1990, (164); Felici, 1991, (165)].

*High Throughput Screening*

Test compounds can be screened for the ability to bind to "BREAST CANCER GENE" polypeptides or polynucleotides or to affect "BREAST CANCER GENE" activity or "BREAST CANCER GENE" expression using high throughput screening. Using high throughput screening, many discrete compounds can be tested in parallel so that large numbers of test compounds can be quickly screened. The most widely established techniques utilize 96-well, 384-well or 1536-well microtiter plates. The wells of the microtiter plates typically require assay volumes that range from 5 to 500 μl. In addition to the plates, many instruments, materials, pipettors, robotics, plate washers, and plate readers are commercially available to fit the microwell formats.

Alternatively, free format assays, or assays that have no physical barrier between samples, can be used. For example, an assay using pigment cells (melanocytes) in a simple homogeneous assay for combinatorial peptide libraries is described by Jayawickreme et al., (166). The cells are placed under agarose in culture dishes, then beads that carry combinatorial compounds are placed on the surface of the agarose. The combinatorial compounds are partially released the compounds from the beads. Active compounds can be visualised as dark pigment areas because, as the compounds diffuse locally into the gel matrix, the active compounds cause the cells to change colors.

Another example of a free format assay is described by Chelsky, (167). Chelsky placed a simple homogenous enzyme assay for carbonic anhydrase inside an agarose gel such that the enzyme in the gel would cause a color change throughout the gel.

Thereafter, beads carrying combinatorial compounds via a photolinker were placed inside the gel and the compounds were partially released by UV light. Compounds that inhibited the enzyme were observed as local zones of inhibition having less color change.

In another example, combinatorial libraries were screened for compounds that had cytotoxic effects on cancer cells growing in agar [Salmon et al., 1996, (168)].

Another high throughput screening method is described in Beutel et al., U.S. Patent 5,976,813. In this method, test samples are placed in a porous matrix. One or more assay components are then placed within, on top of, or at the bottom of a matrix such as a gel, a plastic sheet, a filter, or other form of easily manipulated solid support. When samples are introduced to the porous matrix they diffuse sufficiently slowly, such that the assays can be performed without the test samples running together.

*Binding Assays*

For binding assays, the test compound is preferably a small molecule which binds to and occupies, for example, the ATP/GTP binding site of the enzyme or the active site of a "BREAST CANCER GENE" polypeptide, such that normal biological activity is prevented. Examples of such small molecules include, but are not limited to, small peptides or peptide-like molecules.

In binding assays, either the test compound or a "BREAST CANCER GENE" polypeptide can comprise a detectable label, such as a fluorescent, radioisotopic, chemiluminescent, or enzymatic label, such as horseradish peroxidase, alkaline phosphatase, or luciferase. Detection of a test compound which is bound to a "BREAST CANCER GENE" polypeptide can then be accomplished, for example, by direct counting of radioemmission, by scintillation counting, or by determining conversion of an appropriate substrate to a detectable product.

Alternatively, binding of a test compound to a "BREAST CANCER GENE" polypeptide can be determined without labeling either of the interactants. For example, a microphysiometer can be used to detect binding of a test compound with a "BREAST CANCER GENE" polypeptide. A microphysiometer (e.g., CytosensorJ) is an analytical instrument that measures the rate at which a cell acidifies its environment using a light-addressable potentiometric sensor (LAPS). Changes in this acidification rate can be used as an indicator of the interaction between a test compound and a "BREAST CANCER GENE" polypeptide [McConnell et al., 1992, (169)].

Determining the ability of a test compound to bind to a "BREAST CANCER GENE" polypeptide also can be accomplished using a technology such as real-time Bimolecular Interaction Analysis (BIA) [Sjolander & Urbaniczky, 1991, (170), and Szabo et al., 1995, (171)]. BIA is a technology for studying biospecific interactions in real time, without labeling any of the interactants (e.g., BIAcore™). Changes in the optical phenomenon surface plasmon resonance (SPR) can be used as an indication of real-time reactions between biological molecules.

In yet another aspect of the invention, a "BREAST CANCER GENE" polypeptide can be used as a "bait protein" in a two-hybrid assay or three-hybrid assay [see, e.g., U.S. Patent 5,283,317; Zervos et al., 1993, (172); Madura et al., 1993, (173); Bartel et al., 1993, (174); Iwabuchi et al., 1993, (175) and Brent WO 94/10300], to identify other proteins which bind to or interact with the "BREAST CANCER GENE" polypeptide and modulate its activity.

The two-hybrid system is based on the modular nature of most transcription factors, which consist of separable DNA-binding and activation domains. Briefly, the assay utilizes two different DNA constructs. For example, in one construct, polynucleotide encoding a "BREAST CANCER GENE" polypeptide can be fused to a polynucleotide encoding the DNA binding domain of a known transcription factor (e.g., GAL4). In the other construct a DNA sequence that encodes an unidentified protein ("prey" or "sample") can be fused to a polynucleotide that codes for the activation domain of the known transcription factor. If the "bait" and the "prey" proteins are able to interact in vivo to form an protein- dependent complex, the DNA-binding and activation domains of the transcription factor are brought into close proximity. This proximity allows transcription of a reporter gene (e.g., LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with the "BREAST CANCER GENE" polypeptide.

It may be desirable to immobilize either a "BREAST CANCER GENE" polypeptide (or polynucleotide) or the test compound to facilitate separation of bound from unbound forms of one or both of the interactants, as well as to accommodate automation of the assay. Thus, either a "BREAST CANCER GENE" polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach a "BREAST CANCER GENE" polypeptide (or polynucleotide) or test compound to a solid support, including use of covalent and non-covalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to a "BREAST CANCER GENE" polypeptide (or polynucleotide) can be accomplished in any vessel suitable for containing the reactants. Examples of such vessels include microtiter plates, test tubes, and microcentrifuge tubes.

In one embodiment, a "BREAST CANCER GENE" polypeptide is a fusion protein comprising a domain that allows the "BREAST CANCER GENE" polypeptide to be bound to a solid support. For example, glutathione S-transferase fusion proteins can be adsorbed onto glutathione sepharose beads (Sigma Chemical, St. Louis, Mo.) or glutathione derivatized microtiter plates, which are then combined with the test compound or the test compound and the nonadsorbed "BREAST CANCER GENE" polypeptide; the mixture is then incubated under conditions conducive to complex formation (e.g., at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilising proteins or polynucleotides on a solid support also can be used in the screening assays of the invention. For example, either a "BREAST CANCER GENE" polypeptide (or polynucleotide) or a test compound can be immobilized utilizing conjugation of biotin and streptavidin. Biotinylated "BREAST CANCER GENE" polypeptides (or polynucleotides) or test compounds can be prepared from biotin NHS (N-hydroxysuccinimide) using techniques well known in the art (e.g., biotinylation kit, Pierce Chemicals, Rockford, Ill.) and immobilized in the wells of streptavidin-coated 96 well plates (Pierce Chemical). Alternatively, antibodies which specifically bind to a "BREAST CANCER GENE" polypeptide, polynucleotide, or a test compound, but which do not interfere with a desired binding site, such as the ATP/GTP binding site or the active site of the "BREAST CANCER GENE" polypeptide, can be derivatised to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to a "BRBAST CANCER GENE" polypeptide or test compound, enzyme-linked assays which rely on detecting an activity of a "BREAST CANCER GENE" polypeptide, and SDS gel electrophoresis under non-reducing conditions.

Screening for test compounds which bind to a "BREAST CANCER GENE" polypeptide or polynucleotide also can be carried out in an intact cell. Any cell which comprises a "BREAST CANCER GENE" polypeptide or polynucleotide can be used in a cell-based assay system. A "BREAST CANCER GENE" polynucleotide can be naturally occurring in the cell or can be introduced using techniques such as those described above. Binding of the test compound to a "BREAST CANCER GENE" polypeptide or polynucleotide is determined as described above.

_Modulation of Gene Expression_

In another embodiment, test compounds which increase or decrease "BREAST CANCER GENE" expression are identified. A "BREAST CANCER GENE" polynucleotide is contacted with a test compound, and the expression of an RNA or polypeptide product of the "BREAST CANCER GENE" polynucleotide is determined. The level of expression of appropriate mRNA or polypeptide in the presence of the test compound is compared to the level of expression of mRNA or polypeptide in the absence of the test compound. The test compound can then be identified as a modulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of "BREAST CANCER GENE" mRNA or polypeptide expression in the cells can be determined by methods well known in the art for detecting mRNA or polypeptide. Either qualitative or quantitative methods can be used. The presence of polypeptide products of a "BREAST CANCER GENE" polynucleotide can be determined, for example, using a variety of techniques known in the art, including immunochemical methods such as radioimmunoassay, Western blotting, and immunohistochemistry. Alternatively, polypeptide synthesis can be determined in vivo, in a cell culture, or in an in vitro translation system by detecting incorporation of labeled amino acids into a "BREAST CANCER GENE" polypeptide.

Such screening can be carried out either in a cell-free assay system or in an intact cell. Any cell which expresses a "BREAST CANCER GENE" polynucleotide can be used in a cell-based assay system. A "BREAST CANCER GENE" polynucleotide can be naturally occurring in the cell or can be introduced using techniques such as those described above. Either a primary culture or an established cell line, such as CHO or human embryonic kidney 293 cells, can be used.

*Therapeutic Indications and Methods*

Therapies for treatment of breast cancer primarily relied upon effective chemotherapeutic drugs for intervention on the cell proliferation, cell growth or angiogenesis. The advent of genomics-driven molecular target identification has opened up the possibility of identifying new breast cancer-specific targets for therapeutic intervention that will provide safer, more effective treatments for malignant neoplasia patients and breast cancer patients in particular. Thus, newly discovered breast cancer-associated genes and their products can be used as tools to develop innovative therapies. The identification of the Her2/neu receptor kinase presents exciting new opportunities for treatment of a certain subset of tumor patients as described before. Genes playing important roles in any of the physiological processes outlined above can be characterized as breast cancer targets. Genes or gene fragments identified through genomics can readily be expressed in one or more heterologous expression systems to produce functional recombinant proteins. These proteins are characterized in vitro for their biochemical properties and then used as tools in high-throughput molecular screening programs to identify chemical modulators of their biochemical activities. Modulators of target gene expression or protein activity can be identified in this manner and subsequently tested in cellular and in vivo disease models for therapeutic activity. Optimization of lead compounds with iterative testing in biological models and detailed pharmacokinetic and toxicological analyses form the basis for drug development and subsequent testing in humans.

This invention further pertains to the use of novel agents identified by the screening assays described above. Accordingly, it is within the scope of this invention to use a test compound identified as described herein in an appropriate animal model. For example, an agent identified as described herein (e.g., a modulating agent, an antisense polynucleotide molecule, a specific antibody, ribozyme, or a human "BREAST CANCER GENE" polypeptide binding molecule) can be used in an animal model to determine the efficacy, toxicity, or side effects of treatment with such an agent. Alternatively, an agent identified as described herein can be used in an animal model to determine the mechanism of action of such an agent. Furthermore, this invention pertains to uses of novel agents identified by the above described screening assays for treatments as described herein.

A reagent which affects human "BREAST CANCER GENE" activity can be administered to a human cell, either in vitro or in vivo, to reduce or increase human "BREAST CANCER GENE" activity. The reagent preferably binds to an expression product of a human "BREAST CANCER GENE". If the expression product is a protein, the reagent is preferably an antibody. For treatment of human cells ex vivo, an antibody can be added to a preparation of stem cells which have been removed from the body. The cells can then be replaced in the same or another human body, with or without clonal propagation, as is known in the art.

In one embodiment, the reagent is delivered using a liposome. Preferably, the liposome is stable in the animal into which it has been administered for at least about 30 minutes, more preferably for at least about 1 hour, and even more preferably for at least about 24 hours. A liposome comprises a lipid composition that is capable of targeting a reagent, particularly a polynucleotide, to a particular site in an animal, such as a human. Preferably, the lipid composition of the liposome is capable of targeting to a specific organ of an animal, such as the lung, liver, spleen, heart brain, lymph nodes, and skin.

A liposome useful in the present invention comprises a lipid composition that is capable of fusing with the plasma membrane of the targeted cell to deliver its contents to the cell. Preferably, the transfection efficiency of a liposome is about 0.5 μg of DNA per 16 nmol of liposome delivered to about $10^6$ cells, more preferably about 1.0 μg

of DNA per 16 nmol of liposome delivered to about $10^6$ cells, and even more preferably about 2.0 μg of DNA per 16 nmol of liposome delivered to about $10^6$ cells. Preferably, a liposome is between about 100 and 500 nm, more preferably between about 150 and 450 nm, and even more preferably between about 200 and 400 nm in diameter.

Suitable liposomes for use in the present invention include those liposomes usually used in, for example, gene delivery methods known to those of skill in the art. More preferred liposomes include liposomes having a polycationic lipid composition and/or liposomes having a cholesterol backbone conjugated to polyethylene glycol. Optionally, a liposome comprises a compound capable of targeting the liposome to a particular cell type, such as a cell-specific ligand exposed on the outer surface of the liposome.

Complexing a liposome with a reagent such as an antisense oligonucleotide or ribozyme can be achieved using methods which are standard in the art (see, for example, U.S. Patent 5,705,151). Preferably, from about 0.1 μg to about 10 μg of polynucleotide is combined with about 8 nmol of liposomes, more preferably from about 0.5 μg to about 5 μg of polynucleotides are combined with about 8 nmol liposomes, and even more preferably about 1.0 μg of polynucleotides is combined with about 8 nmol liposomes.

In another embodiment, antibodies can be delivered to specific tissues in vivo using receptor-mediated targeted delivery. Receptor-mediated DNA delivery techniques are taught in, for example, Findeis et al., 1993, (176); Chiou et al., 1994, (177); Wu & Wu, 1988, (178); Wu et al., 1994, (179); Zenke et al., 1990, (180); Wu et al., 1991, (181).

*Determination of a Therapeutically Effective Dose*

The determination of a therapeutically effective dose is well within the capability of those skilled in the art. A therapeutically effective dose refers to that amount of active ingredient which increases or decreases human "BREAST CANCER GENE" activity relative to the human "BREAST CANCER GENE" activity which occurs in the absence of the therapeutically effective dose.

For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, e.g., $ED_{50}$ (the dose therapeutically effective in 50% of the population) and $LD_{50}$ (the dose lethal to 50% of the population), can be determined by standard pharmaceutical procedures in cell cultures or experimental animals. The dose ratio of toxic to therapeutic effects is the therapeutic index, and it can be expressed as the ratio, $LD_{5o}/ED_{5o}$.

Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the $ED_{50}$ with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

If the reagent is a single-chain antibody, polynucleotides encoding the antibody can be constructed and introduced into a cell either ex vivo or in vivo using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, a gene gun, and DEAE- or calcium phosphate-mediated transfection.

Effective in vivo dosages of an antibody are in the range of about 5 μg to about 50 μg/kg, about 50 μg to about 5 mg/kg, about 100 μg to about 500 μg/kg of patient body weight, and about 200 to about 250 μg/kg of patient body weight. For administration of polynucleotides encoding single-chain antibodies, effective in vivo dosages are in the range of about 100 ng to about 200 ng, 500 ng to about 50 mg, about 1 μg to about 2 mg, about 5 μg to about 500 μg, and about 20 μg to about 100 μg of DNA.

If the expression product is mRNA, the reagent is preferably an antisense oligonucleotide or a ribozyme. Polynucleotides which express antisense oligonucleotides or ribozymes can be introduced into cells by a variety of methods, as described above.

Preferably, a reagent reduces expression of a "BREAST CANCER GENE" gene or the activity of a "BREAST CANCER GENE" polypeptide by at least about 10, preferably about 50, more preferably about 75, 90, or 100% relative to the absence of the reagent. The effectiveness of the mechanism chosen to decrease the level of expression of a "BREAST CANCER GENE" gene or the activity of a "BREAST CANCER GENE" polypeptide can be assessed using methods well known in the art, such as hybridization of nucleotide probes to "BREAST CANCER GENE"-specific mRNA, quantitative RT-PCR, immunologic detection of a "BREAST CANCER GENE" polypeptide, or measurement of "BREAST CANCER GENE" activity.

In any of the embodiments described above, any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects.

Any of the therapeutic methods described above can be applied to any subject in need of such therapy, including, for example, birds and mammals such as dogs, cats, cows, pigs, sheep, goats, horses, rabbits, monkeys, and most preferably, humans.

All patents and patent applications cited in this disclosure are expressly incorporated herein by reference. The above disclosure generally describes the present invention. A more complete understanding can be obtained by reference to the following specific examples which are provided for purposes of illustration only and are not intended to limit the scope of the invention.

*Pharmaceutical Compositions*

The invention also provides pharmaceutical compositions which can be administered to a patient to achieve a therapeutic effect. Pharmaceutical compositions of the invention can comprise, for example, a "BREAST CANCER GENE" polypeptide, "BREAST CANCER GENE" polynucleotide, ribozymes or antisense oligonucleotides, antibodies which specifically bind to a "BREAST CANCER GENE" polypeptide, or mimetics, agonists, antagonists, or inhibitors of a "BREAST CANCER GENE" polypeptide activity. The compositions can be administered alone or in combination with at least one other agent, such as stabilizing compound, which can be administered in any sterile, biocompatible pharmaceutical carrier, including, but not limited to, saline, buffered saline, dextrose, and water. The compositions can be administered to a patient alone, or in combination with other agents, drugs or hormones.

In addition to the active ingredients, these pharmaceutical compositions can contain suitable pharmaceutically acceptable carriers comprising excipients and auxiliaries which facilitate processing of the active compounds into preparations which can be used pharmaceutically. Pharmaceutical compositions of the invention can be administered by any number of routes including, but not limited to, oral, intravenous, intramuscular, intraarterial, intramedullary, intrathecal, intraventricular, transdermal, subcutaneous, intraperitoneal, intranasal, parenteral, topical, sublingual, or rectal means. Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethylcellulose, or sodium carboxymethylcellulose; gums including arabic and tragacanth; and proteins such as gelatin and collagen. If desired, disintegrating or solubilizing agents can be added, such as the cross-linked polyvinyl pyrrolidone, agar, alginic acid, or a salt thereof, such as sodium alginate.

Dragee cores can be used in conjunction with suitable coatings, such as concentrated sugar solutions, which also can contain gum arabic, talc, polyvinylpyrrolidone, carbopol gel, polyethylene glycol, and/or titanium dioxide, lacquer solutions, and suitable organic solvents or solvent mixtures. Dyestuffs or pigments can be added to the tablets or dragee coatings for product identification or to characterize the quantity of active compound, i.e., dosage.

Pharmaceutical preparations which can be used orally include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds can be dissolved or suspended in suitable liquids, such as fatty oils, liquid, or liquid polyethylene glycol with or without stabilizers.

Pharmaceutical formulations suitable for parenteral administration can be formulated in aqueous solutions, preferably in physiologically compatible buffers such as Hanks' solution, Ringer's solution, or physiologically buffered saline. Aqueous injection suspensions can contain substances which increase the viscosity of the suspension, such as sodium carboxymethyl cellulose, sorbitol, or dextran. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers also can be used for delivery. Optionally, the suspension also can contain suitable stabilizers or agents which increase the solubility of the compounds to allow for the preparation of highly concentrated solutions. For topical or nasal administration, penetrants appropriate to the particular barrier to be permeated are used in the formulation. Such penetrants are generally known in the art.

The pharmaceutical compositions of the present invention can be manufactured in a manner that is known in the art, e.g., by means of conventional mixing, dissolving, granulating, dragee making, levigating, emulsifying, encapsulating, entrapping, or lyophilizing processes. The pharmaceutical composition can be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation can be a lyophilized powder which can contain any or all of the following: 150 mM histidine, 0.1 %2% sucrose, and 27% mannitol, at a pH range of 4.5 to 5.5, that is combined with buffer prior to use.

Further details on techniques for formulation and administration can be found in the latest edition of REMINGTON'S PHARMACEUTICAL SCIENCES (182). After pharmaceutical compositions have been prepared, they can be placed in an appropriate container and labeled for treatment of an indicated condition. Such labeling would include amount, frequency, and method of administration.

*Material and Methods*

One strategy for identifying genes that are involved in breast cancer is to detect genes that are expressed differentially under conditions associated with the disease versus non-disease conditions. The sub-sections below describe a number of experimental systems which may be used to detect such differentially expressed genes. In general, these experimental systems include at least one experimental condition in which subjects or samples are treated in a manner associated with breast cancer, in addition to at least one experimental control condition lacking such disease associated treatment. Differentially expressed genes are detected, as described below, by comparing the pattern of gene expression between the experimental and control conditions.

Once a particular gene has been identified through the use of one such experiment, its expression pattern may be further characterized by studying its expression in a different experiment and the findings may be validated by an independent technique. Such use of multiple experiments may be useful in distinguishing the roles and relative importance of particular genes in breast cancer. A combined approach, comparing gene expression pattern in cells derived from breast cancer patients to those of *in vitro* cell culture models can give substantial hints on the pathways involved in development and/or progression of breast cancer.

Among the experiments which may be utilized for the identification of differentially expressed genes involved in malignant neoplasia and breast cancer, for example, are experiments designed to analyze those genes which are involved in signal transduction. Such experiments may serve to identify genes involved in the proliferation of cells.

Below are methods described for the identification of genes which are involved in breast cancer. Such represent genes which are differentially expressed in breast cancer conditions relative to their expression in normal, or non-breast cancer conditions or upon experimental manipulation based on clinical observations. Such differentially expressed genes represent "target" and/or "marker" genes. Methods for the further characterization of such differentially expressed genes, and for their identification as target and/or marker genes, are presented below.

Alternatively, a differentially expressed gene may have its expression modulated, i.e., quantitatively increased or decreased, in normal versus breast cancer states, or under control versus experimental conditions. The degree to which expression differs in normal versus breast cancer or control versus experimental states need only be large enough to be visualized via standard characterization techniques, such as, for example, the differential display technique described below. Other such standard characterization techniques by which expression differences may be visualized include but are not limited to quantitative RT-PCR and Northern analyses, which are well known to those of skill in the art.

**EXAMPLE 1**

*Expression profiling*

*a) Expression profiling utilizing quantitative RT-PCR*

For a detailed analysis of gene expression by quantitative PCR methods, one will utilize primers flanking the genomic region of interest and a fluorescent labeled probe hybridizing in-between. Using the PRISM 7700 Sequence Detection System of PE Applied Biosystems (Perkin Elmer, Foster City, CA, USA) with the technique of a fluorogenic probe, consisting of an oligonucleotide labeled with both a fluorescent reporter dye and a quencher dye, one can perform such a expression measurement. Amplification of the probe-specific product causes cleavage of the probe, generating an increase in reporter fluorescence. Primers and probes were selected using the Primer Express software and localized mostly in the 3' region of the coding sequence or in the 3' untranslated region (see Table 5 for primer- and probe- sequences) according to the relative positions of the probe sequence used for the construction of the Affymetrix HG_U95A-E or HG-U133A-B DNA-chips. All primer pairs were checked for specificity by conventional PCR reactions. To standardize the amount of sample RNA, GAPDH was selected as a reference, since it was not differentially regulated in the samples analyzed. TaqMan validation experiments were performed showing that the efficiencies of the target and the control amplifications are approximately equal which is a prerequisite for the relative quantification of gene expression by the comparative $\Delta\Delta C_T$ method, known to those with skills in the art.

As well as the technology provided by Perkin Elmer one may use other technique implementations like Lightcycler™ from Roche Inc. or iCycler from Stratagene Inc..

*b) Expression profiling utilizing DNA microarrays*

Expression profiling can bee carried out using the Affymetrix Array Technology. By hybridization of mRNA to such a DNA-array or DNA-Chip, it is possible to identify the expression value of each transcripts due to signal intensity at certain position of the array. Usually these DNA-arrays are produced by spotting of cDNA, oligonucleotides or subcloned DNA fragments. In case of Affymetrix technology app. 400.000 individual oligonucleotide sequences were synthesized on the surface of a silicon wafer at distinct positions. The minimal length of oligomers is 12 nucleotides, preferable 25 nucleotides or full length of the questioned transcript. Expression profiling may also be carried out by hybridization to nylon or nitrocellulose membrane bound DNA or oligonucleotides. Detection of signals derived from hybridization may be obtained by either colorimetric, fluorescent, electrochemical, electronic, optic or by radioactive readout. Detailed description of array construction have been mentioned above and in other patents cited. To determine the quantitative and qualitative changes in the chromosomal region to analyze, RNA from tumor tissue which is suspected to contain such genomic alterations has to be compared to RNA extracted from benign tissue (e.g. epithelial breast tissue, or micro dissected ductal tissue) on the basis of expression profiles for the whole transcriptome. With minor modifications, the sample preparation protocol followed the Affymetrix GeneChip Expression Analysis Manual (Santa Clara, CA). Total RNA extraction and isolation from tumor or benign tissues, biopsies, cell isolates or cell containing body fluids can be performed by using TRIzol (Life Technologies, Rockville, MD) and Oligotex mRNA Midi kit (Qiagen, Hilden, Germany), and an ethanol precipitation step should be carried out to bring the concentration to 1 mg/ml. Using 5-10 mg of mRNA to create double stranded cDNA by the Superscript system (Life Technologies). First strand cDNA synthesis was primed with a T7-(dT24) oligonucleotide. The cDNA can be extracted with phenol/chloroform and precipitated with ethanol to a final concentration of 1 mg /ml. From the generated cDNA, cRNA can be synthesized using Enzo's (Enzo Diagnostics Inc., Farmingdale, NY) *in vitro* Transcription Kit. Within the same step the cRNA can be labeled with biotin nucleotides Bio-11-CTP and Bio-16-UTP (Enzo Diagnostics Inc., Farmingdale, NY) . After labeling and cleanup (Qiagen, Hilden (Germany) the cRNA then should be fragmented in an appropriated fragmentation buffer (e.g., 40 mM Tris-Acetate, pH 8.1, 100 mM KOAc, 30 mM MgOAc, for 35 minutes at 94°C). As per the Affymetrix protocol, fragmented cRNA should be hybridized on the HG_U133 arrays A and B, comprising app. 40.000 probed transcripts each, for 24 hours at 60 rpm in a 45°C hybridization oven. After Hybridization step the chip surfaces have to be washed and stained with streptavidin phycoerythrin (SAPE; Molecular Probes, Eugene, OR) in Affymetrix fluidics stations. To amplify staining, a second labeling step can be introduced, which is recommended but not compulsive. Here one should add SAPE solution twice with an antistreptavidin biotinylated antibody. Hybridization to the probe arrays may be detected by fluorometric scanning (Hewlett Packard Gene Array Scanner; Hewlett Packard Corporation, Palo Alto, CA).

After hybridization and scanning, the microarray images can be analyzed for quality control, looking for major chip defects or abnormalities in hybridization signal. Therefor either Affymetrix GeneChip MAS 5.0 Software or other microarray image analysis software can be utilized. Primary data analysis should be carried out by software provided by the manufacturer..

In case of the genes analyses in one embodiment of this invention the primary data have been analyzed by further bioinformatic tools and additional filter criteria. The bioinformatic analysis is described in detail below.

*c) Data analysis*

According to Affymetrix measurement technique (Affymetrix GeneChip Expression Analysis Manual, Santa Clara, CA) a single gene expression measurement on one chip yields the average difference value and the absolute call. Each chip contains 16-20 oligonucleotide probe pairs per gene or cDNA clone. These probe pairs include perfectly matched sets and mismatched sets, both of which are necessary for the calculation of the average difference, or expression value, a measure of the intensity difference for each probe pair, calculated by subtracting the intensity of the mismatch from the intensity of the perfect match. This takes into consideration variability in hybridization among probe pairs and other hybridization artifacts that could affect the fluorescence intensities. The average difference is a numeric value supposed to represent the expression value of that gene. The absolute call can take the values 'A' (absent), 'M' (marginal), or 'P' (present) and denotes the quality of a single hybridization. We used both the quantitative information given by the average difference and the qualitative information given by the absolute call to identify the genes which are differentially expressed in biological samples from individuals with breast cancer versus biological samples from the normal population. With other algorithms than the Affymetrix one we have obtained different numerical values representing the same expression values and expression differences upon comparison.

The differential expression E in one of the breast cancer groups compared to the normal population is calculated as follows. Given n average difference values $d_1, d_2, ..., d_n$ in the breast cancer population and m average difference values $c_1, c_2, ..., c_m$ in the population of normal individuals, it is computed by the equation:

$$E \equiv \exp\left(\frac{1}{m}\sum_{i=1}^{m}\ln(c_i) - \frac{1}{n}\sum_{i=1}^{n}\ln(d_i)\right)$$

If $d_j<50$ or $c_i<50$ for one or more values of i and j, these particular values $c_i$ and/or $d_j$ are set to an "artificial" expression value of 50. These particular computation of E allows for a correct comparison to TaqMan results.

A gene is called up-regulated in breast cancer versus normal if $E \geq 1.5$ and if the number of absolute calls equal to 'P' in the breast cancer population is greater than n/2.

A gene is called down-regulated in breast cancer versus normal if $E \leq 1.5$ and if the number of absolute calls equal to 'P' in the normal population is greater than m/2.

The final list of differentially regulated genes consists of all up-regulated and all down-regulated genes in biological samples from individuals with breast cancer versus biological samples from the normal population. Those genes on this list which are interesting for a pharmaceutical application were finally validated by TaqMan. If a good correlation between the expression values/behavior of a transcript could be observed with both techniques, such a gene is listed in Tables 1 to 3.

Since not only the information on differential expression of a single gene within an identified ARCHEON, but also the information on the co-regulation of several members is important for predictive, diagnostic, preventive and therapeutic purposes we have combined expression data with information on the chromosomal position (e.g. golden path) taken from public available databases to develop a picture of the overall transcriptom of a given tumor sample. By this technique not only known or suspected regions of genomes can be inspected but even more valuable, new regions of disregulation with chromosomal linkage can be identified. This is of value in other types of neoplasia or viral integration and chromosomal rearrangements. By SQL based database searches one can retrieve information on expression, qualitative value of a measurement (denoted by Affymetrix MAS 5.0 Software), expression values derived from other techniques than DNA-chip hybridization and chromosomal linkage.

## EXAMPLE 2

*Identification of the ARCHEON*

*a) Identification and localization of genes or gene probes (represented by the so called probe sets on Affymetrix arrays HG-U95A-E or HG-U133A-B) in their chromosomal context and order on the human genome.*

For identification of larger chromosomal changes or aberrations, as they have been described in detail above, a sufficient number of genes, transcripts or DNA-fragments is needed. The density of probes covering a chromosomal region is not necessarily limited to the transcribed genes, in case of the use of array based CGH but by utilizing RNA as probe material the density is given by the distance of genes on a chromosome. The DNA-microarrays provided by Affymetrix Inc. Do contain hitherto all transcripts from the known humane genome, which are be represented by 40.000 - 60.000 probe sets. By BLAST mapping and sorting the sequences of these short DNA-oligomers to the public available sequence of the human genome represented by the so called "golden path", available at the university of California in Santa Cruz or from the NCBI, a chromosomal display of the whole Transcriptome of a tissue specimen evolves. By graphical display of the individual chromosomal regions and color coding of over or under represented transcripts, compared to a reference transcriptome regions with DNA gains and losses can be identified.

*b) Quantification of gene copy numbers by combined IHC and quantitative PCR (PCR karyotyping) or directly by quantitative PCR*

Usually one to three paraffin-embedded tissue sections that are 5 µm thick are used to obtain genomic DNA from the samples. Tissue section are stained by colorimetric IHC after deparaffinization to identify regions containing disease associated cells. Stained regions are macrodissected with a scalpel and transferred into a microcentrifuge tube. The genomic DNA of these isolated tissue sections is extracted using appropriate buffers. The isolated DNA is then used for quantitative PCR with appropriate primers and probes. Optionally the IHC staining can be omitted and the genomic DNA can be directly isolated with or without prior deparaffinization with appropriate buffers. Those who are skilled in the art may vary the conditions and buffers described below to obtain equivalent results.
Reagents from DAKO (HercepTest Code No. K 5204) and TaKaRa were used (Biomedicals Cat.: 9091) according to the manufactures protocol.
It is convenient to prepare the following reagents prior to staining:

**Solution No. 7**

Epitope Retrieval Solution (Citrate buffer + antimicrobial agent) (10xconc.) 20 ml ad 200 ml aqua dest. (stable for 1month at 2-8°C )

**Solution No. 8**

Washing-buffer (Tris-HCl + antimicrobial agent) (10 x conc.) 30 ml ad 300 ml destilled water (stable for 1month at 2-8°C )

**Staining solution: DAB**

1 ml solution is sufficient for 10 slides. The solution were prepared immediately before usage.:
1 ml DAB buffer (Substrate Buffer solution, pH 7.5, containing $H_2O_2$, stabilizer, enhancers and an antimicrobial agent) + 1 drop (25-3 µl) DAB-Chromogen (3,3'-diaminobenzidine chromogen solution). This solution is stable for up to 5 days at 2-8°C. Precipitated substances do not influence the staining result. Additionally required are:2x approx. 100 ml Xylol, 2x approx. 100 ml Ethanol 100%, 2x Ethanol 95%, aqua dest. These solution can be used for up to 40 stainings. A water bath is required for the epitope retrieval step.

Staining procedure:

All reagents are pre-warmed to room temperature (20-25°C) prior to immuno-staining. Likewise all incubations were performed at room temperature. Except the epitope retrieval which is performed in at 95°C water bath. Between the steps excess of liquid is tapped off from the slides with lintless tissue (Kim Wipe).

Deparaffinization

Slides are placed in a xylene bath and incubated for 5 minutes. The bath is changed and the step repeated once. Excess of liquid is tapped off and the slides are placed in absolute ethanol for 3 minutes. The bath is changed and the step repeated once. Excess of liquid is tapped off and the slides are placed in 95% ethanol for 3 minutes. The bath is changed and the step repeated once. Excess of liquid is tapped off and the slides are placed in distilled water for a minimum of 30 seconds.

Epitope Retrival

Staining jars are filled with with diluted epitope retrieval solution and preheated in a water bath at 95°C. The deparaffinized sections are immersed into the preheated solution in the staining jars and incubated for 40 minutes at 95°C. The entire jar is removed from the water bath and allowed to cool down at room temperature for 20 minutes. The epitope retrieval solution is decanted, the sections are rinsed in distilled water and finally soaked in wash buffer for 5 minutes.

Peroxidase Blocking:

Excess of buffer is tapped off and the tissue section encircled with a DAKO pen. The specimen is covered with 3 drops (100 µl) Peroxidase-Blocking solution and incubated for 5 minutes. The slides are rinsed in distilled water and placed into a fresh washing buffer bath.

Antibody Incubation

Excess of liquid is tapped off and the specimen are covered with 3 drops (100 µl) of Anti-Her-2/neu reagent (Rabbit Anti-Human Her2 Protein in 0.05 mol/L Tris/HCl, 0.1 mol/L NaCl, 15 mmol/L pH7.2 $NaN_3$ containing stabilizing protein) or negative control reagent (= IGG fraction of normal rabbit serum at an equivalent protein concentration as the Her2 Ab). After 30 minutes of incubation the slide is rinsed in water and placed into a fresh water bath.

Visualization

Excess of liquid is tapped off and the specimen are covered with 3 drops (100 µl) of visualization reagent. After 30 minutes of incubation the slide is rinsed in water and placed into a fresh water bath. Excess of liquid is tapped off and the specimen are covered with 3 drops (100 µl) of Substrate-Chromogen solution (DAB) for 10 minutes. After rinsing the specimen with distilled water, photographs are taken with a conventional Olympus microscope to document the staining intensity and tumor regions within the specimen. Optionally a counterstain with hematoxylin was performed.

DNA extraction

The whole specimens or dissected subregions are transferred into a microcentrifuge tubes. Optionally a small amount (10µl) of preheated TaKaRa solution (DEXPAT™) is preheated and placed onto the specimen to facilitate sample transfer with a scalpel. 50 to 150 µl of TaKaRa solution were added to the samples depending on the size of the tissue sample selected. The sample are incubated at 100°C for 10 minutes in a block heater, followed by centrifugation at 12.000 rpm in a microcentrifuge. The supernatant is collected using a micropet and placed in a separate microcentrifuge tube. If no deparaffinization step has been undertaken one has to be sure not to withdraw tissue debris and resin. Genomic DNA left in the pellet can be collected by adding resin-free TaKaRa buffer and an additional heating and centrifugation step. Samples are stored at -20°C.

Genomic DNA from different tumor cell lines (MCF-7, BT-20, BT-474, SKBR-3, AU-565, UACC-812, UACC-893, HCC-1008, HCC-2157, HCC-1954, HCC-2218, HCC-1937, HCC1599, SW480), or from lymphocytes is prepared with the QIAamp® DNA Mini Kits or the QIAamp® DNA Blood Mini Kits according to the manufacturers protocol. Usually between Ing up to 1µg DNA is used per reaction.

Quantitative PCR

To measure the gene copy number of the genes within the patient samples the respective primer/probes (see table below) are prepared by mixing 25 µl of the 100 µM stock solution "Upper Primer", 25 µl of the 100 µM stock solution "Lower Primer" with 12,5 µl of the 100 µM stock solution Taq Man Probe (Quencher Tamra) and adjusted to 500 µl with aqua dest. For each reaction 1,25 µl DNA-Extract of the patient samples or 1,25 µl DNA from the cell lines were mixed with 8,75 µl nuclease-free water and added to one well of a 96 Well-Optical Reaction Plate (Applied Biosystems Part No. 4306737). 1,5 µl Primer/Probe mix, 12, µl Taq Man Universal-PCR Mix (2x) (Applied Biosystems Part No. 4318157) and 1 µl Water are then added. The 96 well plates are closed with 8 Caps/Strips (Applied Biosystems Part Number 4323032) and centrifuged for 3 minutes. Measurements of the PCR reaction are done according to the instructions of the manufacturer with a TaqMan 7900 HT from Applied Biosystems (No. 20114) under appropriate conditions (2 min. 50°C, 10 min. 95°C, 0.15mm. 95°C, 1 min. 60°C; 40 cycles). SoftwareSDS 2.0 from Applied Biosysrtems is used according to the respective instructions. CT-values are then further analyzed with appropriate software (Microsoft Excel™).

## REFERENCES

| *Patents cited* | |
|---|---|
| U.S. Pat. No. 4,843,155 | Chomczynski, P. |
| U.S. Pat. No. 5,262,31 | Liang, P., and Pardee, A. B., 1993 |
| U.S. Pat. No. 4,683,202 | Mullis, K. B., 1987 |
| U.S. Pat. No. 5,593,839 | |
| U.S. Pat. No. 5,578,832 | |
| U.S. Pat. No. 5,556,752 | |
| U.S. Pat. No. 5,631,734 | |
| U.S. Pat. No. 5,599,695 | |
| U.S. Pat. No. 4,683,195 | |
| U.S. Pat. No. 5,498,531 | |
| U.S. Pat. No. 5,714,331 | |
| U.S. Pat. No. 5,641,673 | Haseloffet al., |
| U.S. Pat. No. 5,223,409 | Lander, E., |
| U.S. Pat. No. 5,976,813 | Beutel et al. |
| U.S. Pat. No. 5,283,317 | |
| U.S. Pat No. 6,203,987 | |
| U.S. Pat.No. 6,379,895 | |
| WO 97/29212 | |
| WO 97/27317 | |
| WO 95/22058 | |
| WO 99/12826 | |
| WO 97/02357 | |
| WO 94/13804 | |
| WO 94/10300 | |
| WO 97/14028 | |
| WO 99/52708 | |
| EP 0 785 280 | |
| EP 0 799 897 | |
| EP 0 728 520 | |
| EP 0 721 016 | |
| EP 0 321 201 | |

GB2188638B

Publications cited

(1) Gusterson et al., Journal of Clinical Oncology 10, 1049-1056, 1992
(2) Achuthan et al., Cancer Genet Cytogenet. 130:166-72, 2001
(3) Tomasetto et al., FEBS Lett. 373: 245-249, 1995
(4) Pragnell et al., FEBS Lett. 291: 253-258, 1991
(5) Nakamichi et al. 1986
(6) Feo et al., Proc. Nat. Acad. Sci. 86: 6691-6695, 1989
(7) Davies et al.,. Proc. Nat. Acad. Sci. 86: 6691-6695, 1989
(8) Lee et al., Molec. Endocr. 9: 243-254, 1995
(9) Drane et al., Oncogene 15: 3013-3024, 1997
(10) Zhu et al., J. Biol. Chem. 272: 25500-25506, 1997
(11) Yuan et al., Proc. Nat. Acad. Sci. 95: 7939-7944, 1998

(12) Zhu et al., Proc. Nat. Acad. Sci. 96: 10848-10853, 1999
(13) Lee et al., Science 268: 836-844, 1995
(14) McCormick et al., Molec. Cell. Biol. 16: 5792-5800, 1996
(15) Tamimi et al., Genomics 40: 355-357, 1997
(16) Valle et al., *FEBS Lett.* 415: 163-168, 1997
(17) Kaneda et al., J. Biol. Chem. 263: 7672-7677, 1988
(18) Hoehe et al., Hum. Molec. Genet. 1:175-178, 1992
(19) Yang-Feng et al., Abstract Cytogenet. Cell Genet. 40: 784, 1985
(20) Coussens et al., Science 230: 1132-1139, 1985
(21) van de Vijver et al., New Eng. J. Med. 319: 1239-1245, 1988
(22) Slamon et al., Science 244: 707-712, 1989
(23) Fukushige et al., Res. Commun. 134: 477-483, 1986
(24) Kaneko et al., Jpn. J. Cancer Res. 78: 16-19, 1987
(25) Di Fiore et al., Science 237: 178-182, 1987
(26) Popescu et al., Genomics 4: 362-366, 1989
(27) Qiu et al., Nature 393: 83-85, 1998
(28) Yu et al., Molec. Cell 2: 581-591, 1998
(29) Doherty et al., Proc. Nat. Acad. Sci. 96: 10869-10874, 1999
(30) Slamon et al., New Eng. J. Med. 344: 783-792, 2001
(31) Margolis et al., J. Clin. Invest. 102: 821-827, 1998
(32) Tanaka et al., J. Clin. Invest. 102: 821-827, 1998
(33) Dong et al., J. Biol. Chem. 272: 29104-29112, 1997
(34) Stein et al., EMBO J.13:1331-40, 1994
(35) Nagata et al., Nature 319: 415-418, 1986
(36) Le Beau et al., Leukemia 1: 795-799, 1987,
(37) Jansson et al., EMBO J. 2: 561-565, 1983
(38) Thompson et al., Science 237:1610-1614, 1987
(39) Nakai et al., Proc. Nat. Acad. Sci. 85: 2781-2785, 1988
(40) Miyajima et al., Cell 57: 31-39, 1989
(41) Debuire et al., Science 224: 1456-1459, 1984
(42) Petkovich et al., Nature 330: 444-450, 1987
(43) Mattei et al., Hum. Genet. 80: 186-188, 1988.
(44) Williams et al., Molec. Cell. Biol. 18: 2758-2767, 1998
(45) Saha et al, Molec. Cell. Biol. 18: 2758-2767, 1998
(46) Yan et al., Proc. Nat. Acad. Sci. 95: 3603-3608, 1998
(47) Singh et al., Nucleic Acids Res. 16: 3919-3929, 1988
(48) Tsai-Pflugfelder et al., Proc. Nat. Acad. Sci. 85: 7177-7181, 1988
(49) Chung et al., Proc. Nat. Acad. Sci. 86: 9431-9435, 1989
(50) Lang et al., Gene 221: 255-266, 1998
(51) Keith et al., Genes Chromosomes Cancer 4: 169-175, 1992
(52) Kingsmore et al., Mammalian Genome 4: 288-289, 1993
(53) Watt et al., Biochem. J. 303: 681-695, 1994
(54) Kiefer et al., J. Biol. Chem. 267: 12692-12699, 1992
(55) Shimasaki et al., Molec. Endocr. 4: 1451-1458, 1990
(56) Zazzi et al., Genomics 49: 401-410, 1998
(57) Bajalica et al., Hum. Genet. 89: 234-236, 1992
(58) Tonin et al., Genomics 18: 414-417, 1993
(59) Birkenbach et al., J. Virol. 67: 2209-2220, 1993
(60) Wang et al., Proc. Nat. Acad. Sci. 95: 492-498, 1998
(61) Klochendler-Yeivin et al., Curr Opin Genet Dev 121:73-9,2002
(62) Ring et al., Genomics 51:140-3, 1998
(63) Darmon et al., Molec. Biol. Rep. 12: 277-283, 1987
(64) Zhou et al., J. Biol. Chem. 263: 15584-15589, 1988
(65) Korge et al., Proc. Nat. Acad. Sci. 89: 910-914, 1992
(66) Lessin et al., J. Invest. Derm. 91: 572-578, 1988
(67) Romano et al., Cytogenet. Cell Genet. 58: 2009-2010, 1991.
(68) Fuchs et al., Proc. Nat. Acad. Sci. 89: 6906-6910, 1992
(69) Rogaev et al., Nature Genet. 5: 158-162, 1993

(70) Liu et al., Curr. Eye Res. 12: 963-974, 1993
(71) Nishida et al., Invest. Ophthal. Vis. Sci. 37: 1800-1809, 1996
(72) Nishida et al., Am. J. Hum. Genet. 61: 1268-1275, 1997
(73) Meesmann and Wilke, 1939
(74) Corden et al., Exp. Eye Res. 70: 41-49, 2000
(75) van de Vijver et al., Mol Cell Biol 7, 2091-23, 1987
(76) Offlerdinger et al., Biochem Biophys Res Comm 251, 907-13, 1988
(77) Sambrook et al., MOLECULAR CLONING: A LABORATORY MANUAL, 2d ed., 1989
(78) Ausubel et al., CURRENT PROTOCOLS IN MOLECULAR BIOLOGY, John Wiley & Sons, New York, N.Y., 1989.
(79) Tedder, T. F. et al., Proc. Natl. Acad. Sci. U.S.A. 85:208-212, 1988
(80) Hedrick, S. M. et al., Nature 308:149-153, 1984
(81) Lee, S. W. et al., Proc. Natl. Acad. Sci. U.S.A. 88:4225, 1984
(82) Sarkar, PCR Methods Applic. 2, 318-322, 1993
(83) Triglia et al., Nucleic Acids Res. 16, 81-86, 1988
(84) Lagerstrom et al., PCR Methods Applic. 1, 111-119, 1991
(85) Copeland & Jenkins, Trends in Genetics 7: 113-118, 1991
(86) Cohen, et al., Nature 366: 698-701, 1993
(87) Bonner et al., J. Mol. Biol. 81, 123 1973
(88) Bolton and McCarthy, Proc. Natl. Acad. Sci. U.S.A. 48, 1390 1962
(89) Plump et al., Cell 71: 343-353, 1992
(90) Altschul et al., Bull. Math. Bio. 48:603, 1986,
(91) Henikoff & Henikoff, Proc. Natl. Acad. Sci. USA 89:10915, 1992
(92) Pearson & Lipman, Proc. Nat'1 Acad. Sci. USA 85:2444, 1988
(93) Pearson et al., Meth. Enzymol. 183:63, 1990
(94) Needleman & Wunsch, J. Mol. Biol.48:444, 1970
(95) Sellers, SIAM J. Appl. Math.26:787, 1974
(96) Takamatsu, EMBO J. 6, 307-311, 1987
(97) Coruzzi et al., EMBO J. 3, 1671-1680, 1984
(98) Broglie et al., Science 224, 838-843, 1984
(99) Winter et al., Results Probl. Cell Differ. 17, 85-105, 1991
(100) Engelhard et al., Proc. Nat. Acad. Sci. 91, 3224-3227, 1994
(101) Logan & Shenk, Proc. Natl. Acad. Sci. 81, 3655-3659, 1984
(102) Scharf et al., Results Probl. Cell Differ. 20, 125-162, 1994
(103) Freshney R.I., ed., ANIMAL CELL CULTURE , 1986
(104) Wigler et al., Cell 11, 223-232, 1977
(105) Lowy et al., Cell 22, 817-823, 1980
(106) Wigler et al., Proc. Natl. Acad. Sci. 77, 3567-3570, 1980
(107) Colbere-Garapin et al., J. Mol. Biol. 150, 114, 1981
(108) Hartman & Mulligan, Proc. Natl. Acad. Sci. 85, 8047-8051, 1988
(109) Rhodes et al., Methods Mol. Biol. 55, 121-131, 1995
(110) Hampton et al., SEROLOGICAL METHODS: A LABORATORY MANUAL, APS Press, St. Paul, Minn., 1990
(111) Maddox et al., J. Exp. Med. 158, 1211-1216, 1983
(112) Porath et al., Prot. Exp. Purif. 3, 263-281, 1992
(113) Kroll et al., DNA Cell Biol. 12, 441-453, 1993
(114) Caruthers et al., Nucl. Acids Res. Symp. Ser. 215-223, 1980
(115) Horn et al. Nucl. Acids Res. Symp. Ser. 225-232, 1980
(116) Merrifield, J. Am. Chem. Soc. 85, 2149-2154, 1963
(117) Roberge et al., Science 269, 202-204, 1995
(118) Creighton, PROTEINS: STRUCTURES AND MOLECULAR PRINCIPLES, WH and Co., New York, N.Y., 1983
(119) Cronin et al., Human Mutation 7:244, 1996
(120) Landegran et al., Science 241:1077-1080, 1988
(121) Nakazawa et al., PNAS 91:360-364, 1994
(122) Abravaya et al., *Nuc Acid Res* 23:675-682, 1995
(123) Guatelli, J.C. *et* al., Proc. Natl. Acad. Sci. USA 87:1874-1878, 1990
(124) Kwoh, D.Y. et al., Proc. Natl. Acad. Sci. USA 86:1173-1177, 1989
(125) Lizardi, P.M. *et* al., Bio/Technology 6:1197, 1988
(126) Brown, Meth. Mol. Biol. 20, 18, 1994

(127) Sonveaux, Meth. Mol. Biol. 26, 1-72, 1994
(128) Uhlmann et al., Chem. Rev. 90, 543-583, 1990
(129) Gee et al., in Huber & Carr, MOLECULAR AND IMMUNOLOGIC APPROACHES, Publishing Co., Mt. Kisco, N.Y., 1994
(130) Agrawal et al., Trends Biotechnol. 10, 152-158, 1992
(131) Uhlmann et al., Tetrahedron. Lett. 215, 3539-3542, 1987
(132) Cech, Science 236, 1532-1539, 1987
(133) Cech, Ann. Rev. Biochem. 59, 543-568, 1990
(134) Couture & Stinchcomb, Trends Genet. 12, 510-515, 1996
(135) Haseloffet al. Nature 334, 585-591, 1988
(136) Kohler et al., Nature 256,495-497,1985
(137) Kozbor et al., J. Immunol. Methods 81, 3142, 1985
(138) Cote et al., Proc. Natl. Acad. Sci. 80, 2026-2030, 1983
(139) Cole et al., Mol. Cell Biol. 62, 109-120, 1984
(140) Morrison et al., Proc. Natl. Acad. Sci. 81, 6851-6855, 1984
(141) Neuberger et al., Nature 312, 604-608, 1984
(142) Takeda et al., Nature 314, 452-454, 1985
(143) Burton, Proc. Natl. Acad. Sci. 88, 11120-11123, 1991
(144) Thirion et al., Eur. J. Cancer Prev. 5, 507-11, 1996
(145) Coloma & Morrison, Nat. Biotechnol. 15, 159-63, 1997
(146) Mallender & Voss, J. Biol. Chem. 269, 199-206, 1994
(147) Verhaar et al., Int. J. Cancer 61, 497-501, 1995
(148) Nicholls et al., J. Immunol. Meth. 165, 81-91, 1993
(149) Orlandi et al., Proc. Natl. Acad. Sci. 86, 3833-3837, 1989
(150) Winter et al., Nature 349, 293-299, 1991
(151) Lam, Anticancer Drug Des. 12, 145, 1997
(152) DeWitt et al., Proc. Natl. Acad. Sci. U.S.A. 90, 6909, 1993
(153) Erb et al. Proc. Natl. Acad. Sci. U.S.A. 91, 11422, 1994
(154) Zuckermann et al., J. Med. Chem. 37, 2678, 1994
(155) Cho et al., Science 261, 1303, 1993
(156) Carell et al., Angew. Chem. Int. Ed. Engl. 33, 2059 & 2061, 1994
(157) Gallop et al., J. Med. Chem. 37, 1233, 1994
(158) Houghten, BioTechniques 13, 412-421, 1992
(159) Lam, Nature 354, 8284, 1991
(160) Fodor, Nature 364, 555-556, 1993
(161) Cull et al., Proc. Natl. Acad. Sci. U.S.A. 89, 1865-1869, 1992
(162) Scott & Smith, Science 249, 386-390, 1990
(163) Devlin, Science 249, 404-406, 1990
(164) Cwirla et al., Proc. Natl. Acad. Sci. 97, 6378-6382, 1990
(165) Felici, J. Mol. Biol. 222, 301-310, 1991
(166) Jayawickreme et al., Proc. Natl. Acad. Sci. U.S.A. 19, 1614-1618, 1994
(167) Chelsky, Strategies for Screening Combinatorial Libraries 1995
(168) Salmon et al., Molecular Diversity 2, 57-63, 1996
(169) McConnell et al., Science 257, 1906-1912, 1992
(170) Sjolander & Urbaniczky, Anal. Chem. 63, 2338-2345, 1991
(171) Szabo et al., Curr. Opin. Struct. Biol. 5, 699-705, 1995
(172) Zervos et al., Cell 72, 223-232, 1993
(173) Madura et al., J. Biol. Chem. 268, 12046-12054, 1993
(174) Bartel et al., BioTechniques 14, 920-924, 1993
(175) Iwabuchi et al., Oncogene 8, 1693-1696, 1993
(176) Findeis et al. Trends in Biotechnol. 11, 202-205, 1993
(177) Chiou et al., GENE THERAPEUTICS: METHODS AND APPLICATIONS OF DIRECT GENE TRANSFER J. A. Wolf ed., 1994
(178) Wu & Wu, J. Biol. Chem. 263, 621-24, 1988
(179) Wu et al., J. Biol. Chem. 269, 542-46, 1994
(180) Zenke et al., Proc. Natl. Acad. Sci. U.S.A. 87, 3655-59, 1990
(181) Wu et al., J. Biol. Chem. 266, 338-42, 1991
(182) REMINGTON'S PHARMACEUTICAL SCIENCES Maack Publishing Co., Easton, Pa.

(183) Hille, Excitable Membranes, Sunderland, MA, Sinauer Associates, Inc.

Table 1

| DNA SEQ ID NO: | Protein SEQ ID NO: | Genbank ID | Unigene_v133_ID | Locus Link ID | Gene Name |
|---|---|---|---|---|---|
| 1 | 27 | NM_006148.1 | 75080 | 3927 | LASP1 |
| 2 | 28 | NM_000723.1 | 635 | 782 | CACNB1 |
| 3 | 29 | NM_000981.1 | 252723 | 6143 | RPL19RPL19 |
| 4 | 30 | Y13467 | 15589 | 5469 | PPARGBP |
| 5 | 31 | NM_016507.1 | 123073 | | CrkRS |
| 6 | 32 | AB021742.1 | 322431 | 4761 | NEUROD2 |
| 7 | 33 | NM_006804.1 | 77628 | 10948 | MLN64 |
| 8 | 34 | NM_003673.1 | 111110 | 8557 | TELETHONIN |
| 9 | 35 | NM_002686.1 I | 1892 | 5409 | PNMT |
| 10 | 36 | X03363.1 | 323910 | 2064 | ERBB2 |
| 11 | 37 | AB008790.1 | 86859 | 2886 | GRB7 |
| 12 | 38 | NM_002809.1 | 9736 | 5709 | PSMD3 |
| 13 | 39 | NM_000759.1 | 2233 | 1440 | GCSFG |
| 14 | 40 | AI023317 | 23106 | 9862 | KIAA0130 |
| 15 | 41 | X55005 | | 7067 | c-erbA-1 |
| 16 | 42 | X72631 | 211606 | 9572 | NRID1 |
| 17 | 43 | NM_007359.1 | 83422 | 22794 | MLN51 |
| 18 | 44 | U77949.1 | 69563 | 990 | CDC6 |
| 19 | 45 | U41742.1 | | 5914 | RARA |
| 20 | 46 | NM_001067.1 | 156346 | 7153 | TOP2A |
| 21 | 47 | NM_001552.1 | 1516 | | IGFBP4 |
| 22 | 48 | NM_001838.1 | 1652 | | CCR7 EBI1 |
| 23 | 49 | NM_003079.1 | 332848 | 6605 | SMARCE1 BAF57 |
| 24 | 50 | X14487 | 99936 | 3858 | KRT10 |
| 25 | 51 | NM_000223.1 | 66739 | | KRT12 |
| 26 | 52 | NM_002279.2 | 32950 | 3884 | hHKa3-II |
| 53 | 76 | NM_005937 | 349196 | 4302 | MLLT6 |
| 54 | 77 | XM_008147 | 184669 | 7703 | ZNF144 |
| 55 | 78 | NM_138687 | 432736 | 8396 | PIP5K2B |
| 56 | 79 | NM_020405 | 125036 | 57125 | TEM7 |
| 57 | 80 | XM_012694 | 258579 | 22806 | ZNFN1A3 |
| 58 | 81 | XM_085731 | 13996 | 147179 | WIRE |
| 59 | 82 | NM_002795 | 82793 | 5691 | PSMB3 |
| 60 | 83 | NM_033419 | 91668 | 93210 | MGC9753 Variant a |

Table 1 (continued)

| DNA SEQ ID NO: | Protein SEQ ID NO: | Genbank ID | Unigene_v133_ID | Locus Link ID | Gene Name |
|---|---|---|---|---|---|
| 61 | 84 | | | | MGC9753 Variant c |
| 62 | 85 | | | | MGC9753 Variant d |
| 63 | 86 | | | | MGC9753 Variant e |
| 64 | 87 | | | | MGC9753 Variant g |
| 65 | 88 | | | | MGC9753 Variant h |
| 66 | 89 | | | | MGC9753 Variant i |
| 67 | 90 | AF395708 | 374824 | 94103 | ORMDL3 |
| 68 | 91 | NM_032875 | 194498 | 84961 | MGC15482 |
| 69 | 92 | NM_032192 | 286192 | 84152 | PPP1R1B |
| 70 | 93 | NM_032339 | 333526 | 84299 | MGC14832 |
| 71 | 94 | NM_057555 | 12101 | 51242 | LOC51242 |
| 72 | 95 | NM_017748 | 8928 | 54883 | FLJ20291 |
| 73 | 96 | NM_018530 | 19054 | 55876 | Pro2521 |
| 74 | 97 | NM_016339 | 118562 | 51195 | Link-GEFII |
| 75 | 98 | NM_032865 | 294022 | 84951 | CTEN |

Table 2

| DNA SEQ ID NO: | Gene description |
|---|---|
| 1 | Member of a subfamily of LIM proteins that contains a LIM domain and an SH3 (Src homology region 3) domain |
| 2 | Beta 1 subunit of a voltage-dependent calcium channel (dihydropyridine receptor), involved in coupling of excitation and contraction in muscle, also acts as a calcium channel in various other tissues |
| 3 | Ribosomal protein L19, component of the large 60S ribosomal subunit |
| 4 | Protein with similarity to nuclear receptor-interacting proteins; binds and co-activates the nuclear receptors PPARalpha (PPARA), RARalpha (RARA), RXR, TRbeta1, and VDR |
| 5 | we26e0 CDC2-related protein kinase 7 |
| 6 | Neurogenic differentiation, a basic-helix-loop-helix transcription factor that mediates neuronal differentiation |
| 7 | Protein that is overexpressed in malignant tissues, contains a putative trans-membrane region and a StAR Homology Domain (SHD), may function in steroidogenesis and contribute to tumor progression |
| 8 | Telethonin, a sarcomeric protein specifically expressed in skeletal and heart muscle, caps titin (TTN) and is important for structural integrity of the sarcomere |

Table 2 (continued)

| DNA SEQ ID NO: | Gene description |
|---|---|
| 9 | Phenylethanolamine N-methyltransferase, acts in catecholamine biosynthesis to convert norepinephrine to epinephrine |
| 10 | Tyrosine kinase receptor that has similarity to the EGF receptor, a critical component of IL-6 signaling through the MAP kinase pathway, overexpression associated with prostate, ovary and breast cancer |
| 11 | Growth factor receptor-bound protein, an SH2 domain-containing protein that has isoforms which may have a role in cell invasion and metastatic progression of esophageal carcinomas |
| 12 | Non-ATPase subunit of the 26S proteasome (prosome, macropain) |
| 13 | Granulocyte colony stimulating factor, a glycoprotein that regulates growth, differentiation, and survival of neutrophilic granulocytes |
| 14 | Member of the Vitamin D Receptor Interacting Protein co-activator complex, has strong similarity to thyroid hormone receptor-associated protein (murine Trap 100) which function as a transcriptional coregulator |
| 15 | Thyroid hormone receptor alpha, a high affinity receptor for thyroid hormone that activates transcription; homologous to avian erythroblastic leukemia virus oncogene |
| 16 | encoding Rev-ErbAalp nuclear receptor subfamily 1, group D, member 1 |
| 17 | Protein that is overexpressed in breast carcinomas |
| 18 | Protein which interacts with the DNA replication proteins PCNA and Orc1, translocates from the nucleus following onset of S phase; S. cerevisiae homolog Cdc6p is required for initiation of S phase |
| 19 | Retinoic acid receptor alpha, binds retinoic acid and stimulates transcription in a ligand-dependent manner |
| 20 | DNA topoisomerase II alpha, member of a family of proteins that relieves torsional stress created by DNA replication, transcription, and cell division; |
| 21 | Insulin-like growth factor binding protein, the major IGFBP of osteoblast-like cells, binds IGFI and IGF2 and inhibits their effects on promoting DNA and glycogen synthesis in osteoblastic cells |
| 22 | HUMEBI103 G protein-coupled receptor (EBI 1) gene exon 3 chemokine (C-C motif) receptor 7 G protein-coupled receptor |
| 23 | Protein with an HMG 1/2 DNA-binding domain that is subunit of the SNF/SWI complex associated with the nuclear matrix and implicated in regulation of transcription by affecting chromatin structure |
| 24 | Keratin 10, a type I keratin that is a component of intermediate filaments and is expressed in terminally differentiated epidermal cells; mutation of the corresponding gene causes epidermolytic hyperkeratosis |
| 25 | Keratin 12, a component of intermediate filaments in corneal epithelial cells; mutation of the corresponding gene causes Meesmann corneal dystrophy |
| 26 | Hair keratin 3B, a type I keratin that is a member of a family of structural proteins that form intermediate filaments |
| 53 | MLLT6 Myeloid/lymphoid or mixed-lineage leukemia (trithorax homolog, Drosophila); translocated to, 6 |
| 54 | zinc finger protein 144 (Mel-18) |
| 55 | phosphatidylinositol-4-phosphate 5-kinase type II beta isoform a |
| 56 | tumor endothelial marker 7 precursor |

Table 2 (continued)

| DNA SEQ ID NO: | Gene description |
|---|---|
| 57 | zinc finger protein, subfamily 1A, 3 |
| 58 | WASP-binding protein putative cr16 and wip like protein similar to Wiskott-Aldrich syndrome protein |
| 59 | proteasome (prosome, macropain) subunit, beta type, 3 |
| 60 | Predicted |
| 67 | ORM1-like 3 (S. cerevisiae) |
| 68 | F-box domain A Receptor for Ubiquitination Targets |
| 69 | protein phosphatase 1, regulatory (inhibitor) subunit 1B (dopamine and cAMP regulated phosphoprotein, DARPP-32) |
| 70 | Predicted Protein |
| 71 | Predicted Protein |
| 72 | Predicted Protein |
| 73 | Predicted Protein |
| 74 | Link-GEFII: Link guanine nucleotide exchange factor II |
| 75 | C-terminal tensin-like |

**Table 3**

| DNA SEQ ID NO: | Gene function | Subcellular localization |
|---|---|---|
| 1 | SH3/SH2 adapter protein | - |
| | voltage-gated calcium channel membrane fraction Channel [passive transporter] | Plasma membrane |
| 3 | RNA binding structural protein of ribosome protein biosynthesis | Cytoplasm |
| 4 | transcription co-activator nucleus Pol II transcription | Nucleus |
| 5 | - | - |
| 6 | transcription factor transcription regulation from Pol II promoter neurogenesis | - |
| 7 | mitochondrial transport steroid and lipid metabolism | Cytoplasm |
| 8 | structural protein of muscle sarcomere alignment | Cytoplasm |
| 9 | phenylethanolamine N-methyltransferase Transferase | - |
| 10 | Neu/ErbB-2 receptor receptor signaling protein tyrosine kinase | Plasma membrane |
| 11 | SH3/SH2 adapter protein EGF receptor signaling pathway | Cytoplasm |
| 12 | 26S proteasome Protein degradation Proteasome subunit | Cytoplasm |
| 13 | developmental processes positive control of cell proliferation | Extracellular space |
| 14 | fatty acid omega-hydroxylase fatty acid omega-hydroxylase | - |
| 15 | DNA-binding protein Transcription factor | Nucleus |
| 16 | steroid hormone receptor transcription co-repressor | Nucleus |
| 17 | - | - |
| 18 | nucleotide binding cell cycle regulator DNA replication checkpoint regulation of CDK activity | nucleus |
| 19 | retinoic acid receptor transcription co-activator transcription factor | nucleus |
| 20 | DNA binding DNA topoisomerase (ATP-hydrolyzing) | nucleus |
| 21 | skeletal development DNA metabolism signal transduction cell proliferation | |
| 22 | | plasma membrane |
| 23 | chromatin binding transcription co-activator nucleosome disassembly transcription | nucleus nuclear chromosome |
| 24 | Cell structure Cytoskeletal Epidermal Development and Maintenance | cytoplasm |
| 25 | structural protein vision cell shape and cell size control intermediate filament | cytoplasm |
| 26 | cell shape and cell size control Cell structure | cytoplasm |
| 53 | leucine-zipper containing fusion | - |

**Table 3** (continued)

| DNA SEQ ID NO: | Gene function | Subcellular localization |
|---|---|---|
| 54 | | - |
| 55 | Tumor endothelial marker 7 precursor; may be involved in angiogenesis | - |
| 56 | Aiolos; DNA binding protein that may be a transcription factor; has strong similarity to murine Znfn1a3, contains zinc finger domain | - |
| 57 | The WASP-binding protein WIRE has a role in the regulation of the actin filament system downstream of the platelet-derived growth factor receptor | - |
| 58 | | - |
| 59 | | - |
| 60 | | - |
| 67 | | - |
| 68 | | - |
| 69 | Midbrain dopaminergic neurons play a critical role in multiple brain functions, and abnormal signaling through dopaminergic pathways has been implicated in several major neurologic and psychiatric disorders. One well-studied target for the actions of dopamine is DARPP32. | - |
| 70 | | - |
| 71 | | - |
| 72 | | - |
| 73 | | - |
| 74 | Brain-specific guanine nucleotide exchange factor; activates the ERK/MAP kinase cascade plus R-Ras and H-ras; activates targets through a Ca2+- and diacylglycerol-sensitive mechanism; active protein associates with membranes | - |
| 75 | C-terminal tensin-like Phosphotyrosine-binding domain, phosphotyrosine-interaction (PI) domain | - |

Table 4

| DNA SEQ ID NO: | Protein SEQ ID NO: | Gene Name | DBSNPID | Type | Codon | AA-Seq |
|---|---|---|---|---|---|---|
| 9 | 34 | ERBB2 | rs2230698 | coding-synon | TCA\|TCG | S\|S |
| 9 | 34 | ERBB2 | rs2230700 | noncoding | | |
| 9 | 34 | ERBB2 | rs1058808 | coding-nonsynon | CCC\|GCC | P\|A |
| 9 | 34 | ERBB2 | rs1801200 | noncoding | | |
| 9 | 34 | ERBB2 | rs903506 | noncoding | | |
| 9 | 34 | ERBB2 | rs2313170 | noncoding | | |
| 9 | 34 | ERBB2 | rs1136201 | coding-nonsynon | ATC\|GTC | I\|V |
| 9 | 34 | ERBB2 | rs2934968 | noncoding | | |
| 9 | 34 | ERBB2 | rs2172826 | noncoding | | |
| 9 | 34 | ERBB2 | rs1810132 | coding-nonsynon | ATC\|GTC | I\|V |
| 9 | 34 | ERBB2 | rs1801201 | noncoding | | |
| 14 | 39 | c-erbA-1 | rs2230702 | coding-synon | TCC\|TCT | S\|S |
| 14 | 39 | c-erbA-1 | rs2230701 | coding-synon | GCC\|GCT | A\|A |
| 14 | 39 | c-erbA-1 | rs1126503 | coding-nonsynon | ACC\|AGC | T\|S |
| 14 | 39 | c-erbA-1 | rs3471 | noncoding | | |
| 19 | 44 | TOP2A | rs13695 | noncoding | | |
| 19 | 44 | TOP2A | rs471692 | noncoding | | |
| 19 | 44 | TOP2A | rs558068 | noncoding | | |
| 19 | 44 | TOP2A | rs1064288 | noncoding | | |
| 19 | 44 | TOP2A | rs1061692 | coding-synon | GGA\|GGG | G\|G |
| 19 | 44 | TOP2A | rs520630 | noncoding | | |
| 19 | 44 | TOP2A | rs782774 | coding-nonsynon | AAT\|ATT\|ATT\|TTT | N\|I\|I\|F |
| 19 | 44 | TOP2A | rs565121 | noncoding | | |
| 19 | 44 | TOP2A | rs2586112 | noncoding | | |
| 19 | 44 | TOP2A | rs532299 | coding-nonsynon | TTT\|GTT | F\|V |
| 19 | 44 | TOP2A | rs2732786 | noncoding | | |
| 19 | 44 | TOP2A | rs1804539 | noncoding | | |
| 19 | 44 | TOP2A | rs1804538 | noncoding | | |
| 19 | 44 | TOP2A | rs1804537 | noncoding | | |
| 19 | 44 | TOP2A | rs1141364 | coding-synon | AAA\|AAG | K\|K |
| 23 | 48 | KRT10 | rs12231 | noncoding | | |
| 23 | 48 | KRT10 | rs1132259 | coding-nonsynon | CAT\|CGT | H\|R |
| 23 | 48 | KRT10 | rs1132257 | coding-synon | CTG\|TTG | L\|L |
| 23 | 48 | KRT10 | rs1132256 | coding-synon | GCC\|GCT | A\|A |
| 23 | 48 | KRT10 | rs1132255 | coding-synon | CTG\|TTG | L\|L |
| 23 | 48 | KRT10 | rs1132254 | coding-synon | GGC\|GGT | G\|G |
| 23 | 48 | KRT10 | rs1132252 | coding-synon | TTC\|TTT | F\|F |

Table 4 (continued)

| DNA SEQ ID NO: | Protein SEQ ID NO: | Gene Name | DBSNPID | Type | Codon | AA-Seq |
|---|---|---|---|---|---|---|
| 23 | 48 | KRT10 | rs1132268 | coding-nonsynon | CAG\|GAG | Q\|E |
| 23 | 48 | KRT10 | rs1132258 | coding-nonsynon | CGG\|TGG | R\|W |

**Table 5**

| PRIMER | SEQUENCE | |
|---|---|---|
| CACNB1 | FAM 5′ CCATATATAAAACCACTGTCCTGTCCTTTGTGGCT | 3′TAMRA |
| CACNB1FOR | 5′ CCCCCATCTGTCTGTCTATATTTGTC | 3′ |
| CACNB1REV | 5′ TGCCTACGCTGACGACTATGTG | 3′ |
| CDC6 | FAM 5′ TTTGGTTTTCTACAACTGTTGCTAT | 3′TAMRA |
| CDC6 FOR | 5′ GGGCTCCACACACCAGATG | 3′ |
| CDC6 REV | 5′ ACGCTCTGAGCACCCCTCTACA | 3′ |
| EBI1-1 | FAM 5′ TGTCACAGGGACTGAAAACCTCTCCTCATGT | 3′TAMRA |
| EBI1-1 FOR | 5′ CCCAAGGCCACGAGCTT | 3′ |
| EBI1-1 REV | 5′ TGTTGCTCTCTTAACGAATCGAAA | 3′ |
| EBI1-2 | FAM 5′ CTGGTCAAACAAACTCTCTGAACCCCTCC | 3′TAMRA |
| EBI1-2 FOR | 5′ TGGTGAGGAAAAGCGGACAT | 3′ |
| EBI1-2 REV | 5′ CTGGCTTGGAGGACAGTGAAG | 3′ |
| GCSF | FAM 5′ CCAAGCCCTCCCCATCCCATGTAT | 3′TAMRA |
| GCSF FOR | 5′ GAGGTGTCGTACCGCGTTCTA | 3′ |
| GCSF REV | 5′ CCGTTCTGCTCTTCCCTGTCT | 3′ |
| GRB7 | FAM 5′ CCAGACCCGCTTCACTGACCTGC | 3′TAMRA |
| GRB7 FOR | 5′ CGCCTGTACTTCAGCATGGA | 3′ |
| GRB7 REV | 5′ GCGGTTCAGCTGGTGGAA | 3′ |
| HKA3 | FAM 5′ ACCCCGAGGCATCACCACAAATCAT | 3′TAMRA |
| HKA3 FOR | 5′ AGTTCTGCCTCTCTGACAACCAT | 3′ |
| HKA3 REV | 5′ TAGGCTCAGAGTCAGACCCAAAC | 3′ |
| MLN50 | FAM 5′ CCCTCGTGGGCTTGTGCTCGG | 3′TAMRA |
| MLN50 FOR | 5′ AAGCCGCCAGTTCATCTTTTT | 3′ |
| MLN50 REV | 5′ CTTGTGGTTCAAGTCAAATGTTCAG | 3′ |
| MLN64-1 | FAM 5′ TCTGCCTGCGCTCTCGTCGGT | 3′TAMRA |
| MLN64-1 FOR | 5′ GGGCTGGGCACCTGACTT | 3′ |

**Table 5** (continued)

| PRIMER | SEQUENCE |
|---|---|
| MLN64-1REV | 5′ CCCAACAAGGGTCCCAGACT 3′ |
| MLN64-2 | FAM 5′ CGGCGCATTGAGCGGCG 3′TAMRA |
| MLN64-2 FOR | 5′ CCCAAGGGACTTCGTGAATG 3′ |
| MLN64-2REV | 5′ GGCGATCCCTGATGACAAGTA 3′ |
| PPARBP | FAM 5′ AGCACCAACTGTGAACCAGGTACAATGGC 3′TAMRA |
| PPARBP FOR | 5′ GAGGGAGGCTCTGCTTTGG 3′ |
| PPARBP REV | 5′ TCACAACTAGCGGGTGAGGAG 3′ |
| PSMD3 | FAM 5′ TGCAGAGGAACGGCGTGAGCG 3′TAMRA |
| PSMD3 FOR | 5′ TGAGGTTTCCTCCCAAATCGTA 3′ |
| PSMD3 REV | 5′ CAGCTCAAGGGAAGCTGTCATC 3′ |
| RAR | FAM 5′ CCCCCACATGTTCCCCAAGATGCT 3′TAMRA |
| RAR FOR | 5′ GGAGGCGCTAAAGGTCTACGT 3′ |
| RAR REV | 5′ TGATGCTTCGCAGGTCAGTAA 3′ |
| RPL23A | FAM 5′ CTCCTGCCCCTCCTAAAGCTGAAGCC 3′TAMRA |
| RPL23A FOR | 5′ GGACGCGTGGGCTTTTC 3′ |
| RPL23A REV | 5′ TGTGGCTGTGGACACCTTTC 3′ |
| RPL19 | FAM 5′ CCACAAGCTGAAGGCAGACAAGGCC 3′TAMRA |
| RPL19 FOR | 5′ GCGGATTCTCATGGAACACA 3′ |
| RPL19 REV | 5′ GGTCAGCCAGGAGCTTCTTG 3′ |
| NEUROD2 | FAM 5′ ACCACCTTGCGCAGGTTGTCCAG 3′TAMRA |
| NEUROD2 FOR | 5′ CGCATGCACGACCTGAAC 3′ |
| NEUROD2 REV | 5′ GTCTCGATCTTGGACAGCTTCTG 3′ |
| TELE TELETHONIN | FAM 5′ ACACTGTCCACACGGCCCGAGG 3′TAMRA |
| TELE TELETHONIN FOR | 5′ CTGGGCAGAATGGAAGGATCT 3′ |
| TELE TELETHONIN REV | 5′ GGGACTCTAGCAGACCCACACT 3′ |
| PENT PNMT | FAM 5′ CACCCACCTGGATTCCCTGTTC 3′TAMRA |
| PENT PNMT FOR | 5′ CCTTCAGACAGGCGTAGATGATG 3′ |
| PENT PNMT REV | 5′ GGGTATTATTTCTTTATTAGGTGCCACTT 3′ |

**Table 5** (continued)

| PRIMER | SEQUENCE |
|---|---|
| HER2/NEU;ERBB2 | FAM 5′ TTCCCTAAGGCTTTCAGTACCCAGGATCTG 3′TAMRA |
| HER2/NEU;ERBB FOR | 5′ CCAGCTTGGCCCTTTCCT 3′ |
| HER2/NEU;ERBB REV | 5′ GAATGGGTCGCTTTTGTTCTTAG 3′ |
| KIA0130 | FAM 5′ TCACGGACCTCAGCCTGCCCCT 3′TAMRA |
| KIA0130 FOR | 5′ TGGTGAAGGTGTCAGCCATGT 3′ |
| KIA0130 REV | 5′ TCAGAGTGCAGCAATGGCTTT 3′ |
| THRA | FAM 5′ ACCTCCTTCCCCAGCTCCCC 3′TAMRA |
| THRA FOR | 5′ GGCAACATCTTACTTGTCCTTTGA 3′ |
| THRA REV | 5′ CCAAGGAAGCACAGACAACTATTTC 3′ |
| MLN51 | FAM 5′ TCCTCCCTATCCATGGCACTAAACCACTTC 3′TAMRA |
| MLN51 FOR | 5′ TGGGCAAGGGCTCCTATCT 3′ |
| MLN51 REV | 5′ GTTACCCCTGGCAGACGTATG 3′ |
| TOP2A | FAM 5′ TGCCTCTGAGTCTGAATCTCCCAAAGAGAGA 3′TAMRA |
| TOP2A FOR | 5′ GAGTAGTTATGTGATTATTTCAGCTCTTGAC 3′ |
| TOP2A REV | 5′ TCAAATGTTGTCCCCGAGTCT 3′ |
| KRT10 | FAM 5′ CAGAAATTCGGAAGACAGAACTATTGTCATGCCT 3′TAMRA |
| KRT10 FOR | 5′ GATTAGTAACCCATAGCAGTTGAAGGT 3′ |
| KRT10 REV | 5′ ATTTACTGACGGTGGTCTGAACATAC 3′ |
| K12 KRT12 | FAM 5′ TGACAGACTCCAAATCACAAGCACAGTCAAC 3′TAMRA |
| K12 KRT12 FOR | 5′ TGATGGTTTGGAGGAAAGTTTATTT 3′ |
| K12 KRT12 REV | 5′ TTTGGTTGGGTCTTTAGAGGAATC 3′ |
| NR1D1 | FAM 5′ TGCCAACCATGCATCAGGTAGCCC 3′TAMRA |
| NR1D1 FOR | 5′ CAGCTCACCTGGCAACTTCA 3′ |
| NR1D1 REV | 5′ CCTGATTTTCCCAGCGATGT 3′ |
| HSERBT1 | FAM 5′ CGCCGCTCCCGGTTCTGCT 3′TAMRA |
| HSERBT FOR | 5′ TGGCCAAGCGTAAGCTGATT 3′ |
| HSERBT REV | 5′ GCTGCAGTGATCGGATCATCT 3′ |
| MLLT6 | FAM 5′ CACCATGGAGCCCATCGTGCTG 3′TAMRA |
| MLLT6 FOR | 5′ ATCCCCGAGGTGCAATTTG 3′ |

**Table 5** (continued)

| PRIMER | SEQUENCE | |
|---|---|---|
| MLLT6 REV | 5′ AGCGATCATGAGGCACGTACT | 3′ |
| ZNF144 | FAM 5′ CCTGCCAGAGATAGGAGACCCAGACAGCT | 3′TAMRA |
| ZNF144 FOR | 5′ ATCCCCCTGAGCCTTTTCA | 3′ |
| ZNF144 REV | 5′ CAGCCTCTGGTCCCACCAT | 3′ |
| PIP5K2B | FAM 5′ TGATCATCAATTCCAAACCTCTCCCGAA | 3′TAMRA |
| PIP5K2B FOR | 5′ CCCCATGGTGTTCCGAAAC | 3′ |
| PIP5K2B REV | 5′ TGCCAGGAGCCTCCATACC | 3′ |
| TEM7 | FAM 5′ CAGCCTTCTAAAACACAATGTATTCATGT | 3′TAMRA |
| TEM7 FOR | 5′ CCTGAACTTAATGGTAGAATTCAAAGATC | 3′ |
| TEM7 REV | 5′ TATTAACACTGAGAATCCATGCAGAGA | 3′ |
| ZNFN1A3 | FAM 5′ TATCTGGTCTCAGGGATTGCTCCTATGTATTCAGC | 3′TAMRA |
| ZNFN1A3 FOR | 5′ CACAGAGCCCTGCTGAAGTG | 3′ |
| ZNFN1A3 REV | 5′ GCGAGGTCATTGGTTTTTAGAAA | 3′ |
| WIRE | FAM 5′ CTGTGATCCGAAATGGTGCCAG | 3′TAMRA |
| WIRE FOR | 5′ CCGTCTCCACATCCAAACCT | 3′ |
| WIRE REV | 5′ ACCCATGCATTCGGTATGGT | 3′ |
| PSMB3 | FAM 5′ AGTGGCACCTGCGCCGAACAA | 3′TAMRA |
| PSMB3 FOR | 5′ CCCCATGGTGACTGATGACTT | 3′ |
| PSMB3 REV | 5′ CCAGAGGGACTCACACATTCC | 3′ |
| MGC9753 | FAM 5′ CCAGAAACTTTCCATCCCAAAGGCAGTCT | 3′TAMRA |
| MGC9753 FOR | 5′ CTGCCCCACAGGAATAGAATG | 3′ |
| MGC9753 REV | 5′ AAAAATCCAGTCTGCTTCAACCA | 3′ |
| ORMDL3 | FAM 5′ AGCTGCCCCAGCTCCACGGA | 3′TAMRA |
| ORMDL3 FOR | 5′ TCCCTGATGAGCGTGCTTATC | 3′ |
| ORMDL3 REV | 5′ TCTCAGTACTTATTGATTCCAAAAATCC | 3′ |
| MGC15482 | FAM 5′ TCCAGTGGAAGCAACCCCAGTGTTC | 3′TAMRA |
| MGC15482 FOR | 5′ CACTTCTAGAGCTACCGTGGAGTCT | 3′ |
| MGC15482 REV | 5′ CCCTCACTTTGTAACCCTTGCT | 3′ |
| PPP1R1B | FAM 5′ CAGCGTGGCGCAACAACCCA | 3′TAMRA |

**Table 5** (continued)

| PRIMER | SEQUENCE |
|---|---|
| PPP1R1B FOR | 5′ GGGATTGTTTCGCCACACATA 3′ |
| PPP1R1B REV | 5′ CCGATGTTAAGGCCCATAGC 3′ |
| MGC14832 | FAM 5′ TAAAATGTCCGGCCAACATGAGTTCCC 3′TAMRA |
| MGC14832 FOR | 5′ CGCAGTGCCTGGCACAT 3′ |
| MGC14832 REV | 5′ GACACCCCCTGACCTATGGA 3′ |
| LOC51242 | FAM 5′ CAGTGACCTCTCCCGTTCCCTTGGA 3′TAMRA |
| LOC51242 FOR | 5′ TGGGTCCCTGTGTCCTCTTC 3′ |
| LOC51242 REV | 5′ AGGGTCAGGAGGGAGAAAAC 3′ |
| FLJ20291 | FAM 5′ CCAGTGCCCACCCGTTAAAGAGTCAA 3′TAMRA |
| FLJ20291 FOR | 5′ TTGTGGGACACTCAGTAACTTTGG 3′ |
| FLJ20291 REV | 5′ ACAAGCACTCCCACCGAGAT 3′ |
| PRO2521 | FAM 5′ AGTCTGTCCTCACTGCCATCGCCA 3′TAMRA |
| PRO2521 FOR | 5′ AAGCCTCTGGGTTTTCCCTTT 3′ |
| PRO2521 REV | 5′ CCCACTGGTGACAGGATGGT 3′ |
| Link-GEFII | FAM 5′ CATCTGACATCTTTCCCGTGGAG 3′TAMRA |
| Link-GEFII FOR | 5′ CTTTGCACGATGTCTCAACCA 3′ |
| Link-GEFII REV | 5′ TTTCCCGTGGAGCAGGAA 3′ |
| CTEN | FAM 5′ CCGCCGCCTAATATGCAACATTAGGG 3′TAMRA |
| CTEN FOR | 5′ CGAGTATTCCAAAGCTGGTATCG 3′ |
| CTEN REV | 5′ ATCACAGAGAGATGGCCCTTATCT 3′ |

**Table 6**

| No. | ID | forward | reverse | PCR size (bp) | GB_ID |
|---|---|---|---|---|---|
| 1 | D17S946 | ACAGTCTATCAAGCAGAAAAATCCT | TGCCGTGCCAGAGAGA | 128-142 | Z24029 |
| 2 | D17S1181 | GACAACAGAGCGAGACTCCC | GCCCAGCCTGTCACTTATTC | 122 | - |
| 3 | D17S2026 | TGGTCATTCGACAACGAA | CAGCATTGGATGCAATCC | 171-318 | G05498 X53777 |
| 4 | D17S838 | CTCCAGAATCCAGACCATGA | AGGACAGTGTGTAGCCCTTC | 71-103 | Z51080 |
| 5 | D17S250 | GGAAGAATCAAATAGACAAT | GCTGGCCATATATATATTTAAACC | 151 | - |
| 6 | D17S1818 | CATAGGTATGTTCAGAAATGTGA | TGCCTACTGGAAACCAGA | 119-151 | Z52895 |
| 7 | D17S614 | AAGGGGAAGGGGCTTTCAAAGCT | NGGAGGTTGCAGTGAGCCAAGAT | 136 | L29873 |
| 8 | D17S2019 | CAAAAGCTTATGATGCTCAAACC | TTGTTTCCCTTTGACTTTCTGA | 151-152 | G07286 Z39013 |
| 9 | D17S608 | TAGGTTCACCTCTCATTTTCTTCAG | GTCTGGGTCTTTATGGNGCTTGTG | 136 | L29870 |
| 10 | D17S1655 | CGGACCAGAGTGTTCCATGG | GCATACAGCACCCTCTACCT | 240 | - |
| 11 | D17S2147 | AGGGGAGAATAAATAAAATCTGTGG | CAGGAGTGAGACACTCTCCATG | 138 | G15195 |
| 12 | D17S754 | TGGATTCACTGACTCAGCCTGC | GCGTGTCTGTCTCCATGTGTGC | 145 | - |
| 13 | D17S1814 | TCCCCAATGACGGTGATG | CTGGAGGTTGGCTTGTGGAT | 150-166 | Z52854 |
| 14 | D17S2007 | GGTCCCACGAATTTGCTG | CCACCCAGAAAAACAGGAGA | 102-103 | G07073 X03438 |
| 15 | D17S1246 | TCGATCTCCTGACCTTGTGA | TTGTCACCCCATTGCCTTTC | 115 | - |
| 16 | D17S1979 | CCTTGGATAGATTCAGCTCCC | CTTGTCCCTTCTCAATCCTCC | 199 | G11172 X55068 |
| 17 | D17S1984 | TTAAGCAAGGTTTTAATTAAGCTGC | GATTACAGTGCTCCCTCTCCC | 134 | G14779 T50487 |
| 18 | D17S1984 | GGTTTTAATTAAGCTGCATGGC | GATTACAGTGCTCCCTCTCCC | 126 | G11580 T50487 |
| 19 | D17S1867 | AGTTTGACACTGAGGCTTTG | TTTAGACTTGGTAACTGCCG | 94 | Z51301 |
| 20 | D17S1788 | TGCAGATGCCTAAGAACTTTTCAG | GCCATGATCTCCCAAAGCC | 156-168 | Z52160 |
| 21 | D17S1836 | TCGAGGTTATGGTGAGCC | AAACTGTGTGTGTCAAAGGATACT | 167-173 | Z53182 |
| 22 | D17S1787 | GCTGATCTGAAGCCAATGA | TACATGAAGGCATGGTCTG | 239-251 | Z52130 |
| 23 | D17S1660 | CTAATATAATCCTGGGCACATGG | GCTGCGGACCAGACAGAT | 201 | G06069 |
| 24 | D17S2154 | GATAAAAACAAGCACTGGCTCC | CCCACGGCTTTCTTGATCTA | 137 | G15440 |
| 25 | D17S1955 | TGTAATGTAAGCCCCATGAGG | CACTCAACTCAACAGTCTAAAGGTG | 180 | G11900 |
| 26 | D17S2098 | GTGAGTTCAAGCATAGTAATTATCC | ATTCAGCCTCAGTTCACTGCTTC | 181 | G13994 |
| 27 | D17S518 | GATCCAGTGGAGACTCAGAG | TAGTCTCTGGGACACCCAGA | 88 - 100 | X60690 |
| 28 | D17S1851 | ATTCCTGAGTGTCTACCCTGTTGAG | ACTGACTGCGCCACTGC | 237 - 253 | Z53675 |
| 29 | D11S4358 | TCGAGAAGGACAAAATCACC | GAACAGGGTTAGTCCATTCG | 58 | - |

**Table 6** (continued)

| No. | ID | forward | reverse | PCR size (bp) | GB_ID |
|---|---|---|---|---|---|
| 30 | D17S964 | GTTCTTTCCTCTTGTGGGG | AGTCAGCTGAGATTGTGCC | 224 | L36695 |
| 31 | D19S1091 | CAAGCCAAGACATCCCAGTT | CCCCACACACAGCTCATATG | 238 | G14589 |
| 32 | D17S1179 | TTTTCTCTCTCATTCCATTGGG | GCAACAGAGGGAGACTCCAA | 113 - 125 | - |
| 33 | D10S2160 | TCCCATCCCGTAAGACCTC | TATGGAGTACCTACTCTATGCCAGG | 349 | G06592 |
| 34 | D17S1230 | ATTCAAAGCTGGATCCCTTT | AGCTGTGACAAATGCCTGTA | 108 | L32949 |
| 35 | D17S1338 | TCACCTGAGATTGGGAGACC | AAGATGGGGCAGGAATGG | 178 - 200 | - |
| 36 | D17S2011 | TCACTGTCCTCCAAGCCAG | AAACACCACACTCTCCCCTG | 115 | G07143 |
| 37 | D17S1237 | TTCTTGGGCTTCCCGTAGCC | GGGGCAGACGACTTCTCCTT | 186 | L32947 |
| 38 | D17S2038 | GGGGATACAACCTTTAAAGTTCC | ATTCACCTAATGAGGATTCTTCTTT | 228 | G6219 |
| 39 | D17S2091 | GCTGAAATAGCCATCTTGAGCTAC | TCCGCATCCTTTTTAAGAGGCAC | 157 | G13941 |
| 40 | D17S649 | CTTTCACTCTTTCAGCTGAAGAGG | TGACGTGCTATTTCCTGTTTTGTCT | 146 | L36685 |
| 41 | D17S1190 | GTTTGTTGCTATGCCTGC | CAACACACTACCCCAGGA | 122 | L18197 |
| 42 | M87506 | ACTCCTCATCTGTAGGGTCT | GAGTCCGCTACCTGAGTGCT | 102 - 120 | m87506 |

**Claims**

1. A method for the prediction, diagnosis or prognosis of malignant neoplasia by the detection of at least 2 markers **characterized in that** the markers are genes and fragments thereof or genomic nucleic acid sequences that are located on one chromosomal region which is altered in malignant neoplasia.

2. A method for the prediction, diagnosis or prognosis of malignant neoplasia by the detection of at least 2 markers **characterized in that** the markers are:

   a) genes that are located on one or more chromosomal region(s) which is/are altered in malignant neoplasia; and

   b)

      i) receptor and ligand; or
      ii) members of the same signal transduction pathway; or
      iii) members of synergistic signal transduction pathways; or
      iv) members of antagonistic signal transduction pathways; or
      v) transcription factor and transcription factor binding site.

3. The method of claim 1 or 2 wherein the malignant neoplasia is breast cancer, ovarian cancer, gastric cancer, colon cancer, esophageal cancer, mesenchymal cancer, bladder cancer or non-small cell lung cancer.

4. The method of claim 1 or 2 wherein at least one chromosomal region is defined as the cytogenetic region: 1p13, 1q32, 3p21-p24, 5p13-p14, 8q23-q$^{24}$, 1 1q13, 12q13,17q12-q24 or 20q13.

5. The method of claim 1 or 2 wherein at least chromosomal region is defined as the cytogenetic region 17q1 1.2-21.3 and the malignant neoplasia is breast cancer, ovarian cancer, gastric cancer, colon cancer, esophageal cancer, mesenchymal cancer, bladder cancer or non-small cell lung cancer.

6. The method of claim 1 or 2 wherein at least one chromosomal region is defined as the cytogenetic region 3p21-24 and the malignant neoplasia is breast cancer, ovarian cancer, gastric cancer, colon cancer, esophageal cancer, mesenchymal cancer, bladder cancer or non-small cell lung cancer.

7. The method of claim 1 or 2 wherein at least one chromosomal region is defined as the cytogenetic region 12q13 and the malignant neoplasia is breast cancer, ovarian cancer, gastric cancer, colon cancer, esophageal cancer, mesenchymal cancer, bladder cancer or non-small cell lung cancer.

8. A method for the prediction, diagnosis or prognosis of malignant neoplasia by the detection of at least one marker whereby the marker is a VNTR, SNP, RFLP or STS **characterized in that** the marker is located on one chromosomal region which is altered in malignant neoplasia due to amplification and the marker is detected in a cancerous and a non-cancerous tissue or biological sample of the same individual.

9. The method of claim 8 wherein the marker is selected from the group consisting of the VNTRs:

D17S946, D17S1181, D17S2026, D17S838, D17S250, D17S1818, D17S614, D17S2019, D17S608, D17S1655, D17S2147, D17S754, D17S1814, D17S2007, D17S1246, D17S1979, D17S1984, D17S1984, D17S1867, D17S1788, D17S1836, D17S1787, D17S1660, D17S2154, D17S1955, D17S2098, D17S518, D17S1851, D11S4358, D17S964, D19S1091, D17S1179, D10S2160, D17S1230, D17S1338, D17S2011, D17S1237, D17S2038, D17S2091, D17S649, D17S1190 and M87506.

10. The method of claim 8 wherein the marker is selected from the group consisting of the SNPs:

rs2230698, rs2230700, rs1058808, rs1801200, rs903506, rs2313170, rs1136201, rs2934968, rs2172826, rs1810132, rs1801201, rs2230702, rs2230701, rs1126503, rs3471, rs13695, rs471692, rs558068, rs1064288, rs1061692, rs520630, rs782774, rs565121, rs2586112, rs532299, rs2732786, rs1804539, rs1804538, rs1804537, rs1141364 rs12231, rs1132259 rs1132257, rs113225 rs113225 rs1132254, rs113225 rs1132268 and rs11322

11. A method for the prediction, diagnosis or prognosis of malignant neoplasia by the detection of at least one marker **characterized in that** the marker is selected from:

a) a polynucleotide or polynucleotide analog comprising at least one of the sequences of SEQ ID NO: 2 to 6, 8, 9, 11 to 16, 18, 19, 21 to 26 or 53 to 75 ;

b) a polynucleotide or polynucleotide analog which hybridizes under stringent conditions to a polynucleotide specified in (a) and encodes a polypeptide exhibiting the same biological function as specified for the respective sequence in Table 2 or 3

c) a polynucleotide or polynucleotide analog the sequence of which deviates from the polynucleotide specified in (a) and (c) due to the generation of the genetic code encoding a polypeptide exhibiting the same biological function as specified for the respective sequence in Table 2 or 3

d) a polynucleotide or polynucleotide analog which represents a specific fragment, derivative or allelic variation of a polynucleotide sequence specified in (a) to (d)

e) a purified polypeptide encoded by a polynucleotide or polynucleotide analog sequence specified in (a) to (e)

f) a purified polypeptide comprising at least one of the sequences of SEQ ID NO: 28 to 32, 34, 35, 37 to 42, 44, 45, 47 to 52 or 76 to 98;

are detected.

12. A method for the prediction, diagnosis or prognosis of malignant neoplasia by the detection of at least 2 markers **characterized in that** at least 2 markers are selected from:

a) a polynucleotide or polynucleotide analog comprising at least one of the sequences of SEQ ID NO: 1 to 26 or 53 to 75;

b) a polynucleotide or polynucleotide analog which hybridizes under stringent conditions to a polynucleotide specified in (a) and encodes a polypeptide exhibiting the same biological function as specified for the respective sequence in Table 2 or 3

c) a polynucleotide or polynucleotide analog the sequence of which deviates from the polynucleotide specified in (a) and (b) due to the generation of the genetic code encoding a polypeptide exhibiting the same biological function as specified for the respective sequence in Table 2 or 3

d) a polynucleotide or polynucleotide analog which represents a specific fragment, derivative or allelic variation of a polynucleotide sequence specified in (a) to (c)

e) a purified polypeptide encoded by a polynucleotide sequence or polynucleotide analog specified in (a) to (d)

f) a purified polypeptide comprising at least one of the sequences of SEQ ID NO: 27 to 52 or 76 to 98

are detected.

**13.** The method of any of the claims 1 or 12 wherein the detection method comprises the use of PCR, arrays or beads.

**14.** A diagnostic kit comprising instructions for conducting the method of any of claims 1 to 13.

**15.** A composition for the prediction, diagnosis or prognosis of malignant neoplasia comprising:

a) a detection agent for:

i) any polynucleotide or polynucleotide analog comprising at least one of the sequences of SEQ ID NO: 2 to 6, 8, 9, 11 to 16, 18,19,21 to 26 or 53 to 75;

ii) any polynucleotide or polynucleotide analog which hybridizes under stringent conditions to a polynucleotide specified in (a) encoding a polypeptide exhibiting the same biological function as specified for the respective sequence in Table 2 or 3

iii) a polynucleotide or polynucleotide analog the sequence of which deviates from the polynucleotide specified in (a) and (b) due to the generation of the genetic code encoding a polypeptide exhibiting the same biological function as specified for the respective sequence in Table 2 or 3

iv) a polynucleotide or polynucleotide analog which represents a specific fragment, derivative or allelic variation of a polynucleotide sequence specified in (a) to (c)

v) a polypeptide encoded by a polynucleotide or polynucleotide analog sequence specified in (a) to (d);

vi) a polypeptide comprising at least one of the sequences of SEQ ID NO: 28 to 32, 34, 35, 37 to 42, 44, 45, 47 to 52 or 76 to 98.

or

b) at least 2 detection agents for at least 2 markers selected from:

i) any polynucleotide comprising at least one of the sequences of SEQ ID NO: 1 to 26 or 53 to 75;

ii) any polynucleotide which hybridizes under stringent conditions to a polynucleotide specified in (a) encoding a polypeptide exhibiting the same biological function as specified for the respective sequence in Table 2 or 3

iii) a polynucleotide the sequence of which deviates from the polynucleotide specified in (a) and (b) due to the generation of the genetic code encoding a polypeptide exhibiting the same biological function as specified for the respective sequence in Table 2 or 3

iv) a polynucleotide which represents a specific fragment, derivative or allelic variation of a polynucleotide sequence specified in (a) to (c)

v) a polypeptide encoded by a polynucleotide sequence specified in (a) to (d);

vi) a polypeptide comprising at least one of the sequences of SEQ ID NO: 27 to 52 or 76 to 98.

**16.** An array comprising a plurality of polynucleotides or polynucleotide analogs wherein each of the polynucleotides is selected from:

a) a polynucleotide or polynucleotide analog comprising at least one of the sequences of SEQ ID NO: 1 to 26 or 53 to 75;

b) a polynucleotide or polynucleotide analog which hybridizes under stringent conditions to a polynucleotide specified in (a) encoding a polypeptide exhibiting the same biological function as specified for the respective sequence in Table 2 or 3

c) a polynucleotide or polynucleotide analog the sequence of which deviates from the polynucleotide specified in (a) and (b) due to the generation of the genetic code encoding a polypeptide exhibiting the same biological function as specified for the respective sequence in Table 2 or 3

d) a polynucleotide or polynucleotide analog which represents a specific fragment, derivative or allelic variation of a polynucleotide sequence specified in (a) to (c)

attached to a solid support.

**17.** A method of screening for agents which regulate the activity of a polypeptide encoded by a polynucleotide or polynucleotide analog selected from the group consisting of:

a) a polynucleotide or polynucleotide analog comprising at least one of the sequences of SEQ ID NO: 2 to 6, 8, 9, 11 to 16, 18, 19, 21 to 26 or 53 to 75;

b) a polynucleotide or polynucleotide analog which hybridizes under stringent conditions to a polynucleotide specified in (a) encoding a polypeptide exhibiting the same biological function as specified for the respective sequence in Table 2 or 3

c) a polynucleotide or polynucleotide analog the sequence of which deviates from the polynucleotide specified in (a) and (b) due to the generation of the genetic code encoding a polypeptide exhibiting the same biological function as specified for the respective sequence in Table 2 or 3

d) a polynucleotide or polynucleotide analog which represents a specific fragment, derivative or allelic variation of a polynucleotide sequence specified in (a) to (c);

comprising the steps of:

i) contacting a test compound with at least one polypeptide encoded by a polynucleotide specified in (a) to (d); and

ii) detecting binding of the test compound to the polypeptide, wherein a test compound which binds to the polypeptide is identified as a potential therapeutic agent for modulating the activity of the polypeptide in order to prevent of treat malignant neoplasia.

**18.** A method of screening for agents which regulate the activity of a polypeptide encoded by a polynucleotide or polynucleotide analog selected from the group consisting of:

a) a polynucleotide or polynucleotide analog comprising at least one of the sequences of SEQ ID NO: 2 to 6, 8, 9, 11 to 16, 18, 19, 21 to 26 or 53 to 75;

b) a polynucleotide or polynucleotide analog which hybridizes under stringent conditions to a polynucleotide specified in (a) encoding a polypeptide exhibiting the same biological function as specified for the respective sequence in Table 2 or 3

c) a polynucleotide or polynucleotide analog the sequence of which deviates from the polynucleotide specified in (a) and (b) due to the generation of the genetic code encoding a polypeptide exhibiting the same biological function as specified for the respective sequence in Table 2 or 3

d) a polynucleotide or polynucleotide analog which represents a specific fragment, derivative or allelic variation of a polynucleotide sequence specified in (a) to (c)

comprising the steps of:

i) contacting a test compound with at least one polypeptide encoded by a polynucleotide specified in (a) to (d); and

ii) detecting the activity of the polypeptide as specified for the respective sequence in Table 2 or 3, wherein a test compound which increases the activity is identified as a potential preventive or therapeutic agent for increasing the polypeptide acitivity in malignant neoplasia, and wherein a test compound which decreases the activity of the polypeptide is identified as a potential therapeutic agent for decreasing the polypeptide activity in malignant neoplasia.

**19.** A method of screening for agents which regulate the activity of a polynucleotide or polynucleotide analog selected from group consisting of;

a) a polynucleotide or polynucleotide analog comprising at least one of the sequences of SEQ ID NO: 2 to 6, 8, 9, 11 to 16, 18, 19, 21 to 26 or 53 to 75;

b) a polynucleotide or polynucleotide analog which hybridizes under stringent conditions to a polynucleotide specified in (a) encoding a polypeptide exhibiting the same biological function as specified for the respective sequence in Table 2 or 3

c) a polynucleotide or polynucleotide analog the sequence of which deviates from the polynucleotide specified in (a) and (b) due to the generation of the genetic code encoding a polypeptide exhibiting the same biological function as specified for the respective sequence in Table 2 or 3

d) a polynucleotide or polynucleotide analog which represents a specific fragment, derivative or allelic variation of a polynucleotide sequence specified in (a) to (c)

comprising the steps of:

i) contacting a test compound with at least one polynucleotide or polynucleotide analog specified in (a) to (d), and

ii) detecting binding of the test compound to the polynucleotide, wherein a test compound which binds to the polynucleotide is identified as a potential preventive or therapeutic agent for regulating the activity of the polynucleotide in malignant neoplasia.

**20.** Use of

a) a polynucleotide or polynucleotide analog comprising at least one of the sequences of SEQ ID NO: 2 to 6, 8, 9, 11 to 16, 18, 19, 21 to 26 or 53 to 75;

b) a polynucleotide which hybridizes under stringent conditions to a polynucleotide or polynucleotide analog specified in (a) encoding a polypeptide exhibiting the same biological function as specified for the respective sequence in Table 2 or 3;

c) a polynucleotide or polynucleotide analog the sequence of which deviates from the polynucleotide specified in (a) and (b) due to the generation of the genetic code encoding a polypeptide exhibiting the same biological function as specified for the respective sequence in Table 2 or 3;

d) a polynucleotide or polynucleotide analog which represents a specific fragment, derivative or allelic variation of a polynucleotide sequence specified in (a) to (c);

e) an antisense molecule targeting specifically one of the polynucleotide sequences specified in (a) to (d);

f) a purified polypeptide encoded by a polynucleotide or polynucleotide analog sequence specified in (a) to (d)

g) a purified polypeptide comprising at least one of the sequences of SEQ ID NO: 28 to 32, 34, 35, 37 to 42, 44, 45, 47 to 52 or 76 to 98;

h) an antibody capable of binding to one of the polynucleotide specified in (a) to (d) or a polypeptide specified in (f) and (g);

i) a reagent identified by any of the methods of claim 17 to 19 that modulates the amount or activity of a polynucleotide sequence specified in (a) to (d) or a polypeptide specified in (f) and (g);

in the preparation of a composition for the prevention, prediction, diagnosis, prognosis or a medicament for the treatment of malignant neoplasia.

**21.** Use of claim 20 wherein the disease is breast cancer.

**22.** A reagent that regulates the activity of a polypeptide selected from the group consisting of:

a) a polypeptide encoded by any polynucleotide or polynucleotide analog comprising at least one of the sequences of SEQ ID NO: 2 to 6, 8, 9, 11 to 16, 18, 19, 21 to 26 or 53 to 75;

b) a polypeptide encoded by any polynucleotide or polynucleotide analog which hybridizes under stringent conditions to any polynucleotide comprising at least one of the sequences of SEQ ID NO: 2 to 6, 8, 9, 11 to 16, 18, 19, 21 to 26 or 53 to 75 encoding a polypeptide exhibiting the same biological function as specified for the respective sequence in Table 2 or 3

c) a polypeptide encoded by any polynucleotide or polynucleotide analog the sequence of which deviates from the polynucleotide specified in (a) and (b) due to the generation of the genetic code encoding a polypeptide exhibiting the same biological function as specified for the respective sequence in Table 2 or 3

d) a polypeptide encoded by any polynucleotide or polynucleotide analog which represents a specific fragment, derivative or allelic variation of a polynucleotide sequence specified in (a) to (c)_encoding a polypeptide exhibiting the same biological function as specified for the respective sequence in Table 2 or 3

e) or a polypeptide comprising at least one of the sequences of SEQ ID NO: 28 to 32, 34, 35, 37 to 42, 44, 45, 47 to 52 or 76 to 98;

wherein said reagent is identified by the method of any of the claims 17 to 19.

**23.** A reagent that regulates the activity of a polynucleotide or polynucleotide analog selected from the group consisting of:

a) a polynucleotide or polynucleotide analog comprising at least one of the sequences SEQ ID NO: 2 to 6, 8, 9, 11 to 16, 18, 19, 21 to 26 or 53 to 75;

b) a polynucleotide or polynucleotide analog which hybridizes under stringent conditions to a polynucleotide specified in (a) encoding a polypeptide exhibiting the same biological function as specified for the respective sequence in Table 2 or 3

c) a polynucleotide or polynucleotide analog the sequence of which deviates from the polynucleotide specified in (a) and (b) due to the generation of the genetic code encoding a polypeptide exhibiting the same biological function as specified for the respective sequence in Table 2 or 3

d) a polynucleotide or polynucleotide analog which represents a specific fragment, derivative or allelic variation of a polynucleotide sequence specified in (a) to (c)_encoding a polypeptideexhibiting the same biological function as specified for the respective sequence in Table 2 or 3

wherein said reagent is identified by the method of any of the claims 17 to 19.

**24.** A pharmaceutical composition, comprising:

a) an expression vector containing at least one polynucleotide or polynucleotide analog selected from the group consisting of:

i) a polynucleotide or polynucleotide analog comprising at least one of the sequences of SEQ ID NO: 2 to 6, 8, 9, 11 to 16, 18, 19,21 to 26 or 53 to 75;

ii) a polynucleotide or polynucleotide analog which hybridizes under stringent conditions to a polynucleotide specified in (a) encoding a polypeptide exhibiting the same biological function as specified for the respective sequence in Table 2 or 3

iii) a polynucleotide or polynucleotide analog the sequence of which deviates from the polynucleotide specified in (a) and (b) due to the generation of the genetic code_encoding a polypeptide exhibiting the same biological function as specified for the respective sequence in Table 2 or 3

iv) a polynucleotide or polynucleotide analog which represents a specific fragment, derivative or allelic variation of a polynucleotide sequence specified in (a) to (c)_encoding a polypeptide exhibiting the same biological function as specified for the respective sequence in Table 2 or 3;

or the reagent of claim 22 or 23 and a pharmaceutically acceptable carrier.

**25.** A computer-readable medium comprising:

a) at least one digitally encoded value representing a level of expression of at least one polynucleotide sequence of SEQ ID NO: 2 to 6, 8, 9, 11 to 16,18,19,21 to 26 or 53 to 75

b) al least 2 digitally encoded values representing the levels of expression of at least 2 polynucleotide sequences selected from SEQ ID NO: 1 to 26 or 53 to 75

in a cell from the a subject at risk for or having malignant neoplasia.

**26.** A method for the detection of chromosomal alterations **characterized in that** the relative abundance of individual mRNAs, encoded by genes, located in altered chromosomal regions is detected.

**27.** A method for the detection of chromosomal alterations **characterized in that** the copy number of one or more chromosomal region(s) is detected by quantitative PCR.

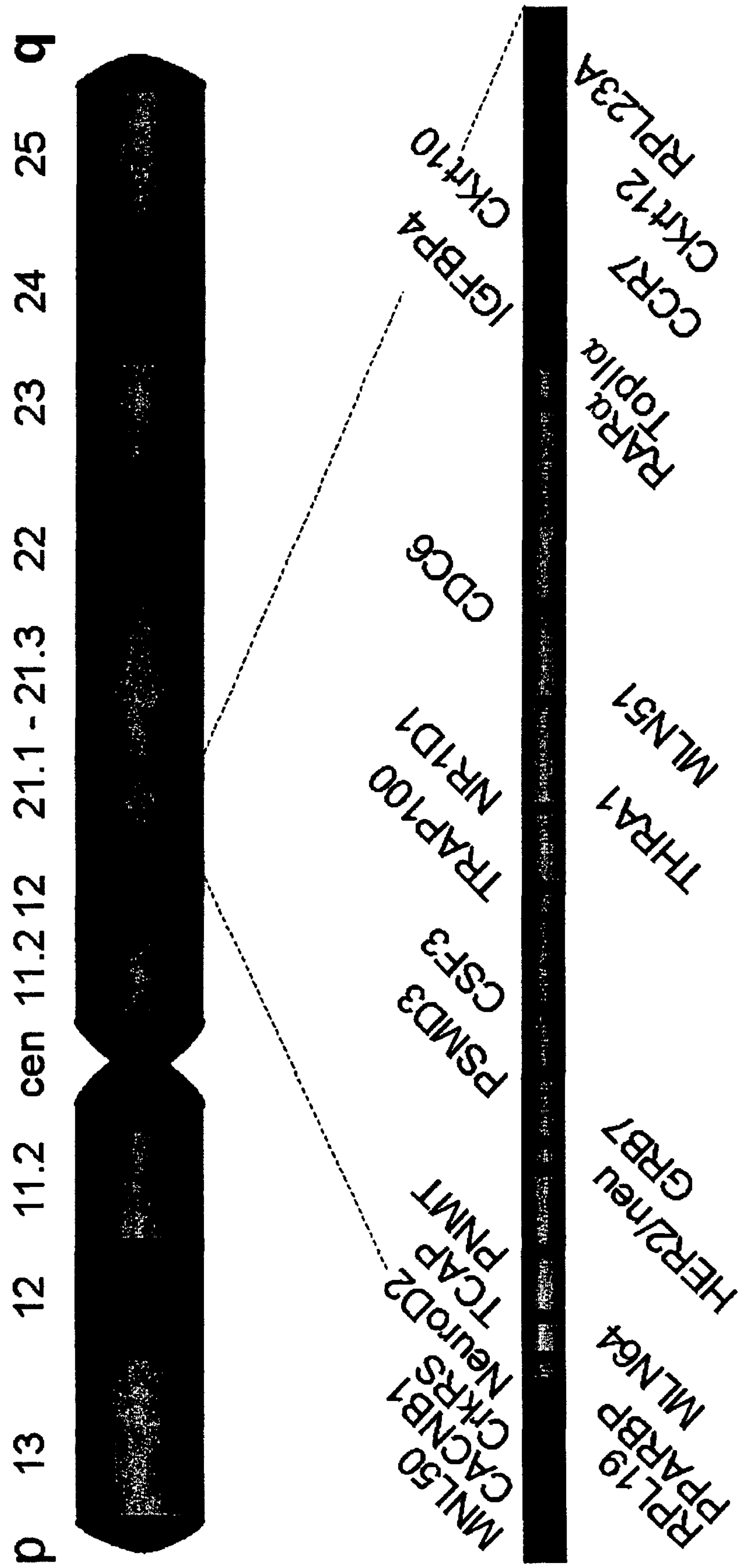

p
13
12
11.2
cen
11.2
12
21.1 - 21.3
22
23
24
25
q
MNL50
CACNB1
CrkRS
NeuroD2
TCAP
PNMT
PSMD3
CSF3
TRAP100
NR1D1
CDC6
IGFBP4
CKrt10
RPL19
PPARBP
MLN64
HER2/neu
GRB7
THRA1
MLN51
RARα
TopIIα
CCR7
CKrt12
RPL23A


Figure 1

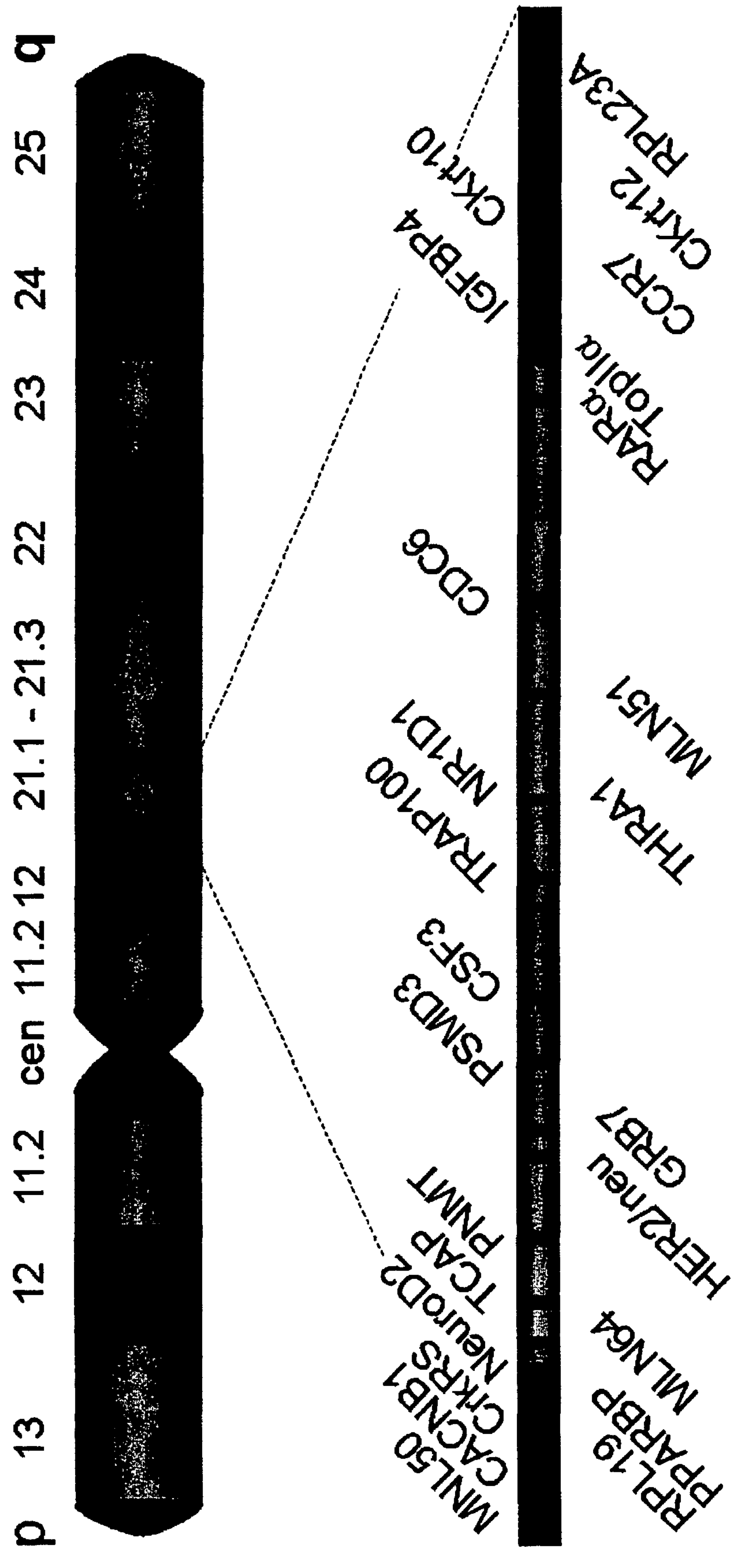

p
13
12
11.2
cen
11.2
12
21.1 - 21.3
22
23
24
25
q
MNL50
CACNB1
CrkRS
NeuroD2
TCAP
PNMT
PSMD3
CSF3
TRAP100
NR1D1
CDC6
IGFBP4
CKrt10
RPL19
PPARBP
MLN64
HER2/neu
GRB7
THRA1
MLN51
RARα
TopIIα
CCR7
CKrt12
RPL23A


Figure 1

Figure 2

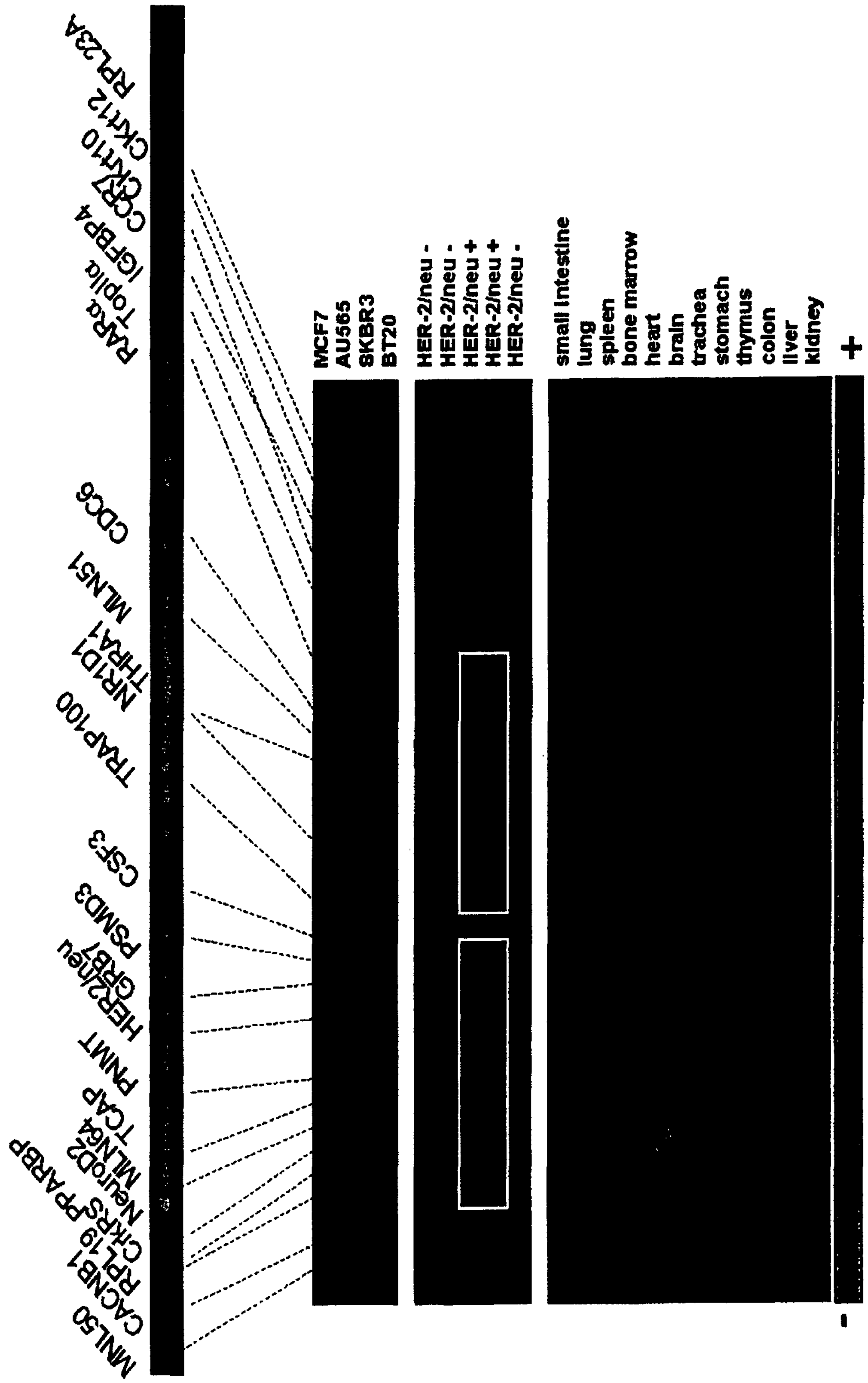
RPL23A
CCR7
IGFBP4
RARα
CDC6
MLN51
NR1D1
THRA1
TRAP100
CSF3
PSMD3
GRB7
HER2/neu
PNMT
TCAP
MLN64
NeuroD2
PPARBP
RPL19
CACNB1
MCF7
AU565
SKBR3
BT20
HER-2/neu -
HER-2/neu -
HER-2/neu +
HER-2/neu +
HER-2/neu -
small intestine
lung
spleen
bone marrow
heart
brain
trachea
stomach
thymus
colon
liver
kidney
+
-

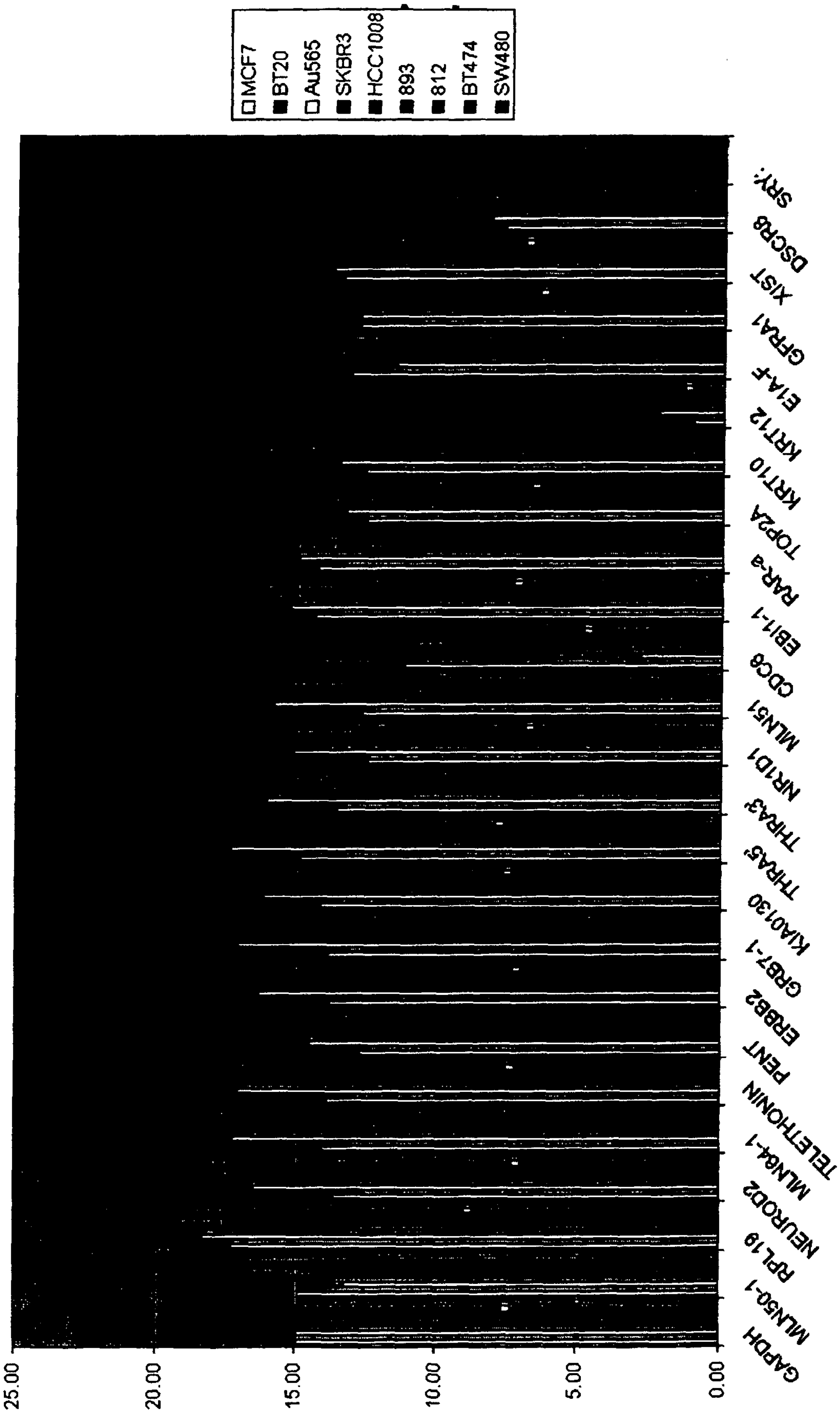
MCF7
BT20
Au565
SKBR3
HCC1008
893
812
BT474
SW480
25.00
20.00
15.00
10.00
5.00
0.00
GAPDH
MLN50-1
RPL19
NEUROD2
MLN64-1
TELETHONIN
PENT
ERBB2
GRB7-1
KIAO130
THRA5'
THRA3'
NR1D1
MLN51
CDC6
EBI1-1
RAR-a
TOP2A
KRT10
KRT12
E1A-F
GFRA1
XIST
DSCR8
SRY.

**Figure 3**

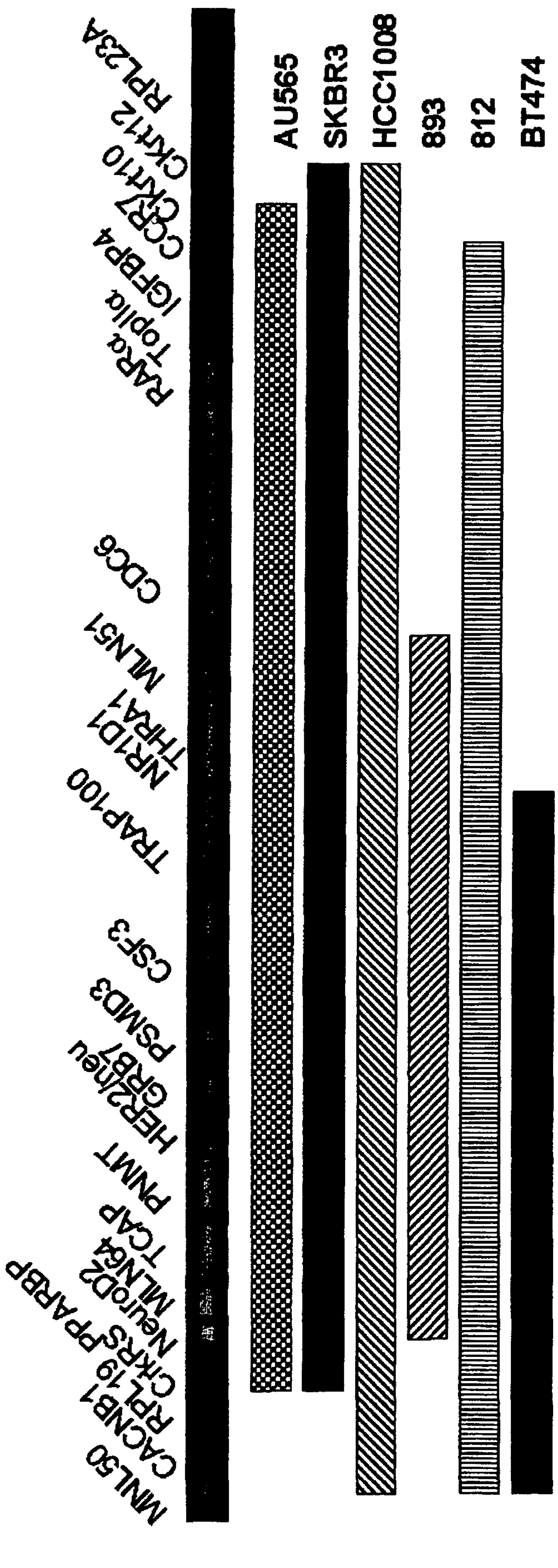
MNL50
CACNB1
RPL19
CrkRS
PPARBP
NeuroD2
MLN64
TCAP
PNMT
HER2/neu
GRB7
PSMD3
CSF3
TRAP100
NR1D1
THRA1
MLN51
CDC6
RARα
Top2α
IGFBP4
CCR7
CKrt10
CKrt12
RPL23A
AU565
SKBR3
HCC1008
893
812
BT474

Figure 4